(19) Europäisches Patentamt
European Patent Office
Office européen des brevets

(11) EP 4 227 307 A1

(12) # EUROPEAN PATENT APPLICATION

(43) Date of publication:
**16.08.2023 Bulletin 2023/33**

(21) Application number: **22156277.0**

(22) Date of filing: **11.02.2022**

(51) International Patent Classification (IPC):
***C07D 487/04*** *(2006.01)* ***C07D 519/00*** *(2006.01)*
***A61K 31/4985*** *(2006.01)* ***A61P 35/00*** *(2006.01)*

(52) Cooperative Patent Classification (CPC):
**C07D 487/04; A61P 35/00; C07D 519/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Genzyme Corporation**
**Cambridge, MA 02142 (US)**
- **Revolution Medicines, Inc.**
**Redwood City, CA 94063 (US)**

(72) Inventor: **The designation of the inventor has not yet been filed**

(74) Representative: **Sanofi**
**Patent Department**
**46 avenue de la Grande Armée**
**75017 Paris (FR)**

(54) **PYRAZOLOPYRAZINE COMPOUNDS AS SHP2 INHIBITORS**

(57) Provided herein are compounds and pharmaceutical compositions thereof for modulating SHP2 and their use in the treatment of disease.

EP 4 227 307 A1

## Description

### FIELD OF INVENTION

The present disclosure relates to inhibitors of protein tyrosine phosphatase SHP2 useful for treating diseases or disorders such as cancer. Specifically, this disclosure describes compounds and compositions inhibiting SHP2, methods of treating diseases associated with SHP2, and methods of synthesizing these compounds.

### BACKGROUND OF THE DISCLOSURE

SH2 domain-containing protein tyrosine phosphatase-2 (SHP2) is a non-receptor protein tyrosine phosphatase encoded by the PTPN11 gene that contributes to multiple cellular functions including proliferation, differentiation, cell cycle maintenance, and migration. SHP2 is involved in signaling through the Ras-mitogen-activated protein kinase, the JAK-STAT, or the phosphoinositol 3-kinase-AKT pathways.

Mutations in the PTPN11 gene and subsequently in SHP2 have been identified in several human diseases such as Noonan Syndrome, Leopard Syndrome, juvenile myelomonocytic leukemias, melanoma, neuroblastoma, acute myeloid leukemia, and cancers of the breast, lung, colon, and brain, including glioblastoma (Chan, G. et al., Cancer Metastasis Rev. 2008, 27, 179-192; Zhang, J. et al., J. Cell. Mol. Med. 2015, 19, 2075-2083; Roccograndi L. et al., J. Neuro-Oncol. 2017, 135, 487-496; Mitra R. et al., ChemMedChem 2021, 16, 777-787). As such, SHP2 represents a highly attractive target for the development of novel therapies for the treatment of various diseases including cancer. For treating or preventing cancers associated with the brain, a SHP2 inhibitor with brain penetration capability is particularly attractive.

Accordingly, in one aspect, provided herein are compounds which are modulators of SHP2 for use in treating diseases such as cancer.

### SUMMARY OF THE DISCLOSURE

Described herein, in certain embodiments, are compounds and compositions thereof for modulating SHP2 for treating diseases such as cancer.

The following embodiments are encompassed.

Embodiment 1. A compound of Formula (I):

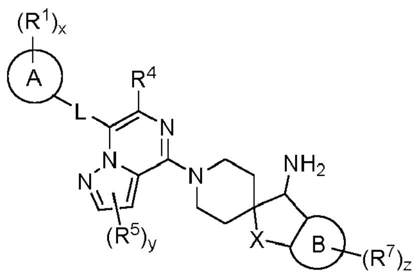

(I)

or a pharmaceutically acceptable salt thereof, wherein:

Ring A is $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S;

each $R^1$ is independently halo, cyano, $-NR^{2a}R^{2b}$, $C_1$-$C_6$ alkyl, oxo, hydroxy, $C_1$-$C_6$ haloalkyl, $C_1$-$C_6$ alkoxy, $C_1$-$C_6$ alkyl-OH, $C_1$-$C_6$ alkyl-CN, $-C(O)NR^{2a}R^{2b}$, $-C(O)(C_1$-$C_6$ alkyl), $-CO_2H$, $-CO_2(C_1$-$C_6$ alkyl), $-Si(R^a)(R^b)(R^c)$, $-P(O)(R^a)(R^b)$, $-OP(O)(R^a)(R^b)$, $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S;

or two $R^1$ groups are taken together with the carbon atoms or heteroatoms to which they are attached to form a fused phenyl, 5- to 6-membered heterocycloalkyl, or 5- to 6-membered heteroaryl, each of which is optionally substituted with 1-4 $R^6$ groups, wherein the fused heterocycloalkyl and heteroaryl contain 1-3 heteroatoms selected from N, O, and S;

each $R^a$, $R^b$, and $R^c$ is independently hydroxy, $C_1$-$C_6$ alkyl, or $C_1$-$C_6$ alkoxy;

each $R^{2a}$ and $R^{2b}$ is independently H, $C_1$-$C_6$ alkyl, or $C_3$-$C_6$ cycloalkyl;

L is a bond, S, O, C(O), or N($R^d$);
$R^d$ is H or $C_1$-$C_6$ alkyl;
X is $CR^{3a}R^{3b}$, $NR^{3a}$, or O;
$R^{3a}$ and $R^{3b}$ are independently H or $C_1$-$C_6$ alkyl;
$R^4$ is H, $C_1$-$C_6$ alkyl, $C_1$-$C_6$ alkyl-OH, $C_1$-$C_6$ haloalkyl, or $-NH_2$;
each $R^5$ is independently halo, $C_1$-$C_6$ alkyl, $C_1$-$C_6$ haloalkyl, -($C_1$-$C_6$ akylene)($C_1$-$C_6$ alkoxy), or $C_1$-$C_6$ alkyl-OH;
Ring B is fused phenyl or 5- to 6-membered heteroaryl containing 1-3 heteroatoms selected from N, O, and S;
each $R^6$ is independently $C_1$-$C_6$ alkyl, halo, or $C_1$-$C_6$ haloalkyl;
each $R^7$ is independently $C_1$-$C_6$ alkyl, halo, $C_1$-$C_6$ alkoxy, $C_1$-$C_6$ alkyl-OH, hydroxy, cyano, -Si($R^a$)($R^b$)($R^c$), -P(O)($R^a$)($R^b$), -OP(O)($R^a$)($R^b$), $-NR^{2a}R^{2b}$, or $C_1$-$C_6$ haloalkyl;
x is 0-5;
y is 0-2; and
z is 0-4;

wherein one or more hydrogen atoms in the compound are optionally replaced by deuterium.

Embodiment 2. The compound of embodiment 1, or a pharmaceutically acceptable salt thereof, wherein: Ring A is $C_3$-$C_5$ cycloalkyl, phenyl, 6-membered heterocycloalkyl, or 5- to 6-membered heteroaryl, wherein the heterocycloalkyl and heteroaryl contain 1-2 heteroatoms selected from N, O, and S.

Embodiment 3. The compound of embodiment 1 or 2, or a pharmaceutically acceptable salt thereof, wherein: Ring A is cyclopropyl, phenyl, dihydropyridinyl, dihydropyranyl, pyridinyl, pyridazinyl, pyrimidinyl, pyrazolyl, imidazolyl, pyrrolyl, thiazolyl, isoxazolyl, or thiophenyl.

Embodiment 4. The compound of any one of embodiments 1-3, or a pharmaceutically acceptable salt thereof, wherein:

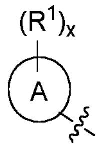 is:

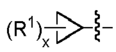, 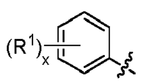, 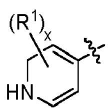, 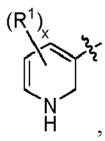, 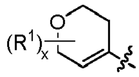, 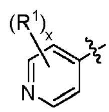,

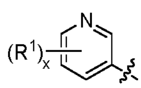, 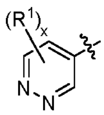, 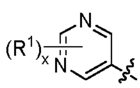, 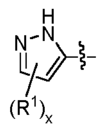, 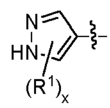, 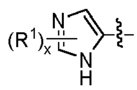,

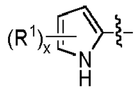, 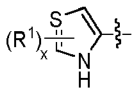, 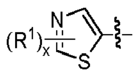, 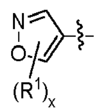, 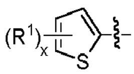, or 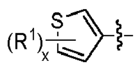.

Embodiment 5. The compound of any one of embodiments 1-4, or a pharmaceutically acceptable salt thereof, wherein: x is 0, 1, 2, or 3.

Embodiment 6. The compound of any one of embodiments 1-5, or a pharmaceutically acceptable salt thereof, wherein:

each $R^1$ is independently halo, cyano, $-NR^{2a}R^{2b}$, $C_1$-$C_3$ alkyl, oxo, hydroxy, $C_1$-$C_3$ haloalkyl, $C_1$-$C_3$ alkoxy, $C_1$-$C_3$ alkyl-OH, $C_1$-$C_3$ alkyl-CN, $-C(O)NR^{2a}R^{2b}$, -C(O)($C_1$-$C_3$ alkyl), $-CO_2H$, $-CO_2$($C_1$-$C_3$ alkyl), -Si($R^a$)($R^b$)($R^c$), -P(O)($R^a$)($R^b$), -OP(O)($R^a$)($R^b$), $C_3$-$C_5$ cycloalkyl, phenyl, or 6-membered heterocycloalkyl, wherein the heterocycloalkyl contains 1-2 heteroatoms selected from N and O;
or two $R^1$ groups are taken together with the carbon atoms or heteroatoms to which they are attached to form a fused phenyl, 5- to 6-membered heterocycloalkyl, or 5- to 6-membered heteroaryl, each of which is optionally

substituted with 1-2 $R^6$ groups, wherein the fused heterocycloalkyl and heteroaryl contain 1-2 heteroatoms selected from N, O, and S;

each $R^a$, $R^b$, and $R^c$ is independently $C_1$-$C_3$ alkyl or $C_1$-$C_3$ alkoxy;

each $R^{2a}$ and $R^{2b}$ is independently H, $C_1$-$C_3$ alkyl, or $C_3$-$C_5$ cycloalkyl; and

each $R^6$ is independently $C_1$-$C_3$ alkyl, halo, or $C_1$-$C_3$ haloalkyl;

Embodiment 7. The compound of any one of embodiments 1-6, or a pharmaceutically acceptable salt thereof, wherein:

each $R^1$ is independently F, Cl, -CN, $-CH_2CN$, $-NH_2$, $-N(H)CH_3$, $-N(CH_3)_2$, $-CH_3$, $-CH_2CH_3$, $-CH(CH_3)_2$, oxo, $-CF_3$, $-OCH_3$, $-CH_2OH$, $-C(O)N(CH_3)_2$, $-C(O)CH_3$, cyclopropyl, or

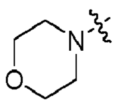 ;

or two $R^1$ groups are taken together with the carbon atoms or heteroatoms to which they are attached to form a fused group selected from:

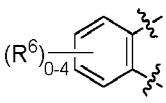 , 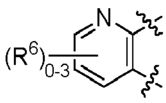 , 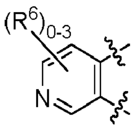 , 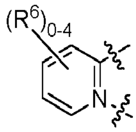 , 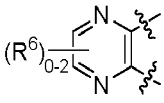 , 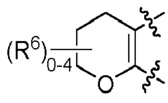 ,

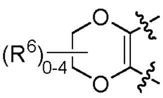 , 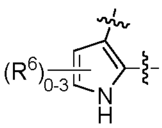 , 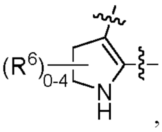 , 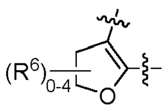 , and 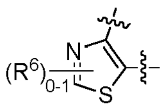

Embodiment 8. The compound of any one of embodiments 1-7, or a pharmaceutically acceptable salt thereof, wherein:

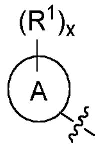 is:

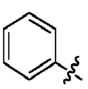 , 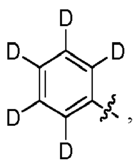 , 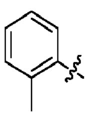 , 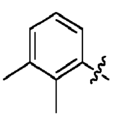 , 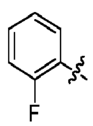 , 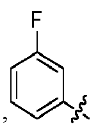 , 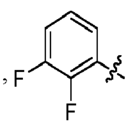 , 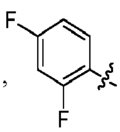 , 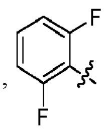 , 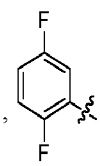 ,

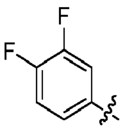 , 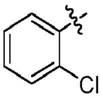 , 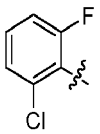 , 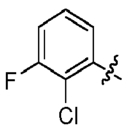 , 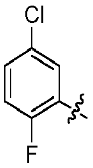 , 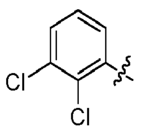 , 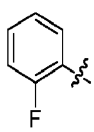 , 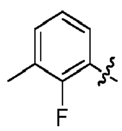 ,

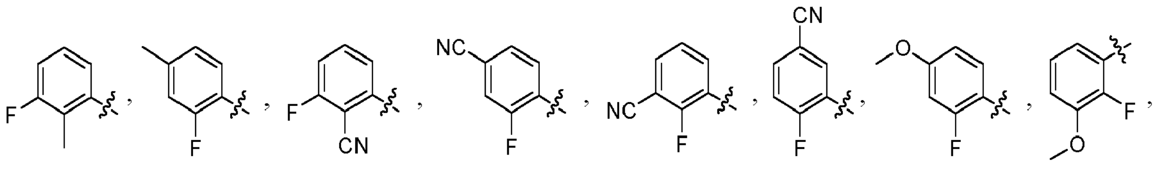
, , , , , , , ,

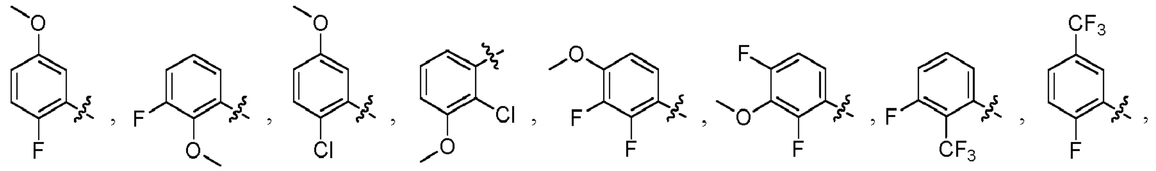
, , , , , , , ,

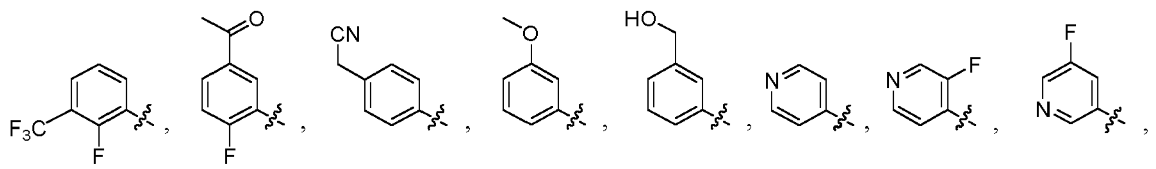
, , , , , , , ,

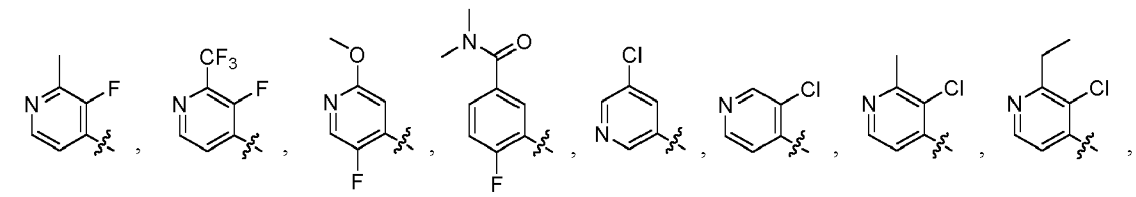
, , , , , , , ,

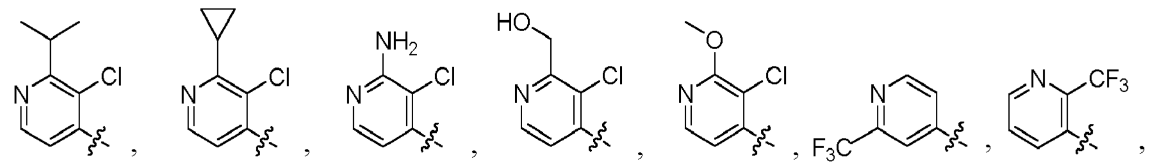
, , , , , , ,

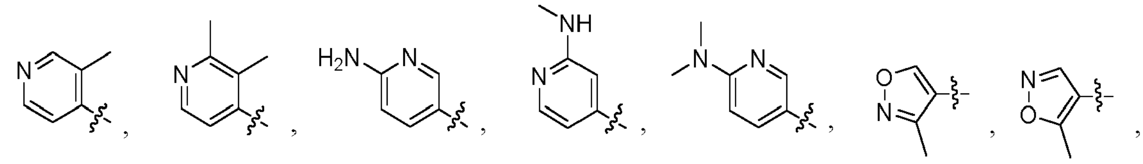
, , , , , , ,

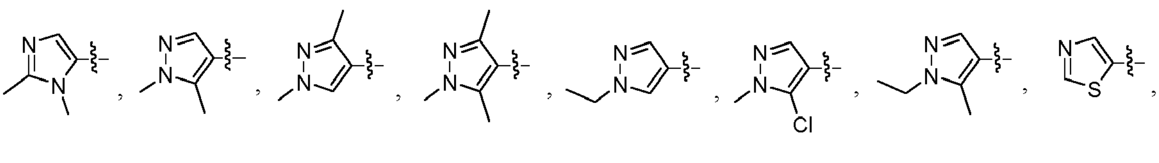
, , , , , , , ,

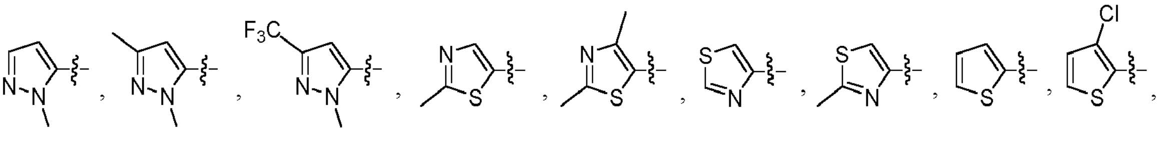
, , , , , , , , ,

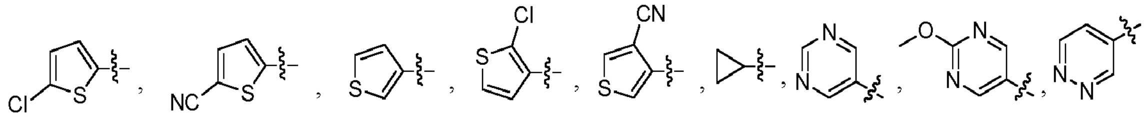

, , , , , , , , ,

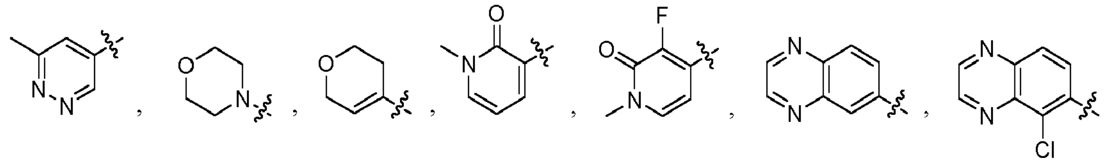

, , , , , , ,

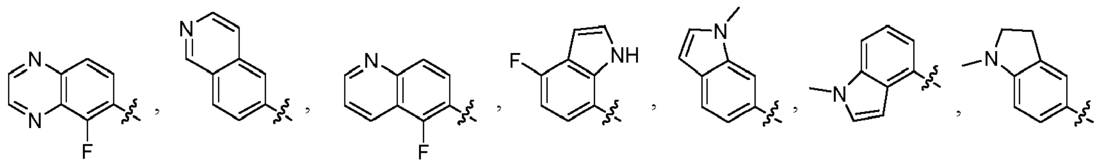

, , , , , , ,

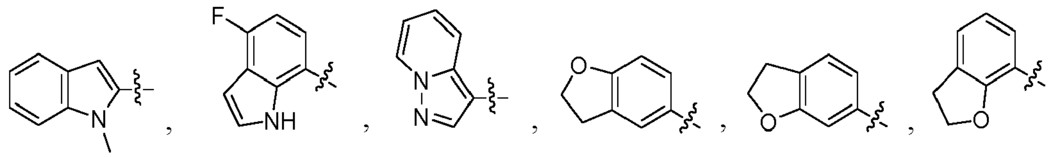

, , , , , ,

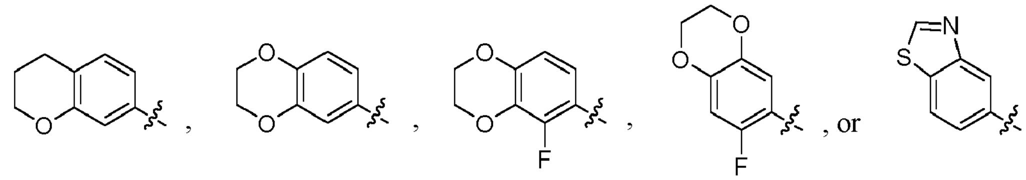

, , , , or .

Embodiment 9. The compound of any one of embodiments 1-8, or a pharmaceutically acceptable salt thereof, wherein: L is a bond.

Embodiment 10. The compound of any one of embodiments 1-8, or a pharmaceutically acceptable salt thereof, wherein: L is S.

Embodiment 11. The compound of any one of embodiments 1-8, or a pharmaceutically acceptable salt thereof, wherein: L is O.

Embodiment 12. The compound of any one of embodiments 1-8, or a pharmaceutically acceptable salt thereof, wherein: L is C(O).

Embodiment 13. The compound of any one of embodiments 1-8, or a pharmaceutically acceptable salt thereof, wherein: L is $N(R^d)$; and $R^d$ is H or $C_1$-$C_3$ alkyl.

Embodiment 14. The compound of any one of embodiments 1-13, or a pharmaceutically acceptable salt thereof, wherein: X is $CR^{3a}R^{3b}$, $NR^{3a}$, or O; and $R^{3a}$ and $R^{3b}$ are independently H or $C_1$-$C_3$ alkyl.

Embodiment 15. The compound of embodiment 14, or a pharmaceutically acceptable salt thereof, wherein: X is $CH_2$, N(H), $N(CH_3)$, or O.

Embodiment 16. The compound of any one of embodiments 1-15, or a pharmaceutically acceptable salt thereof, wherein: $R^4$ is H, $C_1$-$C_3$ alkyl, $C_1$-$C_3$ alkyl-OH, $C_1$-$C_3$ haloalkyl, or $-NH_2$.

Embodiment 17. The compound of embodiment 16, or a pharmaceutically acceptable salt thereof, wherein: $R^4$ is H, $CH_3$, $-CH_2OH$, $-CH_2F$, or $-CHF_2$.

Embodiment 18. The compound of any one of embodiments 1-17, or a pharmaceutically acceptable salt thereof, wherein: y is 0 or 1.

Embodiment 19. The compound of any one of embodiments 1-18, or a pharmaceutically acceptable salt thereof, wherein:

each $R^1$ is independently halo, $C_1$-$C_3$ alkyl, $C_1$-$C_3$ haloalkyl, -($C_1$-$C_3$ alkylene)($C_1$-$C_3$ alkoxy), or $C_1$-$C_3$ alkyl-OH.

Embodiment 20. The compound of embodiment 19, or a pharmaceutically acceptable salt thereof, wherein: each $R^5$ is independently Cl, F, $-CH_2F$, $-CHF_2$, $-CH_2OCH_3$, or $-CH_2OH$.

Embodiment 21. The compound of any one of embodiments 1-20, or a pharmaceutically acceptable salt thereof,

wherein:
Ring B is fused phenyl or 5- to 6-membered heteroaryl containing 1-2 heteroatoms selected from N, O, and S.

Embodiment 22. The compound of embodiment 21, or a pharmaceutically acceptable salt thereof, wherein: Ring B is fused phenyl, pyridyl, pyrimidinyl, pyrazinyl, pyridazinyl, thiazolyl, or oxazolyl.

Embodiment 23. The compound of any one of embodiments 1-22, or a pharmaceutically acceptable salt thereof, wherein: z is 0, 1, or 2.

Embodiment 24. The compound of any one of embodiments 1-23, or a pharmaceutically acceptable salt thereof, wherein:

each $R^7$ is independently $C_1$-$C_3$ alkyl, halo, $C_1$-$C_3$ alkoxy, $C_1$-$C_3$ alkyl-OH, hydroxy, cyano, -Si($R^a$)($R^b$)($R^c$), -P(O)($R^a$)($R^b$), -OP(O)($R^a$)($R^b$), -$NR^{2a}R^{2b}$, or $C_1$-$C_3$ haloalkyl;
each $R^a$, $R^b$, and $R^c$ is independently hydroxy, $C_1$-$C_3$ alkyl, or $C_1$-$C_3$ alkoxy; and
each $R^{2a}$ and $R^{2b}$ is independently H, $C_1$-$C_3$ alkyl, or $C_3$-$C_5$ cycloalkyl.

Embodiment 25. The compound of embodiment 24, or a pharmaceutically acceptable salt thereof, wherein: each $R^7$ is independently $CH_3$, F, $-OCH_3$, $-CH_2OH$, hydroxy, -CN, $-N(CH_3)_2$, or $-CHF_2$.

Embodiment 26. The compound of any one of embodiments 1-25, or a pharmaceutically acceptable salt thereof, wherein:

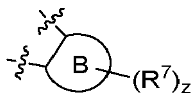

is:

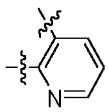, 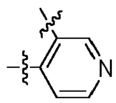, 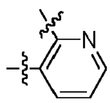, 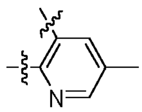, 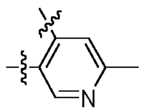, 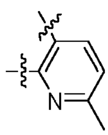, 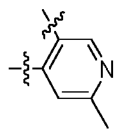,

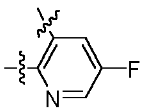, 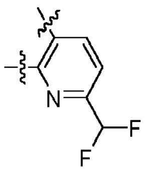, 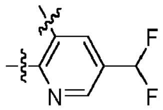, 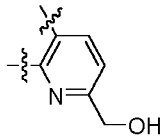, 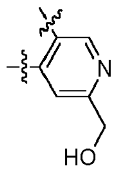, 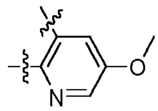,

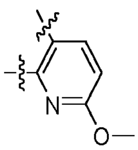, 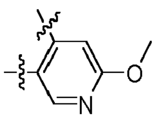, 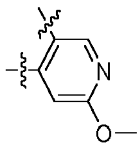, 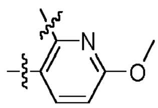, 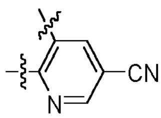, 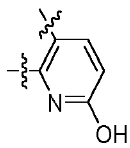,

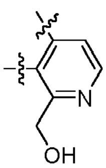, 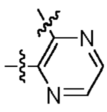, 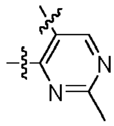, 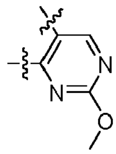, 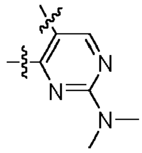, 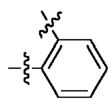,

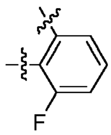, 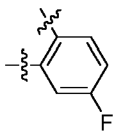, 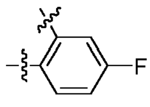, 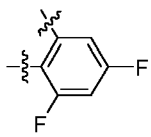, 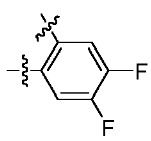, 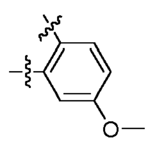,

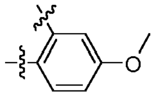, 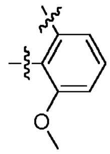, 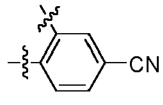, 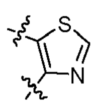, 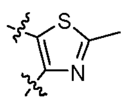, 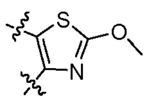,

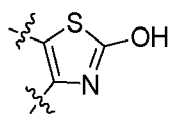, 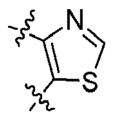, 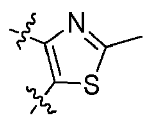, or 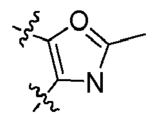.

Embodiment 27. The compound of any one of embodiments 1-26, or a pharmaceutically acceptable salt thereof, wherein the compound is of Formula (IIa), (IIb), (IIc), or (IId):

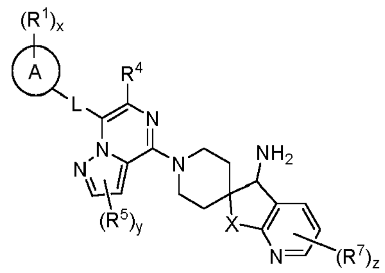

(IIa)

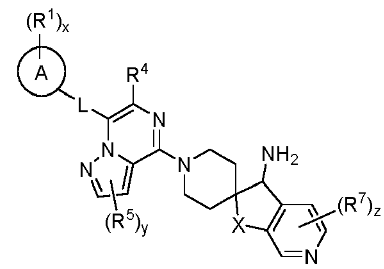

(IIb)

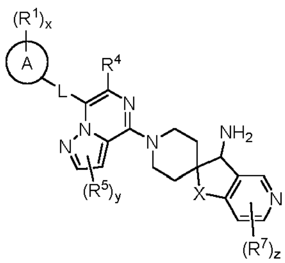

(IIc)

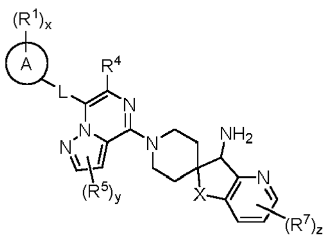

(IId)

Embodiment 28. The compound of embodiment 27, or a pharmaceutically acceptable salt thereof, wherein: L is a bond.

Embodiment 29. The compound of embodiment 27 or 28, or a pharmaceutically acceptable salt thereof, wherein the compound is of Formula (IIa-1):

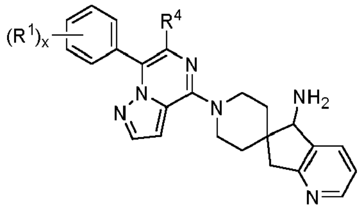

(IIa-1)

Embodiment 30. The compound of embodiment 29, or a pharmaceutically acceptable salt thereof, wherein: each $R^1$ is independently halo; x is 0, 1, or 2; and $R^4$ is $C_1$-$C_6$ alkyl.

Embodiment 31. The compound of embodiment 30, or a pharmaceutically acceptable salt thereof, wherein: $R^1$ is F; xis 0 or 1; and $R^4$ is $-CH_3$.

Embodiment 32. The compound of any one of embodiments 1-26, or a pharmaceutically acceptable salt thereof, wherein the compound is of Formula (IIIa), (IIIb), (IIIc), (IIId), (IIIe), or (IIIf):

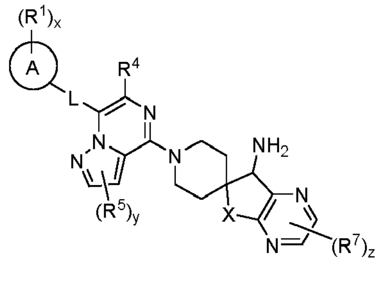

(IIIa)

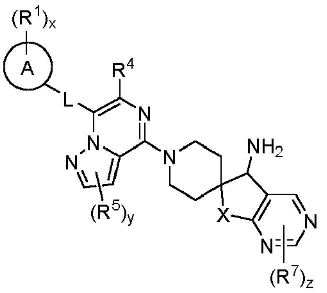

(IIIb)

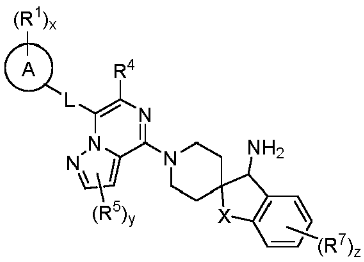

(IIIc)

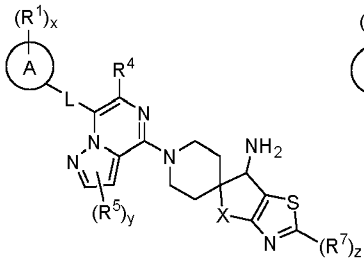

(IIId)

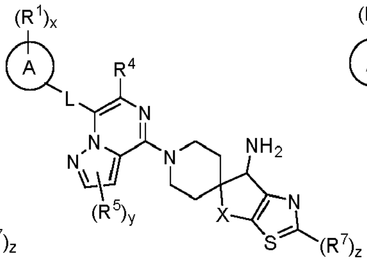

(IIIe)

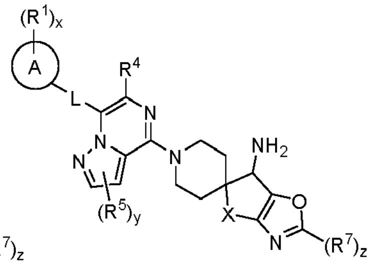

(IIIf)

.

Embodiment 33. The compound of any one of embodiments 1-9 and 14-26, or a pharmaceutically acceptable salt thereof, wherein the compound is of Formula (IVa):

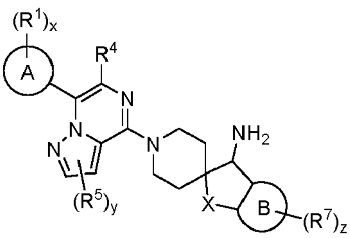

(IVa)

Embodiment 34. The compound of any one of embodiments 1-26, or a pharmaceutically acceptable salt thereof, wherein the compound is of Formula (IVb), (IVc), (IVd), or (IVe):

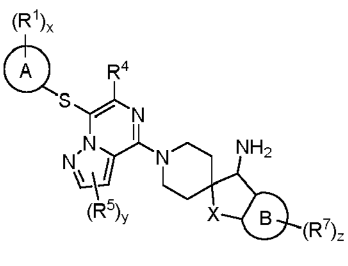

(IVb)

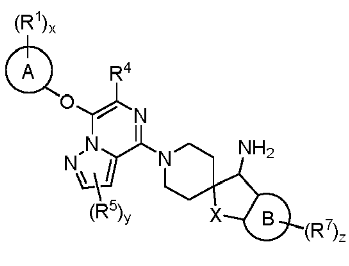

(IVc)

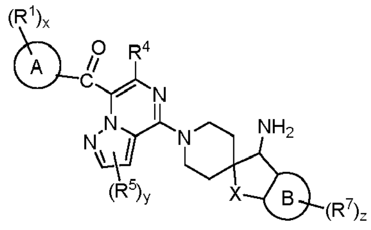

(IVd)

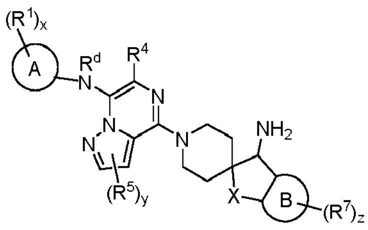

(IVe)

.

Embodiment 35. A compound selected from the compounds of Table 1 or a pharmaceutically acceptable salt thereof.

Embodiment 36. A pharmaceutical composition comprising the compound of any one of embodiments 1-35, or a pharmaceutically acceptable salt thereof, and a pharmaceutically acceptable excipient.

Embodiment 37. A method of inhibiting SHP2 comprising contacting SHP2 with an effective amount of the compound of any one of embodiments 1-35, or a pharmaceutically acceptable salt thereof, or the pharmaceutical composition of embodiment 36.

Embodiment 38. A method of treating a disease associated with SHP2 modulation in a subject in need thereof, comprising administering to the subject an effective amount of the compound of any one of embodiments 1-35, or a pharmaceutically acceptable salt thereof, or the pharmaceutical composition of embodiment 36.

Embodiment 39. The method of embodiment 38, wherein the disease is Noonan Syndrome, Leopard Syndrome, juvenile myelomonocytic leukemias, neuroblastoma, melanoma, acute myeloid leukemia, breast cancer, lung cancer, colon cancer, or brain cancer.

Embodiment 40. The method of embodiment 39, wherein the brain cancer is glioblastoma.

## DETAILED DESCRIPTION OF THE DISCLOSURE

### Definitions

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed. To the extent any material incorporated herein by reference is inconsistent with the express content of this disclosure, the express content controls. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless the context requires otherwise. Furthermore, use of the term "including" as well as other forms, such as "include", "includes," and "included," is not limiting.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

As used herein, ranges and amounts can be expressed as "about" a particular value or range. About also includes the exact amount. Hence "about 5 $\mu$L" means "about 5 $\mu$L" and also "5 $\mu$L." Generally, the term "about" includes an amount that would be expected to be within experimental error, such as for example, within 15%, 10%, or 5%.

The section headings used herein are for organizational purposes only and are not to be construed as limiting the subject matter described.

"Alkyl" refers to an unbranched or branched saturated hydrocarbon chain. As used herein, alkyl has 1 to 20 carbon atoms (i.e., $C_1$-$C_{20}$ alkyl), 1 to 10 carbon atoms (i.e., $C_1$-$C_{10}$ alkyl), 1 to 6 carbon atoms (i.e., $C_1$-$C_6$ alkyl) or 1 to 3 carbon atoms (i.e., $C_1$-$C_3$ alkyl). Examples of alkyl groups include methyl, ethyl, propyl, isopropyl, n-butyl, sec-butyl, iso-butyl, tert-butyl, pentyl, 2-pentyl, isopentyl, neopentyl, hexyl, 2-hexyl, 3-hexyl and 3-methylpentyl. When an alkyl residue having a specific number of carbons is named by chemical name or identified by molecular formula, all positional isomers having that number of carbons may be encompassed; thus, for example, "butyl" includes n-butyl (i.e., $-(CH_2)_3CH_3$), isobutyl (i.e., $-CH_2CH(CH_3)_2$), sec-butyl (i.e., $-CH(CH_3)CH_2CH_3$), and tert-butyl (i.e., $-C(CH_3)_3$); and "propyl" includes n-propyl (i.e., $-(CH_2)_2CH_3$) and isopropyl (i.e., $-CH(CH_3)_2$).

"Alkyl-CN" refers to an unbranched or branched alkyl group as defined above, wherein one or more hydrogen atoms are replaced by -CN. For example, "$C_1$-$C_6$ alkyl-CN" refers to a $C_1$-$C_6$ alkyl which is substituted by one or more -CN groups. An alkyl-CN may contain multiple cyano groups that are attached to the same carbon atom or to multiple

carbon atoms.

"Alkyl-OH" refers to an unbranched or branched alkyl group as defined above, wherein one or more hydrogen atoms are replaced by -OH. For example, "$C_1$-$C_6$ alkyl-OH" refers to a $C_1$-$C_6$ alkyl which is substituted by one or more -OH groups. An alkyl-OH may contain multiple hydroxy groups that are attached to the same carbon atom or to multiple carbon atoms.

An "alkoxy" group is -O-(alkyl), wherein alkyl is defined above.

An "aryl" group is an aromatic carbocyclic group of from 6 to 14 carbon atoms ($C_6$-$C_{14}$ aryl) having a single ring (*e.g.,* phenyl or $C_6$ aryl) or multiple condensed rings (*e.g.,* naphthyl or anthryl). In some embodiments, aryl groups contain 6-14 carbons ($C_6$-$C_{14}$ aryl), and in others from 6 to 12 ($C_6$-$C_{12}$ aryl) or even 6 to 10 carbon atoms ($C_6$-$C_{10}$ aryl) in the ring portions of the groups. Particular aryls include phenyl, biphenyl, naphthyl and the like. An aryl group can be substituted or unsubstituted.

"Cycloalkyl" refers to a saturated or partially unsaturated cyclic alkyl group having a single ring or multiple rings including fused, bridged and spiro ring systems. The term "cycloalkyl" includes cycloalkenyl groups (i.e., the cyclic group having at least one double bond). As used herein, cycloalkyl has from 3 to 20 ring carbon atoms (i.e., $C_3$-$C_{20}$ cycloalkyl), 3 to 10 ring carbon atoms (i.e., $C_3$-$C_{10}$ cycloalkyl), or 3 to 6 ring carbon atoms (i.e., $C_3$-$C_6$ cycloalkyl). Cycloalkyl also includes "spiro cycloalkyl" when there are two positions for substitution on the same carbon atom. Monocyclic radicals include, for example, cyclopropyl, cyclobutyl, cyclopentyl, cyclohexyl, cycloheptyl and cyclooctyl. Polycyclic radicals include, for example, adamantyl, norbornyl, decalinyl, 7,7-dimethyl-bicyclo[2.2.1]heptanyl and the like. Further, the term cycloalkyl is intended to encompass any non-aromatic ring which may be fused to an aryl ring, regardless of the attachment to the remainder of the molecule.

"Haloalkyl" refers to an unbranched or branched alkyl group as defined above, wherein one or more hydrogen atoms are replaced by a halogen. For example, "$C_1$-$C_6$ haloalkyl" refers to a $C_1$-$C_6$ alkyl which is substituted by one or more halogen atoms. A $C_1$ haloalkyl refers to a methyl group that may be substituted by 1-3 halo groups, a $C_2$ haloalkyl refers to an ethyl group that may be substituted by 1-5 halo groups, a $C_3$ haloalkyl refers to a propyl group that may be substituted by 1-7 halo groups, etc. Examples of haloalkyl include trifluoromethyl, difluoromethyl, trichloromethyl, 2,2,2-trifluoroethyl, 1,2-difluoroethyl, 3-bromo-2-fluoropropyl, 1,2-dibromoethyl, and the like. A haloalkyl may contain one or more halo atoms that are the same (i.e., all fluoro) or a mixture of halo atoms (i.e, chloro and fluoro).

"Heteroaryl" refers to an aromatic group (e.g., a 5-14 membered ring system) having a single ring, multiple rings, or multiple fused rings, with one or more ring heteroatoms independently selected from nitrogen, oxygen and sulfur. As used herein, heteroaryl includes 1 to 10 ring carbon atoms and 1 to 4 heteroatoms independently selected from nitrogen, oxygen and sulfur within the ring. Examples of heteroaryl groups include pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl and thiophenyl (i.e., thienyl).

"Heterocyclyl" refers to a saturated or unsaturated cyclic alkyl group, with one or more ring heteroatoms independently selected from nitrogen, oxygen and sulfur. The term "heterocyclyl" includes heterocycloalkenyl groups (i.e., the heterocyclyl group having at least one double bond), bridged-heterocyclyl groups, fused-heterocyclyl groups and spiro-heterocyclyl groups. A heterocyclyl may be a single ring or multiple rings wherein the multiple rings may be fused, bridged or spiro, and may comprise one or more oxo (C=O) or N-oxide (N-O-) moieties. Any non-aromatic ring containing at least one heteroatom is considered a heterocyclyl, regardless of the attachment (i.e., can be bound through a carbon atom or a heteroatom). Further, the term heterocyclyl is intended to encompass any non-aromatic ring containing at least one heteroatom, which ring may be fused to an aryl or heteroaryl ring, regardless of the attachment to the remainder of the molecule. As used herein, heterocyclyl has 1 to 10 ring carbon atoms, 1 to 8 carbon atoms, 1 to 6 carbon atoms, or 1 to 4 carbon atoms, and 1 to 5 ring heteroatoms, 1 to 4 heteroatoms, 1 to 3 heteroatoms, or 1 to 2 heteroatoms independently selected from nitrogen, sulfur and oxygen. Examples of heterocyclyl groups include dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl and 1,1-dioxo-thiomorpholinyl.

"Cyano" refers to the group -CN.

"Halogen" or "halo" includes fluoro, chloro, bromo, and iodo.

"Hydroxy" refers to the group -OH.

"Oxo" refers to the atom (=O) or (O).

Certain commonly used alternative chemical names may be used. For example, a divalent group such as a divalent "alkyl" group, a divalent "phenyl" group, a divalent "heteroaryl" group, a divalent "heterocyclyl" group etc., may also be referred to as an "alkylene" group, an "phenylene" group, a "heteroarylene" group, or a "heterocyclylene" group, respectively.

The terms "optional" or "optionally" means that the subsequently described event or circumstance may or may not occur and that the description includes instances where said event or circumstance occurs and instances in which

it does not. Also, the term "optionally substituted" refers to any one or more hydrogen atoms on the designated atom or group which may or may not be replaced by a moiety other than hydrogen.

Any compound or formula described herein is intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into the disclosed compounds include isotopes of hydrogen, carbon, nitrogen, oxygen, phosphorous, fluorine, chlorine and iodine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{36}Cl$, $^{123}I$ and $^{125}I$, respectively. Various isotopically labeled compounds of the present disclosure, for example those into which radioactive isotopes, such as $^{2}H$, $^{3}H$, $^{13}C$, and $^{14}C$ are incorporated, are included in this disclosure. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients.

The disclosure also includes "deuterated analogs" of compounds described herein in which from 1 to n hydrogens attached to a carbon atom is/are replaced by deuterium, in which n is the number of hydrogens in the molecule. When multiple deuterium atoms are present in a compound, the deuterium atoms may be on the same portion of the molecule (for example, on a single alkyl group or on a single ring) or on different portions of the molecule (for example, on separate alkyl groups or separate rings). Such compounds may exhibit increased resistance to metabolism and thus may be useful for increasing the half-life of any compound when administered to a mammal, particularly a human. See, for example, Foster, "Deuterium Isotope Effects in Studies of Drug Metabolism," Trends Pharmacol. Sci. 5(12):524-527 (1984). Such compounds are synthesized by means well known in the art, for example by employing starting materials in which one or more hydrogens have been replaced by deuterium.

"Pharmaceutically acceptable" refers to compounds, salts, compositions, dosage forms, and other materials which are useful in preparing a pharmaceutical composition that is suitable for veterinary or human pharmaceutical use.

The term "pharmaceutically acceptable salt" of a given compound refers to salts that retain the biological effectiveness and properties of the given compound and which are not biologically or otherwise undesirable. "Pharmaceutically acceptable salts" include, for example, salts with inorganic acids and salts with an organic acid. In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare nontoxic pharmaceutically acceptable addition salts. Pharmaceutically acceptable acid addition salts may be prepared from inorganic and organic acids. Salts derived from inorganic acids include hydrochloric acid, hydrobromic acid, sulfuric acid, nitric acid, phosphoric acid and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Likewise, pharmaceutically acceptable base addition salts can be prepared from inorganic and organic bases. Salts derived from inorganic bases include, by way of example only, sodium, potassium, lithium, ammonium, calcium and magnesium salts. Salts derived from organic bases include, but are not limited to, salts of primary, secondary and tertiary amines, such as alkyl amines. Specific examples of suitable amines include, by way of example only, isopropylamine, trimethyl amine, diethyl amine, tri(isopropyl) amine, tri(n-propyl) amine, ethanolamine, 2-dimethylaminoethanol, piperazine, piperidine, morpholine, N-ethylpiperidine and the like. It is understood that reference to a particular salt, such as hydrochloride or formate, may refer to a single salt, such as monohydrochloride or monoformate, or may refer to a multiple salt, such a dihydrochloride or diformate.

The compounds disclosed herein, or their pharmaceutically acceptable salts, may include an asymmetric center and may thus give rise to enantiomers, diastereomers, and other stereoisomeric forms that may be defined, in terms of absolute stereochemistry, as (*R*)- or (*S*)- or, as (D)- or (L)- for amino acids. The disclosure is meant to include all such possible isomers, as well as their racemic and optically pure forms. Optically active (+) and (-), (*R*)- and (*S*)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

"Tautomer" refers to alternate forms of a compound that differ in the position of a proton, such as enol-keto and imine-enamine tautomers, or the tautomeric forms of heteroaryl groups containing a ring atom attached to both a ring -NH-moiety and a ring=N moiety such as pyrazoles, imidazoles, benzimidazoles, triazoles, and tetrazoles. All tautomeric forms of the compounds described herein are intended to be included.

A "stereoisomer" refers to a compound made up of the same atoms bonded by the same bonds but having different three-dimensional structures, which are not interchangeable. The present disclosure contemplates various

stereoisomers and mixtures thereof and includes "enantiomers", which refers to two stereoisomers whose molecules are nonsuperimposable mirror images of one another.

"Diastereoisomers" are stereoisomers that have at least two asymmetric atoms, but which are not mirror-images of each other.

As used herein, "pharmaceutically acceptable carrier" or "pharmaceutically acceptable excipient" or "excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

"Effective amount" or dose of a compound or a composition refers to that amount of the compound or the composition that results in an intended result as desired based on the disclosure herein. Effective amounts can be determined by standard pharmaceutical procedures in cell cultures or experimental animals including, without limitation, by determining the $LD_{50}$ (the dose lethal to 50% of the population) and the $ED_{50}$ (the dose therapeutically effective in 50% of the population).

"Therapeutically effective amount" or dose of a compound or a composition refers to that amount of the compound or the composition that results in reduction or inhibition of symptoms or a prolongation of survival in a subject (i.e., a human patient). The results may require multiple doses of the compound or the composition.

"Treating" or "treatment" of a disease in a subject refers to 1) preventing the disease from occurring in a patient that is predisposed or does not yet display symptoms of the disease; 2) inhibiting the disease or arresting its development; or 3) ameliorating or causing regression of the disease. As used herein, "treatment" or "treating" is an approach for obtaining beneficial or desired results including clinical results. For the purposes of this disclosures, beneficial or desired results include, but are not limited to, one or more of the following: decreasing one or more symptoms resulting from the disease or disorder, diminishing the extent of the disease or disorder, stabilizing the disease or disorder (e.g., preventing or delaying the worsening of the disease or disorder), delaying the occurrence or recurrence of the disease or disorder, delay or slowing the progression of the disease or disorder, ameliorating the disease or disorder state, providing a remission (whether partial or total) of the disease or disorder, decreasing the dose of one or more other medications required to treat the disease or disorder, enhancing the effect of another medication used to treat the disease or disorder, delaying the progression of the disease or disorder, increasing the quality of life, and/or prolonging survival of a subject. Also encompassed by "treatment" is a reduction of pathological consequence of the disease or disorder. The methods of the invention contemplate any one or more of these aspects of treatment.

As used herein, the terms "subject(s)" and "patient(s)" mean any mammal. In some embodiments, the mammal is a human. In some embodiments, the mammal is a non-human, such as a primate, dog, cat, rabbit, or rodent. None of the terms require or are limited to situations characterized by the supervision (e.g., constant or intermittent) of a health care worker (e.g., a doctor, a registered nurse, a nurse practitioner, a physician's assistant, an orderly or a hospice worker).

As used herein, the terms "pharmaceutical composition" or "medicament" refer to a composition suitable for pharmaceutical use in a subject, e.g., as a SHP2 inhibitor.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Compounds

In one aspect, provided herein is a compound of Formula (I):

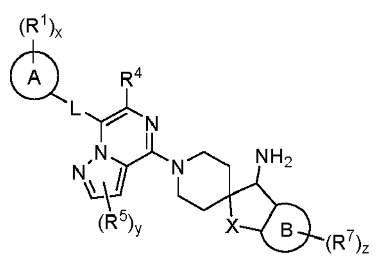

(I)

or a pharmaceutically acceptable salt thereof, wherein:

| | |
|---|---|
| Ring A | is $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S; |
| each $R^1$ | is independently halo, cyano, $-NR^{2a}R^{2b}$, $C_1$-$C_6$ alkyl, oxo, hydroxy, $C_1$-$C_6$ haloalkyl, $C_1$-$C_6$ alkoxy, $C_1$-$C_6$ alkyl-OH, $C_1$-$C_6$ alkyl-CN, $-C(O)NR^{2a}R^{2b}$, $-C(O)(C_1$-$C_6$ alkyl), $-CO_2H$, $-CO_2(C_1$-$C_6$ alkyl), $-Si(R^a)(R^b)(R^c)$, $-P(O)(R^a)(R^b)$, $-OP(O)(R^a)(R^b)$, $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S; |
| or two $R^1$ | groups are taken together with the carbon atoms or heteroatoms to which they are attached to form a fused phenyl, 5- to 6-membered heterocycloalkyl, or 5- to 6-membered heteroaryl, each of which is optionally substituted with 1-4 $R^6$ groups, wherein the fused heterocycloalkyl and heteroaryl contain 1-3 heteroatoms selected from N, O, and S; |
| each $R^a$, $R^b$, and $R^c$ | is independently hydroxy, $C_1$-$C_6$ alkyl, or $C_1$-$C_6$ alkoxy; |
| each $R^{2a}$ and $R^{2b}$ | is independently H, $C_1$-$C_6$ alkyl, or $C_3$-$C_6$ cycloalkyl; |
| L | is a bond, S, O, C(O), or $N(R^d)$; |
| $R^d$ | is H or $C_1$-$C_6$ alkyl; |
| X | is $CR^{3a}R^{3b}$, $NR^{3a}$, or O; |
| $R^{3a}$ and $R^{3b}$ | are independently H or $C_1$-$C_6$ alkyl; |
| $R^4$ | is H, $C_1$-$C_6$ alkyl, $C_1$-$C_6$ alkyl-OH, $C_1$-$C_6$ haloalkyl, or $-NH_2$; |
| each $R^5$ | is independently halo, $C_1$-$C_6$ alkyl, $C_1$-$C_6$ haloalkyl, $-(C_1$-$C_6$ akylene)($C_1$-$C_6$ alkoxy), or $C_1$-$C_6$ alkyl-OH; |
| Ring B | is fused phenyl or 5- to 6-membered heteroaryl containing 1-3 heteroatoms selected from N, O, and S; |
| each $R^6$ | is independently $C_1$-$C_6$ alkyl, halo, or $C_1$-$C_6$ haloalkyl; |
| each $R^7$ | is independently $C_1$-$C_6$ alkyl, halo, $C_1$-$C_6$ alkoxy, $C_1$-$C_6$ alkyl-OH, hydroxy, cyano, $-Si(R^a)(R^b)(R^c)$, $-P(O)(R^a)(R^b)$, $-OP(O)(R^a)(R^b)$, $-NR^{2a}R^{2b}$, or $C_1$-$C_6$ haloalkyl; |
| x | is 0-5; |
| y | is 0-2; and |
| z | is 0-4; |

wherein one or more hydrogen atoms in the compound are optionally replaced by deuterium.

In some embodiments, Ring A is $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S. In some embodiments, Ring A is $C_3$-$C_5$ cycloalkyl, phenyl, 6-membered heterocycloalkyl, or 5- to 6-membered heteroaryl, wherein the heterocycloalkyl and heteroaryl contain 1-2 heteroatoms selected from N, O, and S. In some embodiments, Ring A is cyclopropyl, phenyl, dihydropyridinyl, dihydropyranyl, pyridinyl, pyridazinyl, pyrimidinyl, pyrazolyl, imidazolyl, pyrrolyl, thiazolyl, isoxazolyl, or thiophenyl.

In some embodiments, Ring A is $C_3$-$C_6$ cycloalkyl. In some embodiments, Ring A is $C_3$-$C_5$ cycloalkyl. In some embodiments, Ring A is $C_3$-$C_4$ cycloalkyl. In some embodiments, Ring is A is cyclopropyl, cyclobutyl, cyclopentyl, or cyclohexyl. In some embodiments, Ring is A is cyclopropyl. In some embodiments, Ring is A is cyclobutyl. In some embodiments, Ring is A is cyclopentyl. In some embodiments, Ring is A is cyclohexyl.

In some embodiments, Ring A is phenyl.

In some embodiments, Ring A is 5- to 6-membered heterocycloalkyl containing 1-3 heteroatoms selected from N, O, and S. In some embodiments, Ring A is 5-membered heterocycloalkyl containing 1-3 heteroatoms selected from N, O, and S. In some embodiments, Ring A is 6-membered heterocycloalkyl containing 1-3 heteroatoms selected from N, O, and S. In some embodiments, the heterocycloalkyl contains 1-2 unsaturated bonds. In some embodiments, the heterocycloalkyl contains 1 unsaturated bond. In some embodiments, the heterocycloalkyl contains 2 unsaturated bonds. In some embodiments, the heterocycloalkyl contains 1-2 heteroatoms selected from N, O, and S. In some embodiments, the heterocycloalkyl contains 1-2 heteroatoms selected from N and O. In some embodiments, the heterocycloalkyl contains 1 heteroatom selected from N and O. In some embodiments, the heterocycloalkyl contains 1 nitrogen atom. In some embodiments, the heterocycloalkyl contains 1 oxygen atom. In some embodiments, Ring A is dihydropyridinyl, dihydropyranyl, piperidinyl, or tetrahydropyranyl.

In some embodiments, Ring A is 5- to 6-membered heteroaryl containing 1-3 heteroatoms selected from N, O, and S. In some embodiments, Ring A is 5-membered heteroaryl containing 1-3 heteroatoms selected from N, O, and S. In some embodiments, Ring A is 6-membered heteroaryl containing 1-3 heteroatoms selected from N, O, and S. In some embodiments, the heteroaryl contains 1-2 heteroatoms selected from N, O, and S. In some embodiments, Ring A is 5-membered heteroaryl containing 1-2 nitrogen atoms. In some embodiments, Ring A is 5-membered heteroaryl containing 1 nitrogen atom. In some embodiments, Ring A is 5-membered heteroaryl containing 2 nitrogen atoms. In some embodiments, Ring A is 5-membered heteroaryl containing 1 nitrogen atom and 1 sulfur atom. In some embodiments, Ring A is 5-membered heteroaryl containing 1 nitrogen atom and 1 oxygen atom. In some embodiments, Ring A is 5-membered heteroaryl containing 1 sulfur atom. In some embodiments, Ring A is 6-membered heteroaryl containing 1-2 nitrogen atoms. In some embodiments, Ring A is 6-membered heteroaryl containing 1 nitrogen atom. In some embodiments, Ring A is 6-membered heteroaryl containing 2 nitrogen atoms. In some embodiments, Ring A is pyridyl, pyridazinyl, pyrimidinyl, pyrazolyl, imidazolyl, pyrrolyl, thiazolyl, oxazolyl, isoxazolyl, or thiophenyl.

In some embodiments,

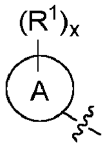

is:

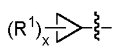 , 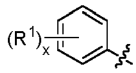 , 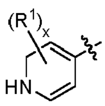 , 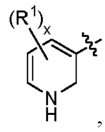 , 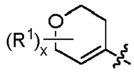 , 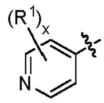 ,

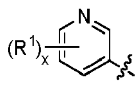 , 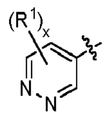 , 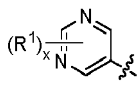 , 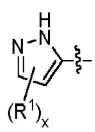 , 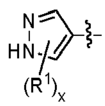 , 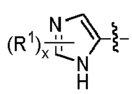 ,

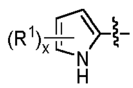 , 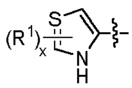 , 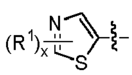 , 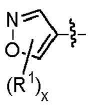 , 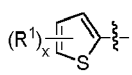 , or 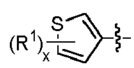 .

In some embodiments, x is 0-5. In some embodiments, x is 0-4. In some embodiments, x is 0, 1, 2, or 3. In some embodiments, x is 0. In some embodiments, x is 1. In some embodiments, x is 2. In some embodiments, x is 3. In some embodiments, x is 4. In some embodiments, x is 5.

In some embodiments, Ring A is unsubstituted. In some embodiments, Ring A is substituted with 1-5 $R^1$ groups. In some variations wherein Ring A is $C_3$-$C_6$ cycloalkyl, Ring A is substituted. In some variations wherein Ring A is pyrimidinyl, Ring A is substituted. In some variations wherein Ring A is a pyridazinyl, Ring A is substituted.

In some embodiments, each $R^1$ is independently halo, cyano, $-NR^{2a}R^{2b}$, $C_1$-$C_6$ alkyl, oxo, hydroxy, $C_1$-$C_6$ haloalkyl, $C_1$-$C_6$ alkoxy, $C_1$-$C_6$ alkyl-OH, $C_1$-$C_6$ alkyl-CN, $-C(O)NR^{2a}R^{2b}$,

$-C(O)(C_1$-$C_6$ alkyl), $-CO_2H$, $-CO_2(C_1$-$C_6$ alkyl), $-Si(R^a)(R^b)(R^c)$, $-P(O)(R^a)(R^b)$, $-OP(O)(R^a)(R^b)$, $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S. In some embodiments, each $R^1$ is independently halo, cyano, $-NR^{2a}R^{2b}$, $C_1$-$C_3$ alkyl, oxo, hydroxy, $C_1$-$C_3$ haloalkyl, $C_1$-$C_3$ alkoxy, $C_1$-$C_3$ alkyl-OH, $C_1$-$C_3$ alkyl-CN, $-C(O)NR^{2a}R^{2b}$, $-C(O)(C_1$-$C_3$ alkyl),

$-CO_2H$, $-CO_2(C_1$-$C_3$ alkyl), $-Si(R^a)(R^b)(R^c)$, $-P(O)(R^a)(R^b)$, $-OP(O)(R^a)(R^b)$, $C_3$-$C_5$ cycloalkyl, phenyl, or 6-membered heterocycloalkyl, wherein the heterocycloalkyl contains 1-2 heteroatoms selected from N and O. In some embodiments, each $R^a$, $R^b$, and $R^c$ is independently $C_1$-$C_3$ alkyl or $C_1$-$C_3$ alkoxy. In some embodiments, each $R^{2a}$ and $R^{2b}$ is independently H, $C_1$-$C_3$ alkyl, or $C_3$-$C_5$ cycloalkyl.

In some embodiments, $R^1$ is halo. In some embodiments, $R^1$ is F, Cl, Br, or I. In some embodiments, $R^1$ is F, Cl, or Br. In some embodiments, $R^1$ is F or Cl. In some embodiments, $R^1$ is F. In some embodiments, $R^1$ is Cl.

In some embodiments, $R^1$ is cyano. In some embodiments, $R^1$ is oxo. In some embodiments, $R^1$ is hydroxy. In some embodiments, $R^1$ is $-CO_2H$.

In some embodiments, $R^1$ is $-NR^{2a}R^{2b}$, wherein $R^{2a}$ and $R^{2b}$ are independently H, $C_1$-$C_6$ alkyl, or $C_3$-$C_6$ cycloalkyl. In some embodiments, $R^{2a}$ and $R^{2b}$ are independently H, $C_1$-$C_3$ alkyl, or $C_3$-$C_5$ cycloalkyl. In some embodiments, $R^{2a}$ and $R^{2b}$ are both H. In some embodiments, $R^{2a}$ and $R^{2b}$ are both $C_1$-$C_3$ alkyl. In some embodiments, one

of $R^{2a}$ and $R^{2b}$ is H and the other is $C_1$-$C_3$ alkyl. In some embodiments, one of $R^{2a}$ and $R^{2b}$ is H and the other is C3-$C_6$ cycloalkyl. In some embodiments, one of $R^{2a}$ and $R^{2b}$ is $C_1$-$C_3$ alkyl and the other is $C_3$-$C_6$ cycloalkyl. In some embodiments, $R^1$ is $-NH_2$, $-N(H)CH_3$, or $-N(CH_3)_2$. In some embodiments, $R^1$ is $-NH_2$. In some embodiments, $R^1$ is - $N(H)CH_3$. In some embodiments, $R^1$ is $-N(CH_3)_2$.

In some embodiments, $R^1$ is $C_1$-$C_6$ alkyl. In some embodiments, $R^1$ is $C_1$-$C_3$ alkyl. In some embodiments, $R^1$ is methyl, ethyl, *n*-propyl, or isopropyl. In some embodiments, $R^1$ is methyl, ethyl, or isopropyl. In some embodiments, $R^1$ is methyl. In some embodiments, $R^1$ is ethyl. In some embodiments, $R^1$ is isopropyl.

In some embodiments, $R^1$ is $C_1$-$C_6$ haloalkyl. In some embodiments, $R^1$ is $C_1$-$C_6$ haloalkyl containing 1-13 halogen atoms. In some embodiments, $R^1$ is $C_1$-$C_3$ haloalkyl. In some embodiments, $R^1$ is $C_1$-$C_3$ haloalkyl containing 1-7 halogen atoms. In some embodiments, $R^1$ is

$-CF_3$, $-CHF_2$, $-CH_2F$, $-CCl_3$, $-CHCl_2$, $-CH_2Cl$, $-CF_2Cl$, $-CFCl_2$, $-CH_2CF_3$, $-CH_2CHF_2$, or $-CH_2CCl_3$. In some embodiments, $R^1$ is $-CF_3$.

In some embodiments, $R^1$ is $C_1$-$C_6$ alkoxy. In some embodiments, $R^1$ is $C_1$-$C_3$ alkoxy. In some embodiments, $R^1$ is $-OCH_3$, $-OCH_2CH_3$, or $-OCH(CH_3)_2$. In some embodiments, $R^1$ is $-OCH_3$. In some embodiments, $R^1$ is $-OCH_2CH_3$.

In some embodiments, $R^1$ is $C_1$-$C_6$ alkyl-OH. In some embodiments, $R^1$ is $C_1$-$C_3$ alkyl-OH. In some embodiments, $R^1$ is $-CH_2OH$, $-CH_2CH_2OH$, $-CH_2CH_2CH_2OH$, $-CH(OH)CH_3$, $-CH(OH)CH_2OH$, or $-CH_2CH(OH)CH_3$. In some embodiments, $R^1$ is $-CH_2OH$. In some embodiments, $R^1$ is - $CH_2CH_2OH$.

In some embodiments, $R^1$ is $C_1$-$C_6$ alkyl-CN. In some embodiments, $R^1$ is $C_1$-$C_3$ alkyl-CN. In some embodiments, $R^1$ is $-CH_2CN$, $-CH_2CH_2CN$, $-CH_2CH_2CH_2CN$, $-CH(CN)CH_3$, $-CH(CN)CH_2OH$, or $-CH_2CH(CN)CH_3$. In some embodiments, $R^1$ is $-CH_2CN$. In some embodiments, $R^1$ is - $CH_2CH_2CN$.

In some embodiments, $R^1$ is $-C(O)NR^{2a}R^{2b}$, wherein $R^{2a}$ and $R^{2b}$ are independently H, $C_1$-$C_6$ alkyl, or $C_3$-$C_6$ cycloalkyl. In some embodiments, $R^{2a}$ and $R^{2b}$ are independently H, $C_1$-$C_3$ alkyl, or $C_3$-$C_5$ cycloalkyl. In some embodiments, $R^{2a}$ and $R^{2b}$ are both H. In some embodiments, $R^{2a}$ and $R^{2b}$ are both $C_1$-$C_3$ alkyl. In some embodiments, one of $R^{2a}$ and $R^{2b}$ is H and the other is $C_1$-$C_3$ alkyl. In some embodiments, one of $R^{2a}$ and $R^{2b}$ is H and the other is $C_3$-$C_6$ cycloalkyl. In some embodiments, one of $R^{2a}$ and $R^{2b}$ is $C_1$-$C_3$ alkyl and the other is $C_3$-$C_6$ cycloalkyl. In some embodiments, $R^1$ is $-C(O)NH_2$, $-C(O)N(CH_3)_2$, or $-C(O)N(H)(CH_3)$. In some embodiments, $R^1$ is $-C(O)N(CH_3)_2$. In some embodiments, $R^1$ is $-C(O)NH_2$. In some embodiments, $R^1$ is $-C(O)N(H)(CH_3)$.

In some embodiments, $R^1$ is $-C(O)(C_1$-$C_6$ alkyl). In some embodiments, $R^1$ is $-C(O)(C_1$-$C_3$ alkyl). In some embodiments, $R^1$ is $-C(O)CH_3$, $-C(O)CH_2CH_3$, or $-C(O)CH(CH_3)$. In some embodiments, $R^1$ is $-C(O)CH_3$.

In some embodiments, $R^1$ is $-CO_2(C_1$-$C_6$ alkyl). In some embodiments, $R^1$ is $-CO_2(C_1$-$C_3$ alkyl). In some embodiments, $R^1$ is $-CO_2CH_3$, $-CO_2CH_2CH_3$, or $-CO_2CH(CH_3)_2$.

In some embodiments, $R^1$ is $-Si(R^a)(R^b)(R^c)$, wherein $R^a$, $R^b$, and $R^c$ are independently hydroxy, $C_1$-$C_6$ alkyl, or $C_1$-$C_6$ alkoxy. In some embodiments, $R^a$, $R^b$, and $R^c$ are independently hydroxy, $C_1$-$C_3$ alkyl, or $C_1$-$C_3$ alkoxy. In some embodiments, $R^a$, $R^b$, and $R^c$ are independently hydroxy, methyl, ethyl, *n*-propyl, isopropyl, methoxy, ethoxy, or isopropoxy. In some embodiments, $R^1$ is $-Si(CH_3)_3$ or $-Si(CH_3)_3$.

In some embodiments, $R^1$ is $-P(O)(R^a)(R^b)$, wherein $R^a$ and $R^b$ are independently hydroxy, $C_1$-$C_6$ alkyl, or $C_1$-$C_6$ alkoxy. In some embodiments, $R^a$ and $R^b$ are independently hydroxy, $C_1$-$C_3$ alkyl, or $C_1$-$C_3$ alkoxy. In some embodiments, $R^a$ and $R^b$ are independently hydroxy, methyl, ethyl, *n*-propyl, isopropyl, methoxy, ethoxy, or isopropoxy. In some embodiments, $R^1$ is $-P(O)(OH)_2$, $-P(O)(CH_3)_2$, $-P(O)(OH)(OCH_3)$, or $-P(O)(OCH_3)_2$.

In some embodiments, $R^1$ is $-OP(O)(R^a)(R^b)$, wherein $R^a$ and $R^b$ are independently hydroxy, $C_1$-$C_6$ alkyl, or $C_1$-$C_6$ alkoxy. In some embodiments, $R^a$ and $R^b$ are independently hydroxy, $C_1$-$C_3$ alkyl, or $C_1$-$C_3$ alkoxy. In some embodiments, $R^a$ and $R^b$ are independently hydroxy, methyl, ethyl, *n*-propyl, isopropyl, methoxy, ethoxy, or isopropoxy. In some embodiments, $R^1$ is $-OP(O)(OH)_2$, $-OP(O)(CH_3)_2$, $-OP(O)(OH)(OCH_3)$, or $-OP(O)(OCH_3)_2$.

In some embodiments, $R^1$ is $C_3$-$C_6$ cycloalkyl. In some embodiments, $R^1$ is $C_3$-$C_5$ cycloalkyl. In some embodiments, $R^1$ is cyclopropyl, cyclobutyl, cyclopentyl, or cyclohexyl. In some embodiments, $R^1$ is cyclopropyl, cyclobutyl, or cyclopentyl. In some embodiments, $R^1$ is cyclopropyl.

In some embodiments, $R^1$ is phenyl.

In some embodiments, $R^1$ is 5- to 6-membered heterocycloalkyl containing 1-3 heteroatoms selected from N, O, and S. In some embodiments, $R^1$ is 5-membered heterocycloalkyl containing 1-3 heteroatoms selected from N, O, and S. In some embodiments, $R^1$ is 6-membered heterocycloalkyl containing 1-3 heteroatoms selected from N, O, and S. In some embodiments, the heterocycloalkyl contains 1-2 heteroatoms selected from N and O. In some embodiments, the heterocycloalkyl contains one nitrogen atom and one oxygen atom. In some embodiments, the heterocycloalkyl

contains 2 nitrogen atoms. In some embodiments, the heterocycloalkyl contains 2 oxygen atoms. In some embodiments, $R^1$ is morpholinyl, piperazinyl, piperidinyl, pyrrolidinyl, tetrahydropyranyl, or tetrahydrofuranyl.

In some embodiments, $R^1$ is 5- to 6-membered heteroaryl containing 1-3 heteroatoms selected from N, O, and S. In some embodiments, $R^1$ is 5-membered heteroaryl containing 1-3 heteroatoms selected from N, O, and S. In some embodiments, $R^1$ is 6-membered heteroaryl containing 1-3 heteroatoms selected from N, O, and S. In some embodiments, the heteroaryl contains 1-2 heteroatoms selected from N and O. In some embodiments, the heteroaryl contains one nitrogen atom and one oxygen atom. In some embodiments, the heteroaryl contains 2 nitrogen atoms. In some embodiments, $R^1$ is pyridyl, pyridazinyl, pyrimidinyl, pyrazolyl, imidazolyl, pyrrolyl, thiazolyl, oxazolyl, isoxazolyl, or thiophenyl.

In some embodiments, each $R^1$ is independently, F, Cl, -CN, $-CH_2CN$, $-NH_2$, $-N(H)CH_3$, $-N(CH_3)_2$, $-CH_3$, $-CH_2CH_3$, $-CH(CH_3)_2$, oxo, $-CF_3$, $-OCH_3$, $-CH_2OH$, $-C(O)N(CH_3)_2$, $-C(O)CH_3$, cyclopropyl, or

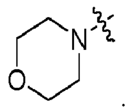.

In some embodiments, two $R^1$ groups are taken together with the carbon atoms or heteroatoms to which they are attached to form a fused phenyl optionally substituted with 1-4 $R^6$ groups

In some embodiments, two $R^1$ groups are taken together with the carbon atoms or heteroatoms to which they are attached to form a fused 5- to 6-membered heterocycloalkyl optionally substituted with 1-4 $R^6$ groups, wherein the fused heterocycloalkyl contains 1-3 heteroatoms selected from N, O, and S. In some embodiments, the fused heterocycloalkyl is optionally substituted with 1-2 $R^6$ groups. In some embodiments, the fused heterocycloalkyl contains 1-2 heteroatoms selected from N, O, and S. In some embodiments, two $R^1$ groups are taken together with the carbon atoms or heteroatoms to which they are attached to form a fused 5-membered heterocycloalkyl optionally substituted with 1-4 $R^6$ groups. In some embodiments, two $R^1$ groups are taken together with the carbon atoms or heteroatoms to which they are attached to form a fused 6-membered heterocycloalkyl optionally substituted with 1-4 $R^6$ groups. In some embodiments, two $R^1$ groups are taken together with the carbon atoms or heteroatoms to which they are attached to form a fused pyranyl, dihydrodioxinyl, or dihydrofuranyl.

In some embodiments, two $R^1$ groups are taken together with the carbon atoms or heteroatoms to form a fused 5- to 6-membered heteroary optionally substituted with 1-4 $R^6$ groups, wherein the fused heteroaryl contains 1-3 heteroatoms selected from N, O, and S. In some embodiments, the fused heteroaryl is optionally substituted with 1-2 $R^6$ groups. In some embodiments, the fused heteroaryl contain 1-2 heteroatoms selected from N, O, and S. In some embodiments, two $R^1$ groups are taken together with the carbon atoms or heteroatoms to which they are attached to form a fused 5-membered heteroaryl optionally substituted with 1-4 $R^6$ groups. In some embodiments, two $R^1$ groups are taken together with the carbon atoms or heteroatoms to which they are attached to form a fused 6-membered heteroaryl optionally substituted with 1-4 $R^6$ groups. In some embodiments, two $R^1$ groups are taken together with the carbon atoms or heteroatoms to which they are attached to form a fused pyridyl, pyrazinyl, pyrrolyl, or thiazolyl.

In some embodiments, two $R^1$ groups are taken together with the carbon atoms or heteroatoms to which they are attached to form a fused group selected from:

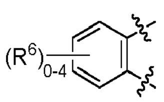, 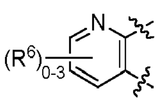, 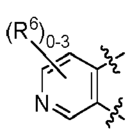, 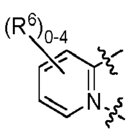, 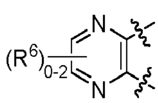, 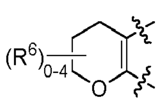,

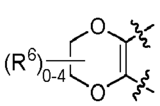, 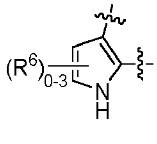, 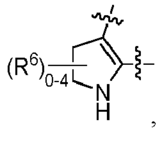, 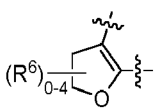, and 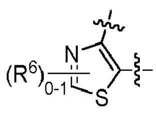

In some embodiments, each $R^6$ is independently $C_1$-$C_6$ alkyl, halo, or $C_1$-$C_6$ haloalkyl. In some embodiments, each $R^6$ is independently $C_1$-$C_3$ alkyl, halo, or $C_1$-$C_3$ haloalkyl.

In some embodiments, $R^6$ is $C_1$-$C_6$ alkyl. In some embodiments, $R^6$ is $C_1$-$C_3$ alkyl. In some embodiments, $R^6$ is methyl, ethyl, *n*-propyl, or isopropyl. In some embodiments, $R^6$ is methyl, ethyl, or isopropyl. In some embodiments, $R^6$ is methyl. In some embodiments, $R^6$ is ethyl. In some embodiments, $R^6$ is isopropyl.

In some embodiments, $R^6$ is halo. In some embodiments, $R^6$ is F, Cl, Br, or I. In some embodiments, $R^6$ is F,

Cl, or Br. In some embodiments, $R^6$ is F or Cl. In some embodiments, $R^6$ is F. In some embodiments, $R^6$ is Cl.

In some embodiments, $R^6$ is $C_1$-$C_6$ haloalkyl. In some embodiments, $R^6$ is $C_1$-$C_6$ haloalkyl containing 1-13 halogen atoms. In some embodiments, $R^6$ is $C_1$-$C_3$ haloalkyl. In some embodiments, $R^6$ is $C_1$-$C_3$ haloalkyl containing 1-7 halogen atoms. In some embodiments, $R^6$ is

$-CF_3$, $-CHF_2$, $-CH_2F$, $-CCl_3$, $-CHCl_2$, $-CH_2Cl$, $-CF_2Cl$, $-CFCl_2$, $-CH_2CF_3$, $-CH_2CHF_2$, or $-CH_2CCl_3$. In some embodiments, $R^6$ is $-CF_3$.

In some embodiments,

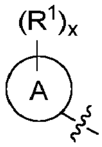

is:

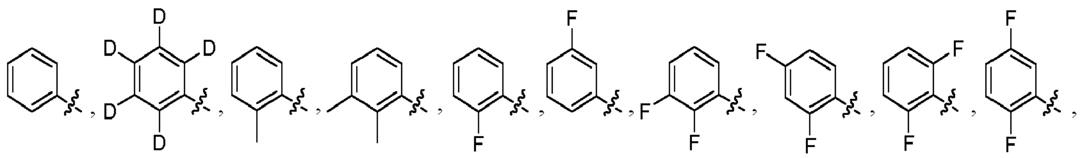
, , , , , , , , , , ,

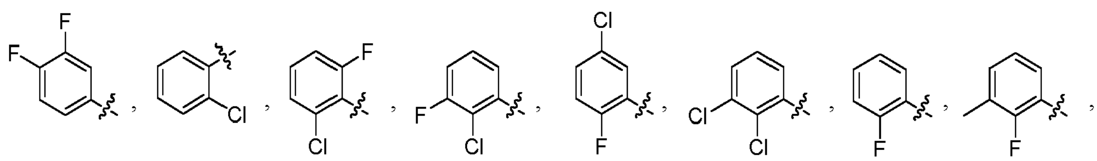
, , , , , , , , ,

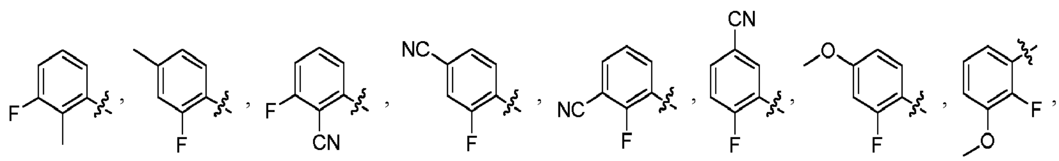
, , , , , , , , ,

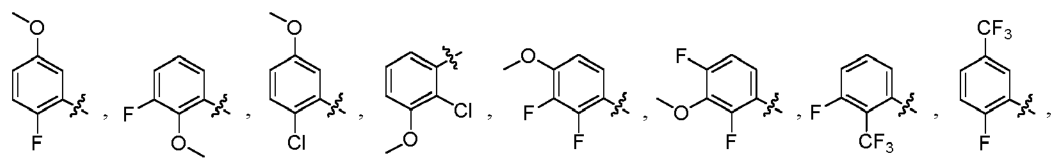
, , , , , , , , ,

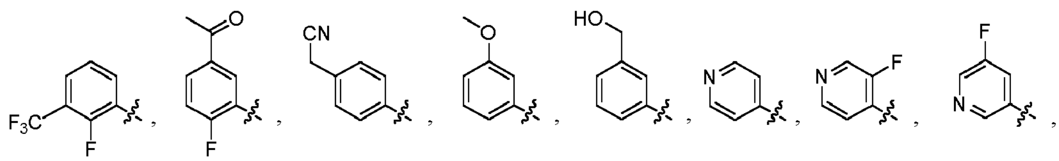
, , , , , , , , ,

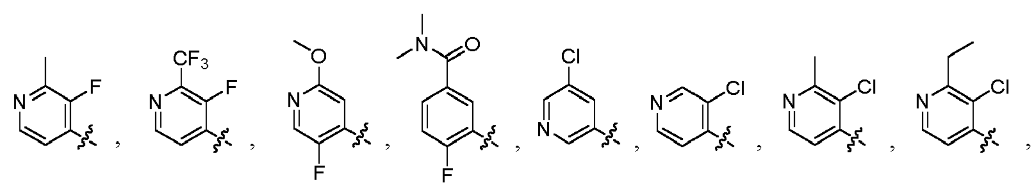
, , , , , , , ,

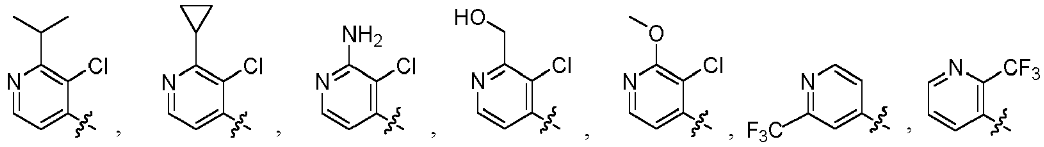,

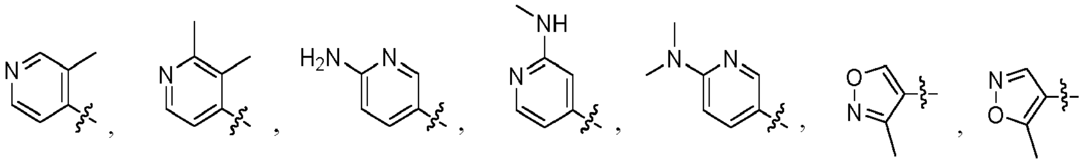,

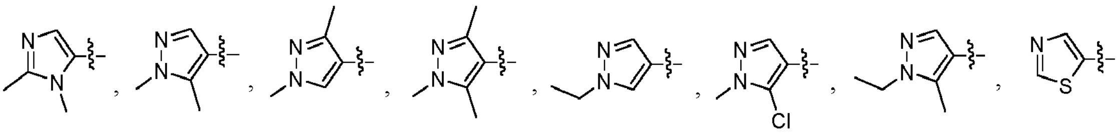,

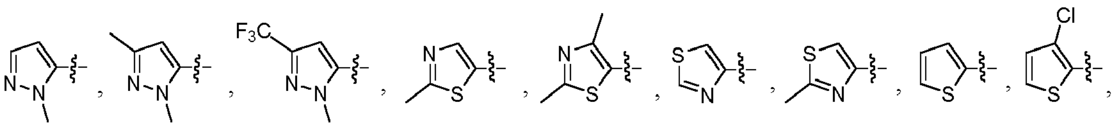,

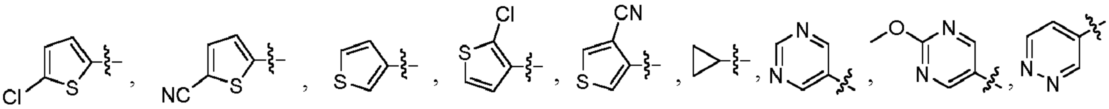,

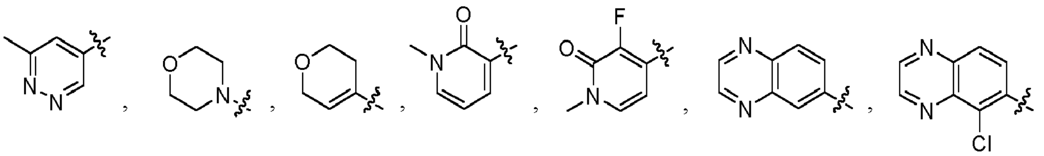,

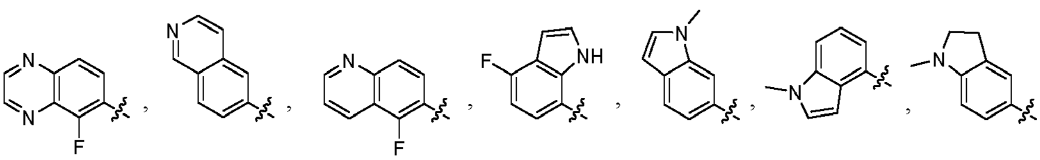,

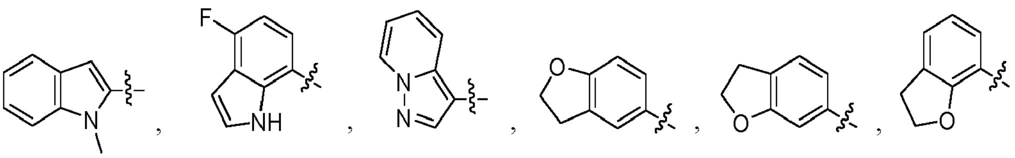,

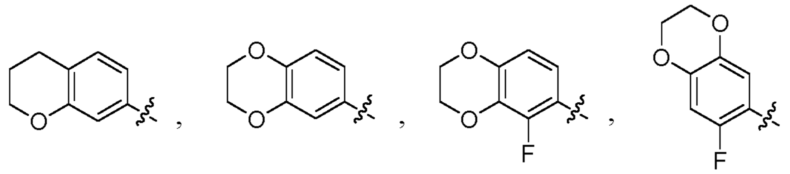, or 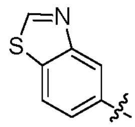.

In some embodiments, L is a bond. In some embodiments, L is S. In some embodiments, L is O. In some embodiments, L is C(O). In some embodiments, L is N($R^d$), wherein $R^d$ is H or $C_1$-$C_6$ alkyl. In some embodiments, $R^d$ is H. In some embodiments, $R^d$ is $C_1$-$C_6$ alkyl. In some embodiments, $R^d$ is $C_1$-$C_3$ alkyl, such as methyl, ethyl, or propyl.

In some embodiments, L is NH. In some embodiments, L is $N(CH_3)$.

In some embodiments, X is $CR^{3a}R^{3b}$, $NR^{3a}$, or O, wherein $R^{3a}$ and $R^{3b}$ are independently H or $C_1$-$C_6$ alkyl. In some embodiments, X is $CR^{3a}R^{3b}$, $NR^{3a}$, or O, wherein $R^{3a}$ and $R^{3b}$ are independently H or $C_1$-$C_3$ alkyl.

In some embodiments, X is O.

In some embodiments, X is $CR^{3a}R^{3b}$, wherein $R^{3a}$ and $R^{3b}$ are independently H or $C_1$-$C_6$ alkyl. In some embodiments, $R^{3a}$ and $R^{3b}$ are independently H or $C_1$-$C_3$ alkyl. In some embodiments, $R^{3a}$ and $R^{3b}$ are both H. In some embodiments, $R^{3a}$ and $R^{3b}$ are both $C_1$-$C_3$ alkyl, such as methyl, ethyl, or propyl. In some embodiments, one of $R^{3a}$ and $R^{3b}$ is H and the other is $C_1$-$C_3$ alkyl. In some embodiments, X is $CH_2$, $CH(CH_3)$, or $C(CH_3)_2$. In some embodiments, X is $CH_2$.

In some embodiments, X is $NR^{3a}$, wherein $R^{3a}$ is H or $C_1$-$C_6$ alkyl. In some embodiments, $R^{3a}$ is H or $C_1$-$C_3$ alkyl. In some embodiments, $R^{3a}$ is H. In some embodiments, $R^{3a}$ is $C_1$-$C_3$ alkyl, such as methyl, ethyl, or propyl. In some embodiments, X is N(H) or $N(CH_3)$. In some embodiments, X is N(H). In some embodiments, X is $N(CH_3)$.

In some embodiments, $R^4$ is H, $C_1$-$C_6$ alkyl, $C_1$-$C_6$ alkyl-OH, $C_1$-$C_6$ haloalkyl, or $-NH_2$. In some embodiments, $R^4$ is H, $C_1$-$C_3$ alkyl, $C_1$-$C_3$ alkyl-OH, $C_1$-$C_3$ haloalkyl, or $-NH_2$.

In some embodiments, $R^4$ is H.

In some embodiments, $R^4$ is $C_1$-$C_6$ alkyl. In some embodiments, $R^4$ is $C_1$-$C_3$ alkyl. In some embodiments, $R^4$ is methyl, ethyl, *n*-propyl, or isopropyl. In some embodiments, $R^4$ is methyl, ethyl, or isopropyl. In some embodiments, $R^4$ is methyl. In some embodiments, $R^4$ is ethyl. In some embodiments, $R^4$ is isopropyl.

In some embodiments, $R^4$ is $C_1$-$C_6$ alkyl-OH. In some embodiments, $R^4$ is $C_1$-$C_3$ alkyl-OH. In some embodiments, $R^4$ is $-CH_2OH$, $-CH_2CH_2OH$, $-CH_2CH_2CH_2OH$, $-CH(OH)CH_3$, $-CH(OH)CH_2OH$, or $-CH_2CH(OH)CH_3$. In some embodiments, $R^4$ is $-CH_2OH$. In some embodiments, $R^4$ is - $CH_2CH_2OH$.

In some embodiments, $R^4$ is $C_1$-$C_6$ haloalkyl. In some embodiments, $R^4$ is $C_1$-$C_6$ haloalkyl containing 1-13 halogen atoms. In some embodiments, $R^4$ is $C_1$-$C_3$ haloalkyl. In some embodiments, $R^4$ is $C_1$-$C_3$ haloalkyl containing 1-7 halogen atoms. In some embodiments, $R^4$ is

$-CF_3$, $-CHF_2$, $-CH_2F$, $-CCl_3$, $-CHCl_2$, $-CH_2Cl$, $-CF_2Cl$, $-CFCl_2$, $-CH_2CF_3$, $-CH_2CHF_2$, or $-CH_2CCl_3$. In some embodiments, $R^4$ is $-CF_3$, $-CHF_2$, or $-CH_2F$. In some embodiments, $R^4$ is $-CHF_2$. In some embodiments, $R^4$ is $-CH_2F$.

In some embodiments, $R^4$ is $-NH_2$.

In some embodiments, each $R^5$ is independently halo, $C_1$-$C_6$ alkyl, $C_1$-$C_6$ haloalkyl, -($C_1$-$C_6$ akylene)($C_1$-$C_6$ alkoxy), or $C_1$-$C_6$ alkyl-OH. In some embodiments, each $R^5$ is independently halo, $C_1$-$C_3$ alkyl, $C_1$-$C_3$ haloalkyl, -($C_1$-$C_3$ alkylene)($C_1$-$C_3$ alkoxy), or $C_1$-$C_3$ alkyl-OH. In some embodiments, each $R^5$ is independently Cl, F, $-CH_2F$, $-CHF_2$, $-CH_2OCH_3$, or $-CH_2OH$.

In some embodiments, $R^5$ is halo. In some embodiments, $R^5$ is F, Cl, Br, or I. In some embodiments, $R^5$ is F, Cl, or Br. In some embodiments, $R^5$ is F or Cl. In some embodiments, $R^5$ is F. In some embodiments, $R^5$ is Cl.

In some embodiments, $R^5$ is $C_1$-$C_6$ alkyl. In some embodiments, $R^5$ is $C_1$-$C_3$ alkyl. In some embodiments, $R^5$ is methyl, ethyl, *n*-propyl, or isopropyl. In some embodiments, $R^5$ is methyl, ethyl, or isopropyl. In some embodiments, $R^5$ is methyl. In some embodiments, $R^5$ is ethyl. In some embodiments, $R^5$ is isopropyl.

In some embodiments, $R^5$ is $C_1$-$C_6$ haloalkyl. In some embodiments, $R^5$ is $C_1$-$C_6$ haloalkyl containing 1-13 halogen atoms. In some embodiments, $R^5$ is $C_1$-$C_3$ haloalkyl. In some embodiments, $R^5$ is $C_1$-$C_3$ haloalkyl containing 1-7 halogen atoms. In some embodiments, $R^5$ is

$-CF_3$, $-CHF_2$, $-CH_2F$, $-CCl_3$, $-CHCl_2$, $-CH_2Cl$, $-CF_2Cl$, $-CFCl_2$, $-CH_2CF_3$, $-CH_2CHF_2$, or $-CH_2CCl_3$. In some embodiments, $R^5$ is $-CF_3$, $-CHF_2$, or $-CH_2F$. In some embodiments, $R^5$ is $-CHF_2$. In some embodiments, $R^5$ is $-CH_2F$.

In some embodiments, $R^5$ is -($C_1$-$C_6$ alkylene)($C_1$-$C_6$ alkoxy). In some embodiments, $R^5$ is -($C_1$-$C_3$ alkylene)($C_1$-$C_3$ alkoxy). In some embodiments, $R^5$ is -($C_1$-alkylene)($C_1$-$C_3$ alkoxy), such as $-CH_2OCH_3$, - $CH_2COH_2CH_3$, or $-CH_2COH_2CH_2CH_3$. In some embodiments, $R^5$ is -($C_2$-alkylene)($C_1$-$C_3$ alkoxy), such as - $CH_2CH_2COH_3$, $-CH_2CH_2COH_2CH_3$, or $-CH_2CH_2COH_2CH_2CH_3$. In some embodiments, $R^5$ is -($C_3$-alkylene)($C_1$-$C_3$ alkoxy), such as $-CH_2CH_2CH_2COH_3$, $-CH_2CH_2CH_2COH_2CH_3$, or $-CH_2CH_2CH_2COH_2CH_2CH_3$. In some embodiments, $R^5$ is $-CH_2COH_3$.

In some embodiments, $R^5$ is $C_1$-$C_6$ alkyl-OH. In some embodiments, $R^5$ is $C_1$-$C_3$ alkyl-OH. In some embodiments, $R^5$ is $-CH_2OH$, $-CH_2CH_2OH$, $-CH_2CH_2CH_2OH$, $-CH(OH)CH_3$, $-CH(OH)CH_2OH$, or $-CH_2CH(OH)CH_3$. In some embodiments, $R^5$ is $-CH_2OH$. In some embodiments, $R^5$ is - $CH_2CH_2OH$.

In some embodiments, y is 0-2. In some embodiments, y is 0 or 1. In some embodiments, y is 0. In some embodiments, y is 1. In some embodiments, y is 2.

In some embodiments, Ring B is fused phenyl or 5- to 6-membered heteroaryl containing 1-3 heteroatoms selected from N, O, and S. In some embodiments, Ring B is fused phenyl or 5- to 6-membered heteroaryl containing 1-2 heteroatoms selected from N, O, and S. In some embodiments, Ring B is fused phenyl, pyridyl, pyrimidinyl, pyrazinyl, pyridazinyl, thiazolyl, or oxazolyl.

In some embodiments, Ring B is fused phenyl.

In some embodiments, Ring B is fused 5- to 6-membered heteroaryl containing 1-3 heteroatoms selected from N, O, and S. In some embodiments, Ring B is fused 5-membered heteroaryl containing 1-3 heteroatoms selected from N, O, and S. In some embodiments, Ring B is fused 6-membered heteroaryl containing 1-3 heteroatoms selected from N, O, and S. In some embodiments, the fused heteroaryl contains 1-2 heteroatoms selected from N, O, and S. In some embodiments, Ring B is pyridyl, pyrimidinyl, pyrazinyl, pyridazinyl, thiazolyl, or oxazolyl.

In some embodiments, each $R^7$ is independently $C_1$-$C_6$ alkyl, halo, $C_1$-$C_6$ alkoxy, $C_1$-$C_6$ alkyl-OH, hydroxy, cyano, $-Si(R^a)(R^b)(R^c)$, $-P(O)(R^a)(R^b)$, $-OP(O)(R^a)(R^b)$, $-NR^{2a}R^{2b}$, or $C_1$-$C_6$ haloalkyl. In some embodiments, each $R^7$ is independently $C_1$-$C_3$ alkyl, halo, $C_1$-$C_3$ alkoxy, $C_1$-$C_3$ alkyl-OH, hydroxy, cyano, $-Si(R^a)(R^b)(R^c)$, $-P(O)(R^a)(R^b)$, $-OP(O)(R^a)(R^b)$, $-NR^{2a}R^{2b}$, or $C_1$-$C_3$ haloalkyl. In some embodiments, each $R^7$ is independently $CH_3$, F, $-OCH_3$, $-CH_2OH$, hydroxy, -CN, $-N(CH_3)_2$, or $-CHF_2$.

In some embodiments, $R^7$ is $C_1$-$C_6$ alkyl. In some embodiments, $R^7$ is $C_1$-$C_3$ alkyl. In some embodiments, $R^7$ is methyl, ethyl, *n*-propyl, or isopropyl. In some embodiments, $R^7$ is methyl, ethyl, or isopropyl. In some embodiments, $R^7$ is methyl. In some embodiments, $R^7$ is ethyl. In some embodiments, $R^7$ is isopropyl.

In some embodiments, $R^7$ is halo. In some embodiments, $R^7$ is F, Cl, Br, or I. In some embodiments, $R^7$ is F, Cl, or Br. In some embodiments, $R^7$ is F or Cl. In some embodiments, $R^7$ is F. In some embodiments, $R^7$ is Cl.

In some embodiments, $R^7$ is $C_1$-$C_6$ alkoxy. In some embodiments, $R^7$ is $C_1$-$C_3$ alkoxy. In some embodiments, $R^7$ is $-OCH_3$, $-OCH_2CH_3$, or $-OCH(CH_3)_2$. In some embodiments, $R^7$ is $-OCH_3$. In some embodiments, $R^7$ is $-OCH_2CH_3$.

In some embodiments, $R^7$ is $C_1$-$C_6$ alkyl-OH. In some embodiments, $R^7$ is $C_1$-$C_3$ alkyl-OH. In some embodiments, $R^7$ is $-CH_2OH$, $-CH_2CH_2OH$, $-CH_2CH_2CH_2OH$, $-CH(OH)CH_3$, $-CH(OH)CH_2OH$, or $-CH_2CH(OH)CH_3$. In some embodiments, $R^7$ is $-CH_2OH$. In some embodiments, $R^7$ is $- CH_2CH_2OH$.

In some embodiments, $R^7$ is hydroxy. In some embodiments, $R^7$ is cyano.

In some embodiments, $R^7$ is $-Si(R^a)(R^b)(R^c)$, wherein $R^a$, $R^b$, and $R^c$ are independently hydroxy, $C_1$-$C_6$ alkyl, or $C_1$-$C_6$ alkoxy. In some embodiments, $R^a$, $R^b$, and $R^c$ are independently hydroxy, $C_1$-$C_3$ alkyl, or $C_1$-$C_3$ alkoxy. In some embodiments, $R^a$, $R^b$, and $R^c$ are independently hydroxy, methyl, ethyl, *n*-propyl, isopropyl, methoxy, ethoxy, or isopropoxy. In some embodiments, $R^7$ is $-Si(CH_3)_3$ or $Si(OCH_3)_3$.

In some embodiments, $R^7$ is $-P(O)(R^a)(R^b)$, wherein $R^a$ and $R^b$ are independently hydroxy, $C_1$-$C_6$ alkyl, or $C_1$-$C_6$ alkoxy. In some embodiments, $R^a$ and $R^b$ are independently hydroxy, $C_1$-$C_3$ alkyl, or $C_1$-$C_3$ alkoxy. In some embodiments, $R^a$ and $R^b$ are independently hydroxy, methyl, ethyl, *n*-propyl, isopropyl, methoxy, ethoxy, or isopropoxy. In some embodiments, $R^7$ is $-P(O)(OH)_2$, $-P(O)(CH_3)_2$, $-P(O)(OH)(OCH_3)$, or $-P(O)(OCH_3)_2$.

In some embodiments, $R^7$ is $-OP(O)(R^a)(R^b)$, wherein $R^a$ and $R^b$ are independently hydroxy, $C_1$-$C_6$ alkyl, or $C_1$-$C_6$ alkoxy. In some embodiments, $R^a$ and $R^b$ are independently hydroxy, $C_1$-$C_3$ alkyl, or $C_1$-$C_3$ alkoxy. In some embodiments, $R^a$ and $R^b$ are independently hydroxy, methyl, ethyl, *n*-propyl, isopropyl, methoxy, ethoxy, or isopropoxy. In some embodiments, $R^7$ is $-OP(O)(OH)_2$, $-OP(O)(CH_3)_2$, $-OP(O)(OH)(OCH_3)$, or $-OP(O)(OCH_3)_2$.

In some embodiments, R is $-NR^{2a}R^{2b}$, wherein $R^{2a}$ and $R^{2b}$ are independently H, $C_1$-$C_6$ alkyl, or $C_3$-$C_6$ cycloalkyl. In some embodiments, $R^{2a}$ and $R^{2b}$ are independently H, $C_1$-$C_3$ alkyl, or $C_3$-$C_5$ cycloalkyl. In some embodiments, $R^{2a}$ and $R^{2b}$ are both H. In some embodiments, $R^{2a}$ and $R^{2b}$ are both $C_1$-$C_3$ alkyl. In some embodiments, one of $R^{2a}$ and $R^{2b}$ is H and the other is $C_1$-$C_3$ alkyl. In some embodiments, one of $R^{2a}$ and $R^{2b}$ is H and the other is $C_3$-$C_6$ cycloalkyl. In some embodiments, one of $R^{2a}$ and $R^{2b}$ is $C_1$-$C_3$ alkyl and the other is $C_3$-$C_6$ cycloalkyl. In some embodiments, $R^7$ is $-NH_2$, $-N(H)CH_3$, or $-N(CH_3)_2$. In some embodiments, $R^7$ is $-N(CH_3)_2$.

In some embodiments, $R^7$ is $C_1$-$C_6$ haloalkyl. In some embodiments, $R^7$ is $C_1$-$C_6$ haloalkyl containing 1-13 halogen atoms. In some embodiments, $R^7$ is $C_1$-$C_3$ haloalkyl. In some embodiments, $R^7$ is $C_1$-$C_3$ haloalkyl containing 1-7 halogen atoms. In some embodiments, $R^7$ is

$-CF_3$, $-CHF_2$, $-CH_2F$, $-CCl_3$, $-CHCl_2$, $-CH_2Cl$, $-CF_2Cl$, $-CFCl_2$, $-CH_2CF_3$, $-CH_2CHF_2$, or $-CH_2CCl_3$. In some embodiments, $R^7$ is $-CF_3$, $-CHF_2$, or $-CH_2F$. In some embodiments, $R^4$ is $-CHF_2$. In some embodiments, $R^7$ is $-CH_2F$.

In some embodiments, z is 0-4. In some embodiments, z is 0-3. In some embodiments, z is 0, 1, or 2. In some embodiments, z is 0. In some embodiments, z is 1. In some embodiments, z is 2. In some embodiments, z is 3. In some embodiments, z is 4.

In some embodiments,

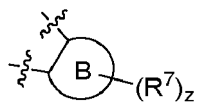

is:

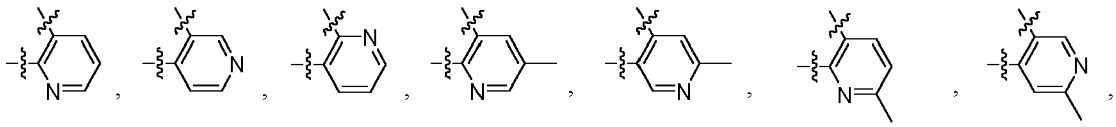

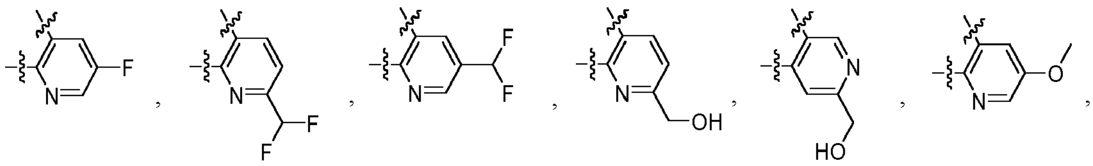

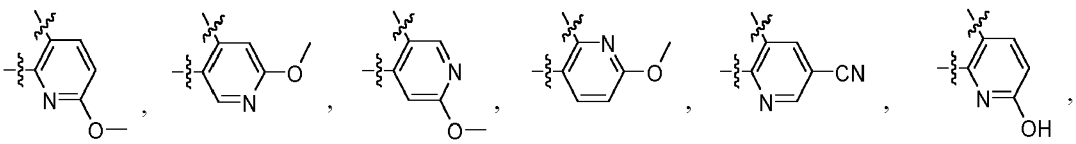

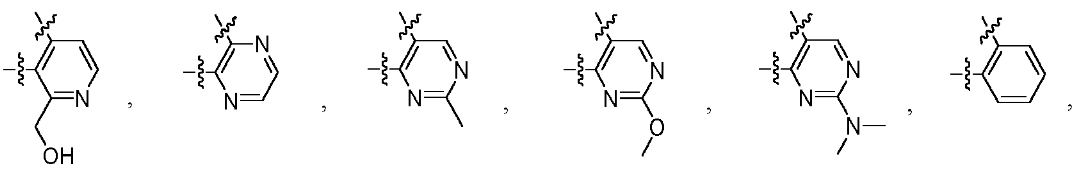

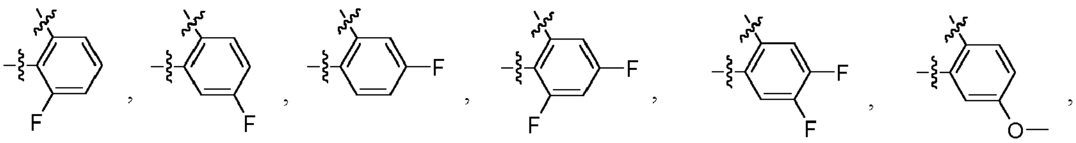

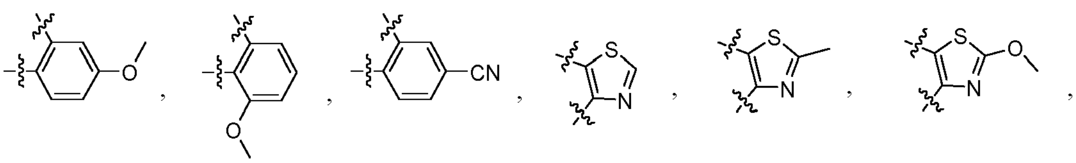

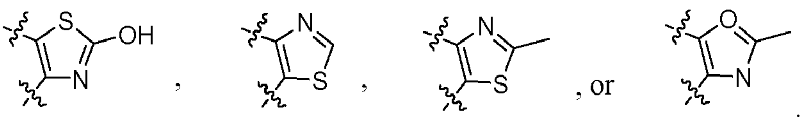

, or .

In some embodiments, each $R^a$, $R^b$, and $R^c$ is independently hydroxy, $C_1$-$C_6$ alkyl, or $C_1$-$C_6$ alkoxy. In some embodiments, each $R^a$, $R^b$, and $R^c$ is independently hydroxy, $C_1$-$C_3$ alkyl, or $C_1$-$C_3$ alkoxy.

In some embodiments, $R^a$ is hydroxy.

In some embodiments, $R^a$ is $C_1$-$C_6$ alkoxy. In some embodiments, $R^a$ is $C_1$-$C_3$ alkoxy. In some embodiments, $R^a$ is $-OCH_3$, $-OCH_2CH_3$, or $-OCH(CH_3)_2$. In some embodiments, $R^a$ is $-OCH_3$. In some embodiments, $R^a$ is $-OCH_2CH_3$.

In some embodiments, $R^a$ is $C_1$-$C_6$ alkyl. In some embodiments, $R^a$ is $C_1$-$C_3$ alkyl. In some embodiments, $R^a$

is methyl, ethyl, *n*-propyl, or isopropyl. In some embodiments, $R^a$ is methyl, ethyl, or isopropyl. In some embodiments, $R^a$ is methyl. In some embodiments, $R^a$ is ethyl. In some embodiments, $R^a$ is isopropyl.

In some embodiments, $R^b$ is hydroxy.

In some embodiments, $R^b$ is $C_1$-$C_6$ alkoxy. In some embodiments, $R^b$ is $C_1$-$C_3$ alkoxy. In some embodiments, $R^b$ is $-OCH_3$, $-OCH_2CH_3$, or $-OCH(CH_3)_2$. In some embodiments, $R^b$ is $-OCH_3$. In some embodiments, $R^b$ is $-OCH_2CH_3$.

In some embodiments, $R^b$ is $C_1$-$C_6$ alkyl. In some embodiments, $R^b$ is $C_1$-$C_3$ alkyl. In some embodiments, $R^b$ is methyl, ethyl, *n*-propyl, or isopropyl. In some embodiments, $R^b$ is methyl, ethyl, or isopropyl. In some embodiments, $R^b$ is methyl. In some embodiments, $R^b$ is ethyl. In some embodiments, $R^b$ is isopropyl.

In some embodiments, $R^c$ is hydroxy.

In some embodiments, $R^c$ is $C_1$-$C_6$ alkoxy. In some embodiments, $R^c$ is $C_1$-$C_3$ alkoxy. In some embodiments, $R^c$ is $-OCH_3$, $-OCH_2CH_3$, or $-OCH(CH_3)_2$. In some embodiments, $R^c$ is $-OCH_3$. In some embodiments, $R^c$ is $-OCH_2CH_3$.

In some embodiments, $R^c$ is $C_1$-$C_6$ alkyl. In some embodiments, $R^c$ is $C_1$-$C_3$ alkyl. In some embodiments, $R^c$ is methyl, ethyl, *n*-propyl, or isopropyl. In some embodiments, $R^c$ is methyl, ethyl, or isopropyl. In some embodiments, $R^c$ is methyl. In some embodiments, $R^c$ is ethyl. In some embodiments, $R^c$ is isopropyl.

In some embodiments, each $R^{2a}$ and $R^{2b}$ is independently H, $C_1$-$C_6$ alkyl, or $C_3$-$C_6$ cycloalkyl. In some embodiments, each $R^{2a}$ and $R^{2b}$ is independently H, $C_1$-$C_3$ alkyl, or $C_3$-$C_5$ cycloalkyl.

In some embodiments, $R^{2a}$ is H.

In some embodiments, $R^{2a}$ is $C_1$-$C_6$ alkyl. In some embodiments, $R^{2a}$ is $C_1$-$C_3$ alkyl. In some embodiments, $R^{2a}$ is methyl, ethyl, *n*-propyl, or isopropyl. In some embodiments, $R^{2a}$ is methyl, ethyl, or isopropyl. In some embodiments, $R^{2a}$ is methyl. In some embodiments, $R^{2a}$ is ethyl. In some embodiments, $R^{2a}$ is isopropyl.

In some embodiments, $R^{2a}$ is $C_3$-$C_6$ cycloalkyl. In some embodiments, $R^{2a}$ is $C_3$-$C_5$ cycloalkyl. In some embodiments, $R^{2a}$ is cyclopropyl, cyclobutyl, cyclopentyl, or cyclohexyl. In some embodiments, $R^{2a}$ is cyclopropyl, cyclobutyl, or cyclopentyl. In some embodiments, $R^{2a}$ is cyclopropyl.

In some embodiments, $R^{2b}$ is H.

In some embodiments, $R^{2b}$ is $C_1$-$C_6$ alkyl. In some embodiments, $R^{2b}$ is $C_1$-$C_3$ alkyl. In some embodiments, $R^{2b}$ is methyl, ethyl, *n*-propyl, or isopropyl. In some embodiments, $R^{2b}$ is methyl, ethyl, or isopropyl. In some embodiments, $R^{2b}$ is methyl. In some embodiments, $R^{2b}$ is ethyl. In some embodiments, $R^{2b}$ is isopropyl.

In some embodiments, $R^{2b}$ is $C_3$-$C_6$ cycloalkyl. In some embodiments, $R^{2b}$ is $C_3$-$C_5$ cycloalkyl. In some embodiments, $R^{2b}$ is cyclopropyl, cyclobutyl, cyclopentyl, or cyclohexyl. In some embodiments, $R^{2b}$ is cyclopropyl, cyclobutyl, or cyclopentyl. In some embodiments, $R^{2b}$ is cyclopropyl.

In some embodiments, $R^{2a}$ and $R^{2b}$ are both H. In some embodiments, $R^{2a}$ and $R^{2b}$ are both $C_1$-$C_3$ alkyl. In some embodiments, one of $R^{2a}$ and $R^{2b}$ is H and the other is $C_1$-$C_3$ alkyl. In some embodiments, one of $R^{2a}$ and $R^{2b}$ is H and the other is $C_3$-$C_6$ cycloalkyl. In some embodiments, one of $R^{2a}$ and $R^{2b}$ is $C_1$-$C_3$ alkyl and the other is $C_3$-$C_6$ cycloalkyl.

In some embodiments, the compound of Formula (I) is a compound of Formula (Ia):

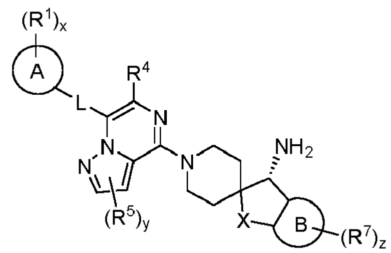

(Ia)

wherein Ring A, $R^1$, $R^4$, $R^5$, $R^7$, x, y, z, X, L, and Ring B are as described for Formula (I).

In some embodiments, the compound of Formula (I) is a compound of Formula (Ib):

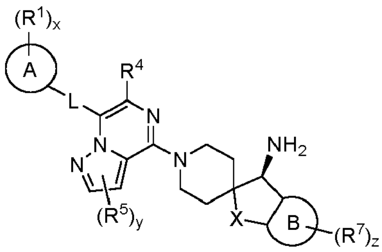

(Ib)

wherein Ring A, $R^1$, $R^4$, $R^5$, $R^7$, x, y, z, X, L, and Ring B are as described for Formula (I).

In some embodiments, the compound of Formula (I) is a compound of Formula (IIa), (IIb), (IIc), or (IId):

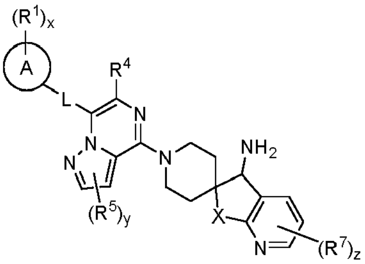

(IIa)

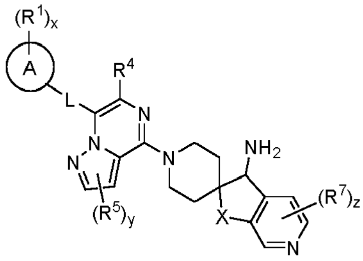

(IIb)

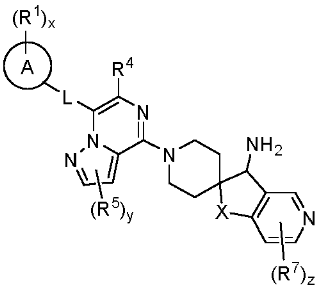

(IIc)

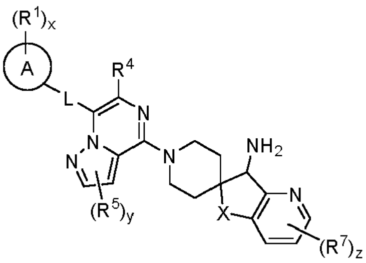

(IId)

wherein Ring A, $R^1$, $R^4$, $R^5$, $R^7$, x, y, z, X, and L are as described for Formula (I). In some embodiments, the compound is of Formula (IIa). In some embodiments, the compound is of Formula (IIb). In some embodiments, the compound is of Formula (IIc). In some embodiments, the compound is of Formula (IId). In some embodiments, L is a bond.

In some embodiments, the compound of Formula (I) is a compound of Formula (IIa-1):

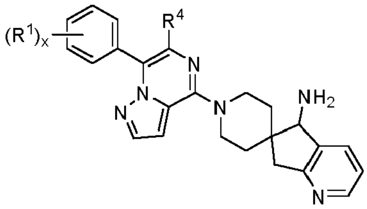

(IIa-1)

wherein $R^1$, $R^4$, and x are as described for Formula (I). In some embodiments, each $R^1$ is independently halo; x is 0, 1, or 2; and $R^4$ is $C_1$-$C_6$ alkyl. In some embodiments, $R^1$ is F; x is 0 or 1; and $R^4$ is $-CH_3$.

In some embodiments, the compound of Formula (I) is a compound of Formula (IIIa), (IIIb), (IIIc), (IIId), (IIIe), or (IIIf):

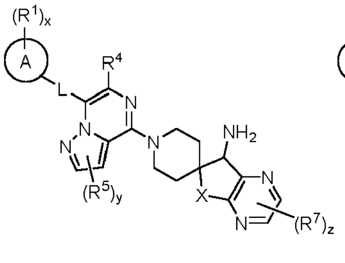
(IIIa)

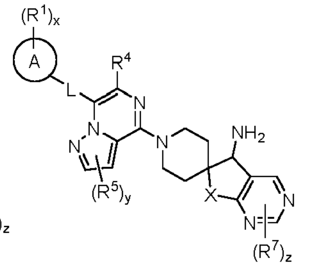
(IIIb)

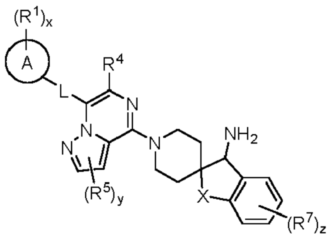
(IIIc)

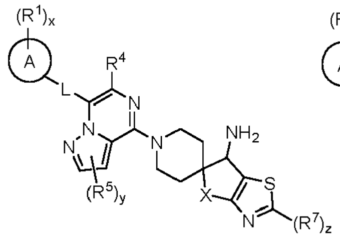
(IIId)

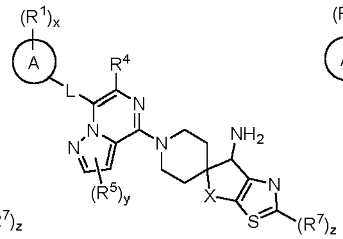
(IIIe)

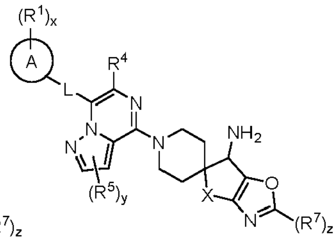
(IIIf)

wherein Ring A, $R^1$, $R^4$, $R^5$, $R^7$, x, y, z, X, and L are as described for Formula (I). In some embodiments, the compound is of Formula (IIIa). In some embodiments, the compound is of Formula (IIIb). In some embodiments, the compound is of Formula (IIIc). In some embodiments, the compound is of Formula (IIId). In some embodiments, the compound is of Formula (IIIe). In some embodiments, the compound is of Formula (IIIf).

In some embodiments, the compound of Formula (I) is a compound of Formula (IVa):

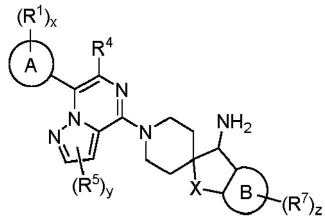
(IVa)

wherein Ring A, $R^1$, $R^4$, $R^5$, $R^7$, x, y, z, X, and Ring B are as described for Formula (I).

In some embodiments, the compound of Formula (I) is a compound of Formula (IVb), (IVc), (IVd), or (IVe):

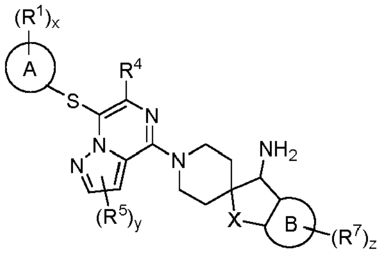
(IVb)

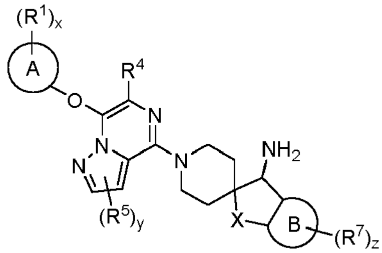
(IVc)

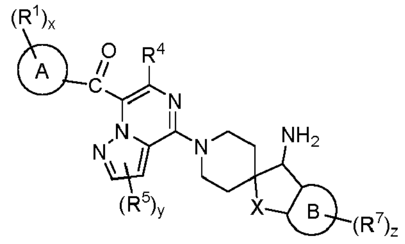

(IVd)

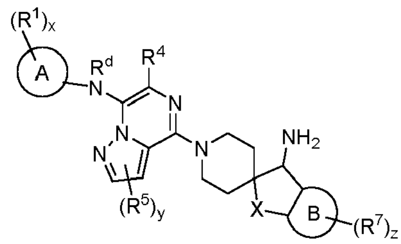

(IVe)

wherein Ring A, $R^1$, $R^4$, $R^5$, $R^7$, $R^d$, x, y, z, X, and Ring B are as described for Formula (I). In some embodiments, the compound is of Formula (IVb). In some embodiments, the compound is of Formula (IVc). In some embodiments, the compound is of Formula (IVd). In some embodiments, the compound is of Formula (IVe).

In any of the embodiments or variations described herein, it is understood that the stereochemistry at the carbon atom attached to the $-NH_2$ group of the spirocyclyl ring, indicated below with "*", has either (R) configuration or (S) configuration.

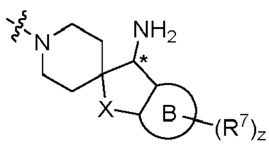

In some embodiments, the carbon atom (*) attached to the $-NH_2$ group of the spirocyclyl ring has (R) configuration. In other embodiments, the carbon atom (*) attached to the $-NH_2$ group of the spirocyclyl ring has (S) configuration. In some embodiments, the configuration of the carbon atom attached to the $-NH_2$ group of the spirocyclyl ring is:

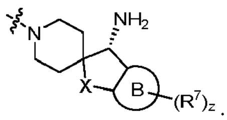

In some embodiments, the configuration of the carbon atom attached to the $-NH_2$ group of the spirocyclyl ring is:

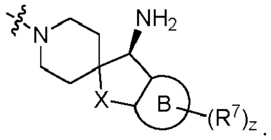

In any of the pharmaceutical compositions described herein, the compound of Formula (I) can be present as an enantiomerically pure compound or as a racemic mixture. In some embodiments, the pharmaceutical compositions comprise the (S) isomer (i.e., (S) configuration at the carbon atom (*) attached to the - $NH_2$ group of the spirocyclyl ring shown above) of the compound of Formula (I) in high purity. In some embodiments, the pharmaceutical compositions comprise the (S) isomer of the compound of Formula (I) in at least about 50%, 60%, 70%, 80%, or 90% of the total amount of the compound of Formula (I). In some embodiments, the pharmaceutical compositions comprise the (S) isomer of the compound of Formula (I) in at least about 90%, 91%, 92%, 93%, 94%, 95%, 96%, 97%, 98%, or 99% of the total amount of the compound of Formula (I). In some embodiments, the pharmaceutical compositions comprise the (R) isomer (i.e., (R) configuration at the carbon atom (*) attached to the - $NH_2$ group of the spirocyclyl ring shown above) of the compound of Formula (I) in small amounts. In some embodiments, the pharmaceutical compositions comprise the (R) isomer of the compound of Formula (I) in less than about 50%, 40%, 30%, 20%, or 10% of the total amount of the compound of Formula (I). In some embodiments, the pharmaceutical compositions comprise the (R) isomer of the compound of Formula (I) in less than about 10%, 9%, 8%, 7%, 6%, 5%, 4%, 3%, 2%, or 1% of the total amount of the compound of Formula (I). In some embodiments, the pharmaceutical compositions comprise the (S) isomer but not the (R) isomer of the compound of Formula (I).

In the descriptions herein, it is understood that every description, variation, embodiment, or aspect of a moiety may be combined with every description, variation, embodiment, or aspect of other moieties the same as if each and

every combination of descriptions is specifically and individually listed. For example, every description, variation, embodiment, or aspect provided herein with respect to Ring A of Formula (I) may be combined with every description, variation, embodiment, or aspect of $R^1$, $R^a$, $R^b$, $R^c$, $R^d$, $R^{2a}$, $R^{2b}$, $R^{3a}$, $R^{3b}$, $R^4$, $R^5$, $R^6$, $R^7$, L, X, Ring B, x, y, and z, the same as if each and every combination were specifically and individually listed. It is also understood that all descriptions, variations, embodiments, or aspects of Formula (I), where applicable, apply equally to other formulae detailed herein, and are equally described, the same as if each and every description, variation, embodiment, or aspect were separately and individually listed for all formulae. For example, all descriptions, variations, embodiments, or aspects of Formula (I), where applicable, apply equally to any of the formulae as detailed herein, such as Formulae (Ia), (Ib), (IIa), (IIb), (IIc), (IId), (IIa-1), (IIIa), (IIIb), (IIIc), (IIId), (IIIe), (IIIf), (IVa), (IVb), (IVc), (IVd), and (IVe), are equally described, the same as if each and every description, variation, embodiment, or aspect were separately and individually listed for all formulae.

In some embodiments, provided is a compound selected from the compounds in Table 1 or a pharmaceutically acceptable salt thereof. Although certain compounds described in the present disclosure, including in Table 1, are presented as specific stereoisomers and/or in a non-stereochemical form, it is understood that any or all stereochemical forms, including any enantiomeric or diastereomeric forms, and any tautomers or other forms of any of the compounds of the present disclosure, including in Table 1, are herein described. Similarly, although certain compounds described in the present disclosure, including in Table 1, are presented as specific salts, it is understood that any pharmaceutically acceptable salt of any of the compounds of the present disclosure, including in Table 1, are herein described. It is further understood that although certain compounds described in the present disclosure, including in Table 1, are presented as specific salts, the free form of the compounds of the present disclosure, including in Table 1, are also herein described.

**Table 1.**

| Compound No. | Structure | Compound No. | Structure |
|---|---|---|---|
| 1 | 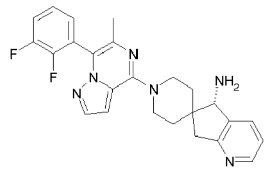 | 2 | 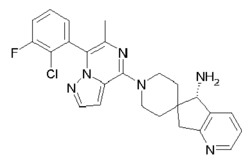 |
| 3 | 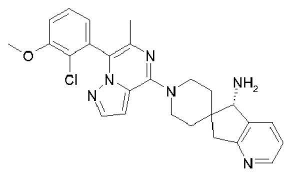 | 4 | 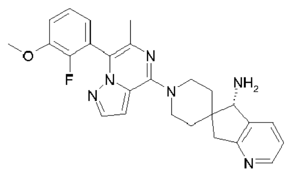 |
| 5 | 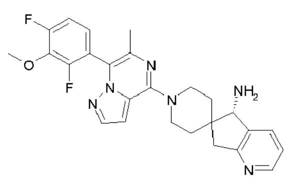 | 6 | 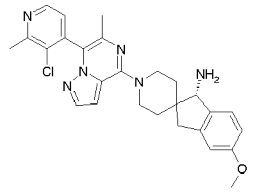 |
| 7 | 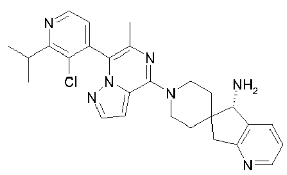 | 8 | 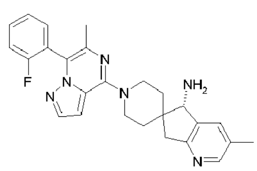 |
| 9 | 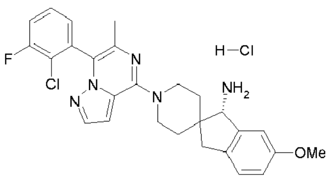 | 10 | 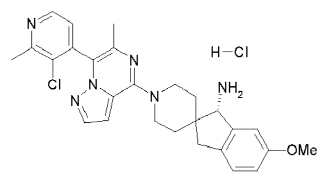 |

(continued)

| Compound No. | Structure | Compound No. | Structure |
|---|---|---|---|
| 11 | 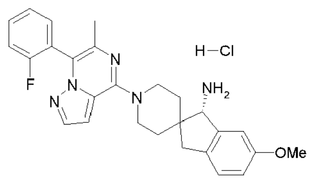 | 12 | 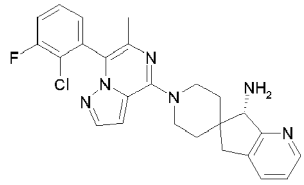 |
| 13 | 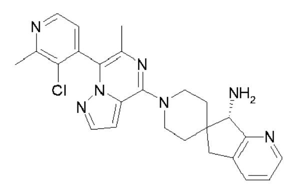 | 14 | 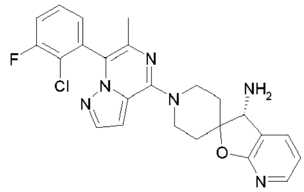 |
| 15 | 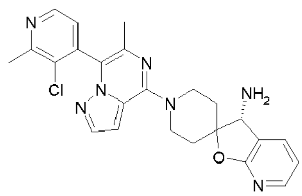 | 16 | 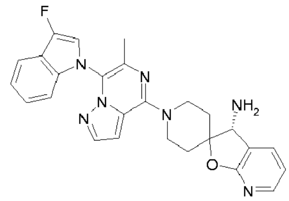 |
| 17 | 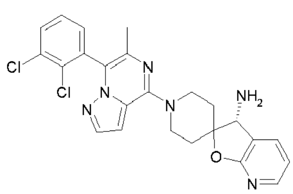 | 18 | 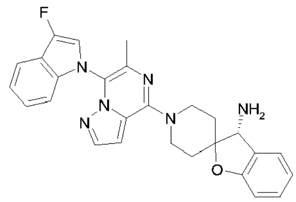 |
| 19 | 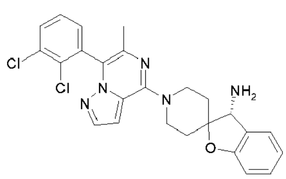 | 20 | 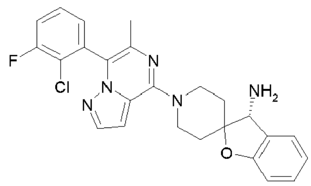 |
| 21 | 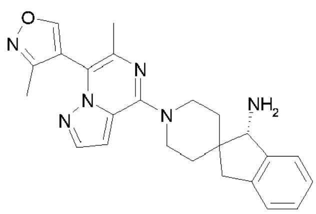 | 22 | 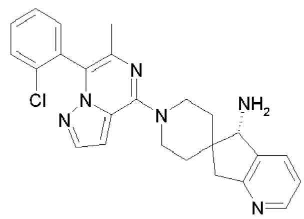 |
| 23 | 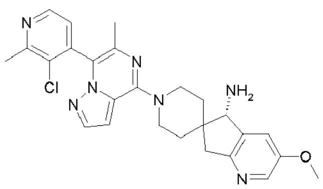 | 24 | 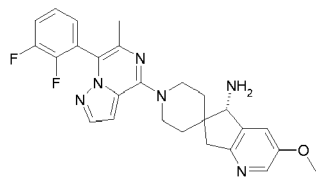 |

(continued)

| Compound No. | Structure | Compound No. | Structure |
|---|---|---|---|
| **25** | 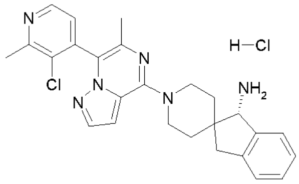 | **26** | 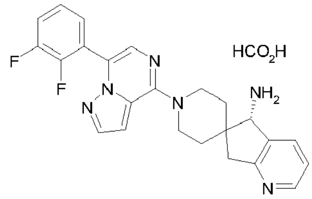 |
| **27** | 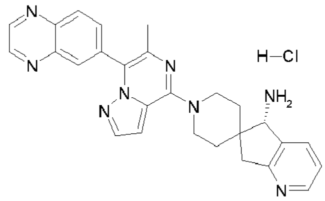 | **28** | 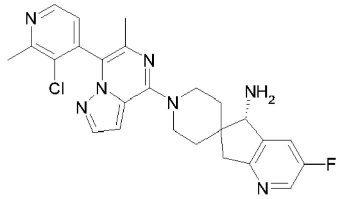 |
| **29** | 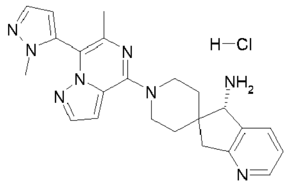 | **30** | 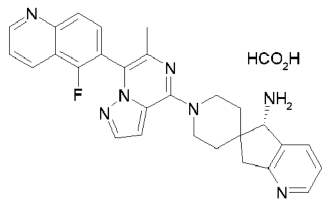 |
| **31** | 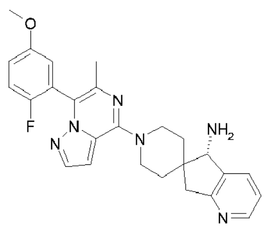 | **32** | 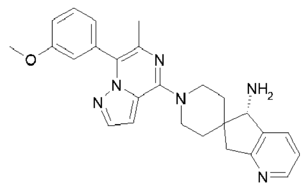 |
| **33** | 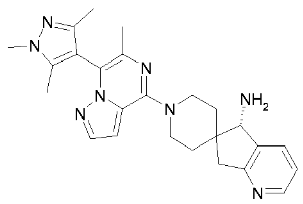 | **34** | 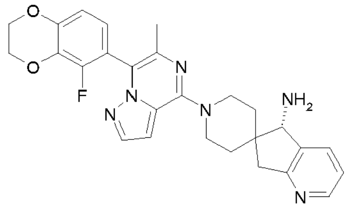 |
| **35** | 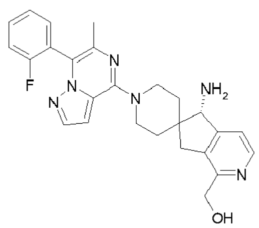 | **36** | 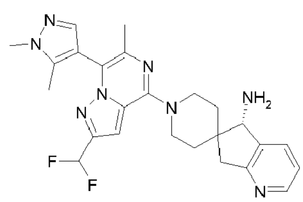 |
| **37** | 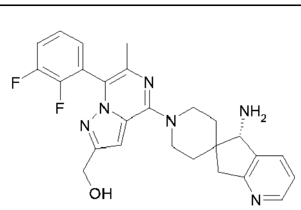 | **38** | 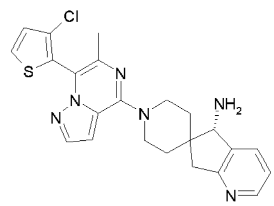 |

(continued)

| Compound No. | Structure | Compound No. | Structure |
|---|---|---|---|
| 39 | 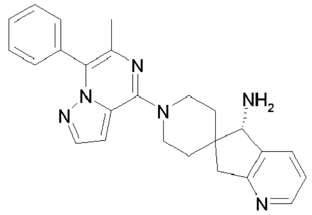 | 40 | 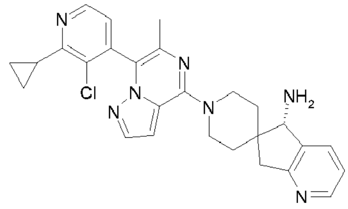 |
| 41 | 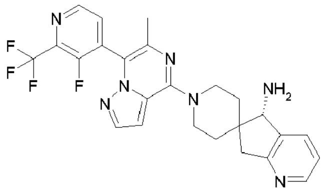 | 42 | 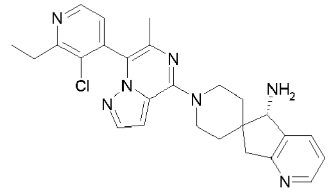 |
| 43 | 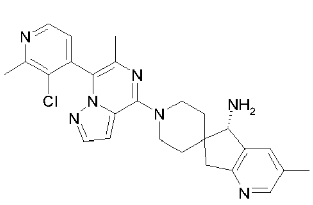 | 44 | 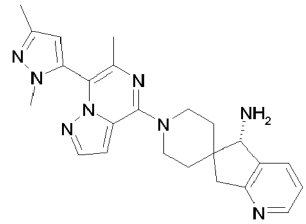 |
| 45 | 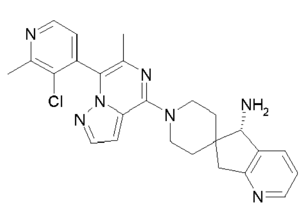 | 46 | 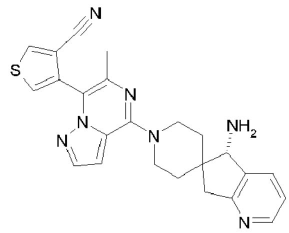 |
| 47 | 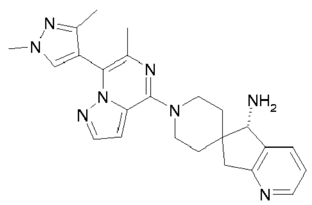 | 48 | 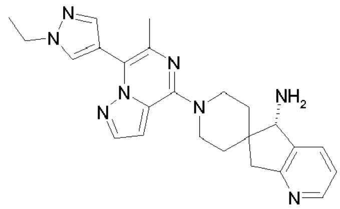 |
| 49 | 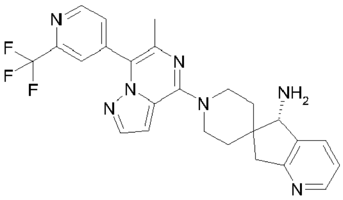 | 50 | 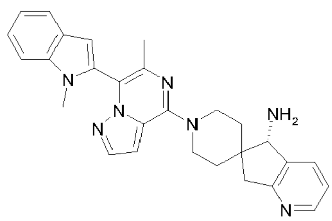 |
| 51 | 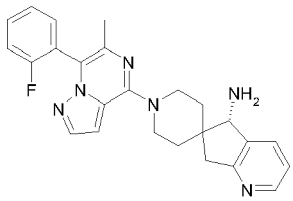 | 52 | 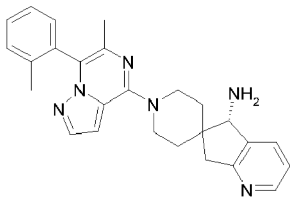 |

(continued)

| Compound No. | Structure | Compound No. | Structure |
|---|---|---|---|
| 53 | 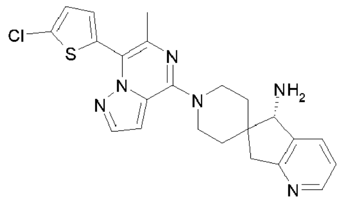 | 54 | 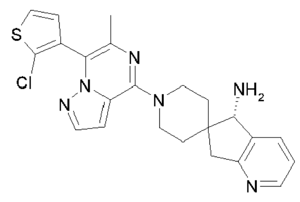 |
| 55 | 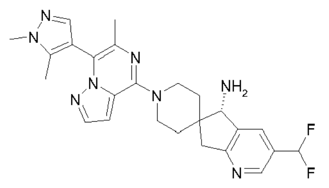 | 56 | 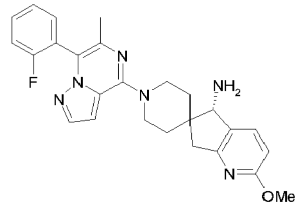 |
| 57 | 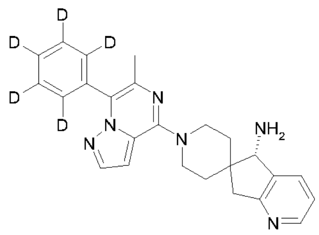 | 58 | 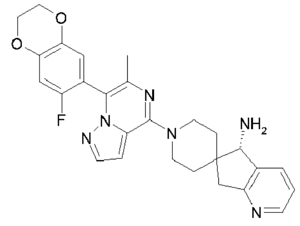 |
| 59 | 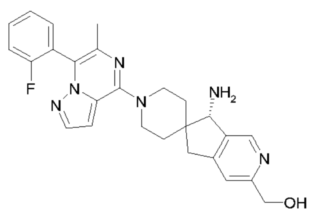 | 60 | 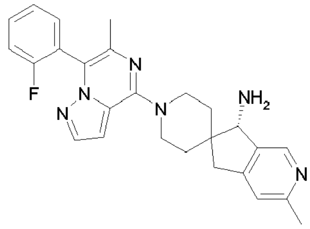 |
| 61 | 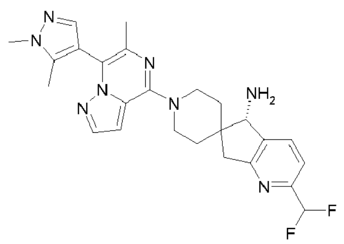 | 62 | 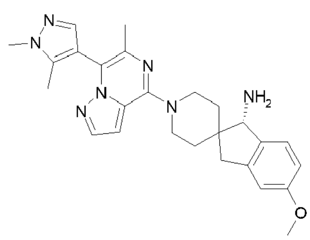 |
| 63 | 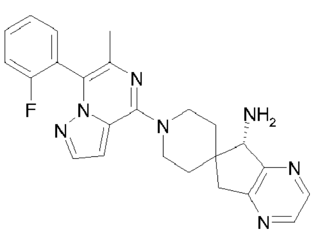 | 64 | 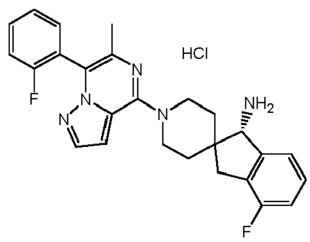 |

(continued)

| Compound No. | Structure | Compound No. | Structure |
|---|---|---|---|
| **65** | 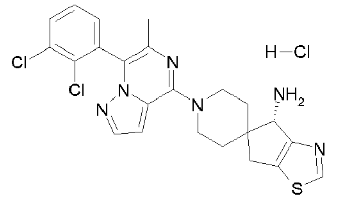 | **66** | 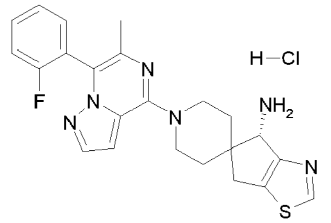 |
| **67** | 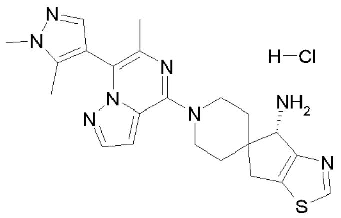 | **68** | 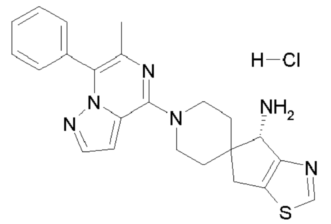 |
| **69** | 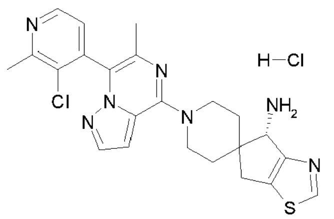 | **70** | 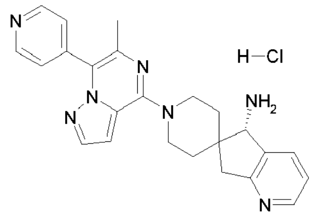 |
| **71** | 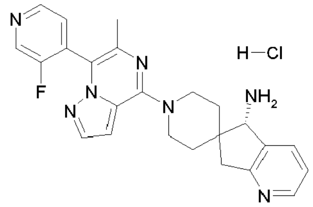 | **72** | 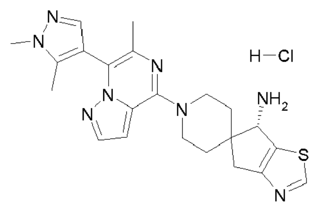 |
| **73** | 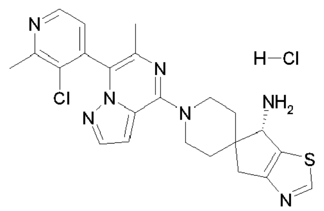 | **74** | 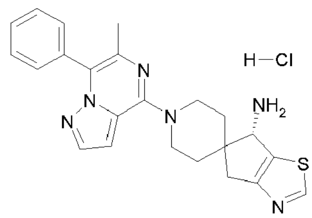 |
| **75** | 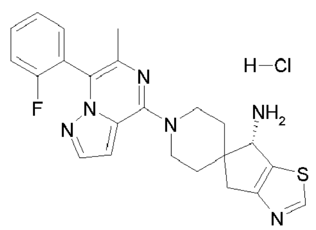 | **76** | 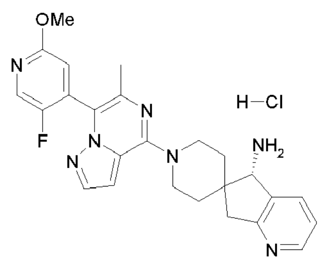 |
| **77** | 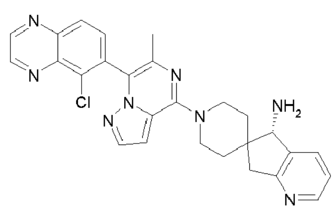 | **78** | 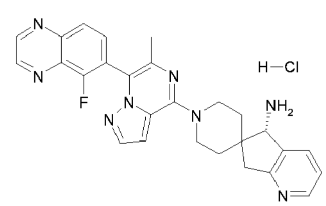 |

(continued)

| Compound No. | Structure | Compound No. | Structure |
|---|---|---|---|
| 79 | 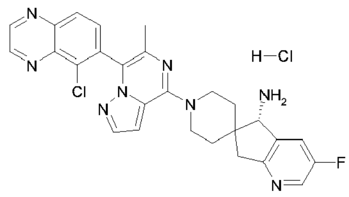 | 80 | 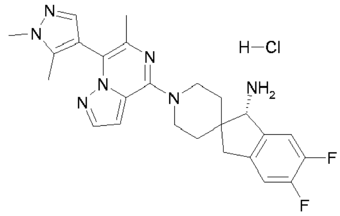 |
| 81 | 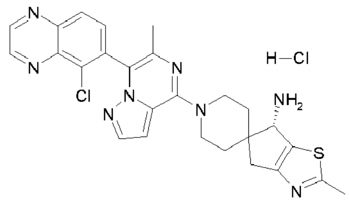 | 82 | 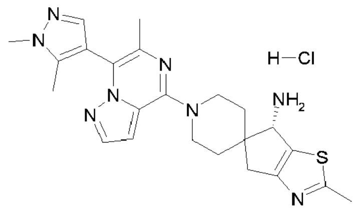 |
| 83 | 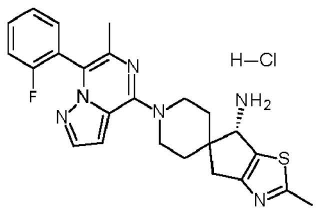 | 84 | 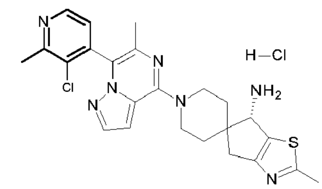 |
| 85 | 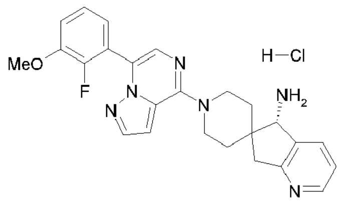 | 86 | 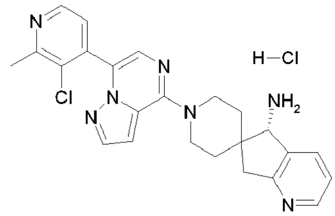 |
| 87 | 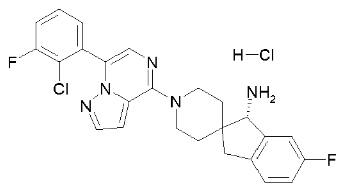 | 88 | 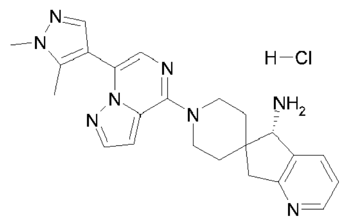 |
| 89 | 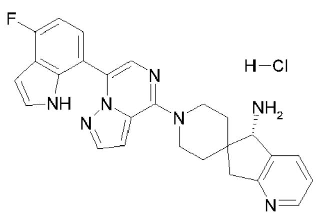 | 90 | 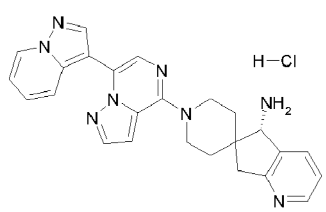 |
| 91 | 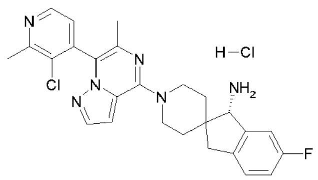 | 92 | 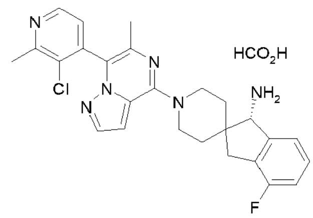 |

(continued)

| Compound No. | Structure | Compound No. | Structure |
|---|---|---|---|
| **93** | 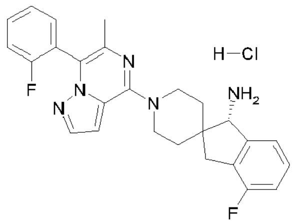 | **94** | 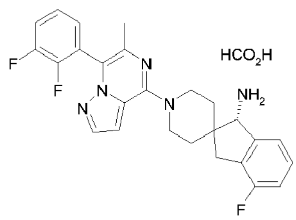 |
| **95** | 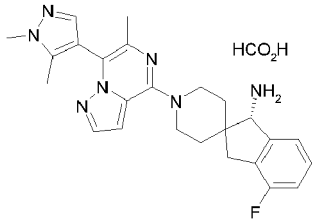 | **96** | 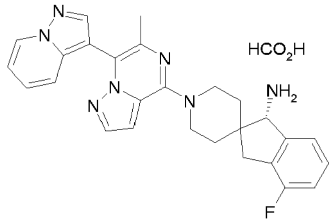 |
| **97** | 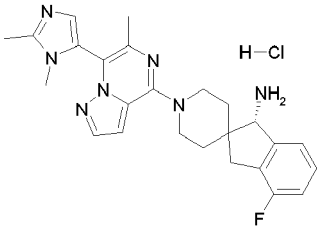 | **98** | 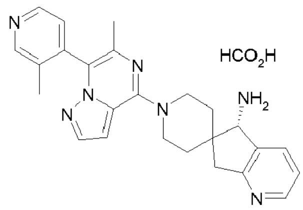 |
| **99** | 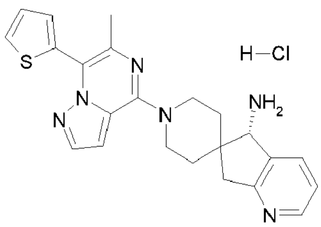 | **100** | 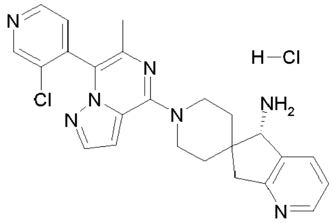 |
| **101** | 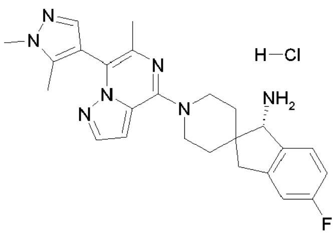 | **102** | 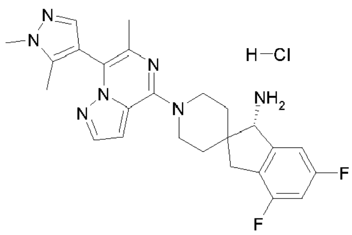 |
| **103** | 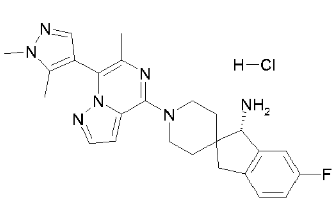 | **104** | 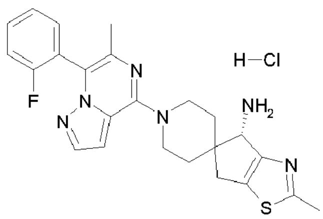 |

(continued)

| Compound No. | Structure | Compound No. | Structure |
|---|---|---|---|
| 105 | 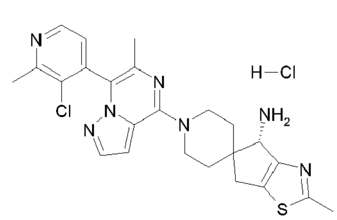 | 106 | 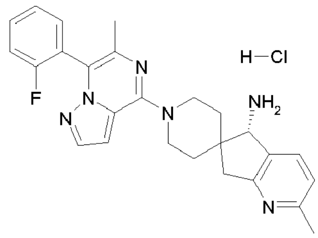 |
| 107 | 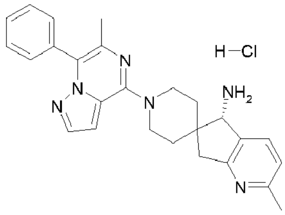 | 108 | 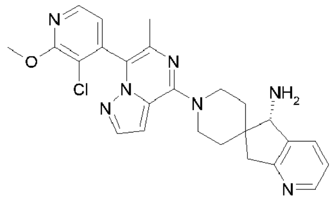 |
| 109 | 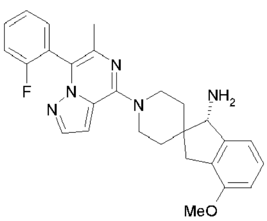 | 110 | 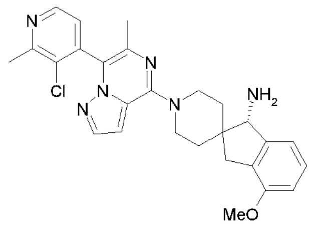 |
| 111 | 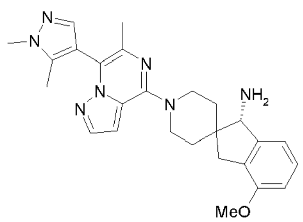 | 112 | 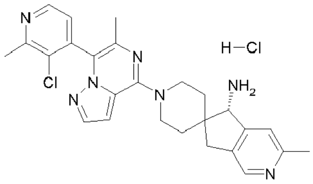 |
| 113 | 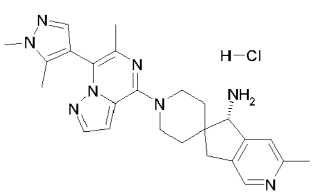 | 114 | 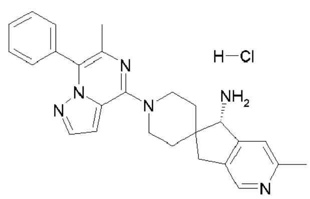 |
| 115 | 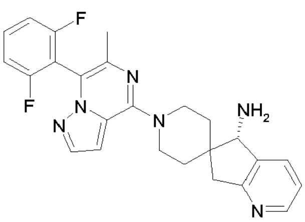 | 116 | 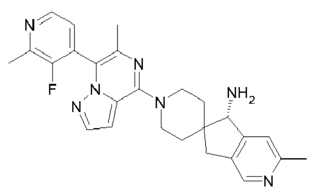 |
| 117 | 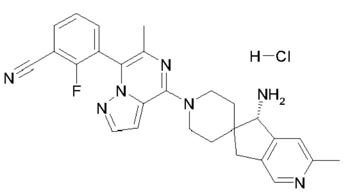 | 118 | 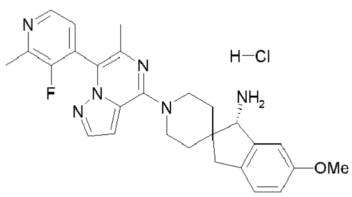 |

(continued)

| Compound No. | Structure | Compound No. | Structure |
|---|---|---|---|
| **119** | 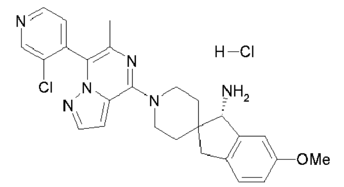 | **120** | 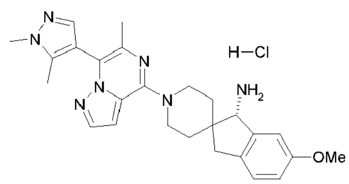 |
| **121** | 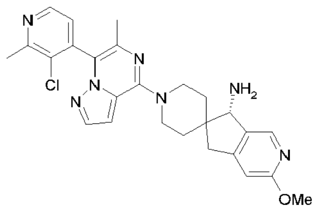 | **122** | 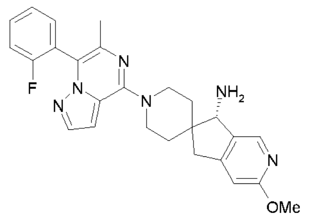 |
| **123** | 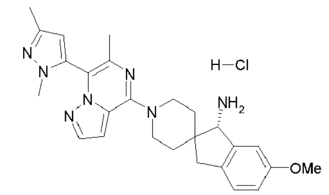 | **124** | 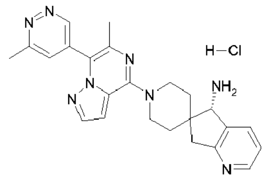 |
| **125** | 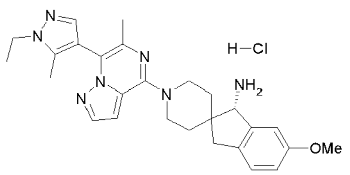 | **126** | 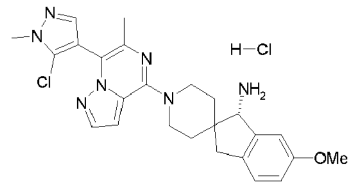 |
| **127** | 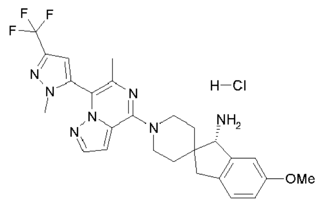 | **128** | 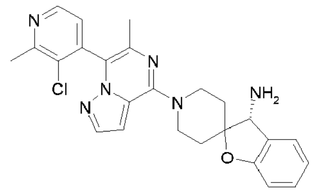 |
| **129** | 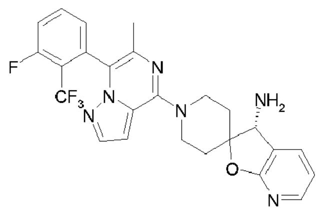 | **130** | 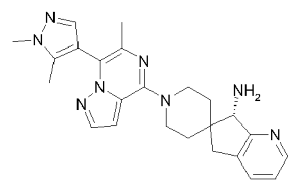 |
| **131** | 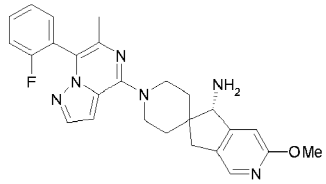 | **132** | 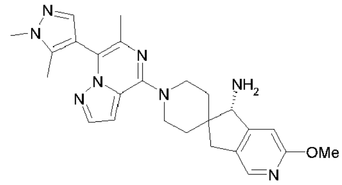 |

(continued)

| Compound No. | Structure | Compound No. | Structure |
|---|---|---|---|
| 133 | 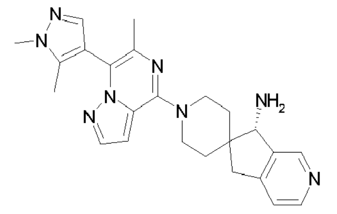 | 134 | 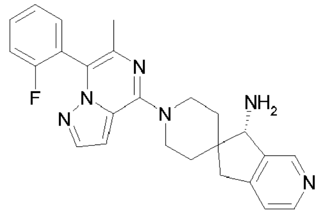 |
| 135 | 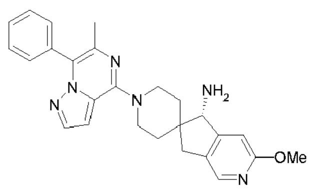 | 136 | 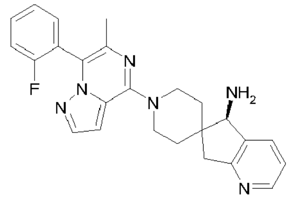 |
| 137 | 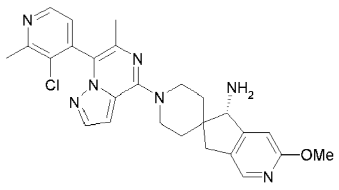 | 138 | 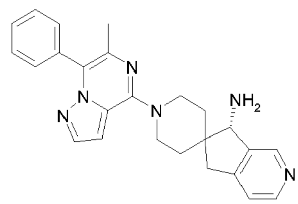 |
| 139 | 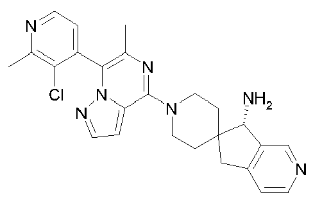 | 140 | 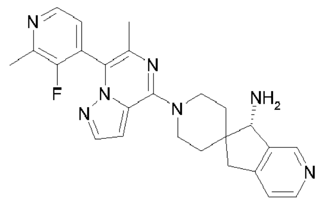 |
| 141 | 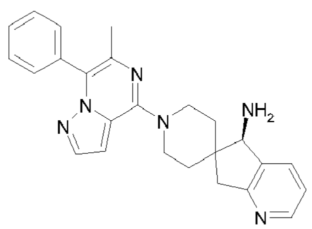 | 142 | 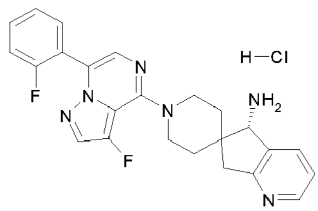 |
| 143 | 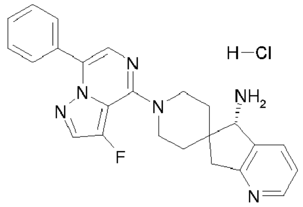 | 144 | 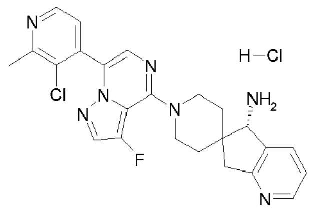 |
| 145 | 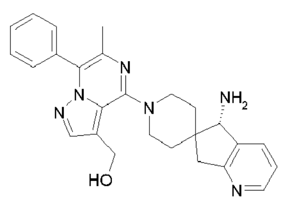 | 146 | 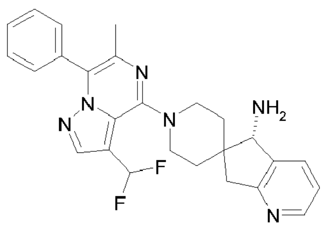 |

(continued)

| Compound No. | Structure | Compound No. | Structure |
|---|---|---|---|
| **147** | 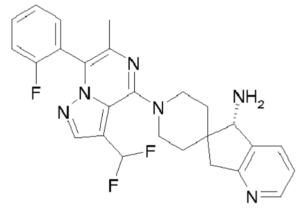 | **148** | 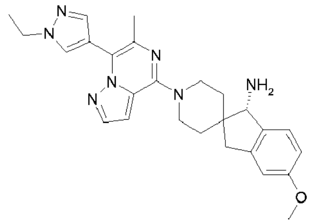 |
| **149** | 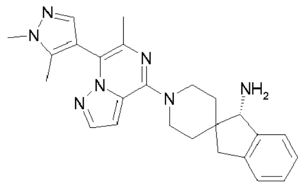 | **150** | 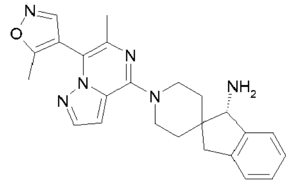 |
| **151** | 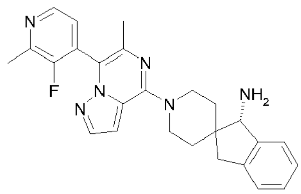 | **152** | 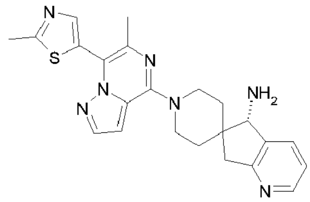 |
| **153** | 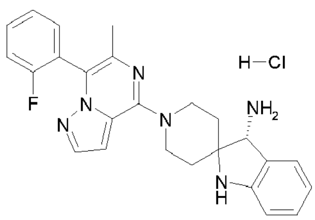 | **154** | 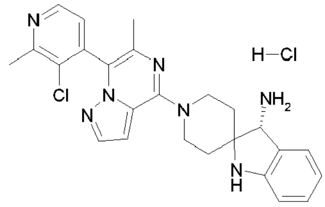 |
| **155** | 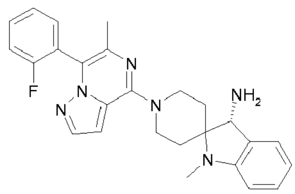 | **156** | 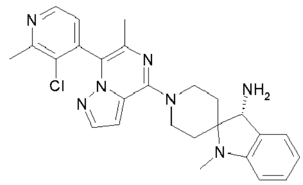 |
| **157** | 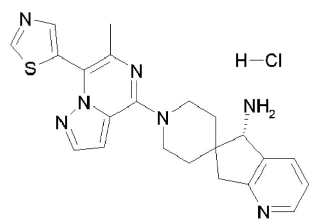 | **158** | 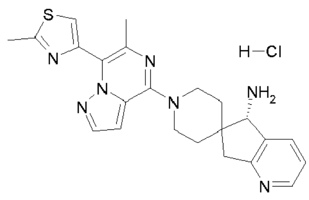 |
| **159** | 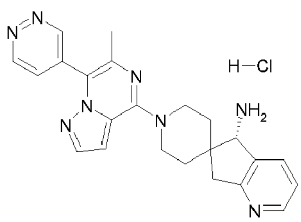 | **160** | 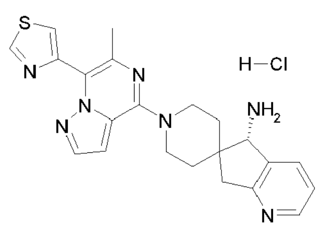 |

(continued)

| Compound No. | Structure | Compound No. | Structure |
|---|---|---|---|
| 161 | 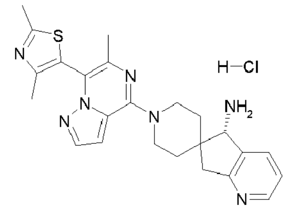 | 162 | 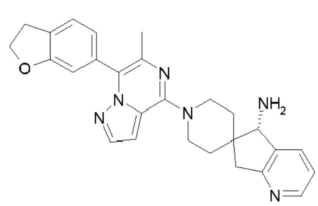 |
| 163 | 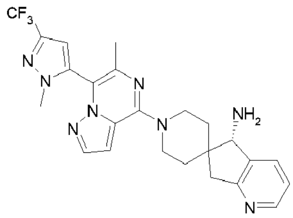 | 164 | 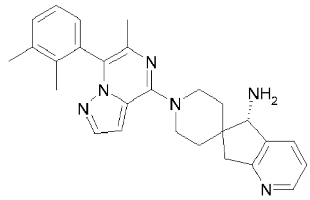 |
| 165 | 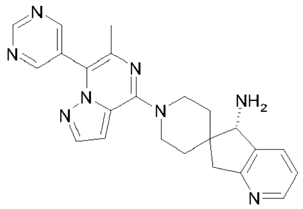 | 166 | 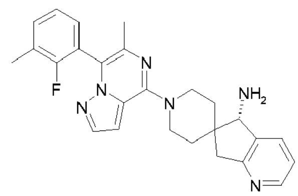 |
| 167 | 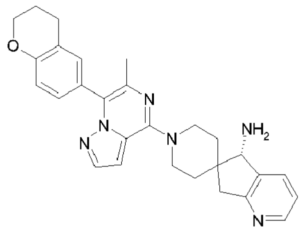 | 168 | 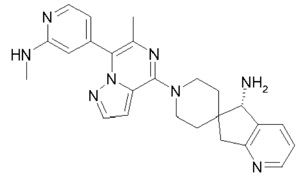 |
| 169 | 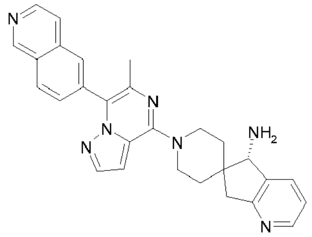 | 170 | 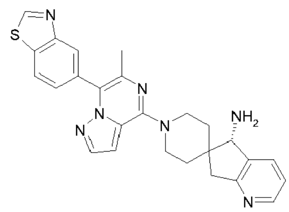 |
| 171 | 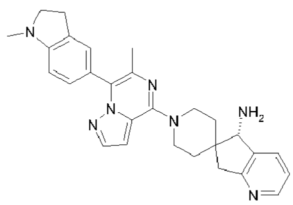 | 172 | 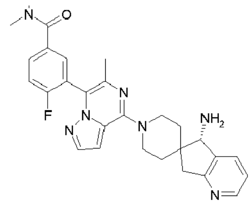 |

(continued)

| Compound No. | Structure | Compound No. | Structure |
|---|---|---|---|
| **173** | 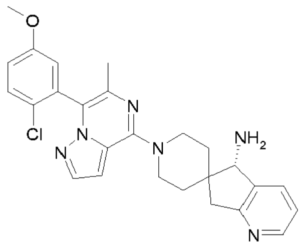 | **174** | 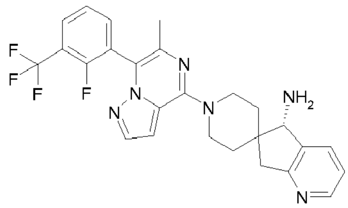 |
| **175** | 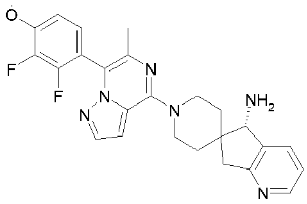 | **176** | 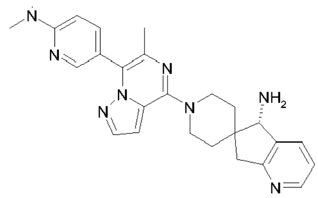 |
| **177** | 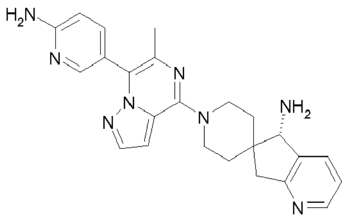 | **178** | 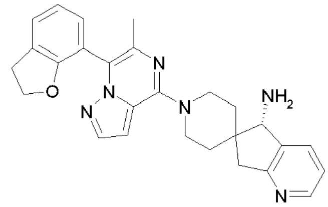 |
| **179** | 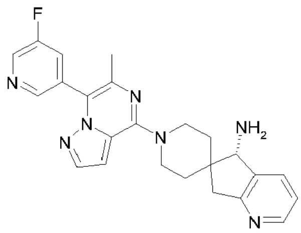 | **180** | 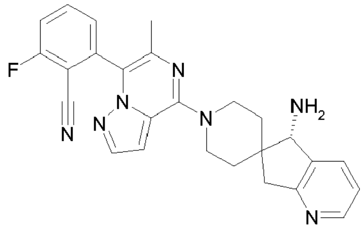 |
| **181** | 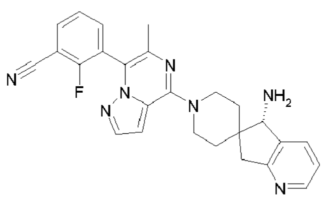 | **182** | 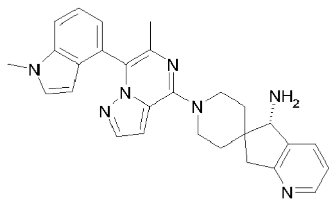 |
| **183** | 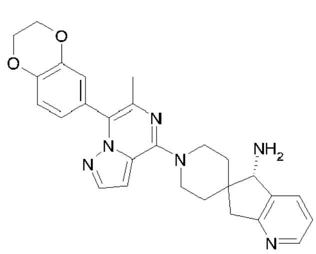 | **184** | 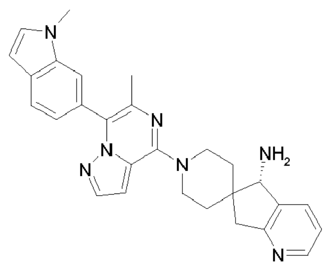 |

(continued)

| Compound No. | Structure | Compound No. | Structure |
|---|---|---|---|
| 185 | 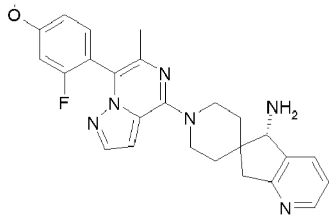 | 186 | 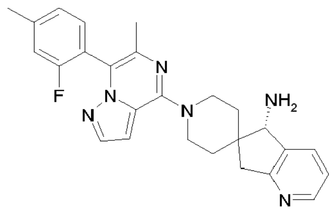 |
| 187 | 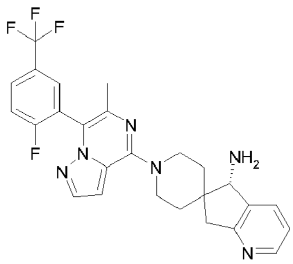 | 188 | 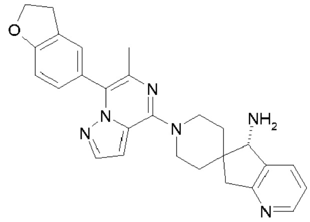 |
| 189 | 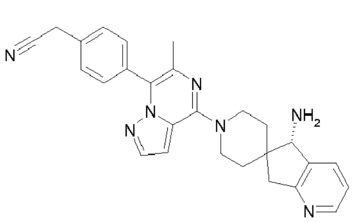 | 190 | 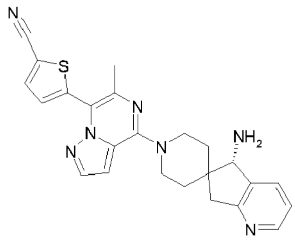 |
| 191 | 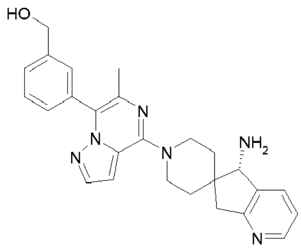 | 192 | 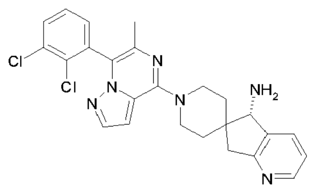 |
| 193 | 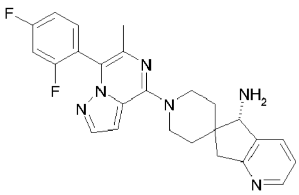 | 194 | 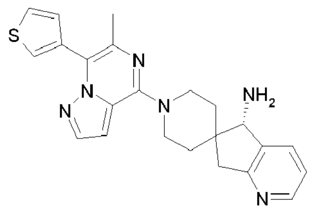 |
| 195 | 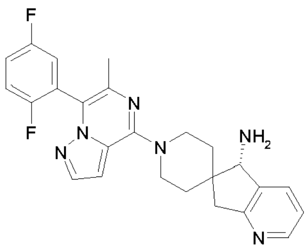 | 196 | 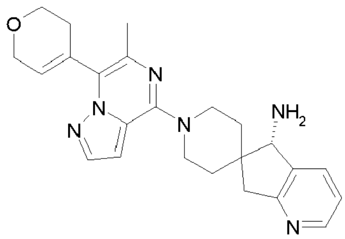 |

(continued)

| Compound No. | Structure | Compound No. | Structure |
|---|---|---|---|
| 197 | 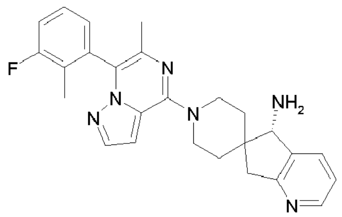 | 198 | 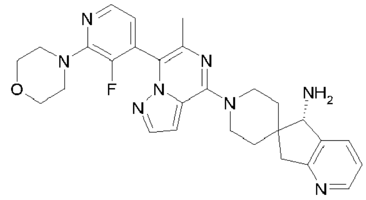 |
| 199 | 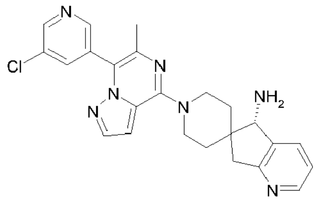 | 200 | 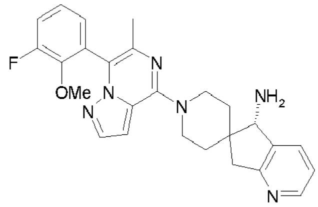 |
| 201 | 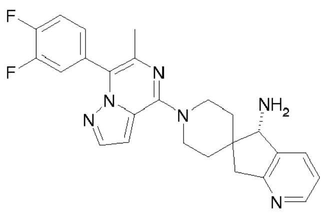 | 202 | 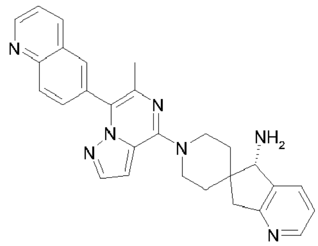 |
| 203 | 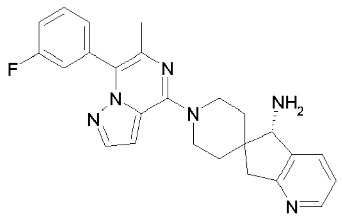 | 204 | 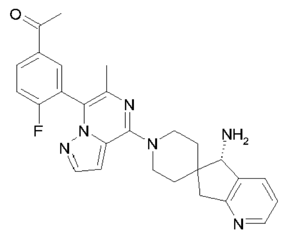 |
| 205 | 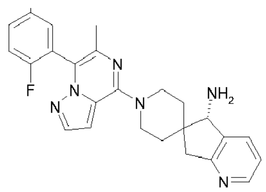 | 206 | 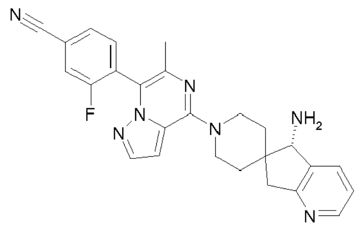 |
| 207 | 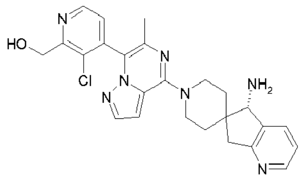 | 208 | 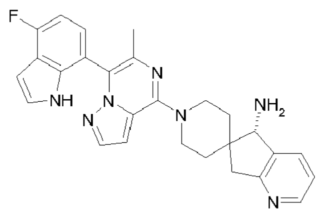 |
| 209 | 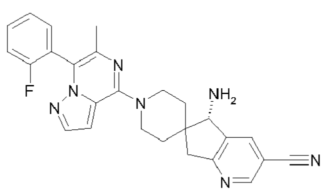 | 210 | 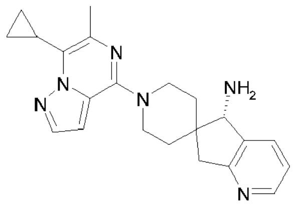 |

(continued)

| Compound No. | Structure | Compound No. | Structure |
|---|---|---|---|
| **211** | 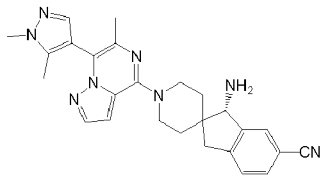 | **212** | 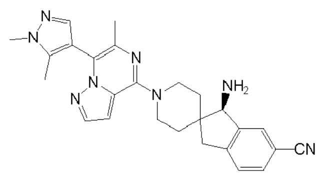 |
| **213** | 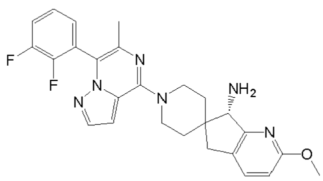 | **214** | 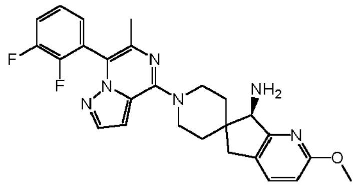 |
| **215** | 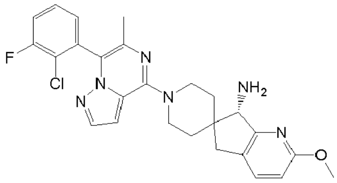 | **216** | 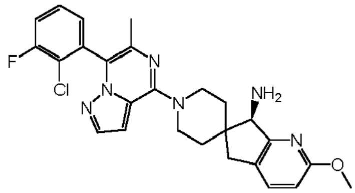 |
| **217** | 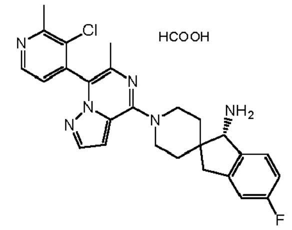 HCOOH | **218** | 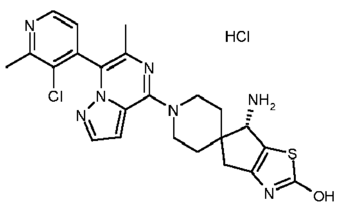 HCl |
| **219** | 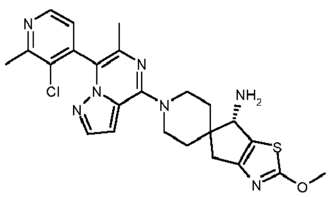 | **220** | 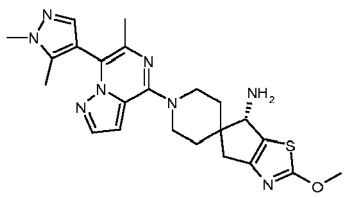 |
| **221** | 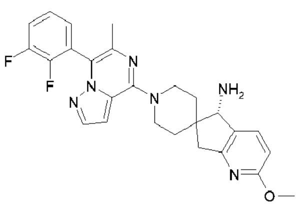 | **222** | 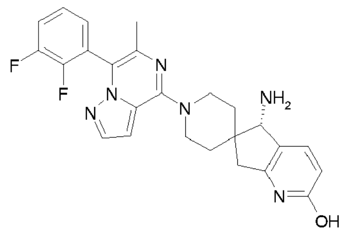 |
| **223** | 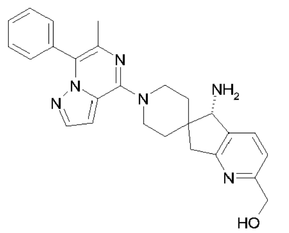 | **224** | 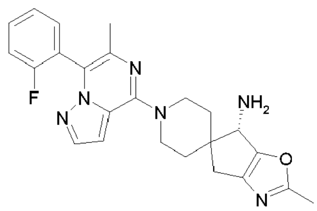 |

(continued)

| Compound No. | Structure | Compound No. | Structure |
|---|---|---|---|
| 225 | 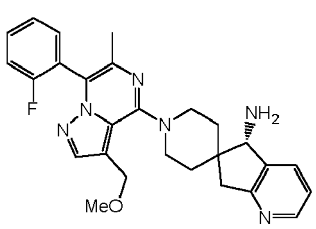 | 226 | 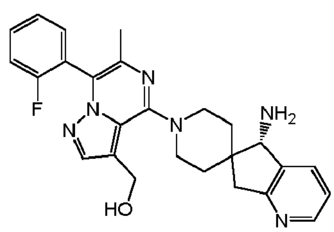 |
| 227 | 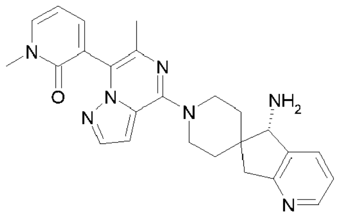 | 228 | 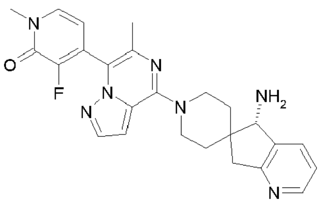 |
| 229 | 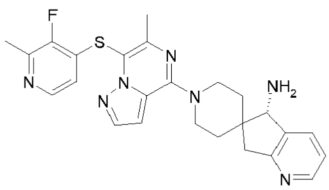 | 230 | 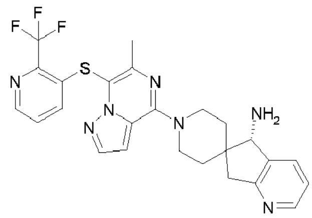 |
| 231 | 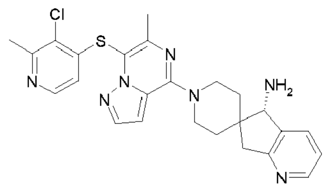 | 232 | 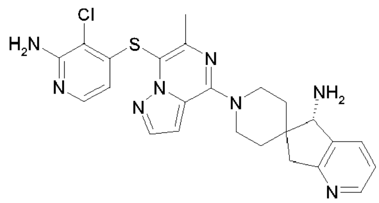 |

or a pharmaceutically acceptable salt thereof.

In some embodiments, the compound of Formula (I), or a pharmaceutically acceptable salt thereof, does not include Compound Nos. 33, 129, 145, 146, 147, 172, 210, 225, 226, and 227.

It is understood that in the present description, combinations of substituents and/or variables of the depicted formulae are permissible only if such contributions result in stable compounds.

Furthermore, all compounds of Formula (I) that exist in free base or acid form can be converted to their pharmaceutically acceptable salts by treatment with the appropriate inorganic or organic base or acid by methods known to one skilled in the art. Salts of the compounds of Formula (I) can be converted to their free base or acid form by standard techniques.

**Methods of Synthesis**

In a further aspect, provided herein are methods of preparing compounds of Formula (I) or pharmaceutically acceptable salts thereof.

Compounds of Formula (I) or any variation thereof may be prepared according to the general reactions shown in Schemes 1 and 2.

**Scheme 1.**

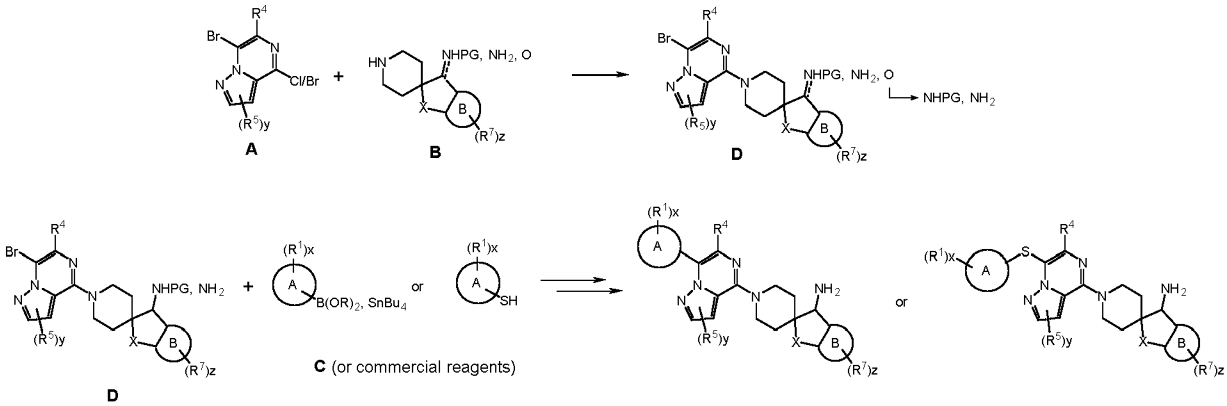


A B D

D

A general synthesis of certain compounds of Formula (I), wherein L is a bond or S, is outlined in Scheme 1. Coupling of 7-bromo-4-chloro (or 4-bromo) pyrazolo[1,5-a]pyrazine (Intermediate A) to a substituted secondary amine (Intermediate B) affords 7-bromo-pyrazolo[1,5-a]pyrazin-4-amine (Intermediate D). In some cases, the substituted secondary amine (Intermediate B) can bear a primary amine, which is optionally protected (PG = protecting group), or a ketone that will be subsequently transformed to a primary amine. Intermediate D can be coupled to a substituted aryl, heteroaryl, or alkyl boronic acid/ester (R is H or alkyl), a stannane, or a substituted heteroaryl-thiol (Intermediate C) in the presence of palladium catalyst, and additional deprotection and/or functionalization steps can be made to produce the final compound of Formula (I).

**Scheme 2.**

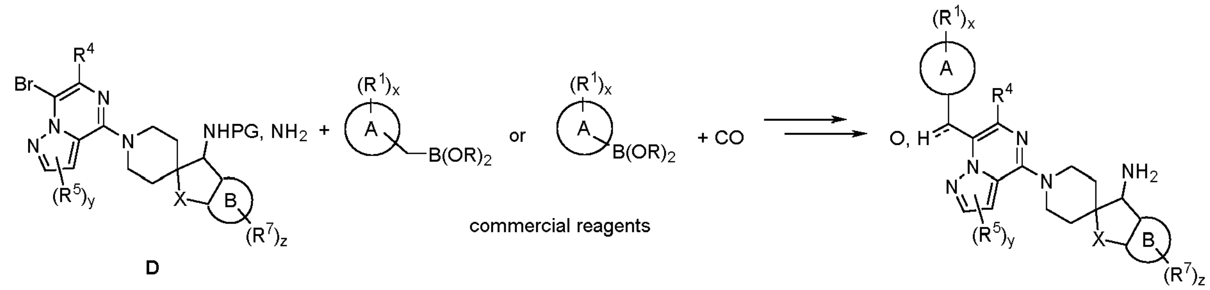


D

Scheme 2 provides a general synthesis of certain additional compounds of Formula (I), such as wherein L is CO. Intermediate D can be coupled to a substituted aryl, heteroaryl, or alkyl boronic acid/ester (R is H or alkyl) in the presence of palladium catalyst and a source of CO, and additional deprotection and/or functionalization steps can be made to produce the final compound of Formula (I).

In some embodiments of the methods described herein, compounds of Formula (I) are synthesized through a reduction step (for example, sulfinime reduction using DIBAL-H), to afford (S) configuration at the carbon atom indicated below with "*" in high purity.

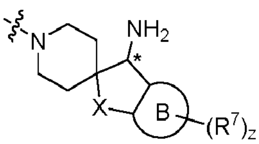

It is understood that the synthetic processes disclosed herein may be modified to arrive at various compounds of the present disclosure by selection of appropriate reagents and starting materials.

All compounds of Formula (I) or any variation thereof as described herein which exist in free base or acid form can be converted to their pharmaceutically acceptable salts by treatment with the appropriate inorganic or organic base or acid by methods known to one skilled in the art. Salts of the compounds of the disclosure can be converted to their free base or acid form by standard techniques.

Also provided herein are intermediate compounds useful for preparing compounds of Formula (I) or pharmaceutically acceptable salts thereof.

**Pharmaceutical Compositions**

In another aspect, provided herein are pharmaceutical compositions of the compounds of Formula (I) or a pharmaceutically acceptable salt thereof. Thus, the present disclosure includes pharmaceutical compositions comprising a compound of Formula (I), or a pharmaceutically acceptable salt thereof, and a pharmaceutically acceptable carrier or excipient. Pharmaceutical compositions according to the disclosure may take a form suitable for oral, buccal, sublingual, parenteral (subcutaneous, intramuscular, intravenous, or intrathecal), nasal, topical, vaginal, rectal, intracerebral, intradermal, intravitreal, intraosseous infusion, intraperitoneal, or inhalation administration. Pharmaceutical compositions of the present disclosure comprise a compound of Formula (I), or a pharmaceutically acceptable salt thereof, and a pharmaceutically acceptable carrier, diluent, or excipient.

A compound described herein can be used in the preparation of a pharmaceutical composition by combining the compound as an active ingredient with a pharmaceutically acceptable excipient. Some examples of materials which can serve as pharmaceutically acceptable excipients include: sugars, such as lactose, glucose and sucrose; starches, such as corn starch and potato starch; cellulose and its derivatives, such as sodium carboxymethyl cellulose, ethyl cellulose and cellulose acetate; surfactants, such as polysorbate 80 (i.e., Tween 80); powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations. Pharmaceutical formulations may be prepared by known pharmaceutical methods. Suitable formulations can be found in, for example, Remington: The Science and Practice of Pharmacy, Lippincott Williams & Wilkins, 21st ed. (2005), which is incorporated herein by reference.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol and the like; and metal chelating agents, such as citric acid, ethylenediamine tetraacetic acid (EDTA), sorbitol, tartaric acid, phosphoric acid and the like.

The pharmaceutical compositions may conveniently be presented in unit dosage form and may be prepared by any methods well known in the art of pharmacy. The amount of active ingredient which can be combined with a carrier material to produce a single dosage form will vary depending upon the subject being treated and the particular mode of administration. The amount of active ingredient that can be combined with a carrier material to produce a single dosage form will generally be that amount of the compound which produces a therapeutic effect. Generally, this amount will range from about 1% to about 99% of active ingredient, preferably from about 5% to about 70%, most preferably from about 10% to about 30%.

In certain embodiments, a pharmaceutical composition of the present disclosure comprises an excipient selected from the group consisting of cyclodextrins, liposomes, micelle forming agents, e.g., bile acids and polymeric carriers, e.g., polyesters and polyanhydrides; and a compound of Formula (I) or a pharmaceutically acceptable salt thereof. In certain embodiments, the pharmaceutical composition renders orally bioavailable a compound of Formula (I) or a pharmaceutically acceptable salt thereof.

Pharmaceutical compositions of the disclosure suitable for oral administration may be in the form of capsules, cachets, pills, tablets, lozenges (using a flavored basis, usually sucrose and acacia or tragacanth), powders, granules or as a solution or a suspension in an aqueous or non-aqueous liquid or as an oil-in-water or water-in-oil liquid emulsion or as an elixir or syrup or as pastilles (using an inert base, such as gelatin and glycerin or sucrose and acacia) and/or as mouth washes and the like, each containing a predetermined amount of a compound of Formula (I), or a pharmaceutically acceptable salt thereof, as an active ingredient. A compound of Formula (I), or a pharmaceutically acceptable salt thereof, may also be administered as a bolus, electuary, or paste.

In solid dosage forms of the disclosure for oral administration (capsules, tablets, pills, dragees, powders, granules and the like), the active ingredient is mixed with one or more pharmaceutically-acceptable carriers, such as sodium citrate or dicalcium phosphate and/or any of the following: fillers or extenders, such as starches, lactose, sucrose, glucose, mannitol and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates and sodium carbonate; solution retarding agents, such

as paraffin; absorption accelerators, such as quaternary ammonium compounds; wetting agents, such as, for example, cetyl alcohol, glycerol monostearate and non-ionic surfactants; absorbents, such as kaolin and bentonite clay; lubricants, such a talc, calcium stearate, magnesium stearate, solid polyethylene glycols, sodium lauryl sulfate and mixtures thereof; and coloring agents. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made in a suitable machine in which a mixture of the powdered compound is moistened with an inert liquid diluent.

The tablets and other solid dosage forms of the pharmaceutical compositions of the present disclosure, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter or by incorporating sterilizing agents in the form of sterile solid compositions that can be dissolved in sterile water or some other sterile injectable medium immediately before use. These compositions may also optionally contain opacifying agents and may be of a composition that they release the active ingredient(s) only or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds of Formula (I), or a pharmaceutically acceptable salt thereof, include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth and mixtures thereof.

Pharmaceutical compositions of the disclosure for rectal or vaginal administration may be presented as a suppository, which may be prepared by mixing one or more compounds of the disclosure with one or more suitable nonirritating excipients or carriers comprising, for example, cocoa butter, polyethylene glycol, a suppository wax or a salicylate and which is solid at room temperature, but liquid at body temperature and, therefore, will melt in the rectum or vaginal cavity and release the active compound.

Dosage forms for the topical or transdermal administration of a compound of this disclosure include powders, sprays, ointments, pastes, creams, lotions, gels, solutions, patches and inhalants. The active compound (i.e., a compound of Formula (I) or a pharmaceutically acceptable salt thereof) may be mixed under sterile conditions with a pharmaceutically-acceptable carrier and with any preservatives, buffers or propellants which may be required.

The ointments, pastes, creams, and gels may contain, in addition to a compound of Formula (I), or a pharmaceutically acceptable salt thereof, excipients such as animal and vegetable fats, oils, waxes, paraffins, starch, tragacanth, cellulose derivatives, polyethylene glycols, silicones, bentonites, silicic acid, talc and zinc oxide or mixtures thereof.

Powders and sprays can contain, in addition to a compound of Formula (I), or a pharmaceutically acceptable salt thereof, excipients such as lactose, talc, silicic acid, aluminum hydroxide, calcium silicates and polyamide powder or mixtures of these substances. Sprays can additionally contain customary propellants, such as chlorofluorohydrocarbons and volatile unsubstituted hydrocarbons, such as butane and propane.

Pharmaceutical compositions of this disclosure suitable for parenteral administration comprise one or more compounds of Formula (I), or a pharmaceutically acceptable salt thereof, in combination with one or more pharmaceutically-acceptable sterile isotonic aqueous or nonaqueous solutions, dispersions, suspensions or emulsions or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions of the disclosure include water, ethanol, polyols (such as glycerol, propylene glycol, polyethylene glycol and the like) and suitable mixtures thereof, vegetable oils, such as olive oil and injectable organic esters, such as ethyl oleate. Proper fluidity can be maintained, for example, by the use of coating materials, such as lecithin, by the maintenance of the required particle size in the case of dispersions and by the use of surfactants.

The pharmaceutical compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenyl sorbic acid and the like. It may also be desirable to include isotonic agents, such as sugars, sodium chloride and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions, which are compatible with body tissue.

In some embodiments of the pharmaceutical compositions described herein, the pharmaceutical composition comprises the (S) isomer (i.e., (S) configuration at the carbon atom (*) attached to the -$NH_2$ group of the spirocyclyl ring shown below) of the compound of Formula (I) in high purity.

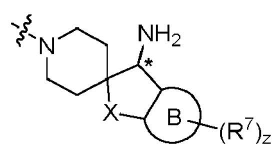

In some embodiments, the pharmaceutical composition comprises the (S) isomer of the compound of Formula (I) in at least about 50%, 60%, 70%, 80%, or 90% of the total amount of the compound of Formula (I). In some embodiments, the pharmaceutical composition comprises the (S) isomer of the compound of Formula (I) in at least about 90%, 91%, 92%, 93%, 94%, 95%, 96%, 97%, 98%, or 99% of the total amount of the compound of Formula (I). In some embodiments, the pharmaceutical composition comprises the (S) isomer of the compound of Formula (I) in at least about 95% of the total amount of the compound of Formula (I). In some embodiments, the pharmaceutical composition comprises the (S) isomer of the compound of Formula (I) as 100% of the total amount of the compound of Formula (I). In some embodiments, the pharmaceutical composition comprises the (R) isomer (i.e., (R) configuration at the carbon atom (*) attached to the -$NH_2$ group of the spirocyclyl ring shown above) of the compound of Formula (I) in small amounts. In some embodiments, the pharmaceutical composition comprises the (R) isomer of the compound of Formula (I) in less than about 50%, 40%, 30%, 20%, or 10% of the total amount of the compound of Formula (I). In some embodiments, the pharmaceutical composition comprises the (R) isomer of the compound of Formula (I) in less than about 10%, 9%, 8%, 7%, 6%, 5%, 4%, 3%, 2%, or 1% of the total amount of the compound of Formula (I). In some embodiments, the pharmaceutical composition comprises the (R) isomer of the compound of Formula (I) in less than about 5% of the total amount of the compound of Formula (I). In some embodiments, the pharmaceutical composition does not comprise the (R) isomer of the compound of Formula (I).

## Methods of Treatment

Compounds of Formula (I), or a pharmaceutically acceptable salt thereof, and pharmaceutical compositions comprising compounds of Formula (I), or a pharmaceutically acceptable salt thereof, may be used in methods of administration and treatment as provided herein. The compounds and pharmaceutical compositions may also be used in *in vitro* methods, such as *in vitro* methods of administering a compound or pharmaceutical composition to cells for screening purposes and/or for conducting quality control assays.

In one aspect, provided herein is a method of modulating SHP2 comprising contacting either an effective amount of a compound described herein, or a pharmaceutically acceptable salt thereof, or an effective amount of a pharmaceutical composition provided herein, with SHP2. Modulation (*e.g.,* inhibition or activation) of SHP2 can be assessed and demonstrated by a wide variety of ways known in the art. Kits and commercially available assays can be utilized for determining whether and to what degree SHP2 has been modulated (*e.g.,* inhibited or activated). In certain embodiments, the compounds of the present disclosure are allosteric modulators of SHP2.

In some embodiments, provided herein is a method of inhibiting SHP2 comprising contacting either an effective amount of a compound described herein, or a pharmaceutically acceptable salt thereof, or an effective amount of a pharmaceutical composition provided herein, with SHP2. In certain embodiments, the compounds of the present disclosure are allosteric inhibitors of SHP2.

In some embodiments, a compound of Formula (I), or a pharmaceutically acceptable salt thereof, modulates the activity of SHP2 by about 1%, 5%, 10%, 15%, 20%, 25%, 30%, 35%, 40%, 45%, 50%, 55%, 60%, 65%, 70%, 75%, 80%, 85%, 90%, 95%, or 100%. In some embodiments, a compound of formula (I) modulates the activity of SHP2 by about 1-100%, 5-100%, 10-100%, 15-100%, 20-100%, 25-100%, 30-100%, 35-100%, 40-100%, 45-100%, 50-100%, 55-100%, 60-100%, 65-100%, 70-100%, 75-100%, 80-100%, 85-100%, 90-100%, 95-100%, 5-95%, 5-90%, 5-85%, 5-80%, 5-75%, 5-70%, 5-65%, 5-60%, 5-55%, 5-50%, 5-45%, 5-40%, 5-35%, 5-30%, 5-25%, 5-20%, 5-15%, 5-10%, 10-90%, 20-80%, 30-70%, or 40-60%.

In some embodiments, a compound of Formula (I), or a pharmaceutically acceptable salt thereof, inhibits the activity of SHP2 by about 1%, 5%, 10%, 15%, 20%, 25%, 30%, 35%, 40%, 45%, 50%, 55%, 60%, 65%, 70%, 75%, 80%, 85%, 90%, 95%, or 100%. In some embodiments, a compound of formula (I) inhibits the activity of SHP2 by about 1-100%, 5-100%, 10-100%, 15-100%, 20-100%, 25-100%, 30-100%, 35-100%, 40-100%, 45-100%, 50-100%, 55-100%, 60-100%, 65-100%, 70-100%, 75-100%, 80-100%, 85-100%, 90-100%, 95-100%, 5-95%, 5-90%, 5-85%, 5-80%, 5-75%, 5-70%, 5-65%, 5-60%, 5-55%, 5-50%, 5-45%, 5-40%, 5-35%, 5-30%, 5-25%, 5-20%, 5-15%, 5-10%, 10-90%, 20-80%, 30-70%, or 40-60%.

In some embodiments, a compound of Formula (I), or a pharmaceutically acceptable salt thereof, permeates the blood brain barrier. Accordingly, in some embodiments, compounds of Formula (I) are useful in treating a disease associated with SHP2 modulation in a subject in need thereof, wherein the disease is a disease of the brain, such as brain cancer.

In another aspect, provided herein is a method for treating a disease associated with SHP2 modulation in a subject in need thereof, comprising administering to the subject an effective amount of a compound of Formula (I) or a pharmaceutically acceptable salt thereof. In some embodiments, provided herein is a method for preventing a disease associated with SHP2 modulation in a subject in need thereof, comprising administering to the subject an effective amount of a compound of Formula (I). Non-limiting examples of a disease associated with SHP2 modulation include Noonan Syndrome, Leopard Syndrome, juvenile myelomonocytic leukemias, neuroblastoma, melanoma, acute myeloid leukemia, breast cancer, lung cancer, colon cancer, or brain cancer. In some embodiments, the brain cancer is glioblastoma. In some embodiments, the disease associated with SHP2 modulation is a genetic disorder. In some embodiments, the disease associated with SHP2 modulation is Noonan Syndrome. In some embodiments, the disease associated with SHP2 modulation is Leopard Syndrome. In some embodiments, the disease associated with SHP2 modulation is a cancer. In some embodiments, the disease associated with SHP2 modulation is juvenile myelomonocytic leukemias. In some embodiments, the disease associated with SHP2 modulation is neuroblastoma. In some embodiments, the disease associated with SHP2 modulation is melanoma. In some embodiments, the disease associated with SHP2 modulation is acute myeloid leukemia. In some embodiments, the disease associated with SHP2 modulation is breast cancer. In some embodiments, the disease associated with SHP2 modulation is lung cancer. In some embodiments, the disease associated with SHP2 modulation is colon cancer. In some embodiments, the disease associated with SHP2 modulation is brain cancer, such as glioblastoma.

In some embodiments, the disease associated with SHP2 modulation includes brain metastases or glioblastomas that are EGFR/RAS pathway dependent. In some variations, brain metastases may develop in non-small cell lung cancer (NSCLC) patients treated with EGFR inhibitors. In some embodiments, treatment of brain metastases or glioblastomas that are EGFR/RAS pathway dependent comprises administering a compound of Formula (I), or a pharmaceutically acceptable salt thereof, in combination with an ALK inhibitor to the subject.

Additional non-limiting examples of a disease associated with SHP2 modulation include the following: hemopoietic cancer; lymphoid system; a myeloproliferative syndrome; a myelodysplastic syndrome; leukemia; acute myeloid leukemia; juvenile myelomonocytic leukemia; esophageal cancer; breast cancer; lung cancer; colon cancer; gastric cancer; neuroblastoma; bladder cancer; prostate cancer; glioblastoma; urothelial carcinoma; uterine carcinoma; adenoid and ovarian sereous cystadenocarcinoma; paraganglioma; phaeochromocytoma; pancreatic cancer; adrenocortical carcinoma; stomach adenocarcinoma; sarcoma; rhabdomyosarcoma; lymphoma; head and neck cancer; skin cancer; peritoneum cancer; intestinal cancer (small and large intestine); thyroid cancer; endometrial cancer, cancer of the biliary tract; soft tissue cancer; ovarian cancer; central nervous system cancer (e.g. primary CNS lymphoma); stomach cancer; pituitary cancer; genital tract cancer; urinary tract cancer; salivary gland cancer; cervical cancer; liver cancer; eye cancer; cancer of the adrenal gland; cancer of autonomic ganglia; cancer of the upper aerodigestive tract; bone cancer; testicular cancer; pleura cancer; kidney cancer; penis cancer; parathyroid cancer; cancer of the meninges; vulvar cancer and

melanoma.

In some embodiments, the disease associated with SHP2 modulation is a cancer selected from the following: lung (e.g., NSCLC), colon, esophageal, rectal, breast, melanoma, pancreatic, Juvenile myelomonocytic leukemia, and Schwannoma. In some embodiments, the disease associated with SHP2 modulation is uterine cancer, endometrial cancer, or ovarian cancer.

In some embodiments, the disease associated with SHP2 modulation is a Ras mutation-driven cancer (e.g., KRAS G12C, KRAS G12D, or KRAS G12V).

In some embodiments, the disease associated with SHP2 modulation is a cancer selected from the following: EGFR-mutant non-small cell lung cancer, KRAS mutant non-small cell lung cancer (NSCLC), head and neck squamous cell lung cancer, melanoma, gastrointestinal stromal tumors, colorectal cancer, a medullary thyroid cancer, and ALK-rearranged NSCLC. In some embodiments, the disease associated with SHP2 modulation is a cancer selected from the following: epithelial cancer (e.g., respiratory cancer, gastrointestinal cancer, genital cancer, cancer of the secretory system, breast cancer), mesothelioma, sarcoma, a hematopoietic tumor, retinoblastoma, or tumors of the central nervous system or of the peripheral nervous system.

In some embodiments, administering a compound of Formula (I), or a pharmaceutically acceptable salt thereof, to a subject in need thereof diminishes the extent of a disease associated with SHP2 modulation (for example, tumor size, tumor growth rate, metastasis) in the subject. In some embodiments, administering a compound of Formula (I), or a pharmaceutically acceptable salt thereof, to a subject in need thereof stabilizes a disease associated with SHP2 modulation (for example, prevents or delays the worsening of a cancer). In some embodiments, administering a compound of Formula (I), or a pharmaceutically acceptable salt thereof, to a subject in need thereof delays the occurrence or recurrence of a disease associated with SHP2 modulation. In some embodiments, administering a compound of Formula (I), or a pharmaceutically acceptable salt thereof, to a subject in need thereof slows the progression of a disease associated with SHP2 modulation. In some embodiments, administering a compound of Formula (I), or a pharmaceutically acceptable salt thereof, to a subject in need thereof provides a partial remission of a disease associated with SHP2 modulation (such as a cancer). In some embodiments, administering a compound of Formula (I), or a pharmaceutically acceptable salt thereof, to a subject in need thereof provides a total remission of a disease associated with SHP2 modulation (such as a cancer). In some embodiments, administering a compound of Formula (I), or a pharmaceutically acceptable salt thereof, to a subject in need thereof decreases the dose of one or more other medications required to treat a disease associated with SHP2 modulation (such as a cancer). In some embodiments, administering a compound of Formula (I), or a pharmaceutically acceptable salt thereof, to a subject in need thereof enhances the effect of another medication used to treat a disease associated with SHP2 modulation (such as a cancer). In some embodiments, administering a compound of Formula (I), or a pharmaceutically acceptable salt thereof, to a subject in need thereof delays the progression of a disease associated with SHP2 modulation (such as a cancer). In some embodiments, administering a compound of Formula (I), or a pharmaceutically acceptable salt thereof, to a subject in need thereof increases the quality of life of the subject having a disease associated with SHP2 modulation (such as a cancer). In some embodiments, administering a compound of Formula (I), or a pharmaceutically acceptable salt thereof, to a subject in need thereof prolongs survival of a subject having a disease associated with SHP2 modulation (such as a cancer).

In some aspects, provided herein is a method of slowing progression of a disease associated with SHP2 modulation (such as a cancer) in a subject, the method comprising administering a compound of Formula (I), or a pharmaceutically acceptable salt thereof, to the subject. In some embodiments, provided herein is a method of stabilizing a disease associated with SHP2 modulation (such as a cancer) in a subject, the method comprising administering a compound of Formula (I), or a pharmaceutically acceptable salt thereof, to the subject. In some embodiments, the method prevents the progression of a disease associated with SHP2 modulation (such as a cancer). In some embodiments, the method delays the progression of a disease associated with SHP2 modulation (such as a cancer). In some embodiments, the method provides a partial or total remission of a disease associated with SHP2 modulation (such as a cancer).

In another aspect, provided herein is a method of delaying the occurrence or recurrence of a disease associated with SHP2 modulation (such as a cancer) in a subject, the method comprising administering a compound of Formula (I), or a pharmaceutically acceptable salt thereof, to the subject.

In further aspects, provided herein is a method of decreasing the dose of one or more other medications required to treat a disease associated with SHP2 modulation (such as a cancer) in a subject, the method comprising administering a compound of Formula (I), or a pharmaceutically acceptable salt thereof, to the subject. In some embodiments, provided herein is a method of enhancing the effect of another medication used to treat a disease associated with SHP2 modulation (such as a cancer) in a subject, the method comprising administering a compound of Formula (I), or a pharmaceutically acceptable salt thereof, to the subject.

Also provided here is a method of delaying the progression of a disease associated with SHP2 modulation (such as a cancer) in a subject, the method comprising administering a compound of Formula (I), or a pharmaceutically acceptable salt thereof, to the subject. In some embodiments, the method increases the quality of life of the subject having a disease associated with SHP2 modulation (such as a cancer). In some embodiments, the method prolongs

survival of the subject having a disease associated with SHP2 modulation (such as a cancer).

In some aspects, provided herein is a compound of Formula (I), or a pharmaceutically acceptable salt thereof, for use in treating a disease associated with SHP2 modulation. In other aspects, provided herein is a compound of Formula (I), or a pharmaceutically acceptable salt thereof, for use in the manufacture of medicament for treating a disease associated with SHP2 modulation.

*Dosing and Method of Administration*

The phrases "parenteral administration" and "administered parenterally" as used herein mean modes of administration other than enteral and topical administration, usually by injection and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracistemally and topically, as by powders, ointments or drops, including buccally and sublingually.

Regardless of the route of administration selected, the compounds of the present disclosure, or the pharmaceutical compositions of the present disclosure, are formulated into pharmaceutically acceptable dosage forms by conventional methods known to those of skill in the art.

Actual dosage levels of the active ingredients in the pharmaceutical compositions of this disclosure may be varied so as to obtain an amount of the active ingredient that is effective to achieve the desired therapeutic response for a particular patient, composition and mode of administration, without being toxic to the patient.

The selected dosage level will depend upon a variety of factors including the activity of the particular compound of the present disclosure employed or the ester, salt or amide thereof, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated and like factors well known in the medical arts. A daily, weekly or monthly dosage (or other time interval) can be used.

A physician or veterinarian having ordinary skill in the art can readily determine and prescribe the effective amount of the pharmaceutical composition required. For example, the physician or veterinarian could start doses of the compounds of the disclosure employed in the pharmaceutical composition at levels lower than that required to achieve the desired therapeutic effect and then gradually increasing the dosage until the desired effect is achieved.

In general, a suitable daily dose of a compound of the disclosure will be that amount of the compound that is the lowest dose effective to produce a therapeutic effect (e.g., inhibit necrosis). Such an effective dose will generally depend upon the factors described above. Generally, doses of the compounds of this disclosure for a patient, when used for the indicated effects, will range from about 0.0001 to about 100 mg per kg of body weight per day. Preferably the daily dosage will range from 0.001 to 50 mg of compound per kg of body weight and even more preferably from 0.01 to 10 mg of compound per kg of body weight.

If desired, the effective daily dose of the active compound may be administered as two, three, four, five, six or more sub-doses administered separately at appropriate intervals throughout the day, optionally, in unit dosage forms.

In certain embodiments, the present disclosure relates to compounds, or pharmaceutically acceptable salts thereof, for modulating SHP2, wherein the compounds are represented by Formula (I). In certain embodiments, the compounds of the present disclosure are allosteric modulators of SHP2. In any event, the compounds of the present disclosure preferably exert their effect on modulating SHP2 at a concentration less than about 50 micromolar, such as less than about 10 micromolar or less than 1 micromolar.

When the compounds of the present disclosure are administered as pharmaceuticals, to humans and animals, they can be given per se or as a pharmaceutical composition containing, for example, 0.1% to 99.5% (such as 0.5% to 90%) of active ingredient in combination with a pharmaceutically acceptable carrier.

The compounds of the present application or the pharmaceutical compositions thereof may be administered once, twice, three, or four times daily, using any suitable mode described above. Also, administration or treatment with the compounds may be continued for a number of days; for example, commonly treatment would continue for at least 7 days, 14 days, or 28 days, for one cycle of treatment. Treatment cycles are well known and are frequently alternated with resting periods of about 1 to 28 days, commonly about 7 days or about 14 days, between cycles. The treatment cycles, in certain embodiments, may also be continuous.

When administered orally, the total daily dosage for a human subject may be between about 1 mg and 1,000

mg, between about 1,000-2,000 mg/day, between about 10-500 mg/day, between about 50-300 mg/day, between about 75-200 mg/day or between about 100-150 mg/day.

The daily dosage may also be described as a total amount of a compound described herein administered per dose or per day. Daily dosage of a compound may be between about 1 mg and 4,000 mg, between about 2,000 to 4,000 mg/day, between about 1 to 2,000 mg/day, between about 1 to 1,000 mg/day, between about 10 to 500 mg/day, between about 20 to 500 mg/day, between about 50 to 300 mg/day, between about 75 to 200 mg/day or between about 15 to 150 mg/day.

In certain embodiments, the method comprises administering to the subject an initial daily dose of about 1 to 800 mg of a compound described herein and increasing the dose by increments until clinical efficacy is achieved. Increments of about 5, 10, 25, 50 or 100 mg can be used to increase the dose. The dosage can be increased daily, every other day, twice per week or once per week.

In certain embodiments, a compound or pharmaceutical preparation is administered orally. In certain embodiments, the compound or pharmaceutical preparation is administered intravenously. Alternative routes of administration include sublingual, intramuscular and transdermal administrations.

The preparations of the present disclosure may be given orally, parenterally, topically, or rectally. They are, of course, given in forms suitable for each administration route. For example, they are administered in tablets or capsule form; by injection, inhalation, eye lotion, ointment, suppository, infusion, inhalation, etc.; topical by lotion or ointment; and rectal by suppositories. In certain embodiments, the administration is oral.

## Combination Therapy

The methods of the present disclosure may include a compound of Formula (I), or a pharmaceutically acceptable salt thereof, used alone or in combination with one or more additional therapies (e.g., non-drug treatments or therapeutic agents). The dosages of one or more of the additional therapies (e.g., non-drug treatments or therapeutic agents) may be reduced from standard dosages when administered alone. For example, doses may be determined empirically from drug combinations and permutations or may be deduced by isobolographic analysis (e.g., Black et al., Neurology 65:S3-S6 (2005)).

A compound of Formula (I), or a pharmaceutically acceptable salt thereof, may be administered before, after, or concurrently with one or more of such additional therapies. When combined, dosages of the compound of Formula (I), or a pharmaceutically acceptable salt thereof, and dosages of the one or more additional therapies (e.g., non-drug treatment or therapeutic agent) provide a therapeutic effect (e.g., synergistic or additive therapeutic effect). A compound of Formula (I), or a pharmaceutically acceptable salt thereof, and an additional therapy, such as an anti-cancer agent, may be administered together, such as in a unitary pharmaceutical composition, or separately and, when administered separately, this may occur simultaneously or sequentially. Such sequential administration may be close or remote in time.

In some embodiments, the additional therapy is the administration of side-effect limiting agents (e.g., agents intended to lessen the occurrence or severity of side effects of treatment). For example, in some embodiments, the compounds of Formula (I), or a pharmaceutically acceptable salt thereof, can also be used in combination with a therapeutic agent that treats nausea. Examples of agents that can be used to treat nausea include: dronabinol, granisetron, metoclopramide, ondansetron, and prochlorperazine, or pharmaceutically acceptable salts thereof.

In some embodiments, the one or more additional therapies includes a non-drug treatment (e.g., surgery or radiation therapy). In some embodiments, the one or more additional therapies includes a therapeutic agent (e.g., a compound or biologic that is an anti-angiogenic agent, signal transduction inhibitor, antiproliferative agent, glycolysis inhibitor, or autophagy inhibitor). In some embodiments, the one or more additional therapies includes a non-drug treatment (e.g., surgery or radiation therapy) and a therapeutic agent (e.g., a compound or biologic that is an anti-angiogenic agent, signal transduction inhibitor, antiproliferative agent, glycolysis inhibitor, or autophagy inhibitor). In other embodiments, the one or more additional therapies includes two therapeutic agents. In still other embodiments, the one or more additional therapies includes three therapeutic agents. In some embodiments, the one or more additional therapies includes four or more therapeutic agents.

In this Combination Therapy section, all references are incorporated by reference for the agents described, or a pharmaceutically acceptable salt, solvate, isomer (e.g., stereoisomer), prodrug, or tautomer thereof, whether explicitly stated as such or not.

### *Non-drug therapies*

Examples of non-drug treatments include, but are not limited to, radiation therapy, cryotherapy, hyperthermia, surgery (e.g., surgical excision of tumor tissue), and T cell adoptive transfer (ACT) therapy.

In some embodiments, the compounds of Formula (I), or a pharmaceutically acceptable salt thereof, may be used as an adjuvant therapy after surgery. In some embodiments, the compounds of Formula (I), or a pharmaceutically

acceptable salt thereof, may be used as a neo-adjuvant therapy prior to surgery.

Radiation therapy may be used for inhibiting abnormal cell growth or treating a hyperproliferative disorder, such as cancer, in a subject (e.g., a mammal, such as a human). Techniques for administering radiation therapy are known in the art. Radiation therapy can be administered through one of several methods, or a combination of methods, including, without limitation, external-beam therapy, internal radiation therapy, implant radiation, stereotactic radiosurgery, systemic radiation therapy, radiotherapy and permanent or temporary interstitial brachy therapy. The term "brachy therapy," as used herein, refers to radiation therapy delivered by a spatially confined radioactive material inserted into the body at or near a tumor or other proliferative tissue disease site. The term is intended, without limitation, to include exposure to radioactive isotopes (e.g., At-211, 1-131, 1-125, Y-90, Re-186, Re-188, Sm-153, Bi-212, P-32, and radioactive isotopes of Lu). Suitable radiation sources for use as a cell conditioner of the present disclosure include both solids and liquids. By way of non-limiting example, the radiation source can be a radionuclide, such as I-125, I-131, Yb-169, Ir-192 as a solid source, 1-125 as a solid source, or other radionuclides that emit photons, beta particles, gamma radiation, or other therapeutic rays. The radioactive material can also be a fluid made from any solution of radionuclide(s), e.g., a solution of I-125 or 1-131, or a radioactive fluid can be produced using a slurry of a suitable fluid containing small particles of solid radionuclides, such as Au-198, or Y-90. Moreover, the radionuclide(s) can be embodied in a gel or radioactive micro spheres.

In some embodiments, the compounds of Formula (I), or a pharmaceutically acceptable salt thereof, can render abnormal cells more sensitive to treatment with radiation for purposes of killing or inhibiting the growth of such cells. Accordingly, the present disclosure further relates to a method for sensitizing abnormal cells in a mammal to treatment with radiation which comprises administering to the mammal an amount of a compound of Formula (I), or a pharmaceutically acceptable salt thereof, which amount is effective to sensitize abnormal cells to treatment with radiation. The amount of the compound in this method can be determined according to the means for ascertaining effective amounts of such compounds described herein. In some embodiments, the compounds of Formula (I), or a pharmaceutically acceptable salt thereof, may be used as an adjuvant therapy after radiation therapy or as a neo-adjuvant therapy prior to radiation therapy.

In some embodiments, the non-drug treatment is a T cell adoptive transfer (ACT) therapy. In some embodiments, the T cell is an activated T cell. The T cell may be modified to express a chimeric antigen receptor (CAR). CAR modified T (CAR-T) cells can be generated by any method known in the art. For example, the CAR-T cells can be generated by introducing a suitable expression vector encoding the CAR to a T cell. Prior to expansion and genetic modification of the T cells, a source of T cells is obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present disclosure, any number of T cell lines available in the art may be used. In some embodiments, the T cell is an autologous T cell. Whether prior to or after genetic modification of the T cells to express a desirable protein (e.g., a CAR), the T cells can be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 7,572,631; 5,883,223; 6,905,874; 6,797,514; and 6,867,041.

*Therapeutic agents*

A therapeutic agent may be a compound used in the treatment of cancer or symptoms associated therewith.

For example, a therapeutic agent may be a steroid. Accordingly, in some embodiments, the one or more additional therapies includes a steroid. Suitable steroids may include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, fiucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts or derivatives thereof.

Further examples of therapeutic agents that may be used in combination therapy with a compound of Formula (I), or a pharmaceutically acceptable salt thereof, include compounds described in the following patents: U.S. Patent Nos. 6,258,812, 6,630,500, 6,515,004, 6,713,485, 5,521,184, 5,770,599, 5,747,498, 5,990,141, 6,235,764, and 8,623,885, and International Patent Applications WO0 1/37820, WO01/32651, WO02/68406, WO02/66470, WO02/55501, WO04/05279, WO04/07481, WO04/07458, WO04/09784, WO02/59110, WO99/45009, WO00/59509, WO99/61422, WO00/12089, and WO00/02871.

A therapeutic agent may be a biologic (e.g., cytokine (e.g., interferon or an interleukin such as IL-2)) used in treatment of cancer or symptoms associated therewith. In some embodiments, the biologic is an immunoglobulin-based biologic, e.g., a monoclonal antibody (e.g., a humanized antibody, a fully human antibody, an Fc fusion protein, or a functional fragment thereof) that agonizes a target to stimulate an anti-cancer response or antagonizes an antigen important for cancer. Also included are antibody-drug conjugates.

A therapeutic agent may be a T-cell checkpoint inhibitor. In one embodiment, the checkpoint inhibitor is an inhibitory antibody (e.g., a monospecific antibody such as a monoclonal antibody). The antibody may be, e.g., humanized or fully human. In some embodiments, the checkpoint inhibitor is a fusion protein, e.g., an Fc-receptor fusion protein. In some embodiments, the checkpoint inhibitor is an agent, such as an antibody, that interacts with a checkpoint protein. In some embodiments, the checkpoint inhibitor is an agent, such as an antibody, that interacts with the ligand of a checkpoint protein. In some embodiments, the checkpoint inhibitor is an inhibitor (e.g., an inhibitory antibody or small molecule inhibitor) of CTLA-4 (e.g., an anti-CTLA-4 antibody or fusion a protein). In some embodiments, the checkpoint inhibitor is an inhibitor or antagonist (e.g., an inhibitory antibody or small molecule inhibitor) of PD-1. In some embodiments, the checkpoint inhibitor is an inhibitor or antagonist (e.g., an inhibitory antibody or small molecule inhibitor) of PD-L1. In some embodiments, the checkpoint inhibitor is an inhibitor or antagonist (e.g., an inhibitory antibody or Fc fusion or small molecule inhibitor) of PD-L2 (e.g., a PD-L2/Ig fusion protein). In some embodiments, the checkpoint inhibitor is an inhibitor or antagonist (e.g., an inhibitory antibody or small molecule inhibitor) of B7-H3, B7-H4, BTLA, HVEM, TIM3, GAL9, LAG3, VISTA, KIR, 2B4, CD160, CGEN-15049, CHK 1, CHK2, A2aR, B-7 family ligands, or a combination thereof. In some embodiments, the checkpoint inhibitor is pembrolizumab, nivolumab, PDR001 (NVS), REGN2810 (Sanofi/Regeneron), a PD-L1 antibody such as, e.g., avelumab, durvalumab, atezolizumab, pidilizumab, JNJ-63723283 (JNJ), BGB-A317 (BeiGene & Celgene) or a checkpoint inhibitor disclosed in Preusser, M. et al. (2015) Nat. Rev. Neurol., including, without limitation, ipilimumab, tremelimumab, nivolumab, pembrolizumab, AMP224, AMP514/ MEDI0680, BMS936559, MED14736, MPDL3280A, MSB0010718C, BMS986016, IMP321, lirilumab, IPH2101, 1-7F9, and KW-6002.

A therapeutic agent may be an anti-TIGIT antibody, such as MBSA43, BMS-986207, MK-7684, COM902, AB154, MTIG7192A or OMP-313M32 (etigilimab).

A therapeutic agent may be an agent that treats cancer or symptoms associated therewith (e.g., a cytotoxic agent, non-peptide small molecules, or other compound useful in the treatment of cancer or symptoms associated therewith, collectively, an "anti-cancer agent"). Anti-cancer agents can be, e.g., chemotherapeutics or targeted therapy agents.

Anti-cancer agents include mitotic inhibitors, intercalating antibiotics, growth factor inhibitors, cell cycle inhibitors, enzymes, topoisomerase inhibitors, biological response modifiers, alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Further anti-cancer agents include leucovorin (LV), irenotecan, oxaliplatin, capecitabine, paclitaxel, and doxetaxel. In some embodiments, the one or more additional therapies includes two or more anti-cancer agents. The two or more anti-cancer agents can be used in a cocktail to be administered in combination or administered separately. Suitable dosing regimens of combination anti-cancer agents are known in the art and described in, for example, Saltz et al., Proc. Am. Soc. Clin. Oncol. 18:233a (1999), and Douillard et al., Lancet 355(9209):1041-1047 (2000).

Other non-limiting examples of anti-cancer agents include Gleevec® (Imatinib Mesylate); Kyprolis® (carfilzomib); Velcade® (bortezomib); Casodex™ (bicalutamide); Iressa® (gefitinib); alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; sarcodictyin A; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimustine; antibiotics such as the enediyne antibiotics (e.g., calicheamicin, such as calicheamicin gammall and calicheamicin omegall (see, e.g., Agnew, Chem. Intl. Ed Engl. 33:183-186 (1994)); dynemicin such as dynemicin A; bisphosphonates such as clodronate; an esperamicin; neocarzinostatin chromophore and related chromoprotein enediyne antiobiotic chromophores, aclacinomysins, actinomycin, authramycin, azaserine, bleomycins, cactinomycin, calicheamicin, carabicin, caminomycin, carminomycin, carzinophilin, chromomycins, dactinomycin, daunorubicin, detorubicin, 6-diazo- 5-oxo-L-norleucine, adriamycin (doxorubicin), morpholino-doxorubicin, cyanomorpholino-doxorubicin, 2-pyrrolino-doxorubicin, deoxydoxorubicin, epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenishers such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone such as epothilone B; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes such as T- 2 toxin, verracurin A, roridin A and anguidine; urethane; vindesine; dacarbazine; mannomustine; mitobronitol; mitolactol; pipobroman; gacytosine; arabinoside ("Ara-C"); cyclophosphamide; thiotepa; taxoids, e.g., Taxol® (paclitaxel), Abraxane® (cremophor-free, albumin-engineered nanoparticle formulation of paclitaxel), and Taxotere® (doxetaxel); chloranbucil; tamoxifen (Nolvadex™); raloxifene; aromatase inhibiting 4(5)-imidazoles; 4-hydroxytamoxifen; trioxifene; keoxifene; LY 117018; onapristone; toremifene (Fareston®); flutamide, nilutamide, bicalutamide, leuprolide, goserelin; chlorambucil; Gemzar® gemcitabine; 6-thioguanine; mercaptopurine; platinum coordination complexes such as cisplatin, oxaliplatin and carboplatin; vinblastine; platinum; etoposide (VP-16); ifosfamide; mitoxantrone; vincristine; Navelbine® (vinorelbine); novantrone; teniposide; edatrexate; daunomycin; aminopterin; ibandronate; irinotecan (e.g., CPT-11); topoisomerase inhibitor RFS 2000; difluoromethylornithine (DMFO); retinoids such as retinoic acid; esperamicins; capecitabine (e.g., Xeloda®); and pharmaceutically acceptable salts of any of the above.

Additional non-limiting examples of anti-cancer agents include trastuzumab (Herceptin®), bevacizumab (Avastin®), cetuximab (Erbitux®), rituximab (Rituxan®), Taxol®, Arimidex®, ABVD, avicine, abagovomab, acridine carboxamide, adecatumumab, 17-N-allylamino-17-demethoxygeldanamycin, alpharadin, alvocidib, 3-aminopyridine-2-carboxaldehyde thiosemicarbazone, amonafide, anthracenedione, anti-CD22 immunotoxins, antineoplastics (e.g., cell-cycle nonspecific antineoplastic agents, and other antineoplastics described herein), antitumorigenic herbs, apaziquone, atiprimod, azathioprine, belotecan, bendamustine, BIBW 2992, biricodar, brostallicin, bryostatin, buthionine sulfoximine, CBV (chemotherapy), calyculin, dichloroacetic acid, discodermolide, elsamitrucin, enocitabine, eribulin, exatecan, exisulind, ferruginol, forodesine, fosfestrol, ICE chemotherapy regimen, IT-101, imexon, imiquimod, indolocarbazole, irofulven, laniquidar, larotaxel, lenalidomide, lucanthone, lurtotecan, mafosfamide, mitozolomide, nafoxidine, nedaplatin, olaparib, ortataxel, PAC-1, pawpaw, pixantrone, proteasome inhibitors, rebeccamycin, resiquimod, rubitecan, SN-38, salinosporamide A, sapacitabine, Stanford V, swainsonine, talaporfin, tariquidar, tegafururacil, temodar, tesetaxel, triplatin tetranitrate, tris(2-chloroethyl)amine, troxacitabine, uramustine, vadimezan, vinflunine, ZD6126, and zosuquidar.

Further non-limiting examples of anti-cancer agents include natural products such as vinca alkaloids (e.g., vinblastine, vincristine, and vinorelbine), epidipodophyllotoxins (e.g., etoposide and teniposide), antibiotics (e.g., dactinomycin (actinomycin D), daunorubicin, and idarubicin), anthracyclines, mitoxantrone, bleomycins, plicamycin (mithramycin), mitomycin, enzymes (e.g., L-asparaginase which systemically metabolizes L-asparagine and deprives cells which do not have the capacity to synthesize their own asparagine), antiplatelet agents, antiproliferative/antimitotic alkylating agents such as nitrogen mustards (e.g., mechlorethamine, cyclophosphamide and analogs, melphalan, and chlorambucil), ethylenimines and methylmelamines (e.g., hexaamethylmelaamine and thiotepa), CDK inhibitors (e.g., a CDK4/6 inhibitor such as abemaciclib, ribociclib, palbociclib; seliciclib, UCN-01, P1446A-05, PD-0332991, dinaciclib, P27-00, AT-7519, RGB286638, and SCH727965), alkyl sulfonates (e.g., busulfan), nitrosoureas (e.g., carmustine (BCNU) and analogs, and streptozocin), trazenes-dacarbazinine (DTIC), antiproliferative/antimitotic antimetabolites such as folic acid analogs, pyrimidine analogs (e.g., fluorouracil, floxuridine, and cytarabine), purine analogs and related inhibitors (e.g., mercaptopurine, thioguanine, pentostatin, and 2-chlorodeoxyadenosine), aromatase inhibitors (e.g., anastrozole, exemestane, and letrozole), and platinum coordination complexes (e.g., cisplatin and carboplatin), procarbazine, hydroxyurea, mitotane, aminoglutethimide, histone deacetylase (HDAC) inhibitors (e.g., trichostatin, sodium butyrate, apicidan, suberoyl anilide hydroamic acid, vorinostat, LBH 589, romidepsin, ACY-1215, and panobinostat), mTOR inhibitors (e.g., vistusertib, temsirolimus, everolimus, ridaforolimus, and sirolimus), KSP(Eg5) inhibitors (e.g., Array 520), DNA binding agents (e.g., Zalypsis®), PI3K inhibitors such as PI3K delta inhibitor (e.g., GS-1101 and TGR-1202), PI3K delta and gamma inhibitor (e.g., CAL-130), copanlisib, alpelisib and idelalisib; multi-kinase inhibitor (e.g., TG02 and sorafenib), hormones (e.g., estrogen) and hormone agonists such as leutinizing hormone releasing hormone (LHRH) agonists (e.g., goserelin, leuprolide and triptorelin), BAFF-neutralizing antibody (e.g., LY2127399), IKK inhibitors, p38MAPK inhibitors, anti-IL-6 (e.g., CNT0328), telomerase inhibitors (e.g., GRN 163L), aurora kinase inhibitors (e.g., MLN8237), cell surface monoclonal antibodies (e.g., anti-CD38 (HUMAX-CD38), anti-CSI (e.g., elotuzumab), HSP90 inhibitors (e.g., 17 AAG and KOS 953), P13K / Akt inhibitors (e.g., perifosine), Akt inhibitors (e.g., GSK-2141795), PKC inhibitors (e.g., enzastaurin), FTIs (e.g., Zarnestra™), anti-CD138 (e.g., BT062), Torcl/2 specific kinase inhibitors (e.g.,

INK128), ER/UPR targeting agents (e.g., MKC-3946), cFMS inhibitors (e.g., ARRY-382), JAK1/2 inhibitors (e.g., CYT387), PARP inhibitors (e.g., olaparib and veliparib (ABT-888)), and BCL-2 antagonists.

In some embodiments, an anti-cancer agent is selected from mechlorethamine, camptothecin, ifosfamide, tamoxifen, raloxifene, gemcitabine, Navelbine®, sorafenib, or any analog or derivative variant of the foregoing.

In some embodiments, the anti-cancer agent is a HER2 inhibitor. Non-limiting examples of HER2 inhibitors include monoclonal antibodies such as trastuzumab (Herceptin®) and pertuzumab (Peijeta®); small molecule tyrosine kinase inhibitors such as gefitinib (Iressa®), erlotinib (Tarceva®), pilitinib, CP-654577, CP-724714, canertinib (CI 1033), HKI-272, lapatinib (GW-572016; Tykerb®), PKI-166, AEE788, BMS-599626, HKI-357, BIBW 2992, ARRY-334543, and JNJ-26483327.

In some embodiments, an anti-cancer agent is an ALK inhibitor. Non-limiting examples of ALK inhibitors include ceritinib, TAE-684 (NVP-TAE694), PF02341066 (crizotinib or 1066), alectinib; brigatinib; entrectinib; ensartinib (X-396); lorlatinib; ASP3026; CEP-37440; 4SC-203; TL-398; PLB1003; TSR-011; CT-707; TPX-0005, and AP26113. Additional examples of ALK kinase inhibitors are described in examples 3-39 of WO 05016894.

In some embodiments, an anti-cancer agent is an inhibitor of a member downstream of a Receptor Tyrosine Kinase (RTK)/Growth Factor Receptor (e.g., a SOS1 inhibitor (e.g., BI-1701963, BI-3406, SDR5, BAY-293, or RMC-5845, or a pharmaceutically acceptable salt, solvate, isomer (e.g., stereoisomer), prodrug, or tautomer thereof), a Raf inhibitor, a MEK inhibitor, an ERK inhibitor, a PI3K inhibitor, a PTEN inhibitor, an AKT inhibitor, or an mTOR inhibitor (e.g., mTORC1 inhibitor or mTORC2 inhibitor). In some embodiments, the anti-cancer agent is JAB-3312.

In some embodiments, an anti-cancer agent is a SOS1 inhibitor. In some embodiments, the SOS1 inhibitor is selected from those disclosed in WO 2021173524, WO 2021130731, WO 2021127429, WO 2021092115, WO 2021105960, WO 2021074227, WO 2020180768, WO 2020180770, WO 2020173935, WO 2020146470, WO 2019201848, WO 2019122129, WO 2018172250, and WO 2018115380, or a pharmaceutically acceptable salt, solvate, isomer (e.g., stereoisomer), prodrug, or tautomer thereof.

In some embodiments, an anti-cancer agent is an additional Ras inhibitor or a Ras vaccine, or another therapeutic modality designed to directly or indirectly decrease the oncogenic activity of Ras. In some embodiments, an anti-cancer agent is an additional Ras inhibitor. In some embodiments, the Ras inhibitor targets Ras in its active, or GTP-bound state. In some embodiments, the Ras inhibitor targets Ras in its inactive, or GDP-bound state. In some embodiments, the Ras inhibitor is, such as an inhibitor of K-Ras G12C, such as AMG 510 (sotorasib), MRTX1257, MRTX849 (adagrasib), JNJ-74699157, LY3499446, ARS-1620, ARS-853, BPI-421286, LY3537982, JDQ443, JAB-21000, RMC-6291, ASP2453, or GDC-6036, or a pharmaceutically acceptable salt, solvate, isomer (e.g., stereoisomer), prodrug, or tautomer thereof. In some embodiments, the Ras inhibitor is an inhibitor of K-Ras G12D, such as MRTX1133 or JAB-22000, or a pharmaceutically acceptable salt, solvate, isomer (e.g., stereoisomer), prodrug, or tautomer thereof. In some embodiments, the Ras inhibitor is a K-Ras G12V inhibitor, such as JAB-23000, or a pharmaceutically acceptable salt, solvate, isomer (e.g., stereoisomer), prodrug, or tautomer thereof. In some embodiments, the Ras inhibitor is RMC-6236, or a pharmaceutically acceptable salt, solvate, isomer (e.g., stereoisomer), prodrug, or tautomer thereof. In some embodiments, the Ras inhibitor is selected from a Ras(ON) inhibitor disclosed in the following, incorporated herein by reference in their entireties, or a pharmaceutically acceptable salt, solvate, isomer (e.g., stereoisomer), prodrug, or tautomer thereof: WO 2021091982, WO 2021091967, WO 2021091956 and WO 2020132597. Other examples of Ras inhibitors that may be combined with a Ras inhibitor of the present disclosure are provided in the following, incorporated herein by reference in their entireties: WO 2021239058, WO 2021236920, WO 2021231526, WO 2021228161, WO 2021222333, WO 2021219091, WO 2021219090, WO 2021219072, WO 2021218939, WO 2021217019, WO 2021216770, WO 2021215545, WO 2021215544, WO 2021211864, WO 2021197499, WO 2021190467, WO 2021185233, WO 2021180181, WO 2021175199, WO 2021173923, WO 2021169990, WO 2021169963, WO 2021168193, WO 2021158071, WO 2021155716, WO 2021152149, WO 2021150613, WO 2021147967, WO 2021147965, WO 2021143693, WO 2021142252, WO 2021141628, WO 2021139748, WO 2021139678, WO 2021129824, WO 2021129820, WO 2021127404, WO 2021126816, WO 2021126799, WO 2021124222, WO 2021121371, WO 2021121367, WO 2021121330, WO 2020050890, WO 2020047192, WO 2020035031, WO 2020028706, WO 2019241157, WO 2019232419, WO 2019217691, WO 2019217307, WO 2019215203, WO 2019213526, WO 2019213516, WO 2019155399, WO 2019150305, WO 2019110751, WO 2019099524, WO 2019051291, WO 2018218070, WO 2018217651, WO 2018218071, WO 2018218069, WO 2018206539, WO 2018143315, WO 2018140600, WO 2018140599, WO 2018140598, WO 2018140514, WO 2018140513, WO 2018140512, WO 2018119183, WO 2018112420, WO 2018068017, WO 2018064510, WO 2017201161, WO 2017172979, WO 2017100546, WO 2017087528, WO 2017058807, WO 2017058805, WO 2017058728, WO 2017058902, WO 2017058792, WO 2017058768, WO 2017058915, WO 2017015562, WO 2016168540, WO 2016164675, WO 2016049568, WO 2016049524, WO 2015054572, WO 2014152588, WO 2014143659, and WO 2013155223, or a pharmaceutically acceptable salt, solvate, isomer (e.g., stereoisomer), prodrug, or tautomer thereof.

In some embodiments, a therapeutic agent that may be combined with a compound of Formula (I), or a pharmaceutically acceptable salt thereof, is an inhibitor of the MAP kinase (MAPK) pathway (or "MAPK inhibitor"). MAPK

inhibitors include, but are not limited to, one or more MAPK inhibitor described in Cancers (Basel) 2015 Sep; 7(3): 1758-1784. For example, the MAPK inhibitor may be selected from one or more of trametinib, binimetinib, selumetinib, cobimetinib, LErafAON (NeoPharm), ISIS 5132; vemurafenib, pimasertib, TAK733, RO4987655 (CH4987655); CI-1040; PD-0325901; CH5126766; MAP855; AZD6244; refametinib (RDEA 119/BAY 86-9766); GDC-0973/XL581; AZD8330 (ARRY-424704/ARRY-704); RO5126766 (Roche, described inPLoS One. 2014 Nov 25;9(11)); and GSK1120212 (or JTP-74057, described in Clin Cancer Res. 2011 Mar 1;17(5):989-1000). The MAPK inhibitor may be PLX8394, LXH254, GDC-5573, or LY3009120.

In some embodiments, an anti-cancer agent is a disrupter or inhibitor of the RAS-RAF-ERK or PI3K-AKT-TOR or PI3K-AKT signaling pathways. The PI3K/AKT inhibitor may include, but is not limited to, one or more PI3K/AKT inhibitor described in Cancers (Basel) 2015 Sep; 7(3): 1758-1784. For example, the PI3K/AKT inhibitor may be selected from one or more of NVP-BEZ235; BGT226; XL765/SAR245409; SF1126; GDC-0980; PI-103; PF-04691502; PKI-587; GSK2126458.

In some embodiments, an anti-cancer agent is a PD-1 or PD-L1 antagonist.

In some embodiments, additional therapeutic agents include ALK inhibitors, HER2 inhibitors, EGFR inhibitors, IGF-1R inhibitors, MEK inhibitors, PI3K inhibitors, AKT inhibitors, TOR inhibitors, MCL-1 inhibitors, BCL-2 inhibitors, proteasome inhibitors, and immune therapies. In some embodiments, a therapeutic agent may be a pan-RTK inhibitor, such as afatinib.

IGF-1R inhibitors include linsitinib, or a pharmaceutically acceptable salt thereof.

EGFR inhibitors include, but are not limited to, small molecule antagonists, antibody inhibitors, or specific antisense nucleotide or siRNA. Useful antibody inhibitors of EGFR include cetuximab (Erbitux®), panitumumab (Vectibix®), zalutumumab, nimotuzumab, and matuzumab. Further antibody-based EGFR inhibitors include any anti-EGFR antibody or antibody fragment that can partially or completely block EGFR activation by its natural ligand. Non-limiting examples of antibody-based EGFR inhibitors include those described in Modjtahedi et al., Br. J. Cancer 1993, 67:247-253; Teramoto et al., Cancer 1996, 77:639-645; Goldstein et al., Clin. Cancer Res. 1995, 1:1311-1318; Huang et al., 1999, Cancer Res. 15:59(8):1935-40; and Yang et al., Cancer Res.1999, 59:1236-1243. The EGFR inhibitor can be monoclonal antibody Mab E7.6.3 (Yang, 1999 supra), or Mab C225 (ATCC Accession No. HB-8508), or an antibody or antibody fragment having the binding specificity thereof.

Small molecule antagonists of EGFR include gefitinib (Iressa®), erlotinib (Tarceva®), and lapatinib (TykerB®). See, e.g., Yan et al., Pharmacogenetics and Pharmacogenomics In Oncology Therapeutic Antibody Development, BioTechniques 2005, 39(4):565-8; and Paez et al., EGFR Mutations In Lung Cancer Correlation With Clinical Response To Gefitinib Therapy, Science 2004, 304(5676):1497-500. In some embodiments, the EGFR inhibitor is osimertinib (Tagrisso®). Further non-limiting examples of small molecule EGFR inhibitors include any of the EGFR inhibitors described in the following patent publications, and all pharmaceutically acceptable salts of such EGFR inhibitors: EP 0520722; EP 0566226; WO96/33980; U.S. Pat. No. 5,747,498; WO96/30347; EP 0787772; WO97/30034; WO97/30044; WO97/38994; WO97/49688; EP 837063; WO98/02434; WO97/38983; WO95/19774; WO95/19970; WO97/13771; WO98/02437; WO98/02438; WO97/32881; DE 19629652; WO98/33798; WO97/32880; WO97/32880; EP 682027; WO97/02266; WO97/27199; WO98/07726; WO97/34895; WO96/31510; WO98/14449; WO98/14450; WO98/14451; WO95/09847; WO97/19065; WO98/17662; U.S. Pat. No. 5,789,427; U.S. Pat. No. 5,650,415; U.S. Pat. No. 5,656,643; WO99/35146; WO99/35132; WO99/07701; and WO92/20642. Additional non-limiting examples of small molecule EGFR inhibitors include any of the EGFR inhibitors described in Traxler et al., Exp. Opin. Ther. Patents 1998, 8(12):1599-1625. In some embodiments, an EGFR inhibitor is an ERBB inhibitor. In humans, the ERBB family contains HER1 (EGFR, ERBB1), HER2 (NEU, ERBB2), HER3 (ERBB3), and HER (ERBB4).

MEK inhibitors include, but are not limited to, pimasertib, selumetinib, cobimetinib (Cotellic®), trametinib (Mekinist®), and binimetinib (Mektovi®). In some embodiments, a MEK inhibitor targets a MEK mutation that is a Class IMEK1 mutation selected from D67N; P124L; P124S; and L177V. In some embodiments, the MEK mutation is a Class IIMEK1 mutation selected from ΔE51-Q58; ΔF53-Q58; E203K; L177M; C121S; F53L; K57E; Q56P; and K57N.

PI3K inhibitors include, but are not limited to, wortmannin; 17-hydroxywortmannin analogs described in WO06/044453; 4-[2-(1H-Indazol-4-yl)-6-[[4-(methylsulfonyl)piperazin-1-yl]methyl]thieno[3,2-d]pyrimidin-4-yl]morpholine (also known as pictilisib or GDC-0941 and described in WO09/036082 and WO09/055730); 2-methyl-2-[4-[3-methyl-2-oxo-8-(quinolin-3-yl)-2,3-dihydroimidazo[4,5-c]quinolin-1-yl]phenyl]propioritrile (also known as BEZ 235 or NVP-BEZ 235, and described in WO06/122806); (S)-1-(4-((2-(2-aminopyrimidin-5-yl)-7-methyl-4-morpholinothieno[3,2-d]pyrimidin-6-yl)methyl)piperazin-l-yl)-2-hydroxypropan-1-one (described in WO08/070740); LY294002 (2-(4-morpholinyl)-8-phenyl-4H-1-benzopyran-4-one (available from Axon Medchem); PI 103 hydrochloride (3-[4-(4-morpholinylpyrido-[3',2':4,5]furo[3,2-d]pyrimidin-2-yl] phenol hydrochloride (available from Axon Medchem); PIK 75 (2-methyl-5-nitro-2-[(6-bromoimidazo[1,2-a]pyridin-3-yl)methylene]-1-methylhydrazide-benzenesulfonic acid, monohydrochloride) (available from Axon Medchem); PIK 90 (N-(7,8-dimethoxy-2,3-dihydro-imidazo[1,2-c]quinazolin-5-yl)-nicotinamide (available from Axon Medchem); AS-252424 (5-[1-[5-(4-fluoro-2-hydroxy-phenyl)-furan-2-yl]-meth-(Z)-ylidene]-thiazolidine-2,4-dione (available from Axon Medchem); TGX-221 (7-methyl-2-(4-morpholinyl)-9-[1-(phenylamino)ethyl]-4H-pyrido-[1,2-a]pyrinidin-4-one (available from Axon Medchem); XL-765; and XL-147. Other PI3K inhibitors include demethoxyviridin, perifosine, CAL101, PX-866, BEZ235, SF1126, INK1117, IPI-145, BKM120, XL147, XL765, Palomid 529, GSK1059615, ZSTK474, PWT33597, IC87114, TGI 00-115, CAL263, PI-103, GNE-477, CUDC-907, and AEZS-136.

AKT inhibitors include, but are not limited to, Akt-1-1 (inhibits Aktl) (Barnett et al., Biochem. J. 2005, 385(Pt. 2): 399-408); Akt-1-1,2 (inhibits Akl and 2) (Barnett et al., Biochem. J. 2005, 385(Pt. 2): 399-408); API-59CJ-Ome (e.g., Jin et al., Br. J. Cancer 2004, 91:1808-12); 1-H-imidazo[4,5-c]pyridinyl compounds (e.g., WO 05/011700); indole-3-carbinol and derivatives thereof (e.g., U.S. Pat. No. 6,656,963; Sarkar and Li J Nutr. 2004, 134(12 Suppl):3493S-3498S); perifosine (e.g., interferes with Akt membrane localization; Dasmahapatra et al. Clin. Cancer Res. 2004, 10(15):5242-52); phosphatidylinositol ether lipid analogues (e.g., Gills and Dennis Expert. Opin. Investig. Drugs 2004, 13:787-97); and triciribine (TCN or API-2 or NCI identifier: NSC 154020; Yang et al., Cancer Res. 2004, 64:4394-9).

mTOR inhibitors include, but are not limited to, ATP-competitive mTORC1/mTORC2 inhibitors, e.g., PI-103, PP242, PP30; Torin 1; FKBP12 enhancers; 4H-1-benzopyran-4-one derivatives; and rapamycin (also known as sirolimus) and derivatives thereof, including: temsirolimus (Torisel®); everolimus (Afinitor®; WO94/09010); ridaforolimus (also known as deforolimus or AP23573); rapalogs, e.g., as disclosed in WO98/02441 and WO01/14387, e.g. AP23464 and AP23841; 40-(2-hydroxyethyl)rapamycin; 40-[3-hydroxy(hydroxymethyl)methylpropanoate]-rapamycin (also known as CC1779); 40-epi-(tetrazolyt)-rapamycin (also called ABT578); 32-deoxorapamycin; 16-pentynyloxy-32(S)-dihydrorapanycin; derivatives disclosed in WO05/005434; derivatives disclosed in U.S. Patent Nos. 5,258,389, 5,118,677, 5,118,678, 5,100,883, 5,151,413, 5,120,842, and 5,256,790, and in WO94/090101, WO92/05179, WO93/111130, WO94/02136, WO94/02485, WO95/14023, WO94/02136, WO95/16691, WO96/41807, WO96/41807, and WO2018204416; and phosphorus-containing rapamycin derivatives (e.g., WO05/016252). In some embodiments, the mTOR inhibitor is a bisteric inhibitor (see, e.g., WO2018204416, WO2019212990 and WO2019212991), such as RMC-5552, having the structure

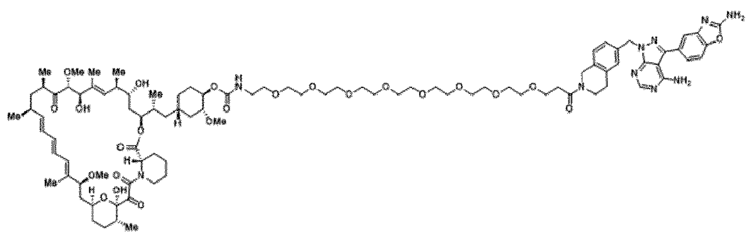

.

BRAF inhibitors that may be used in combination with a compound of Formula (I), or a pharmaceutically acceptable salt thereof, include, for example, vemurafenib, dabrafenib, and encorafenib. A BRAF may comprise a Class 3 BRAF mutation. In some embodiments, the Class 3 BRAF mutation is selected from one or more of the following amino acid substitutions in human BRAF: D287H; P367R; V459L; G466V; G466E; G466A; S467L; G469E; N581S; N581I; D594N; D594G; D594A; D594H; F595L; G596D; G596R and A762E.

MCL-1 inhibitors include, but are not limited to, AMG-176, MIK665, and S63845. The myeloid cell leukemia-1 (MCL-1) protein is one of the key anti-apoptotic members of the B-cell lymphoma-2 (BCL-2) protein family. Over-expression of MCL-1 has been closely related to tumor progression as well as to resistance, not only to traditional chemotherapies but also to targeted therapeutics including BCL-2 inhibitors such as ABT-263.

Proteasome inhibitors include, but are not limited to, carfilzomib (Kyprolis®), bortezomib (Velcade®), and oprozomib.

Immune therapies include, but are not limited to, monoclonal antibodies, immunomodulatory imides (IMiDs), GITR agonists, genetically engineered T-cells (e.g., CAR-T cells), bispecific antibodies (e.g., BiTEs), and anti-PD-1, anti-PD-Ll, anti-CTLA4, anti-LAGl, and anti-OX40 agents).

Immunomodulatory agents (IMiDs) are a class of immunomodulatory drugs (drugs that adjust immune responses) containing an imide group. The IMiD class includes thalidomide and its analogues (lenalidomide, pomalidomide, and apremilast).

Exemplary anti-PD-1 antibodies and methods for their use are described by Goldberg et al., Blood 2007, 110(1):186-192; Thompson et al., Clin. Cancer Res. 2007, 13(6):1757-1761; and WO06/121168 A1), as well as described elsewhere herein.

GITR agonists include, but are not limited to, GITR fusion proteins and anti-GITR antibodies (e.g., bivalent anti-GITR antibodies), such as, a GITR fusion protein described in U.S. Pat. No. 6,111,090, U.S. Pat. No. 8,586,023, WO2010/003118 and WO2011/090754; or an anti-GITR antibody described, e.g., in U.S. Pat. No. 7,025,962, EP 1947183, U.S. Pat. No. 7,812,135, U.S. Pat. No. 8,388,967, U.S. Pat. No. 8,591,886, U.S. Pat. No. 7,618,632, EP 1866339, and WO2011/028683, WO2013/039954, WO05/007190, WO07/133822, WO05/055808, WO99/40196, WO01/03720, WO99/20758, WO06/083289, WO05/115451, and WO2011/051726.

Another example of a therapeutic agent that may be used in combination with a compound of Formula (I), or

a pharmaceutically acceptable salt thereof, is an anti-angiogenic agent. Anti-angiogenic agents are inclusive of, but not limited to, in vitro synthetically prepared chemical compositions, antibodies, antigen binding regions, radionuclides, and combinations and conjugates thereof. An anti-angiogenic agent can be an agonist, antagonist, allosteric modulator, toxin or, more generally, may act to inhibit or stimulate its target (e.g., receptor or enzyme activation or inhibition), and thereby promote cell death or arrest cell growth. In some embodiments, the one or more additional therapies include an anti-angiogenic agent.

Anti-angiogenic agents can be MMP-2 (matrix-metalloproteinase 2) inhibitors, MMP-9 (matrix-metalloprotienase 9) inhibitors, and COX-II (cyclooxygenase 11) inhibitors. Non-limiting examples of anti-angiogenic agents include rapamycin, temsirolimus (CCI-779), everolimus (RAD001), sorafenib, sunitinib, and bevacizumab. Examples of useful COX-II inhibitors include alecoxib, valdecoxib, and rofecoxib. Examples of useful matrix metalloproteinase inhibitors are described in WO96/33172, WO96/27583, WO98/07697, WO98/03516, WO98/34918, WO98/34915, WO98/33768, WO98/30566, WO90/05719, WO99/52910, WO99/52889, WO99/29667, WO99007675, EP0606046, EP0780386, EP1786785, EP1181017, EP0818442, EP1004578, and US20090012085, and U.S. Patent Nos. 5,863,949 and 5,861,510. Preferred MMP-2 and MMP-9 inhibitors are those that have little or no activity inhibiting MMP-1. More preferred, are those that selectively inhibit MMP-2 or AMP-9 relative to the other matrix-metalloproteinases (i.e., MAP-1, MMP-3, MMP-4, MMP-5, MMP-6, MMP-7, MMP-8, MMP-10, MMP-11, MMP-12, and MMP-13). Some specific examples of MMP inhibitors are AG-3340, RO 32-3555, and RS 13-0830.

Further exemplary anti-angiogenic agents include KDR (kinase domain receptor) inhibitory agents (e.g., antibodies and antigen binding regions that specifically bind to the kinase domain receptor), anti-VEGF agents (e.g., antibodies or antigen binding regions that specifically bind VEGF (e.g., bevacizumab), or soluble VEGF receptors or a ligand binding region thereof) such as VEGF-TRAP™, and anti-VEGF receptor agents (e.g., antibodies or antigen binding regions that specifically bind thereto), EGFR inhibitory agents (e.g., antibodies or antigen binding regions that specifically bind thereto) such as Vectibix® (panitumumab), erlotinib (Tarceva®), anti-Angl and anti-Ang2 agents (e.g., antibodies or antigen binding regions specifically binding thereto or to their receptors, e.g., Tie2/Tek), and anti-Tie2 kinase inhibitory agents (e.g., antibodies or antigen binding regions that specifically bind thereto). Other anti-angiogenic agents include Campath, IL-8, B-FGF, Tek antagonists (US2003/0162712; US6,413,932), anti-TWEAK agents (e.g., specifically binding antibodies or antigen binding regions, or soluble TWEAK receptor antagonists; see US6,727,225), ADAM distintegrin domain to antagonize the binding of integrin to its ligands (US 2002/0042368), specifically binding anti-eph receptor or anti-ephrin antibodies or antigen binding regions (U.S. Patent Nos. 5,981,245; 5,728,813; 5,969,110; 6,596,852; 6,232,447; 6,057,124 and patent family members thereof), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto). Additional anti-angiogenic agents include: SD-7784 (Pfizer, USA); cilengitide (Merck KGaA, Germany, EPO 0770622); pegaptanib octasodium, (Gilead Sciences, USA); Alphastatin, (BioActa, UK); M-PGA, (Celgene, USA, US 5712291); ilomastat, (Arriva, USA, US5892112); emaxanib, (Pfizer, USA, US 5792783); vatalanib, (Novartis, Switzerland); 2-methoxyestradiol (EntreMed, USA); TLC ELL-12 (Elan, Ireland); anecortave acetate (Alcon, USA); alpha-D148 Mab (Amgen, USA); CEP-7055 (Cephalon, USA); anti-Vn Mab (Crucell, Netherlands), DACantiangiogenic (ConjuChem, Canada); Angiocidin (InKine Pharmaceutical, USA); KM-2550 (KyowaHakko, Japan); SU-0879 (Pfizer, USA); CGP-79787 (Novartis, Switzerland, EP 0970070); ARGENT technology (Ariad, USA); YIGSR-Stealth (Johnson & Johnson, USA); fibrinogen-E fragment (BioActa, UK); angiogenic inhibitor (Trigen, UK); TBC-1635 (Encysive Pharmaceuticals, USA); SC-236 (Pfizer, USA); ABT-567 (Abbott, USA); Metastatin (EntreMed, USA); maspin (Sosei, Japan); 2-methoxyestradiol (Oncology Sciences Corporation, USA); ER-68203-00 (IV AX, USA); BeneFin (Lane Labs, USA); Tz-93 (Tsumura, Japan); TAN-1120 (Takeda, Japan); FR-111142 (Fujisawa, Japan, JP 02233610); platelet factor 4 (RepliGen, USA, EP 407122); vascular endothelial growth factor antagonist (Borean, Denmark); bevacizumab (pINN) (Genentech, USA); angiogenic inhibitors (SUGEN, USA); XL 784 (Exelixis, USA); XL 647 (Exelixis, USA); MAb, alpha5beta3 integrin, second generation (Applied Molecular Evolution, USA and Medlmmune, USA); enzastaurin hydrochloride (Lilly, USA); CEP 7055 (Cephalon, USA and Sanofi-Synthelabo, France); BC 1 (Genoa Institute of Cancer Research, Italy); rBPI 21 and BPI-derived antiangiogenic (XOMA, USA); PI 88 (Progen, Australia); cilengitide (Merck KGaA, German; Munich Technical University, Germany, Scripps Clinic and Research Foundation, USA); AVE 8062 (Ajinomoto, Japan); AS 1404 (Cancer Research Laboratory, New Zealand); SG 292, (Telios, USA); Endostatin (Boston Childrens Hospital, USA); ATN 161 (Attenuon, USA); 2-methoxyestradiol (Boston Childrens Hospital, USA); ZD 6474, (AstraZeneca, UK); ZD 6126, (Angiogene Pharmaceuticals, UK); PPI 2458, (Praecis, USA); AZD 9935, (AstraZeneca, UK); AZD 2171, (AstraZeneca, UK); vatalanib (pINN), (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); vaccine, gene-based, VEGF-2, (Scripps Clinic and Research Foundation, USA); SPV5.2, (Supratek, Canada); SDX 103, (University of California at San Diego, USA); PX 478, (Pro1X, USA); METASTATIN, (EntreMed, USA); troponin I, (Harvard University, USA); SU 6668, (SUGEN, USA); OXI 4503, (OXiGENE, USA); o-guanidines, (Dimensional Pharmaceuticals, USA); motuporamine C, (British Columbia University, Canada); CDP 791, (Celltech Group, UK); atiprimod (pINN), (GlaxoSmithKline, UK); E

7820, (Eisai, Japan); CYC 381, (Harvard University, USA); AE 941, (Aeterna, Canada); vaccine, angiogenic, (EntreMed, USA); urokinase plasminogen activator inhibitor, (Dendreon, USA); oglufanide (pINN), (Melmotte, USA); HIF-lalfa inhibitors, (Xenova, UK); CEP 5214, (Cephalon, USA); BAY RES 2622, (Bayer, Germany); Angiocidin, (InKine, USA); A6, (Angstrom, USA); KR 31372, (Korea Research Institute of Chemical Technology, South Korea); GW 2286, (GlaxoSmithKline, UK); EHT 0101, (ExonHit, France); CP 868596, (Pfizer, USA); CP 564959, (OSI, USA); CP 547632, (Pfizer, USA); 786034, (GlaxoSmithKline, UK); KRN 633, (Kirin Brewery, Japan); drug delivery system, intraocular, 2-methoxyestradiol; anginex (Maastricht University, Netherlands, and Minnesota University, USA); ABT 510 (Abbott, USA); AAL 993 (Novartis, Switzerland); VEGI (ProteomTech, USA); tumor necrosis factor-alpha inhibitors; SU 11248 (Pfizer, USA and SUGEN USA); ABT 518, (Abbott, USA); YH16 (Yantai Rongchang, China); S-3APG (Boston Childrens Hospital, USA and EntreMed, USA); MAb, KDR (ImClone Systems, USA); MAb, alpha5 beta (Protein Design, USA); KDR kinase inhibitor (Celltech Group, UK, and Johnson & Johnson, USA); GFB 116 (South Florida University, USA and Yale University, USA); CS 706 (Sankyo, Japan); combretastatin A4 prodrug (Arizona State University, USA); chondroitinase AC (IBEX, Canada); BAY RES 2690 (Bayer, Germany); AGM 1470 (Harvard University, USA, Takeda, Japan, and TAP, USA); AG 13925 (Agouron, USA); Tetrathiomolybdate (University of Michigan, USA); GCS 100 (Wayne State University, USA) CV 247 (Ivy Medical, UK); CKD 732 (Chong Kun Dang, South Korea); irsogladine, (Nippon Shinyaku, Japan); RG 13577 (Aventis, France); WX 360 (Wilex, Germany); squalamine, (Genaera, USA); RPI 4610 (Sirna, USA); heparanase inhibitors (InSight, Israel); KL 3106 (Kolon, South Korea); Honokiol (Emory University, USA); ZK CDK (Schering AG, Germany); ZK Angio (Schering AG, Germany); ZK 229561 (Novartis, Switzerland, and Schering AG, Germany); XMP 300 (XOMA, USA); VGA 1102 (Taisho, Japan); VE-cadherin-2 antagonists(ImClone Systems, USA); Vasostatin (National Institutes of Health, USA); Flk-1 (ImClone Systems, USA); TZ 93 (Tsumura, Japan); TumStatin (Beth Israel Hospital, USA); truncated soluble FLT 1 (vascular endothelial growth factor receptor 1) (Merck & Co, USA); Tie-2 ligands (Regeneron, USA); and thrombospondin 1 inhibitor (Allegheny Health, Education and Research Foundation, USA).

Further examples of therapeutic agents that may be used in combination with a compound Formula (I), or a pharmaceutically acceptable salt thereof, include agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor, c-Met.

Another example of a therapeutic agent that may be used in combination with a compound of Formula (I), or a pharmaceutically acceptable salt thereof, is an autophagy inhibitor. Autophagy inhibitors include, but are not limited to chloroquine, 3- methyladenine, hydroxychloroquine (Plaquenil™), bafilomycin A1, 5-amino-4-imidazole carboxamide riboside (AICAR), okadaic acid, autophagy-suppressive algal toxins which inhibit protein phosphatases of type 2A or type 1, analogues of cAMP, and drugs which elevate cAMP levels such as adenosine, LY204002, N6-mercaptopurine riboside, and vinblastine. In addition, antisense or siRNA that inhibits expression of proteins including but not limited to ATG5 (which are implicated in autophagy), may also be used. In some embodiments, the one or more additional therapies include an autophagy inhibitor.

Another example of a therapeutic agent that may be used in combination with a compound of Formula (I), or a pharmaceutically acceptable salt thereof, is an anti-neoplastic agent. In some embodiments, the one or more additional therapies include an anti-neoplastic agent. Non-limiting examples of anti-neoplastic agents include acemannan, aclarubicin, aldesleukin, alemtuzumab, alitretinoin, altretamine, amifostine, aminolevulinic acid, amrubicin, amsacrine, anagrelide, anastrozole, ancer, ancestim, arglabin, arsenic trioxide, BAM-002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-Nl, interferon alfa-n3, interferon alfacon-1, interferon alpha, natural, interferon beta, interferon beta-la, interferon beta-lb, interferon gamma, natural interferon gamma- la, interferon gamma-lb, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan poly sulfate sodium, pentostatin, picibanil, pirarubicin, rabbit antithymocyte polyclonal antibody, polyethylene glycol interferon alfa-2a, porfimer sodium, raloxifene, raltitrexed, rasburiembodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide,

thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, virulizin, zinostatin stimalamer, or zoledronic acid; abarelix; AE 941 (Aeterna), ambamustine, antisense oligonucleotide, bcl-2 (Genta), APC 8015 (Dendreon), decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techni clone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell ly sates vaccine (Royal Newcastle Hospital), or valspodar.

Additional examples of therapeutic agents that may be used in combination with a compound of Formula (I), or a pharmaceutically acceptable salt thereof, include ipilimumab (Yervoy®); tremelimumab; galiximab; nivolumab, also known as BMS-936558 (Opdivo®); pembrolizumab (Keytruda®); avelumab (Bavencio®); AMP224; BMS-936559; MPDL3280A, also known as RG7446; MEDI-570; AMG557; MGA271; IMP321; BMS-663513; PF-05082566; CDX-1127; anti-OX40 (Providence Health Services); huMAbOX40L; atacicept; CP-870893; lucatumumab; dacetuzumab; muromonab-CD3; ipilumumab; MEDI4736 (Imfinzi®); MSB0010718C; AMP 224; adalimumab (Humira®); ado-trastuzumab emtansine (Kadcyla®); aflibercept (Eylea®); alemtuzumab (Campath®); basiliximab (Simulect®); belimumab (Benlysta®); basiliximab (Simulect®); belimumab (Benlysta®); brentuximab vedotin (Adcetris®); canakinumab (Ilaris®); certolizumab pegol (Cimzia®); daclizumab (Zenapax®); daratumumab (Darzalex®); denosumab (Prolia®); eculizumab (Soliris®); efalizumab (Raptiva®); gemtuzumab ozogamicin (Mylotarg®); golimumab (Simponi®); ibritumomab tiuxetan (Zevalin®); infliximab (Remicade®); motavizumab (Numax®); natalizumab (Tysabri®); obinutuzumab (Gazyva®); ofatumumab (Arcerra®); omalizumab (Xolair®); palivizumab (Synagis®); pertuzumab (Peijeta®); ranibizumab (Lucentis®); raxibacumab (Abthrax®); tocilizumab (Actemra®); tositumomab; tositumomab-i-131; ustekinumab (Stelara®); AMG 102; AMG 386; AMG 479; AMG 655; AMG 706; AMG 745; and AMG 951.

In some embodiments, a compound of Formula (I), or a pharmaceutically acceptable salt thereof, may be used in combination with one or more of the following: a SOS1 inhibitor, a Ras inhibitor (e.g., a Ras(ON) inhibitor such as RMC-6291 or RMC-6236) or a Ras(OFF) inhibitor, such as adagrasib or sotorasib), a MEK inhibitor, an EGFR inhibitor, or an immune checkpoint inhibitor (e.g., an anti-PD 1 inhibitor, such as pembrolizumab).

In some embodiments, a compound of Formula (I), or a pharmaceutically acceptable salt thereof, is used in combination with an ERK inhibitor and a BRAF inhibitor (i.e., as part of a triple combination therapy).

The compounds described in the present disclosure can be used in combination with the agents disclosed herein or other suitable agents, depending on the condition being treated. Hence, in some embodiments, the one or more compounds of Formula (I), or pharmaceutically acceptable salts thereof, will be co-administered with other therapies as described herein. When used in combination therapy, the compounds described herein may be administered with the second agent simultaneously or separately. This administration in combination can include simultaneous administration of the two agents in the same dosage form, simultaneous administration in separate dosage forms, and separate administration. That is, a compound of Formula (I), or a pharmaceutically acceptable salt thereof, and any of the agents described herein can be formulated together in the same dosage form and administered simultaneously. Alternatively, a compound of Formula (I), or a pharmaceutically acceptable salt thereof, and any of the therapies described herein can be simultaneously administered, wherein both the agents are present in separate formulations. In another alternative, a compound of Formula (I), or a pharmaceutically acceptable salt thereof, can be administered and followed by any of the therapies described herein, or vice versa. In some embodiments of the separate administration protocol, a compound of Formula (I), or a pharmaceutically acceptable salt thereof, and any of the therapies described herein are administered a few minutes apart, or a few hours apart, or a few days apart.

In some embodiments of any of the methods described herein, the first therapy (e.g., a compound of Formula (I) or a pharmaceutically acceptable salt thereof) and one or more additional therapies are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours, up to 24 hours, or up to 1-7, 1-14, 1-21 or 1-30 days before or after the one or more additional therapies.

## Kits/Article of Manufacture

Disclosed herein, in certain embodiments, are kits and articles of manufacture for use with one or more compounds, compositions, or methods described herein. Such kits include a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. In one embodiment, the containers are formed from a variety of materials such as glass or plastic.

A kit typically includes labels listing contents and/or instructions for use, and package inserts with instructions for use. A set of instructions will also typically be included.

In one embodiment, a label is on or associated with the container. In one embodiment, a label is on a container when letters, numbers or other characters forming the label are attached, molded or etched into the container itself, a label is associated with a container when it is present within a receptacle or carrier that also holds the container, e.g., as a package insert. In one embodiment, a label is used to indicate that the contents are to be used for a specific therapeutic application. The label also indicates directions for use of the contents, such as in the methods described herein.

In certain embodiments, the pharmaceutical compositions are presented in a pack or dispenser device which contains one or more unit dosage forms containing a compound provided herein. The pack, for example, contains metal or plastic foil, such as a blister pack. In one embodiment, the pack or dispenser device is accompanied by instructions for administration. In one embodiment, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for drugs, or the approved product insert. In one embodiment, compositions containing a compound provided herein formulated in a compatible pharmaceutical carrier are also prepared, placed in an appropriate container, and labeled for treatment of an indicated condition.

## EXAMPLES

The examples and preparations provided below further illustrate and exemplify the compounds of the present disclosure and methods for testing such compounds. It is to be understood that the scope of the present disclosure is not limited in any way by the scope of the following examples.

The chemical reactions in the Examples described can be readily adapted to prepare a number of other compounds disclosed herein, and alternative methods for preparing the compounds of this disclosure are deemed to be within the scope of this disclosure. For example, the synthesis of non-exemplified compounds according to the present disclosure can be performed by modifications apparent to those skilled in the art, for example by appropriately protecting interfering groups, by utilizing other suitable reagents known in the art other than those described, or by making routine modification of reaction conditions, reagents, and starting materials. Alternatively, other reactions disclosed herein or known in the art will be recognized as having applicability for preparing other compounds of the present disclosure.

The following abbreviations may be relevant for the application.

Abbreviations

ACN or MeCN: Acetonitrile
AcOH: Acetic acid
AIBN: Azobisisobutyronitrile
aq: Aqueous
$BF_3.Et_2O$: Boron trifluoride etherate
Bis-pin: Bis(pinacolato) diboron
Boc: tert-Butoxycarbonyl
$Boc_2O$: di-tert-Butyl decarbonate
BSA: Bovine serum albumin
conc.: Concentrated
DAST: Diethylaminosulfur trifluoride
DCM: Dichloromethane
DIBAL-H: Diisobutylaluminum hydride
DIEA or DIPEA: Diisopropylethylamine
DiFMUP: 6,8-Difluoro-4-methylumbelliferyl phosphate

Dioxane: 1,4-Dioxane
DMA: N, N-Dimethylacetamide
DMF: Dimethylformamide
DMSO: Dimethyl sulfoxide
DTT: Dithiothreitol
EDTA: Ethylenediaminetetraacetic acid
$Et_2O$: diethyl ether
$Et_3N$: triethylamine
EtOAc: Ethyl acetate
EtOH: Ethanol
h: Hour(s)
HEPES: 4-(2-Hydroxyethyl)-1-piperazineethanesulfonic acid
HPLC: High performance liquid chromatography
IBX: 2-Iodoxybenzoic acid
$iPr_2O$: Diisopropyl ether
iPrOH: Isopropyl alcohol
KOAc: Potassium acetate
L: liter
LCMS: Liquid chromatography / mass spectrometry
LDA: Lithium diisopropylamide
m-CPBA: 3-Chloroperbenzoic acid
mCPBA: meta-Chloroperoxybenzoic acid
Max: maximum
2-MeTHF: 2-Methyltetrahydrofuran
MeOH: Methanol
min: Minute
Min: Minimum
MTBE: tert-Butyl methyl ether
NBS: N-Bromosuccinimide
n-BuLi: n-Butyllithium
NMP: N-Methyl pyrrolidinone
NMR: Nuclear magnetic resonance
$Pd(AmPhos)_2Cl_2$: Dichlorobis(*p*-methylaminophenyl-di-tert-butylphosphine)palladium(II)
$Pd(dba)_2$: Palladium(0) bis(dibenzylideneacetone)
$Pd(dppf)Cl_2$: Dicldoro bis(1,1'-diphenylphospwnoferrocene)-Palladium(II)
$Pd(PPh_3)_4$: Tetrakis(triphenylphosphine)palladium(0)
$Pd_2(dba)_3$: Tris(dibenzylideneacetone)dipalladium(0)
PPTS: Pyridinium p-toluenesulfonate
psi: Pounds per square inch
$Py.HBr_3$: Pyridinium hydrobromide perbromide
rt: Retention time
RT: Room temperature
SFC: Supercritical fluid chromatography
SPhos Pd G2: Chloro(2-dicyclohexylphosphino-2',6'-dimethoxy-1,1'-bipheny1)[2-(2'-amino-1,1'-biphenyl)]palladium(II)
SPhos Pd G4: Methanesulfonato(2-dicyclohexylphosphino-2',6'-dimethoxybiphenyl)(2'-methylamino-1,1'-biphenyl-2-yl)palladium(II)
SPhos: 2-Dicyclohexylphosphino-2',6'-dimethoxybiphenyl
TBAB: Tetrabutylammonium bromide
TBS: tert-Butyldimethylsilyl
t-BuOK: Potassium tert-butoxide
TEA: Triethylamine
TFA: Trifluoroacetic acid
TFAA: Trifluoroacetic anhydride
THF: Tetrahydrofuran
$Ti(OEt)_4$: Titanium (IV) ethoxide
TIPS: Triisopropylsilyl
TIPSCl: Triisopropylsilyl chloride

XantPhos: 4,5-Bis(diphenylphosphino)-9,9-dimethylxanthene
XPhos Pd G4: methanesulfonato(2-dicyclohexylphosphino-2',4',6'-tri-i-propyl-1,1'-biphenyl)(2'-methylamino-1,1'-biphenyl-2-yl)palladium(II)
XPhos: 2-Dicyclohexylphosphino-2',4',6'-triisopropylbiphenyl

*Synthetic Examples*

Intermediate Compounds

**Example i-1. Intermediate A-1 (7-bromo-4-chloro-6-methyl-pyrazolo[1,5-a]pyrazine)**

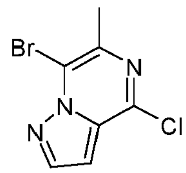

**Step a:** To a solution of ethyl pyrazole-3-carboxylate (510 g, 3.64 mol) inDMF (3 L) was added 1-chloropropan-2-one (504 g, 5.46 mol) and potassium carbonate (1005 g, 7.27 mol) at RT under $N_2$ and the mixture was stirred for 2 h. The residue was poured into water (7.00 L). The aqueous phase was extracted with ethyl acetate (3.00 L * 3). The combined organic phase was washed with brine (3.00 L), dried with anhydrous $Na_2SO_4$, filtered and concentrated under vacuum. The residue was purified by column chromatography ($SiO_2$, petroleum ether/ethyl acetate = 50/1 to 1/1) to give ethyl 2-acetonylpyrazole-3-carboxylate (139 g, 680 mmol) as a yellow solid. $^1H$ NMR (400 MHz, $CDCl_3$) δ 7.56 (d, *J* = 2.0 Hz, 1H), 6.91 (d, *J*= 2.0 Hz, 1H), 5.36 (s, 2H), 4.31 (q, *J*= 7.2 Hz, 2H), 2.20 (s, 3H), 1.36 (t, *J* = 7.2 Hz, 3H).

**Step b:** To a solution of ethyl 2-acetonylpyrazole-3-carboxylate (139 g, 708 mmol) in $CH_3CO_2H$ (690 mL) was added $CH_3CO_2NH_4$ (273 g, 3.54 mol) at RT under $N_2$. The reaction was stirred at 130 °C for 16 h. The solution was concentrated under vacuum. The residue was purified by column chromatography ($SiO_2$, petroleum ether/ethyl acetate = 50/1 to 1/1) to give 6-methylpyrazolo[1,5-a]pyrazin-4(5H)-one (68.0 g, 433 mmol) as a brown solid. $^1H$ NMR (400 MHz, $CDCl_3$) δ 10.8 (s, 1H), 7.79 (d, *J* = 2.4 Hz, 1H), 7.33 (s, 1H), 7.06 (d, *J* = 2.0 Hz, 1H), 2.31 (d, *J* = 1.2 Hz, 3H).

**Step c:** To a solution of 6-methylpyrazolo[1,5-a]pyrazin-4(5H)-one (68.0 g, 455 mmol) in DMF (340 mL) was added NBS (89.2 g, 501 mmol) at 0 °C under $N_2$. The reaction was stirred at 0 °C for 5 min. The residue was poured into a saturated aqueous solution of sodium sulfite (2.00 L) and $H_2O$ (2.00 L). The aqueous phase was extracted with ethyl acetate (1.50 L * 3). The combined organic phase was washed with brine (2.00 L), dried with anhydrous $Na_2SO_4$, filtered and concentrated under vacuum. The crude product was triturated with MTBE (80.0 mL) at RT for 30 min to give 7-bromo-6-methyl-pyrazolo[1,5-a]pyrazin-4(5H)-one (43.0 g, 182 mmol) as a white solid. $^1H$ NMR (400 MHz, $CDCl_3$) δ 11.1 (s, 1H), 7.90 (d, *J* = 2.0 Hz, 1H), 7.22 (d, *J* = 2.0 Hz, 1H), 2.49 (s, 3H).

**Step d:** A solution of 7-bromo-6-methyl-pyrazolo[1,5-a]pyrazin-4(5H)-one (43.0 g, 188 mmol) in $PCl_3$ (215 mL) was stirred at 110 °C for 20 min under $N_2$. The residue was poured carefully into water (1.50 L) and stirred for 5 min. The pH value of the aqueous phase was adjusted to ~7 with saturated aqueous solution of sodium bicarbonate. The aqueous phase was extracted with ethyl acetate (1.00 L * 2). The combined organic phase was washed with brine (1.00 L), dried with anhydrous $Na_2SO_4$, filtered and concentrated under vacuum. The residue was purified by column chromatography ($SiO_2$, petroleum ether/ethyl acetate = 50/1 to 1/1) to give 7-bromo-4-chloro-6-methyl-pyrazolo[1,5-a]pyrazine (33.0 g, 131 mmol) as a off-white solid. $^1H$ NMR (400 MHz, $CDCl_3$) δ 8.09 (d, *J* = 2.0 Hz, 1H), 6.98 (d, *J* = 2.4 Hz, 1H), 2.68 (s, 3H). LCMS m/z $[M+H]^+$ 245.9.

**Example i-2. Intermediate A-2 (4,7-dibromo-6-methyl-pyrazolo[1,5-a]pyrazine)**

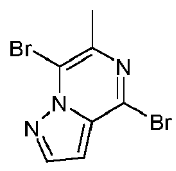

To a solution of 7-bromo-6-methyl-pyrazolo[l,5-a]pyrazin-4(5H)-one (19.0 g, 83.3 mmol) in DCM (200 mL) was added phosphoryl bromide (11 mL, 108 mmol) and DMF (6 mL). The mixture was stirred at 40 °C for 6 h. The mixture was slowly poured into water (200 mL), and extracted with ethyl acetate (200 mL * 2). The combined organic phase was washed with brine (100 mL), dried with anhydrous $Na_2SO_4$, filtered and concentrated under vacuum. The residue was purified by column chromatography ($SiO_2$, petroleum ether/ethyl acetate = 100/1 to 0/1) to give 4,7-dibromo-6-methyl-pyrazolo[1,5-a]pyrazine (15.0 g, 51.5 mmol) as a white solid. $^1H$ NMR (400 MHz, $CDCl_3$) δ 8.06 (d, *J* = 2.40 Hz, 1H), 6.92 (d, *J* = 2.40 Hz, 1H), 2.67 (s, 3H).

**Example i-3. Intermediate A-3 (7-bromo-4-chloro-pyrazolo[1,5-a]pyrazine)**

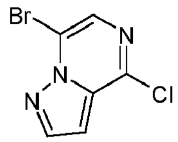

Step a: To a mixture of pyrazole-3-carboxylic acid (200 g, 1.78 mol) in dioxane (1400 mL) was added 1,1'-carbonyldiimidazole (318 g, 1.96 mol) in one portion at RT. The reaction mixture was stirred at 50 °C for 30 min. Then to the mixture was added aminoacetaldehyde dimethyl acetal (214 mL, 1.96 mol) in one portion at 50 °C. The reaction mixture was stirred at 50 °C for 30 min. Then to the mixture was added an aqueous solution of HCl (12 M, 743 mL) in one portion at 50 °C. The reaction mixture was stirred at 100 °C for 16 h. The mixture was then concentrated under vacuo. To the residue was added water (2.00 L) and the mixture was stirred for 30 min. The solid was collected by filtration and washed with water (500 mL * 2) to give crude pyrazolo[1,5-a]pyrazin-4(5H)-one (100 g) as a brown solid that was used into the next step without further purification.

Step b: To a solution of crude pyrazolo[1,5-a]pyrazin-4(5H)-one previously obtained (100 g) in DMF (700 mL) was added AcOH (127 mL, 2.22 mol) and NBS (132 g, 0.74 mol) at 0 °C under $N_2$. The reaction was stirred at 0 °C for 5 min and then poured into ice-water. The aqueous phase was extracted with ethyl acetate. The combined organic phase was washed with brine, dried with anhydrous $Na_2SO_4$ filtered and concentrated in vacuum to give crude 7-bromopyrazolo[1,5-a]pyrazin-4(5H)-one (80 g) that was used into the next step without further purification.

**Step c:** A solution of crude 7-bromopyrazolo[1,5-a]pyrazin-4(5H)-one (80 g) in $PCl_3$ (800 mL, 8.61 mol) was stirred at 100 °C for 2 h. The residue was carefully poured into water (500 mL). The pH value of the aqueous phase was adjusted to ~7 with a saturated solution of $NaHCO_3$. The aqueous phase was extracted with ethyl acetate (300 mL * 2). The combined organic phase was washed with brine (200 mL), dried with anhydrous $Na_2SO_4$, filtered and concentrated under vacuum. The residue was purified by column chromatography ($SiO_2$, petroleum ether/ethyl acetate = 50/1 to 1/1) followed by a purification by prep-HPLC (neutral condition) to give 7-bromo-4-chloro-pyrazolo[1,5-a]pyrazine (30.0 g, 128 mmol) as a white solid. $^1H$ NMR (400 MHz, $CDCl_3$) δ 8.39 (d, *J* = 2.4 Hz, 1H), 8.11 (s, 1H), 7.27 *(d, J* = 1.2 Hz, 1H). LCMS m/z $[M+H]^+$ 233.9.

**Example i-4. Intermediate A-4 (ethyl 7-bromo-4-chloro-6-methyl-pyrazolo[1,5-a]pyrazine-2-carboxylate)**

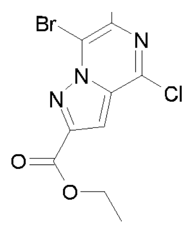

**Step a:** To a mixture of diethyl 3,5-pyrazoledicarboxylate (4.4 g, 21 mmol) in acetone (100 mL) was added potassium carbonate (3.9 g, 28.2 mmol) and then 1-chloropropan-2-one (1.8 mL, 23.0 mmol) at RT. The reaction mixture was stirred at 55 °C for 3 h. The mixture was then concentrated under vacuo. To the residue was added water (100 mL) and the aqueous phase was extracted with ethyl acetate (150 mL * 2). The combined organic phase was washed with brine, dried with anhydrous $Na_2SO_4$, filtered and concentrated under vacuum to give ethyl 4-hydroxy-6-methyl-pyrazolo[1,5-a]pyrazine-2-carboxylate (5.83 g) as a brown oil that was used into the next step without further purification.

**Step b:** To a mixture of crude diethyl 3,5-pyrazoledicarboxylate previously obtained (5.83 g) in AcOH (90 mL) was added ammonium acetate (33.5 g, 435.0 mmol). The reaction mixture was stirred at 120 °C for 20 h. The mixture was cooled down to RT and then poured into water (300 mL) and stirred for 15 min. The precipitate was collected by filtration and washed with water to give crude ethyl 7-bromo-4-hydroxy-6-methyl-pyrazolo[1,5-a]pyrazine-2-carboxylate (2.01 g) as a brown solid that was used into the next step without further purification.

**Step c:** To a mixture of crude ethyl 7-bromo-4-hydroxy-6-methyl-pyrazolo[1,5-a]pyrazine-2-carboxylate previously obtained (2.01 g) in dichloromethane (40 mL), cooled to 0 °C, was added NBS (1.95 g, 11.0 mmol). Then the reaction was stirred at 0 °C for 1 h. Dichloromethane (120 mL) and a saturated aqueous solution of sodium thiosulfate (200 mL) were added and the mixture was stirred for 30 min at RT. The precipitate was collected by filtration and washed with water to give crude ethyl 7-bromo-4-chloro-6-methyl-pyrazolo[1,5-a]pyrazine-2-carboxylate (2.43 g) as a brown solid that was used into the next step without further purification.

**Step d:** A solution of crude ethyl 7-bromo-4-chloro-6-methyl-pyrazolo[1,5-a]pyrazine-2-carboxylate (2.43 g) in $PCl_3$ (38 mL, 408 mmol) was stirred at 120 °C for 2 h. The residue was carefully poured into water (500 mL). The pH value of the aqueous phase was adjusted to ~7 with a saturated solution of $NaHCO_3$. The aqueous phase was extracted with ethyl acetate (300 mL * 2). The combined organic phase was washed with brine, dried with anhydrous $Na_2SO_4$, filtered and concentrated under vacuum to give ethyl 7-bromo-6-methyl-4-(5-oxospiro[7H-cyclopenta[b]pyridine-6,4'-piperidine]-1-yl)pyrazolo[1,5-a]pyrazine-2-carboxylate (2.18 g, 6.84 mmol) as a light brown solid. $^1H$ NMR (400 MHz, DMSO-*d6*) δ 7.60 (s, 1H), 4.41 (d, *J* = 7.1 Hz, 3H), 2.61 (s, 3H), 1.36 (t, *J* = 7.1 Hz, 3H). LCMS m/z $[M+H]^+$ 320.0.

**Example i-5. Intermediate A-5 (ethyl 7-bromo-4-chloro-6-methyl-pyrazolo[1,5-a]pyrazine-2-carboxylate)**

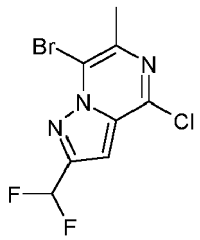

**Step a:** To a mixture of ethyl 7-bromo-6-methyl-4-(5-oxospiro[7H-cyclopenta[b]pyridine-6,4'-piperidine]-1'-yl)pyrazolo[1,5-a]pyrazine-2-carboxylate (1 g, 3.14 mmol) in anhydrous THF (40 mL), cooled at -78 °C, was added dropwise DIBAL-H (1 M in toluene, 7 mL, 7 mmol). The reaction mixture was stirred at -78 °C for 1.5 h and then let to warm to 0 °C. A saturated aqueous solution of Rochelle salt (10 mL) was added and then water (40 mL) and ethyl acetate (40 mL). The mixture was stirred at RT for 16 h. The organic layer was separated and the aqueous phase was extracted with ethyl acetate (40 mL). The combined organic phase was washed with brine, dried with anhydrous $Na_2SO_4$, filtered and concentrated under vacuum. The residue was purified by column chromatography ($SiO_2$, cyclohexane/ethyl acetate = 8/2 to 6/4) to give 7-bromo-4-chloro-6-methyl-pyrazolo[1,5-a]pyrazin-2-yl)methanol (535 mg, 1.93 mmol) as a white solid. $^1H$ NMR (400 MHz, DMSO-*d6*) δ 6.97 (s, 1H), 5.42 (t, *J* = 5.9 Hz, 1H), 4.64 (d, *J* = 5.9 Hz, 2H), 3.21 (s, 3H). LCMS m/z $[M+H]^+$ 277.9.

**Step b:** To a mixture of 7-bromo-4-chloro-6-methyl-pyrazolo[1,5-a]pyrazin-2-yl)methanol (0.53 g, 1.92 mmol) in dichloromethane (20 mL), cooled to 0 °C, was added portionwise Dess-Martin periodinane (1.0 g, 2.36 mmol). The reaction was stirred at 0 °C for 45 min and then 45 min at RT. A saturated aqueous solution of sodium thiosulfate (20 mL), sodium bicarbonate (20 mL) and dichloromethane (20 mL) were added and the mixture stirred for 1 h. The mixture was filtered on a hydrophobic cartridge (liquid / liquid extraction column, Radleys®) and then concentrated in vacuum to give crude 7-bromo-4-chloro-6-methyl-pyrazolo[1,5-a]pyrazine-2-carbaldehyde (0.82 g) as a brown solid that was used into the next step without further purification.

**Step c:** To a mixture of crude 7-bromo-4-chloro-6-methyl-pyrazolo[1,5-a]pyrazine-2-carbaldehyde (0.82 g) in dichloromethane (20 mL), cooled at -20 °C, was added dropwise diethylaminosulfur trifluoride (1.6 mL, 12.1 mmol). The reaction mixture was stirred at -20 °C for 2 h and then at RT for 2 h. The mixture was cooled to 0 °C and a saturated aqueous solution of sodium bicarbonate (20 mL) was slowly added and then solid sodium bicarbonate to adjust the pH value of the aqueous phase to ~8. Water (20 mL) and dichloromethane (30 mL) were added and the mixture was filtered on a hydrophobic cartridge (liquid / liquid extraction column, Radleys®) and then concentrated in vacuum. The residue was purified by column chromatography ($SiO_2$, cyclohexane/ethyl acetate = 100/0 to 95/5) to give 7-bromo-4-chloro-2-(difluoromethyl)-6-methyl-pyrazolo[1,5-a]pyrazine (437 mg, 1.47 mmol) as a white solid. $^1H$ NMR (400 MHz, DMSO-*d6*) δ 7.48 (br s, 1H), 7.37 (t, *J* = 53.7 Hz, 1H), 3.61 (s, 3H). LCMS m/z $[M+H]^+$ 297.9.

**Example i-6. Intermediate A-6 (7-bromo-4-chloro-3-fluoro-6-methyl-pyrazolo[1,5-a]pyrazine)**

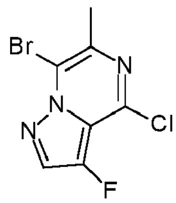

**Step a:** To a mixture of 4-fluoro-1H-pyrazole (5 g, 58 mmol) in DMSO (110 mL) was added cesium carbonate (2.8 g, 87 mmol) and then 1-bromo-2,2-dimethoxy-propane (11 g, 8.2 mL, 61 mmol) at RT. The reaction mixture was stirred at 120 °C for 5 days. The reaction mixture was poured into water (600 mL) and the aqueous phase was extracted with ethyl acetate (2 * 150 mL). The combined organic phase was washed with water, brine, dried with anhydrous $Na_2SO_4$, filtered and concentrated under vacuum to give a yellow oil which was purified by column chromatography ($SiO_2$, heptane/ethyl acetate = 85/15) to give 1-(2,2-dimethoxypropyl)-4-fluoro-pyrazole (7.69 g) as a colorless oil. $^1H$ NMR (400 MHz, DMSO-*d6*) δ 7.76 (dd, *J* = 4.4 Hz, *J* =0.8 Hz 1H), 7.46 (dd, *J* = 4.4 Hz, *J* =0.8 Hz 1H), 4.13 (s, 2H), 3.18 (s, 6H), 1.08 (s, 3H).

**Step b:** To a solution of 1-(2,2-dimethoxypropyl)-4-fluoro-pyrazole (7.69 g, 40.9 mmol) in dry THF (150 mL) was added dropwise LDA (freshly prepared with 21 mL of nBuLi 2.5 M in hexanes and 7.5 mL of diisopropylamine in 10 mL of THF, 52.5 mmol) at -70 °C under $N_2$. The reaction mixture was stirred 1 h at -70 °C and then ethyl chloroformate (6.65 g, 61.3 mmol) was added dropwise at -70 °C. The reaction mixture was stirred 30 min and was then allowed to warm to RT. The reaction mixture was poured into a solution of $NH_4Cl$ and extracted with ethyl acetate (2 * 150 mL). The combined organic phase was washed with brine, dried with anhydrous $Na_2SO_4$, filtered and concentrated under vacuum to give an orange oil which was purified by column chromatography ($SiO_2$, heptane/ethyl acetate = 9/1) to give ethyl 2-(2,2-dimethoxypropyl)-4-fluoro-pyrazole-3-carboxylate (9.73 g) as a pale-yellow oil. $^1H$ NMR (400 MHz, DMSO-*d6*) δ 7.72 (d, *J* = 4.4 Hz, 1H), 4.59 (s, 2H), 4.33 (d, *J* = 7.2 Hz, 2H), 3.15 (s, 6H), 1.30 (m, 3H), 1.03 (s, 3H). LCMS m/z $[M+Na]^+$ 283.2.

**Step c:** To a solution ethyl 2-(2,2-dimethoxypropyl)-4-fluoro-pyrazole-3-carboxylate (9.73 g, 37.4 mmol) in a mixture THF/water 1/1 (25 mL) was added trifluoroacetic acid (4.26 g, 28.6 mL, 374 mmol). The reaction mixture was stirred at RT for 2 h. The reaction mixture was then concentrated under reduced pressure to give crude ethyl 2-acetonyl-4-fluoro-pyrazole-3-carboxylate as a white solid (7.95 g) that was used without further purification. $^1H$ NMR (400 MHz, DMSO-*d6*) δ 7.74 (s, 1H), 5.35 (s, 2H), 4.28 (m, 2H), 2.18 (s, 3H), 1.26 (m, 3H). LCMS m/z $[M+H]^+$ 215.1.

**Step d:** To a mixture of ethyl 2-acetonyl-4-fluoro-pyrazole-3-carboxylate (7.95 g, 37.1 mmol) in AcOH (10.6 mL) was added ammonium acetate (14.3 g, 186 mmol). The reaction mixture was stirred at reflux for 15 h. The mixture was concentrated under vacuum. Water (300 mL) and ethyl acetate (300 mL) were added, the aqueous layer was separated and extracted with DCM (300 mL). The combined organic phase was dried over $Na_2SO_4$, filtered and concentrated to dryness to give crude product which was triturated into diisopropylether (40 mL) and ethyl acetate (2 mL), iced and filtered to give 3-fluoro-6-methyl-5H-pyrazolo[1,5-a]pyrazin-4-one (5.03 g, 30.1 mmol) as a beige solid. $^1H$ NMR (400 MHz, DMSO-*d6*) δ 11.24 (s, 1H), 8.18 (s, 1H), 7.87 (s, 1H), 7.41 (s, 1H), 2.08 (s, 3H). LCMS m/z $[M+H]^+$ 168.1.

**Step e:** To a mixture of 3-fluoro-6-methyl-5H-pyrazolo[1,5-a]pyrazin-4-one (5.03 g, 30.1 mmol) in DMF (150 mL), cooled to 0 °C, was added NBS (5.62 g, 31.6 mmol). The reaction was stirred at 0 °C for 1 h. The reaction mixture was diluted with ethyl acetate (150 mL). Saturated aqueous solution of sodium thiosulfate (700 mL) was added and the mixture was stirred for 30 min at RT and then extracted with ethyl acetate (3 * 200 mL). A solid was filtrered from the interphase to furnish the crude product (1.1 g). The combined organic phase was dried over $Na_2SO_4$, filtered and concentrated to dryness to furnish a brown solid (4.9 g). The combined crude product obtained was triturated into acetonitrile (20 mL), iced and filtered to give 7-bromo-3-fluoro-6-methyl-5H-pyrazolo[1,5-a]pyrazin-4-one (5.17 g, 21 mmol). $^1H$ NMR (400 MHz, DMSO-*d6*) δ 11.63 (s, 1H), 8.03 (s, 1H), 2.25 (s, 3H). LCMS m/z $[M+H]^+$ 246.0.

**Step f:** A solution of ethyl 7-bromo-3-fluoro-6-methyl-5H-pyrazolo[1,5-a]pyrazin-4-one (5.3 g, 22 mmol) in $PCl_3$ (66 g, 40 mL, 430 mmol) was heated to reflux for 2 h. The residue was cooled down to RT and was carefully poured into iced water (800 mL) and the obtained mixture was stirred for 45 min. The aqueous phase was extracted with ethyl acetate (2 * 200 mL). The pH value of the aqueous phase was adjusted to ~7 with a saturated solution of $NaHCO_3$. The aqueous phase was extracted with ethyl acetate (300 mL * 2). The combined organic phase was washed with brine, dried with anhydrous $Na_2SO_4$, filtered and concentrated under vacuum to give crude product which was triturated into diisopropylether (40 mL), iced and filtered to give 7-bromo-4-chloro-3-fluoro-6-methyl-pyrazolo[1,5-a]pyrazine (4.95 g, 18.7 mmol) as a beige powder. $^1H$ NMR (400 MHz, DMSO-*d6*) δ 8.44 (s, 1H), 2.57 (s, 3H). LCMS m/z $[M+H]^+$ 264.0.

**Example i-7. Intermediate A-7 (ethyl 7-bromo-4-chloro-6-methyl-pyrazolo[1,5-a]pyrazine-3-carboxylate)**

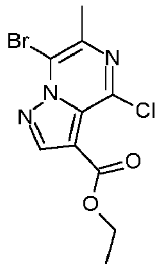

**Step a:** To a mixture of ethyl 1H-pyrazole-4-carboxylate (15 g, 107.0 mmol) in DMSO (210 mL) was added potassium carbonate (5.2 g, 161 mmol) and then 1-bromo-2,2-dimethoxy-propane (21 g, 15.90 mL, 117.7 mmol) at RT. The reaction mixture was stirred at 120 °C for 24 h The reaction mixture was poured into water (500 mL) and the aqueous phase was extracted with ethyl acetate (2 * 400 mL). The combined organic phase was washed with brine, dried with anhydrous $Na_2SO_4$, filtered and concentrated under vacuum to give an orange oil which was purified by column chromatography ($SiO_2$, heptane/ethyl acetate = 7/3) to give ethyl 1-(2,2-dimethoxypropyl)pyrazole-4-carboxylate (17.5 g) as a yellow oil. $^1$H NMR (400 MHz, DMSO-*d6*) δ 8.21 (s, 1H), 7.85 (s, 1H), 4.28 (s, 2H), 4.21 (q, *J*= 7.1 Hz, 2H), 3.20 (s, 6H), 1.27 (t, *J* = 7.1 Hz, 3H), 1.12 (s, 3H). LCMS m/z [M+H]$^+$ 243.2.

**Step b:** To a solution of ethyl 1-(2,2-dimethoxypropyl)pyrazole-4-carboxylate (8.5 g, 35.1 mmol) in dry THF (130 mL) was added dropwise LDA (2 M in THF, 23 mL, 46 mmol) at -70 °C under $N_2$. The reaction mixture was stirred 30 min at -70 °C and then ethyl chloroformate (5.71 g, 52.6 mmol) was added dropwise at -70 °C. The reaction mixture was stirred 30 min and was then allowed to warm to RT. The reaction mixture was poured into a solution of brine and extracted with ethyl acetate (2* 150 mL). The combined organic phase was washed with brine, dried with anhydrous $Na_2SO_4$, filtered and concentrated under vacuum to give an orange oil which was purified by column chromatography ($SiO_2$, heptane/ethyl acetate = 8/2) to give diethyl 2-(2,2-dimethoxypropyl)pyrazole-3,4-dicarboxylate (3.67 g, 11.7 mmol) as a yellow liquid. $^1$H NMR (400 MHz, DMSO-*d6*) δ 7.92 (s, 1H), 4.43 (s, 2H), 4.34 (q, *J* = 7.1 Hz, 2H), 4.21 (q, *J*= 7.1 Hz, 2H), 3.11 (s, 6H), 1.32 (t, *J* = 7.1 Hz, 3H), 1.25 (t, *J* = 7.1 Hz, 3H), 1.05 (s, 3H).

**Step c:** To a solution of diethyl 2-(2,2-dimethoxypropyl)pyrazole-3,4-dicarboxylate (3.67 g, 11.7 mmol) in a mixture THF/water 1/1 (20 mL) was added trifluoroacetic acid (2.66 g, 17.9 mL, 234 mmol). The reaction mixture was stirred at RT for 2 h The reaction mixture was concentrated under reduced pressure. The residue was purified by column chromatography ($SiO_2$, heptane/ethyl acetate = 6/4) to give diethyl 2-acetonylpyrazole-3,4-dicarboxylate as a colorless oil (2.18 g). $^1$H NMR (400 MHz, DMSO-*d6*) δ 7.92 (s, 1H), 5.37 (s, 2H), 4.21-4.31 (m, 4H), 2.17(s, 3H), 1.24-1.28 (m, 6H). LCMS m/z [M+H]$^+$ 269.2.

**Step d:** To a mixture of diethyl 2-acetonylpyrazole-3,4-dicarboxylate (1.62 g, 4.7 mmol) in AcOH (1.34 mL) was added ammonium acetate (1.8 g, 23.4 mmol). The reaction mixture was stirred at reflux for 5 h. The mixture was cooled down to RT and then poured into water (300 mL) and stirred for 15 min. The solid was collected by filtration and washed with water to give crude ethyl 6-methyl-4-oxo-5H-pyrazolo[1,5-a]pyrazine-3-carboxylate (1.1 g) that was used into the next step without further purification as a beige solid. $^1$H NMR (400 MHz, DMSO-*d6*) δ 11.5 (s, 1H), 8.18 (s, 1H), 7.58 (s, 1H), 4.24 (q, *J* = 7.1 Hz, 2H), 2.12 (s, 3H), 1.28 (t, *J* = 7.1 Hz, 3H). LCMS m/z [M+H]$^+$ 222.1.

**Step e:** To a mixture of ethyl 6-methyl-4-oxo-5H-pyrazolo[1,5-a]pyrazine-3-carboxylate previously obtained (1.1 g) inDMF (25 mL), cooled to 0 °C, was added NBS (929 mg, 5.2 mmol). The reaction was stirred at 0 °C for 1 h. Ethyl acetate and a saturated aqueous solution of sodium thiosulfate were added and the mixture was stirred for 30 min at RT. The precipitate was collected by filtration and washed with water to give crude ethyl 7-bromo-6-methyl-4-oxo-5H-pyrazolo[1,5-a]pyrazine-3-carboxylate (1 g) that was used into the next step without further purification as a pink solid. $^1$H NMR (400 MHz, DMSO-*d6*) δ 11.9 (s, 1H), 8.28 (s, 1H), 4.26 (q, *J* = 7.1 Hz, 2H), 2.29 (s, 3H), 1.30 (t, *J* = 7.1 Hz, 3H). LCMS m/z [M+H]$^+$ 300.0.

**Step f:** A solution of crude ethyl 7-bromo-6-methyl-4-oxo-5H-pyrazolo[1,5-a]pyrazine-3-carboxylate (1.14 g) in $PCl_3$ (16.4 g, 10 mL, 106.2 mmol) was heated to reflux for 2 h. The residue was carefully poured into iced water (500 mL). The pH value of the aqueous phase was adjusted to ~7 with saturated $NaHCO_3$. The aqueous phase was extracted with ethyl acetate (300 mL * 2). The combined organic phase was washed with saturated aqueous solution of $NaHCO_3$, brine, dried with anhydrous $Na_2SO_4$, filtered and concentrated under vacuum. The residue was triturated into diisopropylether, iced and filtered to give ethyl 7-bromo-4-chloro-6-methyl-pyrazolo[1,5-a]pyrazine-3-carboxylate (1.11 g, 3.5 mmol) as a beige powder. $^1$H NMR (400 MHz, DMSO-*d6*) δ 8.68 (s, 1H), 4.35 (q, *J* = 7.1 Hz, 3H), 2.62 (s, 3H), 1.34 (t, *J* = 7.1 Hz, 3H). LCMS m/z [M+H]$^+$ 318.0.

**Example i-8. Intermediate A-8 ((7-bromo-4-chloro-6-methyl-pyrazolo[1,5-a]pyrazin-3-yl)methanol)**

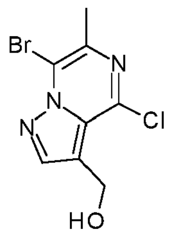

To a solution of **intermediate A-7** (980 mg, 3.47 mmol) in anhydrous THF (40 mL), cooled at -78 °C, was added dropwise DIBAL-H (1 M in toluene, 6.9 mL, 6.9 mmol). The reaction mixture was stirred at -78 °C for 1.5 h and then allowed to warm to 0 °C. A saturated aqueous solution of Rochelle salt (10 mL) was added and then water (40 mL) and ethyl acetate (40 mL). The mixture was stirred at RT for 10 min. The organic layer was separated, and the aqueous phase was extracted with ethyl acetate (40 mL). The combined organic phase was washed with brine, dried with anhydrous $Na_2SO_4$, filtered and concentrated under vacuum to give 7-bromo-4-chloro-6-methyl-pyrazolo[1,5-a]pyrazin-2-yl)methanol (980 mg, 3.47 mmol) which was used in the next step without any further purification as a white solid. $^1$H NMR (400 MHz, DMSO-*d6*) δ 8.24 (s, 1H), 5.28 *(t, J* = 5.4 Hz, 1H), 4.85 (d, *J* = 5.4 Hz, 2H), 2.57 (s, 3H). LCMS m/z $[M+H]^+$ 276.0.

**Example i-9. Intermediate A-9 (7-bromo-4-chloro-3-(difluoromethyl)-6-methyl-pyrazolo[1,5-a]pyrazine)**

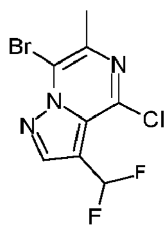

Step a: To a mixture of **intermediate A-8** (400 mg, 1.45 mmol) in dichloromethane (20 mL), cooled to 0 °C, was added portionwise Dess-Martin periodinane (736 mg, 1.74 mmol). The reaction was stirred at 0 °C for 10 min and then 1 hat RT. An additional portion of Dess-Martin periodinane (150 mg) was added. Saturated aqueous solutions of sodium thiosulfate (20 mL), sodium bicarbonate (20 mL) and dichloromethane (20 mL) were added and the mixture was stirred for 1 h. The mixture was filtered on a hydrophobic PTFE cartridge (liquid / liquid extraction column, Radleys®) and then concentrated in vacuum to give crude 7-bromo-4-chloro-6-methyl-pyrazolo[1,5-a]pyrazine-3-carbaldehyde (506 mg) that was used into the next step without further purification as a beige solid. $^1$H NMR (400 MHz, DMSO-*d6*) δ 10.48 (s, 1H), 8.80 (s, 1H), 2.65 (s, 3H). LCMS m/z $[M+H]^+$ 276.0.

**Step b:** To a mixture of crude 7-bromo-4-chloro-6-methyl-pyrazolo[1,5-a]pyrazine-3-carbaldehyde previously obtained in dichloromethane (20 mL), cooled at -20 °C, was added dropwise diethylaminosulfur trifluoride (1.2 mL, 8.66 mmol). The reaction mixture was stirred at -20 °C for 2 h and then at RT for 48 h. The mixture was poured to an iced saturated aqueous solution of sodium bicarbonate (20 mL) under vigorous stirring to adjust the pH value of the aqueous phase to ~8. Water (20 mL) and dichloromethane (30 mL) were added and the mixture was filtered on a hydrophobic PTFE cartridge (liquid / liquid extraction column, Radleys®) and then concentrated in vacuum. The residue was purified by column chromatography ($SiO_2$, heptane/ethyl acetate = 90/10) to give 7-bromo-4-chloro-3-(difluoromethyl)-6-methyl-pyrazolo[1,5-a]pyrazine (345 mg, 1.16 mmol) as a white powder. $^1$H NMR (400 MHz, DMSO-*d6*) δ 8.61 (s, 1H), 7.55 (t, *J* = 54.7 Hz, 1H), 2.61 (s, 3H). LCMS m/z $[M+H]^+$ 298.0.

**Example i-10. Intermediate B-1 (spiro[7H-cyclopenta[b]pyridine-6,4'-piperidine]-5-one hydrochloride)**

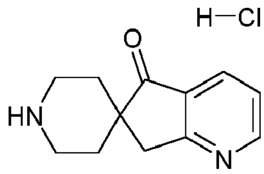

To a mixture of tert-butyl 5-oxo-5,7-dihydrospiro[cyclopenta[b]pyridine-6,4'-piperidine]-1'-carboxylate (Labnetwork, 20 g, 66.14 mmol) in dichloromethane (200 mL) and methanol (100 mL) was added dropwise a solution of HCl (4 M in dioxane, 165.3 mL, 661.4 mmol) at RT. The mixture was stirred for 18 h and then concentrated under vacuo. The residue was taken up in ethyl acetate (50 mL) and stirred for 5 min. The precipitate was collected by filtration and washed with pentane (50 mL) to give spiro[7H-cyclopenta[b]pyridine-6,4'-piperidine]-5-one hydrochloride (18.1 g) as a white solid that was used without further purification $^1$H NMR (400 MHz, DMSO-*d6*) δ 9.43 (s, 1H), 9.12 (s, 1H), 8.92 (d, *J* = 3.2 Hz, 1H), 8.17 (d, *J* = 8.0 Hz, 1H), 7.58 (dd, *J* = 8.0, 5.2 Hz, 1H), 3.30-3.37 (m, 4H), 3.01-3.10 (m, 2H), 1.95-2.03 (m, 2H), 1.67-1.70 (m, 2H).

**Example i-11. Intermediate B-2 (tert-butyl (5S)-5-[[(R)-tert-butylsulfinyl]amino]spiro[5,7-dihydrocyclopenta[b]pyridine-6,4'-piperidine]-1'-carboxylate)**

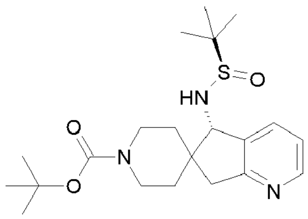

**Step a:** To a solution of tert-butyl 5-oxo-5,7-dihydrospiro[cyclopenta[b]pyridine-6,4'-piperidine]-1-carboxylate (Labnetwork, 14 g, 46.30 mmol) in 2-methyltetrahydrofuran (100 mL) was added (*R*)-2-methylpropane-2-sulfinamide (11.45 g, 92.59 mmol). The mixture was heated at 60 °C and $Ti(OEt)_4$ (58 mL, 185.2 mmol) was added dropwise. The mixture was stirred at 80 °C for 18 h. The mixture was cooled to RT and 2-methyltetrahydrofuran (150 mL), 5% aqueous solution of $Na_2SO_4$ (100 mL) and dicalite (15 g) were added. The mixture was stirred for 30 min and then filtered, and the solid was washed with 2-methyltetrahydrofuran. The filtrate was then washed with water (6 * 300 mL), brine (200 mL), dried over $Na_2SO_4$, filtered and concentrated under reduced pressure. The residue was purified by column chromatography ($SiO_2$, heptane/ethyl acetate= 1/0 to 0/1) to give tert-butyl (5Z)-5-[(R)-tert-butylsulfinyl]iminospiro[7H-cyclopenta[b]pyridine-6,4'-piperidine]-1'-carboxylate (15.42 g, 38.02 mmol) as an off-white solid. LCMS m/z $[M+H]^+$ 406.2.

**Step b:** To a mixture of tert-butyl (5Z)-5-[(R)-tert-butylsulfinyl]iminospiro[7H-cyclopenta[b]pyndine-6,4'-piperidine]-1'-carboxylate (5.7 g, 14.07 mmol) in anhydrous THF (115 mL), cooled at -78 °C, was added dropwise DIBAL-H (1 M in toluene, 17 mL, 17 mmol). The reaction mixture was stirred at -78 °C for 15 min and ethyl acetate (140 mL) was added followed by a saturated aqueous solution of Rochelle salt (100 mL). The cooling batch was removed and the mixture was stirred at RT for 1 h. The aqueous layer was separated and then extracted with ethyl acetate (50 mL). The combined organic phase was washed with brine, dried with anhydrous $Na_2SO_4$, filtered and concentrated under vacuum. The residue was purified by column chromatography ($SiO_2$, dichloromethane/methanol = 100/0 to 95/5) to give tert-butyl (5S)-5-[[(R)-tert-butylsulfinyl]amino]spiro[5,7-dihydrocyclopenta[b]pyridine-6,4'-piperidine]-1'-carboxylate (5.2 g, 12.8 mmol) as a white solid. LCMS m/z $[M+H]^+$ 408.2.

**Example i-12. Intermediate B-3 ((R)-2-methyl-N-[(5S)-spiro[5,7-dihydrocyclopenta[b]pyridine-6,4'-piperidine]-5-yl] propane-2-sulfinamide)**

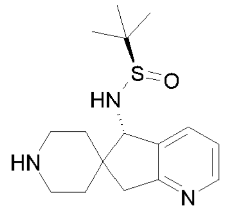

To a solution of **intermediate B-2** (0.7 g, 1.71 mmol) in dichloromethane (5 mL) was added TFA (1.5 mL, 20 mmol). The mixture was stirred at RT for 3 h. Dichloromethane (10 mL) and water (10 mL) were added, the pH value of the aqueous phase was adjusted to 11-12 with 1 N aqueous NaOH solution and the mixture was filtered on a hydrophobic cartridge (liquid / liquid extraction column, Radleys®) and then concentrated in vacuum to give (R)-2-methyl-N-[(5S)-spiro[5,7-dihydrocyclopenta[b]pyridine-6,4'-piperidine]-5-yl]propane-2-sulfinamide (0.34 g, 1.12 mmol) as a beige solid. LCMS m/z $[M+H]^+$ 308.2.

**Example i-13. Intermediate B-4 ((5S)-spiro[5,7-dihydrocyclopenta[b]pyridine-6,4'-piperidine]-5-amine hydrochloride)**

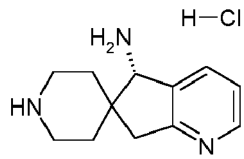

A mixture of **intermediate B-2** (2.35 g, 5.77 mmol) in a solution of HCl (2.5 M in ethanol, 30 mL, 75 mmol) was stirred at RT for 3 h. The reaction mixture was then concentrated under vacuum. The residue was taken up in ethyl acetate (50 mL) and diisopropyl ether (50 mL), triturated, filtered and washed with diisopropyl ether (2 * 50 mL), pentane (50 mL) to give to (5S)-spiro[5,7-dihydrocyclopenta[b]pyridine-6,4'-piperidine]-5-amine hydrochloride (1.8 g, crude) as a white solid that was used without further purification.

**Example i-14. Intermediate B-5 (3-chlorospiro[7H-cyclopenta[b]pyridine-6,4'-piperidine]-5-one hydrochloride)**

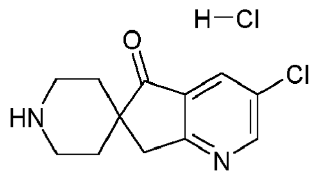

**Step a:** To a solution of (3-bromo-5-chloropyridin-2-yl)methanol (1.25 g, 5.62 mmol) in anhydrous dichloromethane (15 mL) was added triethylamine (1.7 mL, 12.2 mmol) followed by methanesulfonyl chloride (0.5 mL, 6.4 mmol) at -15 °C. After addition, the mixture was stirred at this temperature for 1 h, water (20 mL) was added and the mixture filtered on a hydrophobic cartridge (liquid / liquid extraction column, Radleys®) and then concentrated in vacuum to give (3-bromo-5-chloro-2-pyridyl)methyl methanesulfonate (1.2 g, 3.99 mmol) as an oil. LCMS m/z $[M+H]^+$ = 301.8.

**Step b:** To a solution of ethyl N-Boc-piperidine-4-carboxylate (2.48 g, 9.64 mmol) in anhydrous THF (15 mL) was added dropwise LDA (2 M, 6 mL, 12 mmol) at -78 °C. After addition, the mixture was stirred at this temperature for 1.5 h, and (3-bromo-5-chloro-2-pyridyl)methyl methanesulfonate (2.85 g, 9.48 mmol) in anhydrous THF (6 mL) was added dropwise. The resulting mixture was then let to warm slowly to 0 °C. The reaction mixture was quenched by addition of saturated aqueous solution of $NH_4Cl$ (10 mL). Water (30 mL) was added and the mixture extracted with EtOAc (40 mL * 2). The combined organic layers were washed with brine, dried over $Na_2SO_4$, filtered and concentrated under reduced pressure. The residue was purified by column chromatography ($SiO_2$, cyclohexane/ethyl acetate = 95/5) to give Ol-tert-butyl 04-ethyl 4-[(3-bromo-5-chloro-2-pyridyl)methyl]piperidine-1,4-dicarboxylate (3.23 g, 6.99 mmol) as an orange oil. LCMS m/z $[M-56+H]^+$ = 406.9.

**Step c:** To a solution of Ol-tert-butyl O4-ethyl 4-[(3-bromo-5-chloro-2-pyridyl)methyl]piperidine-1,4-dicarboxylate (3.22 g, 6.97 mmol) in methanol (30 mL) and water (6 mL) was added an aqueous solution of sodium hydroxyde (35%, 6 mL, 72.12 mmol) at RT. The mixture was stirred at 65 °C for 18 h and then let to cool down to RT. Water was added and the mixture filtered; the precipitate was washed with acetonitrile to give sodium salt of 4-[(3-bromo-5-chloro-2-pyridyl)methy1] -1-tert-butoxycarbonyl-piperidine-4-carboxylate (1.96 g, 4.30 mmol) as a white solid.

**Step d:** To a suspension of sodium salt of 4-[(3-bromo-5-chloro-2-pyridyl)methyl]--tert-butoxycarbonyl-piperidine-4-carboxylate (1.96 g, 4.30 mmol) in anhydrous THF (10 mL) was added dropwise n-BuLi (2.1 M in hexanes, 3 mL, 6.3 mmol) at -20 °C. After the end of the addition, the mixture was stirred at this temperature for 1 h and the reaction mixture was quenched by addition of water (40 mL) and then extracted with EtOAc (40 mL x 2). The combined organic layer was washed with brine, dried over $Na_2SO_4$, filtered and concentrated under reduced pressure. The residue was purified by column chromatography ($SiO_2$, cyclohexane/ethyl acetate = 8/2) to give tert-butyl 3-chloro-5-oxo-spiro[7H-cyclopenta[b]pyridine-6,4'-piperidine]-1'-carboxylate (175 mg, 0.52 mmol) as a white solid. LCMS m/z $[M+H]^+$ = 337.1.

**Step e:** To a mixture of tert-butyl 3-chloro-5-oxo-spiro[7H-cyclopenta[b]pyridine-6,4'-piperidine]-r-carboxylate (390 mg, 1.16 mmol) in methanol (10 mL) was added dropwise a solution of HCl (4 M in dioxane, 1.5 mL, 6 mmol). The mixture was stirred for 16 h at RT and then concentrated under vacuo to give crude 3-chlorospiro[7H-cyclopenta[b]pyridine-6,4'-piperidine]-5-one hydrochloride (352 mg) as a pink solid that was used without further purification.

**Example i-15. Intermediate B-6 ((5S)-3-methoxyspiro[5,7-dihydrocyclopenta[b]pyridine-6,4'-piperidine]-5-amine hydrochloride)**

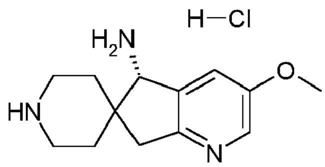

**Step a:** To a solution of 3-bromo-2-chloro-5-methoxypyridine (15.0 g, 67.4 mmol) in THF (150 mL) was added i-PrMgCl (2 M in THF, 40.5 mL, 91 mmol) dropwise at 0 °C under $N_2$. The reaction was stirred at RT for 2 h. Then to the solution was added a solution of *tert*-butyl 4-fomyl-4-methylpiperidine-1-carboxylate (23.0 g, 101 mmol) in THF (75 mL) dropwise at 0 °C. The reaction was stirred at RT for 30 min. The mixture was then poured into water (200 mL). The aqueous phase was extracted with ethyl acetate (80 mL * 3). The combined organic phase was washed with brine (50 mL), dried with anhydrous $Na_2SO_4$, filtered and concentrated under vacuum. The residue was purified by column chromatography ($SiO_2$, petroleum ether/ethyl acetate = 50/1 to 1/1) to give crude tert-butyl 4-((2-chloro-5-methoxypyridin-3-yl)(hydroxy)methyl)-4-methylpiperidine-1-carboxylate (26.0 g) as yellow oil that was used without further purification.

**Step b:** To a solution of crude tert-butyl 4-((2-chloro-5-methoxypyridin-3-yl)(hydroxy)methyl)-4-methylpiperidine-1-carboxylate (26.0 g) in DCM (260 mL) was added Dess-Martin periodinane (59.5 g, 140 mmol) at 0 °C under $N_2$. The reaction was stirred at RT for 3 h. The mixture was poured into saturated aqueous solution of sodium sulfite (300 mL). The aqueous phase was extracted with ethyl acetate (80 mL * 3). The combined organic phase was washed with brine (100 mL), dried with anhydrous $Na_2SO_4$, filtered and concentrated under vacuum. The residue was purified by column chromatography ($SiO_2$, petroleum ether/ethyl acetate = 50/1 to 1/1) to give tert-butyl 4-(2-chloro-5-methoxynicotinoyl)-4-methylpiperidine-1-carboxylate (22.0 g, 59.6 mmol) as yellow oil. $^1H$ NMR (400 MHz, $CDCl_3$) $\delta$ 8.10 *(d, J* = 3.2 Hz, 1H), 6.99 *(d, J*= 2.8 Hz, 1H), 3.88 (s, 3H), 3.69-3.75 (m, 2H), 3.21-3.28 (m, 2H), 1.96-2.03 (m, 2H), 1.57-1.61 (m, 2H), 1.45 (s, 9H), 1.36 (s, 3H).

**Step c:** To a solution of tert-butyl 4-(2-chloro-5- methoxynicotinoyl)-4-methylpiperidine-1-carboxylate (22.0 g, 59.6 mmol) in mesitylene (220 mL) was added $Pd(OAc)_2$ (670 mg, 2.98 mmol), $Cs_2CO_3$ (23.4 g, 71.6 mmol), tricyclohexylphosphonium tetrafluoroborate (2.20 g, 5.96 mmol) and pivalic acid (2.06 mL, 17.9 mmol) at RT under $N_2$. The solution was degassed with $N_2$ for 10 min. The reaction was then stirred at 160 °C for 4 h. The reaction was cooled down to RT and then poured into water (500 mL). The aqueous phase was extracted with ethyl acetate (150 mL * 3). The combined organic phase was washed with brine (150 mL), dried with anhydrous $Na_2SO_4$, filtered and concentrated under vacuum. The residue was purified by column chromatography ($SiO_2$, petroleum ether/ethyl acetate = 50/1 to 1/1) to give tert-butyl 3-methoxy-5-oxo-5,7-dihydrospiro[cyclopenta[*b*]pyridine- 6,4'-piperidine]-1-carboxylate (15.0 g, 45.1 mmol) as a yellow solid. $^1H$ NMR (400 MHz, $CDCl_3$) $\delta$ 8.57 (d, *J* = 2.8 Hz, 1H), 7.43 (d, *J* = 2.8 Hz, 1H), 4.15 (s, 2H), 3.89 (s, 3H), 3.12 (s, 2H), 3.02 (s, 2H), 1.90-1.98 (m, 2H), 1.41-1.49 (m, 11H).

**Step d:** To a solution of tert-butyl 3-methoxy-5-oxo-5,7-dihydrospiro[cyclopenta[*b*]pyridine- 6,4'-piperidine]-1-carboxylate (14.0 g, 21.1 mmol) in$Ti(OEt)_4$ (70.0 mL) was added (R)-2-methylpropane-2-sulfinamide (12.7 g, 105.0 mmol) at RT under $N_2$. The reaction was stirred at 110 °C for 13 h. The mixture was poured into water (500 mL). The

aqueous phase was extracted with ethyl acetate (200 mL * 3). The combined organic phase was washed with brine (200 mL), dried with anhydrous $Na_2SO_4$, filtered and concentrated under vacuum. The residue was purified by column chromatography ($SiO_2$, petroleum ether/ethyl acetate = 50/1 to 1/1) to give tert-butyl ((R,Z)-5-((*tert*-butylsulfinyl)imino)-3-methoxy-5,7-dihydrospiro[cyclopenta[*b*]pyridine-6,4'-piperidine]-1'-carboxylate (6.80 g, 15.6 mmol) as yellow oil. $^1H$ NMR (400 MHz, $CDCl_3$) δ 8.41-8.42 (m, 2H), 4.09-4.17 (m, 2H), 3.90 (s, 3H), 3.09 (s, 2H), 2.94 (s, 2H), 1.94-1.97 (m, 2H), 1.39-1.57 (m, 11H), 1.35(s, 9H).

**Step e:** To a solution of tert-butyl (R,Z)-5-((tert-butylsulfinyl)imino)-3-methoxy-5,7-dihydrospiro[cyclopenta[*b*]pyridine-6,4'-piperidine]-1'-carboxylate (6.8 g, 15.6 mmol) in THF (50 mL) was added DIBAL-H (1 M, 62.4 mL, 62.4 mmol) at -78 °C under $N_2$. The reaction was stirred at -78 °C for 1 h. Water (40 mL) was then slowly added and the mixture was stirred for 15 min. Anhydrous $Na_2SO_4$ (100 g) was added, the mixture was stirred for 5 min and then filtered. The filtrate was concentrated under vacuum and the residue was purified by column chromatography ($SiO_2$, petroleum ether/ethyl acetate = 50/1 to 1/1) to give tert-butyl 5-[[(R)-tert-butylsulfinyl]amino]-3-methoxy-spiro[5,7-dihydrocyclopenta[b]pyridine-6,4'-piperidine]-1'-carboxylate (3.40 g, 7.72 mmol) as a yellow solid. $^1H$ NMR (400 MHz, $CDCl_3$) δ 8.11-8.14 (m, 1H), 7.26 (s, 1H), 4.50 (d, *J*= 8.8 Hz, 1H), 4.01-4.04 (m, 2H), 3.86 (s, 3H), 3.63 (s, 1H), 2.81-3.14 (m, 4H), 2.05(s, 1H), 1.46-1.73 (m, 11H), 1.23-1.45 (m, 10H). LCMS m/z $[M+H]^+$ = 438.2.

**Step f:** To a solution oftert-butyl 5-[[(R)-tert-butylsulfinyl]amino]-3-methoxy-spiro[5,7-dihydrocyclopenta[b]pyridine-6,4'-piperidine]-1'-carboxylate (0.4 g, 0.91 mmol) in dichloromethane (6 mL) and methanol (2 mL) was added dropwise a solution of HCl (4 M in dioxane, 2 mL, 8 mmol) at RT. The mixture was stirred for 4 h and then concentrated under vacuo. The residue was taken up in diethyl ether (40 mL) and stirred for 5 min. The solid was collected by filtration and washed with diethyl ether to give 3-methoxyspiro[5,7-dihydrocyclopenta[b]pyridine-6,4'-piperidine]-5-amine hydrochloride (0.32 g, crude) as a yellow solid that was used without further purification.

**Example i-16. Intermediate B-7 ((5S)-3-fluorospiro[5,7-dihydrocyclopenta[b]pyiidine-6,4'-pipeiidine]-5-amine hydrochloride)**

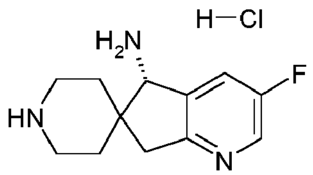

**Step a:** To a solution of 3-bromo-2-chloro-5-fluoropyridine (21.0 g, 99.8 mmol) in THF (420 mL) was added i-PrMgCl-LiCl (1.30 M in THF, 92.1 mL, 119.7 mmol) dropwise at 0 °C under $N_2$. The reaction mixture was stirred at RT for 2 h, then a solution of *tert*-butyl 4-formyl-4-methylpiperidine-1-carboxylate (29.5 g, 130 mmol) in THF (210 mL) was added dropwise at 0 °C. The reaction mixture was stirred for 30 min at RT and then poured into saturated aqueous ammonium chloride solution (800 mL). The aqueous phase was extracted with ethyl acetate (300 mL * 3). The combined organic phase was washed with brine (800 mL), dried with anhydrous $Na_2SO_4$, filtered and concentrated under vacuum. The residue was purified by column chromatography ($SiO_2$, petroleum ether/ethyl acetate = 100/1 to 10/1) to give tert-butyl 4-((2-chloro-5-fluoropyridin-3-yl)(hydroxy)methyl)-4-methylpipendine-1-carboxylate (26.7 g, 74.4 mmol) as yellow oil. $^1H$ NMR (400 MHz, $CDCl_3$) δ 8.19-8.21 (m, 1H), 7.67 (dd, *J* = 12 Hz, 1H), 4.91 (s, 1H), 3.95 (s, 2H), 2.86-2.94 (m, 3H), 1.79-1.80 (m, 1H), 1.54-1.61(m, 3H), 1.45-1.46 (m, 11H), 1.04-1.05 (m, 3H). LCMS m/z $[M-^tBu+H]^+$ = 303.0.

**Step b:** To a stirred solution of tert-butyl 4-((2-chloro-5-fluoropyridin-3-yl)(hydroxy)methyl)-4-methylpiperidine-1-carboxylate (26.7 g, 74.4 mmol) in DCM (160 mL) was added Dess-Martinperiodinane (58.1 g, 137 mmol) at 0 °C. The reaction mixture was stirred at RT for 3 h and then poured into a saturated aqueous solution of $Na_2S0_3$ (200 mL) and then diluted with DCM (150 mL). The suspension was filtered and the filtrate was extracted with DCM (100 mL * 3). The combined organic layers were washed with brine (300 mL), dried over $Na_2SO_4$, filtered and concentrated under reduced pressure. The residue was purified by column chromatography ($SiO_2$, Petroleum ether/Ethyl acetate= 100/1 to 10/1) to give tert-butyl 4-(2-chloro-5-fluororicotinoy1)-4 -methylpiperidine-1-carboxylate (13.9 g, 38.9 mmol) as a white solid. $^1H$ NMR (400 MHz, $CDCl_3$) d 8.33 (d, *J*= 2.8 Hz 1H), 7.27-7.30 (m, 1H), 3.72-3.78 (m, 2H), 3.25-3.31 (m, 2H), 1.98-2.05 (m,2H), 1.59-1.65 (m, 2H), 1.48 (s, 9H), 1.39 (s, 3H).

**Step c:** To a stirred solution of tert-butyl 4-(2-chloro-5-fluoronicotinoyl)-4-methylpiperidine-l-carboxylate (6.50 g, 18.2 mmol) in mesitylene (80 mL) were added tricyclohexylphosphonium tetrafluoroborate (671 mg, 1.82 mmol), pivalic acid (0.63 mL, 5.46 mmol) and $Cs_2CO_3$ (7.12 g, 21.9 mmol) and the mixture was degassed with $N_2$ for 5 min. $Pd(OAc)_2$ (204 mg, 0.91 mmol) was added to the reaction mixture and again degassed with $N_2$. The reaction mixture was stirred at 160 °C for 2 h. The reaction was cooled down to RT and then poured into water (150 mL). The aqueous

phase was extracted with ethyl acetate (60 mL * 3). The combined organic phase was washed with brine (150 mL), dried with anhydrous $Na_2SO_4$, filtered and concentrated under vacuum. The residue was purified by column chromatography ($SiO_2$, petroleum ether/ethyl acetate= 100/1 to 10/1) to give 3-fluoro-5-oxo-5,7-dihydrospiro[cyclopenta[b]pyridine-6,4'-piperidine]-1-carboxylate (4.50 g, 14.1 mmol) as a yellow solid.$^1$H NMR (400 MHz, $CDCl_3$) δ 8.71 (d, *J* = 3.6 Hz, 1H), 7.68 (s, *J* = 9.6 Hz 1H), 4.11-4.15 (m, 2H), 3.16 (s, 2H), 3.00-3.07 (m, 2H), 1.90-1.97 (m, 2H), 1.44-1.48 (m,12H).

**Step d:** To a stirred solution of 3-fluoro-5-oxo-5,7-dihydrospiro[cyclopenta[b]pyridine-6,4'-piperidine]-1-carboxylate (5.00 g, 15.6 mmol) in $Ti(OEt)_4$ (25.0 mL) was added (*R*)-2-methylpropane-2-sulfinamide (3.78 g, 31.2 mmol) at RT and the reaction mixture was then stirred at 100 °C for 16 h. The reaction mixture was poured into water (100 mL) and then diluted with ethyl acetate 80 mL, the suspension was filtered. The filtrate was extracted with ethyl acetate (30 mL * 3). The combined organic layers were washed with brine 150 mL, dried over $Na_2SO_4$, filtered and concentrated under vacuum. The residue was purified by column chromatography ($SiO_2$, petroleum ether/ethyl acetate = 100/1 to 10/1) to give tert-butyl (R,Z)-5-((tert-butylsulfinyl)imino)-3-fluoro-5,7-dihydrospiro[cyclopenta[b]pyridine-6,4'-piperidine]-1-carboxylate (5.80 g, 13.7 mmol) as a yellow solid. $^1$H NMR (400 MHz, $CDCl_3$) δ 8.65 (s, 1H), 8.56 *(d, J*= 2.8 Hz, 1H), 4.12-4.17 (m, 2H), 3.14 (s, 2H), 2.95-3.05 (m, 2H), 1.92-1.97 (m, 2H), 1.50 (s, 11H), 1.36 (s, 9H).

**Step e:** To a stirred solution of tert-butyl (R,Z)-5-((tert-butylsulfinyl)imino)-3-fluoro-5,7-dihydrospiro[cyclopenta[b]pyridine-6,4'-piperidine]-1-carboxylate (5.10 g, 12.0 mmol) in THF (26.0 mL) was added DIBAL-H (1 M, 48 mL, 48 mmol) at -70 °C and the mixture was stirred for 1 h. The reaction mixture was poured into water (100 mL) and the suspension was filtered. The filtrate was extracted with ethyl acetate (40 mL * 3). The combined organic layers were washed with brine (50 mL), dried over $Na_2SO_4$, filtered and concentrated under vacuum. The residue was purified by column chromatography ($SiO_2$, petroleum ether/ethyl acetate= 100/1 to 10/1) to give tert-butyl (5S)-5-(tert-butylsulfinylamino)-3-fluoro-spiro[5,7-dihydrocyclopenta[b]pyridine-6,4'-piperidine]-1'-carboxylate (2.5 g, 5.87 mmol) as a white solid. $^1$H NMR (400 MHz, $CDCl_3$) δ 8.31 (s, 1H), 7.40 (d, *J* = 5.6 Hz 1H), 4.54 (d, *J* = 9.2 Hz 1H), 4.04-4.15 (m, 2H), 3.68 (s, 1H), 3.18 (s, 1H), 2.87-2.96 (m, 3H), 2.05-2.13 (m, 1H), 1.45-1.73 (m, 12H), 1.29-1.49 (m, 9H). LCMS m/z $[M+H]^+$ = 426.2.

**Step f:** To a mixture of tert-butyl (5S)-5-(tert-butylsulfinylamino)-3-fluoro-spiro[5,7-dihydrocyclopenta[b]pyridine-6,4'-piperidine]-1'-carboxylate (1.5 g, 3.53 mmol) in methanol (10 mL) was added dropwise a solution of HCl (4 M in dioxane, 8.8 mL, 35.3 mmol) at 0 °C. The mixture was stirred for 1 h at RT, methanol (14 mL) was added and the mixture was stirred for 16 h. The mixture was then concentrated under vacuo to give crude (5S)-3-fluorospiro[5,7-dihydrocyclopenta[b]pyridine-6,4'-piperidine]-5-amine hydrochloride (1.24 g, crude) as a yellow solid that was used without further purification.

**Example i-17. Intermediate B-8 ((IS)-5-methoxyspiro[indane-2,4'-piperidine]-1-amine hydrochloride)**

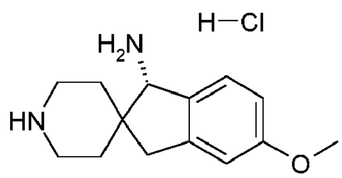

**Step a:** To a solution of 1-boc-4-cyanopiperidine (3.0 g, 14.27 mmol) in anhydrous THF (60 mL) was added dropwise LDA (2 M, 10 mL, 20 mmol) at -78 °C under Ar. After addition, the mixture was stirred at this temperature for 1 h, and 2-bromo-5-methoxybenzyl bromide (4.8 g, 17.27 mmol) was added dropwise. The resulting mixture was stirred at -78 °C for 3 h and then let to warm to 0 °C. The reaction mixture was quenched by addition of water (100 mL * 2), and then extracted with EtOAc (100 mL x 2). The combined organic layers were washed with brine, dried over $Na_2SO_4$, filtered and concentrated under reduced pressure. The residue was purified by column chromatography ($SiO_2$, cyclohexane/ethyl acetate = 95/5 to 80/20) to give tert-butyl 4-[(2-bromo-5-methoxy-phenyl)methyl]-4-cyanopiperidine-1-carboxylate (4.93 g, 12.0 mmol) as a yellow wax. LCMS m/z $[M-100+H]^+$ = 309.0.

**Step b:** A mixture of tert-butyl 4-[(2-bromo-5-methoxy-phenyl)methyl]-4-cyano-piperidine-1-carboxylate (4.93 g, 12 mmol), DIPEA (10 mL, 57.4 mmol), $Pd(AmPhos)_2Cl_2$ (0.86 g, 1.21 mmol) in DMA (80 mL) and $H_2O$ (15 mL) was degassed with Ar for 3 min, then the mixture was stirred at 140 °C for 2 h. The reaction was cooled down to RT, water (350 mL) and EtOAc (350 mL) and then aqueous solution of HCl (37%, 3 mL) were added to reach pH ~2. The organic layer was separated and the aqueous layer extracted with EtOAc (350 mL). The combined organic layers were washed with brine, dried over $Na_2SO_4$, filtered and concentrated under reduced pressure. The residue was taken up in diethyl ether (50 mL), the etheral layer was washed with water (50 mL * 3), dried over $Na_2SO_4$, filtered and concentrated under reduced pressure to give crude methoxy-1-oxo-spiro[indane-2,4'-piperidine]-1'-carboxylate (3.72 g) as an orange solid

that was used without further purification.

**Step c:** A mixture of crude methoxy-1-oxo-spiro[indane-2,4'-piperidine]-1'-carboxylate (3.72 g), $Ti(OEt)_4$ (13.0 mL, 62.01 mmol) and (R)-2-methylpropane-2-sulfinamide (3.0 g, 24.75 mmol) was stirred at 105 °C for 16 h. $Ti(OEt)_4$ (3.0 mL, 14.31 mmol) was added and the mixture was stirred at 105 °C for 24 h and then to cool down to RT. Water (150 mL) and ethyl acetate (100 mL) were added and the mixture was filtered. The organic layer was separated and the aqueous layer extracted with ethyl acetate. The combined organic phase was washed with brine, dried over $Na_2SO_4$, filtered and concentrated under vacuum. The residue was purified by column chromatography ($SiO_2$, cyclohexane/ethyl acetate = 8/2 to 6/4) to give crude tert-butyl (1Z)-1-[(R)-tert-butylsulfinyl]imino-5-methoxy-spiro[indane-2,4'-piperidine]-1'-carboxylate (1.01 g) as a yellow solid. LCMS m/z $[M+H]^+$ = 435.3.

**Step d:** To a mixture of crude tert-butyl (1Z)-1-[(R)-tert-butylsulfinyl]imino-5-methoxy-spiro[indane-2,4'-piperidine]-1'-carboxylate (1.0 g) in anhydrous THF (35 mL), cooled at -78 °C, was added dropwise DIBAL-H (1 M in toluene, 6 mL, 6 mmol). The reaction mixture was stirred at -78 °C for 45 min and then let warm to -20 °C. Saturated aqueous solution of Rochelle salt (10 mL) was added and the mixture was stirred at RT for 1 h. Water and ethyl acetate were added. The organic layer was separated and the aqueous phase was extracted with ethyl acetate. The combined organic phase was washed with brine, dried with anhydrous $Na_2SO_4$, filtered and concentrated under vacuum to give crude tert-butyl (1S)-1-[[(R)-tert-butylsulfinyl]amino]-5-methoxy-spiro[indane-2,4'-piperidine]-1'-carboxylate (984 mg) as a yellow solid. LCMS m/z $[M+H]^+$ = 437.3.

**Step e:** To a mixture of crude crude tert-butyl (1S)-1-[[(R)-tert-butylsulfinyl]amino]-5-methoxy-spiro[indane-2,4'-piperidine]-1'-carboxylate (984 mg) in dichloromethane (12 mL) and methanol (4 mL) was added dropwise a solution of HCl (4 M in dioxane, 3.5 mL, 14 mmol). The mixture was stirred for 16 h at RT and then concentrated under vacuo. The residue was taken up in diethyl ether (40 mL) and filtered to give crude (1S)-5-methoxyspiro[indane-2,4'-piperidine]-1-amine hydrochloride (755 mg) as a yellow solid that was used without further purification.

**Example i-18. Intermediate B-9 (2-methoxy-5,7-dihydrospiro[cyclopenta[b]pyridine-6,4'-piperidin]-7-amine hydrochloride)**

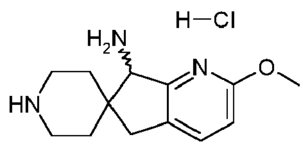

**Step a:** To a solution of 2-bromo-6-methoxynicotinaldehyde (42.0 g, 194 mmol) in MeOH (294 mL) was added $NaBH_4$ (3.64 g, 96.2 mmol) at RT under $N_2$. Then the reaction was stirred for 30 min at RT. The residue was poured into water (500 mL). The aqueous phase was extracted with ethyl acetate (200 mL * 3). The combined organic phase was washed with brine (200 mL), dried with anhydrous $Na_2SO_4$, filtered and concentrated under vacuum to give (2-bromo-6-methoxypyridin-3-yl)methanol (42.0 g, 193 mmol) as colourless oil. $^1H$ NMR (400 MHz, DMSO-*d6*) $\delta$ 7.76-7.80 (m, 1H), 6.87 (*d, J*= 8.0 Hz, 1H), 5.44 (t, *J* = 5.6 Hz, 1H), 4.43 (*d, J*= 5.6 Hz, 2H), 3.83 (s, 3H).

**Step b:** To a solution of (2-bromo-6-methoxypyridin-3-yl)methanol (42.0 g, 193 mmol) and $CBr_4$ (76.7 g, 231 mmol) in DCM (210 mL) was added a solution of $PPh_3$ (60.6 g, 231 mmol) in DCM (126 mL) at RT under $N_2$. The reaction was then stirred at RT for 30 min. The residue was poured into water (500 mL). The aqueous phase was extracted with DCM (200 mL * 3). The combined organic phase was washed with brine (200 mL), dried with anhydrous $Na_2SO_4$, filtered and concentrated under vacuum. The residue was purified by column chromatography ($SiO_2$, petroleum ether/ethyl acetate = 50/1 to 0/1) to give 2-bromo-3-(bromomethyl)-6-methoxypyridine (43.0 g, 153 mmol) as a white solid. $^1H$ NMR (400 MHz, DMSO-*d6*) $\delta$ 7.91-7.94 (m, 1H), 6.89-6.93 (m, 1H), 4.69 (s, 2H), 3.86 (s, 3H).

**Step c:** To a solution of 1-boc-4-cyanopiperidine (35.4 g, 168 mmol) in THF (215 mL) was added LDA (2.00 M, 153 mL, 306 mmol) at 0 °C under $N_2$. The reaction was stirred at 0 °C for 30 min. Then to the reaction was added a solution of 2-bromo-3-(bromomethyl)-6-methoxypyridine (43.0 g, 153 mmol) in THF (215 mL) at 0 °C under $N_2$. The reaction was stirred at RT for 2 h. The residue was poured into $NH_4Cl$ solution (1 L). The aqueous phase was extracted with ethyl acetate (500 mL * 3). The combined organic phase was washed with brine (100 mL), dried with anhydrous $Na_2SO_4$, filtered and concentrated under vacuum. The residue was purified by column chromatography ($SiO_2$, petroleum ether/ethyl acetate = 100/1 to 1/1). The solid obtained was stirred with petroleum ether (100 mL) at RT for 1 hr. The suspension was filtered and the filter cake was dried under vacuum to give tert-butyl 4-[(2-bromo-6-methoxypyridin-3-yl)methyl]-4-cyanopiperidine-1-carboxylate (17.0 g, 41.4 mmol) as a white solid. $^1H$ NMR (400 MHz, $CDCl_3$) $\delta$ 7.69 (d, *J*= 8.4 Hz, 1H), 6.74 (t, *J*= 8.4 Hz, 1H), 4.16 (s, 2H), 3.94 (s, 3H), 2.96-3.03 (m, 4H), 1.72-1.89 (m, 2H), 1.64-1.71 (m, 2H), 1.47 (s, 9H).

**Step d:** To a solution of tert-butyl 4-[(2-bromo-6-methoxypyridin-3-yl)methyl]-4-cyanopiperidine-1-carboxylate (17.0 g, 41.4 mmol) in DMA (170 mL) and $H_2O$ (17 mL) were added $Pd(AmPhos)_2Cl_2$ (2.93 g, 4.14 mmol, 2.93 mL) and TEA (16.8 g, 166 mmol, 23.1 mL) at RT under $N_2$. The reaction was stirred for 12 h at 120 °C under $N_2$. The residue was poured into water (600 mL). The aqueous phase was extracted with ethyl acetate (300 mL * 3). The combined organic phase was washed with brine (200 mL), dried with anhydrous $Na_2SO_4$, filtered and concentrated under vacuum. The residue was purified by column chromatography ($SiO_2$, petroleum ether/ethyl acetate = 50/1 to 1/1) to give tert-butyl 2-methoxy-7-oxo-5,7- dihydrospiro[cyclopenta[b]pyridine-6,4'-piperidine]-1'-carboxylate (12.0 g, 36.1 mmol) as a yellow solid. $^1H$ NMR (400 MHz, $CDCl_3$) $\delta$ 7.70 (d, *J* = 8.4 Hz, 1H), 6.98 (d, *J*= 8.4 Hz, 1H), 4.23 (s, 2H), 4.03 (s, 3H), 2.99-3.06 (m, 2H), 2.95 (s, 2H), 1.91-1.99 (m, 2H), 1.48 (s, 9H), 1.38-1.43 (m, 2H).

**Step e:** To a solution of tert-butyl 2-methoxy-7-oxo-5,7- dihydrospiro[cyclopenta[b]pyridine-6,4'-piperidine]-1-carboxylate (12.0 g, 36.1 mmol) and $Ti(OEt)_4$ (71.9 mL, 347 mmol) in 2-MeTHF (84 mL) was added (R)-2-methylpropane-2-sulfinamide (17.5 g, 144 mmol) at RT under $N_2$. The reaction was stirred at 90 °C for 16 h. The residue was poured into water (300 mL). The suspension was filtered and the filtrate extracted with ethyl acetate (150 mL * 3). The combined organic phase was washed with brine (100 mL), dried with anhydrous $Na_2SO_4$, filtered and concentrated under vacuum. The residue was purified by column chromatography ($SiO_2$, petroleum ether/ethyl acetate = 50/1 to 0/1) to give tert-butyl (7Z)-2-methoxy-7-{[(R)-2-methylpropane2-sulfinyl]imino}-5,7-dihydrospiro[cyclopenta[b]pyridine-6,4'-piperidine]-1'-carboxylate (8.00 g, 18.4 mmol) as a yellow solid. $^1H$ NMR (400 MHz, $CDCl_3$) $\delta$ 7.61-7.64 (m, 1H), 6.86-6.89 (m, 1H), 4.12 (s, 2H), 4.03 (s, 3H), 2.89-3.00 (m, 4H), 2.10-2.17 (m, 1H), 1.90-1.98 (m, 1H), 1.44-1.43 (m, 12H), 1.25-1.32 (m, 9H).

**Step f:** To a solution of tert-butyl (7Z)-2-methoxy-7-{[(R)-2-methylpropane2-sulfinyl]imino}-5,7-dihydrospiro[cyclopenta[b]pyridine-6,4'-piperidine]-1'-carboxylate (8.00 g, 18.4 mmol) in THF (56 mL) was added $NaBH_4$ (2.08 g, 55.1 mmol) at 0 °C under $N_2$. The reaction was stirred for 1 h at RT. The residue was poured into water (100 mL). The aqueous phase was extracted with ethyl acetate (75 mL * 3). The combined organic phase was washed with brine (75 mL), dried with anhydrous $Na_2SO_4$, filtered and concentrated in vacuum. The residue was purified by column chromatography ($SiO_2$, petroleum ether/ethyl acetate = 100/1 to 0/1) to give tert-butyl 2-methoxy-7-{[(R)-2-methylpropane-2-sulfinyl]amino}-5,7-dihydrospiro[cyclopenta[*b*]pyridine-6,4'-piperidine]-1'-carboxylate (2.00 g, 4.55 mmol) as a yellow solid and as a 3/2 mixture of diastereoisomers used without further purification LCMS m/z $[M+H]^+$ = 438.1.

**Step g:** To a 3/2 mixture of diastereoisomers of tert-butyl 2-methoxy-7-{[(R)-2-methylpropane-2-sulfinyl]aminol-5,7-dihydrospiro[cyclopenta[*b*]pyridine-6,4'-piperidine]-1'-carboxylate (875 mg, 2 mmol) in methanol (20 mL) was added dropwise a solution of HCl (4 M in dioxane, 3 mL, 12 mmol) at RT. The mixture was stirred for 4 h and then concentrated under vacuo to give 2-methoxy-5,7-dihydrospiro[cyclopenta[b]pyridine-6,4'-piperidin]-7-amine hydrochloride (800 mg, crude) as a yellow solid and as a mixture of enantiomers that was used without further purification. LCMS m/z $[M+H]^+$ = 234.2.

**Example i-19. Intermediate B-10 (3-aminospiro[indane-2,4'-piperidine]-5-carbonitrile hydrochloride)**

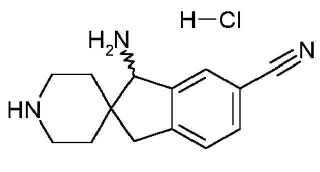

**Step a:** To a solution of 1-boc-4-cyanopiperidine (2.0 g, 9.51 mmol) in anhydrous THF (20 mL) was added dropwise LDA (2 M, 7 mL, 14 mmol) at -78 °C under Ar. After addition, the mixture was stirred at this temperature for 1 h, and 3-bromo-4-(bromomethyl)benzonitrile (2.65 g, 9.64 mmol) was added dropwise at -78 °C. The resulting mixture was stirred at -78 °C for 3 h and then let to warm to 0 °C. The reaction mixture was quenched by addition of saturated aqueous solution of $NH_4Cl$ (10 mL), and then extracted with EtOAc (50 mL * 2). The combined organic layers were washed with brine, dried over $Na_2SO_4$, filtered and concentrated under reduced pressure. The residue was purified by column chromatography ($SiO_2$, cyclohexane/ethyl acetate = 95/5 to 80/20) to give tert-butyl 4-[(2-bromo-4-cyano-phenyl)methyl]-4-cyano-piperidine-l-carboxylate (2.1 g, crude) as a white solid that was used without further purification.

**Step b:** A mixture of crude tert-butyl 4-[(2-bromo-4-cyano-phenyl)methyl]-4-cyano-piperidine-1-carboxylate (2.1 g), DIPEA (4.5 mL, 26 mmol), $Pd(AmPhos)_2Cl_2$ (0.35 g, 0.5 mmol) in DMA (30 mL) and $H_2O$ (5 mL) was degassed with Ar for 3 min, then the mixture was stirred at 140 °C for 2 h. The reaction was cooled down to RT, water (100 mL) and EtOAc (100 mL) and then aqueous solution of HCl (37%, 3 mL) were added. The organic layer was separated and the aqueous layer extracted with EtOAc (100 mL x 2). The combined organic layers were washed with brine, dried over $Na_2SO_4$, filtered and concentrated under reduced pressure. The residue was purified by column chromatography ($SiO_2$, cyclohexane/ethyl acetate = 95/5 to 80/20) to give tert-butyl 6-cyano-1-oxo-spiro[indane-2,4'-piperidine]-1'-carboxylate

(1.14 g, 3.49 mmol) as a white solid.

**Step c:** A mixture of tert-butyl 6-cyano-1-oxo-spiro[indane-2,4'-piperidine]-1'-carboaylate (680 mg, 2.08 mmol), $Ti(OEt)_4$ (6.0 mL, 28.62 mmol) and (R)-2-methylpropane-2-sulfinamide (770 mg, 6.35 mmol) was stirred at 100 °C for 1 h and then at RT for 16 h. Water and dichloromethane were added and the mixture was filtered on a hydrophobic cartridge (liquid / liquid extraction column, Radleys®) and then concentrated in vacuum. The residue was taken up in diethyl ether and water, the organic layer was separated, washed with brine, dried over $Na_2SO_4$, filtered and concentrated under vacuum to give crude tert-butyl (1Z)-1-[(R)-tert-butylsulfinyl]imino-6-cyano-spiro[indane-2,4'-piperidine]-1'-carboxylate (806 mg) as a yellow solid used without further purification.

**Step d:** To a mixture of crude tert-butyl (1Z)-1-[(R)tert-butylsulfinyl]imino-6-cyano-spiro[indane-2,4'-piperidine]-1'-carboxylate (250 mg) in anhydrous THF (7 mL), cooled at -50 °C, was added in one portion $NaBH_4$ (45 mg, 1.19 mmol). The reaction mixture was stirred at -50 °C for 30 min and then let to warm to 0 °C. A saturated aqueous solution of $NH_4Cl$ (3 mL) was slowly added and then water (20 mL) and ethyl acetate (20 mL). The organic layer was separated and the aqueous phase was extracted with ethyl acetate. The combined organic phase was washed with brine, dried with anhydrous $Na_2SO_4$, filtered and concentrated under vacuum to give crude tert-butyl 1-[[(R)-tert-butylsulfinyl]amino]-6-cyano-spiro[indane-2,4'-piperidine]-1'-carboxylate (260 mg) as a mixture of diastereoisomers that was without further purification.

**Step e:** To a mixture of crude tert-butyl 1-[[(R)-tert-butylsulfinyl]amino]-6-cyano-spiro[indane-2,4'-piperidine]-1-carboxylate (260 mg) in dichloromethane (4 mL) and methanol (1 mL) was added dropwise a solution of HCl (4 M in dioxane, 0.85 mL, 3.4 mmol) at 0 °C. The mixture was stirred for 16 h at RT, diethyl ether was added and the mixture filtered to give 3-aminospiro[indane-2,4'-piperidine]-5-carbonitrile hydrochloride (140 mg, crude), as a white solid, as a mixture of enantiomers that was used without further purification.

**Example i-20. Intermediate B-11 ((R)-2-methyl-N-[(7S)-spiro[5,7-dihydrocyclopenta[c]pyridine-6,4'-piperidine]-7-yl] propane-2-sulfinamide)**

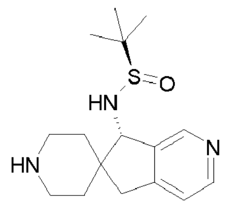

**Step a:** To a solution of 3-bromo-4-pyridinemethanol (40.0 g, 212 mmol) inDCM (200 mL) was added DMF (1.64 mL, 21.2 mmol) and then dropwise $SOCl_2$ (30.8 mL, 425 mmol) at RT under $N_2$. The mixture was stirred at 35 °C for 4 h. The reaction mixture was quenched by addition of an aqueous solution of $NaHCO_3$ (500 mL) at 10 °C, and extracted with EtOAc (100 mL * 3). The combined organic layers were washed with brine (200 mL), dried over $Na_2SO_4$, filtered and concentrated under reduced pressure to give 3-bromo-4-(chloromethyl)pyridine (35.4 g, 171 mmol) as a yellow oil. $^1H$ NMR (400 MHz, $CDCl_3$) $\delta$ 8.72 (s, 1H), 8.54 (d, *J* = 4.8 Hz, 1H), 7.46 (d, *J* = 4.8 Hz, 1H), 4.63 (s, 2H).

**Step b:** To a solution of 1-boc-4-cyanopiperidine (36.0 g, 171 mmol) in THF (720 mL) was added dropwise LDA (2 M, 111 mL, 222 mmol) at -78 °C. After addition, the mixture was stirred at this temperature for 1 h, and 3-bromo-4-(chloromethyl)pyridine (35.4 g, 171 mmol) was then added dropwise. The resulting mixture was stirred at - 78 °C for 2 h. The reaction mixture was quenched by addition of an aqueous solution of $NH_4Cl$ (500 mL) at 0 °C, and extracted with EtOAc (200 mL * 3). The combined organic layers were washed with brine (200 mL), dried over $Na_2SO_4$, filtered and concentrated under reduced pressure. The residue was purified by column chromatography ($SiO_2$, petroleum ether/ethyl acetate = 50/1 to 0/1) to give tert-butyl 4-[(3-bromo-4-pyridinyl)methyl]-4-cyanopiperidine-1-carboxylate (32.2 g, 84.6 mmol) as an off-white solid. $^1H$ NMR (400 MHz, $CDCl_3$) $\delta$ 8.76 (s, 1H), 8.51 (d, *J* = 4.8 Hz, 1H), 7.46 *(d, J*= 4.8 Hz, 1H), 4.17 (s, 2H), 3.10 (s, 2H), 2.99 (s, 2H), 1.85-1.88 (m, 2H), 1.64-1.71 (m, 2H), 1.46 (s, 9H).

**Step c:** A mixture of tert-butyl 4-[(3-bromo-4-pyridinyl)methyl]-4-cyanopiperidine-1-carboxylate (32.0 g, 84.1 mmol), DIPEA (58.6 mL, 336 mmol), $Pd(AmPhos)_2Cl_2$ (5.96 g, 8.41 mmol) in DMA (436 mL) and $H_2O$ (44 mL) was degassed with $N_2$, then stirred at 100 °C for 18 h under $N_2$ atmosphere. The reaction mixture was quenched by addition $H_2O$ (700 mL) at RT, and extracted with EtOAc (200 mL * 7). The combined organic layers were washed with brine (200 mL * 3), dried over $Na_2SO_4$, filtered and concentrated under reduced pressure. The crude product was triturated with MTBE (80 mL) at RT for 1 h and filtered to give tert-butyl 7-oxo-5,7-dihydrospiro[cyclopenta[c]pyridine-6,4'-piperidine]-1'carboxylate (21.0 g, 69.4 mmol) as a white solid. $^1H$ NMR (400 MHz, $CDCl_3$) $\delta$ 8.99 (s, 1H), 8.72 (d, *J*= 4.8 Hz, 1H), 7.43 (d, *J*= 4.8 Hz, 1H), 4.09 (s, 2H), 3.08 (s, 2H), 2.97-3.03 (m, 2H), 1.85-1.92 (m, 2H), 1.46 (s, 9H), 1.36-1.40 (m, 2H).

**Step d:** To a solution of tert-butyl 7-oxo-5,7- dihydrospiro[cyclopenta[*c*]pyridine-6,4'-piperidine]-1'-carboxylate (18.5 g, 61.2 mmol) in 2-MeTHF (130 mL) was added $Ti(OEt)_4$ (76.1 mL, 367 mmol) and (R)-2-methylpropane-2-sulfinamide (29.6 g, 244 mmol). The mixture was stirred at 80 °C for 16 h. The reaction mixture was quenched by addition $H_2O$ (200 mL) and filtered. The filter cake was washed with EtOAc (2.0 L), and the aqueous phase was extracted with EtOAc (200 mL * 3). The combined organic layers were washed with brine (200 mL), dried over $Na_2SO_4$, filtered and concentrated under reduced pressure. The crude product was triturated with MTBE (100 mL) at RT for 30 min to give tert-butyl 7-{[(R)-2-methylpropane-2-sulfinyl]imino}-5,7-dihydrospiro[cyclopenta[c]pyridine-6,4'-piperidine]-1'-carboxylate (20.0 g, 49.3 mmol) as a yellow solid. $^1H$ NMR (400 MHz, $CDCl_3$) δ 9.59 (s, 1H), 8.64 (d, *J* = 4.8 Hz, 1H), 7.36 (d, *J* = 4.8 Hz, 1H), 4.13 (s, 2H), 3.06 (s, 2H), 2.94 (s, 2H), 1.94-1.99 (m, 2H), 1.47 (s, 9H), 1.39-1.42 (m, 2H), 1.33 (s, 9H).

**Step e:** To a solution of tert-butyl 7-{[(R)-2-methylpropane-2-sulfinyl]imino}-5,7-dihydrospiro[cyclopenta[c]pyridine-6,4'-piperidine]-1'-carboxylate (20.0 g, 49.3 mmol) in THF (140 mL) was added dropwise DIBAL-H (1 M in toluene, 98.6 mL, 98.6 mmol) at -78 °C under $N_2$. The resulting mixture was stirred at -78 °C for 3 h. The reaction mixture was quenched by addition of an aqueous solution of $NH_4Cl$ (200 mL) at 0 °C and stirred at RT for 30 min, followed by filtration. The filter cake was washed with EtOAc (500 mL) and the aqueous phase was extracted with EtOAc (100 mL * 3). The combined organic layers were washed with brine (200 mL), dried over $Na_2SO_4$, filtered and concentrated under reduced pressure. The crude product was triturated with MTBE (80 mL) at RT for 1 h and filtered to give tert-butyl (7S)-7-[[(R)-tert-butylsulfinyl]amino]spiro[5,7-dihydrocyclopenta[c]pyridine-6,4'-piperidine]-1'-carboxylate (14.0 g, 33.8 mmol) as an off-white solid. $^1H$ NMR (400 MHz, $CDCl_3$) δ 8.55 (s, 1H), 8.45-8.48 (m, 1H), 7.17 (s, 1H), 4.58 (d, *J*= 9.6 Hz, 1H), 4.02 (d, *J*= 13.6 Hz, 1H), 3.58 (s, 1H), 2.68-3.07 (m, 4H), 2.11 (s, 1H), 1.74 (s, 2H), 1.44-1.60 (m, 11H), 1.28 (s, 9H).

**Step f:** To a mixture of tert-butyl (7S)-7-[[(R)-tert-butylsulfmyl]amino]spiro[5,7-dihydrocyclopenta[c]pyridine-6,4'-piperidine]-1'-carboxylate (900 mg, 2.14 mmol) in dichloromethane (7 mL) was added TFA (1.64 mL, 21.4 mmol) at RT. The mixture was stirred for 1 h and the mixture was poured into a 2 N aqueous solution of NaOH. The mixture was filtered on a hydrophobic cartridge (liquid / liquid extraction column, Radleys®) and then concentrated in vacuum to give (R)-2-methyl-N-[(7S)-spiro[5,7-dihydrocyclopenta[c]pyridine-6,4'-piperidine]-7-yl]propane-2-sulfinamide (663 mg, 2.09 mmol) as a white solid. $^1H$ NMR (400 MHz, DMSO-*d6*) δ 8.43 (s, 1H), 8.37 (d, *J* = 4.9 Hz, 1H), 7.25 (d, *J* = 4.8 Hz, 1H), 5.67 *(d, J* = 10.2 Hz, 1H), 4.42 (d, *J* = 10.2 Hz, 1H), 3.04 (d, *J* = 16.6 Hz, 1H), 2.85-2.89 (m, 2H), 2.60-2.70 (m, 3H), 1.78-1.85 (m, 1H), 1.58-1.65 (m, 1H), 1.37-1.40 (m, 1H), 1.13-1.21 (m, 10H). LCMS m/z $[M+H]^+$ = 308.1.

**Example i-21. Intermediate B-12 ((R)-2-methyl-N-[(3R)-spiro[3H-benzofuran-2,4'-piperidine]-3-yl]propane-2-sulfinamide)**

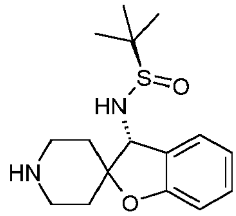

**Step a:** To a solution of 2-fluorobenzaldehyde (45.0 g, 362 mmol) inDCM (225 mL) was added 1,3-propanedithiol (36.3 mL, 362 mmol) and $I_2$ (2.76 g, 10.9 mmol). The mixture was stirred at RT for 4 h. The residue was poured into an aqueous solution of $Na_2S_2O_3$ (180 mL) and NaOH (150 mL). The mixture was extracted withDCM (180 mL * 3), the organic layer was dried over $Na_2SO_4$, filtered and concentrated under reduced pressure. The residue was purified by column chromatography ($SiO_2$, petroleum ether/ethyl acetate = 100/1 to 0/1) to give 2-(2-fluorophenyl)-1,3-dithiolane (41.0 g, 191 mmol) was obtained as a white solid. $^1H$ NMR (400 MHz, $CDCl_3$) δ 7.62-7.63 (m, 1H), 7.28-7.30 (m, 1H), 7.17-7.19 (m, 1H), 7.07-7.09 (m, 1H), 5.57 (s, 1H), 3.11-3.17 (m, 2H), 2.92-2.97 (m, 2H), 2.18-2.22 (m, 1H), 1.95-1.99 (m, 1H).

**Step b:** To a mixture of 2-(2-fluorophenyl)-1,3-dithiolane (18.0 g, 84.0 mmol) in THF (90 mL) was added slowly LDA (2 M in heptane/THF, 84.0 mL, 168 mmol) at -78 °C under $N_2$. The mixture was then stirred at -20 °C for 30 min, then cooled to -78 °C and tert-butyl 4-oxopiperidine-1-carboxylate (16.7 g, 84.0 mmol) was added. The mixture was stirred for 2 h at -78 °C. The mixture was quenched by addition of an aqueous solution of $NH_4Cl$ (180 mL). The mixture was extracted with EtOAc (180 mL * 3). The organic layer was dried over $Na_2SO_4$, filtered and concentrated under reduced pressure. The residue was purified by column chromatography ($SiO_2$, petroleum ether/ethyl acetate = 30/1 to 0/1) to give tert-butyl 4-[2-(2-fluorophenyl)-1,3-dithian-2-yl]-4-hydroxypipendine-1-carboxylate (16.0 g, 38.7 mmol) as a yellow solid. $^1H$ NMR (400 MHz, $CDCl_3$) δ 8.04-8.08 (m, 1H), 7.34-7.36 (m, 1H), 7.19-7.22 (m, 1H), 7.09-7.13 (m, 1H), 3.92 *(d, J*= 12 Hz, 2H), 2.99-3.00 (m, 2H), 2.82-2.86 (m, 2H), 2.64-2.68 (m, 2H), 2.60 (s, 1H), 1.85-1.90 (m, 2H), 1.77

(s, 4H), 1.42 (s, 9H).

**Step c:** A mixture of tert-butyl 4-[2-(2-fluorophenyl)-1,3-dithian-2-yl]-4-hydroxypiperidine-1-carboaylate (10.0 g, 24.2 mmol), TBAB (2.34 g, 7.25 mmol), Py.$HBr_3$ (11.6 g, 36.3 mmol) and pyridine (5.85 mL, 72.5 mmol) in DCM (10 mL) and $H_2O$ (2.5 mL) was stirred at RT for 8 h. Water (50 mL) was added and the mixture extracted with DCM (50 mL * 3), the organic layer was dried over $Na_2SO_4$, filtered and concentrated under reduced. The residue was purified by column chromatography ($SiO_2$, petroleum ether/ethyl acetate = 50/1 to 0/1) to give tert-butyl 4-(2-fluorobenzoyl)-4-hydroxypiperidine-1-carboxylate (7.5 g, 23.2 mmol) as a white solid. $^1$H NMR (400 MHz, $CDCl_3$) δ 7.36-7.50 (m, 1H), 7.35-7.36 (m, 1H), 7.23-7.25 (m, 1H), 7.15-7.17 (m, 1H), 4.04 *(d, J* = 12 Hz, 2H), 3.31 (s, 1H), 3.12-3.20 (m, 2H), 1.98-2.03 (m, 2H), 1.64 *(d, J*= 12.0 Hz, 2H), 1.45 (s, 9H).

**Step d:** A mixture of tert-butyl 4-(2-fluorobenzoyl)-4-hydroxypiperidine-1-carboxylate (13.0 g, 40.2 mmol) and *t*-BuOK (4.96 g, 44.2 mmol) in dioxane (65 mL) was stirred at RT for 2 h under $N_2$. Water (65 mL) was added and the mixture extracted with EtOAc (65 mL * 3), the organic layer was dried over $Na_2SO_4$, filtered and concentrated under reduced pressure. The crude product was triturated with petroleum ether/ethyl acetate = 50/1, filtered and the filter cake was dried under reduced pressure to give tert-butyl 3-oxo-3H-spiro[1-benzofumn-2,4'-piperidine]-1'-carboxylate (5.80 g, 19.1 mmol) as a white solid. $^1$H NMR (400 MHz, $CDCl_3$) δ 7.36-7.507.63-7.69 (m, 2H), 7.08-7.14 (m, 2H), 4.15 *(d, J* = 12 Hz, 2H), 3.21-3.28 (m, 2H), 1.91-1.99 (m, 2H), 1.58 *(d, J* = 12 Hz, 2H), 1.49 (s, 9H).

**Step e:** A mixture of tert-butyl 3-oxo-3H-spiro[1-benzofuran-2,4'-piperidine]-1'-carboxylate (4.78 g, 15.8 mmol) and (R)-2-methylpropane-2-sulfinamide (11.5 g, 94.5 mmol) in $Ti(OEt)_4$ (23.9 mL) was stirred at 80 °C for 2 h under $N_2$. The mixture was extracted with water (75 mL) and EtOAc (30 mL * 3), the organic layer was dried over $Na_2SO_4$, filtered and concentrated under reduced pressure. The residue was purified by column chromatography ($SiO_2$, petroleum ether/ethyl acetate = 20/1 to 0/1) to give tert-butyl (3R)-3-[[(R)-tert-butylsulfinyl]imino]spiro[3H-benzofuran-2,4'-piperidine]-1'-carboxylate (4.68 g, 11.53 mmol) as white solid. $^1$H NMR (400 MHz, $CDCl_3$) δ 8.33 (d, *J* = 8 Hz, 1H), 7.51-7.55 (m, 1H), 7.03-7.08 (m, 2H), 4.17 (s, 1H), 3.20 (s, 2H), 1.93-1.96 (m, 2H), 1.71-1.74 (m, 1H), 1.64 *(d, J* = 12 Hz, 1H), 1.50 (s, 9H), 1.31 (s, 9H).

**Step f:** To a mixture of tert-butyl (3R)-3-[[(R)-tert-butylsulfinyl]imino]spiro[3H-benzofuran-2,4'-piperidine]-1'-carboxylate (7.50 g, 18.5 mmol) in THF (40 mL) was added dropwise DIBAL-H (1 M, 73.8 mL, 73.8 mmol). The mixture was stirred at -78 °C for 45 min under $N_2$ and then quenched by addition of water (120 mL) at -78 °C and then allowed to warm to RT. The mixture was extracted with EtOAc (40 mL * 3), the organic layer was dried over $Na_2SO_4$, filtered and concentrated under reduced pressure. The residue was purified by column chromatography ($SiO_2$, Petroleum ether/Ethyl acetate= 15/1 to 0/1) and then the crude product was triturated with petroleum ether/ethyl acetate = 15/1 at RT, filtered and the filter cake was dried under reduced pressure to give tert-butyl (3R)-3-[[(R)-tert-butylsulfinyl]amino]spiro[3H-benzofuran-2,4'-piperidine]-1'-carboxylate (2.26 g, 5.55 mmol) as white solid. $^1$H NMR (400 MHz, $CDCl_3$) δ 7.23-7.29 (m, 2H), 6.90-6.94 (m, 1H), 6.82 (d, *J* = 8 Hz, 1H), 4.63 (d, *J* = 8.0 Hz, 1H), 4.10 (s, 2H), 3.66 (s, 1H), 3.17 (s, 2H), 1.69-2.01 (m, 4H), 1.47 (s, 9H), 1.26 (s, 9H).

**Step g:** To a mixture of tert-butyl (3R)-3-[[(R)-tert-butylsulfinyl]amino]spiro[3H-benzofuran-2,4'-piperidine]-1'-carboxylate (1.5 g, 3.67 mmol) in dichloromethane (10 mL) was added TFA (2.81 mL, 36.7 mmol) at RT. The mixture was stirred for 2 h and the mixture was poured into a 2 N aqueous solution of NaOH. The mixture was filtered on a hydrophobic cartridge (liquid / liquid extraction column, Radleys®) and then concentrated in vacuum to give (R)-2-methyl-N-[(3R)-spiro[3H-benzofuran-2,4'-piperidine]-3-yl]propane-2-sulfinamide (1.13 g, 3.66 mmol) as an off-white solid. $^1$H NMR (400 MHz, DMSO-*d6*) δ 7.25 (d, *J* = 7.4 Hz, 1H), 7.16-7.20 (m, 1H), 6.85-6.89 (m, 1H), 6.78 *(d, J*= 8.0 Hz, 1H), 5.97 (d, *J* = 10.3 Hz, 1H), 4.52 *(d, J*= 10.3 Hz, 1H), 2.75-2.90 (m, 4H), 1.58-1.85 (m, 4H), 1.18 (s, 9H). LCMS m/z $[M+H]^+$ = 309.5.

**Example i-22. Intermediate B-13 ((R)-2-methyl-N-[(3R)-spiro[3H-furo[2,3-b]pyridine-2,4'-piperidine]-3-yl]propane-2-sulfinamide)**

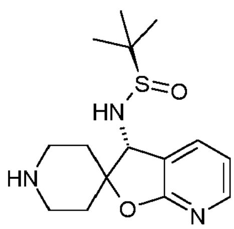

Step a: To a solution of 2-fluoronicotinaldehyde (48.0 g, 384 mmol) and 1,3-propanedithiol (42.4 mL, 422 mmol)

in DCM (240 mL) was added $BF_3.Et_2O$ (47.0% purity, 31.2 mL, 119 mmol) dropwise at RT. The resulting mixture was stirred for 16 h at RT. The reaction was quenched with a saturated aqueous solution of $NaHCO_3$ (300 mL) and extracted with DCM (150 mL * 3). The combined organic layers were dried over anhydrous $Na_2SO_4$, filtered and concentrated. The residue was purified by column chromatography ($SiO_2$, petroleum ether/ethyl acetate = 100/1 to 0/1) to give 3-(1,3-dithian-2-yl)-2-fluoropyridine (46.0 g, 214 mmol) as a colorless oil. $^1H$ NMR (400 MHz, $CDCl_3$) δ 8.15 (d, *J* = 4.0 Hz, 1H), 8.01-8.05 (m, 1H), 7.20-7.27 (m, 1H), 5.44 (s, 1H), 3.09-3.16 (m, 2H), 2.92-2.96 (m, 2H) 2.18-2.23 (m, 1H), 1.93-1.96 (m, 1H).

**Step b:** To a solution of 3-(1,3-dithian-2-yl)-2-fluoropyridine (30.0 g, 138.9 mmol) in THF (150 mL) was added LDA (2 M, 146.4 mL, 292.8 mmol) dropwise at -78 °C. The mixture was stirred for 1 h at -20 °C. A solution of tert-butyl 4-oxopiperidine-1-carboxylate (55.5 g, 278.7 mmol) in THF (60 mL) was added at -78 °C. The reaction was stirred at -78 °C for 1 h. The reaction mixture was poured into an aqueous solution of $NH_4Cl$ (300 mL) at 0 °C. The aqueous phase was extracted with ethyl acetate (200 mL * 3). The combined organic phase was washed with brine, dried with anhydrous $Na_2SO_4$, filtered and concentrated under vacuum. The residue was purified by silica gel chromatography ($SiO_2$, petroleum ether/ethyl acetate = 100/1 to 0/1) to give tert-butyl 4-[2-(2-fluoropyridin-3-yl)-1,3-dithian-2-yl]-4-hydroxypiperidine-1-carboxylate (21.0 g, 50.7 mmol) as a white solid. $^1H$ NMR (400 MHz, $CDCl_3$) δ 8.49-8.54 (m, 1H), 8.21 (d, *J* = 4.0 Hz, 1H), 7.27-7.31 (m, 1H), 3.94 (d, *J* = 12 Hz, 2H), 2.98-2.99 (m, 2H), 2.84-2.88 (m, 2H), 2.57-2.61 (m, 2H), 1.88-1.92 (m, 2H), 1.74-1.81 (m, 4H), 1.43 (s, 9H).

**Step c:** To a solution of tert-butyl 4-[2-(2-fluoropyndin-3-yl)-1,3-dithian-2-yl]-4-hydroxypipendine-1-carboxylate (13.5 g, 32.6 mmol) in $H_2O$ (14.0 mL) and DCM (70.0 mL) were added TBAB (3.15 g, 9.77 mmol), pyridine (3.15 mL, 39.1 mmol) and $Py.HBr_3$ (12.5 g, 39.1 mmol). The mixture was stirred at RT for 10 h. The residue was poured into $H_2O$ (300 mL) at 0 °C. The aqueous phase was extracted with DCM (150 mL * 3). The combined organic phase was washed with brine, dried with anhydrous $Na_2SO_4$, filtered and concentrated under vacuum. The residue was purified by silica gel chromatography ($SiO_2$, petroleum ether/ethyl acetate = 100/1 to 0/1) to give tert-butyl 4-(2-fluoropyridine-3-carbonyl)-4-hydroxypiperidine-1-carboxylate (14.3 g, 44.1 mmol) as a yellow oil. $^1H$ NMR (400 MHz, $CDCl_3$) δ 8.34 (d, *J* = 4.0 Hz, 1H), 7.88-7.92 (m,1H), 7.30-7.33 (m, 1H), 4.03 (d, *J* = 12.0 Hz, 2H), 3.11-3.19 (m, 2H), 1.98-2.02 (m, 2H), 1.68 (d, *J* = 12.0 Hz, 2H), 1.45 (s, 9H).

**Step d:** To a solution of tert-butyl 4-(2-fluoropyridine-3-carbonyl)-4-hydroxypiperidine-1-carboxylate (14.3 g, 44.1 mmol) in dioxane (70 mL) was added *t*-BuOK (5.44 g, 48.5 mmol) at RT. The reaction was stirred at RT for 2 h. The residue was poured into $H_2O$ (200 mL) at 0 °C. The aqueous phase was extracted with ethyl acetate (100 mL * 3). The combined organic phase was washed with brine, dried with anhydrous $Na_2SO_4$, filtered and concentrated under vacuum. The crude product was triturated with petroleum ether/ethyl acetate = 50/1 (25.0 mL) at RT for 30 min to give tert-butyl 3-oxospiro[furo[2,3-b]pyridine-2,4'-piperidine]-1'-carboaylate (9.20 g, 30.2 mmol) as a yellow solid. $^1H$ NMR (400 MHz, $CDCl_3$) δ 8.61-8.62 (m, 1H), 8.04-8.06 (m, 1H), 7.13-7.16 (m, 1H), 4.16 (s, 2H), 3.27-3.33 (m, 2H),1.95-2.04 (m, 2H), 1.65 (d, *J* = 12 Hz, 2H), 1.49 (s, 9H).

**Step e:** To a mixture of tert-butyl 3-oxospiro[furo[2,3-b]pyridine-2,4'-piperidine]-l'-carboxylate (9.2 g, 30.2 mmol) in 2-MeTHF (94 mL) was added (R)-2-methylpropane-2-sulfinamide (22.0 g, 181.4 mmol) and $Ti(OEt)_4$ (25 mL, 121 mmol) at RT under $N_2$. The mixture was stirred at 85 °C for 2 h. The reaction was poured into water (80 mL). The suspension was filtered and the filtrate was extracted with ethyl acetate (30.0 mL * 3). The combined organic phase was washed with brine, dried with anhydrous $Na_2SO_4$, filtered and concentrated under vacuum. The residue was purified by silica gel chromatography ($SiO_2$, petroleum ether/ethyl acetate = 100/1 to 0/1) to give tert-butyl (3R)-3-[[(R)-tert-butylsulfinyl]imino]spiro[3H-furo[2,3-b]pyridine-2,4'-piperidine]-1'-carboxylate (10.2 g, 25.0 mmol) as a yellow solid. $^1H$ NMR (400 MHz, $CDCl_3$) δ 8.79 (d, *J* = 4 Hz, 1H), 8.43-8.44 (m, 1H), 7.06-7.09 (m, 1H), 4.11-4.19 (m, 2H), 3.26 (s, 2H), 1.89-2.04 (m, 2H), 1.77-1.81 (m, 2H), 1.50 (s, 9H), 1.33 (s, 9H).

**Step f:** To a solution of tert-butyl (3R)-3-[[(R)-tert-butylsulfinyl]imino]spiro[3H-furo[2,3-b]pyridine-2,4'-piperidine]-1'-carboxylate (6.00 g, 14.7 mmol) in THF (30.0 mL) was added DIBAL (1 M, 58.9 mL, 58.9 mmol) dropwise at -78 °C. The mixture was stirred for 1 hr at -78 °C. The mixture was poured into $H_2O$ (50 mL) at -20 °C. The aqueous phase was extracted with ethyl acetate (30.0 mL * 3). The combined organic phase was washed with brine, dried with anhydrous $Na_2SO_4$, filtered and concentrated under vacuum. The residue was purified by silica gel chromatography ($SiO_2$, petroleum ether/ethyl acetate = 100/1 to 0/1) to give tert-butyl (3R)-3-[[(R)-tert-butylsulfinyl]amino]spiro[3H-furo[2,3-b]pyridine-2,4'-piperidine]-l'-carboxylate (3.07 g, 7.51 mmol) as a white solid. $^1H$ NMR (400 MHz, $CDCl_3$) δ 8.12 (d, *J* = 4.0 Hz, 1H), 7.63 (d, *J* = 8.0 Hz, 1H), 6.86-6.89 (m, 1H), 4.66 (d, *J* = 8.0 Hz, 1H), 4.11 (s, 2H), 3.76 (d, *J* = 8.0 Hz, 1H), 3.23 (s, 2H), 1.89-1.99 (m, 2H), 1.71-1.79 (m, 2H), 1.46 (s, 9H), 1.25 (s, 9H).

**Step g:** To a mixture of tert-butyl (3R)-3-[[(R)-tert-butylsulfinyl]amino]spiro[3H-furo[2,3-b]pyridine-2,4'-piperidine]-1'-carboxylate (300 mg, 0.73 mmol) in dichloromethane (2 mL) was added TFA (0.56 mL, 7.33 mmol) at RT. The mixture was stirred for 2 h and the mixture was poured into a 2 N aqueous solution of NaOH. The mixture was filtered on a hydrophobic cartridge (liquid / liquid extraction column, Radleys®) and then concentrated in vacuum to give (R)-2-methyl-N-[(3R)-spiro[3H-furo[2,3-b]pyridine-2,4'-piperidine]-3-yl]propane-2-sulfinamide (225 mg, 0.73 mmol) as a white solid. $^1H$ NMR (400 MHz, DMSO-*d6*) δ 8.04 (m, 1H), 7.65 (m, 1H), 6.92 (m, 1H), 6.10 (d, *J* = 10.3 Hz, 1H), 4.57 (d, *J* =

10.3 Hz, 1H), 2.78-2.94 (m, 4H), 1.65-1.88 (m, 4H), 1.17 (s, 9H). LCMS m/z $[M+H]^+$= 310.5.

**Example i-23. Intermediate B-14 (tert-butyl (4S)-4-(tert-butylsulfinylamino)-2-chloro-spiro[4,6-dihydrocyctopenta[d]thiazote-5,4'-piperidine]-1'-carboxylate)**

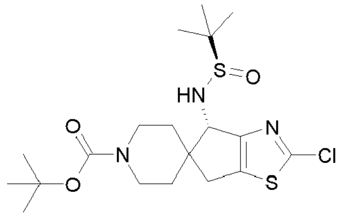

**Step a:** To a solution of 1-tert-butyl 4-ethyl piperidine-1,4-dicarboxylate (90 g, 350 mmol) in THF (500 mL) was added dropwise LDA (2 M, 192.4 mL, 384.8 mmol) at -78 °C. The mixture was stirred at -78 °C for 1 h. 2-chloro-5-(chloromethyl)-1,3-thiazole (58.8 g, 350 mmol) was then added dropwise at -78 °C. The reaction was stirred at -78 °C for 1 h. The reaction was then stirred at RT for 12 h. The reaction was poured into water (800 mL), extracted with petroleum ether/ethyl acetate (1/1, 500 mL * 3), the combined organic layers were washed with brine (500 mL), dried over $Na_2SO_4$, filtered and concentrated under reduced pressure. The residue was purified by column chromatography ($SiO_2$, petroleum ether/ethyl acetate= 50/1 to 0/1) to give 1-(tert-butyl) 4-ethyl 4-((2-chlorothiazol-5-yl)methyl)piperidine-1,4-dicarboxylate (50.0 g, 129 mmol) as a yellow oil. $^1H$ NMR (400 MHz, $CDCl_3$) δ δ 7.14 (s, 1H), 4.10 (q, *J* = 6.8 Hz, 2H), 3.80 (d, *J* = 11.2 Hz, 2H), 2.88-2.93 (m, 4H), 2.04 (d, *J* = 13.6 Hz, 2H), 1.38 (s, 9H), 1.33-1.36 (m, 2H), 1.19 (t, *J* = 7.2 Hz, 3H).

**Step b:** To a solution of 1-(tert-butyl) 4-ethyl 4-((2- chlorothiazol-5yl)methyl)piperidine-1,4-dicarboxylate (50.0 g, 128.6 mmol) in THF (500 mL) was added LDA (2 M, 160.8 mL, 321.6 mmol) at -78 °C. The mixture was stirred at -78 °C for 1 h. The reaction was poured into water (1.0 L) slowly, extracted with EtOAc (200 mL * 3), the combined organic layers were washed with brine (500 mL), dried over $Na_2SO_4$, filtered and concentrated under reduced pressure. The residue was purified by column chromatography ($SiO_2$, petroleum ether/ethyl acetate= 20/1 to 0/1) to give tert-butyl 2-chloro-4-oxo-4,6-dihydrospiro[cyclopenta[*d*]thiazole-5,4'-piperidine]-1'-carboxylate (14.0 g, 40.8 mmol) as a yellow solid. $^1H$ NMR (400 MHz, $CDCl_3$) δ 4.08-4.16 (m, 2H), 3.10 (s, 2H), 2.97 (t, *J* = 12.4 Hz, 2H), 1.92-2.00 (m, 2H), 1.43-1.47 (m, 12H).

**Step c:** A mixture of tert-butyl 2-chloro-4-oxo-4,6-dihydrospiro[cyclopenta[*d*]thiazole-5,4'-piperidine]-1'-carboxylate (10.0 g, 29.2 mmol,) and (R)-2-methylpropane-2-sulfinamide (10.6 g, 87.5 mmol) in $Ti(OEt)_4$ (70 mL) was degassed with $N_2$, and then the mixture was stirred at 100 °C for 16 h under $N_2$. The reaction was poured into water (200 mL) and EtOAc (200 mL), the suspension was filtered, the filtrate was extracted with EtOAc (200 mL * 3), the combined organic layers were washed with brine (200 mL), dried over $Na_2SO_4$, filtered and concentrated under reduced pressure. The residue was purified by column chromatography ($SiO_2$, petroleum ether/ethyl acetate= 30/1 to 0/1) to give tert-butyl (R,Z)-4-((tert-butylsulfinyl)imino)-2-chloro-4,6-dihydrospiro[cyclopenta[*d*]thiazole-5,4'-piperidine]-1'-carboxylate (10.72 g, 24.03 mmol) as a yellow solid. $^1H$ NMR (400 MHz, $CDCl_3$) δ 4.02-4.11 (m, 2H), 3.02 (d, *J* = 4.8 Hz, 2H), 2.85 (br s, 2H), 2.03-2.11 (m, 1H), 1.92-1.96 (m, 1H), 1.45-1.57 (m, 2H), 1.41 (s, 9H), 1.20 (s, 9H).

**Step d:** To a solution of tert-butyl (R,Z)-4-((tert-butylsulfinyl)imino)-2-chloro-4,6-dihydrospiro[cyclopenta[*d*]thiazole-5,4'-piperidine]-1'-carboxylate (10.0 g, 22.4 mmol) in THF (50 mL) was added DIBAL-H (1 M, 89.7 mL, 89.7 mmol) dropwise at -78 °C. The mixture was stirred at -78 °C for 1 h. The reaction was quenched by the addition of $H_2O$ (30 mL) dropwise below 0 °C, the mixture was stirred at RT for 12 h. The suspension was filtered, the filtrate was extracted with EtOAc (30 mL * 2), the combined organic layers were washed with brine (50 mL), dried over $Na_2SO_4$, filtered and concentrated under reduced pressure. The residue was purified by column chromatography ($SiO_2$, petroleum ether/ethyl acetate= 5/1 to 0/1) to give tert-butyl (4S)-4-(tert-butylsulfinylamino)-2-chloro-4,6-dihydrospiro[cyclopenta[d]thiazole-5,4'-piperidine]-1'-carboxylate (6.0 g, 13.4 mmol) as a yellow solid. $^1H$ NMR (400 MHz, $CDCl_3$) δ 4.32 (d, *J* = 9.2 Hz, 1H), 3.84 (d, *J* = 13.6 Hz, 2H), 2.96-3.03 (m, 2H), 2.69-2.78 (m, 2H), 1.79-1.92 (m, 2H), 1.44-1.55 (m, 2H), 1.39 (s, 9H), 1.18 (s, 9H).

**Example i-24. Intermediate B-15 ((4S)-spiro[4,6-dihydrocyclopenta[d]thiazole-5,4'-piperidine]-4-amine hydrochloride)**

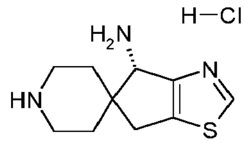

**Step a:** To a solution of **intermediate B-14** (6.0 g, 13.4 mmol) in MeOH (30 mL) and TEA (6 mL) was added Pd/C (10%, 3 g) under $N_2$. The suspension was degassed under vacuum and purged with $H_2$ several times. The mixture was stirred under $H_2$ (40 psi) at 50 °C for 2 h. The suspension was filtered, the filtrate was concentrated under vacuum. The residue was purified by column chromatography ($SiO_2$, petroleum ether/ethyl acetate= 5/1 to 0/1) to give tert-butyl (4S)-4-((R)-tert butylsulfinylamino)spiro[4,6-dihydrocyclopenta[*d*]thiazole-5,4'-piperidine]-1'-carboaylate (2.74 g, 6.47 mmol) as an off-white solid. $^1H$ NMR (400 MHz, MeOD) δ 8.87 (s, 1H), 4.37 (s, 1H), 3.87-3.98 (m, 2H), 3.09-3.19 (m, 2H), 2.94 (q, *J* = 9.2 Hz, 2H), 1.89-1.93 (m, 1H), 1.47-1.76 (m, 3H), 1.26 (s, 9H), 1.23 (s, 9H).

**Step b:** To a mixture of tert-butyl (4S)-4-((R)-tert-butylsulfinylamino)spiro[4,6-dihydrocyclopenta[*d*]thiazole-5,4'-piperidine]-1'-carboxylate (1.5 g, 3.6 mmol) in dichloromethane (25 mL) was added dropwise a solution of HCl (2.5 M in ethanol, 25 mL, 62.5 mmol) at RT. The mixture was stirred for 3 h and then concentrated under vacuo. The residue was taken up in dichloromethane (50 mL), filtered, washed with diisopropylether (30 mL) and then pentane (30 mL) to give (4S)-spiro[4,6-dihydrocyclopenta[d]thiazole-5,4'-piperidine]-4-amine hydrochloride (1.2 g, crude) that was used without further purification.

**Example i-25. Intermediate B-16 ((4S)-2-chlorospiro[4,6-dihydrocyclopenta[d]thiazole-5,4'-piperidine]-4-amine hydrochloride)**

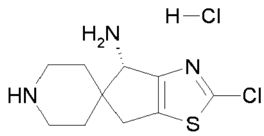

A mixture of **intermediate B-14** (1.5 g, 3.3 mmol) in a solution of HCl (2.5 M in ethanol, 15 mL, 37.5 mmol) was stirred for 3 h and then concentrated under vacuo. The residue was taken up in EtOAc (10 mL) and diisopropylether (10 mL), filtered, washed with diisopropylether and then pentane to give ((4S)-2-chlorospiro[4,6-dihydrocyclopenta[d]thiazole-5,4'-piperidine]-4-amine hydrochloride (1.04 g, crude) that was used without further purification.

**Example i-26. Intermediate B-17 (tert-butyl (6S)-6-[[(R)-tert-butylsulfinyl]amino]-2-chloro-spiro[4,6-dihydrocyclopenta[d]thiazole-5,4'-piperidine]-1'-carboxylate)**

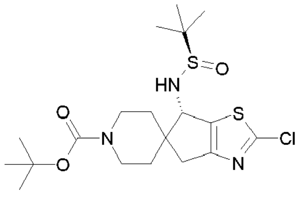

**Step a:** To a solution of 1-tert-butyl 4-ethyl piperidine-1,4-dicarboxylate (50.0 g, 194 mmol) in THF (350 mL) was added dropwise LDA (2 M, 117 mL, 234 mmol) at -78 °C. The mixture was stirred at -78 °C for 1 h. Then 2-chloro-4-(chloromethyl)thiazole (31.0 g, 185 mmol) was added dropwise at -78 °C. The reaction was stirred at -78 °C for 1 h. Then the reaction was stirred at RT for 12 h. The reaction was poured into water (100 mL), extracted with petroleum ether/ethyl acetate (1/1, 50 mL * 3), the combined organic layers were washed with brine (100 mL), dried over $Na_2SO_4$, filtered and concentrated under reduced pressure. The residue was purified by column chromatography ($SiO_2$, petroleum ether/ethyl acetate= 50/1 to 0/1) to give 1-(tert-butyl) 4-ethyl 4-((2-chlorothiazol-4-yl)methyl) piperidine-1,4-dicarboxylate (20.0 g, 51.5 mmol) as yellow oil. $^1H$ NMR (400 MHz, $CDCl_3$) δ 6.80 (s, 1H), 4.15 (q, *J* = 7.2 Hz, 2H), 3.89 (br s, 2H), 2.80-2.94 (m, 3H), 2.11 (d, *J* = 13.2 Hz, 2H), 1.50-1.52 (m, 2H), 1.45 (s, 9H), 1.24 (t, *J* = 7.2 Hz, 3H).

**Step b:** To a solution of 1-(tert-butyl) 4-ethyl 4-((2-chlorothiazol-4-yl)methyl) piperidine-1,4-dicarboxylate (30 g, 77.14 mmol) in THF (210 mL) was added dropwise LDA (2 M, 57.9 mL, 115.8 mmol) at -78 °C. The mixture was stirred at -78 °C for 1 h. The reaction was poured slowly into brine (200 mL) and extracted with EtOAc (100 mL * 3). The combined organic layers were dried over $Na_2SO_4$, filtered and concentrated under reduced pressure. The residue was purified by column chromatography ($SiO_2$, petroleum ether/ethyl acetate= 50/1 to 0/1) to give tert-butyl 2-chloro-6-oxo-4,6-dihydrospiro[cyclopenta[*d*]thiazole-5,4'-piperidine]-1'-carboxylate (13.0 g, 37.9 mmol) as a yellow solid. $^1H$ NMR (400 MHz, $CDCl_3$) $\delta$ 4.09 (d, *J* = 13.2 Hz, 2H), 2.98 (s, 2H), 2.88 (t, *J* = 8.0 Hz, 2H), 1.86-1.93 (m, 2H), 1.41-1.44 (m, 10H).

Step c: To a solution of tert-butyl 2-chloro-6-oxo-4,6-dihydrospiro[cyclopenta[*d*]thiazole-5,4'-piperidine]-1'-carboxylate (13.0 g, 37.9 mmol) in $Ti(OEt)_4$ (65 mL) was added (R)-2-methylpropane-2-sulfinamide (13.8 g, 114 mmol). The mixture was stirred at 100 °C for 16 h and then let to cool down to RT. The reaction was poured into water (50 mL) and EtOAc (50 mL), the suspension was filtered, the filtrate was extracted with EtOAc (20 mL * 3), the combined organic layers were washed with brine (50 mL), dried over $Na_2SO_4$, filtered and concentrated under reduced pressure to give tert-butyl (R,Z)-6-((tert-butylsulfinyl)imino)-2-chloro-4,6-dihydrospiro[cyclopenta[*d*]thiazole-5,4'-piperidine]-1'-carboxylate (11.0 g, 24.7 mmol) as a yellow oil. $^1H$ NMR (400 MHz, $CDCl_3$) $\delta$ 4.06-4.13 (m, 2H), 2.80-2.92 (m, 4H), 1.84-2.01 (m, 3H), 1.41 (s, 9H), 1.16 (s, 9H).

**Step d:** To a solution of tert-butyl 6-(((R)-tert-butylsulfinyl)imino)-2-chloro-4,6-dihydrospiro[cyclopenta[*d*]thiazole-5,4'-piperidine]-1'-carboxylate (12.0 g, 26.9 mmol) in THF (60 mL) was added dropwise DIBAL-H (1 M, 108 mL, 108 mmol) at -78 °C. The mixture was stirred at -78 °C for 1 h. The reaction was quenched by the addition of $H_2O$ (50 mL) dropwise below 0 °C, the mixture was stirred at RT for 12 h. The suspension was filtered, the filtrate was extracted with EtOAc (50 mL * 2), the combined organic layers were washed with brine (50 mL), dried over $Na_2SO_4$, filtered and concentrated under reduced pressure. The residue was purified by column chromatography ($SiO_2$, petroleum ether/ethyl acetate= 5/1 to 0/1) to give tert-butyl (6S)-6-(((R)tert-butylsulfinyl)amino)-2-chloro-4,6-dihydrospiro[cyclopenta[*d*]thiazole-5,4'-piperidine]-1'-carboxylate (7.00 g, 15.6 mmol) as a yellow solid. $^1H$ NMR (400 MHz, $CDCl_3$) $\delta$ 4.49 (d, *J* = 8.8 Hz, 1H), 3.95-3.98 (m, 2H), 3.61 (d, *J* = 7.2 Hz, 1H), 2.79-3.30 (m, 4H), 1.73-1.84 (m, 2H), 1.54-1.61 (m, 2H), 1.44 (s, 9H), 1.19 (s, 9H).

**Example i-27. Intermediate B-18 ((6S)-2-chlorospiro[4,6-dihydrocyclopenta[d]thiazole-5,4'-piperidine]-6-amine hydrochloride)**

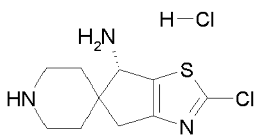

A mixture of **intermediate B-17** (0.81 g, 2.3 mmol) in a solution of HCl (4 M in dioxane, 15 mL, 60 mmol) was stirred for 24 h at RT. The mixture was then filtered, washed with diisopropylether to give (6S)-2-chlorospiro[4,6-dihydrocyclopenta[d]thiazole-5,4'-piperidine]-6-amine hydrochloride (813 mg, crude) as a yellow solid that was used without further purification.

**Example i-28. Intermediate B-19 ((6S)-spiro[4,6-dihydrocyclopenta[d]thiazole-5,4'-piperidine]-6-amine hydrochloride)**

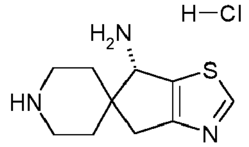

Step a: To a solution of **intermediate B-17** (7.00 g, 15.6 mmol) in MeOH (35 mL) and TEA (7 mL) was added Pd/C (10%, 3.00 g, 15.6 mmol) under $N_2$. The suspension was degassed under vacuum and purged with $H_2$ several times. The mixture was stirred under $H_2$ (40 psi) at 50 °C for 5 h. The suspension was filtered, the filtrate was concentrated under vacuum. The residue was purified by column chromatography ($SiO_2$, petroleum ether/ethyl acetate= 5/1 to 0/1) to give tert-butyl (6S)-6-(((R)tert-butylsulfinyl)amino)-2-chloro-4,6-dihydrospiro[cyclopenta[*d*]thiazole-5,4'-piperidine]-1'-carboxylate (4.5 g, 10.8 mmol) as a white solid. $^1$H NMR (400 MHz, MeOD) δ 8.98 (s, 1H), 4.57 (s, 1H), 3.94-4.03 (m, 2H), 3.04-3.13 (m, 2H), 2.90 (q, *J* = 15.6 Hz, 2H), 1.86-1.91 (m, 1H), 1.76-1.77 (m, 1H), 1.64-1.68 (m, 2H), 1.47 (s, 9H), 1.24 (s, 9H).

**Step b:** A mixture of tert-butyl (6S)-6-(((R)tert-butylsulfinyl)amino)-2-chloro-4,6-dihydrospiro[cyclopenta[*d*]thiazole-5,4'-piperidine]-1'-carboxylate (500 mg, 1.21 mmol) in a solution of HCl (2.5 M in ethanol, 10 mL, 25 mmol) was stirred for 3 h at RT. The mixture was then concentrated under vacuo. The residue was taken up in ethyl acetate (30 mL), filtered, washed with diisopropylether (30 mL) and then pentane (30 mL) to give (6S)-spiro[4,6-dihydrocyclopenta[d]thiazole-5,4'-piperidine]-6-amine hydrochloride (320 mg, crude) that was used without further purification.

**Example i-29. Intermediate B-20 ((6S)-2-methylspiro[4,6-dihydrocyclopenta[d]thiazole-5,4'-piperidine]-6-amine hydrochloride)**

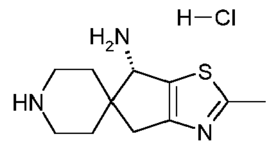

**Step a:** To a mixture of **intermediate B-17** (2.50 g, 5.58 mmol), trimethylboroxine (50% purity, 2.34 mL, 8.37 mmol) and $K_2CO_3$ (1.54 g, 11.2 mmol) in dioxane (5 mL) was added under $N_2$ atmosphere $Pd(dppf)Cl_2.CH_2Cl_2$ (227 mg, 0.28 mmol). The mixture was stirred at 115 °C for 2 h. The suspension was filtered, the filtrate was concentrated under vacuum. The residue was purified by column chromatography ($SiO_2$, petroleum ether/ethyl acetate = 10/1 to 0/1) to give tert-butyl (6S)-6-[[(R)-tert-butylsulfinyl]amino]-2-methyl-spiro[4,6-dihydrocyclopenta[d]thiazole-5,4'-piperidine]-l'-carboxylate (3.00 g, 7.02 mmol) as a light yellow solid. $^1$H NMR (400 MHz, $CDCl_3$) δ 4.49 (d, *J* = 8.4 Hz, 1H), 3.99 (s, 2H), 3.54 (s, 1H), 2.96-3.04 (m, 2H), 2.81 (s, 2H), 2.73 (s, 3H), 1.73-1.84 (m, 2H), 1.58-1.63 (m, 2H), 1.46 (s, 9H), 1.21 (s, 9H).

**Step b:** A mixture of tert-butyl (6S)-6-[[(R)-tert-butylsulfinyl]amino]-2-methyl-spiro[4,6-dihydrocyclopenta[d]thiazole-5,4'-piperidine]-l'-carboxylate (1.0 g, 2.3 mmol) in a solution of HCl (4 M in dioxane, 15 mL, 60 mmol) was stirred for 24 h at RT. The mixture was then filtered and washed with diisopropylether to give (6S)-2-methylspiro[4,6-dihydrocyclopenta[d]thiazole-5,4'-piperidine]-6-amine hydrochloride (763 mg, crude) as a white solid that was used without further purification.

**Example i-30. Intermediate B-21 ((5S)-2-methylspiro[5,7-dihydrocyclopenta[b]pyridine-6,4'-piperidine]-5-amine hydrochloride)**

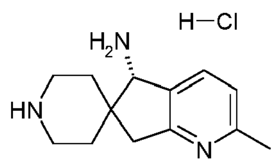

**Step a:** To a solution of 3-bromo-2,6-dimethylpyridine (160 g, 860 mmol) in $CHCl_3$ (1.12 L) at 75 °C under $N_2$ were added NBS (184 g, 1.03 mol) and AIBN (42.4 g, 258 mmol) and the mixture was stirred for 4 h. The residue was poured into water (500 mL). The aqueous phase was extracted with DCM (350 mL * 3). The combined organic phase was washed with brine (200 mL), dried with anhydrous $Na_2SO_4$, filtered and concentrated in vacuum. The residue was purified by column chromatography ($SiO_2$, Petroleum ether/Ethyl acetate = 100/0 to 0/1) to give compound 3-bromo-2-(bromomethyl)-6-methylpyridine (33.3 g, 119.5 mmol) as a brown oil. $^1$H NMR (400 MHz, $CDCl_3$) δ 7.74 (d, *J* = 8.4 Hz, 1H), 6.98 (d, *J* = 8.4 Hz, 1H), 4.68 (s, 2H), 2.53 (s, 3H).

**Step b:** To a mixture of 1-tert-butyl 4-methyl piperidine-1,4-dicarboxylate (21.0 g, 86.3 mmol) in THF (91.0 mL) at -78 °C under $N_2$ was added dropwise LDA (2.00 M, 64.7 mL, 129.4 mmol). The mixture was then stirred at RT for 1 h. 3-Bromo-2-(bromomethyl)-6-methylpyridine (32.3 g, 104 mmol) was added and the mixture was stirred for 2 h. The reaction mixture was poured into water (200 mL). The aqueous phase was extracted with ethyl acetate (100 mL * 3). The combined organic phase was washed with brine (100 mL), dried with anhydrous $Na_2SO_4$, filtered and concentrated in vacuum. The residue was purified by column chromatography ($SiO_2$, Petroleum ether/Ethyl acetate = 100/1 to 0/1)

to give 1-(tert-butyl) 4-methyl 4-((3-bromo-6-methyl-2-pyridinyl)methyl)piperidine-1,4-dicarboxylate (17.0 g, 39.0 mmol) as a yellow oil. $^1$H NMR (400 MHz, $CDCl_3$) δ 7.66 (d, *J* = 8.4 Hz, 1H), 6.85 (d, *J* = 8.4 Hz, 1H), 3.82 (s, 2H), 3.72 (s, 3H), 3.23 (s, 2H), 3.03 (t, *J* = 12 Hz, 2H), 2.43 (s, 3H), 2.09 (d, *J* = 7.6 Hz, 2H), 1.61 (t, *J* = 2 Hz, 2H), 1.46 (s, 9H).

**Step c:** To a mixture of 1-(tert-butyl) 4-methyl 4-((3 bromo-6-methyl-2-pyridinyl)methyl)piperidine-1,4-dicarboxylate (66.9 g, 157 mmol) in $H_2O$ (134 mL) and MeOH (468 mL) was added NaOH (31.3 g, 783 mmol) at RT. The mixture was stirred at 65 °C for 16 h. The mixture was concentrated to dryness, and the crude was dissolved with water (50 mL) and washed with MTBE (20 mL). The aqueous layer was separated and brought to pH 6-7 by additon of a 2 N aqueous solution of HCl. The compound was extracted with EtOAc (25 mL * 2). The combined organic phase was washed with brine (20 mL), dried over $Na_2SO_4$, filtered and concentrated in vacuum to give 1-(tert-butoxycarbonyl)-4-((3-bromo-6-methyl-2-pyridinyl)methyl)piperidine-4-carboxylic acid (75.0 g, crude) as a white solid.

**Step d:** To a mixture of crude 1-(tert-butoxycarbonyl)-4-((3-bromo-6-methyl-2-pyridinyl)methyl)piperidine-4-carboxylic acid (75.0 g) in THF (105 mL) was added NaH (60 %, 6.97 g, 174 mmol) in one portion at -15 °C under $N_2$. The mixture was stirred at -15 °C for 15 min, then cooled to -60 °C and n-BuLi (1.79 M, 114 mL, 204 mmol) was added dropwise then the temperature was raised to -20 °C over 30 min. The reaction mixture was quenched by addition of water (350 mL) at 0 °C, and extracted with ethyl acetate (400 mL * 2). The combined organic layers were washed with brine (200 mL), dried over $Na_2SO_4$, filtered and concentrated under reduced pressure. The residue was purified by column chromatography ($SiO_2$, Petroleum ether/Ethyl acetate = 100/1 to 0/1) to give tert-butyl 2- methyl-5-oxo-spiro[5,7-dihydrocyclopenta[b]pyridine-6,4'-piperidine]-1'-carboxylate (11.0 g, 34.8 mmol) as a yellow oil. $^1$H NMR (400 MHz, $CDCl_3$) δ 7.91 (d, *J* = 3.2, 1H), 7.20 (d, *J* = 4 Hz, 1H), 4.12 (s, 2H), 3.12 (s, 2H), 3.01 (t, *J* = 10.4 Hz, 2H), 2.67 (s, 3H), 1.92 (t, *J* = 12 Hz, 2H), 1.47 (s, 9H), 1.41 (d, *J* = 5.8 Hz, 2H).

Step e: A mixture of tert-butyl 2- methyl-5-oxo-spiro[5,7-dihydrocyclopenta[b]pyridine-6,4'-piperidine]-1'-carboxylate (12.5 g, 39.5 mmol) and (R)-2-methylpropane-2-sulfinamide (14.4 g, 119 mmol) in $Ti(OEt)_4$ (87.5 mL) was stirred at 110 °C for 11 h and then let to cool down to RT. The reaction mixture was quenched by addition of water (100 mL), then filtered and extracted with ethyl acetate (100 mL * 2). The combined organic layers were washed with brine (100 mL * 2), dried over $Na_2SO_4$, filtered and concentrated under reduced pressure. The residue was purified by column chromatography ($SiO_2$, Petroleum ether/Ethyl acetate = 100/1 to 0/1) to give tert-butyl (5S)-5-((tert-butylsulfinyl)imino)-2-methyl-spiro[5,7-dihydrocyclopenta[b]pyridine-6,4'-piperidine]-l'-carboxylate (18.5 g, crude) as a white solid that was used without further purification.

**Step f:** To a mixture of tert-butyl (5S)-5-((tert-butylsulfinyl)imino)-2-methyl-spiro[5,7-dihydrocyclopenta[b]pyridine-6,4'-piperidine]-l'-carboxylate (18.5 g, crude) in THF (116 mL) was added dropwise DIBAL-H (1.00 M, 172 mL, 172 mmol) at -78 °C. The mixture was stirred at -78 °C for 1 h. The reaction mixture was quenched by addition of water (300 mL) at 0 °C and stirred for 40 min, then filtered and extracted with ethyl acetate (200 mL * 2). The combined organic layers were washed with brine (100 mL * 2), dried over $Na_2SO_4$, filtered and concentrated under reduced pressure. The crude product was triturated with MTBE (100 mL) at RT for 20 min, filtered to give tert-butyl (5S)-5-((tert-butylsulfinyl)amino)-2-methyl-spiro[5,7-dihydrocyclopenta[b]pyridine-6,4'-piperidine]-1'-carboxylate (2.90 g, 6.81 mmol) as a white solid. $^1$H NMR (400 MHz, $CDCl_3$) δ 7.55 (d, *J* = 3.6 Hz, 1H), 7.01 (d, *J* = 4 Hz, 1H), 4.48(d, *J* = 4.4 Hz, 1H), 4.01 (d, *J* = 8.8 Hz, 2H), 3.55 (s, 1H), 3.11 (s, 1H), 2.945 (t, *J* = 10.4 Hz, 2H), 2.84 (d, *J* = 7.2 Hz, 1H), 2.561 (s, 3H), 2.03-2.05 (m, 1H), 1.53 (s, 1H), 1.49 (s, 1 H), 1.46 (s, 9H), 1.35 (s, 1H), 1.26 (s, 9H).

**Step g:** To a mixture of tert-butyl (5S)-5-((tert-butylsulfinyl)amino)-2-methyl-spiro[5,7-dihydrocyclopenta[b]pyridine-6,4'-piperidine]-l'-carboxylate (1.0 g, 2.37 mmol) in dichloromethane (5 mL) was added a solution of HCl (2.5 M in ethanol, 15 mL, 38 mmol). The mixture was stirred for 3 h at RT and then concentrated under vacuo to give (5S)-2-methylspiro[5,7-dihydrocyclopenta[b]pyridine-6,4'-piperidine]-5-amine hydrochloride (1.045 g, crude) as a yellow solid that was used without further purification.

**Example i-31. Intermediate B-22 (1-(triisopropylsilyloxymethyl)spiro[7H-cyclopenta[c]pyridine-6,4'-piperidine]-5-one)**

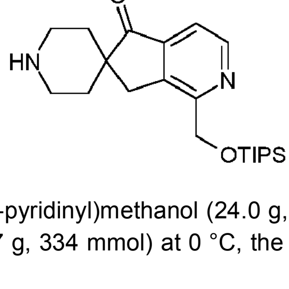

**Step a:** To a solution of (3-chloro-2-pyridinyl)methanol (24.0 g, 167 mmol) and TIPSCl (39.3 mL, 184 mmol) in DCM (120 mL) was added imidazole (22.7 g, 334 mmol) at 0 °C, the mixture was then stirred at RT for 2 h. Water

was added (100 mL) and the mixture extracted with DCM. The combined organic layer was dried over $Na_2SO_4$, filtered and concentrated under reduced pressure. The residue was purified by column chromatography ($SiO_2$, petroleum ether/ethyl acetate = 1/0 to 0/1) to give 3-chloro-2-(triisopropylsilyloxymethyl)pyridine (50.0 g, 167 mmol) as a colorless oil. $^1H$ NMR (400 MHz, $CDCl_3$) δ 8.48 (dd, *J* = 4.4 Hz, 1.2 Hz, 1H), 7.67 (dd, *J* = 8.0 Hz, 1.2 Hz, 1H), 7.17 (dd, *J* = 8.0 Hz, 4.8 Hz, 1H), 4.99 (s, 2H), 1.16-1.22 (m, 3H), 1.08-1.10 (m, 18H).

**Step b:** To a solution of 3-chloro-2-(triisopropylsilyloxymethyl)pyridine (30.0 g, 100 mmol) in THF (600 mL) was added LDA (2 M, 60.0 mL, 120 mmol) dropwise at -78 °C under $N_2$, the mixture was stirred at -78 °C for 1.5 h. Tert-butyl 4-formyl-4-methylpiperidine-1-carboxylate (22.7 g, 100 mmol) in THF (30 mL) was added to the mixture and the mixture was stirred at -78 °C for 1 h. The mixture was quenched by water (1000 mL) and extracted with EtOAc (500 mL * 2), the organic layer was dried over $Na_2SO_4$, filtered and concentrated under reduced pressure. The residue was purified by column chromatography ($SiO_2$, petroleum ether/ethyl acetate= 1/0 to 0/1) to give tert-butyl 4-[[3-chloro-2-(triisopropylsilyloxymethyl)-4-pyridyl]-hydroxy-methyl]-4-methyl-piperidine-1-carboxylate (19.0 g, 36.0 mmol) as a white solid. $^1H$ NMR (400 MHz, $CDCl_3$) δ 8.45 (d, *J* = 4.8 Hz, 1H), 7.40 (d, *J* = 5.2 Hz, 1H), 4.96-5.05 (m, 3H), 3.92 (m, 2H), 2.81-2.89 (m, 2H), 2.23 (s, 1H), 1.79-1.82 (m, 1H), 1.55-1.61 (m, 2H), 1.45 (s, 9H), 1.17-1.20 (m, 4H), 1.06-1.09(m, 18H), 1.03 (s, 3H).

**Step c:** To a solution of tert-butyl 4-[[3-chloro-2-(triisopropylsilyloxymethyl)-4-pyridyl]-hydroxy-methyl]-4-methyl-piperidine-1-carboxylate (10.0 g, 19.0 mmol) in MeCN (150 mL) and DCM (50 mL) was added IBX (10.6 g, 37.9 mmol), the mixture was stirred at 65 °C for 12 h. Water (100 mL) was added and the mixture extracted with DCM (100 mL), the organic layer was dried over $Na_2SO_4$, filtered and concentrated under reduced pressure. The residue was purified by column chromatography ($SiO_2$, petroleum ether/ethyl acetate= 1/0 to 0/1) to give tert-butyl 4-[3-chloro-2-(triisopropylsilyloxymethyl)pyridine-4-carbonyl]-4-methyl-piperidine-1-carboxylate (9.00 g, 17.1 mmol) as a colorless oil. $^1H$ NMR (400 MHz, $CDCl_3$) δ 8.52 (d, *J* = 5.2 Hz, 1H), 6.99 (d, *J* = 4.8 Hz, 1H), 5.01 (s, 2H), 3.69-3.73 (m, 2H), 3.20-3.27 (m, 2H), 1.96-2.02 (m, 2H), 1.50-1.53 (m, 2H), 1.46 (s, 9H), 1.34 (s, 3H), 1.15-1.21 (m, 3H), 1.07-1.09 (m, 18H).

**Step d:** A mixture of tert-butyl 4-[3-chloro-2-(triisopropylsilyloxymethyl)pyridine-4-carbonyl]-4-methylpiperidine-1-carboxylate (8.50 g, 16.2 mmol), $Cs_2CO_3$ (6.33 g, 19.4 mmol), tricyclohexylphosphonium tetrafluoroborate (596 mg, 1.62 mmol) and pivalic acid (496 mg, 4.86 mmol) in mesitylene (59.5 mL) was degassed with $N_2$ and then was added $Pd(OAc)_2$ (182 mg, 0.81 mmol) was added. The solution and stirred at 160 °C for 4 h. The mixture was cooled to RT and water (100 mL) was added. The mixture was extracted with EtOAc (200 mL), the organic layer was separated, dried over $Na_2SO_4$, filtered and concentrated under reduced pressure. The residue was purified by column chromatography ($SiO_2$, Petroleum ether/Ethyl acetate= 1/0 to 0/1) to give tert-butyl 5-oxo-1-(triisopropylsilyloxymethyl)spiro[7H-cyclopenta[c]pyridine-6,4'-piperidine]-1'-carboxylate (6.03 g, 12.0 mmol) as a yellow solid. $^1H$ NMR (400 MHz, $CDCl_3$) δ 8.59 (d, *J* = 4.8 Hz, 1H), 7.50 (d, *J* = 4.8 Hz, 1H), 5.10 (s, 2H), 4.14 (s, 2H), 3.33 (s, 2H), 2.98-3.04 (m, 2H), 1.86-1.92 (m, 2H), 1.49 (s, 9H), 1.35-1.42 (m, 2H), 1.11-1.23 (m, 3H), 1.08-1.10 (m, 18H).

**Step e:** To a solution of tert-butyl 5-oxo-1-(triisopropylsilyloxymethyl)spiro[7H-cyclopenta[c]pyridine-6,4'-piperidine]-1'-carboxylate (1.0 g, 2.05 mmol) in dichloromethane (10 mL) was added TFA (1.6 mL, 21.6 mmol). The mixture was stirred at RT for 18 h. Dichloromethane (20 mL) and water (20 mL) were added, the pH value of the aqueous phase was adjusted to 11-12 with aqueous 35% NaOH solution and the mixture was filtered on a hydrophobic cartridge (liquid / liquid extraction column, Radleys®) and then concentrated in vacuum to give 1-(triisopropylsilyloxymethyl)spiro[7H-cyclopenta[c]pyridine-6,4'-piperidine]-5-one (739 mg, 1.9 mmol) as an orange oil. LCMS m/z $[M+H]^+$ 389.7.

**Example i-32. Intermediate B-23 ((R)-N-[(7S)-3-(hydroxymethyl)spiro[5,7-dihydrocyclopenta[c]pyridine-6,4'-piperidine]-7-yl]-2-methyl-propane-2-sulfinamide)**

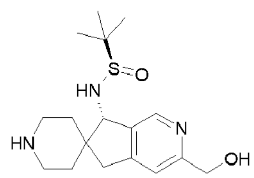

**Step a:** To a mixture of 5-bromo-2-methylpyridine (70.0 g, 406 mmol) in DCM (420 mL) was added m-CPBA (85% pure, 99.1 g, 488 mmol) at 0 °C and stirred at RT for 12 h. The reaction mixture was poured into an aqueous solution of $Na_2SO_3$. The mixture was stirred for 10 min, then an aqueous solution of $NaHCO_3$ was added. The aqueous layer was separated and then extracted with DCM, the organic layer was dried over $Na_2SO_4$, filtered and concentrated under reduced pressure to give 5-bromo-2-methylpyridine 1-oxide (77 g, crude) as a yellow solid that was used without further purification.

**Step b:** To a mixture of 5-bromo-2-methylpyridine 1-oxide (70.0 g, crude) in $H_2SO_4$ (350 mL) was added $HNO_3$

(83.7 mL, 1.86 mol) at 60 °C. The mixture was stirred at 90 °C for 4 h. The combined reaction mixture was poured into ice-water (1 L) and extracted with DCM (300 mL * 6), the organic layer was dried over $Na_2SO_4$. filtered and concentrated under reduced pressure. The crude product was triturated with MTBE (100 mL) at RT and filtered. The residue was purified by column chromatography ($SiO_2$, petroleum ether/ethyl acetate = 50/1 to 0/1) to give 5-bromo-2-methylene-4-nitro-1,2-dihydropyridine 1-oxide (50 g, 214 mmol) as a yellow solid. $^1$H NMR (400 MHz, $CDCl_3$) $\delta$ 8.53 (s, 1H), 7.99 (s, 1H), 2.50 (s, 3H).

**Step c:** To a mixture of 5-bromo-2-methylene-4-nitro-1,2-dihydropyridine 1-oxide (50.0 g, 214 mmol,) in DCM (300 mL) was added $PCl_3$ (59.8 mL, 643 mmol). The mixture was stirred at RT for 12 h and then poured slowly into water (750 mL). The aqueous layer was separated and then extracted with DCM (300 mL * 3). The combined organic layer was dried over $Na_2SO_4$, filtered and concentrated under reduced pressure. The crude product was triturated with MTBE (100 mL) at RT and filtered to give 5-bromo-4-chloro-2-methylpyridine 1-oxide (35.7 g, 160 mmol) as a white solid. $^1$H NMR (400 MHz, DMSO-*d6*) $\delta$ 8.73 (s, 1H), 7.85 (s, 1H), 2.28 (s, 3H).

**Step d:** To a mixture of 5-bromo-4-chloro-2-methylpyridine 1-oxide (45.0 g, 202 mmol) in $CHCl_3$ (225 mL) was added TFAA (84.4 mL, 606 mmol) at 0 °C under $N_2$. The mixture was stirred at 60 °C for 12 h. The reaction mixture was poured into water (500 mL). The pH of the aqueous layer was adjusted to pH ~ 8 with an aqueous solution of NaOH and extracted with DCM (500 mL * 3). The combined organic layer was dried over $Na_2SO_4$, filtered and concentrated under reduced pressure. The residue was purified by column chromatography ($SiO_2$, petroleum ether/ethyl acetate = 50/1 to 0/1) to give (5-bromo-4-chloro-2-pyridinyl)methanol (27 g, 121 mmol) as a white solid. $^1$H NMR (400 MHz, DMSO-*d6*) $\delta$ 8.74 (s, 1H), 7.66 (s, 1H), 4.53 (s, 2H).

**Step e:** To a mixture of (5-bromo-4-chloro-2-pyridinyl)methanol (15.0 g, 67.4 mmol) in DCM (75 mL) was added 3,4-dihydropyran (9.25 mL, 101 mmol) and p-toluenesulfonic acid monohydrate (1.28 g, 6.74 mmol). The reaction was stirred at RT for 1 h and then poured into water (100 mL). The pH of the aqueous layer was adjusted to pH ~ 7 with an aqueous solution of $NaHCO_3$ and extracted with DCM (200 mL * 3). The combined organic layer was dried over $Na_2SO_4$, filtered and concentrated under reduced pressure. The residue was purified by column chromatography ($SiO_2$, petroleum ether/ethyl acetate = 50/1 to 0/1) to give 5-bromo-4-chloro-2-(tetrahydropyran-2-yloxymethyl)pyridine (19.0 g, 61.9 mmol) as a colorless oil. $^1$H NMR (400 MHz, $CDCl_3$) $\delta$ 8.65 (s, 1H), 7.58 (s, 1H), 4.83 (d, *J* = 4.0 Hz, 1H), 4.75-4.77 (m, 1H), 4.56 (d, *J* = 8.0 Hz, 1H), 3.84-3.89 (m, 1H), 3.54-3.57 (m, 1H), 1.56-1.83 (m, 6H).

**Step f:** To a mixture of 5-bromo-4-chloro-2-(tetrahydropyran-2-yloxymethyl)pyridine (19.0 g, 61.9 mmol) in THF (47.5 mL) was added i-PrMgCl-LiCl (1.30 M, 57.2 mL, 74.4 mmol) at 0 °C under $N_2$. The mixture was stirred at 0 °C for 2 h and then a solution of *tert*-butyl 4-formyl-4-methylpiperidine-1-carboxylate (16.9 g, 74.3 mmol) in THF (47.5 mL) was added at 0 °C. The mixture was stirred at RT for 1 h and then poured into an aqueous solution of $NH_4Cl$ (400 mL) and extracted with ethyl acetate (500 mL * 3). The combined organic layer was dried over $Na_2SO_4$, filtered and concentrated under reduced pressure. The residue was purified by column chromatography ($SiO_2$, petroleum ether/ethyl acetate = 50/1 to 0/1) to give tert-butyl 4-[[4-chloro-6-(tetrahydropyran-2-yloxymethyl)-3-pyridyl]-hydroxymethyl]-4-methyl-piperidine-l-carboxylate (16.0 g, 35.1 mmol) as yellow oil. $^1$H NMR (400 MHz, $CDCl_3$) $\delta$ 8.68 (s, 1H), 7.51 (s, 1H), 4.97 (d, *J* = 4.0 Hz, 1H), 4.85-4.88 (m, 1H), 4.78-4.97 (m, 1H), 4.62-4.63 (m, 1H), 3.88-3.93 (m, 3H), 3.56-3.59 (m, 1H), 2.82-2.89 (m, 2H), 2.17 (s, 1H), 1.80-1.89 (m, 1H), 1.75-1.78 (m, 3H), 1.58-1.64 (m, 5H), 1.45 (s, 9H), 1.05 (s, 3H).

**Step g:** To a mixture of tert-butyl 4-[[4-chloro-6-(tetrahydropyran-2-yloxymethyl)-3-pyridyl]-hydroxymethyl]-4-methyl-piperidine-1-carboxylate (16.0 g, 35.1 mmol) ) in MeCN (80 mL) was added IBX (19.6 g, 70.3 mmol) at RT under $N_2$. The mixture was stirred at 50 °C for 2 h. The mixture was filtered and the filter liquor was concentrated under reduced pressure. The residue was purified by column chromatography ($SiO_2$, petroleum ether/ethyl acetate = 50/1 to 0/1) to give tert-butyl 4-[4-chloro-6-(tetrahydropyran-2-yloxymethyl)pyridine-3-carbonyl]-4-methylpiperidine-1-carboxylate (14.0 g, 30.9 mmol) as a yellow oil. $^1$H NMR (400 MHz, $CDCl_3$) $\delta$ 8.36 (s, 1H), 7.58 (s, 1H), 4.89 (d, *J* = 8.0 Hz, 1H), 4.77-4.79 (m, 1H), 4.63 (d, *J* = 8.0 Hz, 1H), 3.88-3.91 (m, 1H), 3.56-3.60 (m, 3H), 3.29-3.35 (m, 2H), 1.99-2.06 (m, 2H), 1.89-1.92 (m, 1H), 1.71-1.81 (m, 2H), 1.54-1.65 (m, 5H), 1.45 (s, 9H), 1.34 (s, 3H).

Step h: To a mixture of tert-butyl 4-[4-chloro-6-(tetrahydropyran-2-yloxymethyl)pyridine-3-carbonyl]-4-methyl-piperidine-1-carboxylate (12.0 g, 26.4 mmol) in mesitylene (60 mL) was added $Pd(OAc)_2$ (297 mg, 1.32 mmol), cesium carbonate (10.3 g, 31.7 mmol), tricyclohexylphosphine tetrafluoroborate (975 mg, 2.65 mmol) and pivalic acid (0.91 mL, 7.95 mmol) at RT under $N_2$. The mixture was degassed with $N_2$ for 10 min. The mixture was then stirred at 160 °C for 4 h. The reaction mixture was poured into water (300 mL) and extracted with ethyl acetate (100 mL * 3). The combined organic layer was dried over $Na_2SO_4$, filtered and concentrated under reduced pressure. The residue was purified by column chromatography ($SiO_2$, petroleum ether/ethyl acetate = 50/1 to 0/1) to give tert-butyl 7-oxo-3-(tetrahydropyran-2-yloxymethyl)spiro[5H-cyclopenta[c]pyridine-6,4'-piperidine]-1'-carboxylate (6.90 g, 16.2 mmol) as a yellow solid. $^1$H NMR (400 MHz, DMSO-*d6*) $\delta$ 8.81 (s, 1H), 7.68 (s, 1H), 4.78-4.84 (m, 2H), 4.66 (d, *J* = 8.0 Hz, 1H), 3.92-3.95 (m, 2H), 3.76-3.81 (m, 1H), 3.48-3.51 (m, 1H), 3.19 (s, 2H), 2.95 (s, 2H), 1.71-1.78 (m, 2H), 1.51-1.58 (m, 6H), 1.41 (s, 9H), 1.37-1.38 (m, 2H).

**Step i:** To a solution of tert-butyl 7-oxo-3-(tetrahydropyran-2-yloxymethyl)spiro[5H-cyclopenta[c]pyridine-6,4'-piperidine]-1'-carboxylate (1.5 g, 3.6 mmol) in 2-MeTHF (4.5 mL) was added $Ti(OEt)_4$ (4 mL, 18.0 mmol) and (R)-2-methylpropane-2-sulfinamide (1.1 g, 9.0 mmol). The mixture was stirred at 80 °C for 16 h. Water and dichloromethane were added and the mixture was filtered on a hydrophobic cartridge (liquid / liquid extraction column, Radleys®). The organic phase was washed with brine, dried with anhydrous $Na_2SO_4$, filtered and concentrated under vacuum. The residue was purified by column chromatography ($SiO_2$, cyclohexane/ethyl acetate = 1/1 to 1/9) to give tert-butyl rac-(7Z)-7-[(R)-tert-butylsulfinyl]imino-3-(tetrahydropyran-2-yloxymethyl)spiro[5H-cyclopenta[c]pyridine-6,4'-piperidine]-l'-carboxylate (1.3 g, 2.5 mmol) as a yellow wax. LCMS m/z $[M+H]^+$ 520.4.

**Step j:** To a solution of tert-butyl rac-(7Z)-7-[(R)-tert-butylsulfinyl]imino-3-(tetrahydropyran-2-yloxymethyl)spiro[5H-cyclopenta[c]pyridine-6,4'-piperidine]-1'-carboxylate (1.3 g, 2.5 mmol) in dry THF (25 mL) was added dropwise DIBAL-H (1 M in toluene, 5.3 mL, 5.3 mmol) at -78 °C under Ar. The mixture was stirred at -78 °C for 3 h. A concentrated aqueous solution of Rochelle salt (30 mL) and then ethyl acetate (30 mL) were added, the mixture was stirred at RT for 1 h. The organic layer was separated and the aqueous phase was extracted with ethyl acetate. The combined organic phase was washed with brine, dried with anhydrous $Na_2SO_4$, filtered and concentrated under vacuum. The residue was purified by column chromatography ($SiO_2$, cyclohexane/ethyl acetate = 1/0 to 0/1 followed by ethyl acetate/methanol 95/5) to give tert-butyl (7S)-7-[[(R)-tert-butylsulfinyl]amino]-3-(tetrahydropyran-2-yloxymethyl)spiro[5,7-dihydrocyclopenta[c]pyridine-6,4'-piperidine]-1'-carboxylate (1.14 g, 2.19 mmol) as a white foam. LCMS m/z $[M+H]^+$ 522.7.

**Step k:** To a solution of tert-butyl (7S)-7-[[(R)-tert-butylsulfinyl]amino]-3-(tetrahydropyran-2-yloxymethyl)spiro[5,7-dihydrocyclopenta[c]pyridine-6,4'-piperidine]-1'-carboxylate (1.13 g, 2.17 mmol) in dichloromethane (6 mL) was added TFA (2.23 mL, 29.5 mmol) at 0 °C. The mixture was stirred at RT for 5 h. Dichloromethane (10 mL) and water (10 mL) were added, the pH value of the aqueous phase was adjusted to 11-12 with 1 N aqueous NaOH solution and the mixture was filtered on a hydrophobic cartridge (liquid / liquid extraction column, Radleys®) and then concentrated in vacuum to give (R)-N-[(7S)-3-(hydroxymethyl)spiro[5,7-dihydrocyclopenta[c]pyridine-6,4'-piperidine]-7-yl]-2-methyl-propane-2-sulfinamide (0.43 g, crude) as a beige wax that was used without further purification.

**Example i-33. Intermediate B-24 ((R)-2-methyl-N-[(7S)-spiro[5,7-dihydrocyclopenta[c]pyridine-6,4'-piperidine]-7-yl]propane-2-sulfinamide)**

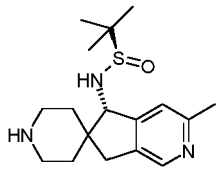

**Step a:** To a solution of 1-boc-4-cyanopiperidine (4.42 g, 20 mmol) in THF (50 mL) was added dropwise LDA (1 M THF:hexane, 21.4 mL, 21.4 mmol) at -78 °C. After addition, the mixture was stirred at this temperature for 1 h, and a solution of 4-bromo-5-(bromomethyl)-2-methyl-pyridine (4.42 g, 15.8 mmol) in THF (100 mL) was added dropwise. The resulting mixture was stirred at -78 °C for 30 min, then allowed to reach RT overnight. The reaction mixture was quenched by addition of an aqueous solution of $NH_4Cl$ (100 mL) at 0 °C, and extracted with EtOAc (50 mL * 3). The combined organic layers were washed with brine (50 mL), dried over $Na_2SO_4$, filtered and concentrated under reduced pressure. The residue was purified by column chromatography ($SiO_2$, heptane/ethyl acetate = 6/4) to give tert-butyl 4-[(4-bromo-6-methylpyridin-3-yl)methyl]-4-cyanopiperidine-1-carboxylate (5.31 g, 13.5 mmol) as an white solid. $^1H$ NMR (400 MHz, DMSO-*d6*) δ 8.45 (s, 1H), 7.63 (s, 1H), 4.05 (m, 2H), 3.10 (s, 2H), 2.82 (m, 2H), 2.45 (s, 3H), 1.81-1.89 (m, 2H), 1.62-1.71 (m, 2H), 1.40 (s, 9H). LCMS m/z $[M+H]^+$ 394.1.

**Step b:** A mixture of tert-butyl 4-[(4-bromo-6-methylpyridin-3-yl)methyl]-4-cyanopiperidine-1-carboxylate (5.29 g, 13.4 mmol), DIPEA (6.94 g, 53.7 mmol), $Pd(AmPhos)_2Cl_2$ (712 mg, 1.01 mmol) in DMA (270 mL) and $H_2O$ (30 mL) was degassed with $N_2$, then the mixture was stirred at 130 °C for 18 h under $N_2$ atmosphere. The reaction mixture was quenched by addition $H_2O$ (150 mL) at RT and extracted with EtOAc (200 mL * 3). The combined organic layers were washed with brine (50 mL * 3), dried over $Na_2SO_4$, filtered and concentrated under reduced pressure. The residue was purified by column chromatography ($SiO_2$, heptane/ethyl acetate = 80/20) to furnish crude product which was triturated in $iPr_2O$ (50 mL), iced and filtered to give tert-butyl 3-methyl-5-oxospiro[7H-cyclopenta[c]pyridine-6,4'-piperidine]-1'-carboxylate as an beige solid (3.05 g, 9.64 mmol). $^1H$ NMR (400 MHz, DMSO-*d6*) δ 8.82 (s, 1H), 7.45 (s, 1H), 3.88-3.99 (m, 2H), 3.12 (s, 2H), 2.89-3.07 (m, 2H), 2.56 (s, 3H), 1.53-1.63 (m, 2H), 1.46 (s, 9H), 1.39-1.40 (m, 2H). LCMS m/z $[M+H]^+$ 317.2.

**Step c:** To a solution of tert-butyl 3-methyl-5-oxospiro[7H-cyclopenta[c]pyridine-6,4'-piperidine]-1'-carboxylate (3.04 g, 9.61 mmol) in 2-MeTHF (20 mL) was added $Ti(OEt)_4$ (67% pure containing 33% of $TiO_2$, 12 mL, 38.4 mmol)

and (*R*)-2-methylpropane-2-sulfinamide (2.38 g, 19.2 mmol). The mixture was stirred at 90 °C for 16 h. The reaction mixture was quenched by addition of brine (20 mL), stirred 15 min before being filtered on dicalite. The filtrate solution was concentrated to dryness to give a yellow oil which was taken up in $Et_2O$ (50 mL) and the organic layers were washed with brine (20 mL * 3), dried over $Na_2SO_4$, filtered and concentrated under reduced pressure. The crude product was triturated with $iPr_2O$ (30 mL), iced and filtered to give tert-butyl (5E)-5-[(R)-tert-butylsulfinyl]imino-3-methylspiro[7H-cyclopenta[c]pyridine-6,4'-piperidine]-1'-carboxylate (3.38 g, 7.89 mmol) as a yellow powder. $^1H$ NMR (400 MHz, DMSO-*d6*) δ 8.73 (s, 1H), 8.02 (br s, 1H), 3.92-3.99 (m, 2H), 3.13 (s, 2H), 2.90-3.06 (m, 2H), 2.56 (s, 3H), 1.60-1.79 (m, 2H), 1.46-1.55 (m, 1.47 (s, 11H), 1.23 (s, 9H). LCMS m/z $[M+H]^+$ 420.2.

**Step d:** To a solution of tert-butyl (5E)-5-[(R)-tert-butylsulfinyl]imino-3-methylspiro[7H-cyclopenta[c]pyridine-6,4'-piperidine]-1'-carboxylate (3.10 g, 7.4 mmol) in THF (74 mL) was added dropwise DIBAL-H (1 M in toluene, 30 mL, 30 mmol) at -78 °C under $N_2$. The resulting mixture was stirred at -78 °C for 1.5 h. The reaction mixture was diluted with ethyl acetate (100 mL) at -65 °C, then quenched by addition of a solution of Rochelle salts (1 M, 41 mL). The mixture was stirred at RT for 1h, and then filtered. The separated organic phase was concentrated under reduced pressure. The residue was taken up in EtOAc (100 mL), toluene (10 mL) and brine (200 mL). The organic layer was separated, dried over $Na_2SO_4$, filtered and concentrated under reduced pressure. The crude product was triturated with $iPr_2O$ (50 mL) and AcOEt (40 mL) at RT for 1 h, iced and filtered to give tert-butyl (5S)-5-[[(R)-tert-butylsulfinyl]amino]-3-methylspiro[5,7-dihydrocyclopenta[c]pyridine-6,4'-piperidine]-1'-carboxylate (2.37 g, 5.61 mmol) as a white solid. $^1H$ NMR (400 MHz, DMSO-*d6*) δ 8.25 (s, 1H), 7.10 (s, 1H), 5.68 (d, *J* = 10.5 Hz, 1H), 4.42 (d, *J* = 10.5 Hz, 1H), 3.80-3.91 (m, 2H), 3.08 (d, *J* = 15.7 Hz, 1H), 2.72-3.01 (m, 2H), 2.60 (d, *J* = 15.7 Hz, 1H), 2.45 (m, 4H), 1.89-1.96 (m, 1H), 1.47-1.53 (m, 2H), 1.40 (s, 9H), 1.21 (s, 9H), 1.08 (m, 1H). LCMS m/z $[M+H]^+$ 422.7.

**Step e:** To a mixture of tert-butyl (5S)-5-[[(R)-tert-butylsulfinyl]amino]-3-methylspiro[5,7-dihydrocyclopenta[c]pyridine-6,4'-piperidine]-1'-carboxylate (975 mg, 2.31 mmol) in dichloromethane (8 mL) was added TFA (1.77 mL, 23.3 mmol) at RT. The mixture was stirred for 5 h and the mixture was poured in an aqueous 2 N solution of NaOH (30 mL). The mixture was filtered on a hydrophobic cartridge (liquid / liquid extraction column, Radleys®) and then concentrated in vacuum to give (R)-2-methyl-N-[(7S)-spiro[5,7-dihydrocyclopenta[c]pyridine-6,4'-piperidine]-7-yl]propane-2-sulfinamide (704 mg, 2.19 mmol) as a white solid. $^1H$ NMR (400 MHz, DMSO-*d6*) δ 8.27 (s, 1H), 7.08 (s, 1H), 5.63 (d, *J* = 10.4 Hz, 1H), 4.32 (d, *J* = 10.4 Hz, 1H), 3.03 (d, *J* = 15.7 Hz, 1H), 2.82-2.86 (m, 2H), 2.61-2.70 (m, 2H), 2.56 (d, *J* = 15.8 Hz, 1H), 2.43 (s, 3H), 1.86-1.94 (m, 2H), 1.38-1.58 (m, 2H), 1.40 (s, 9H), 1.22 (s, 9H), 1.03-1.07 (m, 2H). LCMS m/z $[M+H]^+$ 322.2.

**Example i-34. Intermediate B-25 ((R)-N-[(5S)-3-methoxyspiro[5,7-dihydrocyclopenta[c]pyridine-6,4'-piperidine]-5-yl]-2-methylpropane-2-sulfinamide)**

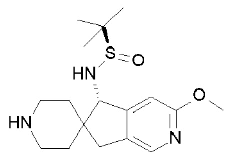

Step a: To a solution of 4-bromo-6-methoxypyridin-3-carbaldehyde (500 mg, 2.31 mmol) in THF (11 mL) and water (3 mL) was added $NaBH_4$ (96 mg, 2.54 mmol) at RT under $N_2$. Then the reaction was stirred for 45 min at RT. The residue was poured into a saturated aqueous solution of $NH_4Cl$ (15 mL). The aqueous phase was extracted with ethyl acetate (50 mL * 3). The combined organic phase was washed with brine (20 mL), dried with anhydrous $Na_2SO_4$, filtered and concentrated under vacuum to give (4-bromo-6-methoxy-3-pyridyl)methanol (417 mg, 1.91 mmol) as an orange solid. $^1H$ NMR (400 MHz, DMSO-*d6*) δ 8.17 (s, 1H), 7.13 (s, 1H), 5.30 (t, *J* = 5.4 Hz, 1H), 4.48 (d, *J* = 5.2 Hz, 2H), 3.85 (s, 3H). LCMS m/z $[M+H]^+$ 218.0.

Step b: To a solution of (4-bromo-6-methoxy-3-pyridyl)methanol (415 mg, 1.90 mmol) inDCM (9.5 mL) was added DMF (0.5 mL) and then dropwise $SOCl_2$ (271 mg, 2.28 mmol) at 0 °C under $N_2$. The mixture was stirred at RT for 1 h. The reaction mixture was quenched by addition of an aqueous solution of $NaHCO_3$ (5 mL) at 10 °C. The mixture was filtered on a hydrophobic cartridge (liquid / liquid extraction column, Radleys®) and then concentrated in vacuum to give 4-bromo-5-(chloromethyl)-2-methoxypyridine (365 mg, 1.54 mmol) as a yellow solid. $^1H$ NMR (400 MHz, DMSO-*d6*) δ 8.36 (s, 1H), 7.24 (s, 1H), 4.81 (s, 2H), 3.87 (s, 3H). LCMS m/z $[M+H]^+$ 236.0.

**Step c:** To a solution of 1-boc-4-cyanopiperidine (1.64 g, 7.4 mmol) in THF (45 mL) was added dropwise LDA (1 M THF:hexane, 7.8 mL, 7.8 mmol) at -78 °C. After addition, the mixture was stirred at this temperature for 1 h, and a solution of 4-bromo-5-(chloromethyl)-2-methoxypyridine (1.58 g, 6.74 mmol) in THF (15 mL) was added dropwise. The resulting mixture was stirred at -78 °C for 30 min then allowed to reach room temperature overnight. The reaction

mixture was quenched by addition of $NH_4Cl$ (100 mL) at 0 °C, and extracted with EtOAc (50 mL * 3). The combined organic layers were washed with brine (50 mL), dried over $Na_2SO_4$, filtered and concentrated under reduced pressure. The residue was purified by column chromatography ($SiO_2$, heptane/ethyl acetate = 8/2 followed by a second column, $SiO_2$, dichloromethane/methanol containing 10% of $NH_4OH$ 99.5/0.5) to give tert-butyl 4-[(4-bromo-6-methylpyridin-3-yl)methyl]-4-cyanopiperidine-1-carboxylate (0.82 g, 2.0 mmol) as a colorless gummy solid. $^1H$ NMR (400 MHz, DMSO-*d6*) δ 8.20 (s, 1H), 7.20 (s, 1H), 4.01 (m, 2H), 3.87 (s, 3H), 3.06 (s, 2H), 2.81 (m, 2H), 2.45 (s, 3H), 1.83-1.86 (m, 2H), 1.58-1.66 (m, 2H), 1.40 (s, 9H). LCMS m/z $[M+H]^+$ 410.1.

**Step d:** A mixture of tert-butyl 4-[(4-bromo-6-methylpyridin-3-yl)methyl]-4-cyanopiperidine-1-carboxylate (710 mg, 1.73 mmol), DIPEA (1.21 mL, 6.92 mmol), $Pd(AmPhos)_2Cl_2$ (62 mg, 0.09 mmol) in DMA (27 mL) and $H_2O$ (3 mL) was degassed with $N_2$, then the mixture was stirred at 130 °C for 3 h. $Pd(AmPhos)_2Cl_2$ (62 mg, 0.09 mmol) was added and the mixture was stirred at 130 °C for another 3 h. Ethyl acetate was added and the organic layer were washed with water, brine, dried over $Na_2SO_4$, filtered and concentrated under reduced pressure. The residue was purified by column chromatography ($SiO_2$, heptane/ethyl acetate = 7/3) to give tert-butyl 3-methoxy-5-oxospiro[7H-cyclopenta[c]pyridine-6,4'-piperidine]-1-carboxylate (380 mg, 1.14 mmol) as a beige solid. $^1H$ NMR (400 MHz, DMSO-*d6*) δ 8.53 (s, 1H), 6.96 (s, 1H), 3.94 (m, 2H), 3.90 (s, 3H), 2.97-3.08 (m, 4H), 1.53-1.60 (m, 2H), 1.43 (s, 9H), 1.39-1.40 (m, 2H). LCMS m/z $[M+H]^+$ 333.2.

**Step e:** To a solution of tert-butyl 3-methoxy-5-oxospiro[7H-cyclopenta[c]pyndine-6,4'-pipendine]-1'-carboxylate (440 mg, 1.32 mmol) in 2-MeTHF (3 mL) was added $Ti(OEt)_4$ (1.7 mL, 5.3 mmol) and (*R*)-2-methylpropane-2-sulfinamide (327 mg, 2.65 mmol). The mixture was stirred at 80 °C for 3 h. Ethyl acetate (20 mL) and then water (2 mL) were added. The mixture was filtered on dicalite and concentrated to dryness. The residue was taken up in ethyl acetate (50 mL) and the organic layer was washed with brine (20 mL * 3), dried over $Na_2SO_4$, filtered and concentrated under reduced pressure. The residue was purified by column chromatography ($SiO_2$, heptane/ethyl acetate = 6/4) to give tert-butyl (5E)-5-[(R)-tert-butylsulfinyl]imino-3-methoxyspiro[7H-cyclopenta[c]pyridine-6,4'-piperidine]-1'-carboxylate (525 mg, 1.21 mmol) as a yellow semi solid.$^1H$ NMR (400 MHz, DMSO-*d6*) δ 8.41 (s, 1H), 7.62 (br s, 1H), 3.92-3.95 (m, 2H), 3.88 (s, 3H), 3.07 (s, 2H), 2.95-2.97 (m, 2H), 1.44-1.66 (m, 4H), 1.41 (s, 9H), 1.24 (s, 9H). LCMS m/z $[M+H]^+$ 436.2.

**Step f:** To a solution of tert-butyl (5E)-5-[(R)-tert-butylsulfinyl]imino-3-methoxyspiro[7H-cyclopenta[c]pyridine-6,4'-piperidine]-1'-carboxylate (525 mg, 1.21 mmol) in THF (12 mL) was added dropwise DIBAL-H (1 M in toluene, 3.6 mL, 3.6 mmol) at -78 °C under $N_2$. The resulting mixture was stirred at -78 °C for 1 h. The reaction mixture was diluted with ethyl acetate (30 mL) at -65 °C, then quenched by addition of a 1 M aqueous solution of Rochelle salts (10 mL). and stirred at RT for 1h, followed by filtration. The organic phase was separated and then concentrated under reduced pressure. The residue was taken up in EtOAc (30 mL) and brine (50 mL), the organic layer was separated and then dried over $Na_2SO_4$, filtered and concentrated under reduced pressure. The crude product was triturated with $iPr_2O$ (50 mL), iced and filtered to give tert-butyl (5S)-5-[[(R)-tert-butylsulfinyl]amino]-3-methoxyspiro[5,7-dihydrocyclopenta[c]pyridine-6,4'-piperidine]-1'-carboxylate (524 mg, 1.14 mmol) as a beige powder. $^1H$ NMR (400 MHz, DMSO-*d6*) δ 7.96 (s, 1H), 6.59 (s, 1H), 5.73 (d, *J*= 10.5 Hz, 1H), 4.39 (d, *J* = 10.5 Hz, 1H), 3.86-3.90 (m, 2H), 3.80 (s, 3H), 3.04 (d, *J* = 15.5 Hz, 1H), 2.61-2.96 (m, 2H), 2.55 (m 1H), 1.92 (m, 1H), 1.44-1.60 (m, 2H), 1.40 (s, 9H), 1.21 (s, 9H), 1.03 (m, 1H). LCMS m/z $[M+H]^+$ 438.1.

**Step g:** To a mixture of tert-butyl (5S)-5-[[(R)-tert-butylsulfinyl]amino]-3-methoxyspiro[5,7-dihydrocyclopenta[c]pyridine-6,4'-piperidine]-1'-carboxylate (520 mg, 1.13 mmol) in dichloromethane (4 mL) was added TFA (0.9 mL, 11.29 mmol) at RT. The mixture was stirred for 1.5 h and then poured into an aqueous 2 N solution of NaOH (10 mL). The mixture was filtered on a hydrophobic cartridge (liquid / liquid extraction column, Radleys®) and then concentrated in vacuum to give (R)-N-[(5S)-3-methoxyspiro[5,7-dihydrocyclopenta[c]pyridine-6,4'-piperidine]-5-yl]-2-methyl-propane-2-sulfinamide (380 mg, 1.13 mmol) as a white solid. $^1H$ NMR (400 MHz, DMSO-*d6*) δ 7.96 (s, 1H), 6.60 (s, 1H), 5.66 (d, *J* = 10.4 Hz, 1H), 4.29 (d, *J* = 10.1 Hz, 1H), 3.80 (s, 3H), 3.01 (d, *J* = 15.4 Hz, 1H), 2.79-2.86 (m, 2H), 2.57-2.66 (m, 2H), 2.48 (m, 1H), 1.93 (m, 1H), 1.37-1.51 (m, 2H), 1.21 (s, 9H), 0.98-1.02 (m, 1H). LCMS m/z $[M+H]^+$ 338.1.

**Example i-35. Intermediate B-26 ((R)-N-[(7S)-3-methoxyspiro[5,7-dihydrocyclopenta[c]pyridine-6,4'-piperidine]-7-yl]-2-methylpropane-2-sulfinamide)**

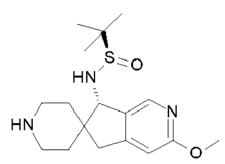

Step a: To a solution of 1-boc-4-cyanopiperidine (1.56 g, 7.44 mmol) in THF (30 mL) was added dropwise LDA

(1 M in THF:hexane, 7.8 mL, 7.8 mmol) at -78 °C. After addition, the mixture was stirred at this temperature for 1 h, and a solution of 5-bromo-4-(bromomethyl)-2-methoxypyridine (2.00 g, 6.76 mmol) in THF (10 mL) was added dropwise. The resulting mixture was stirred at -78 °C for 30 min then allowed to reach room temperature overnight. The reaction mixture was quenched by addition of an aqueous solution of $NH_4Cl$ (100 mL) at 0 °C, and extracted with EtOAc (50 mL * 3). The combined organic layers were washed with brine (50 mL), dried over $Na_2SO_4$, filtered and concentrated under reduced pressure. The residue was purified by column chromatography ($SiO_2$, heptane/ethyl acetate = 9/1 to 8/2 followed by a second column, $SiO_2$, dichloromethane/methanol 99.5/0.5) to give tert-butyl 4-[(5-bromo-2-methoxypyridin-4-yl)methyl]-4-cyanopiperidine-1-carboxylate (1.68 g, 4.1 mmol, purity 62%) as a colorless oil. $^1H$ NMR (400 MHz, DMSO-*d6*) δ 8.37 (s, 1H), 6.96 (s, 1H), 4.02 (m, 2H), 3.86 (s, 3H), 3.72 (s, 1H), 3.08 (s, 2H), 1.84-1.87 (m, 2H), 1.60-1.68 (m, 2H), 1.40 (s, 9H), 0.84-0.88 (m, 1H). LCMS m/z $[M+H]^+$ 410.0.

**Step b:** A mixture of tert-butyl 4-[(5-bromo-2-methoxypyridin-4-yl)methyl]-4-cyanopiperidine-1-carboxylate (993 mg, 2.42 mmol, purity 62%), DIPEA (1.6 g, 12.4 mmol), $Pd(AmPhos)_2Cl_2$ (171 mg, 0.25 mmol) in DMA (5 mL) and $H_2O$ (1 mL) was degassed with $N_2$, then the mixture was stirred at 130 °C for 18 h under $N_2$ atmosphere. Water and ethyl acetate were added, the aqueous layer was separated and then extracted with ethyl acetate. The combined organic layers were washed with water, dried over $Na_2SO_4$, filtered and concentrated under reduced pressure. The residue was purified by column chromatography ($SiO_2$, heptane/ethyl acetate = 85/15) to give tert-butyl 3-methoxy-7-oxospiro[5H-cyclopenta[c]pyridine-6,4'-piperidine]-1'-carboxylate (406 mg, 1.22 mmol) as a yellow oil. $^1H$ NMR (400 MHz, DMSO-*d6*) δ 8.53 (s, 1H), 6.96 (s, 1H), 3.94 (m, 2H), 3.90 (s, 3H), 2.97-3.08 (m, 4H), 1.53-1.60 (m, 2H), 1.39-1.43 (m, 11H). LCMS m/z $[M+H]^+$ 333.2.

**Step c:** To a solution of tert-butyl 3-methoxy-7-oxospiro[5H-cyclopenta[c]pyndine-6,4'-pipendine]-1'-carboxylate (577mg, 1.74 mmol) in 2-MeTHF (3 mL) was added $Ti(OEt)_4$ (67% pure containing 33% of $TiO_2$, 2.17 mL, 6.94 mmol) and (*R*)-2-methylpropane-2-sulfinamide (421 mg, 3.47 mmol). The mixture was stirred at 90 °C for 3 days. Ethyl acetate (20 mL) and then water (3 mL) were added. The mixture was stirred for 45 min at RT and then filtered on dicalite. The organic layer was washed with water, (20 mL * 3), dried over $Na_2SO_4$, filtered and concentrated under reduced pressure. The residue was purified by column chromatography ($SiO_2$, heptane/ethyl acetate = 8/2) to give tert-butyl (7E)-7-[(R)-tert-butylsulfinyl]imino-3-methoxyspiro[5H-cyclopenta[c]pyridine-6,4'-piperidine]-1'-carboxylate (509 mg, 1.17 mmol) as a yellow solid. $^1H$ NMR (400 MHz, DMSO-*d6*) δ 9.09 (s, 1H), 6.92 (s, 1H), 3.94 (m, 5H), , 3.10 (m, 2H), 2.93-2.95 (m, 2H), 1.66-1.69 (m, 2H), 1.41 (m, 11H), 1.23 (s, 9H). LCMS m/z $[M+H]^+$ 436.1.

**Step d:** To a solution of tert-butyl (7E)-7-[(R)-tert-butylsulfinyl]imino-3-methoxyspiro[5H-cyclopenta[c]pyridine-6,4'-piperidine]-1'-carboxylate (467 mg, 1.07 mmol) in THF (6 mL) was added dropwise DIBAL-H (1 M in toluene, 4.3 mL, 4.3 mmol) at -78 °C under $N_2$. The resulting mixture was stirred at -78 °C for 1 h. Ethyl acetate (25 mL) was added at -65 °C, followed by a 1 M aqueous solution of Rochelle salts (10 mL), the reaction mixture was stirred for 2 h at RT. The organic layer was separated and then washed with water (15 mL * 3), dried over $Na_2SO_4$, filtered and concentrated under reduced pressure to give tert-butyl (7S)-7-[[(R)-tert-butylsulfinyl]amino]-3-methoxyspiro[5,7-dihydrocyclopenta[c]pyridine-6,4'-piperidine]-1'-carboxylate (393 mg, 0.90 mmol) as a beige solid. $^1H$ NMR (400 MHz, DMSO-*d6*) δ 7.97 (s, 1H), 6.64 (s, 1H), 5.62 (d, *J* = 10.1 Hz, 1H), 4.38 (d, *J* = 10.0 Hz, 1H), 3.80 (m, 5H), 2.99 (d, *J* = 16.7 Hz, 1H), 2.87-2.92 (m, 2H), 2.61 (d, *J* = 16.6 Hz, 1H), 2.60 (m, 1H), 1.74-1.79 (m, 1H), 1.54-1.59 (m, 1H), 1.40 (s, 9H), 1.19 (m, 10H). LCMS m/z $[M+H]^+$ 438.2.

**Step e:** To a mixture tert-butyl (7S)-7-[[(R)-tert-butylsulfinyl]amino]-3-methoxyspiro[5,7-dihydrocyclopenta[c]pyridine-6,4'-piperidine]-1'-carboxylate (393 mg, 0.9 mmol) in dichloromethane (2.5 mL) was added TFA (0.7 mL, 9 mmol) at RT. The mixture was stirred for 1.5 h and the mixture was poured in an aqueous 2 N solution of NaOH (10 mL). The mixture was filtered on a hydrophobic cartridge (liquid / liquid extraction column, Radleys®) and then concentrated in vacuum to give (R)-N-[(7S)-3-methoxyspiro[5,7-dihydrocyclopenta[c]pyridine-6,4'-piperidine]-7-yl]-2-methylpropane-2-sulfinamide (249 mg, 0.82 mmol) as a white solid. $^1H$ NMR (400 MHz, DMSO-*d6*) δ 8.00 (s, 1H), 6.64 (s, 1H), 5.73 (d, *J* = 10.0 Hz, 1H), 4.42 (d, *J* = 9.9 Hz, 1H), 3.81 (s, 3H), 3.10-3.16 (m, 2H), 3.03 (d, *J* = 16.8 Hz, 1H), 2.84-2.93 (m, 2H), 2.70 (m, 1H), 2.30 (m, 1H), 1.87 (m, 1H), 1.76-1.84 (m, 2H), 1.35-1.55 (m, 1H), 1.19 (s, 9H). LCMS m/z $[M+H]^+$ 338.2.

**Example i-36. Intermediate B-27 (4-methoxyspiro[3H-indene-2,4'-piperidine]-1-one)**

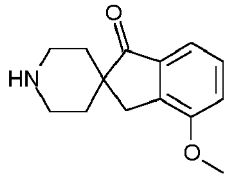

**Step a:** To a solution of 1-boc-4-cyanopiperidine (3.74 g, 17.8 mmol) in THF (100 mL) was added dropwise LDA (1 M THF/hexane, 20 mL, 20 mmol) at -78 °C. After addition, the mixture was stirred at this temperature for 1 h, and a solution of 1-bromo-2-(bromomethyl)-3-methoxy-benzene (4.5 g, 16 mmol) in THF (4 mL) was added dropwise. The resulting mixture was stirred at -78 °C for 30 min then allowed to reach room temperature overnight. The reaction mixture was quenched by addition of an aqueous solution of $NH_4Cl$ (100 mL) at 0 °C, and extracted with EtOAc (50 mL * 3). The combined organic layers were washed with brine (50 mL), dried over $Na_2SO_4$, filtered and concentrated under reduced pressure. The residue was purified by column chromatography ($SiO_2$, heptane/ethyl acetate = 85/15) to give tert-butyl 4-[(2-bromo-6-methoxy-phenyl)methyl]-4-cyano-piperidine-1-carboxylate (4.6 g, 11.2 mmol) as a colorless semi solid. $^1H$ NMR (400 MHz, DMSO-*d6*) $\delta$ 7.22-7.24 (m, 2H), 7.05-7.08 (m, 1H), 3.95-3.99 (m, 2H), 3.79 (s, 3H), 3.11 (m, 2H), 2.84-2.90 (m, 2H), 1.84-1.87 (m, 2H), 1.59-1.67 (m, 2H), 1.40 (s, 9H). LCMS m/z $[M+H]^+$ 409.1.

**Step b:** A mixture tert-butyl 4-[(2-bromo-6-methoxyphenyl)methyl]-4-cyanopiperidine-1-carboxylate (4.6 g, 11 mmol), DIPEA (7.3 g, 56.5 mmol), $Pd(AmPhos)_2Cl_2$ (820 mg, 1.16 mmol) in DMA (100 mL) and $H_2O$ (15 mL) was degassed with Ar, then the mixture was stirred at 130 °C for 18 h. Water and ethyl acetate were added, the aqueous layer was separated and then extracted with ethyl acetate. The combined organic layers were washed with water, dried over $Na_2SO_4$, filtered and concentrated under reduced pressure to give crude tert-butyl 4-methoxy-1-oxo-spiro[indane-2,4'-piperidine]-1'-carboxylate that was used in the next step without furhter purification.

**Step c:** A mixture of crude tert-butyl 4-methoxy-1-oxo-spiro[indane-2,4'-piperidine]-1'-carboxylate previously obtained in methanol (40 mL) and in an aqueous solution of HCl (2 M, 55 mL, 110 mmol) was stirred at reflux for 2 h Methanol was then evaporated under reduced pressure. Aqueous 2 N solution of NaOH and ethyl acetate were added. The organic layer was separated, washed with brine, dried over $Na_2SO_4$, filtered and concentrated under reduced pressure. The residue was purified by column chromatography ($SiO_2$, dichloromethane/ methanol = 90/10 and then dichloromethane/ methanol containing 10% of conc. $NH_{3aq}$ = 90/10) to give 4-methoxyspiro[indane-2,4'-piperidine]-1-one (2.17 g, 9.4 mmol) as an orange viscous oil. $^1H$ NMR (400 MHz, DMSO-*d6*) $\delta$ 7.40-7.42 (m, 1H), 7.22-7.27 (m, 2H), 3.88 (s, 3H), 2.93-3.00 (m, 5H), 2.66 (m, 2H), 1.62-1.70 (m, 2H), 1.23-1.34 (m, 2H). LCMS m/z $[M+H]^+$231.1.

**Example i-37. Intermediate B-28 (6-methoxyspiro[indene-2,4'-piperidin]-1(3H)-one hydrochloride)**

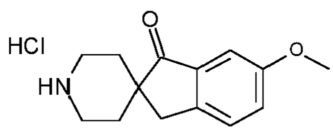

**Step a:** To a solution of 1-boc-4-cyanopiperidine (2.43 g, 10.97 mmol) in dry THF (70 mL) was added dropwise LDA (1 M THF/hexane, 11.5 mL, 11.5 mmol) at -78 °C. After addition, the mixture was stirred at this temperature for 1 h, and a solution of 2-bromo-1-(bromomethyl)-4-methoxybenzene (2.94 g, 9.98 mmol) in dry THF (12 mL) was added dropwise. The resulting mixture was stirred at -78 °C for 30 min then allowed to reach room temperature overnight. The reaction mixture was quenched by addition of an aqueous solution of $NH_4Cl$ (100 mL) at 0 °C, and extracted with EtOAc (50 mL * 3). The combined organic layers were washed with brine (50 mL), dried over $Na_2SO_4$, filtered and concentrated under reduced pressure. The residue was purified by column chromatography ($SiO_2$, heptane/ethyl acetate = 70/30) to give tert-butyl 4-(2-bromo-4-methoxybenzyl)-4-cyanopiperidine-1-carboxylate (3.87 g, 9.44 mmol) as a white solid. LCMS m/z$[M+H]^+$ 409.2 .

**Step b:** A mixture tert-butyl 4-(2-bromo-4-methoxybenzyl)-4-cyanopiperidine-1-carboaylate (3.78 g, 9.24 mmol), DIPEA (8.3 mL, 47.2 mmol), $Pd(AmPhos)_2Cl_2$ (650 mg, 0.92 mmol) in DMA (20 mL) and $H_2O$ (3.4 mL) was degassed with Ar, then the mixture was stirred at 130 °C for 18 h. Water and ethyl acetate were added, the aqueous layer was separated and then extracted with ethyl acetate. The combined organic layers were washed with water, dried over $Na_2SO_4$, filtered and concentrated under reduced pressure. The residue was purified by column chromatography ($SiO_2$, heptane/ethyl acetate = 9/1 to 7/3) to give tert-butyl 6-methoxy-1-oxo-1,3-dihydrospiro[indene-2,4'-piperidine]-1'-carboxylate (2.76 g, 8.3 mmol). LCMS m/z $[M+H]^+$ = 332.2.

**Step c:** To a solution of tert-butyl 6-methoxy-1-oxo-1,3-dihydrospiro[indene-2,4'-piperidine]-1'-carboxylate (2.37 g, 7.15 mmol) in methanol (70 mL) was added a solution of HCl (4 M in dioxane, 12.5 mL, 50 mmol). The mixture was stirred for 18 h and then concentrated under reduced pressure. The residue was triturated in $iPr_2O$ to give 6-methoxyspiro[indene-2,4'-piperidin]-1(3H)-one hydrochloride (1.9 g, 7.15 mmol) as a white solid, that was used without further purification. LCMS m/z $[M+H]^+$ = 232.1.

**Example i-38. Intermediate B-29 ((3R)-spiro[1,3-dihydroindole-2,4'-piperidine]-3-amine hydrochloride)**

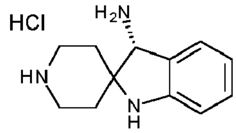

**Step a:** To a solution of 1-boc-4-piperidone (11.58 g, 58.13 mmol) and 2-bromoaniline (10 g, 53.13 mmol) in dry glacial acetic acid (80 mL) was cautiously added dropwise trimethylsilyl cyanide (8.0 mL, 63.95 mmol) at RT. The mixture was stirred at RT for 18 h. 5 M aqueous solution of NaOH (100 mL) and EtOAc (50 mL) were added, the aqueous layer was separated and then extracted with EtOAc (50 mL * 2). The combined organic layers were washed with brine (50 mL), dried over $Na_2SO_4$, filtered and concentrated under reduced pressure. The residue was triturated in a mixture of $iPr_2O$ (30 mL) and petroleum ether (50 mL), iced and filtered to give tert-butyl 4-(2-bromoanilino)-4-cyanopiperidine-1-carboxylate (19.24 g, 50.59 mmol) as an white powder. $^1H$ NMR (400 MHz, DMSO-*d6*) δ 7.56 (dd, *J* = 7.9, , 1.5 Hz 1H); 7.31 (td, *J* = 7.8, 1.4 Hz 1H); 7.17 (dd, *J* = 7.9, 1.5 Hz 1H); 6.80 (td, *J* = 7.5, 1.5 Hz 1H); 5.20 (s, 1H); 3.68 (m, 2H); 3.23 (m, 2H); 2.32 (m, 2H); 1.95 (m, 2H); 1.41 (s, 9H). LCMS m/z $[M-100+H_2O]^+$ 297.0; $[M+Na]^+$ 402.1.

**Step b:** A mixture of tert-butyl 4-(2-bromoanilino)-4-cyano-piperidine-1-carboaylate (10 g, 26.3 mmol), triethylamine (14.7 mL, 105.2 mmol), $Pd(AmPhos)_2Cl_2$ (1.86 g, 2.63 mmol) in DMA (180 mL) and $H_2O$ (40 mL) was degassed with Ar, then the mixture was stirred at 120 °C for 18 h. Water and ethyl acetate were added, the aqueous layer was separated and then extracted with ethyl acetate. The combined organic layers were washed with water, dried over $Na_2SO_4$, filtered and concentrated under reduced pressure. The residue was purified by column chromatography ($SiO_2$, heptane/ethyl acetate = 1/0 to 65/35) to give tert-butyl 3-oxospiro[indoline-2,4'-piperidine]-1'-carboaylate (3.05 g, 10.2 mmol). $^1H$ NMR (400 MHz, DMSO-*d6*) δ 7.82 (s, 1H); 7.45 (m, 2H); 6.89 (d, *J* = 8.4 Hz, 1H); 6.71 (m, 1H); 3.96 (m, 2H); 3.13 (br s 1, 2H); 1.59 (m, 2H); 1.42 (s, 9H); 1.29 (m, 2H). LCMS m/z $[M-H]^-$ 301.1

**Step c:** To a solution of tert-butyl 3-oxospiro[indoline-2,4'-piperidine]-1'-carboaylate (3.75 g, 12.4 mmol) in 2-MeTHF (30 mL) and $Ti(OEt)_4$ (38.7 mL) was added (*R*)-2-methylpropane-2-sulfinamide (6.0 g, 49.6 mmol). The mixture was stirred at 90 °C for 16 h. (*R*)-2-methylpropane-2-sulfinamide (6.0 g, 49.6 mmol) was added and the mixture was stirred at 90 °C for 3 days. Ethyl acetate and then brine (10 mL) were added. The mixture was stirred for 15 min at RT and then filtered on dicalite and concentrated under reduced pressure. The residue was taken up in $Et_2O$ (50 mL) and the organic layer was washed with brine (20 mL * 2), dried over $Na_2SO_4$, filtered and concentrated under reduced pressure. The residue was purified by column chromatography ($SiO_2$, heptane/ethyl acetate = 1/1) to give tert-butyl (3E)-3-[(R)-tert-butylsulfinyl]iminospiro[indoline-2,4'-piperidine]-1'-carboxylate (2.05 g, 5.05 mmol) as a yellow solid. $^1H$ NMR (400 MHz, DMSO-*d6*) δ 8.04-8.00 (m, 1H); 7.42-7.38 (m, 1H); 6.84-6.82 (m, 1H); 6.72-6.68 (m, 1H); 4.05-3.95 (m, 2H); 3.1 (br s, 2H); 1.74-1.60 (m, 2H); 1.42 (s, 9H); 1.40-1.33 (m, 2H); 1.19 (s, 9H). LCMS m/z $[M+H]^+$ 406.6.

**Step d:** To a solution of tert-butyl (3E)-3-[(R)-tert-butylsulfinyl]iminospiro[indoline-2,4'-piperidine]-1'-carboxylate (1.37 g, 3.38 mmol) in THF (40 mL) was added dropwise DIBAL-H (1 M in toluene, 13.5 mL, 13.5 mmol) at -78 °C under $N_2$. The resulting mixture was stirred at -78 °C for 1.5 h. Ethyl acetate (100 mL) was added at -65 °C, followed by a 1 M aqueous solution of Rochelle salts (20 mL), the reaction mixture was stirred for 1 h at RT. The aqueous layer was separated and extracted with ethyl acetate. The combined organic layer was then washed with brine, dried over $Na_2SO_4$, filtered and concentrated under reduced pressure. The residue was purified by column chromatography ($SiO_2$, heptane/ethyl acetate = 45/55) to furnish the expected product which was triturated with $iPr_2O$ (15 mL), iced and filtered to give tert-butyl (3S)-3-[[(R)-tert-butylsulfinyl]amino]spiro[indoline-2,4'-piperidine]-1'-carboxylate (1.15 g, 2.82 mmol) as a white powder. $^1H$ NMR (400 MHz, DMSO-*d6*) δ 7.05 (d, *J* = 7.3 Hz 1H); 7.01-6.97 (m, 1H); 6.59-6.54 (m, 1H); 6.49 (d, *J* = 7.5 Hz 1H); 6.15 (s, 1H); 5.75 (d, *J*= 10.5 Hz 1H); 4.38 (d, *J*= 10.6 Hz 1H); 3.91-3.79 (m, 2H); 3.02 (s br, 2H); 1.81 (td, *J* = 13.0, 3.7 Hz 1H); 1.67 (td, *J* = 12.5, 4.3 Hz 1H); 1.50-1.43 (m, 2H); 1.41 (s, 9H); 1.20 (s, 9H). LCMS m/z $[M+H]^+$ 408.2.

**Step e:** To a mixture of tert-butyl (3S)-3-[[(R)-tert-butylsulfinyl]amino]spiro[indoline-2,4'-pipendine]-1'-carboxylate (950 mg, 2.3 mmol) in methanol (30 mL) was added dropwise a solution of HCl (4 M in dioxane, 8.2 mL, 32.6 mmol) at RT. The mixture was stirred at RT for18 h and then concentrated under reduced pressure. The residue was triturated into acetonitrile (4 mL), iced and filtered to give crude (3R)-spiro[1,3-dihydroindole-2,4'-piperidine]-3-amine hydrochloride (707 mg) as a white solid that was used without further purification. $^1H$ NMR (400 MHz, DMSO-*d6)* δ 9.23 (br d, *J* = 9.6 Hz 1H) ; 9.00 (br d, *J* = 9.6 Hz 1H) ; 8.45 (br s, 3H) ; 7.43 (d, *J* = 7.2 Hz 1H) ; 7.13-7.17 (m, 1 H) ; 6.66-6.70 (m, 1H); 6.62 (m, 1H); 4.41 (m, 1H) ; 3.10-3.40 (m, 4H) ; 2.16-2.23 (m, 1H) ; 1.78-1.85 (m, 2H); 1.61-1.65 (m, 1H).

**Example i-39. Intermediate B-30 ((3R)-1-methylspiro[3H-indole-2,4'-pipeiidine]-3-aminehydrochloride)**

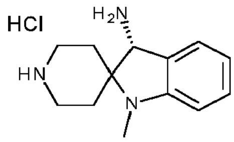

**Step a:** To a solution of tert-butyl (3E)-3-[(R)-tert-butylsulfinyl]iminospiro[indoline-2,4'-piperidine]-1'-carboxylate (1.94 g, 4.78 mmol) in THF (70 mL) under inert atmosphere was added NaHMDS (1 M in THF, 7 mL, 7 mmol) at 0 °C. After addition, the mixture was stirred at this temperature for 30 min, and methyliodide (15 mL, 24 mmol) was added dropwise. The reaction mixture was stirred at 0 °C for 30 min. The reaction mixture was poured into a saturated aqueous solution of sodium bicarbonate (500 mL), and extracted with EtOAc (250 mL ∗ 3). The combined organic layers were washed with brine (500 mL), dried over $Na_2SO_4$, filtered and concentrated under reduced pressure. The residue was purified by column chromatography ($SiO_2$, heptane/ethyl acetate = 1/1) to give tert-butyl (3E)-3-[(R)-tert-butylsulfinyl]imino-1-methyl-spiro[indoline-2,4'-piperidine]-1'-carboxylate (1.89 g, 4.50 mmol) as a yellow solid. $^1$H NMR (400 MHz, DMSO-*d6*) δ 8. 02 (d, *J* = 7.4 Hz 1H); 7.47-7.43 (m, 1H); 6.86 (d, *J* = 8.3 Hz, 1H); 6.73-6.69 (m, 1H); 3.88-3.77 (m, 2H); 3.73-3.60 (m, 2H); 2.89 (s, 3H); 1.99-1.87 (m, 2H); 1.57-1.47 (m, 2H); 1.43 (s, 9H); 1.22 (s, 9H). LCMS m/z $[M+H]^+$ 420.1.

**Step b:** To a solution of tert-butyl (3E)-3-[(R)-tert-butylsulfinyl]imino-l-methyl-spiro[indoline-2,4'-piperidine]-1'-carboxylate (1.89 g, 4.50 mmol) in THF (60 mL) was added dropwise DIBAL-H (1 M in toluene, 18 mL) at -78 °C under $N_2$. The resulting mixture was stirred at -78 °C for 1.5 h. Ethyl acetate (100 mL) was added at -65 °C, followed by a 1 M aqueous solution of Rochelle salts (20 mL), the reaction mixture was stirred for 1 h at RT. The aqueous layer was separated and extracted with ethyl acetate. The combined organic layer was then washed with brine, dried over $Na_2SO_4$, filtered and concentrated under reduced pressure. The residue was purified by column chromatography ($SiO_2$, heptane/ethyl acetate = 2/3) to give tert-butyl (3S)-3-[[(R)-tert-butylsulfinyl]amino]-l-methyl-spiro[indoline-2,4'-piperidine]-1'-carboaylate (1.13 g, 2.68 mmol) as a white powder. $^1$H NMR (400 MHz, DMSO-*d6*) δ 7.05 05 (d, *J* = 7.2 Hz 1H); 7.01-6.95 (m, 1H); 6.52-6.46 (m, 1H); 6.33 (d, *J* = 7.9 Hz 1H); 5.66 *(d, J*= 9.3 Hz 1H); 4.48 (d, *J* = 9.3 Hz 1H); 3.82-3.72 (m, 2H); 2.88 (br s, 2H); 2.50 (s, 3H); 1.92-1.85 (m, 2H); 1.61 (td, *J* = 13.2, 5.1 Hz 1H); 1.47 (td, *J* = 13.3, 4.9 Hz 1H); 1.31 (s, 9H); 1.21-1.02 (m, 2H); 1.00 (s, 9H). LCMS m/z $[M+H]^+$ 422.2.

**Step c:** To a mixture of tert-butyl (3S)-3-[[(R)-tert-butylsulfinyl]amino]-1-methyl-spiro[indoline-2,4'-piperidine]-1'-carboxylate (1.13 g, 2.68 mmol) in methanol (20 mL) was added dropwise a solution of HCl (4 M in dioxane, 6.7 mL, 26.8 mmol) at RT. The mixture was stirred at RT for 1 h and then concentrated under reduced pressure. The residue was triturated into acetonitrile (10 mL), iced and filtered to give crude (3S)-1-methylspiro[indoline-2,4'-piperidine]-3-amine hydrochloride (878 mg) as a white solid, that was used without further purification. $^1$H NMR (400 MHz, DMSO-*d6*) δ 9.30 (br s, 3H); 8.40 (br s, 2H); 7.42 (d, *J* = 6.5 Hz, 1H); 7.24 (td, *J* = 7.8 Hz and 1.1 Hz, 1H) ; 6.73 (td, *J* = 7.4 Hz and 0.76 Hz, 1H) ; 6.59 (d, *J* = 7.9 Hz, 1H) ; 4.82 (q, *J* = 47 Hz, 1H) ; 3.37-3.17 (m, 4H) ; 2.67 (s, 3H) ; 1.89 (d, *J* = 14.2 Hz, 1H) ; 1.29 (d, *J* = 13.9 Hz, 1H) ; 1.10 (s, 1H); 1.04 (s, 1H).

**Example i-40. Intermediate B-31 (spiro[5H-cyclopenta[b]pyridine-6,4'-piperidine]-7-one hydrochloride)**

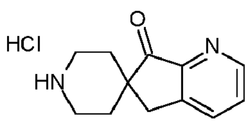

**Step a:** To a solution of 1-boc-4-cyanopiperidine (2.75 g, 12.4 mmol) in THF (75 mL) was added dropwise LDA (1 M THF/hexane, 13 mL, 13 mmol) at -78 °C. After addition, the mixture was stirred at this temperature for 1 h, and a solution of 2-bromo-3-(bromomethyl)pyridine (2.95 g, 11.3 mmol) in THF (25 mL) was added dropwise. The resulting mixture was stirred at -78 °C for 30 min then allowed to reach room temperature overnight. The reaction mixture was quenched by addition of an aqueous solution of $NH_4Cl$ (100 mL) at 0 °C, and extracted with EtOAc (50 mL ∗ 3). The combined organic layers were washed with brine (50 mL), dried over $Na_2SO_4$, filtered and concentrated under reduced pressure. The residue was purified by column chromatography ($SiO_2$, heptane/ethyl acetate = 3/2) to give tert-butyl 4-[(2-bromo-3-pyridyl)methyl]-4-cyano-piperidine-1-carboaylate (4.2 g, 11.0 mmol) as a beige solid. $^1$H NMR (400 MHz, DMSO-*d6*) δ 8.33 (m, 1H), 7.88 (m, 1H), 7.47-7.50 (m, 1H), 4.00-4.04 (m, 2H), 3.13 (s, 3H), 2.83 (m, 2H), 1.84-1.87 (m,

2H), 1.62-1.70 (m, 2H), 1.40 (s, 9H).

**Step b:** To a solution of tert-butyl 4-[(2-bromo-3-pyridyl)methyl]-4-cyano-piperidine-1-carboaylate (1.66 g, 4.4 mmol) in THF (27 mL) was added dropwise Turbo Grignard reagent iPrMgCl.LiCl complex (1.3 M, 6.7 mL, 8.7 mmol) at 0 °C. After addition, the mixture was stirred at this temperature for 2 h. The reaction mixture was cooled down to -78 °C and a solution of butyllithium (2.5 M in hexanes, 1.92 mL, 4.8 mmol) was added dropwise. The resulting mixture was stirred at -78 °C for 2 h then was poured into an aqueous solution of $NH_4Cl$ (100 mL) at 0 °C, and extracted with EtOAc (50 mL ∗ 3). The combined organic layers were washed with brine (50 mL), dried over $Na_2SO_4$, filtered and concentrated under reduced pressure. The residue was purified by column chromatography ($SiO_2$, heptane/ethyl acetate = 7/3) to furnish the expected product as a ketone/imine mixture of tert-butyl 7-oxospiro[5H-cyclopenta[b]pyridine-6,4'-piperidine]-1'-carboxylate and tert-butyl 7-iminospiro[5H-cyclopenta[b]pyridine-6,4'-piperidine]-1'-carboxylate (ketone/imine 9/1, 542 mg) as a beige solid. To a solution of the obtained product in methanol (18 mL) was added an aqueous solution of HCl (2 N, 9 mL, 18 mmol). The mixture was heated to reflux and stirred for 1 h and then concentrated under reduced pressure to give spiro[5H-cyclopenta[b]pyridine-6,4'-piperidine]-7-one hydrochloride (0.55 g, 4.4 mmol) as a beige solid which was used without any further pruification. $^1H$ NMR (400 MHz, DMSO-*d6*) δ 9.25 (br s, 1H) ; 8.93 (br s, 1H) ; 8.77 (m, 1H), 8.07-8.10 (m, 1H), 7.64-7.67 (m, 1H), 3.33-3.36 (m, 2H), 3.17 (s, 5H), 3.00-3.09 (m, 2H), 1.93-2.00 (m, 2H), 1.64-1.67 (m, 2H).

**Example i-41. Intermediate B-32 ((R)-N-[(5S)-3-(difluoromethyl)spiro[5,7-dihydrocyclopenta[b]pyridine-6,4'-piperidine]-5-yl]-2-methyl-propane-2-sulfinamide)**

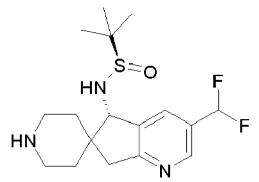

**Step a:** To a solution of (5-bromo-6-chloro-3-pyridyl)methanol (4.0 g, 18.0 mmol) inDCM (40 mL) was added 3,4-dihydro-2H-pyran (3.38 mL, 36 mmol) and pyridinium p-toluenesulfonate (0.46 g, 1.8 mmol) and the mixture was stirred at 40 °C for 18 h. 1 N aqueous solution of NaOH was added and the organic layer was separated and then washed with brine, dried over $Na_2SO_4$, filtered and concentrated under reduced pressure. The residue was purified by silica gel chromatography ($SiO_2$, cyclohexane/ethyl acetate = 1/0 to 8/2) to give 3-bromo-2-chloro-5-(tetrahydropyran-2-yloxymethyl)pyridine (5.27 g, 17.2 mmol) as a colourless oil.

**Step b:** To a solution of 3-bromo-2-chloro-5-(tetrahydropyran-2-yloxymethyl)pyridine (5.25 g, 17.1 mmol) in THF (60 mL) was added n-BuLi (2.1 M, 9.4 mL, 19.7 mmol) dropwise at -78 °C under Ar, the mixture was stirred at -78 °C for 35 min. Tert-butyl 4-formyl-4-methylpiperidine-1-carboxylate (4.5 g, 19 mmol) in THF (20 mL) was added dropwise to the mixture and the mixture was stirred at -78 °C for 2 h. The mixture was quenched by water (75 mL) and extracted with EtOAc (75 mL ∗ 2), the organic layer was washed with brine (75 mL), dried over $Na_2SO_4$, filtered and concentrated under reduced pressure. The residue was purified by column chromatography ($SiO_2$, cyclohexane/ethyl acetate= 1/0 to 1/1) to give tert-butyl 4-[[2-chloro-5-(tetrahydropyran-2-yloxymethyl)-3-pyridyl]-hydroxy-methyl]-4-methyl-piperidine-1-carboxylate (1.51 g, 3.32 mmol) as a beige foam. LCMS m/z $[M+H]^+$ = 455.3.

**Step c:** To a solution of tert-butyl 4-[[2-chloro-5-(tetrahydropyran-2-yloxymethyl)-3-pyridyl]-hydroxy-methyl]-4-methyl-piperidine-1-carboxylate (1.51 g, 3.3 mmol) in DCM (16 mL) was added Dess-Martin periodinane (2.1 g, 5.0 mmol) at 0 °C under Ar. The reaction was stirred at RT for 2 h. The mixture was poured into a 20% aqueous solution of sodium sulfite. The organic layer was separated and then washed with a saturated aqueous solution of $NaHCO_3$, brine, dried with anhydrous $Na_2SO_4$, filtered and concentrated under vacuum. The residue was purified by column chromatography ($SiO_2$, cyclohexane/ethyl acetate= 1/0 to 7/3) to give tert-butyl 4-[2-chloro-5-(tetrahydropyran-2-yloxymethyl)pyridine-3-carbonyl]-4-methyl-piperidine-1-carboxylate (1.0 g, 2.2 mmol) as a yellow oil. LCMS m/z $[M+H]^+$ = 453.3.

**Step d:** A mixture tert-butyl 4-[2-chloro-5-(tetrahydropyran-2-yloxymethyl)pyridine-3-carbonyl]-4-methylpiperidine-1-carboxylate (1.0 g, 2.2 mmol), $Cs_2CO_3$ (0.86 g, 2.2 mmol), pivalic acid (67 mg, 0.66 mmol), $Pd(OAc)_2$ (25 mg, 0.11 mmol), tricyclohexylphosphonium tetrafluoroborate (81 mg, 0.22 mmol) in mesitylene (11 mL) was degassed with Ar for 10 min. Then the reaction was stirred at 140 °C for 20 h. The reaction was cooled down to RT, ethyl acetate and water were added. The aqueous layer was separated and extracted with ethyl acetate. The combined organic phase was washed with brine, dried with anhydrous $Na_2SO_4$, filtered and concentrated under vacuum. Remaining mesitylene was azeotroped twice with ethanol. The residue was purified by column chromatography ($SiO_2$, cyclohexane/ethyl acetate= 1/0 to 3/7) to give tert-butyl 5-oxo-3-(tetrahydropyran-2-yloxymethyl)spiro[7H-cyclopenta[b]pyridine-6,4'-piperidine]-1'-carboxylate (0.5 g, 1.2 mmol) as a orange wax. LCMS m/z $[M+H]^+$ = 417.5.

**Step e:** To a solution of tert-butyl 5-oxo-3-(tetrahydropyran-2-yloxymethyl)spiro[7H-cyclopenta[b]pyridine-6,4'-piperidine]-1'-carboaylate (0.5 g, 1.2 mmol) in $Ti(OEt)_4$ (1.0 mL) and Me-THF (1.5 mL) was added (R)-2-methylpropane-2-sulfinamide (0.3 g, 2.4 mmol) at RT under Ar. The reaction was stirred at 100 °C for 18 h. The residue was taken up in water and dichloromethane and the mixture was filtered on a hydrophobic cartridge (liquid / liquid extraction column, Radleys®) and then concentrated in vacuum. The residue was purified by column chromatography ($SiO_2$, cyclohexane/ethyl acetate = 1/0 to 0/1) to give tert-butyl (5Z)-5-[(R)-tert-butylsulfinyl]imino-3-(tetrahydropyran-2-yloxymethyl)spiro[7H-cyclopenta[b]pyridine-6,4'-piperidine]-1'-carboxylate (0.475 g, crude) as a yellow wax that was used without further purification.

**Step f:** To a solution of crude tert-butyl (5Z)-5-[(R)-tert-butylsulfinyl]imino-3-(tetrahydropyran-2-yloxymethyl)spiro[7H-cyclopenta[b]pyridine-6,4'-piperidine]-1'-carboxylate (0.475 g) in dry THF (8 mL) was added dropwise DIBAL-H (1 M in toluene, 1.7 mL, 1.7 mmol) at -78 °C under Ar. The mixture was stirred at -78 °C for 1 h; ethyl acetate and then a saturated aqueous solution of Rochelle salt were added, the mixture was allowed to warm to RT and stirred for 2 h. The organic layer was separated and the aqueous phase was extracted twice with ethyl acetate. The combined organic phase was washed with brine, dried with anhydrous $Na_2SO_4$, filtered and concentrated under vacuum. The residue was purified by column chromatography ($SiO_2$, cyclohexane/ethyl acetate = 1/0 to 0/1 then dichloromethane/methanol = 9/1) to give tert-butyl (5S)-5-[[(R)-tert-butylsulfinyl]amino]-3-(tetrahydropyran-2-yloxymethyl)spiro[5,7-dihydrocyclopenta[b]pyridine-6,4'-piperidine]-1'-carboxylate (0.44 g, 0.84 mmol) as an off-white foam. LCMS m/z $[M+H]^+$ = 522.7.

**Step g:** A mixture of give tert-butyl (5S)-5-[[(R)-tert-butylsulfinyl]amino]-3-(tetrahydropyran-2-yloxymethyl)spiro[5,7-dihydrocyclopenta[b]pyridine-6,4'-piperidine]-1'-carboxylate (0.44 g, 0.84 mmol) and p-toluenesulfonic acid monohydrate (29 mg, 0.17 mmol) in methanol (4.2 mL) was stirred at 50 °C for 24 h. More p-toluenesulfonic acid monohydrate (29 mg, 0.17 mmol) was added and the mixture was stirred at 50 °C for 20 h and then concentrated under vacuum. The residue was taken up in ethyl acetate and a saturated aqueous solution of $NaHCO_3$, the organic layer was separated and then washed with brine, dried with anhydrous $Na_2SO_4$, filtered and concentrated under vacuum to give tert-butyl (5S)-5-[[(R)-tert-butylsulfinyl]amino]-3-(hydroxymethyl)spiro[5,7-dihydrocyclopenta[b]pyridine-6,4'-piperidine]-1'-carboxylate (0.3 g, crude) as a yellow foam that was used without further purification.

**Step h:** To a solution of tert-butyl (5S)-5-[[(R)-tert-butylsulfinyl]amino]-3-(hydroxymethyl)spiro[5,7-dihydrocyclopenta[b]pyridine-6,4'-piperidine]-1'-carboxylate (0.3 g, crude) in DCM (6.6 mL) was added Dess-Martin periodinane (0.43 g, 1.0 mmol) at 0 °C under Ar. The reaction was stirred at RT for 1 h. The mixture was poured into a 20% aqueous solution of sodium sulfite and then stirred for 2 h. The organic layer was separated and then washed with a saturated aqueous solution of $NaHCO_3$, brine, dried with anhydrous $Na_2SO_4$, filtered and concentrated under vacuum to give tert-butyl (5S)-5-[[(R)-tert-butylsulfinyl]amino]-3-formyl-spiro[5,7-dihydrocyclopenta[b]pyridine-6,4'-piperidine]-1'-carboxylate (0.27 g, crude) as an off-white foam that was used without further purification.

**Step i:** To a solution of tert-butyl (5S)-5-[[(R)-tert-butylsulfinyl]amino]-3-formyl-spiro[5,7-dihydrocyclopenta[b]pyridine-6,4'-piperidine]-1'-carboxylate (0.27 g, crude) in dichloromethane (6.2 mL) was added dropwise DAST (0.4 mL, 3 mmol) at -20 °C under Ar. The mixture was allowed to slowy warm to 10 °C (in approximately 2 h), the mixture was cooled to 0 °C and a saturated aqueous solution of $NaHCO_3$ was slowly added. Dichloromethane was added and the organic layer was separated. The aqueous phase was extracted with dichloromethane. The combined organic phase was washed with brine, dried with anhydrous $Na_2SO_4$, filtered and concentrated under vacuum to give a 7/3 mixture of tert-butyl (5S)-5-[[(R)-tert-butylsulfinyl]amino]-3-(difluoromethyl)spiro[5,7-dihydrocyclopenta[b]pyridine-6,4'-piperidine]-1'-carboxylate and a by-product (0.3 g, crude) as a beige solid that was used without further purification.

**Step j:** To the crude product previously obtained (0.3 g) in dichloromethane (3 mL) was added TFA (0.48 mL, 6.2 mmol) at 0 °C. The mixture was stirred at RT for 2 h. Dichloromethane (5 mL) and a 35% solution of NaOH were added and the organic layer was separated. The aqueous phase was extracted with dichloromethane. The combined organic phase was washed with brine, dried with anhydrous $Na_2SO_4$, filtered and concentrated under vacuum to give a 7/3 mixture of (R)-N-[(5S)-3-(difluoromethyl)spiro[5,7-dihydrocyclopenta[b]pyridine-6,4'-piperidine]-5-yl]-2-methylpropane-2-sulfinamide and a by-product (0.22 g, crude) as a brown foam that was used without further purification.

**Example i-42. Intermediate B-33 (2-(triisopropylsilyloxymethyl)spiro[7H-cyclopenta[b]pytidine-6,4'-pipetidine]-5-one)**

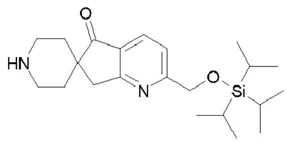

Step a: To a solution of (5-bromo-6-chloro-2-pyridyl)methanol (4.0 g, 18.0 mmol) in DCM (45 mL) was added 3,4-dihydro-2H-pyran (3.35 mL, 35.6 mmol) and pyridinium p-toluenesulfonate (0.45 g, 1.8 mmol) and the mixture was stirred at 40 °C for 16 h. 1 N aqueous solution of NaOH was added and organic layer was separated and then washed with brine, dried over $Na_2SO_4$, filtered and concentrated under reduced pressure. The residue was purified by silica gel chromatography ($SiO_2$, cyclohexane/ethyl acetate = 1/0 to 95/5) to give 3-bromo-2-chloro-6-(tetrahydropyran-2-yloxymethyl)pyridine (5.5 g, 18 mmol) as a colourless oil.

**Step b:** To a solution of 3-bromo-2-chloro-6-(tetrahydropyran-2-yloxymethyl)pyridine (5.5 g, 18 mmol) in THF (80 mL) was added n-BuLi (2.1 M, 9.9 mL, 20.7 mmol) dropwise at -78 °C under Ar, the mixture was stirred at - 78 °C for 25 min. Tert-butyl 4-formyl-4-methylpiperidine-1-carboxylate (4.95 g, 20.7 mmol) in THF (20 mL) was added dropwise to the mixture and the mixture was stirred at -78 °C for 1 h. The mixture was quenched by water (100 mL) and extracted with EtOAc (75 mL * 2), the organic layer was washed with brine (75 mL), dried over $Na_2SO_4$, filtered and concentrated under reduced pressure. The residue was purified by column chromatography ($SiO_2$, cyclohexane/ethyl acetate= 1/0 to 6/4) to give tert-butyl 4-[[2-chloro-6-(tetrahydropyran-2-yloxymethyl)-3-pyridyl]-hydroxy-methyl]-4-methyl-piperidine-1-carboxylate (1.45 g, 3.2 mmol) as an off-white foam. LCMS m/z $[M+H]^+$ = 455.3.

**Step c:** To a solution of tert-butyl 4-[[2-chloro-6-(tetrahydropyran-2-yloxymethyl)-3-pyridyl]-hydroxymethyl]-4-methyl-piperidine-1-carboxylate (1.45 g, 3.2 mmol) in DCM (16 mL) was added Dess-Martin periodinane (2.0 g, 4.8 mmol) at 0 °C under Ar. The reaction was stirred at RT for 2 h. The mixture was poured into a 20% aqueous solution of sodium sulfite. The organic layer was separated and then washed with a saturated aqueous solution of $NaHCO_3$, brine, dried with anhydrous $Na_2SO_4$, filtered and concentrated under vacuum. The residue was purified by column chromatography ($SiO_2$, cyclohexane/ethyl acetate= 1/0 to 75/25) to give tert-butyl 4-[2-chloro-6-(tetrahydropyran-2-yloxymethyl)pyridine-3-carbonyl]-4-methyl-piperidine-1-carboxylate (0.94 g, 2.07 mmol) as a colourless wax. LCMS m/z $[M+H]^+$ = 453.3.

**Step d:** A mixture tert-butyl 4-[2-chloro-6-(tetrahydropyran-2-yloxymethyl)pyridine-3-carbonyl]-4-methylpiperidine-1-carboxylate (0.935 g, 2.06 mmol), $Cs_2CO_3$ (0.81 g, 2.5 mmol), pivalic acid (63 mg, 0.62 mmol), $Pd(OAc)_2$ (23 mg, 0.1 mmol), tricyclohexylphosphonium tetrafluoroborate (76 mg, 0.2 mmol) in mesitylene (10 mL) was degassed with Ar for 10 min. Then the reaction was stirred at 140 °C for 22 h. The reaction was cooled down to RT, filtered through a pad of dicalite, washed with ethyl acetate. The organic phase was washed with brine, dried with anhydrous $Na_2SO_4$, filtered and concentrated under vacuum. Remaining mesitylene was azeotroped twice with ethanol. The residue was purified by column chromatography ($SiO_2$, cyclohexane/ethyl acetate= 1/0 to 7/3) to give tert-butyl 5-oxo-2-(tetrahydropyran-2-yloxymethyl)spiro[7H-cyclopenta[b]pyridine-6,4'-piperidine]-1'-carboxylate (0.52 g, 1.26 mmol) as a orange wax. LCMS m/z $[M+H]^+$ = 417.3.

**Step e:** A mixture of give tert-butyl 5-oxo-2-(tetrahydropyran-2-yloxymethyl)spiro[7H-cyclopenta[b]pyridine-6,4'-piperidine]-1'-carboxylate (0.28 g, 0.67 mmol) and p-toluenesulfonic acid monohydrate (25 mg, 0.15 mmol) in methanol (3.4 mL) was stirred at 50 °C for 2.5 h. The mixture was then concentrated under vacuum. The residue was taken in ethyl acetate and a saturated aqueous solution of $NaHCO_3$, the organic layer was separated and then washed with brine, dried with anhydrous $Na_2SO_4$, filtered and concentrated under vacuum to give tert-butyl 2-(hydroxymethyl)-5-oxo-spiro[7H-cyclopenta[b]pyridine-6,4'-piperidine]-1'-carboxylate (0.22 g, 0.67 mmol) as a yellow solid. LCMS m/z $[M+H]^+$ = 333.3.

**Step f:** To a solution of tert-butyl 2-(hydroxymethyl)-5-oxo-spiro[7H-cyclopenta[b]pyridine-6,4'-piperidine]-1'-carboxylate (0.22 g, 0.67 mmol) in dichloromethane (2.7 mL) was added imidazole (91 mg, 1.34 mmol) and triisopropylsilyl chloride (0.16 mL, 0.74 mmol) at 0 °C under Ar. After 30 min, the mixture was stirred at RT for 4 h. Imidazole (91 mg, 1.34 mmol) and triisopropylsilyl chloride (0.16 mL, 0.74 mmol) were added at 0 °C and the mixture was stirred at RT for 16 h. Dichloromethane and water were added, the organic layer was separated and then washed with brine, dried with anhydrous $Na_2SO_4$, filtered and concentrated under vacuum. The residue was purified by column chromatography ($SiO_2$, cyclohexane/ethyl acetate= 1/0 to 9/1) to give tert-butyl 5-oxo-2-(triisopropylsilyloxymethyl)spiro[7H-cyclopenta[b]pyridine-6,4'-piperidine]-1'-carboxylate (0.2 g, 0.41 mmol) as an off-white solid. LCMS m/z $[M+H]^+$ = 489.6.

**Step g:** To a solution of tert-butyl 5-oxo-2-(triisopropylsilyloxymethyl)spiro[7H-cyclopenta[b]pyridine-6,4'-piperidine]-1'-carboxylate (0.2 g, 0.41 mmol) in dichloromethane (2 mL) was added TFA (0.31 mL, 4.1 mmol) at 0 °C. The mixture was stirred at RT for 1 h. Dichloromethane and a 35% solution of NaOH were added and the organic layer was separated. The aqueous phase was extracted four times with dichloromethane. The combined organic phase was washed with brine, dried with anhydrous $Na_2SO_4$, filtered and concentrated under vacuum to give 2-(triisopropylsilyloxymethyl)spiro[7H-cyclopenta[b]pyridine-6,4'-piperidine]-5-one (0.16 g, 0.41 mmol) as a yellow oil. LCMS m/z $[M+H]^+$

= 389.4.

**Example i-43. Intermediate B-34 ((R)-N-[(5S)-2-methoxyspiro[5,7-dihydrocyclopenta[b]pyridine-6,4'-piperidine]-5-yl]-2-methyl-propane-2-sulfinamide)**

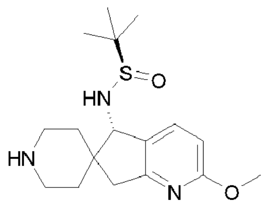

**Step a:** To a mixture of (3-bromo-6-methoxypyridin-2-yl)methanol (14.0 g, 64.2 mmol) and $CBr_4$ (25.4 g, 77.0 mmol) in DCM (60 mL) was added $PPh_3$ (20.2 g, 77 mmol) in DCM (30 mL) at 0 °C and the mixture was stirred at 0 °C for 30 min. The reaction mixture was poured into water (300 mL) and extracted with DCM (100 mL * 3), the organic layer was dried over $Na_2SO_4$, filtered and concentrated under reduced pressure. The residue was purified by column chromatography ($SiO_2$, petroleum ether/ethyl acetate = 50/1 to 0/1) to give 3-bromo-2-(bromomethyl)-6-methoxypyridine (12.0 g, 42.7 mmol) was obtained as a yellow oil. $^1H$ NMR (400 MHz, $CDCl_3$) δ 7.66 (d, *J* = 8.8 Hz, 1H), 6.59 (d, *J*= 4.0 Hz, 1H), 4.60 (s, 2H), 3.93 (s, 3H).

**Step b:** To a solution of 1-boc-4-cyanopiperidine (8.00 g, 38.0 mmol) in THF (80 mL) was added LDA (2 M, 18.4 mL, 36.8 mmol) at -78 °C and the mixture was stirred at this temperature for 1 h. 3-bromo-2-(bromomethyl)-6-methoxypyridine (7.98 g, 28.3 mmol) in THF (40 mL) was added at -78 °C and the mixture stirred at -78 °C for 1 h. The reaction mixture was poured into water (500 mL) and extracted with ethyl acetate (200 mL * 3), the organic layer was dried over $Na_2SO_4$, filtered and concentrated under reduced pressure. The residue was purified by column chromatography ($SiO_2$, petroleum ether/ethyl acetate = 50/1 to 0/1) to give tert-butyl 4-[(3-bromo-6-methoxy-2-pyridyl)methyl]-4-cyano-piperidine-1-carboxylate (10.0 g, 24.3 mmol) as a yellow oil. $^1H$ NMR (400 MHz, $CDCl_3$) δ 7.67 (d, *J* = 8.8 Hz, 1H), 6.56 (d, *J* = 4.4 Hz, 1H), 4.10-4.15 (m, 2H), 4.06 (s, 3H), 3.16 (s, 2H), 3.07-3.14 (m, 2H), 2.07 (d, *J* = 12.4 Hz, 2H), 1.57-1.68 (m, 2H), 1.46 (s, 9H).

**Step c:** To a mixture of tert-butyl 4-[(3-bromo-6-methoay-2-pyridyl)methyl]-4-cyano-piperidine-1-carboxylate (10.0 g, 24.3 mmol) in DMA (200 mL) and $H_2O$ (10 mL) was added TEA (13.5 mL, 97.4 mmol) and $PdCl_2(Amphos)_2$ (1.73 g, 2.44 mmol) at RT under $N_2$. The solution was degassed with $N_2$ for 10 min. The mixture was stirred at 130 °C for 2 h. The reaction mixture was poured into water (500 mL) and extracted with ethyl acetate (100 mL * 3), the organic layer was dried over $Na_2SO_4$, filtered and concentrated under reduced pressure. The residue was purified by column chromatography ($SiO_2$, petroleum ether/ethyl acetate = 50/1 to 0/1) to give tert-butyl 2-methoxy-5-oxo-spiro[7H-cyclopenta[b]pyridine-6,4'-piperidine]-1'-carboxylate (4.00 g, 12.0 mmol) as a yellow solid. $^1H$ NMR (400 MHz, $CDCl_3$) δ 7.87 (d, *J* = 8.8 Hz, 1H), 6.73 (d, *J* = 8.4 Hz, 1H), 4.11-4.18 (m, 2H), 4.05 (s, 3H), 3.04 (s, 2H), 2.95-3.01 (m, 2H), 1.90-1.98 (m, 2H), 1.48 (s, 9H), 1.40 *(d, J*= 13.6 Hz, 2H).

**Step d:** To a mixture of tert-butyl 2-methoxy-5-oxo-spiro[7H-cyclopenta[b]pyridine-6,4'-piperidine]-1'-carboxylate (4.50 g, 13.5 mmol) in 2-MeTHF (27 mL) was added (R)-2-methylpropane-2-sulfinamide (9.85 g, 81.2 mmol) and $Ti(OEt)_4$ (14.0 mL, 67.6 mmol) and the mixture was then stirred at 100 °C for 12 h. The reaction mixture was poured into water (200 mL) and extracted with ethyl acetate (50 mL * 3), the organic layer was dried over $Na_2SO_4$, filtered and concentrated under reduced pressure. The residue was purified by column chromatography ($SiO_2$, petroleum ether/ethyl acetate = 50/1 to 0/1) to give tert-butyl (5Z)-5-[(R)-tert-butylsulfinyl]imino-2-methoxy-spiro[7H-cyclopenta[b]pyridine-6,4'-piperidine]-1'-carboxylate (3.00 g, 6.89 mmol) as a yellow solid. $^1H$ NMR (400 MHz, $CDCl_3$) δ 7.59 (d, *J* = 8.4 Hz, 1H), 6.70 (d, *J* = 8.8 Hz, 1H), 4.03-4.18 (m, 2H), 4.00 (s, 3H), 3.04 (s, 2H), 2.88-2.95 (m, 2H), 1.96-2.04 (m, 2H), 1.48 (s, 9H), 1.31 (s, 9H).

**Step e:** To a mixture of tert-butyl (5Z)-5-[(R)-tert-butylsulfinyl]imino-2-methoxy-spiro[7H-cyclopenta[b]pyridine-6,4'-piperidine]-1'-carboxylate (3.00 g, 6.89 mmol) in THF (21 mL) was added DIBAL-H (1 M, 27.5 mL, 27.5 mmol) at -78 °C under $N_2$. The mixture was stirred at -78 °C for 45 min. The reaction mixture was poured into water (200 mL). The aqueous phase was extracted with ethyl acetate. The combined organic layer was dried over $Na_2SO_4$, filtered and concentrated under reduced pressure. The crude product was triturated with petroleum ether/ethyl acetate = 30/1 at RT, filtered and the filter cake was dried under reduced pressure to give tert-butyl (5S)-5-[[(R)-tert-butylsulfinyl]amino]-2-methoxy-spiro[5,7-dihydrocyclopenta[b]pyridine-6,4'-pipendine]-1'-carboxylate (2.27 g, 5.14 mmol) as a white solid. $^1H$ NMR (400 MHz, $CDCl_3$) δ 7.52 (d, *J* = 8.0 Hz, 1H), 6.58 (d, *J* = 8.0 Hz, 1H), 4.42 (d, *J* = 8.8 Hz, 1H), 3.99-4.11 (m, 2H), 3.93 (s, 3H), 3.50 (s, 1H), 2.92-2.99 (m, 3H), 2.72-2.80 (m, 1H), 1.52-1.76 (m, 2H), 1.46 (s, 1H), 1.41 (s, 9H),

1.27-1.39 (m, 1H), 1.25 (s, 9H). LCMS m/z $[M+H]^+$ = 338.2.

**Step f:** To a tert-butyl (5S)-5-[[(R)-tert-butylsulfinyl]amino]-2-methoxy-spiro[5,7-dihydrocyclopenta[b]pyridine-6,4'-piperidine]-1'-carboxylate (0.5 g, 1.1 mmol) in dichloromethane (10 mL) was added TFA (0.78 mL, 10.5 mmol) at 0 °C. The mixture was stirred at RT for 6 h. Dichloromethane (10 mL) and water (20 mL) were added, the pH value of the aqueous phase was adjusted to 11-12 with 35% aqueous NaOH solution and the mixture was filtered on a hydrophobic cartridge (liquid / liquid extraction column, Radleys®) and then concentrated in vacuum to give (R)-N-[(5S)-2-methoxyspiro[5,7-dihydrocyclopenta[b]pyridine-6,4'-piperidine]-5-yl]-2-methylpropane-2-sulfinamide (0.36 g, 1.07 mmol) as a white solid that was used without further purification.

**Example i-44. Intermediate B-35 ((R)-N-[(5S)-2-(difluoromethyl)spiro[5,7-dihydrocyclopenta[b]pyridine-6,4'-piperidine]-5-yl]-2-methyl-propane-2-sulfinamide)**

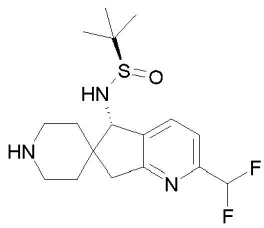

**Step a:** To a solution of tert-butyl 5-oxo-2-(tetrahydropyran-2-yloxymethyl)spiro[7H-cyclopenta[b]pyridine-6,4'-piperidine]-1'-carboxylate (0.79 g, 1.9 mmol) in $Ti(OEt)_4$ (1.7 mL) and Me-THF (2.3 mL) was added (R)-2-methylpropane-2-sulfinamide (0.47 g, 3.8 mmol) at RT under Ar. The reaction was stirred at 100 °C for 18 h. The residue was taken up in water and dichloromethane and the mixture was filtered on a hydrophobic cartridge (liquid / liquid extraction column, Radleys®) and then concentrated in vacuum. The residue was purified by column chromatography ($SiO_2$, cyclohexane/ethyl acetate = 1/0 to 0/1) to give tert-butyl (5Z)-5-[(R)-tert-butylsulfinyl]imino-2-(tetrahydropyran-2-yloxymethyl)spiro[7H-cyclopenta[b]pyridine-6,4'-piperidine]-1'-carboxylate (0.85 mg, crude) as a yellow solid that was used without further purification.

**Step b:** To a solution of crude tert-butyl (5Z)-5-[(R)-tert-butylsulfinyl]imino-2-(tetrahydropyran-2-yloxymethyl)spiro[7H-cyclopenta[b]pyridine-6,4'-piperidine]-1'-carboxylate (0.58 g) in dry THF (15 mL) was added dropwise DIBAL-H (1 M in toluene, 3.1 mL, 3.1 mmol) at -78 °C under Ar. The mixture was stirred at -78 °C for 2 h. A saturated aqueous solution of Rochelle salt and then ethyl acetate were added, the mixture was allowed to warm to RT and stirred for 2 h. The organic layer was separated and the aqueous phase was extracted twice with ethyl acetate. The combined organic phase was washed with brine, dried with anhydrous $Na_2SO_4$, filtered and concentrated under vacuum. The residue was purified by column chromatography ($SiO_2$, cyclohexane/ethyl acetate = 1/0 to 0/1) to give tert-butyl (5S)-5-[[(R)-tert-butylsulfinyl]amino]-2-(tetrahydropyran-2-yloxymethyl)spiro[5,7-dihydrocyclopenta[b]pyridine-6,4'-piperidine]-1'-carboxylate (0.58 g, 1.11 mmol) as an off-white foam. LCMS m/z $[M+H]^+$ = 522.4.

**Step c:** A mixture of give tert-butyl (5S)-5-[[(R)-tert-butylsulfinyl]amino]-2-(tetrahydropyran-2-yloxymethyl)spiro[5,7-dihydrocyclopenta[b]pyridine-6,4'-piperidine]-1'-carboxylate (0.56 g, 1.11 mmol) and p-toluenesulfonic acid monohydrate (43 mg, 0.25 mmol) in methanol (5.5 mL) was stirred at 60 °C for 16 h. p-toluenesulfonic acid monohydrate (38 mg, 0.17 mmol) was added and the mixture was stirred at 70 °C for 4 h and then concentrated under vacuum. The residue was taken in ethyl acetate and a saturated aqueous solution of $NaHCO_3$, the organic layer was separated and then washed with brine, dried with anhydrous $Na_2SO_4$, filtered and concentrated under vacuum to give tert-butyl (5S)-5-[[(R)-tert-butylsulfinyl]amino]-2-(hydroxymethyl)spiro[5,7-dihydrocyclopenta[b]pyridine-6,4'-piperidine]-1'-carboxylate (0.45 g, crude) as an off-white solid that was used without further purification.

**Step d:** To a solution of tert-butyl (5S)-5-[[(R)-tert-butylsulfinyl]amino]-2-(hydroxymethyl)spiro[5,7-dihydrocyclopenta[b]pyridine-6,4'-piperidine]-1'-carboxylate (0.45 g, crude) in DCM (10 mL) was added Dess-Martin periodinane (0.65 g, 1.5 mmol) at 0 °C under Ar. The reaction was stirred at RT for 3 h. The mixture was poured into a 20% aqueous solution of sodium sulfite and then stirred for 30 min. The organic layer was separated and then washed with a saturated aqueous solution of $NaHCO_3$, brine, dried with anhydrous $Na_2SO_4$, filtered and concentrated under vacuum to give tert-butyl (5S)-5-[[(R)-tert-butylsulfinyl]amino]-2-formyl-spiro[5,7-dihydrocyclopenta[b]pyridine-6,4'-piperidine]-1'-carboxylate (0.45 g, crude) as an off-white foam that was used without further purification.

**Step e:** To a solution of tert-butyl (5S)-5-[[(R)-tert-butylsulfinyl]amino]-2-formyl-spiro[5,7-dihydrocyclopenta[b]pyridine-6,4'-piperidine]-1'-carboxylate (445 mg, crude) in dichloromethane (10 mL) was added dropwise DAST (0.67 mL, 5.1 mmol) at -20 °C under Ar. The mixture was allowed to slowy warm to 0 °C (in approximately 2 h), the

mixture was cooled to 0 °C and a saturated aqueous solution of $NaHCO_3$ was slowly added. Dichloromethane was added and the organic layer was separated. The aqueous phase was extracted with dichloromethane. The combined organic phase was washed with brine, dried with anhydrous $Na_2SO_4$, filtered and concentrated under vacuum to give a~1/1 mixture of tert-butyl (5S)-5-[[(R)-tert-butylsulfinyl]amino]-2-(difluoromethyl)spiro[5,7-dihydrocyclopenta[b]pyridine-6,4'-piperidine]-1'-carboxylate and a by-product (0.46 g, crude) as a beige semi-solid that was used without further purification.

**Step f:** Tothe mixture previously obtained (0.46 g, crude) in dichloromethane (7 mL) was added TFA (1.1 mL, 14 mmol) at 0 °C. The mixture was stirred at RT for 2 h. Dichloromethane (5 mL) and a 35% solution of NaOH were added and the organic layer was separated. The aqueous phase was extracted with dichloromethane. The combined organic phase was washed with brine, dried with anhydrous $Na_2SO_4$, filtered and concentrated under vacuum to give a mixture containing (R)-N-[(5S)-2-(difluoromethyl)spiro[5,7-dihydrocyclopenta[b]pyridine-6,4'-piperidine]-5-yl]-2-methyl-propane-2-sulfinamide (0.36 g, crude) as a brown foam that was used without further purification.

**Example i-45. Intermediate B-36 ((R)-2-methyl-N-[(7S)-3-methylspiro[5,7-dihydrocyclopenta[c]pyridine-6,4'-piperidine]-7-yl]propane-2-sulfinamide)**

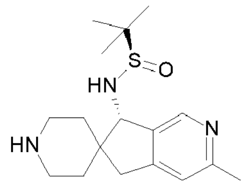

**Step a:** To a solution of tert-butyl 4-cyanopiperidine-1-carboxylate (4.74 g, 21.4 mmol) in THF (79 mL) was added dropwise LDA (freshly prepared with n-BuLi (2.5 M in hexanes, 9 mL) and diisopropylamine (3.18 mL) in THF (3.2 mL) at -78 °C. The mixture was then stirred at this temperature for 1 h, and a solution of 4-bromo-5-(bromomethyl)-2-methyl-pyridine (3.15 g, 11.9 mmol) in THF (20 mL) was added dropwise. The resulting mixture was stirred at -78 °C for 30 min then allowed to reach room temperature overnight. The reaction mixture was quenched by addition of an aqueous solution of $NH_4Cl$ (100 mL) at 0 °C, and extracted with EtOAc (50 mL ∗ 3). The combined organic layers were washed with brine (50 mL), dried over $Na_2SO_4$, filtered and concentrated under reduced pressure. The residue was purified by column chromatography ($SiO_2$, heptane/ethyl acetate = 9/1 to 7/3) to give tert-butyl 4-[(5-bromo-2-methyl-4-pyridyl)methyl]-4-cyano-piperidine-1-carboxylate (1.75 g, 4.31 mmol) as pale-yellow oil. LCMS m/z $[M+H]^+$ 394.2.

**Step b:** A mixture tert-butyl 4-[(5-bromo-2-methyl-4-pyridyl)methyl]-4-cyano-piperidine-1-carboxylate (2.85 g, 7.23 mmol), DIPEA (5.05 mL, 28.9 mmol), $Pd(AmPhos)_2Cl_2$ (384 mg, 0.54 mmol) in DMA/$H_2O$ 9/1 (153 mL) was degassed with Ar, then the mixture was stirred at 130 °C for 18 h. Water (150 mL) and ethyl acetate (200 mL) were added, the aqueous layer was separated and then extracted with ethyl acetate (200 mL).The combined organic layers were washed with water, dried over $Na_2SO_4$, filtered and concentrated under reduced pressure. The residue was purified by column chromatography ($SiO_2$, heptane/ethyl acetate = 65/35) to furnish a crude product which was triturated in $iPr_2O$ (40 mL) and acetonitrile (3 mL), iced and filtered to give tert-butyl 3-methyl-7-oxo-spiro[5H-cyclopenta[c]pyridine-6,4'-piperidine]-1'-carboxylate (400 mg, 1.26 mmol). $^1H$ NMR (400 MHz, DMSO-*d6*) δ 8.76 (s, 1H), 7.49 (s, 1H), 3.91-3.95 (m, 2H), 3.11 (s, 2H), 2.97 (m, 2H), 2.58 (s, 3H), 1.54-1.62 (m, 2H), 1.29-1.42 (m, 11H). LCMS m/z $[M+H]^+$ 317.3.

**Step c:** To a solution of tert-butyl 3-methyl-7-oxo-spiro[5H-cyclopenta[c]pyridine-6,4'-piperidine]-1'-carboxylate (396 mg, 1.25 mmol) in 2-MeTHF (10 mL) was added $Ti(OEt)_4$ (67% pure containing 33% of $TiO_2$, 1.6 mL, 5.0 mmol) and (*R*)-2-methylpropane-2-sulfinamide (310 mg, 2.5 mmol). The mixture was stirred at 90 °C for 16 h. Ethyl acetate (70 mL) and then brine (3 mL) were added. The mixture was stirred for 5 min at RT, filtered on dicalite and then concentrated under reduced pressure. The residue was taken up in $Et_2O$ (50 mL) and the organic layer was washed with brine (20 mL ∗ 3), dried over $Na_2SO_4$, filtered and concentrated under reduced pressure. The crude product was triturated with $iPr_2O$ (10 mL), iced and filtered to give tert-butyl (7Z)-7-[(R)-tert-butylsulfinyl]imino-3-methyl-spiro[5H-cyclopenta[c]pyridine-6,4'-piperidine]-1'-carboxylate (439 mg, 1.05 mmol) as a white powder. $^1H$ NMR (400 MHz, DMSO-*d6*) δ 9.30 (s, 1H), 7.44 (s, 1H), 3.93-3.96 (m, 2H), 3.13 (s, 2H), 2.83-2.98 (m, 2H), 2.55 (s, 3H), 1.62-1.81 (m, 2H), 1.41-1.59 (m, 11H), 1.23 (s, 9H). LCMS m/z $[M+H]^+$ 420.6.

**Step d:** To a solution of tert-butyl (7Z)-7-[(R)-tert-butylsulfinyl]imino-3-methyl-spiro[5H-cyclopenta[c]pyridine-6,4'-piperidine]-1'-carboxylate (435 mg, 1.05 mmol) in THF (25 mL) was added dropwise DIBAL-H (1 M in toluene, 4.1 mL, 4.1 mmol) at -78 °C under $N_2$. The resulting mixture was stirred at -78 °C for 2 h. Ethyl acetate (35 mL) was added at -65 °C, followed by a 1 M aqueous solution of Rochelle salts (6 mL), the reaction mixture was stirred for 1 h at RT and then concentrated under reduced pressure. The residue was taken up in ethyl acetate (25 mL) and brine (25 mL),

the organic layer was separated and then dried over $Na_2SO_4$, filtered and concentrated under reduced pressure. The crude product was triturated with $iPr_2O$ (25 mL), iced and filtered to give tert-butyl (7S)-7-[[(R)-tert-butylsulfinyl]amino]-3-methyl-spiro[5,7-dihydrocyclopenta[c]pyridine-6,4'-pipendine]-1'-carboxylate (378 mg, 0.9 mmol) as a white powder. $^1H$ NMR (400 MHz, DMSO-*d6*) δ 8.28 (s, 1H), 7.10 (s, 1H), 5.66 (d, *J* = 10.4 Hz, 1H), 4.43 *(d, J*= 10.4 Hz, 1H), 3.80-3.84 (m, 2H), 2.88-3.02 (m, 3H), 2.63 (d, *J* = 16.8 Hz, 1H), 2.43 (m, 3H), 1.77-1.82 (m, 1H), 1.56-1.61 (m, 1H), 1.40 (s, 9H), 1.19 (s, 9H). LCMS m/z $[M+H]^+$ 422.4.

**Step e:** To a tert-butyl (7S)-7-[[(R)-tert-butylsulfinyl]amino]-3-methyl-spiro[5,7-dihydrocyclopenta[c]pyridine-6,4'-piperidine]-1'-carboxylate (375 mg, 0.89 mmol) in dichloromethane (3 mL) was added TFA (0.7 mL, 8.9 mmol) at RT. The mixture was stirred for 2 h. The reaction mixture was diluted with dichloromethane (10 mL) and the mixture was poured in an aqueous 2 N solution of NaOH (15 mL). The mixture was filtered on a hydrophobic PTFE cartridge (liquid / liquid extraction column, Radleys®) and then concentrated in vacuum to give crude (R)-2-methyl-N-[(7S)-3-methyl-spiro[5,7-dihydrocyclopenta[c]pyridine-6,4'-piperidine]-7-yl]propane-2-sulfinamide (289 mg) as a white solid that was used without further purification. $^1H$ NMR (400 MHz, DMSO-*d6*) δ 8.28 (s, 1H), 7.10 (s, 1H), 5.58 (d, *J* = 10.0 Hz, 1H), 4.35 (d*, J*= 10.4 Hz, 1H), 2.96 (d*, J* = 16.8 Hz, 1H), 2.84-2.87 (m, 2H), 2.60-2.72 (m, 3H), 2.42 (s, 3H), 1.72-1.79 (m, 1H), 1.53-1.64 (m, 1H) 1.41-1.48 (m, 1H), 1.19 (s, 10H). LCMS m/z $[M+H]^+$ 322.3.

**Example i-46. Intermediate B-37 ((5S)-spiro[5,7-dihydrocyclopenta[b]pyrazine-6,4'-piperidine]-5-amine hydrochloride)**

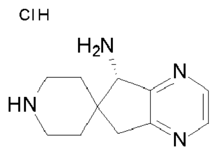

**Step a:** To a solution of LDA freshly prepared with nBuLi (2.5 M in hexanes, 57 mL) and diisopropylamine (19.5 mL) in THF (380 mL) at -78 °C was added dropwise a solution of 2-bromopyrazine (10 g, 62.9 mmol) in THF (20 mL). After addition, the mixture was stirred at this temperature for 30 min, and dimethylformamide (12.1 mL, 157 mmol) was added dropwise. The resulting mixture was stirred at -78 °C for 2 h. At this temperature, methanol (150 mL) was added dropwise and the reaction mixture was stirred for 10 min and $NaBH_4$ (4.76 g, 126 mmol) was then added portionwise. The temperature was then allowed to warm to 0 °C for 1 h. The reaction mixture was quenched by addition of an aqueous solution of $NH_4Cl$ (1000 mL) at 0 °C, and extracted with EtOAc (500 mL ∗ 3). The combined organic layers were washed with brine (300 mL), dried over $Na_2SO_4$, filtered and concentrated under reduced pressure. The residue was purified by column chromatography ($SiO_2$, elution with dichloromethane) to give (3-bromopyrazin-2-yl)methanol (4.04 g, 21.4 mmol) as yellow solid. $^1H$ NMR (400 MHz, DMSO-*d6*) δ 8.69 (d, *J* = 4.0 Hz, 1H), 8.41 (d, *J* = 4.0 Hz, 1H), 5.42 (t, *J* = 4.0 Hz, 1H), 4.65 (d, *J* = 4.0 Hz, 1H). LCMS m/z $[M+H]^+$ 191.0.

**Step b:** To a solution of (3-bromopyrazin-2-yl)methanol (8 g, 42.3 mmol) in $Et_2O$ (200 mL) was added dropwise phosphorus tribromide (4.4 mL, 46.5 mmol) at 0 °C. The reaction mixture was stirred at reflux for 4 h The reaction mixture was cooled down to RT and poured into a saturated solution of sodium bicarbonate (200 mL) and extracted with dichloromethane (200 mL ∗ 2). The combined organic phase was dried over $Na_2SO_4$, filtered and concentrated under reduced pressure. The residue was purified by column chromatography ($SiO_2$, heptane/ethyl acetate 9/1) to give 2-bromo-3-(bromomethyl)pyrazine (7.2 g, 29 mmol) as pink solid. $^1H$ NMR (400 MHz, DMSO-*d6*) δ 8.65 (d, *J* = 2.4 Hz, 1H), 8.47 (d, *J* = 2.4 Hz, 1H), 4.77 (s, 2H). LCMS m/z $[M+H]^+$ 252.9.

**Step c:** To a solution of ethyl 1-(tert-butoxycarbonyl)-4-piperidinecarboxylate (6.74 g, 26.2 mmol) in dry THF (200 mL) was added dropwise LDA (1 M in THF, 33.3 mL, 33.3 mmol) at -78 °C under inert atmosphere. After addition, the mixture was stirred at this temperature for 1 h, and a solution of 2-bromo-3-(bromomethyl)pyrazine (6 g, 23.8 mmol) in THF (30 mL) was added dropwise. The resulting mixture was stirred at -78 °C for 30 min then allowed to reach RT overnight. The reaction mixture was quenched by addition of an aqueous solution of $NH_4Cl$ (450 mL) at 0 °C, and extracted with EtOAc (150 mL ∗ 3). The combined organic layers were washed with brine (150 mL), dried over $Na_2SO_4$, filtered and concentrated under reduced pressure. The residue was purified by column chromatography ($SiO_2$, heptane/ethyl acetate = 7/3) to give 1-O-tert-butyl 4-O-ethyl 4-[(3-bromopyrazin-2-yl)methyl]piperidine-1,4-dicarboxylate (7.74 g, 18.1 mmol) as a brown oil. $^1H$ NMR (400 MHz, DMSO-*d6*) δ 8.57 (d, *J* = 4.0 Hz, 1H), 8.34 (d, *J* = 4 Hz, 1H), 4.03 (q, *J* = 8 Hz, 1H), 3.66-3.71 (m, 2H), 3.22 (s, 2H), 2.90-3.02 (m, 2H), 1.96-2.00 (m, 2H), 1.54-1.61 (m, 2H), 1.39 (s, 9H), 1.06 (t, *J* = 8 Hz, 3H). LCMS m/z $[M+H-BoC]^+$ 330.0.

**Step d:** To a solution 1-O-tert-butyl 4-O-ethyl 4-[(3-bromopyrazin-2-yl)methyl]piperidine-1,4-dicarboxylate (7.74 g, 18.1 mmol) in anhydrous THF (350 mL) at -78 °C under inert atmosphere was added dropwise a solution of nBuLi

(2.5 M in hexanes, 13 mL, 32.5 mmol). The reaction mixture was warm to -10 °C and stirred at this temperature for 3 h The reaction mixture was quenched at RT by addition saturated $NH_4Cl$ (500 mL) at RT and extracted with EtOAc (200 mL * 3). The combined organic layers were washed with brine (50 mL * 3), dried over $Na_2SO_4$, filtered and concentrated under reduced pressure. The residue was purified by 2 successive column chromatographies ($SiO_2$, ethyl acetate/methanol = 98/2) and then ($SiO_2$, heptane/ethyl acetate = 7/3) to give tert-butyl 5-oxospiro[7H-cyclopenta[b]pyrazine-6,4'-piperidine]-1'-carboxylate (3.54 g, 10.9 mmol) as an orange oil. $^1H$ NMR (400 MHz, DMSO-*d6*) δ 8.76 (d, *J* = 2.4 Hz, 1H), 8.54 (d, *J* = 2.4 Hz, 1H), 3.91-3.95 (m, 2H), 3.23 (s, 2H), 2.97-3.05 (m, 2H), 2.58 (s, 3H), 1.60-1.68 (m, 2H), 1.51-1.54 (m, 2H), 1.43 (s, 9H). LCMS m/z [M+H-57-18] 230.0.

**Step e:** To a solution of tert-butyl 5-oxospiro[7H-cyclopenta[b]pyrazine-6,4'-pipendine]-1'-carboxylate (3.54 g, 11.7 mmol) in 2-MeTHF (60 mL) was added $Ti(OEt)_4$ (67% pure containing 33% of $TiO_2$, 14.6 mL, 46.7 mmol) and (*R*)-2-methylpropane-2-sulfinamide (2.83 g, 23.3 mmol). The mixture was stirred at 90 °C for 16 h. Ethyl acetate (300 mL) and then brine (5 mL) were added. The mixture was stirred for 5 min at RT, filtered on dicalite and then concentrated under reduced pressure. The residue was taken up in $Et_2O$ (100 mL) and the organic layer was washed with brine (40 mL * 3), dried over $Na_2SO_4$, filtered and concentrated under reduced pressure. The crude product was purified by column chromatography ($SiO_2$, elution with ethyl acetate) to give tert-butyl (5Z)-5-[(R)-tert-butylsulfinyl]iminospiro[7H-cyclopenta[b]pyrazine-6,4'-piperidine]-1'-carboxylate (1.2g, 3.0 mmol) as an orange solid. $^1H$ NMR (400 MHz, DMSO-*d6*) δ 8.83 (d, *J*= 4.4 Hz, 1H), 8.54 (d, *J* = 4 Hz, 1H), 3.93-4.00 (m, 2H), 3.26 (s, 2H), 2.90-3.09 (m, 2H), 1.79-1.86 (m, 2H), 1.67-1.74 (m, 2H), 1.58-1.64 (m, 2H), 1.43 (s, 9H), 1.17 (s, 9H). LCMS m/z $[M+H]^+$ 407.3.

**Step f:** To a solution of tert-butyl (5Z)-5-[(R)-tert-butylsulfinyl]iminospiro[7H-cyclopenta[b]pyrazine-6,4'-piperidine]-1'-carboxylate (745 mg, 1.8 mmol) in THF (18 mL) was added dropwise DIBAL-H (1 M in toluene, 3.6 mL, 3.6 mmol) at -78 °C under $N_2$. The resulting mixture was stirred at -78 °C for 1.5 h. A saturated aqueous solution of Rochelle salts (30 mL) and then ethyl acetate (20 mL) were added, the mixture was stirred at RT for 2 h. The aqueous layer was separated and extracted with ethyl acetate (20 mL). The combined organic phase was washed with brine (20 mL), over $Na_2SO_4$, filtered and concentrated under reduced pressure to give tert-butyl (5S)-5-[[(R)-tert-butylsulfinyl]amino]spiro[5,7-dihydrocyclopenta[b]pyrazine-6,4'-piperidine]-1'-carboxylate (790 mg, crude) as a brown wax. $^1H$ NMR (400 MHz, DMSO-*d6*) δ 8.42 (m, 2H), 5.76 (d, *J* = 10.0 Hz, 1H), 4.46 (d, *J* = 10.0 Hz, 1H), 3.81-3.84 (m, 2H), 2.79-3.12 (m, 4H), 1.50-1.78 (m, 2H), 1.41 (s, 9H), 1.26 (m, 10H). LCMS m/z $[M+H]^+$ 409.4.

**Step g:** To a solution of tert-butyl (5S)-5-[[(R)-tert-butylsulfinyl]amino]spiro[5,7-dihydrocyclopenta[b]pyrazine-6,4'-piperidine]-1'-carboxylate (752 mg, 1.84 mmol) in methanol (20 mL) was added a solution of HCl (4 M in dioxane, 4.6 mL, 18.4 mmol) at 0 °C. The mixture was stirred for 18 h at RT and then concentrated under vacuum. The residue was triturated in acetonitrile, the solid was filtered and then washed with diethyl ether to give (5S)-spiro[5,7-dihydrocyclopenta[b]pyrazine-6,4'-piperidine]-5-amine hydrochloride (476 mg, crude) as a black solid that was used without further purification.

**Example i-47. Intermediate B-38 ((1S)-6-Fluorospiro[indane-2,4'-piperidine]-1-amine hydrochloride)**

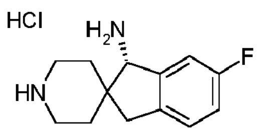

**Step a:** To a solution of 1-boc-4-cyanopiperidine (4.09 g, 18.48 mmol) in tetrahydrofuran (70 mL) was added dropwise LDA (1 M tetrahydrofuran / hexane, 19.32 mL, 19.32 mmol) at -78 °C. After addition, the mixture was stirred 1 h at the same temperature, and a solution of 2-bromo-4-fluorobenzyl bromide (4.5 g, 16.8 mmol) in THF (20 mL) was added dropwise. The resulting mixture was stirred at -78 °C for 30 min then allowed to reach room temperature overnight and then quenched by addition of a saturated solution of ammonium chloride (100 mL). Ethyl acetate (200 mL) was added and the mixture decanted. The organic layer was washed with water (5 x 250 mL), brine (20 mL), dried over $MgSO_4$, filtered and concentrated under reduced pressure. The residue was purified by column chromatography ($SiO_2$, Heptane/Ethyl acetate = 100/0 to 80/20) to give tert-butyl 4-[(2-bromo-4-fluoro-phenyl)methyl]-4-cyano-piperidine-1-carboxylate tert-butyl (5.75 g, 14.12 mmol) as a white solid. LCMS m/z $[M+H]^+$ = 397.

**Step b:** A mixture of tert-butyl 4-[(2-bromo-4-fluoro-phenyl)methyl]-4-cyano-piperidine-1-carboxylate (5.0 g, 12.59 mmol), DIPEA (7.1 mL, 50.34 mmol), $Pd(AmPhos)_2Cl_2$ (0.45 g, 0.63 mmol) in DMA (250 mL) and $H_2O$ (5 mL) was degassed with Ar for 10 min, then the mixture was stirred at 120 °C for 18 h. The reaction was cooled down to RT, water (500 mL) and EtOAc (500 mL) were added. The organic layer was separated and the aqueous layer extracted with EtOAc. The combined organic layers were washed with water, brine, dried over $MgSO_4$, filtered and concentrated

under reduced pressure. The residue was purified by column chromatography ($SiO_2$, Heptane/Ethyl acetate= 100/0 to 75/25) to give tert-butyl 6-fluoro-1-oxo-spiro[indane-2,4'-piperidine]-1'-carboaylate (3.46 g, 10.8 mmol) as a beige solid. LCMS m/z $[M+H]^+$ = 320.

**Step c:** A mixture of tert-butyl 6-fluoro-1-oxo-spiro[indane-2,4'-piperidine]-1'-carboxylate (2 g, 6.26 mmol), (R)-2-methylpropane-2-sulfinamide (1.52 g, 12.52 mmol) and tetraethoxytitanium (5.71 g, 25.05 mmol) was stirred at 100 °C for 15 h. The mixture was diluted with dichloromethane (150 mL) and water (140 mL). After 3 h of stirring, salts were removed by filtration and washed by dichloromethane (100 mL). The organic layer was washed with brine (100 mL), dried over $MgSO_4$ and concentrated under reduced pressure to give tert-butyl (1Z)-1-[(R)-tert-butylsulfinyl]imino-6-fluoro-spiro[indane-2,4'-piperidine]-1'-carboxylate (2.6 g, 6.2 mmol) as a yellow solid used without further purification.

**Step d:** To a solution of tert-butyl (1Z)-1-[(R)-tert-butylsulfinyl]imino-6-fluoro-spiro[indane-2,4'-piperidine]-1'-carboxylate (2.6 g, 6.2 mmol) in dry THF (30 ml) was added dropwise DIBAL-H (1 M in toluene, 30 mL, 30 mmol) at -65 °C under Ar. The mixture was stirred at -65 °C for 45 min and then a saturated aqueous solution of Rochelle salts (60 mL) and water (20 mL) were added, the mixture was allowed to warm to RT and stirred for 40 min. The mixture was extracted with ethyl acetate (200 mL). The combined organic phase was washed with a saturated aqueous solution of Rochelle salts (2 x 50 mL), brine (100 mL), dried with anhydrous $MgSO_4$, filtered and concentrated under vacuum. The residue was purified by column chromatography ($SiO_2$, Heptane/Ethyl acetate= 100/0 to 50/50) to tert-butyl (1S)-1-[[(R)-tert-butylsulfinyl]amino]-6-fluoro-spiro[indane-2,4'-piperidine]-1'-carboxylate (1.75 g, 4.12 mmol) as a white solid. LCMS m/z $[M+H]^+$ = 425.

**Step e:** A mixture of tert-butyl (1S)-1-[[(R)-tert-butylsulfinyl]amino]-6-fluoro-spiro[indane-2,4'-piperidine]-1'-carboxylate (1.155 g, 2.72 mmol) and a solution of HCl (2.5 N in ethanol, 30 mL, 75 mmol) was stirred 3 h at RT and was then concentrated under reduced pressure. The residue was solubilized in methanol (50 mL) and the solution concentrated under reduced pressure. This operation was repeated once. The crude product was triturated with ethyl acetate (50 mL) and diisopropyl ether (50 mL). The solid was filtrate, washed with diisopropyl ether (2 x 50 mL) and pentane (50 mL) to give (1S)-6-fluorospiro[indane-2,4'-piperidine]-1-amine hydrochloride (1.45 g) as a white solid used without further purification.

**Example i-48. Intermediate B-39 ((1S)-4-Fluorospiro[indane-2,4'-piperidine]-1-amine hydrochloride)**

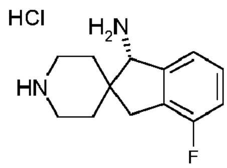

**Step a:** To a solution of 1-boc-4-cyanopiperidine (3.9 g, 18 mmol) in tetrahydrofuran (50 mL) was added dropwise LDA (1 M tetrahydrofuran /hexane, 19 mL, 19 mmol) at -78 °C. After addition, the mixture was stirred 1 h at the same temperature, and a solution of 1-bromo-2-(bromomethyl)-3-fluoro-benzene (4.5 g, 17 mmol) in THF (20 mL) was added dropwise. The resulting mixture was stirred at -78 °C for 30 min, then allowed to reach RT overnight and quenched by addition of a saturated solution of ammonium chloride (500 mL). Ethyl acetate (400 mL) was added and the mixture decanted. The organic layer was washed with water (5 x 250 mL), brine (200 mL), dried over $MgSO_4$, filtered and concentrated under reduced pressure. The residue was purified by column chromatography ($SiO_2$, Heptane/Ethyl acetate = 100/0 to 80/20) to give tert-butyl 4-[(2-bromo-6-fluoro-phenyl)methyl]-4-cyano-piperidine-1-carboxylate (5.7 g, 14 mmol) as a colorless oil. LCMS m/z $[M+H]^+$ = 397.

**Step b:** A mixture of tert-butyl 4-fluoro-1-oxo-spiro[indane-2,4'-piperidine]-1'-carboxylate (3.8 g, 12 mmol), DIPEA (7.1 mL, 50.34 mmol), $Pd(AmPhos)_2Cl_2$ (0.45 g, 0.63 mmol) in DMA (250 mL) and $H_2O$ (5 mL) was degassed with Ar for 10 min, then the mixture was stirred at 120 °C for 18 h. The reaction was cooled down to RT, water (500 mL) and EtOAc (500 mL) were added. The organic layer was separated and the aqueous layer extracted with EtOAc. The combined organic layers were washed with water, brine, dried over $MgSO_4$, filtered and concentrated under reduced pressure. The residue was purified by column chromatography ($SiO_2$, Heptane/Ethyl acetate= 100/0 to 75/25) to give tert-butyl 4-fluoro-1-oxo-spiro[indane-2,4'-pipendine]-1'-carboxylate (3.8 g, 12 mmol) as a beige solid. LCMS m/z $[M+H]^+$ = 320.

**Step c:** A mixture of tert-butyl 4-fluoro-1-oxo-spiro[indane-2,4'-piperidine]-1'-carboxylate (1010 mg, 3.16 mmol), (R)-2-methylpropane-2-sulfinamide (766 mg, 6.32 mmol) and tetraethoxytitanium (2.88 g, 12.65 mmol) was stirred at 100 °C for 15 h. The mixture was diluted with dichloromethane and water. Salts were removed by filtration and washed by dichloromethane. The organic layer was washed with brine, dried over $MgSO_4$ and concentrated under reduced

pressure to give tert-butyl (1Z)-1-[(R)-tert-butylsulfinyl]-imino-4-fluoro-spiro[indane-2,4'-piperidine]-1'-carboxylate (1.33 g, 3.15 mmol) as solid used without further purification. LCMS m/z $[M+H]^+$ = 423.

**Step d:** To a solution of tert-butyl (1Z)-1-[(R)-tert-butylsulfinyl]imino-4-fluoro-spiro[indane-2,4'-piperidine]-1'-carboxylate (1330 mg, 3.15 mmol) in dry THF (22 ml) was added dropwise DIBAL-H (1 M in toluene, 15.1 mL, 15.1 mmol) at -65 °C under Ar. The mixture was stirred at -65 °C for 45 min and then a saturated aqueous solution of Rochelle salts (60 mL) and water (20 mL) was added, the mixture was allowed to warm to RT and stirred for 40 min. The mixture was extracted with ethyl acetate (200 mL). The combined organic phase was washed with a saturated aqueous solution of Rochelle salts (2 x 50 mL), brine (100 mL), dried with anhydrous $MgSO_4$, filtered and concentrated under vacuum. The residue was solubilized in diisopropyl ether. Silica (3 g) was added, the suspension was stirred for 1 h, filtered and washed with ethyl acetate (50 mL). The filtrate was concentrated under reduced pressure to give (tert-butyl (1S)-1-[[(R)-tert-butylsulfinyl]amino]-4-fluoro-spiro[indane-2,4'-piperidine]-1'-carboxylate as a white solid (1.32 g, 3.11 mmol). LCMS m/z $[M+H]^+$ = 425.

**Step e:** A mixture of tert-butyl (1S)-1-[[(R)-tert-butylsulfinyl]amino]-4-fluoro-spiro[indane-2,4'-piperidine]-1'-carboxylate (1.32 g, 3.11 mmol) and a solution of HCl (2.5 N in ethanol, 30 mL, 75 mmol) was stirred 3 h at RT and concentrated under reduced pressure. The residue was solubilized in methanol (50 mL) and the solution concentrated under reduced pressure. This operation was repeated once. The crude product was triturated with ethyl acetate (50 mL) and diisopropyl ether (50 mL). The solid was filtrate, washed with diisopropyl ether (2 x 50 mL) and pentane (50 mL) to give (1S)-4-fluorospiro[indane-2,4'-piperidine]-1-amine hydrochloride (0.985 g, crude) as a white solid used without further purification.

**Example i-49. Intermediate B-40 ((1S)-5,6-Difluorospiro[indane-2,4'-piperidine]-1-amine hydrochloride)**

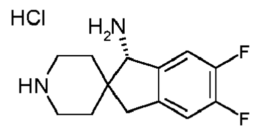

**Step a:** To a solution of 1-boc-4-cyanopiperidine (1.54 g, 7.31 mmol) in tetrahydrofuran (10 mL) was added dropwise LDA (2 M tetrahydrofuran / hexane, 3.82 mL, 7.64 mmol) at -70 °C. After addition, the mixture was stirred 1 h at the same temperature, and a solution of 1-bromo-2-(bromomethyl)-3-fluoro-benzene (2 g, 6.65 mmol) in THF (20 mL) was added dropwise. The resulting mixture was stirred at -70 °C for 30 min then allowed to reach RT overnight, quenched by addition of a saturated solution of sodium chloride and extracted with ethyl acetate. The combined organic layers were dried over $MgSO_4$, filtered and concentrated under reduced pressure. The residue was purified by column chromatography ($SiO_2$, Heptane/Ethyl acetate = 100/0 to 70/30) to give tert-butyl 4-[(2-bromo-4,5-difluorophenyl)methyl]-4-cyano-piperidine-1-carboxylate (970 mg, 2.34 mmol). LCMS m/z $[M-Boc+H]^+$ = 315.

**Step b:** A mixture of tert-butyl 4-[(2-bromo-4,5-difluoro-phenyl)methyl]-4-cyano-pipendine-1-carboxylate (970 mg, 2.34 mmol), DIPEA (1.3 mL, 9.34 mmol), $Pd(AmPhos)_2Cl_2$ (83 mg, 0.117 mmol) in DMA (25 mL) and $H_2O$ (1 mL) was degassed with Ar for 10 min, then the mixture was stirred at 120 °C for 18 h. The reaction was cooled down to RT, water (50 mL) and EtOAc (50 mL) were added. The organic layer was separated and the aqueous layer extracted with EtOAc. The combined organic layers were washed with water, brine, dried over $MgSO_4$, filtered and concentrated under reduced pressure. The residue was purified by column chromatography ($SiO_2$, Heptane/Ethyl acetate= 100/0 to 75/25) to give tert-butyl 5,6-difluoro-1-oxo-spiro[indane-2,4'-pipendine]-1'-carboxylate (150 mg).

**Step c:** A mixture of tert-butyl 5,6-difluoro-1-oxo-spiro[indane-2,4'-piperidine]-1'-carboxylate (150 mg, 0.444 mmol), (R)-2-methylpropane-2-sulfinamide (108 mg, 0.889 mmol) and tetraethoxytitanium (1014 mg, 4.45 mmol) was stirred at 100 °C for 15 h. The mixture was diluted with dichloromethane (50 mL) and water (100 mL). After 1 h of stirring, salts were removed by filtration and washed by dichloromethane (50 mL). The organic layer was washed with brine, dried over $MgSO_4$ and concentrated under reduced pressure. The residue was purified by chromatography ($SiO_2$, Heptane/Ethyl acetate= 100/0 to 70/30) to give tert-butyl (1Z)-1-[(R)-tert-butylsulfinyl]imino-5,6-difluoro-spiro[indane-2,4'-piperidine]-1'-carboxylate (144 mg, 0.327 mmol) as a yellow solid. LCMS m/z $[M+H]^+$ = 441.

**Step d:** To a solution of tert-butyl (1Z)-1-[(R)-tert-butylsulfinyl]imino-5,6-difluoro-spiro[indane-2,4'-piperidine]-1'-carboxylate (144 mg, 0.326 mmol) in dry THF (10 ml) was added dropwise DIBAL-H (1 M in toluene, 1.57 mL, 1.57 mmol) at -65 °C under Ar. The mixture was stirred at -65 °C for 1 h and then a saturated aqueous solution of Rochelle salts (60 mL) and water (20 mL) was added, the mixture was allowed to warm to RT and stirred for 1 h. The mixture was extracted with ethyl acetate (30 mL). The combined organic phase was washed with a saturated aqueous solution of Rochelle salts (2 x 50 mL), brine (100 mL), dried with anhydrous $MgSO_4$, filtered and concentrated under vacuum.

The residue was purified by column chromatography ($SiO_2$, Heptane/Ethyl acetate= 100/0 to 80/20) to tert-butyl (1S)-1-[[(R)-tert-butylsulfinyl]amino]-5,6-difluoro-spiro[indane-2,4'-piperidine]-1'-carboxylate (120 mg, 0.27 mmol) as a yellow solid. LCMS m/z $[M+H]^+$ = 443.

**Step e:** A mixture of tert-butyl (1S)-1-[[(R)-tert-butylsulfinyl]amino]-5,6-difluoro-spiro[indane-2,4'-piperidine]-1'-carboxylate (120 mg, 0.271 mmol) and a solution of HCl (4 N in dioxane, 5 mL, 20 mmol) was stirred 15 h at RT and concentrated under reduced pressure. The crude product was triturated with diisopropyl ether. The solid was filtrate to give (1S)-5,6-difluorospiro[indane-2,4'-piperidine]-1-amine hydrochloride (85 mg) as yellow solid used without further purification.

**Example i-50. Intermediate B-41 ((1S)-4,6-Difluorospiro[indane-2,4'-piperidine]-1-amine hydrochloride)**

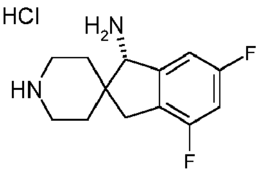

**Step a:** To a solution of ethyl N-boc-piperidine-4-carboxylate (5 g, 19.43 mmol) in tetrahydrofuran (75 mL) was added dropwise LDA (2 M tetrahydrofuran /hexane, 11.66 mL, 23.32 mmol) at -70 °C. After addition, the mixture was stirred 1 h at the same temperature, and a solution of 2,4-difluorobenzyl bromide (4.22 g, 20.4 mmol) in THF (20 mL) was added dropwise. The resulting mixture was stirred at -70 °C for 30 min then allowed to reach RT overnight, quenched by addition of a saturated solution of sodium chloride (500 mL). Ethyl acetate (700 mL) and water (200 mL) were added and the mixture decanted. The organic layer was washed with water (500 mL), brine (2 x 500 mL), dried over $MgSO_4$, filtered and concentrated under reduced pressure. The residue was purified by column chromatography ($SiO_2$, Heptane/Diisopropyl ether = 100/0 to 50/50) to give O1-tert-butyl 04-ethyl 4-[(2,4-difluorophenyl)methyl]piperidine-1,4-dicarboxylate (6.4 g, 17 mmol) as a light yellow oil. LCMS m/z $[M+H]^+$ = 384.

**Step b:** A mixture of O1-tert-butyl 04-ethyl 4-[(2,4-difluorophenyl)methyl]piperidine-1,4-dicarboxylate (1.2 g, 3,1 mmol), a solution of sodium hydroxide (5 N, 10 mL, 50 mmol) in ethanol (15 mL) was heated at reflux for 20 h and allowed to cool to RT. After elimination of ethanol under reduced pressure, the mixture was acidified by addition of a solution of hydrochloride acid (2 N, 25 mL) and extracted with dichloromethane (50 mL). The organic layer was washed brine (40 mL), dried over $MgSO_4$, filtered and concentrated under reduced pressure to give 1-tert-butoxycarbonyl-4-[(2,4-difluorophenyl)methyl]piperidine-4-carboxylic acid (1.03 g, 2.9 mmol) as beige solid. LCMS m/z $[M+H]^+$ = 356.

**Step c:** A solution of 1-tert-butoxycarbonyl-4-[(2,4-difluorophenyl)methyl]piperidine-4-carboxylic acid (3.2 g, 9 mmol) and thionyl chloride (2.6 mL, 36 mmol) in dichloromethane (70 mL) was stirred 1 h at RT. More thionyl chloride (2.6 mL, 36 mmol) was added, the stirring was pursued for 2 h at RT and the mixture was concentrated under reduced pressure. The residue was dissolved in dichloromethane (70 mL). The solution was cooled to 0 °C, aluminum chloride (2.4 g, 18 mmol) was added and the mixture was stirred at RT for 15 h and then poured on ice (40 g). After basification to pH 10 by addition of sodium hydroxide 2 N, di-tert-butyl pyrocarbonate (18 mmol, 3.9 g) was added. The mixture was stirred 2 h at RT. Dichloromethane (50 mL) was added and the organic phase was washed with water (50 mL), brine (100 mL), dried over $MgSO_4$, filtered and concentrated under reduced pressure. The residue was purified by column chromatography ($SiO_2$, Heptane/Ethyl acetate= 100/0 to 80/20) to give tert-butyl 4,6-difluoro-1-oxo-spiro[indane-2,4'-piperidine]-1'-carboxylate (650 mg, 1.84 mmol) as a white solid. LCMS m/z $[M+H]^+$ = 338.

**Step d:** A mixture of tert-butyl 4,6-difluoro-1-oxo-spiro[indane-2,4'-pipendine]-1'-carboxylate (1.2 g, 3.6 mmol), (R)-2-methylpropane-2-sulfinamide (0.86 g, 7.1 mmol) and tetraethoxytitanium (3.2 g, 14 mmol) was stirred at 100 °C for 15 h. The mixture was diluted with dichloromethane (150 mL) and water (550 mL). After 3 h of stirring, salts were removed by filtration and washed by dichloromethane (50 mL). The organic layer was washed with brine (100 mL), dried over $MgSO_4$ and concentrated under reduced pressure to give tert-butyl (1Z)-1-[(R)-tert-butylsulfinyl]imino-4,6-difluoro-spiro[indane-2,4'-piperidine]-1'-carboxylate (1.6 g, 3.6 mmol) as a yellow solid used without further purification.

**Step e:** To a solution of tert-butyl (1Z)-1-[(R)-tert-butylsulfinyl]imino-4,6-difluoro-spiro[indane-2,4'-piperidine]-1'-carboxylate (1.6 g, 3.6 mmol) in dry THF (30 ml) was added dropwise DIBAL-H (1 M in toluene, 17 mL, 17 mmol) at -65 °C under Ar. The mixture was stirred at -65 °C for 45 min and then a saturated aqueous solution of Rochelle salts (60 mL) and water (20 mL) was added, the mixture was allowed to warm to RT and stirred for 40 min. The mixture was extracted with ethyl acetate (200 mL). The combined organic phase was washed with a saturated aqueous solution of Rochelle salts (2 x 50 mL), brine (100 mL), dried with anhydrous $MgSO_4$, filtered and concentrated under vacuum. The residue was purified by column chromatography ($SiO_2$, Heptane/Ethyl acetate= 100/0 to 50/50) to give tert-butyl (1S)-1-[[(R)-tert-butylsulfinyl]amino]-4,6-difluoro-spiro[indane-2,4'-piperidine]-1'-carboxylate (1.15 g, 2.6 mmol) as a white solid. LCMS m/z $[M+H]^+$ = 443.

**Step f:** A mixture of tert-butyl (1S)-1-[[(R)-tert-butylsulfinyl]amino]-4,6-difluoro-spiro[indane-2,4'-piperidine]-1'-carboxylate (1.15 g, 2.6 mmol) and a solution of HCl (2.5 N in ethanol, 30 mL, 75 mmol) was stirred 3 h at RT and was then concentrated under reduced pressure. The residue was solubilized in methanol (50 mL) and the solution concentrated under reduced pressure. This operation was repeated once. The crude product was triturated with ethyl acetate (50 mL) and diisopropyl ether (50 mL). The solid was filtrated, washed with diisopropyl ether (2 x 50 mL) and pentane (50 mL) to give (1S)-4,6-difluorospiro[indane-2,4'-piperidine]-1-amine hydrochloride (745 mg, 2.14 mmol) as a white solid used without further purification.

**Example i-51. Intermediate C-1 ((3-chloro-2-cyclopropyl-4-pyridyl)boronic acid)**

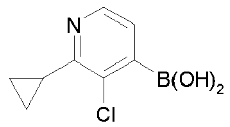

To a solution of 3-chloro-2-cyclopropyl-pyridine (1.04 g, 6.7 mmol) in anhydrous THF (5 mL) was added dropwise LDA (2 M, 6.7 mL, 13.4 mmol) at -78 °C. After addition, the mixture was stirred at -78 °C for 2 h and then triisopropyl borate (2.49 mL, 10.8 mmol) was added dropwise. The mixture was stirred at -78 °C for 3 h and then allowed to warm to 0 °C. The mixture was then quenched by addition of water (2.5 mL). The mixture was stirred 1 h at RT. Water was added, followed by 1 N aqueous solution of NaOH to reach a pH value of the aqueous phase of 11-12. The aqueous layer was washed twice with ethyl acetate. The pH value of the aqueous phase was adjusted to pH 6 with a 5 N aqueous solution of HCl. The aqueous layer was extracted twice with EtOAc. The combined organic layers were washed with brine, dried over $Na_2SO_4$, filtered and concentrated under reduced pressure. The residue was triturated in diethyl ether, filtered and washed with diethyl ether to give (3-chloro-2-cyclopropyl-4-pyridyl)boronic acid (507 mg, 2.56 mmol) as a beige solid.

**Example i-52. Intermediate C-2 ((3-chloro-2-isopropyl-4-pyridyl)boronic acid)**

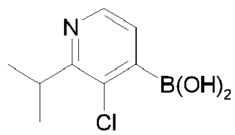

**Step a:** To a solution of 3-chloro-2-fluoro-pyridine (600 mg, 4.33 mmol) and tert-butyl 2-methylpropanoate (750 mg, 5.2 mmol) in anhydrous toluene (5 mL) was added dropwise NaHMDS (1 M in THF, 6.5 mL, 6.5 mmol) at 0 °C. After 30 min, the mixture was stirred at RT for 16 h. A concentrated aqueous solution of $NH_4Cl$ (20 mL) was added and the aqueous layer was extracted twice with EtOAc. The combined organic layers were washed with brine, dried over $Na_2SO_4$, filtered and concentrated under reduced pressure. The residue was purified by column chromatography ($SiO_2$, Petroleum ether/Ethyl acetate= 1/0 to 95/5) to give tert-butyl 2-(3-chloro-2-pyridyl)-2-methyl-propanoate (885 mg, 3.46 mmol) as a colourless oil.

**Step b:** A mixture of tert-butyl 2-(3-chloro-2-pyridyl)-2-methyl-propanoate (880 mg, 3.44 mmol) and 4-methylbenzenesulfonic acid mono hydrate (327 mg, 1.72 mmol) in toluene (6.9 mL) was stirred at 110 °C for 16 h The mixture was allowed to cool down to RT, ethyl acetate was then added. The organic phase was washed with a 1 N solution of NaOH, then with brine, dried over $Na_2SO_4$, filtered and concentrated under reduced pressure to give 3-chloro-2-isopropyl-pyridine (563 mg, crude) as yellow liquid that was used without further purification.

**Step c:** As described for **intermediate C-1**, a similar procedure was performed starting with 3-chloro-2-isopropyl-pyridine (625 mg, crude) to give (3-chloro-2-isopropyl-4-pyridyl)boronic acid (332 mg, 1.66 mmol) as a beige solid.

**Example i-53. Intermediate C-3 ((3-chloro-2-ethyl-4-pyridyl)boronic acid)**

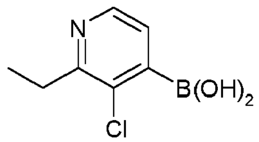

**Step a:** A mixture of 3-chloro-2-fluoro-pyridine (3.0 g, 22.81 mmol), diethyl malonate (4.15 mL, 27.4 mmol) and cesium carbonate (10.4 g, 31.9 mmol) in DMSO (45 mL) was stirred at 90 °C for 16 h. The mixture was allowed to cool down to RT, water (180 mL) was added and the aqueous layer was extracted twice with EtOAc. The combined organic layers were washed with brine, dried over $Na_2SO_4$, filtered and concentrated under reduced pressure. The residue was purified by column chromatography ($SiO_2$, Petroleum ether/Ethyl acetate= 1/0 to 8/2) to give diethyl 2-(3-chloro-2-pyridyl)propanedioate (3.09 g, 11.4 mmol) as a colourless oil.

**Step b:** A mixture of diethyl 2-(3-chloro-2-pyridyl)propanedioate (3.09 g, 11.4 mmol), methyl iodide (2.12 mL, 34.1 mmol) and potassium carbonate (3.9 g, 28.4 mmol) in DMF (25 mL) was stirred at RT for 18 h. Water (100 mL) was added and the aqueous layer was extracted twice with EtOAc. The combined organic layers were washed with brine, dried over $Na_2SO_4$, filtered and concentrated under reduced pressure. The residue was purified by column chromatography ($SiO_2$, Petroleum ether/Ethyl acetate= 1/0 to 8/2) to give diethyl 2-(3-chloro-2-pyridyl)-2-methyl-propanedioate (2.67 g, 9.34 mmol) as a colourless oil.

**Step c:** To a solution of diethyl 2-(3-chloro-2-pyridyl)-2-methyl-propanedioate (2.66 g, 9.3 mmol) in methanol (23 mL) was added 2 N aqueous solution of NaOH (28 mL, 56 mmol). The mixture was stirred at 70 °C for 18 h and then methanol was evaporated under reduced pressure. The pH value of the mixture was adjusted to 4 with 5 M aqueous solution of HCl, methanol (20 mL) was added and the mixture was stirred at 70 °C for 20 h. 5 M aqueous solution of HCl (5 mL) was added and the mixture was stirred at 100 °C for 4 h and then methanol was evaporated under reduced pressure. The pH value of the mixture was adjusted to 11-12 with 1 N aqueous NaOH solution. The aqueous layer was separated and extracted twice with ethyl acetate. The combined organic layers were washed with brine, dried over $Na_2SO_4$, filtered and concentrated under reduced pressure to give 3-chloro-2-ethyl-pyridine (975 mg, 6.89 mmol) as a colourless oil, that was used without further purification.

**Step d:** As described for **intermediate C-1**, a similar procedure was performed starting with 3-chloro-2-ethyl-pyridine (965 mg, 6.81 mmol) to give (3-chloro-2-ethyl-4-pyridyl)boronic acid (710 mg, 3.83 mmol) as a beige solid.

**Example i-54. Intermediate C-4 ([3-chloro-2-(tetrahydropyran-2-yloxymethyl)-4-pyridyl]boronic acid)**

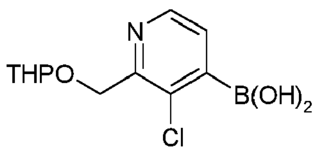

Step a: A mixture of (3-chloro-2-pyridyl)methanol (1.0 g, 6.62 mmol), 3,4-dihydro-2H-pyran (1.24 mL, 13.23 mmol) and PPTS (167 mg, 0.66 mmol) in dichloromethane (16.5 mL) was heated at 40 °C for 16 h The mixture was allowed to cool down to RT and the mixture was washed with a 1 N solution of NaOH, with brine, dried over $Na_2SO_4$, filtered and concentrated under reduced pressure. The residue was purified by column chromatography ($SiO_2$, cyclohexane/ethyl acetate= 1/0 to 7/3) to give 3-chloro-2-(tetrahydropyran-2-yloxymethyl)pyridine (1.41 g, 6.19 mmol) as a colourless oil.

Step b: As described for **intermediate C-1**, a similar procedure was performed starting with 3-chloro-2-(tetrahydropyran-2-yloxymethyl)pyridine (1.4 g, 6.15 mmol) to give [3-chloro-2-(tetrahydropyran-2-yloxymethyl)-4-pyridyl]boronic acid (540 mg, 1.99 mmol) as a beige solid.

**Example i-55. Intermediate C-5 ([5-fluoro-2-(trifluoromethyl)-4-pyridyl]boronic acid)**

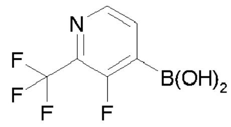

As described for **intermediate C-1**, a similar procedure was performed starting with 5-fluoro-2-trifluoromethylpyridine (1.0 g, 5.92 mmol) to give [5-fluoro-2-(trifluoromethyl)-4-pyridyl]boronic acid (206 mg, 0.99 mmol) as an off-white solid.

**Example i-56. Intermediate C-6 ((5-fluoro-2,3-dihydro-1,4-benzodioxin-6-yl)boronic acid)**

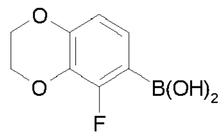

**Step a:** To a solution of 5-fluoro-2,3-dihydro-1,4-benzodioxine (780 mg, 5.06 mmol) in methanol (10 mL) was added NBS (900 mg, 5.06 mmol) at RT. The reaction mixture was stirred at 70 °C for 3 h and then concentrated under reduced pressure. The residue was taken up in water (30 mL) and dichloromethane (70 mL) and the mixture was filtered on a hydrophobic cartridge (liquid / liquid extraction column, Radleys®) and then concentrated in vacuum. The residue was purified by column chromatography ($SiO_2$, cyclohexane/ethyl acetate = 100/0 to 95/5) to give a ~3/1 mixture of 6-bromo-5-fluoro-2,3-dihydro-1,4-benzodioxine and 5-bromo-8-fluoro-2,3-dihydro-1,4-benzodioxine (713 mg) as a beige solid, that was used without further purification.

**Step b:** To a solution of the mixture previously obtained (713 mg) in anhydrous THF (10 mL) was added dropwise n-BuLi (2.1 M in hexanes, 2.2 mL, 4.6 mmol) at -78 °C. After addition, the mixture was stirred at this temperature for 1 h and triisopropyl borate (1.1 mL, 4.8 mmol) was then added dropwise. After 1 h at -78 °C, the mixture was allowed to warm to 0 °C and was then quenched with 2.5 N solution of HCl (6 mL). The reaction mixture was stirred at RT for 30 min. Water (25 mL) was added and the mixture was extracted with EtOAc (25 mL * 3). The combined organic layer was dried over $Na_2SO_4$, filtered and concentrated under reduced pressure. The residue was taken up in pentane, filtered to give a mixture containing as the major product (5-fluoro-2,3-dihydro-1,4-benzodioxin-6-yl)boronic acid (185 mg, crude) as a white solid, that was used without further purification.

**Example i-57. Intermediate C-7 (2-(6-fluoro-2,3-dihydro-1,4-benzodioxin-7-yl)-4,4,5,5-tetramethyl-1,3,2-dioxaborolane)**

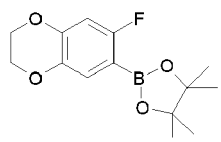

**Step a:** To a solution of 4-fluorocatechol (2.0 g, 15.6 mmol) in DMF (30 mL) was added cesium carbonate (12.7 g, 39.0 mmol) at RT. The reaction mixture was stirred at 40 °C for 30 min and then ethylene bromide (1.7 mL, 19.7 mmol) was added. The reaction mixture was stirred at 80 °C for 18 h and then allowed to cool down to RT. The solid was filtered and water (100 mL) and EtOAc (200 mL) were added to the filtrate. The aqueous layer was separated and then extracted with EtOAc (100 mL). The combined organic layer was washed with brine, dried over $Na_2SO_4$, concentrated under reduced pressure. The residue was purified by column chromatography ($SiO_2$, cyclohexane/ethyl acetate = 95/5 to 80/20) to give 6-fluoro-2,3-dihydro-1,4-benzodioxine (365 mg, 2.35 mmol) as a colourless oil.

**Step b:** To a solution of 6-fluoro-2,3-dihydro-1,4-benzodioxine (360 mg, 2.34 mmol) in acetonitrile (5 mL) were added NBS (620 mg, 3.48 mmol) and TFA (20 μL, 0.26 mmol) at RT. The mixture was stirred for 2 h and was then concentrated in vacuum. The residue was purified by column chromatography ($SiO_2$, cyclohexane/ethyl acetate = 100/0 to 95/5) to give 6-bromo-7-fluoro-2,3 -dihydro-1,4-benzodioxine (392 mg, 1.68 mmol) as a white solid.

**Step c:** A mixture of 6-bromo-7-fluoro-2,3-dihydro-1,4-benzodioxine (670 mg, 2.76 mmol), potassium acetate (495 mg, 5.04 mmol), bis(pinacolato)diboron (470 mg, 1.85 mmol) and Pd(dppf)$Cl_2$·DCM (45 mg, 0.05 mmol) in dioxane (6 mL) was stirred at 80 °C for 18 h. To the mixture was added potassium acetate (495 mg, 5.04 mmol), bis(pinacolato)diboron (470 mg, 1.85 mmol) and Pd(dppf)$Cl_2$·DCM (90 mg, 0.11 mmol) and the mixture was stirred at 80 °C for 24 h. The mixture was allowed to cool down to RT, water (100 mL) and EtOAc (100 mL) were added and the mixture was filtered through a pad of dicalite. The aqueous layer was separated and then extracted with EtOAc (100 mL). The combined organic layers were washed with brine, dried over $Na_2SO_4$, filtered and concentrated under reduced pressure. The residue was purified by column chromatography ($SiO_2$, cyclohexane/ethyl acetate = 95/5 to 80/20) to give 2-(6-fluoro-2,3-dihydro-1,4-benzodioxin-7-yl)-4,4,5,5-tetramethyl-1,3,2-dioxaborolane along with ~1 mol of bis(pinacolato)diboron (171 mg) as a white solid that was used without further purification.

**Example i-58. Intermediate C-8 (1-ethyl-5-methyl-4-(4,4,5,5-tetramethyl-1,3,2-dioxaborolan-2-yl)pyrazole)**

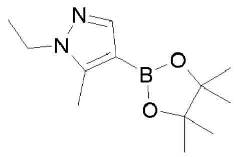

To a suspension of 4-bromo-1-ethyl-5-methyl-1H-pyrazole (400 mg, 2.11 mmol) in anhydrous THF (6 mL) was added dropwise n-BuLi (2.1 M in hexanes, 1.2 mL, 2.5 mmol) at -50 °C. After addition, the mixture was stirred at this temperature for 10 min and 2-isopropoxy-4,4,5,5-tetramethyl-1,3,2-dioxaborolane (0.44 mL, 2.16 mmol) was added dropwise. The mixture was allowed to warm to RT. The mixture was stirred at RT for 1 h and was then quenched by addition of a concentrated solution of $NH_4Cl$ (5 mL). Water (40 mL) was added and the mixture was extracted with EtOAc (30 mL $*$ 2). The combined organic layer was washed with brine, dried over $Na_2SO_4$, filtered and concentrated under reduced pressure to give 1-ethyl-5-methyl-4-(4,4,5,5-tetramethyl-1,3,2-dioxaborolan-2-yl)pyrazole (508 mg, crude) as a yellow oil that was used without further purification.

**Example i-59. Intermediate C-9 (5-chloro-1-methyl-4-(4,4,5,5-tetramethyl-1,3,2-dioxaborolan-2-yl)pyrazole)**

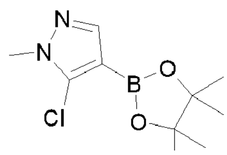

**Step a:** To a suspension of 4-iodo-1-methyl-1H-pyrazole (1.0 g, 4.8 mmol) in anhydrous THF (8 mL) was added dropwise LDA (2 M, 3.2 mL, 6.4 mmol) at -78 °C. After addition, the mixture was stirred at this temperature for 20 min and then a solution of hexachloroethane (1.4 g, 5.9 mmol) in anhydrous THF (2.5 mL) was added dropwise. The mixture was stirred at -78 °C for 30 min and then allowed to warm to -10 °C. The mixture was then quenched by addition of water (5 mL). Concentrated solution of $NH_4Cl$ (50 mL) was added and the mixture was extracted with EtOAc (50 mL x 2). The combined organic layers were washed with brine, dried over $Na_2SO_4$, filtered and concentrated under reduced pressure. The residue was purified by column chromatography ($SiO_2$, cyclohexane/ethyl acetate = 1/0 to 95/5) to give 5-chloro-4-iodo-1-methyl-pyrazole (670 mg, 2.76 mmol) as a yellow solid. $^1$H NMR (400 MHz, DMSO-*d6*) $\delta$ 7.63 (s, 1H), 3.86 (s, 3H).

**Step b:** A mixture of 5-chloro-4-iodo-1-methyl-pyrazole (670 mg, 2.76 mmol), potassium acetate (820 mg, 8.35 mmol), bis(pinacolato)diboron (1.06 g, 4.17 mmol) and X Phos Pd G2 (220 mg, 0.28 mmol) in DMSO (4 mL) was stirred at 55 °C for 4 h. The mixture was allowed to cool down to RT, water (30 mL) was added and the mixture was extracted with EtOAc (30 mL x 2). The combined organic layers were washed with brine, dried over $Na_2SO_4$, filtered and concentrated under reduced pressure. The residue was purified by column chromatography ($SiO_2$, cyclohexane/ethyl acetate = 1/0 to 0/1) to give 5-chloro-1-methyl-4-(4,4,5,5-tetramethyl-1,3,2-dioxaborolan-2-yl)pyrazole (820 mg, crude) as a black solid that was used without further purification.

**Example i-60. Intermediate C-10 ((5-Fluoroquinoxalin-6-yl)boronic acid)**

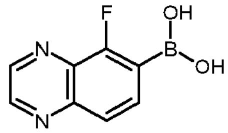

**Step a:** A mixture of 4-bromo-3-fluoro-benzene-1,2-diamine (270 mg, 1.3 mmol) and 1,4-dioxane-2,3-diol (221 mg, 1.8 mmol) in ethanol (5 mL) was stirred at RT for 18 h. 1,4-Dioxane-2,3-diol (55 mg, 0.45 mmol) was added and the mixture was stirred for an additional hour. The mixture was then concentrated under reduced pressure to dryness. Water and ethyl acetate were added, the aqueous layer was separated and extracted twice with ethyl acetate. The combined organic layers were washed with brine, dried over $Na_2SO_4$, filtered and concentrated under reduced pressure. The residue was purified by column chromatography ($SiO_2$, heptane/ethyl acetate = 1/0 to 0/1) to give 6-bromo-5-fluoro-quinoxaline (190 mg, 0.84 mmol) as a white solid. LCMS m/z $[M+H]^+$ = 227.

**Step b:** A mixture of 6-bromo-5-fluoro-quinoxaline (100 mg, 0,44 mmol), 4,4,5,5-tetramethyl-2-(4,4,5,5-tetramethyl-1,3,2-dioxaborolan-2-yl)-1,3,2-dioxaborolane (336 mg, 1.32 mmol), and potassium acetate (130 mg, 1.32 mmol) in 1,4-dioxane (5 mL) was degassed with argon for 10 min. [1,1'-Bis(diphenylphosphino)ferrocene]dichloropalladium (37 mg, 0.053 mmol) was added and the resulting mixture was degassed under argon for 5 additional min and heated at 100 °C for 10 h. The reaction mixture was filtered on a 0.45 μm Whatman filter and the solid material washed with methanol and acetonitrile. The resulting liquor was concentrated under reduced pressure to dryness. The residue was purified by preparative HPLC chromatography (column C18 Sun Fire 30x100, 5 μm, Water with 0.1% formic acid /Acetonitrile with 0.1% formic acid, 95/5 to 50/50) to give (5-fluoroquinoxalin-6-yl)boronic acid as a white solid (42 mg, 0.21 mmol). LCMS m/z $[M+H]^+$ = 193.

**Example i-61. Intermediate C-11 ((5-Chloroquinoxalin-6-yl)boronic acid)**

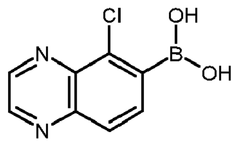

A mixture of 6-bromo-5-chloro-quinoxaline (200 mg, 0.82 mmol), 4,4,5,5-tetramethyl-2-(4,4,5,5-tetramethyl-1,3,2-dioxaborolan-2-yl)-1,3,2-dioxaborolane (417 mg, 1.64 mmol), and potassium acetate (242 mg, 46 mmol) in 1,4-dioxane (5 mL) was degassed with argon for 10 minutes. [[1,1'-Bis(diphenylphosphino)ferrocene]dichloropalladium (72 mg, 0.10 mmol) was then added and the resulting mixture was degassed with argon for 5 additional min and stirred at 75 °C for 1 h. The reaction mixture was filtered on a 0.45 μm Whatman filter and the solid material was washed with methanol and acetonitrile. The resulting liquor was concentrated under reduced pressure to dryness. The residue was purified by preparative HPLC chromatography (column C18 Sun Fire 30x100, 5 μm, Water with 0.1% formic acid /Acetonitrile with 0.1% formic acid, 80/20 to 30/70) to give 5-chloroquinoxalin-6-yl)boronic acid as a white solid (115 mg). LCMS m/z $[M+H]^+$ = 209.1, purity UV/DAD = 76%. The product was used in the following step without further purification.

**Example i-62. Intermediate D-1 ((R)-N-[(5S)-1'-(7-bromo-6-methyl-pyrazolo[1,5-a]pyrazin-4-yl)spiro[5,7-dihydrocyclopenta[b]pyridine-6,4'-piperidine]-5-yl]-2-methyl-propane-2-sulfinamide)**

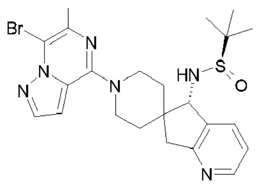

**Step a:** To a solution of **intermediate B-1** (15.0 g, 62.8 mmol) and **intermediate A-1** (20.1 g, 69.1 mmol) in NMP (300 mL) was added DIEA (57 mL, 327 mmol). The mixture was stirred at 90 °C for 6 h. The mixture was poured into water (1.5 L), and then extracted with ethyl acetate (1.0 L ∗ 2). The combined organic phase was washed with brine (500 mL), dried with anhydrous $Na_2SO_4$, filtered and concentrated. The residue was purified by column chromatography ($SiO_2$, Petroleum ether/Ethyl acetate= 100/1 to 0/1) to give 1'-(7-bromo-6-methyl-pyrazolo[1,5-a]pyrazin-4-yl)spiro[7H-cyclopenta[b]pyridine-6,4'-piperidine]-5-one (20 g, 48.5 mmol) as a yellow solid. $^1H$ NMR (400 MHz, DMSO-*d6*) δ 8.91 (d, *J* = 1.6 Hz, 1H), 8.06-8.11 (m, 2H), 7.49-7.53 (m, 1H), 7.19 (d, *J* = 2.4 Hz, 1H), 4.43 *(d, J*= 13.6 Hz, 2H), 3.36-3.40 (m, 3H), 2.46 (s, 3H), 1.88-1.95 (m, 2H), 1.60-1.63 (m, 2H).

**Step b:** To a solution of 1'-(7-bromo-6-methyl-pyrazolo[1,5-a]pyrazin-4-yl)spiro[7H-cyclopenta[b]pyridine-6,4'-piperidine]-5-one (20.0 g, 48.5 mmol) in $Ti(OEt)_4$ (200 mL) was added (R)-2-methylpropane-2-sulfinamide (11.7 g, 97.0 mmol). The mixture was stirred at 90 °C for 12 h. The mixture was poured into water (1.0 L) and EtOAc (1.0 L) and was then filtered. The aqueous layer was separated and then extracted with EtOAc (1.0 L). The combined organic layers were washed with brine (500 mL), dried over $Na_2SO_4$, filtered and concentrated under reduced pressure. The residue was purified by column chromatography ($SiO_2$, Petroleum ether/Ethyl acetate= 100/1 to 0/1) to give (NZ,R)-N-[1'-(7-bromo-6-methyl-pyrazolo[1,5-a]pyrazin-4-yl)spiro[7H-cyclopenta[b]pyridine-6,4'-piperidine]-5-ylidene]-2-methyl-propane-2-sulfinamide (15.0 g, 29.1 mmol) as a yellow solid. $^1H$ NMR (400 MHz, DMSO-*d6*) δ 8.73 (d, *J* = 1.6 Hz, 1H), 8.61 (s, 1H), 8.01 (d, *J* = 8.4 Hz, 1H), 7.42-7.45 (m, 1H), 5.26 (s, 2H), 4.40 (d, *J* = 11.6 Hz, 2H), 3.24 (s, 3H), 2.40 (s, 3H), 1.95-1.97 (m, 2H), 1.61-1.69 (m, 2H), 1.17 (s, 9H).

**Step c:** To a solution of (NZ,R)-N-[1'-(7-bromo-6-methyl-pyrazolo[1,5-a]pyrazin-4-yl)spiro[7H-cyclopenta[b]pyridine-6,4'-piperidine]-5-ylidene]-2-methyl-propane-2-sulfinamide (11.5 g, 22.3 mmol) in dry THF (230 mL) was added dropwise DIBAL-H (1 M in toluene, 33.5 mL, 33.5 mmol) at -78 °C under Ar. The mixture was stirred at -78 °C for 30 min and ethyl acetate (350 mL) and then a 1 M aqueous solution of Rochelle salt (100 mL) were added, the mixture was allowed to warm to RT and stirred for 30 min. The organic layer was separated and the aqueous phase was extracted with ethyl acetate (100 mL). The combined organic phase was washed with brine, dried with anhydrous $Na_2SO_4$, filtered and concentrated under vacuum. The residue was taken up in diisopropyl ether (200 mL) and stirred for 30 min, the precipitate was filtered and washed with diisopropyl ether (20 mL) and then pentane (50 mL) to give (R)-N-[(5S)-1'-(7-bromo-6-methyl-pyrazolo[1,5-a]pyrazin-4-yl)spiro[5,7-dihydrocyclopenta[b]pyridine-6,4'-piperidine]-5-yl]-2-methyl-propane-2-sulfinamide (9.0 g, 17.39 mmol) as a white solid. $^1H$ NMR (400 MHz, DMSO-*d6*) δ 8.38 (d, *J* = 4.8 Hz, 1H), 8.02 (s, 1H), 7.61 (d, *J* = 7.6 Hz, 1H), 7.22 (m, 1H), 7.10 (s, 1H), 5.78 (d, *J* = 10 Hz, 1H), 4.48 (d, *J*= 10.4 Hz, 1H), 4.37 *(d, J*= 13.2 Hz, 2H), 3.17-3.30 (m, 3H), 2.85 *(d, J*= 16.4 Hz, 2H), 2.41 (s, 3 H), 2.02-2.07 (m, 2H), 1.82-1.89 (m, 2H), 1.63-1.67 (m, 1H), 1.36-1.39 (m, 2H), 1.18 (s, 9H).

**Example i-63. Intermediate D-2 ((5S)-1'-(7-bromo-6-methyl-pyrazolo[1,5-a]pyrazin-4-yl)spiro[5,7-dihydrocyclopenta[b]pyridine-6,4'-piperidine]-5-amine)**

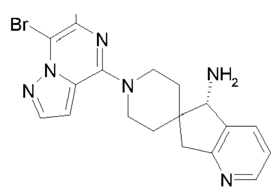

To a mixture of intermediate **D-1** (980 mg, 1.89 mmol) in dichloromethane (10 mL) and methanol (10 mL) was added dropwise a solution of HCl (4 M in dioxane, 3 mL, 12 mmol) at RT. The mixture was stirred for 2 h and then concentrated under vacuo. The residue was taken up in diethyl ether and the solid was collected by filtration. The solid was taken up in ethyl acetate (25 mL) and water (25 mL) and the pH value of the aqueous phase was adjusted to 11-12 with 35% aqueous NaOH solution. The aqueous layer was separated and extracted with ethyl acetate (25 mL). The combined organic phase was washed with brine (25 mL), dried with anhydrous $Na_2SO_4$, filtered and concentrated to give (5S)-1'-(7-bromo-6-methyl-pyrazolo[1,5-a]pyrazin-4-yl)spiro[5,7-dihydrocyclopenta[b]pyridine-6,4'-piperidine]-5-amine (640 mg, 1.55 mmol) as a beige solid. LCMS m/z $[M+H]^+$ = 413.2.

**Example i-64. Intermediate D-3 (tert-butyl N-[(5S)-1'-(7-bromo-6-methyl-pyrazolo[1,5-a]pyrazin-4-yl)spiro[5,7-dihydrocyclopenta[b]pyridine-6,4'-piperidine]-5-yl]carbamate)**

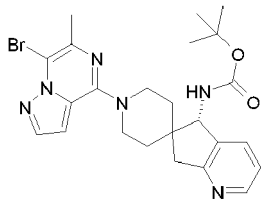

**Step a:** To a mixture of **intermediate D-1** (6.0 g, 11.59 mmol) in methanol (60 mL) was added slowly a solution of HCl (4 M in dioxane, 15 mL, 60 mmol) at RT. The mixture was stirred for 1 h and then concentrated under vacuo. The residue was taken up in diethyl ether, the mixture was stirred for 16h and then filtered to give (5S)-1'-(7-bromo-6-methyl-pyrazolo[1,5-a]pyrazin-4-yl)spiro[5,7-dihydrocyclopenta[b]pyridine-6,4'-piperidine]-5-amine hydrochloride (6.15 g, crude) as a white solid that was used without further purification.

**Step b:** To a mixture of (5S)-1'-(7-bromo-6-methyl-pyrazolo[1,5-a]pyrazin-4-yl)spiro[5,7-dihydrocyclopenta[b]pyridine-6,4'-piperidine]-5-amine hydrochloride (6.15 g, crude) in dichloromethane (200 mL), at 0 °C, was added di-tert-butyl dicarbonate (3.35 g, 15.3 mmol) followed by triethylamine (8.2 mL, 59 mmol). The mixture was stirred for 3 h at RT, then cooled to 0 °C, and di-tert-butyl dicarbonate (1.0 g, 4.6 mmol) and triethylamine (3 mL, 21.5 mmol) were added. The mixture was stirred at RT for 48 h. A concentrated aqueous solution of $NaHCO_3$ (150 mL) was added. The aqueous layer was separated and extracted with dichloromethane (200 mL). The combined organic phase was washed with brine (100 mL), dried with anhydrous $Na_2SO_4$, filtered and concentrated under vacuo. The residue was purified by column chromatography ($SiO_2$, cyclohexane/ethyl acetate = 6/4 to 4/6) to give tert-butyl N-[(5S)-1'-(7-bromo-6-methyl-pyrazolo[1,5-a]pyrazin-4-yl)spiro[5,7-dihydrocyclopenta[b]pyridine-6,4'-piperidine]-5-yl]carbamate (4.87 g, 9.49 mmol) as a pink solid. LCMS m/z $[M+H]^+$ = 513.2.

**Example i-65. Intermediate D-4 ((R)-N-[(5S)-1'-(7-bromo-6-methyl-pyrazolo[1,5-a]pyrazin-4-yl)-3-chloro-spiro[5,7-dihydrocyclopenta[b]pyridine-6,4'-piperidine]-5-yl]-2-methyl-propane-2-sulfinamide)**

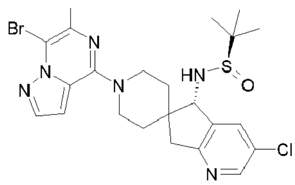

Step a: As described for **intermediate D-1** step a, a similar procedure was performed using **intermediate B-**5 (350 mg, 1.28 mmol) and **intermediate A-1** (340 mg, 1.38 mmol) to give 1'-(7-bromo-6-methyl-pyrazolo[1,5-a]pyrazin-4-yl)-3-chloro-spiro[7H-cyclopenta[b]pyridine-6,4'-piperidine]-5-one (185 mg, 0.41 mmol) as a yellow solid. LCMS m/z $[M+H]^+$ = 446.0.

Step b: As described for **intermediate D-1** step b, a similar procedure was performed using 1'-(7-bromo-6-methy-pyrazolo[1,5-a]pyrazin-4-yl)-3-chloro-spiro[7H-cyclopenta[b]pyridine-6,4'-piperidine]-5-one(170 mg, 0.38 mmol), (R)-2-methylpropane-2-sulfinamide (195 mg, 1.61 mmol) in $Ti(OEt)_4$ (1.2 mL) to give (NZ,R)-N-[1'-(7-bromo-6-methyl-pyrazolo[1,5-a]pyrazin-4-yl)-3-chloro-spiro[7H-cyclopenta[b]pyridine-6,4'-piperidine]-5-ylidene]-2-methylpropane-2-sulfinamide (147 mg, 0.27 mmol) as a yellow solid. LCMS m/z $[M+H]^+$ = 549.0.

**Step c**: As described for **intermediate D-1** step c, a similar procedure was performed using (NZ,R)-N-[1'-(7-bromo-6-methyl-pyrazolo[1,5-a]pyrazin-4-yl)-3-chloro-spiro[7H-cyclopenta[b]pyridine-6,4'-piperidine]-5-ylidene]-2-methyl-propane-2-sulfinamide (145 mg, 0.26 mmol) and DIBAL-H (1 M in toluene, 0.6 mL, 0.6 mmol) to give (R)-N-[(5S)-1'-(7-bromo-6-methyl-pyrazolo[1,5-a]pyrazin-4-yl)-3-chloro-spiro[5,7-dihydrocyclopenta[b]pyridine-6,4'-piperidine]-5-yl]-2-methyl-propane-2-sulfinamide (167 mg, crude) as an orange solid. LCMS m/z $[M+H]^+$ = 551.0.

**Example i-66. Intermediate D-5 (1'-(7-bromo-6-methyl-pyrazolo[1,5-a]pyrazin-4-yl)-3-methoxy-spiro[5,7-dihydrocyclopenta[b]pyridine-6,4'-piperidine]-5-amine)**

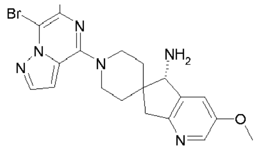

To a solution of **intermediate B-6** (320 mg, crude) and **intermediate A-1** (260 mg, 1.05 mmol) in NMP (8 mL) was added DIEA (1 mL, 5.74 mmol). The mixture was stirred at 80 °C for 18 h. The mixture was poured into water (40 mL) and extracted with ethyl acetate (40 mL * 2). The combined organic phase was washed with brine (40 mL), dried with anhydrous $Na_2SO_4$, filtered and concentrated. The residue was purified by column chromatography ($SiO_2$, dichloromethane: methanol = 1:0 to 95:5) to give 1'-(7-bromo-6-methyl-pyrazolo[1,5-a]pyrazin-4-yl)-3-methoxy-spiro[5,7-dihydrocyclopenta[b]pyridine-6,4'-piperidine]-5-amine (76 mg, 0.17 mmol) as a brown wax. LCMS m/z $[M+H]^+$ = 443.1.

**Example i-67. Intermediate D-6 (tert-butyl N-[(5S)-1'-(7-bromo-6-methyl-pyrazolo[1,5-a]pyrazin-4-yl)-3-fluoro-spiro[5,7-dihydrocyclopenta[b]pyridine-6,4'-piperidine]-5-yl]carbamate)**

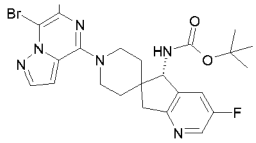

To a solution of **intermediate B-7** (900 mg, crude) and **intermediate A-1** (860 mg, 3.5 mmol) in DMF (17.5 mL) was added DIEA (3.1 mL, 5.74 mmol). The mixture was stirred at 60 °C for 18 h. The mixture was poured into 1 N aqueous solution of NaOH (70 mL) and extracted with ethyl acetate (50 mL * 3). The combined organic phase was washed with brine (40 mL), dried with anhydrous $Na_2SO_4$, filtered and concentrated. The residue was taken up in THF (10 mL) and to this mixture was added $Boc_2O$ (1.15 g, 5.27 mmol) and triethylamine (1 mL, 7.17 mmol). The mixture was stirred at RT for 20 h and water and ethyl acetate were then added. The aqueous layer was separated and extracted with ethyl acetate. The combined organic phase was washed with brine, dried with anhydrous $Na_2SO_4$, filtered and concentrated. The residue was purified by column chromatography ($SiO_2$, cyclohexane: ethyl acetate= 1:0 to 6:4) to give tert-butyl N-[(5S)-1'-(7-bromo-6-methyl-pyrazolo[1,5-a]pyrazin-4-yl)-3-fluoro-spiro[5,7-dihydrocyclopenta[b]pyridine-6,4'-piperidine]-5-yl]carbamate (855 mg, 1.61 mmol) as a white foam. LCMS m/z $[M+H]^+$ = 531.2.

**Example i-68. Intermediate D-7 ((1S)-1'-(7-bromo-6-methyl-pyrazolo[1,5-a]pyrazin-4-yl)-5-methoxy-spiro[indane-2,4'-piperidine]-1-amine)**

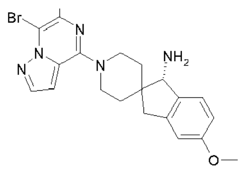

As described for **intermediate D-5**, a similar procedure was performed using **intermediate B-8** (750 mg, 2.46 mmol) and **intermediate A-1** (730 mg, 2.96 mmol) to give (1S)-1'-(7-bromo-6-methyl-pyrazolo[1,5-a]pyrazin-4-yl)-5-methoxy-spiro[indane-2,4'-piperidine]-1-amine (62 mg, 0.14 mmol). LCMS m/z $[M+H]^+$ = 442.1.

**Example i-69. Intermediate D-8 (tert-butyl (1'-(7-bromo-6-methylpyrazolo[1,5-a]pyrazin-4-yl)-2-methoxy-5,7-**dihydrospiro[cyclopenta[b]pyridine-6,4'-piperidin]-7-yl)carbamate)

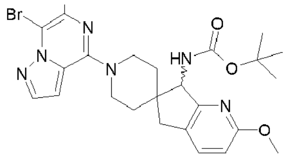

As described for **intermediate D-6,** a similar procedure was performed using **intermediate B-9** (685 mg, 2 mmol) and **intermediate A-1** (493 mg, 2 mmol) to give tert-butyl (1'-(7-bromo-6-methylpyrazolo[1,5-a]pyrazin-4-yl)-2-methoxy-5,7-dihydrospiro[cyclopenta[b]pyridine-6,4'-piperidin]-7-yl)carbamate (500 mg, 0.92 mmol) as a brown foam and as a mixture of enantiomers that was used without further purification. LCMS m/z $[M+H]^+$ = 543.3. Example i-70. Intermediate D-9 (3-amino-1'-(7-bromo-6-methyl-pyrazolo[1,5-a]pyrazin-4-yl)spiro[indane-2,4'-piperidine] -5-carbonitrile)

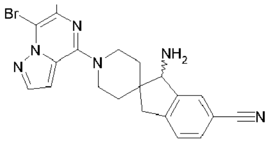

As described for **intermediate D-5**, a similar procedure was performed using **intermediate B-10** (335 mg, crude) and **Intermediate A-1** (330 mg, 1.34 mmol) to give 3-amino-1'-(7-bromo-6-methyl-pyrazolo[1,5-a]pyrazin-4-yl)spiro[indane-2,4'-piperidine]-5-carbonitrile (167 mg, 0.38 mmol) as an orange solid and as a mixture of enantiomers that was used without further purification. LCMS m/z $[M+H]^+$ = 443.1.

**Example i-71. Intermediate D-10 (1'-(7-bromo-6-methyl-pyrazolo[1,5-a]pyrazin-4-yl)-spiro[5,7-dihydrocyclopenta[b]pyridine-6,4'-piperidine]-5-amine)**

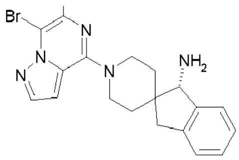

As described for **intermediate D-5,** a similar procedure was performed using (1S)-1,3-dihydrospiro[indene-2,4'-piperidin]-1-amine dihydrochloride (750 mg, 2.64 mmol) and **intermediate A-1** (700 mg, 2.84 mmol) to give (S)-1'-(7-bromo-6-methylpyrazolo[1,5-a]pyrazin-4-yl)-1,3-dihydrospiro[indene-2,4'-piperidin]-1-amine (461 mg, 1.12 mmol) as a red solid. LCMS m/z $[M+H]^+$ = 412.2.

**Example i-72. Intermediate D-11 (1'-(7-bromo-6-methyl-pyrazolo[1,5-a]pyrazin-4-yl)-spiro[5,7-dihydrocyclopenta[b]pyridine-6,4'-piperidine]-5-amine)**

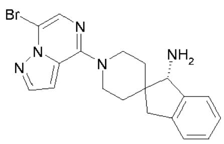

As described for **intermediate** D-5, a similar procedure was performed using (1S)-1,3-dihydrospiro[indene-2,4'-piperidin]-1-amine dihydrochloride (240 mg, 0.85 mmol) and **intermediate A-3** (250 mg, 1.08 mmol) to give (S)-1'-(7-bromopyrazolo[1,5-a]pyrazin-4-yl)-1,3-dihydrospiro[indene-2,4'-piperidin]-1-amine (137 mg, 0.34 mmol) as an orange oil. LCMS m/z $[M+H]^+$ = 398.1.

**Example i-73. Intermediate D-12 (tert-butyl N-[(5S)-1'-(7-bromopyrazolo[1,5-a]pyrazin-4-yl)spiro[5,7-dihydrocyclopenta[b]pyridine-6,4'-piperidine]-5-yl]carbamate)**

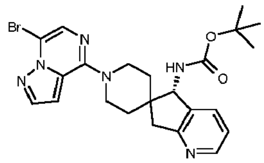

A mixture of **intermediate B-4** (900 mg), **intermediate A-2** (703 mg, 3.022 mmol), potassium carbonate (2.39 g, 17.3 mmol) in dimethylformamide (20 mL) was stirred at 80 °C for 20 h and cooled down to RT. After removal of the solid material by filtration, the filtrate was concentrated under reduced pressure. The residue was taken up in dichloromethane (40 mL), and to the resulting solution under stirring at RT, were added triethylamine (1.2 mL, 8.63 mmol) and di-tert-butyl pyrocarbonate (659 mg, 3.021 mmol). After 20 h at RT, a saturated solution of sodium carbonate (100 mL) was added to the reaction medium and the mixture decanted. The organic layer was washed with brine (50 mL), dried over $MgSO_4$ and concentrated under reduced pressure. The residue was purified by column chromatography ($SiO_2$, Heptane/Ethyl acetate= 50/50 to 0/100) to give tert-butyl N-[(5S)-1'-(7-bromopyrazolo[1,5-a]pyrazin-4-yl)spiro[5,7-dihydrocyclopenta[b]pyridine-6,4'-piperidine]-5-yl]carbamate (1.03 g, 2.06 mmol) as a white solid. LCMS m/z $[M+H]^+$ = 499.

**Example i-74. Intermediate D-13 ((R)-N-[(5S)-1'-(7-bromo-6-methyl-pyrazolo[1,5-a]pyrazin-4-yl)-2-methoxy-spiro[5,7-dihydrocyclopenta[b]pyridine-6,4'-piperidine]-5-yl]-2-methyl-propane-2-sulfinamide)**

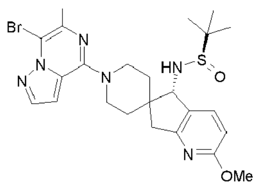

A mixture of **intermediate B-34** (360 mg, 1.07 mmol), **intermediate A-1** (290 mg, 1.17 mmol) and potassium carbonate (450 mg, 3.26 mmol) in DMF (6 mL) was stirred at 85 °C for 1 h. Water (30 mL) and ethyl acetate (30 mL) were added, the aqueous layer was separated and then extracted with ethyl acetate (30 mL). The combined organic phase was washed with brine, dried over anhydrous $Na_2SO_4$, filtered and concentrated under reduced pressure. The residue was purified by column chromatography ($SiO_2$, dichloromethane / methanol = 1/0 to 95/5) to give (R)-N-[(5S)-1'-(7-bromo-6-methyl-pyrazolo[1,5-a]pyrazin-4-yl)-2-methoxy-spiro[5,7-dihydrocyclopenta[b]pyridine-6,4'-piperidine]-5-yl]-2-methyl-propane-2-sulfinamide (421 mg, 0.77 mmol) as a pink foam. LCMS m/z $[M+H]^+$ = 457.3.

**Example i-75. Intermediate D-14 (N-[(7S)-1'-(7-bromo-6-methyl-pyrazolo[1,5-a]pyrazin-4-yl)-3-methyl-spiro[5,7-dihydrocyclopenta[c]pyridine-6,4'-piperidine]-7-yl]-2-methyl-propane-2-sulfinamide)**

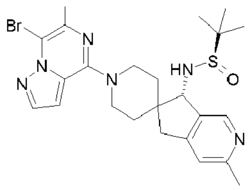

As described for **intermediate D-13**, a similar procedure was performed using **intermediate B-36** (284 mg, 0.88 mmol) and **intermediate A-2** (298 mg, 0.97 mmol) to give N-[(7S)-1'-(7-bromo-6-methyl-pyrazolo[1,5-a]pyrazin-4-yl)-3-methyl-spiro[5,7-dihydrocyclopenta[c]pyridine-6,4'-piperidine]-7-yl]-2-methyl-propane-2-sulfinamide (355 mg, 0.67

mmol) as a pink foam. LCMS m/z $[M+H]^+$ = 531.2.

**Example i-76. Intermediate D-15 ((R)-N-[(7S)-1'-(7-bromo-6-methylpyrazolo[1,5-a]pyrazin-4-yl)spiro[5,7-dihydrocyclopenta[b]pyridine-6,4'-piperidine]-7-yl]-2-methylpropane-2-sulfinamide)**

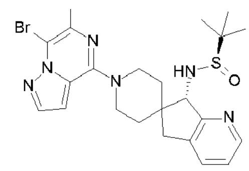

Step a: To a solution of **intermediate B-31** (490 mg, 1.79 mmol) and **intermediate A-1** (400 mg, 1.79 mmol) in NMP (8 mL) was added DIEA (1.07 g, 8.14 mmol). The mixture was stirred at 90 °C for 4 h. The mixture was poured into water (50 mL) and extracted with ethyl acetate (50 mL * 2). The combined organic phase was washed with brine (50 mL), dried with anhydrous $Na_2SO_4$, filtered and concentrated. The residue was purified by column chromatography ($SiO_2$, dichloromethane: methanol= 98:2) to furnish the expected product which was triturated in acetonitrile (10 mL), iced and filtered to give 1'-(7-bromo-6-methylpyrazolo[1,5-a]pyrazin-4-yl)spiro[5H-cyclopenta[b]pyridine-6,4'-piperidine]-7-one (515 mg, 1.24 mmol) as a beige solid. $^1$H NMR (400 MHz, DMSO-*d6*) δ 8.77 (m, 1H), 8.09-8.11 (m, 1H), 8.05 (d, *J* = 2.4 Hz 1H), 7.63-7.66 (m, 1H), 7.18 (d, *J* = 2.5 Hz, 1H), 4.38-4.43 (m, 2H), 3.33-3.40 (m, 2H), 3.23 (s, 2H), 2.45 (s, 3H), 1.85-1.92 (m, 2H), 1.56-1.59 (m, 2H). LCMS m/z $[M+H]^+$ 412.1.

**Step b:** To a solution of 1'-(7-bromo-6-methylpyrazolo[1,5-a]pyrazin-4-yl)spiro[5H-cyclopenta[b]pyridine-6,4'-piperidine]-7-one (420 mg, 1.02 mmol) in ethyl acetate (14 mL) was added $Ti(OEt)_4$ (1.07 mL, 5.1 mmol) and (R)-2-methylpropane-2-sulfinamide (252 mg, 2.04 mmol). The reaction mixture was stirred at 105 °C for 4 h $Ti(OEt)_4$ (0.53 mL, 2.55 mmol) and (R)-2-methylpropane-2-sulfinamide (126 mg, 1.02 mmol) were added and the reaction mixture was stirred at 105 °C for 2 h. The mixture was quenched with addition of water (1 mL) and diluted with ethyl acetate (20 mL). The reaction mixture was stirred for 15 min and then filtered. The organic layer was washed with water (50 mL), dried over $Na_2SO_4$, filtered and concentrated under reduced pressure. The residue was purified by column chromatography ($SiO_2$, ethyl acetate= 100 %) to give (NZ,R)-N-[1'-(7-bromo-6-methyl-pyrazolo[1,5-a]pyrazin-4-yl)spiro[5H-cyclopenta[b]pyridine-6,4'-piperidine]-7-ylidene]-2-methyl-propane-2-sulfinamide (367 mg, 0.64 mmol) as a yellow solid. $^1$H NMR (400 MHz, DMSO-*d6*) δ 8.76 (m, 1H), 8.02-8.05 (m, 2H), 7.56-7.59 (m, 1H), 7.20 (d, *J* = 2.5 Hz, 1H), 4.40-4.48 (m, 2H), 3.32-3.38 (m, 2H), 3.25 (m, 2H), 3.24 (s, 3H), 2.44 (s, 3H), 1.92-2.10 (m, 2H), 1.67-1.71 (m, 2H), 1.15 (s, 9H). LCMS m/z $[M+H]^+$ 515.2.

**Step c:** To a solution of (NZ,R)-N-[1'-(7-bromo-6-methyl-pyrazolo[1,5-a]pyrazin-4-yl)spiro[5H-cyclopenta[b]pyridine-6,4'-piperidine]-7-ylidene]-2-methyl-propane-2-sulfinamide (365 mg, 0.63 mmol) in dry THF (6 mL) was added dropwise DIBAL-H (1 M in toluene, 1.9 mL, 1.9 mmol) at -78 °C under Ar. The mixture was stirred at - 78 °C for 1.5 h and ethyl acetate (15 mL) and then a 1 M aqueous solution of Rochelle salt (3 mL) were added, the mixture was allowed to warm to RT and stirred for 30 min. The organic layer was separated and the aqueous phase was extracted with ethyl acetate (100 mL). The combined organic phase was washed with brine, dried with anhydrous $Na_2SO_4$, filtered and concentrated under vacuum. The residue was purified by column chromatography ($SiO_2$, dichloromethane:methanol = 95:5) to give (R)-N-[(7S)-1'-(7-bromo-6-methyl-pyrazolo[1,5-a]pyrazin-4-yl)spiro[5,7-dihydrocyclopenta[b]pyridine-6,4'-piperidine]-7-yl]-2-methyl-propane-2-sulfinamide (330 mg, 0.59 mmol) as a pink solid. $^1$H NMR (400 MHz, DMSO-*d6*) δ 8.39 (m, 1H), 8.02 (d, *J* = 2.4 Hz, 1H), 7.64 (m, 1H), 7.20-7.23 (m, 1H), 7.11 (d, *J* = 2.4 Hz, 1H), 5.62 (d, *J* = 9.7 Hz, 1H), 4.40 *(d, J*= 9.7 Hz, 1H), 4.25-4.29 (m, 2H), 3.36-3.42 (m, 2H), 3.13 (d, *J* = 16.1 Hz, 1H), 2.76 *(d, J*= 16.1 Hz, 1H), 2.42 (s, 3 H), 1.85-1.97 (m, 2H), 1.63-1.66 (m, 1H), 1.41-1.44 (m, 2H), 1.19 (s, 9H). LCMS m/z $[M+H]^+$ 517.2.

**Example i-77. Intermediate D-16 ((R)-N-[(1S)-1'-(7-bromo-6-methyl-pyrazolo[1,5-a]pyrazin-4-yl)-4-methoxy-spiro[indane-2,4'-piperidine]-1-yl]-2-methyl-propane-2-sulfinamide)**

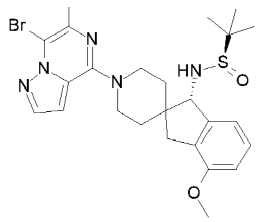

**Step a:** To a solution of **intermediate B-27** (2.41 g, 10.4 mmol) and **intermediate A-1** (2.33 g, 9.45 mmol) in DMF (50 mL) was added DIEA (4.89 g, 37.8 mmol). The mixture was stirred at 90 °C for 18 h. The mixture was diluted with ethyl acetate (50 mL) and aqueous solution $NH_4Cl$ (50 mL) and extracted with ethyl acetate (50 mL * 2). The combined organic phase was washed with brine (50 mL), dried with anhydrous $Na_2SO_4$, filtered and concentrated. The residue was purified by column chromatography ($SiO_2$, dichloromethane: methanol= 100:0 to 98:2) to furnish the expected product which was triturated in $iPr_2O$ and ethyl acetate (10 mL), iced and filtered to give 1'-(7-bromo-6-methyl-pyrazolo[1,5-a]pyrazin-4-yl)-4-methoxy-spiro[indane-2,4'-piperidine]-1-one (3.33g, 7.54 mmol) as an orange solid. LCMS m/z $[M+H]^+$ = 441.1.

**Step b:** To a solution of 1'-(7-bromo-6-methyl-pyrazolo[1,5-a]pyrazin-4-yl)-4-methoxy-spiro[indane-2,4'-piperidine]-1-one (3.27 g, 7.4 mmol) in 2-Me-THF (4.5 mL) was added $Ti(OEt)_4$ (67% pure containing 33% of $TiO_2$, 23.2 mL, 74.1 mmol) and (R)-2-methylpropane-2-sulfinamide (1.83 g, 14.8 mmol). The reaction mixture was stirred at 90 °C for 18 h. The mixture was diluted with ethyl acetate (50 mL) and THF (200 mL). Brine (50 mL) was added and the mixture was stirred 15 min. The mixture was filtered and concentrated under reduced pressure. The residue was taken up in $Et_2O$ (50 mL). The etheral layer was washed with brine (25 mL * 3), dried over $Na_2SO_4$, filtered and concentrated under reduced pressure to give a mixture of starting material and expected product. The same protocol was performed on the crude product to give a residue that was purified by column chromatography ($SiO_2$, dichloromethane:methanol 99:1) to give (NZ,R)-N-[1'-(7-bromo-6-methyl-pyrazolo[1,5-a]pyrazin-4-yl)-4-methoxy-spiro[indane-2,4'-piperidine]-1-ylidene]-2-methyl-propane-2-sulfinamide (2.7 g, 4.66 mmol) as a yellow solid. $^1$H NMR (400 MHz, DMSO-*d6*) $\delta$ 8.04 (d, *J* = 2.4 Hz, 1H), 7.87 (m, 1H), 7.44 (m, 1H), 7.24 *(d, J* = 8.0 Hz, 1H), 7.17 (d, *J* = 2.4 Hz, 1H), 4.46 *(d, J*= 13.5 Hz, 1H), 3.90 (s, 3H), 3.10 (s, 2H), 2.45 (s, 3H), 2.00 (m, 2H), 1.61 (m,2H), 1.33 (m, 1H), 1.18 (s, 9H). LCMS m/z $[M+H]^+$ = 544.1.

**Step c:** To a solution of (NZ,R)-N-[1'-(7-bromo-6-methyl-pyrazolo[1,5-a]pyrazin-4-yl)-4-methoxy-spiro[indane-2,4'-piperidine]-1-ylidene]-2-methyl-propane-2-sulfinamide (2.7 g, 4.66 mmol) in dry THF (50 mL) was added dropwise DIBAL-H (1 M in toluene, 14 mL, 14 mmol) at -78 °C under Ar. The mixture was stirred at -78 °C for 1.5 h and ethyl acetate (15 mL) and then a 1 M aqueous solution of Rochelle salt (3 mL) were added, the mixture was allowed to warm to RT and stirred for 30 min. The organic layer was separated and the aqueous phase was extracted with ethyl acetate (100 mL). The combined organic phase was washed with brine, dried with anhydrous $Na_2SO_4$, filtered and concentrated under vacuum. The residue was taken up in diisopropyl ether (40 mL) heated to 45 °C and stirred for 30 min in a sonic bath, the precipitate was iced and filtered to give (R)-N-[(1S)-1'-(7-bromo-6-methyl-pyrazolo[1,5-a]pyrazin-4-yl)-4-methoxy-spiro[indane-2,4'-piperidine]-1-yl]-2-methyl-propane-2-sulfinamide (1.97 g, 3.6 mmol) as a white powder. $^1$H NMR (400 MHz, DMSO-*d6*) d 8.39 (m, 1H), 8.02 (d, *J* = 2.4 Hz, 1H), 7.20 (m, 1H), 7.10 (d, *J* = 2.4 Hz, 1H), 6.83-6.89 (m, 2H), 5.62 *(d, J*= 10.4 Hz, 1H), 4.39 (m, 3H), 3.79 (s, 3H), 3.04 (d, *J*= 16.1 Hz, 1H), 2.63 (d, *J*= 16.1 Hz, 1H), 2.42 (s, 3H), 2.00-2.07 (m, 1H), 1.79-1.85 (m, 1H), 1.60 (m, 1H), 1.36 (m, 1H), 1.17 (s, 9H). LCMS m/z $[M+H]^+$ = 546.1.

**Example i-78. Intermediate D-17 ((R)-N-[(1S)-1'-(7-bromo-6-methyl-pyrazolo[1,5-a]pyrazin-4-yl)-6-methoxy-spiro[indane-2,4'-piperidine]-1-yl]-2-methyl-propane-2-sulfinamide)**

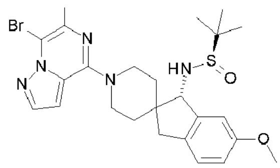

Step a: As described for **intermediate D-16** step a, a similar procedure was performed using **intermediate B-28** (1.98 g, 7.38 mmol) and **intermediate A-1** to give (1.98 g, 7.38 mmol) to give 1'-(7-bromo-6-methyl-pyrazolo[1,5-a]pyrazin-4-yl)-6-methoxy-spiro[indane-2,4'-piperidine]-1-one (2.82 g, 6.4 mmol). LCMS m/z $[M+H]^+$ = 441.1.

**Step b:** As described for **intermediate D-16** step b, a similar procedure was performed using 1'-(7-bromo-6-methyl-pyrazolo[1,5-a]pyrazin-4-yl)-6-methoxy-spiro[indane-2,4'-piperidine]-1-one (2.20 g, 4.99 mmol) (R)-2-methylpropane-2-sulfinamide (1.21 g, 9.97 mmol) in $Ti(OEt)_4$ (5.2 mL) to give (NZ,R)-N-[1'-(7-bromo-6-methyl-pyrazolo[1,5-a]pyrazin-4-yl)-6-methoxy-spiro[indane-2,4'-piperidine]-1-ylidene]-2-methyl-propane-2-sulfinamide (1.59 g, 2.9 mmol) as a yellow solid. $^1$H NMR (400 MHz, DMSO-*d6*) δ 8.04 (d, *J* = 2.4 Hz, 1H), 7.88 (m, 1H), 7.47 (d, *J* = 8.5 Hz, 1H), 7.25 (dd, *J* = 8.4 Hz, *J* = 2.5 Hz, 1H), 7.17 (d, *J* = 2.5 Hz, 1H), 4.44 (d, *J* = 13.6 Hz, 1H), 3.79 (s, 3H), 3.25 (m, 2H), 3.15 (s, 2H), 2.44 (s, 3H), 1.99 (m, 2H), 1.61 (m, 2H), 1.18 (s, 11H), 0.86 (m, 1H). LCMS m/z $[M+H]^+$ = 544.1.

**Step c:** As described for **intermediate D-16** step c, a similar procedure was performed using (NZ,R)-N-[1'-(7-bromo-6-methyl-pyrazolo[1,5-a]pyrazin-4-yl)-6-methoxy-spiro[indane-2,4'-piperidine]-1-ylidene]-2-methylpropane-2-sulfinamide (1.43g, 2.64 mmol) and DIBAL-H (1 M in toluene, 7.9 mL, 7.9 mmol) to give (R)-N-[(1S)-1'-(7-bromo-6-methyl-pyrazolo[1,5-a]pyrazin-4-yl)-6-methoxy-spiro[indane-2,4'-piperidine]-1-yl]-2-methyl-propane-2-sulfinamide (1.40 g, 2.56 mmol) as an white solid. $^1$H NMR (400 MHz, DMSO-*d6*) δ 8.02 (d, *J* = 2.4 Hz, 1H), 7.10-7.15 (m, 2H), 6.80 (m, 2H), 5.62 *(d, J*= 10.3 Hz, 1H), 4.35-4.41 (m, 3H), 3.70 (s, 3H), 3.25 (m, 2H), 3.08 *(d, J*= 15.6 Hz, 1H), 2.67 (m, 1H), 2.42 (s, 3H), 2.06-2.12 (m, 1H), 1.75-1.82 (m, 1H), 1.62-1.65 (m, 1H), 1.34 (m, 1H), 1.19 (m, 10H). LCMS m/z $[M+H]^+$ = 546.2.

**Example i-79. Intermediate D-18 ((R)-N-[(5S)-1'-(7-bromo-6-methyl-pyrazolo[1,5-a]pyrazin-4-yl)-3-methyl-spiro[5,7-dihydrocyclopenta[c]pyridine-6,4'-piperidine]-5-yl]-2-methyl-propane-2-sulfinamide)**

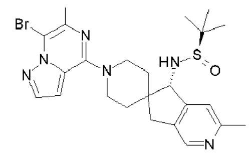

As described for **intermediate D-13,** a similar procedure was performed using **intermediate B-24** (704 mg, 2.19 mmol) and **intermediate A-1** (500 mg, 2.03 mmol) to give (R)-N-[(5S)-1'-(7-bromo-6-methyl-pyrazolo[1,5-a]pyrazin-4-yl)-3-methyl-spiro[5,7-dihydrocyclopenta[c]pyridine-6,4'-piperidine]-5-yl]-2-methyl-propane-2-sulfinamide (929 mg, 1.75 mmol) as an off white powder. $^1$H NMR (400 MHz, DMSO-*d6*) δ 8.32 (s, 1H), 8.03 (d, *J* = 2.4 Hz, 1H), 7.12 (s, 1H), 5.73 (d, *J* = 10.4 Hz, 1H), 4.35-4.48 (m, 3H), 3.17-3.28 (m, 3H), 2.70 (m, 1H), 2.46 (s, 3H), 2.43 (s, 3H), 2.14 (m, 1H), 1.73-1.80 (m, 1H), 1.67 (m, 1H), 1.30 (m, 1H), 1.20 (s, 9H). LCMS, m/z $[M+H]^+$ = 531.1.

**Example i-80. Intermediate D-19 ((R)-N-[(7S)-1'-(7-bromo-6-methyl-pyrazolo[1,5-a]pyrazin-4-yl)spiro[5,7-dihydrocyclopenta[c]pyridine-6,4'-piperidine]-7-yl]-2-methyl-propane-2-sulfinamide)**

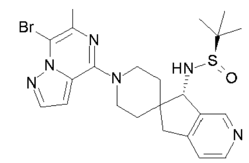

As described for **intermediate D-13**, a similar procedure was performed using **intermediate B-11** (660 mg, 2.08 mmol) and **intermediate A-1** (467 mg, 1.89 mmol) to give (R)-N-[(7S)-1'-(7-bromo-6-methyl-pyrazolo[1,5-a]pyrazin-4-yl)spiro[5,7-dihydrocyclopenta[c]pyridine-6,4'-piperidine]-7-y1]-2-methyl-propane-2-sulfinamide (827 mg, 1.58 mmol) as a light yellow solid. $^1$H NMR (400 MHz, DMSO-*d6*) δ 8.45 (s, 1H), 8.41 (d, *J* = 4.9 Hz, 1H), 8.02 (d, *J* = 2.4 Hz, 1H), 7.30 *(d, J* = 4.9 Hz, 1H), 7.11 *(d, J* = 2.5 Hz, 1H), 5.78 *(d, J* = 10.2 Hz, 1H), 4.56 *(d, J* = 10.2 Hz, 1H), 4.32-4.37 (m, 2H), 3.34 (m, 2H+$H_2O$), 3.16 (m, 1H), 2.80 (d, *J* = 16.7 Hz, 1H), 2.42 (s, 3 H), 2.01-2.08 (m, 1H), 1.79-1.86 (m, 1H), 1.62-1.65 (m, 1H), 1.34-1.38 (m, 1H), 1.19 (s, 9H). LCMS m/z $[M+H]^+$ = 517.0.

**Example i-81. Intermediate D-20 ((R)-N-[(5S)-1'-(7-bromo-6-methyl-pyrazolo[1,5-a]pyrazin-4-yl)-3-methoxy-spiro[5,7-dihydrocyclopenta[c]pyridine-6,4'-pipeiidine]-5-yl]-2-methyl-propane-2-sulfinamide)**

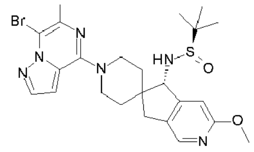

As described for **intermediate D-13,** a similar procedure was performed using **intermediate B-25** (380 mg, 1.13 mmol) and **intermediate A-1** (252 mg, 1.02 mmol) to give (R)-N-[(5S)-1'-(7-bromo-6-methyl-pyrazolo[1,5-a]pyrazin-4-yl)-3-methoxy-spiro[5,7-dihydrocyclopenta[c]pyridine-6,4'-piperidine]-5-yl]-2-methyl-propane-2-sulfinamide (510 mg, 0.93 mmol) as a white solid. $^1$H NMR (400 MHz, DMSO-*d6*) δ 8.02 (d, *J* = 10.1 Hz, 2H), 7.12 (s, 1H), 6.63 (s, 1H), 5.77 *(d, J*= 10.4 Hz, 1H), 4.34-4.46 (m, 3H), 3.82 (s, 3H), 3.14-3.27 (m, 3H), 2.66 (d, *J* = 15.5 Hz, 1H), 2.42 (s, 3 H), 2.10-2.17 (m, 1H), 1.66-1.76 (m, 2H), 1.22-1.25 (m, 1H), 1.18 (s, 9H). LCMS m/z [M+H]$^+$ = 547.1.

**Example i-82. Intermediate D-21 ((R)-N-[(7S)-1'-(7-bromo-6-methyl-pyrazolo[1,5-a]pyrazin-4-yl)-3-methoxy-spiro[5,7-dihydrocyclopenta[c]pyridine-6,4'-piperidine]-7-yl]-2-methyl-propane-2-sulfinamide)**

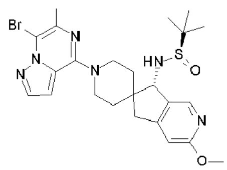

As described for **intermediate D-13**, a similar procedure was performed using **intermediate B-26** (248 mg, 0.63 mmol) and **intermediate A-1** (140 mg, 0.57 mmol) to give (R)-N-[(7S)-1'-(7-bromo-6-methyl-pyrazolo[1,5-a]pyrazin-4-yl)-3-methoxy-spiro[5,7-dihydrocyclopenta[c]pyridine-6,4'-piperidine]-7-yl]-2-methyl-propane-2-sulfinamide (263 mg, 0.48 mmol) as a white solid. $^1$H NMR (400 MHz, DMSO-*d6*) δ 8.02 (d, *J* = 2.5 Hz, 2H), 7.12 (d, *J* = 2.5 Hz, 2H), 6.68 (s, 1H), 5.65 (d, *J*= 10.0 Hz, 1H), 4.44 (d, *J* = 9.8 Hz, 1H), 4.30 (m, 2H), 3.83 (s, 3H), 3.08-3.12 (m, 1H), 2.89 (m, 1H), 2.66 (m, 2H), 2.30-2.42 m, 5 H), 1.99 (m, 2H), 1.79-1.86 (m, 1H), 1.58-61 (m, 1H), 1.37-1.40 (m, 1H), 1.17 (s, 10H). LCMS m/z [M+H]$^+$ = 547.1.

**Example i-83. Intermediate D-22 ((R)-N-[(3R)-1'-(7-bromo-6-methyl-pyrazolo[1,5-a]pyrazin-4-yl)spiro[3H-benzofuran-2,4'-piperidine]-3-yl]-2-methyl-propane-2-sulfinamide)**

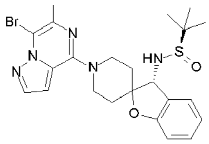

As described for **intermediate D-13,** a similar procedure was performed using **intermediate B-12** (1.13 g, 3.65 mmol) and **intermediate A-1** (820 mg, 3.33 mmol) to give (R)-N-[(3R)-1'-(7-bromo-6-methyl-pyrazolo[1,5-a]pyrazin-4-yl)spiro[3H-benzofuran-2,4'-piperidine]-3-yl]-2-methyl-propane-2-sulfinamide (1.46 g, 2.66 mmol) as a white solid. $^1$H NMR (400 MHz, DMSO-*d6*) δ 8.04 (d, *J* = 2.5 Hz, 2H), 7.17-7.29 (m, 3H), 6.85-6.94 (m, 2H), 6.10 (d, *J*= 10.4 Hz, 1H), 4.65 *(d, J*= 10.3 Hz, 1H), 4.40-4.49 (m, 2H), 3.37-3.48 (m, 2H), 2.44 (s, 3H), 2.66 *(d, J*= 15.5 Hz, 1H), 2.42 (s, 3 H), 1.84-2.10 (m, 4H), 1.16 (s, 9H). LCMS m/z [M+H]$^+$ = 518.1.

**Example i-84. Intermediate D-23 ((R)-N-[(3R)-1'-(7-bromo-6-methyl-pyrazolo[1,5-a]pyrazin-4-yl)spiro[3H-furo[2,3-b]pyridine-2,4'-piperidine]-3-yl]-2-methyl-propane-2-sulfinamide)**

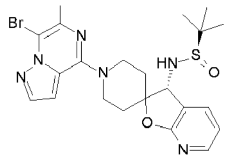

As described for **intermediate D-13,** a similar procedure was performed using **intermediate B-13** (1.22 g, 3.94 mmol) and **intermediate A-1** (884 mg, 3.59 mmol) to give (R)-N-[(3R)-1'-(7-bromo-6-methyl-pyrazolo[1,5-a]pyrazin-4-yl)spiro[3H-furo[2,3-b]pyridine-2,4'-piperidine]-3-yl]-2-methyl-propane-2-sulfinamide (1.59 g, 2.75 mmol) as a yellow solid. $^1$H NMK (400 MHz, DMSO-*d6*) δ 8.05-8.10 (m, 2H), 7.67-7.70 (m, 1H), 7.19 (d, *J* = 2.5 Hz, 1H), 6.96-6.99 (m, 1H), 4.71 (d, *J* = 10.3 Hz, 1H), 4.41-4.51 (m, 2H), 3.38-3.49 (m, 2H), 2.44 (s, 3H), 2.66 (d, *J* = 15.5 Hz, 1H), 2.42 (s, 3 H), 1.87-2.11 (m, 4H), 1.16 (s, 9H). LCMS m/z $[M+H]^+$ = 519.2.

**Example i-85. Intermediate D-24 (tert-butyl N-[(3R)-1'-(7-bromo-6-methyl-pyrazolo[1,5-a]pyrazin-4-yl)spiro[indoline-2,4'-piperidine]-3-yl]carbamate)**

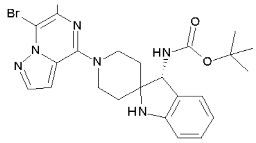

As described for **intermediate D-12,** a similar procedure was performed using **intermediate B-29** (419 mg, 2.06 mmol) and **intermediate A-1** (484 mg, 1.98 mmol) to give (3R)-1'-(7-bromo-6-methyl-pyrazolo[1,5-a]pyrazin-4-yl)spiro[indoline-2,4'-piperidine]-3-amine hydrochloride (691 mg, 1.67 mmol) that was then treated with $Boc_2O$ (438 mg, 2.0 mmol) and triethylamine (0.46 mL, 3.34 mmol) in THF (20 mL) to give after chromatography ($SiO_2$, heptane: Ethyl acetate= 7:3) tert-butyl N-[(3R)-1'-(7-bromo-6-methyl-pyrazolo[1,5-a]pyrazin-4-yl)spiro[indoline-2,4'-piperidine]-3-yl]carbamate (811 mg, 1.58 mmol) as a white foam. LCMS m/z $[M+H]^+$ = 513.2.

**Example i-86. Intermediate D-25 (tert-butyl N-[(3R)-1'-(7-bromo-6-methyl-pyrazolo[1,5-a]pyrazin-4-yl)-1**-methyl-spiro[indoline-2,4'-piperidine]-3-yl]carbamate)

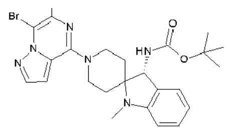

As described for **intermediate D-12**, a similar procedure was performed using **intermediate B-30** (780 mg, 2.69 mmol) and **intermediate A-1** (602 mg, 2.44 mmol) to give (3R)-1'-(7-bromo-6-methyl-pyrazolo[1,5-a]pyrazin-4-yl)-1-methyl-spiro[indoline-2,4'-piperidine]-3-amine (769 mg, 1.8 mmol) that was then treated with $Boc_2O$ (471 mg, 2.16 mmol) and triethylamine (0.5 mL, 3.60 mmol) in DCM (10 mL) to give after chromatography ($SiO_2$, heptane: ethyl acetate= 8:2) tert-butyl N-[(3R)-1'-(7-bromo-6-methyl-pyrazolo[1,5-a]pyrazin-4-yl)-1-methyl-spiro[indoline-2,4'-piperidine]-3-yl]carbamate (703 mg, 1.33 mmol) as a pink foam. LCMS m/z $[M+H]^+$ = 527.2.

**Example i-87. Intermediate D-26 (N-[(SR)-1'-(7-bromo-6-methyl-pyrazolo[1,5-a]pyrazin-4-yl)-3-(difluoromethyl)spiro[5,7-dihydrocyclopenta[b]pyridine-6,4'-piperidine]-5-yl]-2-methyl-propane-2-sulfinamide)**

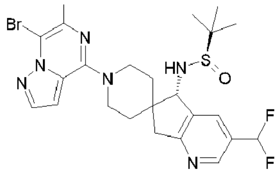

As described for **intermediate D-13**, a similar procedure was performed using **intermediate B-32** (220 mg, crude) and **intermediate A-2** (210 mg, 0.67 mmol) to give N-[(5R)-1'-(7-bromo-6-methyl-pyrazolo[1,5-a]pyrazin-4-yl)-3-(difluoromethyl)spiro[5,7-dihydrocyclopenta[b]pyridine-6,4'-piperidine]-5-yl]-2-methyl-propane-2-sulfinamide (148 mg, 0.26 mmol) as a pink foam. LCMS m/z $[M+H]^+$ = 567.2.

**Example i-88. Intermediate D-27 (N-[(5S)-1'-(7-bromo-6-methyl-pyrazolo[1,5-a]pyrazin-4-yl)-2-(difluoromethyl)spiro[5,7-dihydrocyclopenta[b]pyridine-6,4'-piperidine]-5-yl]-2-methyl-propane-2-sulfinamide)**

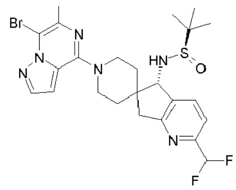

As described for **intermediate D-13,** a similar procedure was performed using **intermediate B-35** (360 mg, crude) and **intermediate A-2** (470 mg, 1.5 mmol) to give, after purification by column chromatography ($SiO_2$, cyclohexane / ethyl acetate = 1/0 to 2/8), N-[(5S)-1'-(7-bromo-6-methyl-pyrazolo[1,5-a]pyrazin-4-yl)-2-(difluoromethyl)spiro[5,7-dihydrocyclopenta[b]pyridine-6,4'-piperidine]-5-yl]-2-methyl-propane-2-sulfinamide (321 mg, 0.57 mmol) as a pink foam. LCMS m/z $[M+H]^+$ = 567.2.

**Example i-89. Intermediate D-28 ((R)-N-[(5S)-1'-(7-bromo-6-methyl-pyrazolo[1,5-a]pyrazin-4-yl)-1-(hydroxymethyl)spiro[5,7-dihydrocyclopenta[c]pyridine-6,4'-piperidine]-5-yl]-2-methyl-propane-2-sulfinamide)**

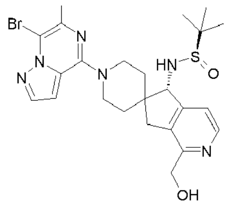

**Step a:** As described for **intermediate D-13**, a similar procedure was performed using **intermediate B-22** (735 mg, 1.89 mmol) and **intermediate A-1** (515 mg, 2.09 mmol) to give 1'-(7-bromo-6-methyl-pyrazolo[1,5-a]pyrazin-4-yl)-1-(triisopropylsilyloxymethyl)spiro[7H-cyclopenta[c]pyridine-6,4'-piperidine]-5-one (841 mg, 1.40 mmol) as a pink solid. LCMS m/z $[M+H]^+$ = 598.3.

**Step b:** As described for **intermediate D-1** step b, a similar procedure was performed using 1'-(7-bromo-6-methyl-pyrazolo[1,5-a]pyrazin-4-yl)-1-(triisopropylsilyloxymethyl)spiro[7H-cyclopenta[c]pyridine-6,4'-piperidine]-5-one (835 mg, 1.39 mmol), (R)-2-methylpropane-2-sulfinamide (375 mg, 3.09 mmol) and $Ti(OEt)_4$ (1.5 mL, 7.2 mmol) in MeTHF (10 mL) to give (NZ,R)-N-[1'-(7-bromo-6-methyl-pyrazolo[1,5-a]pyrazin-4-yl)-1-(triisopropylsilyloxymethyl)spiro[7H-cyclopenta[c]pyridine-6,4'-piperidine]-5-ylidene]-2-methyl-propane-2-sulfinamide (834 mg, 1.19 mmol) as a yellow solid. LCMS m/z $[M+H]^+$ = 701.3.

**Step c:** As described for **intermediate D-1** step c, a similar procedure was performed using (NZ,R)-N-[1'-(7-bromo-6-methyl-pyrazolo[1,5-a]pyrazin-4-yl)-1-(triisopropylsilyloxymethyl)spiro[7H-cyclopenta[c]pyridine-6,4'-piperidine]-5-ylidene]-2-methyl-propane-2-sulfinamide (830 mg, 1.18 mmol) and DIBAL-H (1 M in toluene, 2.4 mL, 2.4 mmol) to give (R)-N-[(5S)-1'-(7-bromo-6-methyl-pyrazolo[1,5-a]pyrazin-4-yl)-1-(hydroxymethyl)spiro[5,7-dihydrocyclopenta[c]pyridine-6,4'-piperidine]-5-yl]-2-methyl-propane-2-sulfinamide (313 mg, 0.57 mmol) as a pink solid. LCMS m/z

$[M+H]^+$ = 547.2.

**Example i-90. Intermediate D-29 (N-[(7S)-1'-(7-bromo-6-methyl-pyrazolo[1,5-a]pyrazin-4-yl)-3-(hydroxymethyl)spiro[5,7-dihydrocyclopenta[c]pyridine-6,4'-piperidine]-7-yl]-2-methyl-propane-2-sulfinamide)**

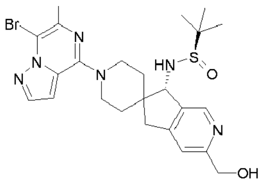

As described for **intermediate D-13**, a similar procedure was performed using **intermediate B-23** (430 mg, crude) and **intermediate A-2** (610 mg, 2.1 mmol) to give (N-[(7S)-1'-(7-bromo-6-methyl-pyrazolo[1,5-a]pyrazin-4-yl)-3-(hydroxymethyl)spiro[5,7-dihydrocyclopenta[c]pyridine-6,4'-piperidine]-7-yl]-2-methyl-propane-2-sulfinamide (105 mg, 0.2 mmol) as an off-white solid. LCMS m/z $[M+H]^+$ = 547.3.

**Example i-91. Intermediate D-30 ((5S)-1'-(7-bromo-6-methyl-pyrazolo[1,5-a]pyrazin-4-yl)spiro[5,7-dihydrocyclopenta[b]pyrazine-6,4'-piperidine]-5-amine)**

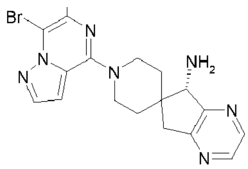

As described for **intermediate D-13,** a similar procedure was performed using **intermediate B-37** (476 mg, crude) and **intermediate A-2** (470 mg, 1.5 mmol) to give, after purification by column chromatography ($SiO_2$, dichloromethane / methanol = 1/0 to 9/1), (5S)-1'-(7-bromo-6-methyl-pyrazolo[1,5-a]pyrazin-4-yl)spiro[5,7-dihydrocyclopenta[b]pyrazine-6,4'-piperidine]-5-amine (46 mg, 0.11 mmol) as a brown semi solid. LCMS m/z $[M+H]^+$ = 414.2.

**Example i-92. Intermediate D-31 (1'-(7-Bromo-6-methyl-pyrazolo[1,5-a]pyrazin-4-yl)-5-fluoro-spiro[indane-2,4'-piperidine]-1-one)**

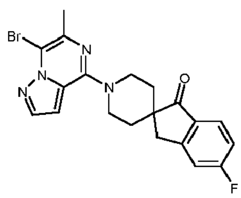

A mixture of 5-fluorospiro[indane-2,4'-piperidine]-1-one hydrochloride (702 mg, 2.744 mmol), **intermediate A-1** (615 mg, 2.49 mmol), potassium carbonate (1.03 g, 7.49 mmol) in dimethylformamide (20 mL) was stirred at 90 °C for 20 h and cooled down to RT. The solid was filtered and the filtrate concentrated under reduced pressure. The residue was taken up with ethyl acetate (100 mL) and water (50 mL). The organic layer was separated, washed with brine (50 mL), dried over $MgSO_4$ and concentrated under reduced pressure. The residue was purified by column chromatography ($SiO_2$, Heptane/Ethyl acetate= 100/0 to 77/23) to give 1'-(7-bromo-6-methyl-pyrazolo[1,5-a]pyrazin-4-yl)-5-fluoro-spiro[indane-2,4'-piperidine]-1-one (860 mg, 2 mmol) as a beige solid. LCMS m/z $[M+H]^+$ = 429.

**Example i-93. Intermediate D-32 ((R)-N-[(1S)-1'-(7-Bromo-6-methyl-pyrazolo[1,5-a]pyrazin-4-yl)-5-fluoro-spiro[indane-2,4'-piperidine]-1-yl]-2-methyl-propane-2-sulfinamide)**

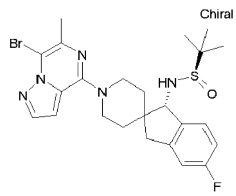

**Step a:** A mixture of **intermediate D-31** (860 mg, 2 mmol), (R)-2-methylpropane-2-sulfinamide (486 mg, 4 mmol) and tetraethoxytitanium (1.83 g, 8.01 mmol) was stirred at 100 °C for 15 h. The mixture was diluted with dichloromethane (500 mL) and water (550 mL) and stirred 3 h. Salts were removed by filtration and washed by dichloromethane (50 mL). The organic layer was separated from the filtrate and then washed with brine (50 mL), dried over $MgSO_4$ and concentrated under reduced pressure. The residue was purified by column chromatography ($SiO_2$, Heptane/Ethyl acetate= 80/20 to 45/55) to give (NZ,R)-N-[1'-(7-bromo-6-methyl-pyrazolo[1,5-a]pyrazin-4-yl)-5-fluoro-spiro[indane-2,4'-piperidine]-1-ylidene]-2-methyl-propane-2-sulfinamide (845 mg, 1.53 mmol) as a light green solid. LCMS m/z $[M+H]^+$ = 532.

**Step b:** To a solution of (NZ,R)-N-[1'-(7-bromo-6-methyl-pyrazolo[1,5-a]pyrazin-4-yl)-5-fluoro-spiro[indane-2,4'-piperidine]-1-ylidene]-2-methyl-propane-2-sulfinamide (840 mg, 1.56 mmol) in dry THF (22 mL) was added dropwise DIBAL-H (1 M in toluene, 7.57 mL, 7.57 mmol) at -65 °C under Ar. The mixture was stirred at -65 °C for 1 h and then a saturated aqueous solution of Rochelle salts (80 mL) diluted with water (20 mL) was added. The mixture was then stirred for 40 min at RT, and then extracted with ethyl acetate (200 mL). The combined organic phase was washed with brine, dried with anhydrous $MgSO_4$, filtered and concentrated under reduced pressure. The residue was purified by column chromatography ($SiO_2$, Heptane/Ethyl acetate= 100/0 to 50/50) to give (R)-N-[(1S)-1'-(7-bromo-6-methyl-pyrazolo[1,5-a]pyrazin-4-yl)-5-fluoro-spiro[indane-2,4'-piperidine]-1-yl]-2-methyl-propane-2-sulfinamide as a white solid (501 mg, 0.935 mmol) as a colorless oil. LCMS m/z $[M+H]^+$ = 534.

**Example i-94. Intermediate D-33 (tert-butyl N-[(1S)-1'-(7-bromo-6-methyl-pyrazolo[1,5-a]pyrazin-4-yl)-6-fluoro-spiro[indane-2,4'-piperidine]-1-yl]carbamate)**

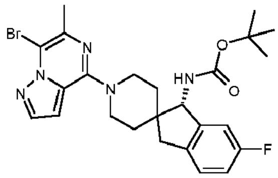

A mixture of **intermediate B-38** (897 mg, 2.72 mmol), **intermediate A-1** (704 mg, 2.86 mmol), potassium carbonate (2.26 g, 16.3 mmol) in dimethylacetamide (10 mL) was stirred at 90 °C for 20 h and cooled down to RT. The solid was filtered and rinsed with dimethylacetamide (5 mL). To the filtrate under stirring was added triethylamine (1.1 mL, 8.1 mmol) and di-tert-butyl pyrocarbonate (1.2 g, 5.4 mmol). After 15 h at RT, a saturated solution of sodium carbonate (200 mL) and ethyl acetate (150 mL) were added to the reaction medium. Insoluble material was removed by filtration and the filtrate was decanted. The organic layer was washed with water (25 mL), brine (40 mL), dried over $MgSO_4$ and concentrated under reduced pressure. The residue was purified by column chromatography ($SiO_2$, Heptane/Ethyl acetate= 100/0 to 75/25) to give tert-butyl N-[(1S)-1'-(7-bromo-6-methyl-pyrazolo[1,5-a]pyrazin-4-yl)-6-fluoro-spiro[indane-2,4'-piperidine]-1-yl]carbamate (1.14 g, 1.59 mmol) as a white solid. LCMS m/z $[M+H]^+$ = 530.

**Example i-95. Intermediate D-34 (tert-butyl N-[(1S)-1'-(7-bromo-6-methyl-pyrazolo[1,5-a]pyrazin-4-yl)-4-fluoro-spiro[indane-2,4'-piperidine]-1-yl]carbamate)**

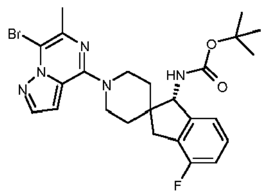

A mixture of **intermediate B-39** (980 mg, 3 mmol), **intermediate A-1** (770 mg, 3.1 mmol), potassium carbonate (2.5 g, 18 mmol) in dimethylacetamide (20 mL) was stirred at 90 °C for 20 h and cooled down to RT. The solid was filtered and rinsed with dimethylacetamide (5 mL). To the filtrate under stirring was added triethylamine (1.3 mL, 9.1 mmol) and di-tert-butyl pyrocarbonate (690 mg, 3.2 mmol). After 15 h at RT, a saturated solution of sodium carbonate (200 mL) and ethyl acetate (150 mL) were added to the reaction medium. Insoluble material was removed by filtration and the filtrate was decanted. The organic layer was washed with water (7 x 40 mL), brine (40 mL), dried over $MgSO_4$ and concentrated under reduced pressure. The residue was purified by column chromatography ($SiO_2$, Heptane/Ethyl acetate= 100/0 to 75/25) to give tert-butyl N-[(1S)-1'-(7-bromo-6-methyl-pyrazolo[1,5-a]pyrazin-4-yl)-4-fluoro-spiro[indane-2,4'-piperidine]-1-yl]carbamate (845 mg, 1.59 mmol) as a white solid. LCMS m/z $[M+H]^+$ = 530.

**Example i-96. Intermediate D-35 (tert-butyl N-[(1S)-1'-(7-bromo-6-methyl-pyrazolo[1,5-a]pyrazin-4-yl)-5,6-difluoro-spiro[indane-2,4'-piperidine]-1-yl]carbamate)**

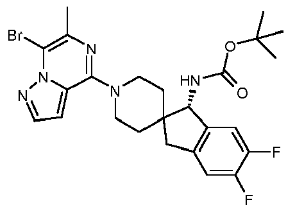

A mixture of **intermediate B-40** (85 mg, 0.244 mmol) **intermediate A-1** (56 mg, 0.227 mmol), diethyl isopropyl amine (0.119 mL, 0.682 mmol) in dimethylformamide (5 mL) was stirred at 90 °C for 18 h and cooled down to RT. To the solution under stirring was added di-tert-butylpyrocarbonate (198 mg, 0.908 mmol). After 72 h at RT, the reaction medium was concentrated under reduced pressure. The residue was dissolved in ethyl acetate (100 mL) and a saturated solution of ammonium chloride (100 mL) was added. The organic layer was decanted and the aqueous phase was extracted with ethyl acetate (2 * 100 mL). The organic layers were combined, washed with brine, dried over $MgSO_4$ and concentrated under reduced pressure. The residue was purified by column chromatography ($SiO_2$, Heptane/Ethyl acetate= 100/0 to 70/30) to yield tert-butyl N-[(1S)-1'-(7-bromo-6-methyl-pyrazolo[1,5-a]pyrazin-4-yl)-5,6-difluoro-spiro[indane-2,4'-piperidine]-1-yl]carbamate (76 mg, 0.139 mmol) as a white solid. LCMS m/z $[M+H]^+$ = 548.

**Example i-97. Intermediate D-36 (tert-butyl N-[(1S)-1'-(7-bromo-6-methyl-pyrazolo[1,5-a]pyrazin-4-yl)-4,6-difluoro-spiro[indane-2,4'-piperidine]-1-yl]carbamate)**

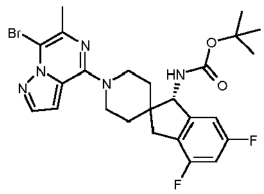

A mixture of **intermediate B-41** (400 mg, 1.15 mmol) **intermediate A-1** (298 mg, 1.21 mmol), potassium carbonate (954 mg, 6.9 mmol) in dimethylacetamide (10 mL) was stirred at 90 °C for 20 h and cooled down to RT. The solid was filtered and rinsed with dimethylacetamide (5 mL). To the filtrate under stirring was added triethylamine (0.481 mL, 3.45 mmol) and di-tert-butyl pyrocarbonate (502 mg, 2.3 mmol). After 20 h at RT, a saturated solution of sodium

carbonate (200 mL) and ethyl acetate (150 mL) were added to the reaction medium. The filtrate was decanted and the organic layer was washed with water (25 mL), brine (40 mL), dried over $MgSO_4$ and concentrated under reduced pressure. The residue was purified by column chromatography ($SiO_2$, Heptane/Ethyl acetate= 100/0 to 75/25) to give tert-butyl N-[(1S)-1'-(7-bromo-6-methyl-pyrazolo[1,5-a]pyrazin-4-yl)-4,6-difluoro-spiro[indane-2,4'-piperidine]-1-yl]carbamate (510 mg, 0.81 mmol) as a white solid. LCMS m/z $[M+H]^+$ = 548.

**Example i-98. Intermediate D-37 (tert-butyl N-[(4S)-1'-(7-bromo-6-methyl-pyrazolo[1,5-a]pyrazin-4-yl)-2-chloro-**spiro[4,6-dihydrocyclopenta[d]thiazole-5,4'-piperidine]-4-yl]carbamate)

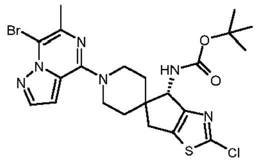

A mixture of **intermediate B-16** (1.04 g), **intermediate A-1** (1.1 g, 4.48 mmol), potassium carbonate (2.36 g, 17.1 mmol) in dimethylformamide (10 mL) was stirred at 80 °C for 15 h and cooled down to RT. After removal of the solid material by filtration, the filtrate was concentrated under reduced pressure. The residue was taken up in dichloromethane (40 mL), and to the resulting solution under stirring at RT, were added triethylamine (1.19 mL, 8.54 mmol) and di-tert-butyl pyrocarbonate (1.03 g, 4.7 mmol). After 15 h at RT, a saturated solution of sodium carbonate (50 mL) was added to the reaction medium and the mixture was decanted. The organic layer was washed with brine (50 mL), dried over $MgSO_4$ and concentrated under reduced pressure. The residue was purified by column chromatography ($SiO_2$, Heptane/Ethyl acetate= 100/0 to 70/30) to give tert-butyl N-[(4S)-1'-(7 bromo-6-methyl-pyrazolo[1,5-a]pyrazin-4-yl)-2-chloro-spiro[4,6-dihydrocyclopenta[d]thiazole-5,4'-piperidine]-4-yl]carbamate (854 mg, 1.54 mmol) as a white solid. LCMS m/z $[M+H]^+$ = 553.

**Example i-99. Intermediate D-38 (tert-butyl N-[(4S)-1'-(7-bromo-6-methyl-pyrazolo[1,5-a]pyrazin-4-yl)-2-methyl-spiro[4,6-dihydrocyclopenta[d]thiazole-5,4'-piperidine]-4-yl]carbamate)**

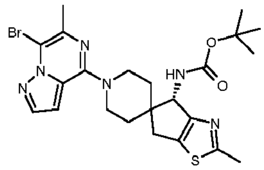

To a mixture of **intermediate D-37** (400 mg, 0.722 mmol), trimethylboroxine (0.151 mL, 1.083 mmol) and $K_2CO_3$ (200 mg, 1.444 mmol) in dioxane (5 mL) was added $Pd(dppf)Cl_2.CH_2Cl_2$ (118 mg, 0.144 mmol). The mixture was degassed with Ar and then stirred at 95 °C for 2 h. The suspension was filtered and the filtrate was concentrated under vacuum. The residue was purified by column chromatography ($SiO_2$, Heptane/Ethyl acetate = 100/0 to 50/50) to give tert-butyl N-[(4S)-1'-(7-bromo-6-methyl-pyrazolo[1,5-a]pyrazin-4-yl)-2-methyl-spiro[4,6-dihydrocyclopenta[d]thiazole-5,4'-piperidine]-4-yl]carbamate (285 mg, 0.534 mmol) as a beige solid.

**Example i-100. Intermediate D-39 (tert-butyl N-[(4S)-1'-(7-bromo-6-methyl-pyrazolo[1,5-a]pyrazin-4-yl)spiro[4,6-dihydrocyclopenta[d]thiazole-5,4'-piperidine]-4-yl]carbamate)**

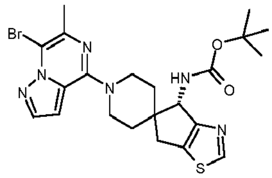

A mixture of **intermediate B-15** (1.21 g), **intermediate A-1** (1.11 g, 4.5 mmol), potassium carbonate (3.56 g, 25.7 mmol) in dimethylformamide (30 mL) was stirred at 80 °C for 16 h. After addition of more potassium carbonate (2.0 g, 14.44 mmol) to the reaction medium, the reaction was pursued at the same temperature for 6 h and then 15 h at RT. After removal of the solid material by filtration, the filtrate was concentrated under reduced pressure. The residue was taken up in dichloromethane (70 mL) and to the resulting solution, under stirring at RT, were added triethylamine (1.7 mL, 12.16 mmol) and di-tert-butyl pyrocarbonate (983 mg, 4.46 mmol) in dichloromethane (15 mL). After 15 h at RT, a saturated solution of sodium carbonate (150 mL) was added to the reaction medium and the mixture decanted. The organic layer was washed with brine (100 mL), dried over $MgSO_4$ and concentrated under reduced pressure. The residue was purified by column chromatography ($SiO_2$, Heptane/Ethyl acetate= 50/50 to 0/100) to give tert-butyl N-[(4S)-1'-(7-bromo-6-methyl-pyrazolo[1,5-a]pyrazin-4-yl)spiro[4,6-dihydrocyclopenta[d]thiazole-5,4'-piperidine]-4-yl]carbamate (770 mg, 1.48 mmol) as a white solid. LCMS m/z $[M+H]^+$ = 519.

**Example i-101. Intermediate D-40 (tert-butyl N-[(6S)-1'-(7-bromo-6-methyl-pyrazolo[1,5-a]pyrazin-4-yl)spiro[4,6-dihydrocyclopenta[d]thiazole-5,4'-piperidine]-6-yl]carbamate)**

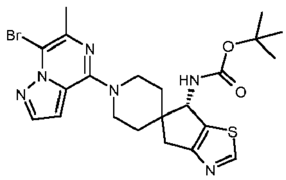

A mixture of **intermediate B-19** (1.2 g), **intermediate A-1** (1.2 g, 4.9 mmol), potassium carbonate (3.6 g, 26.0 mmol) in dimethylformamide (50 mL) was stirred at 80 °C for 3 h and 15 h at RT. After removal of the solid material by filtration, the filtrate was concentrated under reduced pressure. The residue was taken up in dichloromethane (85 mL), and to the resulting solution under stirring at RT, were added di-tert-butyl dicarbonate (1.09 g, 4.96 mmol) and triethylamine (2.13 mL, 15.26 mmol). After 4 h, more triethylamine (1 mL, 7.16 mmol) was added and the mixture was stirred at RT for 48 h. A saturated solution of sodium carbonate (150 mL) was added to the reaction medium and the mixture decanted. The organic layer was washed with brine (100 mL), dried over $MgSO_4$ and concentrated under reduced pressure. The reaction medium was concentrated under reduced pressure. The residue was purified by column chromatography ($SiO_2$, Heptane/Ethyl acetate= 80/20 to 40/60) to give tert-butyl N-[(6S)-1'-(7-bromo-6-methyl-pyrazolo[1,5-a]pyrazin-4-yl)spiro[4,6-dihydrocyclopenta[d]thiazole-5,4'-piperidine]-6-yl]carbamate (1.96 g, 3.77 mmol) as a white solid. LCMS m/z $[M+H]^+$ = 519.

**Example i-102. Intermediate D-41 (tert-butyl N-[(6S)-1'-(7-bromo-6-methyl-pyrazolo[1,5-a]pyrazin-4-yl)-2-chloro-spiro[4,6-dihydrocyclopenta[d]thiazole-5,4'-piperidine]-6-yl]carbamate)**

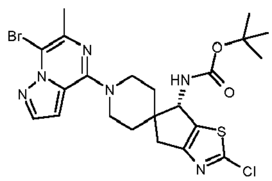

A mixture of **intermediate B-18** (813 mg), **intermediate A-1** (565 mg, 2.29 mmol), diethyl isopropyl amine (2.4 mL, 13.75 mmol) in dimethylformamide (15 mL) was stirred at 90 °C for 18 h. After addition of more **intermediate A-1** (280 mg, 1.146 mmol), the reaction was pursued for 3 h at the same temperature. The mixture was then left cooled

down to RT. To the solution under stirring was added di-tert-butyl pyrocarbonate (2.0 g, 9.165 mmol). After 15 h at RT, the reaction medium was concentrated under reduced pressure. The residue was dissolved in ethyl acetate (100 mL) and a saturated solution of ammonium chloride (100 mL). The organic layer was decanted and the aqueous phase was extracted with ethyl acetate (2 * 100 mL). The organic layers were combined, washed with brine, dried over $MgSO_4$ and concentrated under reduced pressure. The residue was purified by column chromatography ($SiO_2$, Heptane/Ethyl acetate= 100/0 to 85/15) to yield tert-butyl N-[(6S)-1'-(7-bromo-6-methyl-pyrazolo[1,5-a]pyrazin-4-yl)-2-chloro-spiro[4,6-dihydrocyclopenta[d]thiazole-5,4'-piperidine]-6-yl]carbamate (637 mg, 1.15 mmol) as a yellow solid. LCMS m/z $[M+H]^+$ = 555.

**Example i-103. Intermediate D-42 (tert-butyl N-[(6S)-1'-(7-bromo-6-methyl-pyrazolo[1,5-a]pyrazin-4-yl)-2-methyl-spiro[4,6-dihydrocyclopenta[d]thiazole-5,4'-piperidine]-6-yl]carbamate)**

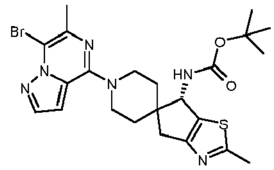

A mixture of **intermediate B-20 (**763 mg), **intermediate A-1** (565 mg, 2.292 mmol), diethyl isopropyl amine (1.2 mL, 6.877 mmol) in dimethylformamide (15 mL) was stirred at 90 °C for 18 h. After addition of more **intermediate A-1** (280 mg, 1.146 mmol), the reaction was pursued for 3 h at the same temperature. The mixture was then left cooled down to RT. To the solution under stirring was added di-tert-butyl dicarbonate (2.0 g, 9.165 mmol). After 15 h at RT, the reaction medium was concentrated under reduced pressure. The residue was dissolved in ethyl acetate (100 mL) and a saturated solution of ammonium chloride (100 mL). The organic layer was decanted and the aqueous phase was extracted with ethyl acetate (2 * 100 mL). The organic layers were combined, washed with brine, dried over $MgSO_4$ and concentrated under reduced pressure. The residue was purified by column chromatography ($SiO_2$, Heptane/Ethyl acetate= 100/0 to 65/35) to yield tert-butyl N-[(6S)-1'-(7-bromo-6-methyl-pyrazolo[1,5-a]pyrazin-4-yl)-2-methyl-spiro[4,6-dihydrocyclopenta[d]thiazole-5,4'-piperidine]-6-yl]carbamate (343 mg, 0.64 mmol) as a beige solid. LCMS m/z $[M+H]^+$ = 535.

**Example i-104. Intermediate D-43 (tert-butyl N-[(6S)-1'-(7-bromo-6-methyl-pyrazolo[1,5-a]pyrazin-4-yl)-2-methoxy-spiro[4,6-dihydrocyclopenta[d]thiazole-5,4'-piperidine]-6-yl]carbamate)**

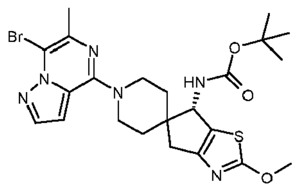

To a solution of **intermediate D-41** (290 mg) in methanol (10 mL) was added sodium methylate (113 mg). The mixture was stirred at 90 °C for 22 h, more sodium methylate (113 mg) was added and the reaction was pursued for 6 days at the same temperature. The reaction medium was poured into a saturated solution of potassium dihydrogenophosphate and the mixture was extracted by ethyl acetate (x 3). The organic layers were combined, washed with brine, dried over $MgSO_4$ and concentrated under reduced pressure. The residue was purified by column chromatography ($SiO_2$, Heptane/Ethyl acetate= 100/0 to 70/30) to give tert-butyl N-[(6S)-1'-(7-bromo-6-methyl-pyrazolo[1,5-a]pyrazin-4-yl)-2-methoxy-spiro[4,6-dihydrocyclopenta[d]thiazole-5,4'-piperidine]-6-yl]carbamate (200 mg, 0.364 mmol) as a white solid. LCMS m/z $[M+H]^+$ = 549.

**Example i-105. Intermediate D-44 (tert-butyl N-[(5S)-1'-(7-bromo-6-methyl-pyrazolo[1,5-a]pyrazin-4-yl)-2-methyl-spiro[5,7-dihydrocyclopenta[b]pyridine-6,4'-piperidine]-5-yl]carbamate)**

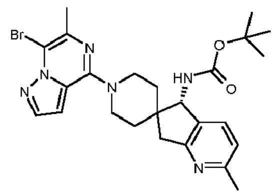

A mixture of **intermediate B-21** (774 mg, 2.37 mmol), **intermediate A-1** (613 mg, 2.49 mmol), potassium carbonate (3.3 g, 17 mmol) in dimethylformamide (32 mL) was stirred at 80 °C for 20 h and left to cool to RT. After removal of the solid material by filtration, the filtrate was concentrated under reduced pressure. The residue was taken up in dichloromethane (100 mL) and to the resulting solution under stirring at RT were added di-tert-butyl dicarbonate (1.31 g, 5.93 mmol) and triethylamine (2.64 mL, 19 mmol). After 15 h at RT, a saturated solution of sodium carbonate (150 mL) was added to the reaction medium and the was mixture decanted. The organic layer was washed with brine (100 mL), dried over $MgSO_4$ and concentrated under reduced pressure. The reaction medium was concentrated under reduced pressure. The residue was purified by column chromatography ($SiO_2$, Heptane/Ethyl acetate= 80/20 to 90/10) to give tert butyl N-[(5S)-1'-(7-bromo-6-methyl-pyrazolo[1,5-a]pyrazin-4-yl)-2-methyl-spiro[5,7-dihydrocyclopenta[b]pyridine-6,4'-piperidine]-5-yl]carbamate (0.65 g, 1.24 mmol) as a white foam. LCMS m/z $[M+H]^+$ = 527.

**Example i-106. Intermediate D-45 ((R)-N-[(5S)-1'-[7-bromo-2-(hydroxymethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide)**

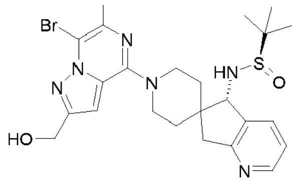

Step a: As described for **intermediate D-1**, a similar procedure was performed using **intermediate B-1** (655 mg, 2.74 mmol) and **intermediate A-4** (680 mg, 2.13 mmol) to give ethyl 7-bromo-6-methyl-4-(5-oxospiro[7H-cyclopenta[b]pyridine-6,4'-piperidine]-1'-yl)pyrazolo[1,5-a]pyrazine-2-carboxylate (803 mg, 1.66 mmol) as an orange solid. LCMS m/z $[M+H]^+$ = 484.2.

**Step b:** As described for **intermediate D-1-** step b, a similar procedure was performed using ethyl 7-bromo-6-methyl-4-(5-oxospiro[7H-cyclopenta[b]pyridine-6,4'-piperidine]-1'-yl)pyrazolo[1,5-a]pyrazine-2-carboxylate (760 mg, 1.57 mmol), (R)-2-methylpropane-2-sulfinamide (800 mg, 6.60 mmol) in $Ti(OEt)_4$ (3.5 mL) to give ethyl 7-bromo-4-[(5Z)-5-[(R)-tert-butylsulfinyl]iminospiro[7H-cyclopenta[b]pyridine-6,4'-piperidine]-1'-yl]-6-methyl-pyrazolo[1,5-a]pyrazine-2-carboxylate (550 mg, 0.94 mmol) as a yellow solid. LCMS m/z $[M+H]^+$ = 587.0.

**Step c:** As described for **intermediate D-1-** step c, a similar procedure was performed using ethyl 7-bromo-4-[(5Z)-5-[(R)-tert-butylsulfinyl]iminospiro[7H-cyclopenta[b]pyndine-6,4'-piperidine]-1'-yl]-6-methyl-pyrazolo[1,5-a]pyrazine-2-carboxylate (545 mg, 0.93 mmol) and DIBAL-H (1 M in toluene, 4.0 mL, 4.0 mmol) to give (R)-N-[(5S)-1'-[7-bromo-2-(hydroxymethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (383 mg, 0.70 mmol) as a white solid. LCMS m/z $[M+H]^+$ = 547.1.

**Example i-107. Intermediate D-46 ((R)-N-[(5S)-1'-[7-bromo-2-(difluoromethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide)**

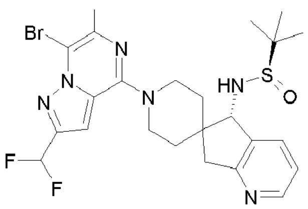

As described for **Intermediate D-13,** a similar procedure was performed using **intermediate B-3** (355 mg, 1.15 mmol) and **intermediate A-5** (315 mg, 1.06 mmol) to give (R)-N-[(5S)-1'-[7-bromo-2-(difluoromethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (475 mg, 0.84 mmol) as a pink solid. LCMS m/z $[M+H]^+$ = 567.2.

**Example i-108. Intermediate D-47 ((R)-N-[(5S)-1'-(7-bromo-3-fluoro-6-methyl-pyrazolo[1,5-a]pyrazin-4-yl)spiro[5,7-dihydrocyclopenta[b]pyridine-6,4'-piperidine]-5-yl]-2-methyl-propane-2-sulfinamide)**

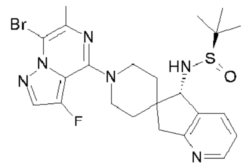

As described for **intermediate D-13**, a similar procedure was performed using **intermediate B-3** (589 mg, 1.92 mmol) and **intermediate A-6** (469 mg, 1.77 mmol) to give (R)-N-[(5S)-1'-(7-bromo-3-fluoro-6-methyl-pyrazolo[1,5-a]pyrazin-4-yl)spiro[5,7-dihydrocyclopenta[b]pyridine-6,4'-piperidine]-5-yl]-2-methyl-propane-2-sulfinamide (911 mg, 1.70 mmol) as a white solid. LCMS m/z $[M+H]^+$ = 537.2. $^1$H NMR (400 MHz, DMSO-*d6*) δ 8.38 (s, 1H), 8.18 (d, *J* = 4.0 Hz, 1H), 7.63 (d, *J* = 7.2 Hz, 1H), 7.22 (dd, *J* = 7.6 Hz, *J* = 4.8 Hz, 1H), 5.79 (d ,*J* = 10.4 Hz, 1H), 4.50 (d, *J* = 10.4 Hz, 1H), 3.89-3.97 (m, 2H), 3.15-3.25 (m, 3H), 2.82-2.89 (m, 1H), 2.42 (s, 3 H), 2.05-2.12 (m, 1H), 1.87-1.98 (m, 1H), 1.63-1.70 (m, 1H), 1.35-1.42 (m, 1H), 1.20 (s, 9H).

**Example i-109. Intermediate D-48 ((R)-N-[(5S)-1'-[7-bromo-3-(hydroxymethyl)-6-methyl-pyrazolo[1,5-a]pyrazin-**4-yl]spiro[5,7-dihydrocyclopenta[b]pyridine-6,4'-piperidine]-5-yl]-2-methyl-propane-2-sulfinamide)

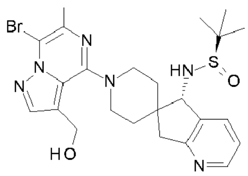

As described for **intermediate D-13,** a similar procedure was performed using **intermediate B-3** (400 mg, 1.3 mmol) and **intermediate A-8** (300 mg, 1.08 mmol) to give (R)-N-[(5S)-1'-[7-bromo-3-(hydroxymethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (529 mg, 0.97 mmol) as a white powder. LCMS m/z $[M+H]^+$ = 547.2. $^1$H NMR (400 MHz, DMSO-*d6*) δ 8.38 (d, *J* = 4.9 Hz, 1H), 8.04 (s, 1H), 7.64 (d, *J* = 7.5 Hz, 1H), 7.22 (dd, *J* = 7.5 Hz, *J* = 4.9 Hz, 1H), 5.80 (d, *J* = 10.2 Hz, 1H), 5.20-5.23 (m, 1H), 4.70-4.78 (m, 2H), 4.53 *(d, J*= 10.2 Hz, 1H), 3.65-3.76 (m, 2H), 2.99-3.16 (m, 3H), 2.80 *(d, J*= 16.4 Hz, 1H), 2.46 (s, 3 H), 2.14-2.21 (m, 1H), 1.94-2.01 (m, 1H), 1.64-1.68 (m, 1H), 1.39-1.42 (m, 1H), 1.21 (s, 9H).

**Example i-110. Intermediate D-49 ((R)-N-[(5S)-1'-[7-bromo-3-(difluoromethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide)**

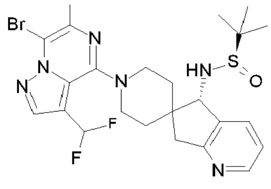

As described for **intermediate D-13,** a similar procedure was performed using **intermediate B-3** (423 mg, 1.4

mmol) and **intermediate A-9** (340 mg, 1.15 mmol) to give (R)-N-[(5S)-1'-[7-bromo-3-(difluoromethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (609 mg, 1.04 mmol) as a white powder. LCMS m/z $[M+H]^+$ = 567.3. $^1H$ NMR (400 MHz, DMSO-*d6*) δ 8.45 (s, 1H), 8.38 *(d, J*= 4.2 Hz, 1H), 7.63 (d, *J* = 7.5 Hz, 1H), 7.36 (t, *J* = 55 Hz, 1H), 7.22 (dd, *J* = 7.4 Hz, *J* = 5.1 Hz, 1H), 5.81 *(d, J*= 10.3 Hz, 1H), 4.54 *(d, J*= 10.3 Hz, 1H), 3.54-3.60 (m, 2H), 3.03-3.16 (m, 3H), 2.82 *(d, J*= 16.5 Hz, 1H), 2.52 (s, 3 H), 2.21-2.28 (m, 1H), 1.98-2.06 (m, 1H), 1.69-1.72 (m, 1H), 1.36-1.39 (m, 1H), 1.23 (s, 9H).

Final Compounds

**Example S1. (5S)-1'-[7-(2,3-difluorophenyl)-6-methyl-pyrazolo[1,5-a]pyrazin-4-yl]spiro[5,7-dihydrocyclopenta[b]pyridine-6,4'-piperidine]-5-amine (Compound 1)**

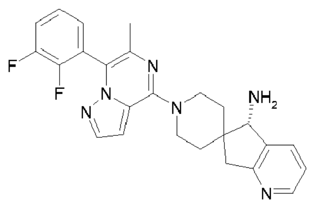

**Step a**: A mixture of **intermediate D-1** (120 mg, 0.23 mmol), 2,3-difluorophenylboronic acid (93 mg, 0.58 mmol), sodium bicarbonate (59 mg, 0.70 mmol) and tetrakis(triphenylphosphine)palladium (8 mg, 6.9 μmol) in a mixture of dioxane (1.8 mL) and water (0.3 mL) was degassed with Ar for 5 min and then heated under microwave irradiation at 120 °C for 30 min. As the reaction was not completed, 2,3-difluorophenylboronic acid (36.5 mg, 0.23 mmol), sodium bicarbonate (29.5 mg, 0.35 mmol) and tetrakis(triphenylphosphine)palladium (8 mg, 6.9 μmol) were added to the reaction mixture. The mixture was degassed with Ar for 5 min and then heated under microwave irradiation at 120 °C for 30 min. Ethyl acetate (10 mL) was added and the organic layer was washed with water (5 mL * 2), brine (5 mL), dried over $Na_2SO_4$ and filtered and concentrated under reduced pressure. The residue was purified by column chromatography ($SiO_2$, ethyl acetate/methanol = 1/0 to 97/3) to give (R)-N-[(5S)-1'-[7-(2,3-difluorophenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (104 mg, 0.19 mmol) as a white foam. LCMS m/z $[M+H]^+$ = 551.2.

**Step b:** To a solution of (R)-N-[(5S)-1'-[7-(2,3-difluorophenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (101 mg, 0.18 mmol) in a mixture of dichloromethane (1 mL) and methanol (1 mL) was added a solution of HCl (4 N in dioxane, 0.23 mL, 0.92 mmol) at 0 °C. The mixture was stirred at RT for 2 h and then concentrated under reduced pressure. Water (5 mL) was added and the pH value of the aqueous phase was adjusted to 11-12 with a 1 N aqueous solution of NaOH. The solid was filtered and washed with water to (5S)-1'-[7-(2,3-difluorophenyl)-6-methyl-pyrazolo[1,5-a]pyrazin-4-yl]spiro[5,7-dihydrocyclopenta[b]pyridine-6,4'-piperidine]-5-amine (80 mg, 0.18 mmol) as a white solid. LCMS m/z $[M+H]^+$ = 447.1. $^1H$ NMR (400 MHz, DMSO-*d6*) δ 1.22 (br d, *J* = 13 Hz, 1 H), 1.64 (br d, *J* = 1 Hz, 1 H), 1.74 - 2.00 (m, 4 H), 2.16 (s, 3 H), 2.79 (d, *J* = 16 Hz, 1 H), 3.15 (d, *J* = 16 Hz, 1 H), 3.35 - 3.47 (m, 2 H), 3.92 (s, 1 H), 4.35 - 4.48 (m, 2 H), 7.01 (d, *J* = 3 Hz, 1 H), 7.18 (dd, *J* = 8, 5 Hz, 1 H), 7.30 - 7.40 (m, 2 H), 7.57 (m, 1 H), 7.66 (dt, *J* = 8, 2 Hz, 1 H), 7.86 (d, *J* = 2 Hz, 1 H), 8.32 (m, 1 H). SHP2 $IC_{50}$ is 6 nM.

**Example S1a. Compounds 2-20**

The following compounds listed in Table 2 were made using the procedure or modifications to the procedure as exemplified for **Example S1** (Compound 1) using appropriate starting materials and intermediates. Compounds were isolated as their free base or hydrochloride salt.

Table 2.

| Cpd No. | Name | Characterization | SHP2 $IC_{50}$ (nM) |
|---|---|---|---|
| 2 | (5S)-1'-(7-(2-chloro-3-fluorophenyl)-6-methylpyrazolo[1,5-a]pyrazin-4-yl)-5,7-dihydrospiro [cyclopenta[b]pyr idine-6,4'-piperidin]-5-amine | $^1$H NMR (400 MHz, DMSO-*d6*) δ ppm 1.25 (br d, *J* = 11 Hz, 1 H), 1.62 (br d, *J* = 14 Hz, 1 H), 1.87 (m, 2 H), 2.07 (s, 3 H), 2.11 - 2.31 (m, 2 H), 2.80 (d, *J* = 16 Hz, 1 H), 3.15 (d, *J* = 16 Hz, 1 H), 3.34 - 3.46 (m, 2 H), 3.95 (s, 1 H), 4.41 (td, *J* = 8, 4 Hz, 2 H), 6.99 (dd, *J* = 3, 1 Hz, 1 H), 7.18 (dd, *J* = 8, 5 Hz, 1 H), 7.36 (m, 1 H), 7.45 - 7.61 (m, 2 H), 7.67 (dt, *J* = 7, 1 Hz, 1 H), 7.84 (d, *J* = 2 Hz, 1 H), 8.31 (m, 1 H) LCMS m/z $[M+H]^+$ = 463.2 | 6 |
| 3 | (5S)-1'-(7-(2-chloro-3-methoxyphenyl)-6-methylpyrazolo[1,5-a]pyrazin-4-yl)-5,7-dihydrospiro [cyclopenta[b]pyr idine-6,4'-piperidin] -5-amine hydrochloride | as HCl salt: $^1$H NMR (400 MHz, DMSO-*d6*) δ ppm 1.68 (br d, *J* = 13 Hz, 1 H), 1.81 (br d, *J* = 13 Hz, 1 H), 1.90 - 2.05 (m, 2 H), 2.11 (s, 3 H), 3.27 (d, *J* = 17 Hz, 1 H), 3.41 - 3.58 (m, 3 H), 3.94 (s, 3 H), 4.42 - 4.66 (m, 3 H), 7.04 (d, *J* = 8 Hz, 1 H), 7.09 (s, 1 H), 7.31 (dd, *J* = 8, 1 Hz, 1 H), 7.47 (t, *J* = 8 Hz, 1 H), 7.59 (t, *J* = 6 Hz, 1 H), 7.89 (d, *J* = 2 Hz, 1 H), 8.33 (d, *J* = 7 Hz, 1 H), 8.67 (d, *J* = 4 Hz, 1 H), 8.86 (s, 3 H) LCMS m/z $[M+H]^+$ = 475.2 | 3 |
| 4 | (5S)-1'-[7-(2-fluoro-3-methoxy-phenyl)-6-methyl-pyrazolo [1 ,5-a]pyrazin-4-yl] spiro[5,7-dihydrocyclopenta[b] pyridine-6,4'-piperidine] -5-amine | $^1$H NMR (400 MHz, DMSO-*d6*) δ ppm 1.22 (br d, *J* = 14 Hz, 1 H), 1.61 (br d, *J* = 13 Hz, 1 H), 1.73 - 2.01 (m, 4 H), 2.13 (s, 3 H), 2.78 (d, *J* = 16 Hz, 1 H), 3.14 (d, *J* = 16 Hz, 1 H), 3.32 - 3.44 (m, 2 H), 3.90 (s, 3 H), 3.92 (s, 1 H), 4.31 - 4.45 (m, 2 H), 6.95 - 7.03 (m, 2 H), 7.17 (dd, *J* = 8, 5 Hz, 1 H), 7.22 - 7.33 (m, 2 H), 7.66 (dt, *J* = 7, 1 Hz, 1 H), 7.83 (d, *J* = 2 Hz, 1 H), 8.32 (dd, *J* = 5, 1 Hz, 1 H) LCMS m/z $[M+H]^+$ = 459.3 | 7 |
| 5 | (5S)-1'-[7-(2,4-difluoro-3-methoxy -pheny l)-6-methyl-pyrazolo [1 ,5-a]pyrazin-4-yl] spiro[5,7-dihydrocyclopenta[b] pyridine-6,4'-piperidine] -5-amine | $^1$H NMR (400 MHz, DMSO-*d6*) δ ppm 1.25 (d, *J* = 12 Hz, 1 H), 1.62 (d, *J* = 12 Hz, 1 H), 1.75 - 1.96 (m, 2 H), 2.15 (s, 3 H), 2.22 - 2.37 (m, 2 H), 2.81 (d, *J* = 16 Hz, 1 H), 3.16 (d, *J* = 16 Hz, 1 H), 3.33 - 3.48 (m, 2 H), 3.95 (s, 1 H), 3.97 (s, 3 H), 4.41 (td, *J*= 8, 4 Hz, 2 H), 7.00 (d, *J* = 2 Hz, 1 H), 7.19 (dd, *J* = 8, 5 Hz, 1 H), 7.22 - 7.33 (m, 2 H), 7.68 (d, *J* = 8 Hz, 1 H), 7.86 (d, *J* = 3 Hz, 1 H), 8.34 (m, 1 H) LCMS m/z $[M+H]^+$ = 477.3 | 3 |
| 6 | (1S)-1'-[7-(3-chloro-2-methyl-4-pyridyl)-6-methyl-pyrazolo [1,5-a]pyrazin-4-yl]-5-methoxy-spiro[indane-2,4'-piperidine]-1-amine | $^1$H NMR (400 MHz, DMSO-*d6*) δ ppm 1.25 (br d, *J* = 15 Hz, 1 H), 1.59 (br d, *J* = 13 Hz, 1 H), 1.65 - 1.93 (m, 4H), 2.07 (s, 3 H), 2.61 - 2.69 (m, 4H), 3.07 (d, *J* = 16 Hz, 1 H), 3.34 - 3.48 (m, 2 H), 3.74 (s, 3 H), 3.81 (s, 1 H), 4.31 - 4.47 (m, 2 H), 6.74 (dd, *J* = 8, 3 Hz, 1 H), 6.79 (d, *J* = 2 Hz, 1 H), 7.01 (dd, *J* = 3, 1 Hz, 1 H), 7.21 (d, *J* = 8 Hz, 1 H), 7.41 (d, *J* = 5 Hz, 1 H), 7.85 (d, *J* = 3 Hz, 1 H), 8.55 (d, *J* = 5 Hz, 1 H) LCMS m/z $[M+H]^+$ = 489.2 | 8 |
| 7 | (5S)-1'-[7-(3-chloro-2-isopropyl-4-pyridyl)-6-methyl-pyrazolo[1,5-a]pyrazin-4-yl] spiro[5,7-dihydrocyclopenta[b] pyridine-6,4'-piperidine] -5-amine hydrochloride | As HCl salt: $^1$H NMR (400 MHz, DMSO-*d6*) δ ppm 1.30 (t, *J* = 6 Hz, 6 H), 1.67 (br d, *J* = 13 Hz, 1 H), 1.79 (br d, *J* = 13 Hz, 1 H), 1.88 - 2.03 (m, 2 H), 2.10 (s, 3 H), 3.26 (br d, *J* = 17 Hz, 1 H), 3.41 - 3.52 (m, 3 H), 3.60 (dquin, *J* = 14, 7, 7, 7, 7 Hz, 1 H), 4.39 - 4.62 (m, 3 H), 7.08 (d, *J* = 2 Hz, 1 H), 7.40 (dd, *J* = 5, 1 Hz, 1 H), 7.58 (dd, *J* = 8, 5 Hz, 1 H), 7.91 (d, *J* = 2 Hz, 1 H), 8.31 (d, *J* = 8 Hz, 1 H), 8.59 - 8.71 (m, 2 H), 8.83 (br s, 3 H) LCMS m/z $[M+H]^+$ = 488.2 | 13 |
| 8 | (5S)-1'-[7-(2-fluorophenyl)-6-methyl-pyrazolo[1,5-a] pyrazin-4-yl]-3-methyl-spiro [5,7-dihydrocyclopenta[b] pyridine-6,4'-piperidine] -5-amine | $^1$H NMR (400 MHz, DMSO-*d6*) δ ppm 1.23 (d, *J* = 14 Hz, 1 H), 1.61 (br d, *J* = 13 Hz, 1 H), 1.76 - 1.96 (m, 2 H), 1.98 - 2.13 (m, 2 H), 2.14 (s, 3 H), 2.28 (s, 3 H), 2.73 (d, *J* = 16 Hz, 1 H), 3.10 (d, *J* = 16 Hz, 1 H), 3.32 - 3.44 (m, 2 H), 3.90 (s, 1 H), 4.34 - 4.45 (m, 2 H), 6.98 (d, *J* = 2 Hz, 1 H), 7.31 - 7.40 (m, 2 H), 7.45 - 7.61 (m, 3 H), 7.84 (d, *J* = 2 Hz, 1 H), 8.17 (m, 1 H). LCMS m/z $[M+H]^+$ = 443.2 | 6 |

(continued)

| Cpd No. | Name | Characterization | SHP2 $IC_{50}$ (nM) |
|---|---|---|---|
| **9** | (1S)-1'-[7-(2-chloro-3-fluorophenyl)-6-methyl-pyrazolo[1,5-a]pyrazin-4-yl]-6-methoxy-spiro[indane-2,4'-piperidine]-1-amine hydrochloride | As HCl salt: $^{1}$H NMR (400 MHz, DMSO-*d6*) δ ppm 1.57 - 1.71 (m, 2 H), 1.83 - 1.93 (m, 2 H), 2.09 (s, 3 H), 2.98 (d, *J* = 16 Hz, 1 H), 3.14 (d, *J* = 16 Hz, 1 H), 3.39 (m hidden, 2 H), 3.77 (s, 3 H), 4.33 - 4.53 (m, 3 H), 6.94 (dd, *J* = 8, 2 Hz, 1 H), 7.03 (d, *J* = 2 Hz, 1 H), 7.19 (d, *J* = 1 Hz, 1 H), 7.26 (d, *J* = 8 Hz, 1 H), 7.35 (d, *J* = 7 Hz, 1 H), 7.51 - 7.61 (m, 2 H), 7.87 (d, *J* = 2 Hz, 1 H), 8.38 (br s, 3 H) LCMS m/z $[M+H]^{+}$ = 492.2 | 43 |
| **10** | (1S)-1'-[7-(3-chloro-2-methyl-4-pyridyl)-6-methyl-pyrazolo [1,5-a]pyrazin-4-yl]-6-methoxy-spiro[indane-2,4'-piperidine]-1-amine hydrochloride | As HCl salt: $^{1}$H NMR (400 MHz, DMSO-*d6*) δ ppm 1.57 - 1.69 (m, 2 H), 1.86 (m, 2 H), 2.08 (s, 3 H), 2.66 (s, 3 H), 2.99 (d, *J* = 16 Hz, 1 H), 3.13 (d, *J*= 16 Hz, 1 H), 3.36 - 3.53 (m hidden, 2 H), 3.77 (s, 3 H), 4.34 - 4.53 (m, 3 H), 6.95 (dd, *J* = 8, 2 Hz, 1 H), 7.04 (s, 1 H), 7.15 (m, 1 H), 7.26 *(d, J*= 8 Hz, 1 H), 7.40 *(d, J*= 5 Hz, 1 H), 7.88 (d, *J*= 2 Hz, 1 H), 8.30 (br s, 3 H), 8.56 (d, *J* = 5 Hz, 1 H) LCMS m/z $[M+H]^{+}$ = 489.2 | 11 |
| **11** | (1S)-1'-[7-(2-fluorophenyl)-6-methyl-pyrazolo[1,5-a] pyrazin-4yl]-6-methoxy-spiro [indane-2,4'-piperidine]-1-amine hydrochloride | As HCl salt: $^{1}$H NMR (400 MHz, DMSO-*d6*) δ ppm 1.51 - 1.70 (m, 2 H), 1.79 - 1.93 (m, 2 H), 2.15 (s, 3 H), 2.98 (d, *J* = 16 Hz, 1 H), 3.14 (d, *J* = 16 Hz, 1 H), 3.43 - 3.48 (m hidden, 2 H), 3.77 (s, 3 H), 4.29 - 4.55 (m, 3 H), 6.94 (dd, *J* = 8, 3 Hz, 1 H), 7.02 (t, *J* = 2 Hz, 1 H), 7.18 (d, *J* = 3 Hz, 1 H), 7.26 (d, *J* = 8 Hz, 1 H), 7.32 - 7.41 (m, 2 H), 7.49 (m, 1 H), 7.58 (m, 1 H), 7.87 (d, *J* = 3 Hz, 1 H), 8.35 (br s, 3 H) LCMS m/z $[M+H]^{+}$ = 458.2 | 23 |
| **12** | (7S)-1'-[7-(2-chloro-3-fluorophenyl)-6-methyl-pyrazolo[1,5-a]pyrazin-4-yl] spiro[5,7-dihydrocyclopenta[b] pyridine-6,4'-piperidine]-7-amine | $^{1}$H NMR (400 MHz, DMSO-*d6*) δ ppm 1.24 (br d, *J* = 13 Hz, 1 H), 1.66 (br d, *J* = 14 Hz, 1 H), 1.83 - 2.03 (m, 4 H), 2.08 (s, 3 H), 2.72 (d, *J* = 16 Hz, 1 H), 3.12 (d, *J* = 16 Hz, 1 H), 3.35 - 3.56 (m, 2 H), 3.93 (s, 1 H), 4.26 - 4.39 (m, 2 H), 7.00 (dd, *J* = 2, 1 Hz, 1 H), 7.18 (dd, *J* = 8, 5 Hz, 1 H), 7.37 (m, 1 H), 7.51 - 7.61 (m, 2 H), 7.62 (d, *J* = 7 Hz, 1 H), 7.84 (d, *J* = 2 Hz, 1 H), 8.36 (d, *J* = 4 Hz, 1 H) LCMS m/z $[M+H]^{+}$ = 463.2 | 14 |
| **13** | (7S)-1'-[7-(3-chloro-2-methyl-4-pyridyl)-6-methyl-pyrazolo [1,5-a]pyrazin-4-yl]spiro[5,7-dihydrocyclopenta[b]pyridine-6,4'-piperidine]-7-amine | $^{1}$H NMR (400 MHz, DMSO-*d6*) δ ppm 1.24 (br d, *J* = 13 Hz, 1 H), 1.66 (br d, *J* = 13 Hz, 1 H), 1.81 - 2.00 (m, 4 H), 2.08 (s, 3 H), 2.66 (s, 3 H), 2.71 (d, *J* = 16 Hz, 1 H), 3.12 (d, *J* = 16 Hz, 1 H), 3.42 - 3.54 (m, 2 H), 3.93 (s, 1 H), 4.33 (ddd, *J* = 17, 9, 5 Hz, 2 H), 7.02 (dd, *J* = 2, 2 Hz, 1 H), 7.18 (dd, *J* = 7, 5 Hz, 1 H), 7.42 (d, *J* = 5 Hz, 1 H), 7.63 (d, *J* = 7 Hz, 1 H), 7.85 (d, *J* = 2 Hz, 1 H), 8.36 (d, *J* = 4 Hz, 1 H), 8.55 (d, *J* = 5 Hz, 1 H) LCMS m/z $[M+H]^{+}$ = 460.3 | 7 |
| **14** | (3R)-1'-[7-(2-chloro-3-fluorophenyl)-6-methyl-pyrazolo [1 ,5-a]pyrazin-4-yl] spiro[3H-furo[2,3-b]pyridine-2,4'-piperidine]-3-amine | $^{1}$H NMR (500 MHz, DMSO-*d6*) δ ppm 1.82 - 1.88 (m, 1 H), 1.88 - 1.98 (m, 2 H), 2.00 - 2.12 (m, 4 H), 2.17 - 2.30 (m, 2 H), 3.49 - 3.65 (m, 2 H), 4.17 (s, 1 H), 4.37 - 4.53 (m, 2 H), 6.93 (dd, *J* = 7, 5 Hz, 1 H), 7.07 (d, *J* = 2 Hz, 1 H), 7.38 (d, *J* = 7 Hz, 1 H), 7.52 - 7.60 (m, 2 H), 7.73 (d, *J* = 7 Hz, 1 H), 7.87 (d, *J* = 2 Hz, 1 H), 8.02 (dd, *J* = 5, 1 Hz, 1 H) LCMS m/z $[M+H]^{+}$ = 465.2 | 40 |
| **15** | (3R)-1'-[7-(3-chloro-2-methyl-4-pyridyl)-6-methyl-pyrazolo [1,5-a]pyrazin-4-yl]spiro[3H-furo[2,3-b]pyridine-2,4'-piperidine]-3-amine | $^{1}$H NMR (400 MHz, DMSO-*d6*) δ ppm 1.82 - 2.00 (m, 3 H), 2.01 - 2.21 (m, 6 H), 2.67 (s, 3 H), 3.50 - 3.68 (m, 2 H), 4.16 (s, 1 H), 4.38 - 4.53 (m, 2 H), 6.93 (dd, *J* = 7, 5 Hz, 1 H), 7.08 (d, *J* = 3 Hz, 1 H), 7.42 (d, *J* = 5 Hz, 1 H), 7.73 (d, *J* = 7 Hz, 1 H), 7.88 (d, *J* = 2 Hz, 1 H), 8.02 (dd, *J*= 5, 1 Hz, 1 H), 8.56 (d, *J* = 5 Hz, 1 H) LCMS m/z $[M+H]^{+}$ = 462.2 | 20 |

(continued)

| Cpd No. | Name | Characterization | SHP2 $IC_{50}$ (nM) |
|---|---|---|---|
| **16** | (3R)-1'-[7-(4-fluoro-1H-indol-7-yl)-6-methyl-pyrazolo[1,5-a]pyrazin-4-yl]spiro[3H-furo[2,3-b]pyridine-2,4'-piperidine]-3-amine | $^1$H NMR (400 MHz, DMSO-*d6*) δ ppm 1.85 - 2.01 (m, 3 H), 2.06 - 2.17 (m, 6 H), 3.52 - 3.67 (m, 2 H), 4.18 (s, 1 H), 4.33 - 4.50 (m, 2 H), 6.57 (dd, *J* = 3, 2 Hz, 1 H), 6.86 - 6.98 (m, 2 H), 7.02 (dd, *J* = 2, 1 Hz, 1 H), 7.10 (dd, *J* = 8, 5 Hz, 1 H), 7.28 (t, *J* = 3 Hz, 1 H), 7.74 (d, *J* = 7 Hz, 1 H), 7.79 (d, *J* = 2 Hz, 1 H), 8.02 (dd, *J* = 5, 1 Hz, 1 H), 11.05 (br s, 1 H) LCMS m/z $[M+H]^+$ = 470.2 | 42 |
| **17** | (3R)-1'-[7-(2,3-dichlorophenyl)-6-methyl-pyrazolo[1,5-a]pyrazin-4-yl] spiro[3H-furo[2,3-b]pyridine-2,4'-piperidine]-3-amine | $^1$H NMR (500 MHz, DMSO-*d6*) δ ppm 1.80 - 1.97 (m, 3 H), 2.03 - 2.18 (m, 6 H), 3.51 - 3.65 (m, 2 H), 4.16 (s, 1 H), 4.41 (m, 1 H), 4.49 (m, 1 H), 6.93 (dd, *J* = 7, 5 Hz, 1 H), 7.06 (d, *J* = 2 Hz, 1 H), 7.47 - 7.56 (m, 2 H), 7.72 (m, 1 H), 7.80 (dd, *J* = 8, 2 Hz, 1 H), 7.87 (d, *J* = 2 Hz, 1 H), 8.02 (ddd, *J*= 5, 2, 1 Hz, 1 H) LCMS m/z $[M+H]^+$ = 481.1 | 39 |
| **18** | (3R)-1'-[7-(4-fluoro-1H-indol-7-yl)-6-methyl-pyrazolo[1,5-a] pyrazin-4-yl] spiro [3H-benzofuran-2,4'-piperidine] -3-amine | $^1$H NMR (400 MHz, DMSO-*d6*) δ ppm 1.81 - 1.95 (m, 3 H), 1.98 (br s, 2 H), 2.06 (m, 1 H), 2.11 (s, 3 H), 3.52 - 3.68 (m, 2 H), 4.14 (s, 1 H), 4.27 - 4.47 (m, 2 H), 6.57 (dd, *J* = 3, 2 Hz, 1 H), 6.81 (d, *J* = 8 Hz, 1 H), 6.86 - 6.94 (m, 2 H), 7.00 (d, *J* = 2 Hz, 1 H), 7.10 (dd, *J* = 8, 5 Hz, 1 H), 7.17 (td, *J* = 8, 1 Hz, 1 H), 7.28 (t, *J* = 3 Hz, 1 H), 7.35 (d, *J* = 7 Hz, 1 H), 7.78 (d, *J* = 3 Hz, 1 H), 11.04 (br s, 1 H) LCMS m/z $[M+H]^+$ = 469.2 | 132 |
| **19** | (3R)-1'-[7-(2,3-dichlorophenyl)-6-methyl-pyrazolo [1 ,5-a]pyrazin-4-yl] spiro[3H-benzofuran-2,4'-piperidine]-3-amine | $^1$H NMR (400 MHz, DMSO-*d6*) δ ppm 1.73 - 2.07 (m, 6 H), 2.09 (s, 3 H), 3.51 - 3.66 (m, 2 H), 4.13 (s, 1 H), 4.31 - 4.53 (m, 2 H), 6.80 (d, *J* = 8 Hz, 1 H), 6.88 (td, *J* = 8, 1 Hz, 1 H), 7.05 (d, *J* = 3 Hz, 1 H), 7.16 (td, *J* = 8, 2 Hz, 1 H), 7.34 (d, *J* = 7 Hz, 1 H), 7.45 - 7.59 (m, 2 H), 7.80 (dd, *J* = 8, 2 Hz, 1 H), 7.86 (d, *J* = 3 Hz, 1 H) LCMS m/z $[M+H]^+$ = 480.1 | 102 |
| **20** | (3R)-1'-[7-(2-chloro-3-fluorophenyl)-6-methyl-pyrazolo[1,5-a]pyrazin-4-yl] spiro[3H-benzofuran-2,4'-piperidine]-3-amine | $^1$H NMR (500 MHz, DMSO-*d6*) δ ppm 1.79 - 1.94 (m, 3 H), 1.96 (br s, 2 H), 2.03 - 2.09 (m, 1 H), 2.10 (s, 3 H), 3.46 - 3.69 (m, 2 H), 4.13 (s, 1 H), 4.34 - 4.49 (m, 2 H), 6.80 (d, *J* = 8 Hz, 1 H), 6.89 (t, *J* = 7 Hz, 1 H), 7.05 (d, *J* = 2 Hz, 1 H), 7.16 (t, *J* = 8 Hz, 1 H), 7.34 (d, *J* = 7 Hz, 1 H), 7.37 (dd, *J* = 7, 2 Hz, 1 H), 7.52 - 7.63 (m, 2 H), 7.86 (d, *J* = 2 Hz, 1 H) LCMS m/z $[M+H]^+$ = 464.2 | 115 |

**Example S2. (1S)-1'-[6-methyl-7-(3-methylisoxazol-4-yl)pyrazolo[1,5-a]pyrazin-4-yl]spiro[indane-2,4'-piperidine]-1-amine (Compound 21)**

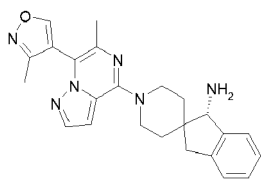

A mixture of **intermediate D-10** (75 mg, 0.18 mmol), 3-methyl-4-(4,4,5,5-tetramethyl-1,3,2-dioxaborolan-2-yl)isoxazole (55 mg, 0.26 mmol), sodium bicarbonate (40 mg, 0.38 mmol) and tetrakis(triphenylphosphine)palladium (21 mg, 18.2 μmol) in a mixture of dimethoxyethane (2 mL) and water (1 mL) was degassed with Ar for 5 min and then heated under microwave irradiation at 150 °C for 25 min. As the reaction was not completed, 3-methyl-4-(4,4,5,5-tetramethyl-1,3,2-dioxaborolan-2-yl)isoxazole (27 mg, 0.13 mmol), sodium bicarbonate (40 mg, 0.37 mmol) and tetrakis(triphenylphosphine)palladium (11 mg, 9.5 μmol) were added to the reaction mixture. The mixture was degassed

with Ar for 3 min and then heated under microwave irradiation at 150 °C for 15 min. Ethyl acetate (20 mL) and water (20 mL) were added, the aqueous layer was separated and then extracted with ethyl acetate (20 mL). The combined organic layer was washed with brine (20 mL), dried over $Na_2SO_4$, filtered and concentrated under reduced pressure. The residue was purified by purified by preparative HPLC chromatography (0.1% $HCO_2H$ in $H_2O$ / $CH_3CN$ with gradient elution 90:10 to 0:100 XSELECT CSH Prep C18 5μm OBD 50 * 250mm). The solid was taken up in dichloromethane and water, the pH value of the aqueous layer was adjusted to 11-12 with a 1 N aqueous solution of NaOH. The mixture was filtered on a hydrophobic cartridge (liquid / liquid extraction column, Radleys®), dried over $Na_2SO_4$ and then concentrated in vacuum to give (1S)-1'-[6-methyl-7-(3-methylisoxazol-4-yl)pyrazolo[1,5-a]pyrazin-4-yl]spiro[indane-2,4'-piperidine]-1-amine (13 mg, 0.03 mmol) as a white solid. LCMS m/z $[M+H]^+$ = 415.2. $^1H$ NMR (500 MHz, DMSO-*d6*) δ ppm 1.20 (br d, *J* = 13 Hz, 1 H), 1.63 (m, 1 H), 1.79 (td, *J* = 13, 4 Hz, 1 H), 1.90 (td, *J* = 12, 4 Hz, 1 H), 1.88 - 1.97 (m, 2 H), 2.08 (s, 3 H), 2.21 (s, 3 H), 2.68 (d, *J* = 16 Hz, 1 H), 3.12 (d, *J* = 16 Hz, 1 H), 3.31 - 3.43 (m, 2 H), 3.88 (s, 1 H), 4.39 (dd, *J* = 19, 14 Hz, 2 H), 7.01 (d, *J* = 2 Hz, 1 H), 7.15 - 7.23 (m, 3 H), 7.32 (d, *J* = 7 Hz, 1 H), 7.90 (d, *J* = 2 Hz, 1 H), 9.09 (s, 1 H). SHP2 $IC_{50}$ is 58 nM.

**Example S2a. Compounds 22-26**

The following compounds listed in Table 3 were made using the procedure or modifications to the procedure as exemplified for **Example S2** (Compound **21**) using appropriate starting materials and intermediates. Compounds were isolated as their free base, hydrochloride or formate salt.

**Table 3.**

| Cpd. No. | Name | Characterization | SHP2 $IC_{50}$ (nM) |
|---|---|---|---|
| **22** | (5S)-1'-[7-(2-chlorophenyl)-6-methyl-pyrazolo[1,5-a] pyrazin-4-yl]spiro[5,7-dihydrocyclopenta[b]pyridine-6,4'-piperidine]-5-amine | $^1H$ NMR (400 MHz, DMSO-*d6*) δ ppm 1.34 (br d, *J* = 14 Hz, 1 H), 1.63 (br d, *J* = 13 Hz, 1 H), 1.79 - 2.02 (m, 2 H), 2.16 (s, 3 H), 2.61 - 2.67 (m, 1 H), 2.98 (d, *J* = 16 Hz, 1 H), 3.42 - 3.55 (m, 2 H), 3.87 (s, 3 H), 3.88 (s, 1 H), 4.24 - 4.36 (m, 3 H), 6.62 (d, *J* = 8 Hz, 1 H), 7.01 *(d, J*= 3 Hz, 1 H), 7.28 - 7.41 (m, 2 H), 7.54 (m, 1 H), 7.86 (d, *J* = 3 Hz, 1 H) LCMS m/z $[M+H]^+$ = 445.2 | 11 |
| | | | |
| **23** | (5S)-1'-[7-(3-chloro-2-methyl-4-pyridyl)-6-methyl-pyrazolo [1,5-a]pyrazin-4-yl]-3-methoxy-spiro[5,7-dihydrocyclopenta[b]pyridine-6,4'-piperidine]-5-amine | $^1H$ NMR (500 MHz, DMSO-*d6*) δ 1.24 (br d, *J* = 13 Hz, 1ppm H), 1.64 (br d, *J* = 13 Hz, 1 H), 1.79 - 1.96 (m, 2 H), 2.08 (s, 3 H), 2.12 - 2.31 (m, 2 H), 2.66 (s, 3 H), 2.72 (d, *J* = 16 Hz, 1 H), 3.08 (d, *J* = 16 Hz, 1 H), 3.31 - 3.45 (m, 2 H), 3.82 (s, 3 H), 3.92 (s, 1 H), 4.44 (ddd, *J* = 13, 8, 4 Hz, 2 H), 7.01 (t, *J* = 2 Hz, 1 H), 7.31 (d, *J* = 2 Hz, 1 H), 7.42 (d, *J* = 5 Hz, 1 H), 7.85 (d, *J* = 2 Hz, 1 H), 8.04 (d, *J* = 3 Hz, 1 H), 8.55 (d, *J* = 5 Hz, 1 H) LCMS m/z $[M+H]^+$ = 490.2 | 10 |
| **24** | (5S)-1'-[7-(2,3-difluorophenyl)-6-methyl-pyrazolo[1,5-a]pyrazin-4-yl]-3-methoxy-spiro[5,7-dihydrocyclopenta[b]pyridine-6,4'-piperidine]-5-amine | $^1H$ NMR (400 MHz, DMSO-*d6*) δ ppm 1.22 (br d, *J* = 14 Hz, 1 H), 1.62 (br d, *J* = 13 Hz, 1 H), 1.72 - 2.03 (m, 4H), 2.15 (s, 3 H), 2.70 (br d, *J* = 16 Hz, 1 H), 3.07 (d, *J*= 16 Hz, 1 H), 3.31 - 3.46 (m, 2 H), 3.80 (s, 3 H), 3.89 (s, 1 H), 4.35 - 4.50 (m, 2 H), 7.00 (dd, *J* = 3, 1 Hz, 1 H), 7.29 (dd, *J* = 3, 1 Hz, 1 H), 7.31 - 7.42 (m, 2 H), 7.50 - 7.66 (m, 1 H), 7.86 (d, *J* = 3 Hz, 1 H), 8.02 (dd, *J* = 3, 1 Hz, 1 H) LCMS m/z $[M+H]^+$ = 477.2 | 11 |
| **25** | (S)-1'-(7-(3-chloro-2-methylpyridin-4-yl)-6-methylpyrazolo[1,5-a]pyrazin-4-yl)-1,3-dihydrospiro[indene-2,4'-piperidin]-1-amine hydrochloride | As HCl salt: $^1H$ NMR (400 MHz, DMSO-*d6*) δ ppm 1.56 - 1.70 (m, 2 H), 1.84 - 1.94 (m, 2 H), 2.09 (s, 3 H), 2.66 (s, 3 H), 3.07 (d, *J*= 16 Hz, 1 H), 3.23 (br d, *J*= 16 Hz, 1 H), 3.45 - 3.56 (m hidden, 2 H), 4.38 - 4.54 (m, 3 H), 7.03 - 7.06 (m, 1 H), 7.28 - 7.39 (m, 3 H), 7.41 (d, *J* = 5 Hz, 1 H), 7.57 (d, *J* = 7 Hz, 1 H), 7.88 (d, *J* = 2 Hz, 1 H), 8.39 (br s, 3 H), 8.56 (d, *J* = 5 Hz, 1 H) LCMS m/z $[M+H]^+$ = 459.1 | 6 |

(continued)

| Cpd. No. | Name | Characterization | SHP2 $IC_{50}$ (nM) |
|---|---|---|---|
| **26** | (5S)-1'-[7-(2,3-difluorophenyl) pyrazolo[1,5-a]pyrazin-4-yl] spiro[5,7-dihydrocyclopenta[b] pyridine-6,4'-piperidine] -5-amine formate | As $HCO_2H$ salt: $^1H$ NMR (400 MHz, DMSO-*d6*) δ ppm 1.21 (br d, *J*= 13 Hz, 1 H), 1.55 (br d, *J* = 12 Hz, 1 H), 1.74 - 1.88 (m, 2 H), 2.78 *(d, J*= 16 Hz, 1 H), 3.11 (br d, *J* = 16 Hz, 4 H), 3.22 - 3.27 (m hidden, 2 H), 3.93 (s, 1 H), 4.32 - 4.40 (m, 2 H), 7.03 (d, *J* = 3 Hz, 1 H), 7.13 (dd, *J* = 7, 5 Hz, 1 H), 7.29 (m, 1 H), 7.39 (m, 1 H), 7.45 (s, 1 H), 7.50 (m, 1 H), 7.63 (d, *J* = 8 Hz, 1 H), 7.93 *(d, J*= 2 Hz, 1 H), 8.13 (s, 1 H), 8.28 (d, *J* = 5 Hz, 1 H) LCMS m/z $[M+H]^+$ = 433.1 | 23 |

**Example S3. (5S)-1'-(6-methyl-7-quinoxalin-6-yl-pyrazolo[1,5-a]pyrazin-4-yl)spiro[5,7-dihydrocyclopenta[b]pyridine-6,4'-piperidine]-5-amine hydrochloride (Compound 27)**

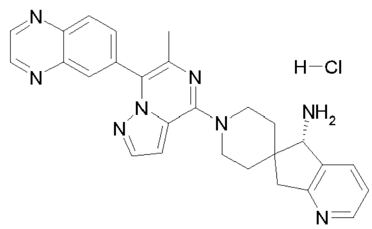

**Step a:** A mixture of **intermediate D-3** (80 mg, 0.16 mmol), quinoxaline-6-boronic acid pinacol ester (126 mg, 0.47 mmol), sodium bicarbonate (39 mg, 0.47 mmol) and tetrakis(triphenylphosphine)palladium (22 mg, 19 μmol) in a mixture of dioxane (4 mL) and water (1 mL) was degassed with Ar for 5 min and then heated under microwave irradiation at 150 °C for 20 min. The reaction mixture was filtered and then poured into water. The aqueous layer was extracted twice with EtOAc. The combined organic layer was washed with water brine, dried over $Na_2SO_4$ and filtered and concentrated under reduced pressure. The residue was purified by column chromatography ($SiO_2$, cyclohexane / ethyl acetate = 1/0 to 0/1) to give tert-butyl N-[(5S)-1'-(6-methyl-7-quinoxalin-6-yl-pyrazolo[1,5-a]pyrazin-4-yl)spiro[5,7-dihydrocyclopenta[b]pyridine-6,4'-piperidine]-5-yl]carbamate (76 mg, 0.135 mmol) as a white foam. LCMS m/z $[M+H]^+$ = 563.6.

**Step b:** A mixture of tert-butyl N-[(5S)-1'-(6-methyl-7-quinoxalin-6-yl-pyrazolo[1,5-a]pyrazin-4-yl)spiro[5,7-dihydrocyclopenta[b]pyridine-6,4'-piperidine]-5-yl]carbamate (76 mg, 0.135 mmol) in a solution of HCl (2.5 N in ethanol, 2 mL, 5 mmol) was stirred 18 h at RT. Diisopropyl ether was added, and the precipitate was then filtered, washed with diisopropyl ether to give (5S)-1'-(6-methyl-7-quinoxalin-6-yl-pyrazolo[1,5-a]pyrazin-4-yl)spiro[5,7-dihydrocyclopenta[b]pyridine-6,4'-piperidine]-5-amine hydrochloride (45 mg, 0.09 mmol) as a brown solid. LCMS m/z $[M+H]^+$ = 463.2. $^1H$ (400 MHz, DMSO-*d6*) δ ppm 1.69 (br d, *J* = 14 Hz, 1 H), 1.81 (br d, *J* = 13 Hz, 1 H), 1.87 - 2.10 (m, 2 H), 2.30 (s, 3 H), 3.28 (d, *J* = 18 Hz, 1 H), 3.46 - 3.61 (m, 3 H), 4.35 - 4.59 (m, 3 H), 7.16 (br s, 1 H), 7.61 (m, 1 H), 7.93 - 7.99 (m, 2 H), 8.20 - 8.25 (m, 2 H), 8.34 (d, *J* = 8 Hz, 1 H), 8.68 (d, *J* = 5 Hz, 1 H), 8.87 (br s, 3 H), 9.02 (d, *J* = 3 Hz, 1 H), 9.03 (d, *J* = 3 Hz, 1 H). SHP2 $IC_{50}$ is 16 nM.

**Example S3a. Compounds 28-30**

The following compounds listed in Table 4 were made using the procedure or modifications to the procedure as exemplified for **Example S3** (Compound **27**) using appropriate starting materials and intermediates. Compounds were isolated as their free base, hydrochloride or formate salt.

Table 4.

| Cpd. No. | Name | Characterization | SHP2 $IC_{50}$ (nM) |
|---|---|---|---|
| 28 | (5S)-1'-[7-(3-chloro-2-methyl-4-pyridyl)-6-methyl-pyrazolo [1,5-a]pyrazin-4-yl]-3-fluoro-spiro[5,7-dihydrocyclopenta [b]pyridine-6,4'-piperidine] -5-amine | $^1$H NMR (400 MHz, DMSO-*d6*) δ ppm 1.24 (br d, *J* = 2 Hz, 1 H), 1.65 (br d, *J* = 14 Hz, 1 H), 1.83 (td, *J* = 13, 4 Hz, 1 H), 1.91 (ddd, *J* = 14, 11, 4 Hz, 1 H), 1.99 - 2.14 (m, 2 H), 2.07 (s, 3 H), 2.65 (s, 3 H), 2.77 (br d, *J* = 16 Hz, 1 H), 3.14 (d, *J*= 16 Hz, 1 H), 3.32 - 3.46 (m, 2 H), 3.95 (s, 1 H), 4.34 - 4.48 (m, 2 H), 7.01 (dd, *J* = 2, 2 Hz, 1 H), 7.41 (d, *J* = 5 Hz, 1 H), 7.55 (m, 1 H), 7.84 (d, *J* = 2 Hz, 1 H), 8.29 (m, 1 H), 8.54 (d, *J* = 5 Hz, 1 H) LCMS m/z $[M+H]^+$ = 478.1 | 15 |
| 29 | (5S)-1'-[6-methyl-7-(2-methylpyrazol-3-yl)pyrazolo [1,5-a]pyrazin-4-yl]spiro[5,7-dihydrocyclopenta[b]pyridine-6,4'-piperidine]-5-amine hydrochloride | As HCl salt: $^1$H NMR (400 MHz, DMSO-*d6*) δ ppm 1.65 (br d, *J* = 13 Hz, 1 H), 1.75 (br d, *J* = 13 Hz, 1 H), 1.85 - 2.02 (m, 2 H), 2.17 (s, 3 H), 3.23 (d, *J* = 17 Hz, 1 H), 3.39 - 3.53 (m, 3 H), 3.55 (s, 3 H), 4.42 - 4.60 (m, 3 H), 6.45 (d, *J* = 2 Hz, 1 H), 7.09 (dd, *J* = 2, 1 Hz, 1 H), 7.52 (dd, *J* = 8, 5 Hz, 1 H), 7.58 (d, *J* = 2 Hz, 1 H), 7.94 (d, *J*= 2 Hz, 1 H), 8.23 (d, *J* = 8 Hz, 1 H), 8.63 (dd, *J* = 5, 1 Hz, 1 H), 8.75 (br s, 3 H) LCMS m/z $[M+H]^+$ = 415.2 | 38 |
| 30 | (5S)-1'-[7-(5-fluoro-6-quinolyl)-6-methyl-pyrazolo [1 ,5-a]pyrazin-4-yl]spiro[5,7-dihydrocyclopenta[b]pyridine-6,4'-piperidine] -5-amine formate | As $HCO_2H$ salt: $^1$H NMR (500 MHz, DMSO-*d6*) δ ppm 1.32 (br d, *J* = 13 Hz, 1 H), 1.63 (br d, *J* = 13 Hz, 1 H), 1.83 - 1.96 (m, 2 H), 2.19 (s, 3 H), 2.87 (d, *J*= 16 Hz, 1 H), 3.19 (d, *J* = 16 Hz, 1 H), 3.35 - 3.49 (m, 2 H), 4.03 (s, 1 H), 4.42 - 4.48 (m, 2 H), 7.04 (d, *J* = 2 Hz, 1 H), 7.21 (dd, *J*= 7, 5 Hz, 1 H), 7.71 (dt, *J* = 8, 4 Hz, 2 H), 7.82 - 7.88 (m, 2 H), 8.01 *(d, J*= 9 Hz, 1 H), 8.19 (s, 2 H), 8.36 (dd, *J*= 5, 1 Hz, 1 H), 8.56 (d, *J* = 8 Hz, 1 H), 9.07 (dd, *J* = 4, 2 Hz, 1 H) LCMS m/z $[M+H]^+$ = 480.2 | 17 |

**Example S4. (5S)-1'-[7-(2-fluoro-5-methoxy-phenyl)-6-methyl-pyrazolo[1,5-a]pyrazin-4-yl]spiro[5,7-dihydrocyclopenta[b]pyridine-6,4'-piperidine]-5-amine (Compound 31)**

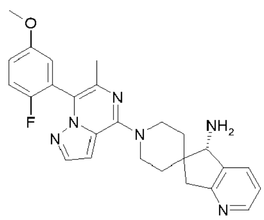

Step a: A mixture of **intermediate D-1** (200 mg, 0.37 mmol), (2-fluoro-5-methoxy-phenyl)boronic acid (94 mg, 0.55 mmol), $K_3PO_4$ (171 mg, 0.81 mmol) and XPhos (17.5 mg, 0.037 mmol) in a mixture of THF (6.2 mL) and water (1.1 mL) was degassed with Ar for 5 min, XPhos Pd G4 (32 mg, 0.037 mmol) was then added and the mixture was stirred at 80 °C for 2 h. Water (10 mL) was added and the aqueous layer was extracted with EtOAc (10 mL * 2). The combined organic layers were washed with brine (10 mL), dried over $Na_2SO_4$ and filtered and concentrated under reduced pressure. The residue was purified by column chromatography ($SiO_2$, cyclohexane/ethyl acetate 1/0 to 0/1 followed by ethyl acetate/methanol = 1/0 to 9/1) to give (R)-N-[(5S)-1'-[7-(2-fluoro-5-methoxy-phenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (146 mg, 0.26 mmol) as an off-white foam. LCMS m/z $[M+H]^+$ = 563.1.

**Step b:** To a solution of (R)-N-[(5S)-1'-[7-(2-fluoro-5-methoxy-phenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (146 mg, 0.26 mmol) in dichloromethane (2.6 mL) was added a solution of HCl (4 N in dioxane, 0.39 mL, 1.56 mmol) at RT. The mixture was stirred for 45 min, dichloromethane was added and the precipitate was filtered and washed with dichloromethane. The solid was taken up in water and the pH value of the mixture was adjusted to 11-12 with 1 N aqueous solution of NaOH. The

solid was filtered and washed with water to give (5S)-1'-[7-(2-fluoro-5-methoxy-phenyl)-6-methyl-pyrazolo[1,5-a]pyrazin-4-yl]spiro[5,7-dihydrocyclopenta[b]pyridine-6,4'-piperidine]-5-amine (111 mg, 0.24 mmol) as a white solid. LCMS m/z $[M+H]^+$ = 459.2. $^1$H NMR (400 MHz, DMSO-*d6*) δ 1.22 (br d, *J* = 14 Hz, 1 H), 1.61 (br d, *J* = 14 Hz, 1 H), 1.77 - 1.98 (m, 4H), 2.15 (s, 3 H), 2.79 (d, *J* = 16 Hz, 1 H), 3.15 (d, *J* = 16 Hz, 1 H), 3.32 - 3.44 (m, 2 H), 3.77 (s, 3 H), 3.92 (s, 1 H), 4.38 (tt, *J* = 9, 4 Hz, 2 H), 6.97 (d, *J* = 2 Hz, 1 H), 7.03 (dd, *J* = 6, 3 Hz, 1 H), 7.08 (m, 1 H), 7.18 (dd, *J* = 7, 5 Hz, 1 H), 7.27 (t, *J* = 9 Hz, 1 H), 7.66 (d, *J* = 7 Hz, 1 H), 7.84 (d, *J* = 3 Hz, 1 H), 8.32 (d, *J* = 5 Hz, 1 H). SHP2 $IC_{50}$ is 13 nM.

**Example S4a. Compounds 32-61**

The following compounds listed in Table 5 were made using the procedure or modifications to the procedure as exemplified for **Example S4** (Compound **31**) using appropriate starting materials and intermediates.

**Table 5.**

| Cpd. No. | Name | Characterization | SHP2 $IC_{50}$ (nM) |
|---|---|---|---|
| **32** | (5S)-1'-[7-(3-methoxyphenyl)-6-methyl-pyrazolo[1,5-a] pyrazin-4-yl]spiro[5,7-dihydrocyclopenta[b]pyri dine-6,4'-piperidine]-5-amine | $^1$H NMR (400 MHz, DMSO-*d6*) δ ppm 1.21 (br d, *J* = 13 Hz, 1 H), 1.61 (br d, *J* = 12 Hz, 1 H), 1.77 - 1.97 (m, 4 H), 2.17 (s, 3 H), 2.78 (d, *J* = 16 Hz, 1 H), 3.14 (d, *J* = 16 Hz, 1 H), 3.33 - 3.41 (m, 2 H), 3.78 (s, 3 H), 3.92 (s, 1 H), 4.33 (tt, *J* = 9, 4 Hz, 2 H), 6.94 *(d, J*= 3 Hz, 1 H), 6.98 - 7.05 (m, 3 H), 7.17 (dd, *J* = 8, 5 Hz, 1 H), 7.41 (t, *J* = 8 Hz, 1 H), 7.66 (dt, *J* = 7, 1 Hz, 1 H), 7.83 (d, *J* = 2 Hz, 1 H), 8.32 (dd, *J* = 5, 1 Hz, 1 H). LCMS m/z $[M+H]^+$ = 441.2 | 6 |
| **33** | (5S)-1'-[6-methyl-7-(1,3,5-trimethylpyrazol-4-yl)pyrazolo [1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.23 (br d, *J* = 11 Hz, 1 H), 1.61 (br d, *J*= 13 Hz, 1 H), 1.93 (d, *J* = 45 Hz, 10 H), 2.11 (s, 3 H), 2.78 (d, *J*= 16 Hz, 1 H), 3.13 (d, *J*= 16 Hz, 1 H), 3.33 - 3.47 (m, 2 H), 3.74 (s, 3 H), 3.93 (s, 1 H), 4.33 (td, *J* = 9, 4 Hz, 2 H), 6.91 (d, *J* = 2 Hz, 1 H), 7.18 (dd, *J* = 8, 5 Hz, 1 H), 7.67 (d, *J* = 8 Hz, 1 H), 7.82 (d, *J* = 2 Hz, 1 H), 8.32 (d, *J* = 4 Hz, 1 H). LCMS m/z $[M+H]^+$ = 443.2 | > 1000 |
| **34** | (5S)-1'-[7-(5-fluoro-2,3-dihydro-1,4-benzodioxin-6-yl)-6-methyl-pyrazolo[1,5-a] pyrazin-4-yl]spiro[5,7-dihydrocyclopenta[b]pyri dine-6,4'-piperidine]-5-amine | $^1$H NMR (400 MHz, DMSO-*d6*) δ ppm 1.22 (br d, *J* = 15 Hz, 1 H), 1.61 (br d, *J* = 13 Hz, 1 H), 1.78 - 2.03 (m, 4 H), 2.14 (s, 3 H), 2.78 (d, *J* = 16 Hz, 1 H), 3.14 (m, 1 H), 3.33 - 3.42 (m, 2 H), 3.92 (s, 1 H), 4.33 - 4.40 (m, 6 H), 6.80 - 6.90 (m, 2 H), 6.96 (d, *J* = 2 Hz, 1 H), 7.17 (dd, *J* = 7, 5 Hz, 1 H), 7.66 (d, *J* = 7 Hz, 1 H), 7.83 (d, *J* = 2 Hz, 1 H), 8.32 (d, *J* = 4 Hz, 1 H). LCMS m/z $[M+H]^+$ = 487.2 | 3 |
| **35** | [(5S)-5-amino-1'-[7-(2-fluorophenyl)-6-methyl-pyrazolo[1,5-a]pyrazin-4-yl] spiro[5,7-dihydrocyclopenta[c] pyridine-6,4'-piperidine]-1-yl] methanol | $^1$H NMR (400 MHz, DMSO-*d6*) δ ppm 1.16 (br d, *J* = 13 Hz, 1 H), 1.63 (br d, *J* = 13 Hz, 1 H), 1.79 (t, *J* = 12 Hz, 1 H), 1.88 - 2.09 (m, 3 H), 2.14 (s, 3 H), 2.72 (br d, *J* = 16 Hz, 1 H), 3.20 - 3.47 (m hidden, 3 H), 3.90 (m, 1 H), 4.31 - 4.47 (m, 2 H), 4.51 - 4.64 (m, 2 H), 5.05 (t, *J* = 6 Hz, 1 H), 6.98 (d, *J* = 2 Hz, 1 H), 7.26 (d, *J* = 5 Hz, 1 H), 7.31 - 7.39 (m, 2 H), 7.44 - 7.61 (m, 2 H), 7.84 (d, *J* = 2 Hz, 1 H), 8.34 (d, *J* = 5 Hz, 1 H). LCMS m/z $[M+H]^+$ = 459.1 | 8 |
| **36** | (5S)-1'-[2-(difluoromethyl)-7-(1,5-dimethylpyrazol-4-yl)-6-methyl-pyrazolo[1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.22 (br d, *J* = 13 Hz, 1 H), 1.61 (br d, *J* = 13 Hz, 1 H), 1.78 - 1.98 (m, 4 H), 2.06 (s, 3 H), 2.19 (s, 3 H), 2.78 (d, *J* = 16 Hz, 1 H), 3.14 (d, *J* = 16 Hz, 1 H), 3.31 - 3.41 (m, 2 H), 3.83 (s, 3 H), 3.92 (s, 1 H), 4.27 - 4.40 (m, 2 H), 7.15 (t, *J*= 54 Hz, 1 H), 7.14 - 7.23 (m, 2 H), 7.47 (s, 1 H), 7.66 (d, *J* = 7 Hz, 1 H), 8.32 (d, *J* = 5 Hz, 1 H). LCMS m/z $[M+H]^+$ = 479.2 | 228 |

(continued)

| Cpd. No. | Name | Characterization | SHP2 $IC_{50}$ (nM) |
|---|---|---|---|
| **37** | [4-[(5S)-5-aminospiro[5,7-dihydrocyclopenta[b]pyri dine-6,4'-piperidine]-1'-yl]-7-(2,3-difluorophenyl)-6-methyl-pyrazolo[1,5-a]pyrazin-2-yl] methanol | $^1$H NMR (400 MHz, DMSO-*d6*) δ ppm 1.22 (br d, *J* = 13 Hz, 1 H), 1.62 (br d, *J* = 13 Hz, 1 H), 1.80 - 2.03 (m, 4 H), 2.13 (s, 3 H), 2.79 (d, *J* = 16 Hz, 1 H), 3.15 (br d, *J* = 16 Hz, 1 H), 3.33 - 3.46 (m, 2 H), 3.93 (s, 1 H), 4.35 - 4.45 (m, 2 H), 4.51 (d, *J* = 6 Hz, 2 H), 5.21 (t, *J* = 6 Hz, 1 H), 6.88 (s, 1 H), 7.18 (dd, *J* = 7, 5 Hz, 1 H), 7.28 - 7.41 (m, 2 H), 7.58 (m, 1 H), 7.66 (d, *J* = 7 Hz, 1 H), 8.33 (d, *J* = 5 Hz, 1 H). LCMS m/z $[M+H]^+$ = 477.2 | 9 |
| **38** | (5S)-1'-[7-(3-chloro-2-thienyl)-6-methyl-pyrazolo[1,5-a] pyrazin-4-yl]spiro[5,7-dihydrocyclopenta[b]pyri dine-6,4'-piperidine]-5-amine | $^1$H NMR (500 MHz, DMSO-*d6*) δ ppm 1.24 (s, 1 H), 1.62 (d, *J* = 13 Hz, 1 H), 1.77 - 1.99 (m, 2 H), 2.15 (s, 3 H), 2.79 (d, *J* = 16 Hz, 1 H), 3.15 (d, *J* = 16 Hz, 1 H), 3.34 - 3.48 (m, 2 H), 3.93 (s, 1 H), 4.37 - 4.51 (m, 2 H), 7.02 (d, *J* = 2 Hz, 1 H), 7.18 (dd, *J* = 7, 5 Hz, 1 H), 7.24 (d, *J* = 5 Hz, 1 H), 7.66 (dt, *J* = 8, 1 Hz, 1 H), 7.87 (d, *J* = 2 Hz, 1 H), 7.94 (d, *J* = 6 Hz, 1 H), 8.32 (m, 1 H) LCMS m/z $[M+H]^+$ = 451.1 | 12 |
| **39** | (5S)-1'-(6-methyl-7-phenyl-pyrazolo[1,5-a]pyrazin-4-yl) spiro[5,7-dihydrocyclopenta[b] pyri dine-6,4'-piperidine]-5-amine | $^1$H NMR (500 MHz, DMSO-*d6*) δ ppm 1.22 (br d, *J* = 13 Hz, 1 H), 1.61 (br d, *J* = 13 Hz, 1 H), 1.80 - 1.96 (m, 2 H), 1.98 - 2.08 (m, 2 H), 2.17 (s, 3 H), 2.79 *(d, J*= 16 Hz, 1 H), 3.14 (d, *J* = 16 Hz, 1 H), 3.33 - 3.51 (m, 2 H), 3.93 (s, 1 H), 4.30 - 4.40 (m, 2 H), 6.95 (d, *J* = 2 Hz, 1 H), 7.18 (dd, *J* = 7, 5 Hz, 1 H), 7.43 - 7.47 (m, 3 H), 7.47 - 7.52 (m, 2 H), 7.67 (d, *J* = 7 Hz, 1 H), 7.83 (d, *J* = 2 Hz, 1 H), 8.32 (d, *J* = 4 Hz, 1 H). LCMS m/z $[M+H]^+$ = 411.2 | 5 |
| **40** | (5S)-1'-[7-(3-chloro-2-cyclopropyl-4-pyridyl)-6-methyl-pyrazolo[1,5-a]pyrazin-4-yl]spiro[5,7-dihydrocyclopenta[b]pyridine-6,4'-piperidine]-5-amine | $^1$H NMR (400 MHz, DMSO-*d6*) δ ppm 1.02 - 1.15 (m, 4 H), 1.23 (br d, *J* = 13 Hz, 1 H), 1.63 (br d, *J* = 13 Hz, 1 H), 1.77 - 1.99 (m, 4H), 2.08 (s, 3 H), 2.59 (m, 1 H), 2.79 (br d, *J* = 16 Hz, 1 H), 3.15 (d, *J* = 16 Hz, 1 H), 3.34 - 3.46 (m, 2 H), 3.93 (s, 1 H), 4.34 - 4.50 (m, 2 H), 7.01 (t, *J* = 2 Hz, 1 H), 7.18 (dd, *J* = 7, 5 Hz, 1 H), 7.31 (d, *J*= 5 Hz, 1 H), 7.67 (d, *J* = 7 Hz, 1 H), 7.85 (d, *J* = 2 Hz, 1 H), 8.32 (d, *J* = 4 Hz, 1 H), 8.50 (d, *J* = 5 Hz, 1 H). LCMS m/z $[M+H]^+$ = 486.2 | 10 |
| **41** | (5S)-1'-[7-[3-fluoro-2-(trifluoromethyl)-4-pyridyl]-6-methyl-pyrazolo[1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.24 (br d, *J* = 13 Hz, 1 H), 1.64 (br d, *J* = 13 Hz, 1 H), 1.73 - 2.11 (m, 4 H), 2.20 (s, 3 H), 2.81 (d, *J* = 16 Hz, 1 H), 3.17 (d, *J* = 16 Hz, 1 H), 3.39 - 3.52 (m, 2 H), 3.94 (s, 1 H), 4.43 - 4.55 (m, 2 H), 7.08 (s, 1 H), 7.19 (dd, *J* = 7, 5 Hz, 1 H), 7.67 (d, *J* = 7 Hz, 1 H), 7.92 (d, *J* = 3 Hz, 1 H), 8.07 (t, *J* = 5 Hz, 1 H), 8.33 (d, *J* = 4 Hz, 1 H), 8.76 (d, *J* = 5 Hz, 1 H). LCMS m/z $[M+H]^+$ = 498.2 | 15 |
| **42** | (5S)-1'-[7-(3chloro-2-ethyl-4-pyridyl)-6-methyl-pyrazolo[1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.24 (br d, *J* = 13 Hz, 1 H), 1.31 (t, *J* = 7 Hz, 3 H), 1.64 (br d, *J* = 13 Hz, 1 H), 1.79 - 1.99 (m, 4 H), 2.07 (s, 3 H), 2.80 (d, *J* = 16 Hz, 1 H), 3.00 (q, *J* = 8 Hz, 2 H), 3.16 (d, *J* = 16 Hz, 1 H), 3.35 - 3.47 (m, 2 H), 3.94 (s, 1 H), 4.43 (ddd, *J* = 13, 9, 4 Hz, 2 H), 7.02 (m, 1 H), 7.19 (dd, *J* = 7, 5 Hz, 1 H), 7.42 (d, *J* = 5 Hz, 1 H), 7.67 (d, *J* = 7 Hz, 1 H), 7.85 (d, *J* = 2 Hz, 1 H), 8.33 (d, *J* = 4 Hz, 1 H), 8.60 (d, *J* = 5 Hz, 1 H). LCMS m/z $[M+H]^+$ = 474.2 | 3 |

(continued)

| Cpd. No. | Name | Characterization | SHP2 $IC_{50}$ (nM) |
|---|---|---|---|
| **43** | (5S)-1'-[7-(3-chloro-2-methyl-4-pyridyl)-6-methyl-pyrazolo [1,5-a]pyrazin-4-yl]-3-methyl-spiro[5,7-dihydrocyclopenta[b] pyri dine-6,4'-piperidine]-5-amine | $^1$H NMR (400 MHz, DMSO-*d6*) δ ppm 1.23 (br d, *J* = 13 Hz, 1 H), 1.62 (br d, *J* = 13 Hz, 1 H), 1.75 - 2.00 (m, 4 H), 2.08 (s, 3 H), 2.28 (s, 3 H), 2.66 (s, 3 H), 2.73 (br d, *J* = 16 Hz, 1 H), 3.10 (d, *J* = 16 Hz, 1 H), 3.35 - 3.49 (m, 2 H), 3.90 (s, 1 H), 4.42 (t, *J* = 9 Hz, 2 H), 7.01 (s, 1 H), 7.42 (d, *J* = 5 Hz, 1 H), 7.49 (s, 1 H), 7.85 (d, *J* = 2 Hz, 1 H), 8.16 (s, 1 H), 8.55 (d, *J* = 5 Hz, 1 H). LCMS m/z $[M+H]^+$ = 474.1 | 4 |
| **44** | (5S)-1'-[7-(2,5-dimethylpyrazol-3-yl)-6-methyl-pyrazolo[1,5-a]pyrazin-4-yl] spiro[5,7-dihydrocyclopenta[b] pyri dine-6,4'-piperidine]-5-amine | $^1$H NMR (400 MHz, DMSO-*d6*) δ ppm 1.24 (br d, *J* = 12 Hz, 1 H), 1.62 (br d, *J* = 13 Hz, 1 H), 1.79 - 1.94 (m, 2 H), 2.16 (s, 3 H), 2.23 (s, 3 H), 2.22 - 2.32 (m, 2 H), 2.80 (d, *J* = 16 Hz, 1 H), 3.16 (d, *J*= 16 Hz, 1 H), 3.34 - 3.44 (m, 2 H), 3.46 (s, 3 H), 3.94 (s, 1 H), 4.44 (t, *J* = 9 Hz, 2 H), 6.21 (s, 1 H), 7.02 (dd, *J* = 3, 1 Hz, 1 H), 7.19 (dd, *J* = 7, 5 Hz, 1 H), 7.68 (d, *J* = 7 Hz, 1 H), 7.89 (d, *J* = 2 Hz, 1 H), 8.33 (d, *J* = 4 Hz, 1 H). LCMS m/z $[M+H]^+$ = 429.2 | 15 |
| **45** | (5S)-1'-[7-(3-cldoro-2-methyl-4-pyridyl)-6-methyl-pyrazolo [1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.23 (br d, *J* = 13 Hz, 1 H), 1.63 (br d, *J* = 13 Hz, 1 H), 1.72 - 1.98 (m, 4 H), 2.07 (s, 3 H), 2.65 (s, 3 H), 2.79 (d, *J* = 16 Hz, 1 H), 3.15 (d, *J* = 16 Hz, 1 H), 3.32 - 3.47 (m, 2 H), 3.93 (s, 1 H), 4.42 (td, *J* = 11, 4 Hz, 2 H), 7.01 (dd, *J* = 3, 2 Hz, 1 H), 7.18 (dd, *J* = 8, 5 Hz, 1 H), 7.41 (d, J = 5 Hz, 1 H), 7.59 - 7.74 (m, 1 H), 7.84 (d, *J* = 2 Hz, 1 H), 8.32 (dd, *J* = 5, 1 Hz, 1 H), 8.54 (d, *J* = 5 Hz, 1 H). LCMS m/z $[M+H]^+$ = 460.1 | 8 |
| **46** | 4-[4-[(5S)-5-aminospiro[5,7-dihydrocyclopenta[b]pyri dine-6,4'-piperidine]-1'-yl]-6-methyl-pyrazolo[1,5-a]pyrazin-7-yl] thiophene-3-carbonitrile | $^1$H NMR (400 MHz, DMSO-*d6*) δ ppm 1.24 (br d, *J* = 14 Hz, 1 H), 1.63 (br d, *J* = 13 Hz, 1 H), 1.75 - 2.05 (m, 4 H), 2.20 (s, 3 H), 2.80 (d, *J* = 16 Hz, 1 H), 3.16 (d, *J* = 16 Hz, 1 H), 3.34 - 3.49 (m, 2 H), 3.94 (s, 1 H), 4.44 (td, *J* = 9, 4 Hz, 2 H), 7.02 (d, *J* = 3 Hz, 1 H), 7.18 (dd, *J* = 8, 5 Hz, 1 H), 7.67 (d, J = 7 Hz, 1 H), 7.89 (d, J = 3 Hz, 1 H), 8.00 (d, *J* = 3 Hz, 1 H), 8.33 (dd, *J* = 5, 1 Hz, 1 H), 8.73 (d, *J* = 3 Hz, 1 H). LCMS m/z $[M+H]^+$ = 442.1 | 73 |
| **47** | (5S)-1'-[7-(1,3-dimethy lpyrazol-4-yl)-6-methy l-pyrazolo[1,5-a]pyrazin-4-yl] spiro [5,7-dihydrocyclopenta[b] pyri dine-6,4'-piperidine]-5-amine | $^1$H NMR (400 MHz, DMSO-*d6*) δ ppm 1.21 (br d, *J* = 13 Hz, 1 H), 1.60 (br d, *J* = 13 Hz, 1 H), 1.76 - 1.94 (m, 4 H), 1.94 (s, 3 H), 2.17 (s, 3 H), 2.77 (d, *J* = 16 Hz, 1 H), 3.13 (d, *J* = 16 Hz, 1 H), 3.27 - 3.30 (m hidden, 2 H), 3.84 (s, 3 H), 3.92 (s, 1 H), 4.31 (td, *J* = 9, 4 Hz, 2 H), 6.92 (d, *J* = 3 Hz, 1 H), 7.17 *(dd, J*= 7, 5 Hz, 1 H), 7.66 (dt, *J* = 7, 1 Hz, 1 H), 7.77 (s, 1 H), 7.84 (d, *J* = 3 Hz, 1 H), 8.32 (dd, *J* = 5, 1 Hz, 1 H). LCMS m/z $[M+H]^+$ = 429.2 | 154 |
| **48** | (5S)-1'-[7-(1-ethylpyrazol-4-yl)-6-methyl-pyrazolo[1,5-a] pyrazin-4-yl]spiro[5,7-dihydrocyclopenta[b]pyri dine-6,4'-piperidine]-5-amine | $^1$H NMR (400 MHz, DMSO-*d6*) δ ppm 1.20 (br d, *J* = 13 Hz, 1 H), 1.44 (t, *J* = 7 Hz, 3 H), 1.59 (br d, *J* = 13 Hz, 1 H), 1.73 - 1.97 (m, 4 H), 2.38 (s, 3 H), 2.77 (d, *J* = 16 Hz, 1 H), 3.13 (d, *J* = 16 Hz, 1 H), 3.22 - 3.27 (m hidden, 2 H), 3.91 (s, 1 H), 4.22 (q, *J* = 7 Hz, 2 H), 4.26 - 4.36 (m, 2 H), 6.94 (d, *J* = 3 Hz, 1 H), 7.17 (dd, *J* = 7, 5 Hz, 1 H), 7.66 (dt, *J* = 7, 1 Hz, 1 H), 7.81 (d, *J* = 1 Hz, 1 H), 7.92 (d, *J* = 2 Hz, 1 H), 8.18 (d, *J* = 1 Hz, 1 H), 8.31 (d, *J* = 5 Hz, 1 H). LCMS m/z $[M+H]^+$ = 429.2 | 17 |

(continued)

| Cpd. No. | Name | Characterization | SHP2 $IC_{50}$ (nM) |
|---|---|---|---|
| **49** | (5S)-1'-[6-methyl-7-[2-(trifluoromethyl)-4-pyridyl] pyrazolo[1,5-a]pyrazin-4-yl] spiro[5,7-dihydrocyclopenta[b] pyri dine-6,4'-piperidine]-5-amine | $^1$H NMR (400 MHz, DMSO-*d6*) δ ppm 1.22 (br d, *J* = 13 Hz, 1 H), 1.62 (br d, *J* = 13 Hz, 1 H), 1.75 - 1.98 (m, 4 H), 2.23 (s, 3 H), 2.79 (d, *J* = 16 Hz, 1 H), 3.16 (d, *J* = 16 Hz, 1 H), 3.33 - 3.49 (m, 2 H), 3.92 (s, 1 H), 4.38 - 4.52 (m, 2 H), 7.05 (d, *J* = 2 Hz, 1 H), 7.18 (dd, *J* = 8, 5 Hz, 1 H), 7.66 (dt, *J* = 7, 1 Hz, 1 H), 7.87 - 7.93 (m, 2 H), 8.10 (dd, *J* = 2, 1 Hz, 1 H), 8.32 (d, *J* = 4 Hz, 1 H), 8.90 (d, *J* = 5 Hz, 1 H). LCMS m/z $[M+H]^+$ = 480.3 | 130 |
| **50** | (5S)-1'-[6-methyl-7-(1-methylindol-2-yl)pyrazolo[1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.24 (br d, *J* = 13 Hz, 1 H), 1.64 (br d, *J* = 13 Hz, 1 H), 1.79 - 1.98 (m, 4 H), 2.22 (s, 3 H), 2.80 (d, *J* = 16 Hz, 1 H), 3.16 (d, *J* = 16 Hz, 1 H), 3.35 - 3.47 (m, 2 H), 3.43 (s, 3 H), 3.93 (s, 1 H), 4.39 - 4.52 (m, 2 H), 6.62 (s, 1 H), 7.02 (dd, *J* = 2, 2 Hz, 1 H), 7.09 (m, 1 H), 7.18 (dd, *J* = 8, 5 Hz, 1 H), 7.24 (m, 1 H), 7.51 (dd, *J* = 8, 1 Hz, 1 H), 7.61 (d, *J* = 8 Hz, 1 H), 7.67 (dt, *J* = 7, 1 Hz, 1 H), 7.86 (d, *J* = 3 Hz, 1 H), 8.36 (m, 1 H). LCMS m/z $[M+H]^+$ = 464.2 | 10 |
| **51** | (5S)-1'-[7-(2-fluorophenyl)-6-methyl-pyrazolo[1,5-a]pyrazin-4-yl]spiro[5,7-dihydrocyclopenta[b]pyri dine-6,4'-piperidine]-5-amine | $^1$H NMR (400 MHz, DMSO-*d6*) δ ppm 1.23 (br d, *J* = 13 Hz, 1 H), 1.62 (br d, *J* = 14 Hz, 1 H), 1.77 - 2.07 (m, 4 H), 2.14 (s, 3 H), 2.80 (d, *J* = 16 Hz, 1 H), 3.16 (d, *J* = 16 Hz, 1 H), 3.32 - 3.46 (m, 2 H), 3.94 (s, 1 H), 4.40 (td, *J* = 9, 5 Hz, 2 H), 6.98 (d, *J* = 3 Hz, 1 H), 7.18 (dd, *J* = 8, 5 Hz, 1 H), 7.32 - 7.40 (m, 2 H), 7.46 - 7.60 (m, 2 H), 7.67 (d, *J* = 7 Hz, 1 H), 7.84 (d, *J* = 2 Hz, 1 H), 8.33 (d, *J* = 5 Hz, 1 H). LCMS m/z $[M+H]^+$ = 429.2 | 5 |
| **52** | (5S)-1'-[6-methyl-7-(o-tolyl) pyrazolo[1,5-a]pyrazin-4-yl] spiro[5,7-dihydrocyclopenta[b] pyri dine-6,4'-piperidine]-5-amine | $^1$H NMR (400 MHz, DMSO-*d6*) δ ppm 1.23 (br d, *J* = 13 Hz, 1 H), 1.62 (br d, *J* = 14 Hz, 1 H), 1.73 - 2.01 (m, 7 H), 2.05 (s, 3 H), 2.79 (d, *J* = 16 Hz, 1 H), 3.14 (d, *J* = 16 Hz, 1 H), 3.32 - 3.42 (m, 2 H), 3.93 (s, 1 H), 4.35 (ddd, *J* = 13, 9, 5 Hz, 2 H), 6.95 (dd, *J* = 3, 1 Hz, 1 H), 7.18 (dd, *J* = 8, 5 Hz, 1 H), 7.24 (d, *J* = 7 Hz, 1 H), 7.31 (td, *J* = 7, 2 Hz, 1 H), 7.34 - 7.42 (m, 2 H), 7.67 (dt, *J* = 7, 1 Hz, 1 H), 7.82 (d, *J* = 3 Hz, 1 H), 8.32 (dd, *J* = 5, 1 Hz, 1 H). LCMS m/z $[M+H]^+$ = 425.2 | 24 |
| **53** | (5S)-1'-[7-(5-chloro-2-thienyl)-6-methyl-pyrazolo[1,5-a] pyrazin-4-yl]spiro[5,7-dihydrocyclopenta[b]pyri dine-6,4'-piperidine]-5-amine | $^1$H NMR (400 MHz, DMSO-*d6*) δ ppm 1.22 (m, 1 H), 1.60 (br d, *J* = 14, 1 H), 1.73 - 1.93 (m, 2 H), 1.95 - 2.14 (m, 2 H), 2.35 (s, 3 H), 2.78 (d, *J* = 16 Hz, 1 H), 3.14 (d, *J* = 16 Hz, 1 H), 3.32 - 3.48 (m, 2 H), 3.91 (s, 1 H), 4.42 (td, *J* = 9, 4 Hz, 2 H), 7.03 (d, *J* = 2 Hz, 1 H), 7.17 (dd, *J* = 7, 5 Hz, 1 H), 7.22 (q, *J* = 4 Hz, 2 H), 7.65 (d, *J* = 7 Hz, 1 H), 7.94 (d, *J* = 2 Hz, 1 H), 8.32 (d, *J* = 5 Hz, 1 H). LCMS m/z $[M+H]^+$ = 451.1 | 3 |
| **54** | (5S)-1'-[7-(2-chloro-3-thienyl)-6-methyl-pyrazolo[1,5-a] pyrazin-4-yl]spiro[5,7-dihydrocyclopenta[b]pyri dine-6,4'-piperidine]-5-amine | $^1$H NMR (400 MHz, DMSO-*d6*) δ ppm 1.22 (d, *J* = 14 Hz, 1 H), 1.61 (br d, *J* = 13 Hz, 1 H), 1.77 - 2.05 (m, 4 H), 2.14 (s, 3 H), 2.78 (d, *J* = 16 Hz, 1 H), 3.14 (d, *J* = 16 Hz, 1 H), 3.31 - 3.45 (m, 2 H), 3.92 (s, 1 H), 4.32 - 4.46 (m, 2 H), 6.98 (dd, *J* = 3, 1 Hz, 1 H), 7.13 (dd, *J* = 6, 1 Hz, 1 H), 7.17 (dd, *J* = 8, 5 Hz, 1 H), 7.60 (d, *J* = 6 Hz, 1 H), 7.66 (dt, *J* = 8, 2 Hz, 1 H), 7.86 (d, *J* = 3 Hz, 1 H), 8.32 (dd, *J* = 5, 1 Hz, 1 H). LCMS m/z $[M+H]^+$ = 451.1 | 4 |

(continued)

| Cpd. No. | Name | Characterization | SHP2 $IC_{50}$ (nM) |
|---|---|---|---|
| **55** | (5S)-3-(difluoromethyl)-1'-[7-(1,5-dimethy lpyrazol-4-yl)-6-methy l-pyrazolo[1,5-a] pyrazin-4-yl]spiro [5,7-dihydrocyclopenta[b]pyri dine-6,4'-piperidine]-5-amine | $^1$H NMR (400 MHz, DMSO-*d6*) δ ppm 1.19 (br d, *J* = 12 Hz, 1 H), 1.64 (m, 1 H), 1.83 (m, 1 H), 1.93 (m, 1 H), 2.06 (s, 3 H), 2.18 (s, 3 H), 2.84 (br d, *J* = 17 Hz, 1 H), 3.21 (br d, *J* = 17 Hz, 1 H), 3.38 (m hidden, 2 H), 3.82 (s, 3 H), 3.98 (s, 1 H), 4.26 - 4.38 (m, 2 H), 6.92 (d, *J* = 2 Hz, 1 H), 7.12 (t, *J* = 55 Hz, 1 H), 7.45 (s, 1 H), 7.84 (d, *J* = 3 Hz, 1 H), 7.88 (m, 1 H), 8.53 (d, *J* = 1 Hz, 1 H). LCMS m/z $[M+H]^+$ = 479.3 | 30 |
| **56** | (5S)-1'-[7-(2-fluorophenyl)-6-methyl-pyrazolo[1,5-a]pyrazin-4-yl]-2-methoxy-spiro[5,7-dihydrocyclopenta[b]pyri dine-6,4'-piperidine]-5-amine | $^1$H NMR (400 MHz, DMSO-*d6*) δ ppm 1.34 (br d, *J* = 13 Hz, 1 H), 1.59 (br d, *J* = 14 Hz, 1 H), 1.73 - 1.93 (m, 3 H), 2.14 (s, 3 H), 2.74 (d, *J* = 17 Hz, 1 H), 2.99 - 3.07 (m, 1 H), 3.35 - 3.57 (m, 2 H), 3.83 (s, 3 H), 4.36 (br d, *J* = 14 Hz, 2 H), 6.60 (d, *J* = 8 Hz, 1 H), 6.98 (d, *J* = 2 Hz, 1 H), 7.31 - 7.39 (m, 2 H), 7.47 - 7.65 (m, 3 H), 7.84 (d, *J* = 3 Hz, 1 H). LCMS m/z $[M+H]^+$ = 459.3 | 17 |
| **57** | (5S)-1'-[6-methyl-7-(2,3,4,5,6-pentadeuteriophenyl)pyra zolo [1,5-a]pyrazin-4-yl]spiro[5,7-dihydrocyclopenta[b]pyri dine-6,4'-piperidine]-5-amine | $^1$H NMR (500 MHz, DMSO-*d6*) δ ppm 1.28 (br d, *J* = 13 Hz, 1 H), 1.61 (br d, J = 13 Hz, 1 H), 1.80 - 1.96 (m, 2 H), 1.98 - 2.08 (m, 2 H), 2.17 (s, 3 H), 2.84 (d, *J* = 16 Hz, 1 H), 3.16 (d, *J* = 16 Hz, 1 H), 3.30 - 3.46 (m, 2 H, partially masked by water), 4.00 (s, 1 H), 4.30 - 4.40 (m, 2 H), 6.96 (d, *J* = 2 Hz, 1 H), 7.18 (dd, *J* = 7, 5 Hz, 1 H), 7.67 (br d, *J* = 7 Hz, 1 H), 7.83 (d, *J* = 2 Hz, 1 H), 8.35 (dd, *J* = 5, 1 Hz, 1 H). LCMS m/z $[M+H]^+$ = 416.3 | 10 |
| **58** | (5S)-1'-[7-(6-fluoro-2,3-dihydro-1,4-benzodioxin-7-yl)-6-methyl-pyrazolo[1,5-a] pyrazin-4-yl]spiro[5,7-dihydrocyclopenta[b]pyri dine-6,4'-piperidine]-5-amine | $^1$H NMR (400 MHz, DMSO-*d6*) δ ppm 1.19 (m, 1 H), 1.61 (br d, *J* = 12 Hz, 1 H), 1.79 - 1.96 (m, 3 H), 2.14 (s, 3 H), 2.78 (d, *J* = 16 Hz, 1 H), 3.14 (d, *J* = 16 Hz, 1 H), 3.32 - 3.49 (m hidden, 2 H), 4.23 - 4.41 (m, 6 H), 6.89 - 6.99 (m, 3 H), 7.17 (dd, *J* = 8, 5 Hz, 1 H), 7.66 (d, *J* = 7 Hz, 1 H), 7.83 (d, *J* = 2 Hz, 1 H), 8.32 (d, *J* = 5 Hz, 1 H). LCMS m/z $[M+H]^+$ = 497.3 | 35 |
| **59** | [(7S)-7-amino-1'-[7-(2-fluorophenyl)-6-methyl-pyrazolo[1,5-a]pyrazin-4-yl] spiro[5,7-dihydrocyclopenta[c] pyri dine-6,4'-piperidine]-3-yl] methanol | $^1$H NMR (400 MHz, DMSO-*d6*) δ ppm 1.20 - 1.29 (m, 1 H), 1.58 (br d, *J* = 14 Hz, 1 H), 1.77 - 1.92 (m, 2 H), 2.14 (s, 3 H), 2.72 (d, *J* = 16 Hz, 1 H), 3.14 (d, *J* = 16 Hz, 1 H), 3.30 - 3.46 (m, 2 H, partially masked by water), 3.97 (s, 1 H), 4.29 - 4.40 (m, 2 H), 4.54 (d, *J* = 5 Hz, 2 H), 5.30 (t, *J* = 6 Hz, 1 H), 6.98 (d, *J* = 2 Hz, 1 H), 7.32 - 7.39 (m, 3 H), 7.47 - 7.57 (m, 2 H), 7.84 (d, *J* = 2 Hz, 1 H), 8.38 (d, *J* = 5 Hz, 1 H). LCMS m/z $[M+H]^+$ = 459.3 | 11 |
| **60** | (7S)-1'-[7-(2-fluorophenyl)-6-methyl-pyrazolo[1,5-a]pyrazin-4-yl]-3-methyl-spiro[5,7-dihydrocyclopenta[c]pyridine-6,4'-piperidine]-7-amine | $^1$H NMR (400 MHz, DMSO-*d6*) δ ppm 1.22 (br d, *J* = 13 Hz, 1 H), 1.56 (br d, *J* = 13 Hz, 1 H), 1.74 - 1.91 (m, 2 H), 2.13 (s, 3 H), 2.43 (s, 3 H), 2.66 (d, *J* = 17 Hz, 1 H), 3.07 (d, *J*= 16 Hz, 1 H), 3.35 - 3.44 (m, 2 H), 3.93 (s, 1 H), 4.25 - 4.40 (m, 2 H), 6.97 (d, *J* = 2 Hz, 1 H), 7.10 (s, 1 H), 7.28 - 7.40 (m, 2 H), 7.45 - 7.61 (m, 2 H), 7.83 (d, *J* = 3 Hz, 1 H), 8.33 (s, 1 H). LCMS m/z $[M+H]^+$ = 443.3 | 7 |
| **61** | (5S)-2-(difluoromethyl)-1'-[7-(1,5-dimethy lpyrazol-4-yl)-6-methy l-pyrazolo[1,5-a] pyrazin-4-yl]spiro [5,7-dihydrocyclopenta[b]pyri dine-6,4'-piperidine]-5-amine | $^1$H NMR (400 MHz, DMSO-*d6*) δ ppm 1.19 (br d, *J* = 12 Hz, 1 H), 1.62 (br d, *J* = 12 Hz, 1 H), 1.78 - 1.99 (m, 4 H), 2.06 (s, 3 H), 2.18 (s, 3 H), 2.83 (br d, *J* = 17 Hz, 1 H), 3.20 (br d, *J* = 17 Hz, 1 H), 3.30 - 3.42 (m hidden, 2 H), 3.82 (s, 3 H), 3.97 (br s, 1 H), 4.26 - 4.38 (m, 2 H), 6.90 (t, *J* = 55 Hz, 1 H), 6.91 (d, *J* = 2 Hz, 1 H), 7.44 (s, 1 H), 7.50 (d, *J* = 8 Hz, 1 H), 7.82 -7.86 (m, 2 H). LCMS m/z $[M+H]^+$ = 479.3 | 211 |

**Example S5**. **(1S)-1'-[7-(1,5-dimethylpyrazol-4-yl)-6-methyl-pyrazolo[1,5-a]pyrazin-4-yl]-5-methoxy-spiro[indane-2,4'-piperidine]-1-amine** (**Compound 62)**

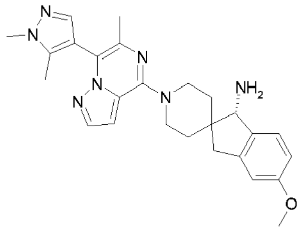

A mixture of **intermediate D-7** (60 mg, 0.14 mmol), 1,5-dimethyl-4-(4,4,5,5-tetramethyl-1,3,2-dioxaborolan-2-yl)-1H-pyrazole (45 mg, 0.20 mmol), $K_3PO_4$ (65 mg, 31 mmol) and XPhos (4 mg, 8.4 μmol) in a mixture of THF (2 mL) and water (0.5 mL) was degassed with Ar for 5 min, XPhos Pd G4 (6 mg, 7 μmol) was then added and the mixture was stirred at 80 °C for 2 h. Water (20 mL) was added and the aqueous layer was extracted with EtOAc (20 mL * 2). The combined organic layers were washed with brine (20 mL), dried over $Na_2SO_4$ and filtered and concentrated under reduced pressure. The residue was purified by column chromatography ($SiO_2$, DCM/methanol = 1/0 to 9/1) to give (1S)-1'-[7-(1,5-dimethylpyrazol-4-yl)-6-methyl-pyrazolo[1,5-a]pyrazin-4-yl]-5-methoxy-spiro[indane-2,4'-piperidine]-1-amine (24 mg, 0.05 mmol) as an orange solid. LCMS m/z $[M+H]^+$ = 458.2. $^1H$ NMR (400 MHz, DMSO-*d6*) δ 1.22 (br d, *J* = 12 Hz, 1 H), 1.55 (br d, *J* = 13 Hz, 1 H), 1.71 - 1.95 (m, 2 H), 2.05 (s, 3 H), 2.17 (s, 3 H), 2.63 (d, *J* = 16 Hz, 1 H), 3.04 (d, *J* = 16 Hz, 1 H), 3.23 - 3.27 (m hidden, 2 H), 3.73 (s, 3 H), 3.80 (s, 1 H), 3.82 (s, 3 H), 4.27 (td, *J* = 9, 4 Hz, 2 H), 6.73 (dd, *J* = 8, 2 Hz, 1 H), 6.76 - 6.80 (m, 1 H), 6.91 (d, *J* = 2 Hz, 1 H), 7.20 (d, *J* = 8 Hz, 1 H), 7.44 (s, 1 H), 7.83 (d, *J* = 2 Hz, 1 H). SHP2 $IC_{50}$ is 17 nM.

**Example S6. (5S)-1'-[7-(2-fluorophenyl)-6-methyl-pyrazolo[1,5-a]pyrazin-4-yl]spiro[5,7-dihydrocyclopenta[b]pyrazine-6,4'-piperidine]-5-amine** (Compound 63)

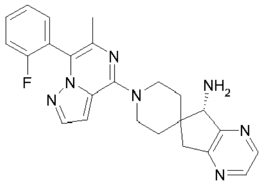

As described for **Example S5** (Compound **62)**, a similar procedure was performed using **intermediate D-30** (45 mg, 0.11 mmol) and 2-fluorophenylboronic acid (21 mg, 0.15 mmol) to give (5S)-1'-[7-(2-fluorophenyl)-6-methyl-pyrazolo[1,5-a]pyrazin-4-yl]spiro[5,7-dihydrocyclopenta[b]pyrazine-6,4'-piperidine]-5-amine (16 mg, 0.04 mmol) as an off-white solid. LCMS m/z $[M+H]^+$ = 430.3. $^1H$ NMR (400 MHz, DMSO-*d6*) δ ppm 1.27 (br d, *J* = 13 Hz, 1 H), 1.67 (br d, *J* = 13 Hz, 1 H), 1.84 - 2.04 (m, 4 H), 2.15 (s, 3 H), 2.87 (d, *J* = 17 Hz, 1 H), 3.20 (d, *J* = 16 Hz, 1 H), 3.43 - 3.54 (m, 2 H), 4.00 (s, 1 H), 4.26 - 4.38 (m, 2 H), 6.99 (dd, *J* = 2, 1 Hz, 1 H), 7.32 - 7.39 (m, 2 H), 7.48 - 7.60 (m, 2 H), 7.84 (d, *J* = 2 Hz, 1 H), 8.37 - 8.41 (m, 2 H). SHP2 $IC_{50}$ is 112 nM.

Example S7. **(1S)-4-Fluoro-1'-[7-(2-fluorophenyl)-6-methyl-pyrazolo[1,5-a]pyrazin-4-yl]spiro[indane-2,4'-piperidine]-1-amine hydrochloride (Compound 64)**

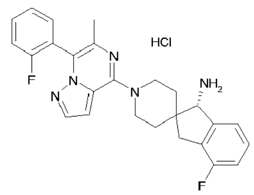

**Step a**: A mixture of **intermediate D-34** (125 mg, 0.205 mmol), potassium phosphate tribasic (91 mg, 0,431 mmol), XPhos (9.8 mg, 0.0205 mmol) and tetrahydrofuran (4 mL) and water (1 mL) was degassed under Ar for 10 min. After addition of 2-fluorophenylboronic acid (43 mg, 0.308 mmol) and Xphos Pd G4 (17.6 mg, 0.0205 mmol), the reaction medium under Ar was heated under microwave irradiation at 70 °C for 2 h and allowed to cool to RT. The mixture was filtered, and the insoluble material was washed with ethyl acetate. The organic layer was separated, washed with brine (3 × 10 mL), dried over $MgSO_4$, filtered and concentrated under reduced pressure. The residue was purified by column chromatography ($SiO_2$, Heptane/Ethyl acetate= 100/0 to 85/15) to give tert-butyl N-[(1S)-4-fluoro-1'-[7-(2-fluorophenyl)-6-methyl-pyrazolo[1,5-a]pyrazin-4-yl]spiro[indane-2,4'-piperidine]-1-yl]carbamate (98 mg, 0.175 mmol) as a white solid. LCMS m/z $[M+H]^+$ = 546.

**Step b:** A solution of tert-butyl N-[(1S)-4-fluoro-1'-[7-(2-fluorophenyl)-6-methyl-pyrazolo[1,5-a]pyrazin-4-yl]spiro[indane-2,4'-piperidine]-1-yl]carbamate (96 mg, 0.180 mmol) in a solution of HCl (2.5 M in ethanol, 15 mL, 37.5 mmol) was stirred 1 h at RT. The mixture was concentrated under reduced pressure to dryness. The residue was solubilized in methanol and the solution concentrated under reduced pressure to dryness. The operation was repeated once more. The product was triturated in ethyl acetate (5 mL), filtered and the solid was washed with pentane to yield (1S)-4-fluoro-1'-[7-(2-fluorophenyl)-6-methyl-pyrazolo[1,5-a]pyrazin-4-yl]spiro[indane-2,4'-piperidine]-1-amine hydrochloride (76 mg, 0.137 mmol) as a white solid. LCMS m/z $[M+H]^+$ = 446.1. $^1$H NMR (400 MHz, DMSO-*d6*) δ ppm 1.62 (br d, *J* = 14 Hz, 1 H), 1.69 (br d, *J* = 12 Hz, 1 H), 1.81 - 1.99 (m, 2 H), 2.16 (s, 3 H), 3.12 (m, 1 H), 3.24 (m, 1 H), 3.40 - 3.51 (m, 2 H), 4.37 - 4.52 (m, 3 H), 7.04 (s, 1 H), 7.22 (t, *J* = 9 Hz, 1 H), 7.30 - 7.46 (m, 4 H), 7.49 (t, *J* = 7 Hz, 1 H), 7.58 (m, 1 H), 7.88 (d, *J* = 2 Hz, 1 H), 8.51 (br s, 3 H). $IC_{50}$ is 46 nM.

**Example S7a. Compounds 65-107**

The following compounds listed in Table 6 were made using the procedure or modifications to the procedure as exemplified for **Example S7** (Compound **64)** using appropriate starting materials and intermediates. Compounds were isolated as their hydrochloride or formate salt.

**Table 6.**

| Cpd. No. | Name | Characterization | SHP2 $IC_{50}$ (nM) |
|---|---|---|---|
| **65** | (4S)-1'-[7-(2,3-difluorophenyl)-6-methyl-pyrazolo[1,5-a]pyrazin-4-yl]spiro[4,6-dihydrocyclopenta[d]thiazole-5,4'-piperidine]-4-amine hydrochloride | As HCl salt: $^1$H NMR (400 MHz, DMSO-*d6*) δ ppm 1.75 (br d, *J* = 14 Hz, 1 H), 1.85 - 2.11 (m, 3 H), 2.19 (s, 3 H), 3.06 - 3.26 (m, 2 H), 3.34 - 3.59 (m, 2 H), 4.34 (m, 1 H), 4.38 - 4.58 (m, 2 H), 7.09 (t, *J* = 2 Hz, 1 H), 7.28 - 7.43 (m, 2 H), 7.60 (dtd, *J* = 10, 8, 8, 2 Hz, 1 H), 7.92 (d, *J* = 3 Hz, 1 H), 8.48 (br d, *J* = 1 Hz, 3 H), 9.14 (s, 1 H). LCMS m/z $[M+H]^+$ = 453.1 | 22 |
| **66** | (4S)-1'-[7-(2-fluorophenyl)-6-methyl-pyrazolo[1,5-a]pyrazin-4-yl]spiro[4,6-dihydrocyclopenta[d]thiazole-5,4'-piperidine]-4-amine hydrochloride | As HCl salt: $^1$H NMR (400 MHz, DMSO-*d6*) δ ppm 1.75 (br d, *J* = 14 Hz, 1 H), 1.85 - 2.12 (m, 3 H), 2.17 (s, 3 H), 3.07 - 3.23 (m, 2 H), 3.32 - 3.55 (m, 2 H), 4.31 - 4.55 (m, 3 H), 7.08 (br s, 1 H), 7.32 - 7.42 (m, 2 H), 7.50 (td, *J* = 8, 2 Hz, 1 H), 7.54 - 7.63 (m, 1 H), 7.90 (d, *J* = 3 Hz, 1 H), 8.47 (br s, 3 H), 9.14 (s, 1 H). LCMS m/z $[M+H]^+$ = 436.1 | 22 |
| **67** | (4S)-1'-[7-(1,5-dimethylpyrazol-4-yl)-6-methyl-pyrazolo[1,5-a]pyrazin-4-yl]spiro[4,6-dihydrocyclopenta[d]thiazole-5,4'-piperidine]-4-amine hydrochloride | As HCl salt: $^1$H NMR (400 MHz, DMSO-*d6*) δ ppm 1.77 (d, *J* = 13 Hz, 1 H), 1.88 - 2.01 (m, 2 H), 2.03 - 2.14 (m, 4 H), 2.23 (s, 3 H), 3.16 (m, 2 H), 3.41 - 3.47 (m hidden, 2 H), 3.83 (s, 3 H), 4.27 - 4.55 (m, 3 H), 7.14 (br s, 1 H), 7.48 (s, 1 H), 7.96 (d, *J* = 3 Hz, 1 H), 8.37 - 8.65 (br s, 3 H), 9.14 (s, 1 H). LCMS m/z $[M+H]^+$ = 436.1 | 115 |

(continued)

| Cpd. No. | Name | Characterization | SHP2 $IC_{50}$ (nM) |
|---|---|---|---|
| **68** | (4S)-1'-(6-methyl-7-phenyl-pyrazolo[1,5-a]pyrazin-4-yl) spiro[4,6-dihydrocyclopenta[d] thiazole-5,4'-piperidine]-4-amine hydrochloride | As HCl salt: $^1$H NMR (400 MHz, DMSO-*d6*) δ ppm 1.76 (br d, *J* = 13 Hz, 1 H), 1.87 - 2.00 (m, 2 H), 2.06 (m, 1 H), 2.21 (s, 3 H), 3.08 - 3.24 (m, 2 H), 3.35 - 3.58 (m, 2 H), 4.30 - 4.50 (m, 3 H), 7.11 (br s, 1 H), 7.40 - 7.58 (m, 5 H), 7.92 (d, *J* = 2 Hz, 1 H), 8.50 (br s, 3 H), 9.14 (s, 1 H). LCMS m/z [M+H]$^+$ = 417.1 | 18 |
| **69** | (4S)-1'-[7-(3-chloro-2-methyl-4-pyridyl)-6-methyl-pyrazolo [1,5-a]pyrazin-4-yl]spiro[4,6-dihydrocyclopenta[d]thiazole-5,4'-piperidine]-4-amine hydrochloride | As HCl salt: $^1$H NMR (400 MHz, DMSO-*d6*) δ ppm 1.76 (br d, *J* = 14 Hz, 1 H), 1.86 - 2.07 (m, 3 H), 2.10 (s, 3 H), 2.68 (s, 3 H), 3.07 - 3.25 (m, 2 H), 3.34 - 3.56 (m, 2 H), 4.34 (m, 1 H), 4.43 (d, *J* = 14 Hz, 1 H), 4.51 (d, *J* = 14 Hz, 1 H), 7.09 (s, 1 H), 7.46 (d, *J* = 5 Hz, 1 H), 7.90 (d, *J* = 2 Hz, 1 H), 8.51 (br s, 3 H), 8.59 (d, *J* = 5 Hz, 1 H), 9.14 (s, 1 H). LCMS m/z [M+H]$^+$ = 466.1 | 10 |
| **70** | (5S)-1'-[6-methyl-7-(4-pyridyl) pyrazolo[1,5-a]pyrazin-4-yl] spiro[5,7-dihydrocyclopenta[b] pyridine-6,4'-piperidine]-5-amine hydrochloride | As HCl salt: $^1$H NMR (400 MHz, DMSO-*d6*) δ ppm 1.65 (br d, *J* = 14 Hz, 1 H), 1.77 (br d, *J* = 13 Hz, 1 H), 1.85 - 2.04 (m, 2 H), 2.32 (s, 3 H), 3.42 - 3.63 (m, 4 H), 4.46 - 4.65 (m, 3 H), 7.14 (d, *J* = 3 Hz, 1 H), 7.51 (m, 1 H), 7.97 (d, *J* = 2 Hz, 1 H), 8.03 - 8.34 (m, 3 H), 8.62 (dd, *J* = 5, 1 Hz, 1 H), 8.73 - 8.89 (m, 3 H), 8.94 (m, 2 H). LCMS m/z [M+H]$^+$ = 412.2 | 73 |
| **71** | (5S)-1'-[7-(3-fluoro-4-pyridyl)-6-methyl-pyrazolo[1,5-a] pyrazin-4-yl]spiro[5,7-dihydrocyclopenta[b]pyridine-6,4'-piperidine]-5-amine hydrochloride | As HCl salt: $^1$H NMR (400 MHz, DMSO-*d6*) δ ppm 1.63 (br d, *J* = 12 Hz, 1 H), 1.71 (br d, *J* = 14 Hz, 1 H), 1.80 - 1.99 (m, 2 H), 2.19 (s, 3 H), 3.20 (br d, *J* = 17 Hz, 1 H), 3.37 (br d, *J* = 17 Hz, 1 H), 3.45 - 3.52 (m, 2 H), 4.37 - 4.66 (m, 3 H), 7.08 (t, *J* = 2 Hz, 1 H), 7.44 (br dd, *J* = 7, 5 Hz, 1 H), 7.65 (t, *J* = 6 Hz, 1 H), 7.92 (d, *J* = 3 Hz, 1 H), 8.10 (d, *J* = 7 Hz, 1 H), 8.50 - 8.68 (m, 5 H), 8.76 (d, *J* = 2 Hz, 1 H). LCMS m/z [M+H]$^+$ = 430.2 | 30 |
| **72** | (6S)-1'-[7-(1,5-dimethylpyrazol-4-yl)-6-methyl-pyrazolo[1,5-a]pyrazin-4-yl] spiro[4,6-dihydrocyclopenta[d] thiazole-5,4'-piperidine]-6-amine hydrochloride | As HCl salt: $^1$H NMR (400 MHz, DMSO-*d6*) δ ppm 1.71 (br d, *J* = 12 Hz, 1 H), 1.84 - 1.99 (m, 2 H), 2.01 - 2.10 (m, 4 H), 2.22 (s, 3 H), 2.93 - 3.14 (m, 2 H), 3.31 - 3.44 (m hidden, 2 H), 3.83 (s, 3 H), 4.34 (m, 1 H), 4.41 - 4.62 (m, 2 H), 7.10 (br s, 1 H), 7.47 (s, 1 H), 7.94 (d, *J* = 3 Hz, 1 H), 8.54 (br s, 3 H), 9.19 (s, 1 H). LCMS m/z [M+H]$^+$ = 436.1 | 27 |
| **73** | (6S)-1'-[7-(3-chloro-2-methyl-4-pyridyl)-6-methyl-pyrazolo [1,5-a]pyrazin-4-yl]spiro[4,6-dihydrocyclopenta[d]thiazole-5,4'-piperidine]-6-amine hydrochloride | As HCl salt: $^1$H NMR (400 MHz, DMSO-*d6*) δ ppm 1.71 (d, *J* = 14 Hz, 1 H), 1.80 - 2.07 (m, 3 H), 2.10 (s, 3 H), 2.68 (s, 3 H), 2.95 - 3.19 (m, 2 H), 3.27 - 3.39 (m, 2 H), 4.37 - 4.66 (m, 3 H), 7.08 (dd, *J* = 2, 1 Hz, 1 H), 7.44 (d, *J* = 5 Hz, 1 H), 7.90 (d, *J* = 3 Hz, 1 H), 8.51 (br s, 3 H), 8.58 (d, *J* = 5 Hz, 1 H), 9.20 (s, 1 H). LCMS m/z [M+H]$^+$ = 466.1 | 5 |
| **74** | (6S)-1'-(6-methyl-7-phenyl-pyrazolo [1,5-a]pyrazin-4-yl) spiro[4,6-dihydrocyclopenta[d] thiazole-5,4'-piperidine] -6-amine hydrochloride | As HCl salt: $^1$H NMR (400 MHz, DMSO-*d6*) δ ppm 1.72 (br d, *J* = 12 Hz, 1 H), 1.86 - 2.01 (m, 2 H), 2.08 (td, *J* = 12, 3 Hz, 1 H), 2.21 (s, 3 H), 3.08 (q, *J* = 16 Hz, 2 H), 3.31 - 3.46 (m, 2 H), 4.35 - 4.59 (m, 3 H), 7.12 (br s, 1 H), 7.40 - 7.59 (m, 5 H), 7.93 (d, *J* = 2 Hz, 1 H), 8.57 (br d, J = 2 Hz, 3 H), 9.19 (s, 1 H). LCMS m/z [M+H]$^+$ = 417.1 | 5 |
| **75** | (6S)-1'-[7-(2-fluorophenyl)-6-methyl-pyrazolo[1,5-a]pyrazin-4-yl]spiro[4,6-dihydrocyclopenta[d]thiazole-5,4'-piperidine]-6-amine hydrochloride | As HCl salt: $^1$H NMR (400 MHz, DMSO-*d6*) δ ppm 1.71 (br d, *J* = 13 Hz, 1 H), 1.81 - 2.12 (m, 3 H), 2.18 (s, 3 H), 2.95 - 3.18 (m, 2 H), 3.29 - 3.42 (m, 2 H), 4.34 - 4.62 (m, 3 H), 7.09 (t, *J* = 2 Hz, 1 H), 7.32 - 7.43 (m, 2 H), 7.51 (td, *J* = 8, 2 Hz, 1 H), 7.60 (m, 1 H), 7.91 (d, *J* = 3 Hz, 1 H), 8.53 (br s, 3 H), 9.19 (s, 1 H). LCMS m/z [M+H]$^+$ = 435.1 | 7 |

(continued)

| Cpd. No. | Name | Characterization | SHP2 $IC_{50}$ (nM) |
|---|---|---|---|
| **76** | (5S)-1'-[7-(5-fluoro-2-methoxy-4-pyridyl)-6-methyl-pyrazolo [1,5-a]pyrazin-4-yl]spiro[5,7-dihydrocyclopenta[b]pyridine-6,4'-piperidine] -5 -amine hydrochloride | As HCl salt: $^1H$ NMR (400 MHz, DMSO-*d6*) δ ppm 1.67 (br d, *J* = 12 Hz, 1 H), 1.78 (br d, *J* = 13 Hz, 1 H), 1.84 - 2.04 (m, 2 H), 2.21 (s, 3 H), 3.28 (d, *J* = 18 Hz, 1 H), 3.38 - 3.54 (m, 3 H), 3.91 (s, 3 H), 4.36 - 4.71 (m, 3 H), 7.05 (dd, *J* = 5, 1 Hz, 1 H), 7.09 (t, *J* = 2 Hz, 1 H), 7.62 (dd, *J* = 8, 5 Hz, 1 H), 7.93 (d, *J* = 3 Hz, 1 H), 8.31 (d, *J* = 1 Hz, 1 H), 8.36 (d, *J* = 8 Hz, 1 H), 8.69 (dd, *J* = 5, 1 Hz, 1 H), 8.85 (br d, *J* = 2 Hz, 3 H). LCMS m/z $[M+H]^+$ = 460.2 | 72 |
| **77** | (5S)-1'-[7-(5-chloroquinoxalin-6-yl)-6-methyl-pyrazolo[1,5-a] pyrazin-4-yl]spiro[5,7-dihydrocyclopenta[b]pyridine-6,4'-piperidine]-5-amine | $^1H$ NMR (400 MHz, DMSO-*d6*) δ ppm 1.25 (d, *J* = 14 Hz, 1 H), 1.65 (d, *J* = 14 Hz, 1 H), 1.81 - 1.98 (m, 2 H), 2.10 - 2.20 (m, 2 H), 2.12 (s, 1 H), 2.81 (d, *J* = 16 Hz, 1 H), 3.17 (d, *J* = 16 Hz, 1 H), 3.35 - 3.53 (m, 2 H), 3.95 (s, 1 H), 4.45 (td, *J* = 11, 2 Hz, 2 H), 7.03 (dd, *J* = 3, 1 Hz, 1 H), 7.19 (dd, *J* = 8, 5 Hz, 1 H), 7.68 (d, *J* = 7 Hz, 1 H), 7.83 (d, *J* = 3 Hz, 1 H), 7.94 (d, *J* = 9 Hz, 1 H), 8.21 (d, *J* = 9 Hz, 1 H), 8.33 (dd, *J* = 5, 1 Hz, 1 H), 9.13 (s, 2 H). LCMS m/z $[M+H]^+$ = 497.2 | 4 |
| **78** | (5S)-1'-[7-(5-fluoroquinoxalin-6-yl)-6-methyl-pyrazolo[1,5-a] pyrazin-4-yl]spiro [5,7-dihydrocyclopenta[b]pyridine-6,4'-piperidine] -5-amine hydrochloride | As HCl salt: $^1H$ NMR (400 MHz, DMSO-*d6*) δ ppm 1.68 (br d, *J* = 13 Hz, 1 H), 1.79 (br d, *J* = 12 Hz, 1 H), 1.88 - 2.06 (m, 2 H), 2.24 (s, 3 H), 3.27 (d, *J* = 17 Hz, 1 H), 3.39 - 3.48 (m, 3 H), 4.43 - 4.64 (m, 3 H), 7.13 (dd, *J* = 3, 1 Hz, 1 H), 7.56 (dd, *J* = 8, 5 Hz, 1 H), 7.92 (d, *J* = 3 Hz, 1 H), 7.93 - 8.00 (m, 1 H), 8.09 (dd, *J* = 9, 1 Hz, 1 H), 8.28 (d, *J* = 8 Hz, 1 H), 8.66 (br d, *J* = 5 Hz, 1 H), 8.80 (br d, *J* = 1 Hz, 3 H), 9.08 (d, *J* = 2 Hz, 1 H), 9.13 (d, *J* = 2 Hz, 1 H). LCMS m/z $[M+H]^+$ = 481.2 | 12 |
| **79** | (5S)-1'-[7-(5-chloroquinoxalin-6-yl)-6-methyl-pyrazolo[1,5-a] pyrazin-4-yl]-3-fluoro-spiro[5,7-dihydrocyclopenta[b]pyridine-6,4'-piperidine]-5-amine hydrochloride | As HCl salt: $^1H$ NMR (400 MHz, DMSO-*d6*) δ ppm 1.62 - 1.78 (m, 2 H), 1.87 - 2.01 (m, 2 H), 2.13 (s, 3 H), 3.4 (m, 1 H), 3.26 - 3.36 (m, 3 H), 4.40 - 4.63 (m, 3 H), 7.08 (d, *J* = 3 Hz, 1 H), 7.87 (d, *J* = 3 Hz, 1 H), 7.92 (dd, *J* = 9, 1 Hz, 2 H), 8.22 (d, *J* = 9 Hz, 1 H), 8.56 (dd, *J* = 3, 1 Hz, 1 H), 8.60 - 8.79 (br s, 3 H), 9.03 - 9.24 (m, 2 H). LCMS m/z $[M+H]^+$ = 515.1 | 10 |
| **80** | (1S)-1'-[7-(1,5-dimethylpyrazol-4-yl)-6-methyl-pyrazolo[1,5-a]pyrazin-4-yl]-5,6-difluoro-spiro [indane-2,4'-piperidine]-1-amine hydrochloride | As HCl salt: $^1H$ NMR (400 MHz, DMSO-*d6*) δ ppm 1.62 (br s, 1 H), 1.72 (br s, 1 H), 1.81 - 1.97 (m, 2 H), 2.07 (s, 3 H), 2.21 (s, 3 H), 2.97 - 3.09 (m, 1 H), 3.15 - 3.26 (m, 1 H), 3.33 - 3.55 (m hidden, 3 H), 3.83 (s, 3 H), 4.24 - 4.35 (m, 1 H), 4.36 - 4.48 (m, 2 H), 7.05 (br s, 1 H), 7.43 - 7.46 (m, 1 H), 7.46 (s, 1 H), 7.62 - 7.68 (m, 1 H), 7.92 (br s, 1 H), 8.54 (br s, 2 H). LCMS m/z $[M+H]^+$ = 464.2 | 17 |
| **81** | (6S)-1'-[7-(5-chloroquinoxalin-6-yl)-6-methyl-pyrazolo[1,5-a] pyrazin-4-yl]-2-methyl-spiro [4,6-dihydrocyclopenta[d] thiazole-5,4'-piperidine]-6-amine hydrochloride | As HCl salt: $^1H$ NMR (400 MHz, DMSO-*d6*) δ ppm 1.71 (br d, *J* = 13 Hz, 1 H), 1.82 - 2.10 (m, 3 H), 2.14 (s, 3 H), 2.70 (s, 3 H), 2.91 - 3.10 (m, 2 H), 3.28 - 3.39 (m hidden, 2 H), 4.40 (d, *J* = 4 Hz, 1 H), 4.48 (d, *J* = 14 Hz, 1 H), 4.58 (d, *J* = 12 Hz, 1 H), 7.08 (m, 1 H), 7.87 (d, *J* = 3 Hz, 1 H), 7.92 (d, *J* = 9 Hz, 1 H), 8.22 (d, *J* = 9 Hz, 1 H), 8.41 (br s, 3 H), 9.14 (s, 2 H). LCMS m/z $[M+H]^+$ = 517.1 | 4 |

(continued)

| Cpd. No. | Name | Characterization | SHP2 $IC_{50}$ (nM) |
|---|---|---|---|
| **82** | (6S)-1'-[7-(1,5-dimethylpyrazol-4-yl)-6-methyl-pyrazolo[1,5-a]pyrazin-4-yl]-2-methyl-spiro[4,6-dihydrocyclopenta[d]thiazole-5,4'-piperidine] -6-amine hydrochloride | $^1$H NMR (400 MHz, DMSO-*d6*) δ ppm 1.73 (br s, 1 H), 1.85 - 2.01 (m, 2 H), 2.03 - 2.14 (m, 4 H), 2.23 (s, 3 H), 2.69 (s, 3 H), 2.90 - 3.06 (m, 2 H), 3.34 - 3.63 (m, 2 H), 3.83 (s, 3 H), 4.33 - 4.56 (m, 3 H), 7.16 (s, 1 H), 7.48 (s, 1 H), 7.96 (d, *J* = 2 Hz, 1 H), 8.56 (br s, 3 H). LCMS m/z $[M+H]^+$ = 449.2 | 27 |
| **83** | (6S)-1'-[7-(2-fluorophenyl)-6-methyl-pyrazolo[1,5-a]pyrazin-4-yl]-2-methyl-spiro[4,6-dihydrocyclopenta[d]thiazole-5,4'-piperidine]-6-amine hydrochloride | $^1$H NMR (400 MHz, DMSO-d6) δ ppm 1.69 (br d, J=14 Hz, 1 H), 1.81 - 2.10 (m, 3 H), 2.17 (s, 3 H), 2.69 (s, 3 H), 2.90 - 3.08 (m, 2 H), 3.34 (m, 1 H), 3.49 (m, 1 H), 4.30 - 4.48 (m, 2 H), 4.53 (m, 1 H), 7.08 (s, 1 H), 7.32 - 7.40 (m, 2 H), 7.50 (td, J=8, 2 Hz, 1 H), 7.58 (m, 1 H), 7.90 (d, J=3 Hz, 1 H), 8.48 (br s, 3 H). LCMS m/z [M+H]+ = 449.1 | 10 |
| **84** | (6S)-1'-[7-(3-chloro-2-methyl-4-pyridyl)-6-methyl-pyrazolo [1,5-a]pyrazin-4-yl]-2-methyl-spiro[4,6-dihydrocyclopenta[d] thiazole-5,4'-piperidine]-6-amine hydrochloride | $^1$H NMR (400 MHz, DMSO-d6) δ ppm 1.69 (br d, J=13 Hz, 1 H), 1.78 - 2.05 (m, 3 H), 2.10 (s, 3 H), 2.67 (s, 3 H), 2.69 (s, 3 H), 2.89 - 3.09 (m, 2 H), 3.32 (t, J=13 Hz, 1 H), 3.48 (t, J=12 Hz, 1 H), 4.37 (m, J=4 Hz, 1 H), 4.46 (d, J=14 Hz, 1 H), 4.56 (d, J=13 Hz, 1 H), 7.08 (s, 1 H), 7.44 (d, J=5 Hz, 1 H), 7.89 (d, J=2 Hz, 1 H), 8.47 (br d, J=1 Hz, 3 H), 8.58 (d, J=5 Hz, 1 H). LCMS m/z [M+H]+ = 480.1 | 5 |
| **85** | (5S)-1'-[7-(2-fluoro-3-methoxyphenyl)pyrazolo[1,5-a] pyrazin-4-yl]spiro[5,7-dihydrocyclopenta[b]pyridine-6,4'-piperidine] -5-amine hydrochloride | As HCl salt: $^1$H NMR (500 MHz, DMSO-*d6*) δ ppm 1.65 (br d, *J* = 13 Hz, 1 H), 1.74 (br d, *J* = 13 Hz, 1 H), 1.90 - 1.99 (m, 2 H), 3.22 (d, *J* = 17 Hz, 1 H), 3.39 - 3.54 (m, 3 H), 3.90 (s, 3 H), 4.42 (d, *J* = 14 Hz, 1 H), 4.48 - 4.56 (m, 2 H), 7.11 - 7.14 (m, 1 H), 7.18 (s, 1 H), 7.27 (t, *J*= 9 Hz, 1 H), 7.33 (td, *J* = 8, 1 Hz, 1 H), 7.47 (s, 1 H), 7.49 (m, 1 H), 8.04 (d, *J* = 2 Hz, 1 H), 8.18 (d, *J* = 7 Hz, 1 H), 8.62 (d, *J* = 5 Hz, 1 H), 8.70 (br s, 3 H). LCMS m/z $[M+H]^+$ = 445.2 | 27 |
| **86** | (5S)-1'-[7-(3-chloro-2-methyl-4-pyridyl)pyrazolo[1,5-a] pyrazin-4-yl]spiro[5,7-dihydrocyclopenta[b]pyridine-6,4'-piperidine]-5-amine hydrochloride | As HCl salt: $^1$H NMR (500 MHz, DMSO-*d6*) δ ppm 1.65 (br d, *J* = 13 Hz, 1 H), 1.74 (br d, *J* = 14 Hz, 1 H), 1.86 - 2.00 (m, 2 H), 2.66 (s, 3 H), 3.23 (d, *J* = 17 Hz, 1 H), 3.41 (br d, *J* = 17 Hz, 1 H), 3.44 - 3.52 (m, 2 H), 4.46 (br d, *J* = 14 Hz, 1 H), 4.49 - 4.58 (m, 2 H), 7.17 (d, *J* = 2 Hz, 1 H), 7.49 (q, *J* = 4 Hz, 2 H), 7.52 (s, 1 H), 8.03 (d, *J* = 2 Hz, 1 H), 8.18 (br d, *J* = 8 Hz, 1 H), 8.56 (d, *J* = 5 Hz, 1 H), 8.62 (dd, *J* = 5, 1 Hz, 1 H), 8.69 (br s, 3 H). LCMS m/z $[M+H]^+$ = 446.2 | 60 |
| **87** | (5 S)-1'-[7-(2-chloro-3-fluorophenyl)pyrazolo[1,5-a] pyrazin-4-yl]spiro [5,7-dihydrocyclopenta[b]pyridine-6,4'-piperidine] -5-amine hydrochloride | As HCl salt: $^1$H NMR (400 MHz, DMSO-*d6*) δ ppm 1.67 (br d, *J* = 14 Hz, 1 H), 1.77 (br d, *J* = 13 Hz, 1 H), 1.87 - 2.07 (m, 2 H), 3.25 (br d, *J* = 17 Hz, 1 H), 3.39 - 3.59 (m, 3 H), 4.38 - 4.63 (m, 3 H), 7.20 (d, *J* = 2 Hz, 1 H), 7.43 (dd, *J* = 7, 1 Hz, 1 H), 7.48 (s, 1 H), 7.51 - 7.69 (m, 3 H), 8.03 (d, *J* = 3 Hz, 1 H), 8.26 (d, *J* = 8 Hz, 1 H), 8.64 (d, *J* = 5 Hz, 1 H), 8.79 (br s, 3 H). LCMS m/z $[M+H]^+$ = 449.1 | 22 |

(continued)

| Cpd. No. | Name | Characterization | SHP2 $IC_{50}$ (nM) |
|---|---|---|---|
| **88** | (5S)-1'-[7-(1,5-dimethy lpyrazol-4-yl)pyrazolo [1,5-a] pyrazin-4-yl]spiro[5,7-dihydrocyclopenta[b]pyridine-6,4'-piperidine] -5-amine hydrochloride | As HCl salt: $^1$H NMR (400 MHz, DMSO-*d6*) δ ppm 1.71 (br d, *J* = 14 Hz, 1 H), 1.82 (br d, *J* = 14 Hz, 1 H), 1.93 - 2.11 (m, 2 H), 2.30 (s, 3 H), 3.26 (br d, *J* = 17 Hz, 1 H), 3.51 (br d, *J* = 17 Hz, 1 H), 3.54 - 3.64 (m, 2 H), 3.84 (s, 3 H), 4.36 (br d, *J* = 15 Hz, 1 H), 4.44 (br d, *J* = 14 Hz, 1 H), 4.59 (m, 1 H), 7.30 (s, 1 H), 7.32 (s, 1 H), 7.61 (dd, *J* = 8, 5 Hz, 1 H), 7.74 (s, 1 H), 8.16 (d, *J* = 2 Hz, 1 H), 8.34 (d, *J* = 8 Hz, 1 H), 8.67 (dd, *J* = 5, 1 Hz, 1 H), 8.91 (br s, 3 H). LCMS m/z $[M+H]^+$ = 415.2 | 346 |
| **89** | (5S)-1'-[7-(4-fluoro-1H-indol-7-yl)pyrazolo[1,5-a]pyrazin-4-yl] spiro[5,7-dihydrocyclopenta[b] pyridine-6,4'-piperidine] -5-amine hydrochloride | As HCl salt: $^1$H NMR (400 MHz, DMSO-*d6*) δ ppm 1.70 (d, *J* = 14 Hz, 1 H), 1.80 (d, *J* = 14 Hz, 1 H), 1.92 - 2.12 (m, 2 H), 3.24 (d, *J* = 17 Hz, 1 H), 3.38 - 3.46 (m, 3 H), 4.43 (d, *J* = 14 Hz, 1 H), 4.51 (d, *J* = 14 Hz, 1 H), 4.59 (m, 1 H), 6.58 (dd, *J* = 3, 2 Hz, 1 H), 6.92 (dd, *J* = 10, 8 Hz, 1 H), 7.25 (dt, *J* = 8, 4 Hz, 2 H), 7.34 (t, *J* = 3 Hz, 1 H), 7.49 (s, 1 H), 7.51 (dd, *J* = 8, 5 Hz, 1 H), 8.04 (d, *J* = 3 Hz, 1 H), 8.22 (d, *J* = 8 Hz, 1 H), 8.63 (dd, *J* = 5, 2 Hz, 1 H), 8.72 - 8.99 (m, 3 H), 11.07 (br s, 1 H). LCMS m/z $[M+H]^+$ = 454.2 | 9 |
| **90** | (5S)-1'-(7-pyrazolo[1,5-a] pyridin-3-ylpyrazolo[1,5-a] pyrazin-4-yl)spiro [5,7-dihydrocyclopenta[b]pyridine-6,4'-piperidine] -5-amine hydrochloride | As HCl salt: $^1$H NMR (400 MHz, DMSO-*d6*) δ ppm 1.71 (br d, *J* = 14 Hz, 1 H), 1.81 (br d, *J* = 14 Hz, 1 H), 1.93 - 2.11 (m, 2 H), 3.27 (d, J = 17 Hz, 1 H), 3.39 - 3.48 (m hidden, 3 H), 4.39 *(d, J*= 14 Hz, 1 H), 4.47 (d, *J* = 14 Hz, 1 H), 4.60 (m, 1 H), 7.05 (td, *J* = 7, 1 Hz, 1 H), 7.30 (d, *J* = 2 Hz, 1 H), 7.39 (ddd, *J* = 10, 7, 1 Hz, 1 H), 7.59 *(dd, J*= 8, 5 Hz, 1 H), 7.66 (s, 1 H), 7.78 (d, *J* = 9 Hz, 1 H), 8.19 (d, *J* = 3 Hz, 1 H), 8.31 (d, *J* = 8 Hz, 1 H), 8.59 (s, 1 H), 8.67 (dd, J = 5, 1 Hz, 1 H), 8.83 (d, *J* = 7 Hz, 1 H), 8.86 (br s, 3 H). LCMS m/z $[M+H]^+$ = 437.1 | 13 |
| **91** | (1S)-1'-[7-(3-chloro-2-methyl-4-pyridyl)-6-methyl-pyrazolo [1,5-a]pyrazin-4-yl]-6-fluoro-spiro[indane-2,4'-piperidine]-1-amine hydrochloride | As HCl salt: $^1$H NMR (400 MHz, DMSO-*d6*) δ ppm 1.56 - 1.73 (m, 2 H), 1.81 - 1.97 (m, 2 H), 2.09 (s, 3 H), 2.67 (s, 3 H), 3.02 (br d, *J* = 16 Hz, 1 H), 3.21 (br d, *J* = 16 Hz, 1 H), 3.33 - 3.51 (m, 3 H), 4.35 - 4.57 (m, 3 H), 7.06 (s, 1 H), 7.20 (td, *J*=9, 3 Hz, 1 H), 7.38 *(dd, J*= 8, 5 Hz, 1 H), 7.41 - 7.49 (m, 2 H), 7.89 *(d, J*= 3 Hz, 1 H), 8.46 - 8.70 (m, 3 H). LCMS m/z $[M+H]^+$ = 477.2 | 19 |
| **92** | (1S)-1'-[7-(3-chloro-2-methyl-4-pyridyl)-6-methyl-pyrazolo [1,5-a]pyrazin-4-yl]-4-fluoro-spiro[indane-2,4'-piperidine]-1-amine formate | As $HCO_2H$ salt: $^1$H NMR (400 MHz, DMSO-*d6*) δ ppm 1.29 (br d, *J*= 13 Hz, 1 H), 1.63 (br d, *J* = 13 Hz, 1 H), 1.77 - 1.95 (m, 2 H), 2.07 (s, 3 H), 2.65 (s, 3 H), 2.74 (br d, *J* = 16 Hz, 1 H), 3.17 (br d, *J* = 16 Hz, 1 H), 3.33 - 3.49 (m, 2 H), 4.00 (s, 1 H), 4.30 - 4.49 (m, 2 H), 6.97 - 7.06 (m, 2 H), 7.20 (m, 1 H), 7.29 (m, 1 H), 7.41 (d, *J* = 5 Hz, 1 H), 7.85 (d, *J* = 3 Hz, 1 H), 8.54 (d, *J* = 5 Hz, 1 H). LCMS m/z $[M+H]^+$ = 477.2 | 8 |
| **93** | (1S)-4-fluoro-1'-[7-(2-fluorophenyl)-6-methyl-pyrazolo[1,5-a]pyrazin-4-yl] spiro[indane-2,4'-piperidine]-1-amine hydrochloride | As HCl salt: $^1$H NMR (400 MHz, DMSO-*d6*) δ ppm 1.62 (br d, *J* = 14 Hz, 1 H), 1.69 (br d, *J* = 12 Hz, 1 H), 1.81 - 1.99 (m, 2 H), 2.16 (s, 3 H), 3.12 (m, 1 H), 3.24 (m, 1 H), 3.40 - 3.51 (m, 2 H), 4.37 - 4.52 (m, 3 H), 7.04 (s, 1 H), 7.22 (t, *J* = 9 Hz, 1 H), 7.30 - 7.46 (m, 4 H), 7.49 (t, *J* = 7 Hz, 1 H), 7.58 (m, 1 H), 7.88 (d, *J* = 2 Hz, 1 H), 8.51 (br s, 3 H). LCMS m/z $[M+H]^+$ = 446.1 | 46 |

(continued)

| Cpd. No. | Name | Characterization | SHP2 $IC_{50}$ (nM) |
|---|---|---|---|
| **94** | (1S)-1'-[7-(2,3-difluorophenyl)-6-methyl-pyrazolo[1,5-a] pyrazin-4-yl]-4-fluoro-spiro [indane-2,4'-piperidine]-1-amine formate | As $HCO_2H$ salt: $^1H$ NMR (400 MHz, DMSO-*d6*) δ ppm 1.29 (br d, *J* = 13 Hz, 1 H), 1.62 (br d, *J* = 13 Hz, 1 H), 1.78 - 1.94 (m, 2 H), 2.16 (s, 3 H), 2.74 (d, *J* = 16 Hz, 1 H), 3.17 (d, *J* = 16 Hz, 1 H), 3.32 - 3.50 (m, 3 H), 4.00 (s, 1 H), 4.37 - 4.46 (m, 2 H), 6.98 - 7.05 (m, 2 H), 7.19 - 7.39 (m, 4 H), 7.58 (m, 1 H), 7.86 (d, *J* = 2 Hz, 1 H), 8.19 (s, 1 H). LCMS m/z $[M+H]^+$ = 464.2 | 146 |
| **95** | (1S)-1'-[7-(1,5-dimethylpyrazol-4-yl)-6-methyl-pyrazolo[1,5-a]pyrazin-4-yl]-4-fluoro-spiro[indane-2,4'-piperidine]-1-amine formate | As $HCO_2H$ salt: $^1H$ NMR (400 MHz, DMSO-*d6*) δ ppm 1.29 (br d, *J*= 13 Hz, 1 H), 1.60 (br d, *J* = 13 Hz, 1 H), 1.79 - 1.93 (m, 2 H), 2.05 (s, 3 H), 2.18 (s, 3 H), 2.74 (br d, *J* = 16 Hz, 1 H), 3.15 (d, *J* = 16 Hz, 1 H), 3.27 - 3.40 (m, 3 H), 3.82 (s, 3 H), 4.00 (s, 1 H), 4.27 - 4.35 (m, 2 H), 6.92 (d, *J* = 3 Hz, 1 H), 7.02 (t, *J* = 9 Hz, 1 H), 7.16 - 7.31 (m, 2 H), 7.45 (s, 1 H), 7.84 (d, *J* = 2 Hz, 1 H), 8.19 (br s, 1 H). LCMS m/z $[M+H]^+$ = 446.2 | 19 |
| **96** | (1S)-4-fluoro-1'-(6-methyl-7-pyrazolo[1,5-a]pyridin-3-yl-pyrazolo[1,5-a]pyrazin-4-yl) spiro[indane-2,4'-piperidine]-1-amine formate | As $HCO_2H$ salt: $^1H$ NMR (400 MHz, DMSO-*d6*) δ ppm 1.34 (br d, *J* = 14 Hz, 1 H), 1.61 (br d, *J* = 14 Hz, 1 H), 1.78 - 1.97 (m, 2 H), 2.23 (s, 3 H), 2.78 (br d, *J* = 16 Hz, 1 H), 3.17 (br d, *J* = 16 Hz, 1 H), 3.33 - 3.37 (m hidden, 2 H), 4.05 (s, 1 H), 4.28 - 4.43 (m, 2 H), 6.93 - 7.01 (m, 2 H), 7.04 (t, *J* = 9 Hz, 1 H), 7.18 - 7.33 (m, 4 H), 7.85 (d, *J* = 2 Hz, 1 H), 8.16 (s, 1 H), 8.22 (s, 1 H), 8.78 (dt, *J* = 7, 1 Hz, 1 H). LCMS m/z $[M+H]^+$ = 468.2 | 11 |
| **97** | (1S)-1'-[7-(2,3-dimethylimidazol-4-yl)-6-methyl-pyrazolo[1,5-a]pyrazin-4-yl]-4-fluoro-spiro[indane-2,4'-piperidine]-1-amine hydrochloride | As HCl salt: $^1H$ NMR (400 MHz, DMSO-*d6*) δ ppm 1.64 (m, 1 H), 1.74 (m, 1 H), 1.83 - 2.01 (m, 2 H), 2.24 (s, 3 H), 2.72 (s, 3 H), 3.12 (br dd, *J* = 16, 3 Hz, 1 H), 3.27 (br dd, *J* = 17, 2 Hz, 1 H), 3.41 (s, 3 H), 3.52 - 3.59 (m, 2 H), 4.40 - 4.65 (m, 3 H), 7.13 (dd, *J* = 3, 1 Hz, 1 H), 7.22 (t, *J* = 9 Hz, 1 H), 7.39 (td, *J* = 8, 5 Hz, 1 H), 7.48 (d, *J* = 8 Hz, 1 H), 7.88 (s, 1 H), 7.96 (d, *J* = 3 Hz, 1 H), 8.71 (d, *J* = 2 Hz, 2 H), 14.56 - 15.09 (br s, 1 H). LCMS m/z $[M+H]^+$ = 446.1 | 42 |
| **98** | (5S)-1'-[6-methyl-7-(3-methyl-4-pyridyl)pyrazolo[1,5-a] pyrazin-4-yl]spiro [5,7-dihydrocyclopenta[b]pyridine-6,4'-piperidine] -5-amine formate | As $HCO_2H$ salt: $^1H$ NMR (400 MHz, DMSO-*d6*) δ ppm 1.49 - 1.65 (m, 2 H), 1.83 - 1.94 (m, 2 H), 1.96 (s, 3 H), 2.07 (s, 3 H), 3.02 (d, *J* = 17 Hz, 1 H), 3.24 (d, *J* = 17 Hz, 1 H), 3.36 - 3.47 (m, 2 H), 4.30 (s, 1 H), 4.35 - 4.54 (m, 2 H), 7.02 (dd, *J* = 3, 1 Hz, 1 H), 7.28 (dd, *J*= 8, 5 Hz, 1 H), 7.32 *(d, J*= 5 Hz, 1 H), 7.83 - 7.89 (m, 2 H), 8.46 (dd, *J*= 5, 2 Hz, 1 H), 8.54 (d, *J* = 5 Hz, 1 H), 8.61 (s, 1 H). LCMS m/z $[M+H]^+$ = 426.2 | 145 |
| **99** | (5S)-1'-[6-methyl-7-(2-thienyl) pyrazolo[1,5-a]pyrazin-4-yl] spiro [5,7-dihydrocyclopenta[b] pyridine-6,4'-piperidine]-5-amine hydrochloride | As HCl salt: $^1H$ NMR (400 MHz, DMSO-*d6*) δ ppm 1.63 (br d, *J* = 12 Hz, 1 H), 1.74 (br d, *J* = 14 Hz, 1 H), 1.83 - 2.04 (m, 2 H), 2.32 (s, 3 H), 3.22 (d, *J* = 17 Hz, 1 H), 3.31 - 3.52 (m, 3 H), 4.36 - 4.56 (m, 3 H), 7.08 (m, 1 H), 7.21 (m, 1 H), 7.33 (m, 1 H), 7.49 (m, 1 H), 7.80 (d, *J* = 4 Hz, 1 H), 7.96 (m, 1 H), 8.22 (d, *J* = 5 Hz, 1 H), 8.63 (s, 1 H), 8.75 (br s, 3 H). LCMS m/z $[M+H]^+$ = 417.1 | 9 |
| **100** | (5S)-1'-[7-(3-chloro-4-pyridyl)-6-methyl-pyrazolo[1,5-a] pyrazin-4-yl]spiro[5,7-dihydrocyclopenta[b]pyridine-6,4'-piperidine]-5-amine hydrochloride | As HCl salt: $^1H$ NMR (400 MHz, DMSO-*d6*) δ ppm 1.66 (br d, *J* = 14 Hz, 1 H), 1.78 (br d, *J* = 12 Hz, 1 H), 1.84 - 2.04 (m, 2 H), 2.11 (s, 3 H), 3.26 (d, *J* = 17 Hz, 1 H), 3.37 - 3.53 (m, 3 H), 4.37 - 4.65 (m, 3 H), 7.08 (s, 1 H), 7.49 - 7.69 (m, 2 H), 7.90 (s, 1 H), 8.30 (d, *J* = 6 Hz, 1 H), 8.57 - 8.76 (m, 2 H), 8.76 - 9.00 (m, 4 H). LCMS m/z $[M+H]^+$ = 446.2 | 13 |

(continued)

| Cpd. No. | Name | Characterization | SHP2 $IC_{50}$ (nM) |
|---|---|---|---|
| **101** | (1S)-1'-[7-(1,5-dimethylpyrazol-4-yl)-6-methyl-pyrazolo[1,5-a]pyrazin-4-yl]-5-fluoro-spiro[indane-2,4'-piperidine]-1-amine hydrochloride | As HCl salt: $^1$H NMR (400 MHz, DMSO-*d6*) δ ppm 1.57 (m, 1 H), 1.69 (m, 1 H), 1.77 - 1.97 (m, 2 H), 2.06 (s, 3 H), 2.20 (s, 3 H), 3.07 (d, *J* = 16 Hz, 1 H), 3.22 (d, *J* = 16 Hz, 1 H), 3.58 - 3.66 (m hidden, 2 H), 3.82 (s, 3 H), 4.20 - 4.50 (m, 3 H), 7.01 (br s, 1 H), 7.09 - 7.30 (m, 2 H), 7.46 (s, 1 H), 7.60 (br dd, *J* = 7, 6 Hz, 1 H), 7.90 (br s, 1 H), 8.40 (br s, 3 H). LCMS m/z $[M+H]^+$ = 446.2 | 15 |
| **102** | (1S)-1'-[7-(1,5-dimethylpyrazol-4-yl)-6-methyl-pyrazolo[1,5-a]pyrazin-4-yl]-4,6-difluoro-spiro[indane-2,4'-piperidine]-1-amine hydrochloride | As HCl salt: $^1$H NMR (400 MHz, DMSO-*d6*) δ ppm 1.61 - 1.78 (m, 2 H), 1.88 - 1.98 (m, 2 H), 2.07 (s, 3 H), 2.23 (s, 3 H), 3.06 (d, *J*= 16 Hz, 1 H), 3.22 (d, *J* = 17 Hz, 1 H), 3.43 - 3.53 (m hidden, 2 H), 3.83 (s, 3 H), 4.36 (d, *J* = 14 Hz, 1 H), 4.44 *(d, J*= 14 Hz, 1 H), 4.51 (m, 1 H), 7.13 (br s, 1 H), 7.27 (td, *J* = 9, 2 Hz, 1 H), 7.38 (dd, *J* = 8, 2 Hz, 1 H), 7.47 (s, 1 H), 7.95 (d, *J* = 2 Hz, 1 H), 8.73 (br s, 3 H). LCMS m/z $[M+H]^+$ = 464.2 | 17 |
| **103** | (1S)-1'-[7-(1,5-dimethylpyrazol-4-yl)-6-methyl-pyrazolo[1,5-a]pyrazin-4-yl]-6-fluoro-spiro [indane-2,4'-piperidine]-1-amine hydrochloride | As HCl salt: $^1$H NMR (400 MHz, DMSO-*d6*) δ ppm 1.56 - 1.75 (m, 2 H), 1.93 (td, *J* = 12, 4 Hz, 2 H), 2.07 (s, 3 H), 2.22 (s, 3 H), 3.01 (d, *J* = 16 Hz, 1 H), 3.21 (d, *J*= 16 Hz, 1 H), 3.55 - 3.60 (m, 2 H), 3.83 (s, 3 H), 4.29 - 4.48 (m, 3 H), 7.09 (br s, 1 H), 7.20 (td, *J* = 9, 3 Hz, 1 H), 7.38 (dd, *J* = 8, 5 Hz, 1 H), 7.44 (dd, *J* = 9, 3 Hz, 1 H), 7.47 (s, 1 H), 7.93 (d, *J* = 2 Hz, 1 H), 8.59 (br s, 3 H). LCMS m/z $[M+H]^+$ = 446.2 | 12 |
| **104** | (4S)-1'-[7-(2-fluorophenyl)-6-methyl-pyrazolo[1,5-a]pyrazin-4-yl]-2-methyl-spiro[4,6-dihydrocyclopenta[d]thiazole-5,4'-piperidine]-4-amine hydrochloride | As HCl salt: $^1$H NMR (400 MHz, DMSO-*d6*) δ ppm 1.76(m, 1 H), 1.84 - 2.12 (m, 3 H), 2.17 (s, 3 H), 2.71 (s, 3 H), 3.03 - 3.18 (m, 2 H), 3.44 (m hidden, 2 H), 4.24 (m, 1 H), 4.34 - 4.59 (m, 2 H), 7.09 (t, *J* = 2 Hz, 1 H), 7.31 - 7.41 (m, 2 H), 7.50 (td, *J* = 8, 2 Hz, 1 H), 7.59 (m, 1 H), 7.91 (d, *J* = 3 Hz, 1 H), 8.43 (br d, *J* = 2 Hz, 3 H). LCMS m/z $[M+H]^+$ = 449.0 | 9 |
| **105** | (4S)-1'-[7-(3-chloro-2-methyl-4-pyridyl)-6-methyl-pyrazolo [1,5-a]pyrazin-4-yl]-2-methyl-spiro[4,6-dihydrocyclopenta[d] thiazole-5,4'-piperidine]-4-amine hydrochloride | As HCl salt: $^1$H NMR (400 MHz, DMSO-*d6*) δ ppm 1.75 (br d, *J* = 14 Hz, 1 H), 1.84 - 2.05 (m, 3 H), 2.09 (s, 3 H), 2.67 (s, 3 H), 2.71 (s, 3 H), 3.03 - 3.18 (m, 2 H), 3.26 - 3.43 (m, 1 H), 3.43 - 3.57 (m, 1 H), 4.23 - 4.30 (m, 1 H), 4.36 - 4.57 (m, 2 H), 7.07 (s, 1 H), 7.44 (d, *J*= 5 Hz, 1 H), 7.89 *(d, J*= 3 Hz, 1 H), 8.42 (br s, 3 H), 8.58 (d, *J* = 5 Hz, 1H). LCMS m/z $[M+H]^+$ = 480.3 | 6 |
| **106** | (5S)-1'-[7-(2-fluorophenyl)-6-methyl-pyrazolo[1,5-a]pyrazin-4-yl]-2-methyl-spiro[5,7-dihydrocyclopenta[b]pyridine-6,4'-piperidine]-5-amine hydrochloride | As HCl salt: $^1$H NMR (400 MHz, DMSO-*d6*) δ ppm 1.66 (br d, *J* = 13 Hz, 1 H), 1.82 (br d, *J* = 13 Hz, 1 H), 1.85 - 2.07 (m, 2 H), 2.17 (s, 3 H), 2.66 (s, 3 H), 3.28 (br d, *J* = 16 Hz, 1 H), 3.30 - 3.50 (m, 2 H masked by water), 4.34 - 4.61 (m, 3 H), 7.07 (br s, 1 H), 7.32 - 7.41 (m, 2 H), 7.46 - 7.62 (m, 3 H), 7.90 (d, *J* = 2 Hz, 1 H), 8.31 (br d, *J* = 8 Hz, 1 H), 8.81 (br s, 3 H). LCMS m/z $[M+H]^+$ = 443.3 | 14 |
| **107** | (5 S)-2-methyl-1'-(6-methyl-7-phenyl-pyrazolo[1,5-a]pyrazin-4-yl)spiro[5,7-dihydrocyclopenta[b]pyridine-6,4'-piperidine]-5-amine hydrochloride | As HCl salt: $^1$H NMR (400 MHz, DMSO-*d6*) δ ppm 1.62 (br d, *J* = 13 Hz, 1 H), 1.82 (br d, *J* = 13 Hz, 1 H), 1.85 - 2.05 (m, 2 H), 2.20 (s, 3 H), 2.64 (s, 3 H), 3.25 (br d, *J* = 16 Hz, 1 H), 3.30 - 3.50 (m, 2 H masked by water), 4.30 - 4.58 (m, 3 H), 7.08 (br s, 1 H), 7.43 - 7.57 (m, 5 H), 7.91 (d, *J* = 2 Hz, 1 H), 8.28 (br d, *J* = 8 Hz, 1 H), 8.79 (br s, 2 H) LCMS m/z $[M+H]^+$ = 425.3 | 12 |
| N.D. = not determined | | | |

**Example S8. Compounds 108-147**

The following compounds listed in Table 7 were made using the procedure or modifications to the procedure, as exemplified for **Example S4** (Compound **31)** but using SPhos/SPhos Pd G4 instead of XPhos/XPhos Pd G4 for Suzuki cross coupling reactions using appropriate starting materials and intermediates. Compounds were isolated as their free base or hydrochloride salt.

**Table 7.**

| Cpd. No. | Name | Characterization | SHP2 $IC_{50}$ (nM) |
|---|---|---|---|
| **108** | (5S)-1'-[7-(3-chloro-2-methoxy-4-pyridyl)-6-methyl-pyrazolo[1,5-a]pyrazin-4-yl] spiro[5,7-dihydrocyclopenta[b] pyridine -6,4'-piperidine]-5-amine | $^1$H NMR (400 MHz, DMSO-*d6*) δ ppm 1.22 (br d, *J* = 11 Hz, 1 H), 1.62 (br d, *J* = 13 Hz, 1 H), 1.76 - 2.01 (m, 4 H), 2.08 (s, 3 H), 2.79 (d, *J* = 16 Hz, 1 H), 3.15 (d, *J* = 16 Hz, 1 H), 3.35 - 3.48 (m, 2 H), 3.93 (s, 1 H), 4.02 (s, 3 H), 4.33 - 4.48 (m, 2 H), 7.00 (dd, *J* = 3, 1 Hz, 1 H), 7.15 (dd, *J* = 5, 1 Hz, 1 H), 7.18 (dd, *J* = 8, 5 Hz, 1 H), 7.66 (dt, *J* = 7, 1 Hz, 1 H), 7.84 (d, *J* = 3 Hz, 1 H), 8.26 (d, *J* = 5 Hz, 1 H), 8.33 (m, 1 H). LCMS m/z $[M+H]^+$ = 476.3 | 6 |
| **109** | (1S)-1'-[7-(2-fluorophenyl)-6-methyl-pyrazolo[1,5-a]pyrazin-4-yl] -4-methoxy-spiro[indane-2,4'-piperidine]-1-amine | $^1$H NMR (400 MHz, DMSO-*d6*) δ ppm 1.21 (br d, *J* = 13 Hz, 1 H), 1.57 (br d, *J* = 13 Hz, 1 H), 1.66 - 1.94 (m, 4 H), 2.13 (s, 3 H), 2.55(m, 1 H), 3.03 *(d, J*= 16 Hz, 1 H), 3.35 - 3.42 (m, 2 H), 3.78 (s, 3 H), 3.84 (s, 1 H), 4.38 (m, *J* = 13, 13 Hz, 2 H), 6.79 (d, *J* = 8 Hz, 1 H), 6.93 (d, *J*= 8 Hz, 1 H), 6.98 (dd, *J* = 2, 1 Hz, 1 H), 7.17 (t, *J*= 8 Hz, 1 H), 7.29 - 7.40 (m, 2 H), 7.46 - 7.61 (m, 2 H), 7.84 (d, *J* = 3 Hz, 1 H). LCMS m/z $[M+H]^+$ = 458.2 | 10 |
| **110** | (1S)-1'-[7-(3-chloro-2-methyl-4-pyridyl)-6-methyl-pyrazolo [1,5-a]pyrazin-4-yl]-4-methoxy-spiro[indane-2,4'-piperidine]-1-amine | $^1$H NMR (400 MHz, DMSO-*d6*) δ ppm 1.23 (br d, *J* = 13 Hz, 1 H), 1.58 (br d, *J* = 12 Hz, 1 H), 1.67 - 1.93 (m, 4 H), 2.06 (s, 3 H), 2.55 (d, *J* = 16 Hz, 1 H), 2.65 (s, 3 H), 3.03 (d, *J* = 16 Hz, 1 H), 3.32 - 3.47 (m, 2 H), 3.78 (s, 3 H), 3.84 (s, 1 H), 4.20 - 4.52 (m, 2 H), 6.79 (d, *J* = 8 Hz, 1 H), 6.93 (d, *J* = 8 Hz, 1 H), 6.99 (d, *J* = 3 Hz, 1 H), 7.17 (t, *J* = 8 Hz, 1 H), 7.41 (d, *J* = 5 Hz, 1 H), 7.84 (d, *J* = 3 Hz, 1 H), 8.54 (d, *J* = 5 Hz, 1 H). LCMS m/z $[M+H]^+$ = 489.2 | 11 |
| **111** | (1S)-1-[7-(1,5-dimethylpyrazol-4-yl)-6-methyl-pyrazolo[1,5-a] pyrazin-4-yl] -4-methoxy-spiro [indane-2,4'-piperidine]-1-amine | $^1$H NMR (400 MHz, DMSO-*d6*) δ ppm 1.21 (br d, *J* = 13 Hz, 1 H), 1.56 (br d, *J* = 13 Hz, 1 H), 1.71 - 1.94 (m, 4 H), 2.05 (s, 3 H), 2.17 (s, 3 H), 2.54 (m, 1 H), 3.01 (d, *J* = 16 Hz, 1 H), 3.26 (m, *J* = 3 Hz, 2 H), 3.78 (s, 3 H), 3.82 (s, 3 H), 3.84 (s, 1 H), 4.30 (m, 2 H), 6.79 (d, *J* = 8 Hz, 1 H), 6.89 - 6.95 (m, 2 H), 7.17 (t, *J* = 8 Hz, 1 H), 7.44 (s, 1 H), 7.83 (d, *J* = 2 Hz, 1 H). LCMS m/z $[M+H]^+$ = 458.2 | 16 |
| **112** | (5S)-1'-[7-(3-chloro-2-methyl-4-pyridyl)-6-methyl-pyrazolo [1,5-a]pyrazin-4-yl]-3-methyl-spiro[5,7-dihydrocyclopenta[c] pyridine -6,4'-piperidine]-5-amine hydrochloride | As HCl salt: $^1$H NMR (400 MHz, DMSO-*d6*) δ ppm 1.61 (d, *J* = 13 Hz, 1 H), 1.76 (d, *J* = 13 Hz, 1 H), 1.87 (t, *J* = 13 Hz, 1 H), 2.11 (m 4 H), 2.68 (s, 3 H), 2.79 (s, 3 H), 3.15 (d, *J* = 16 Hz, 1 H), 3.31 - 3.51 (m, 2 H), 3.55 (d, *J*= 17 Hz, 1 H), 4.52 (t, *J*= 13 Hz, 2 H), 4.79 (m, 1 H), 7.10 (s, 1 H), 7.48 (d, *J* = 5 Hz, 1 H), 7.91 (d, *J* = 2 Hz, 1 H), 8.18 (s, 1 H), 8.60 *(d, J*= 5 Hz, 1 H), 8.79 (s, 1 H), 9.29 (br s, 3 H). LCMS m/z $[M+H]^+$ = 474.2 | 5 |
| **113** | (5S)-1'-[7-(1,5-dimethylpyrazol-4-yl)-6-methy l-pyrazolo[1,5-a]pyrazin-4-yl]-3-methyl-spiro[5,7-dihydrocyclopenta[c]pyridine -6,4'-piperidine]-5-amine hydrochloride | As HCl salt: $^1$H NMR (400 MHz, DMSO-*d6*) δ ppm 1.62 (d, *J* = 13 Hz, 1 H), 1.76 (d, *J* = 14 Hz, 1 H), 1.90 (m, 1 H), 2.07 *(d, J*= 1 Hz, 3 H), 2.13 (m, 1 H), 2.24 (s, 3 H), 2.78 (s, 3 H), 3.14 (d, *J* = 16 Hz, 1 H), 3.44 (m, 2 H), 3.54 (d, *J* = 17 Hz, 1 H), 3.83 (s, 3 H), 4.44 (dd, *J*= 13, 9 Hz, 2 H), 4.78 (m, 1 H), 7.13 (s, 1 H), 7.48 (s, 1 H), 7.96 (s, 1 H), 8.14 (s, 1 H), 8.78 (s, 1 H), 9.26 (m, 3 H). LCMS m/z $[M+H]^+$ = 474.2 | 28 |

(continued)

| Cpd. No. | Name | Characterization | SHP2 $IC_{50}$ (nM) |
|---|---|---|---|
| **114** | (5S)-3-methyl-1'-(6-methyl-7-phenyl-pyrazolo[1,5-a]pyrazin-4-yl)spiro[5,7-dihydrocyclopenta[c]pyridine -6,4'-piperidine]-5-amine hydrochloride | As HCl salt: $^1$H NMR (400 MHz, DMSO-*d6*) δ ppm 1.60 (d, *J* = 13 Hz, 1 H), 1.75 (m, 1 H), 1.88 (t, *J* = 10 Hz, 1 H), 2.11 (m, 1 H), 2.21 (s, 3 H), 2.77 (s, 3 H), 3.14 (d, *J* = 17 Hz, 1 H), 3.33 - 3.48 (m, 2 H), 3.54 (d, *J* = 17 Hz, 1 H), 4.39 - 4.50 (m, 2 H), 4.77 (m, 1 H), 7.10 (br s, 1 H), 7.49 (m, 5 H), 7.92 (d, *J* = 2 Hz, 1 H), 8.13 (s, 1 H), 8.78 (s, 1 H), 9.23 (br s, 3 H). LCMS m/z $[M+H]^+$ = 425.2 | 10 |
| **115** | (5S)-1'-[7-(2,6-difluorophenyl)-6-methyl-pyrazolo[1,5-a] pyrazin-4-yl]spiro[5,7-dihydrocyclopenta[b]pyridine -6,4'-piperidine]-5-amine | $^1$H NMR (400 MHz, DMSO-*d6*) δ ppm 1.23 (d, *J* = 14 Hz, 1 H), 1.64 (d, *J* = 1 Hz, 1 H), 1.74 - 1.99 (m, 2 H), 2.13 (s, 3 H), 2.79 (d, *J* = 16 Hz, 1 H), 3.15 (d, *J* = 16 Hz, 1 H), 3.35 - 3.50 (m, 2 H), 3.93 (s, 1 H), 4.32 - 4.56 (m, 2 H), 7.01 (d, *J* = 3 Hz, 1 H), 7.18 (dd, *J* = 8, 5 Hz, 1 H), 7.28 (t, *J* = 8 Hz, 2 H), 7.55 - 7.74 (m, 2 H), 7.85 (d, *J* = 3 Hz, 1 H), 8.26 - 8.39 (m, 1 H). LCMS m/z $[M+H]^+$ = 447.2 | 11 |
| **116** | (5S)-1'-[7-(3-fluoro-2-methyl-4-pyridyl)-6-methyl-pyrazolo[1,5-a]pyrazin-4-yl]-3-methyl-spiro [5,7-dihydrocyclopenta[c] pyridine -6,4'-piperidine]-5-amine | $^1$H NMR (400 MHz, DMSO-*d6*) δ ppm 1.13 (br d, *J* = 13 Hz, 1 H), 1.62 (br d, *J*= 13 Hz, 1 H), 1.70 - 2.01 (m, 4 H), 2.15 (s, 3 H), 2.45 (s, 3 H), 2.52 - 2.54 (m hidden, 3 H), 2.64 (br d, *J* = 16 Hz, 1 H), 3.13 (d, *J* = 16 Hz, 1 H), 3.38 - 3.44 (m hidden, 2 H), 3.87 (s, 1 H), 4.27 - 4.53 (m, 2 H), 7.02 (d, *J* = 2 Hz, 1 H), 7.19 (s, 1 H), 7.43 (t, *J* = 5 Hz, 1 H), 7.87 (d, *J* = 3 Hz, 1 H), 8.27 (s, 1 H), 8.43 (d, *J* = 5 Hz, 1 H). LCMS m/z $[M+H]^+$ = 458.0 | 8 |
| **117** | 3-[4-[(5S)-5-amino-3-methyl-spiro[5,7-dihydrocyclopenta[c] pyridine -6,4'-piperidine]-1'-yl]-6-methyl-pyrazolo[1,5-a] pyrazin-7-yl]-2-fluoro-benzonitrile hydrochloride | $^1$H NMR (400 MHz, DMSO-*d6*) δ ppm 1.58 (br d, *J* = 13 Hz, 1 H), 1.75 (br d, *J* = 13 Hz, 1 H), 1.86 (m, 1 H), 2.06 (m, 1 H), 2.18 (s, 3 H), 2.77 (s, 3 H), 3.15 (br d, *J* = 17 Hz, 1 H), 3.32 - 3.67 (m hidden, 3 H), 4.44 - 4.60 (m, 2 H), 4.76 (m, 1 H), 7.09 (dd, *J* = 3, 1 Hz, 1 H), 7.59 (t, *J* = 8 Hz, 1 H), 7.87 - 7.97 (m, 2 H), 8.05 - 8.15 (m, 2 H), 8.78 (s, 1 H), 9.19 (br s, 3 H). LCMS m/z $[M+H]^+$ = 468.3 | 10 |
| **118** | (1S)-1'-[7-(3-fluoro-2-methyl-4-pyridyl)-6-methyl-pyrazolo[1,5-a]pyrazin-4-yl]-6-methoxy-spiro [indane-2,4'-piperidine]-1-amine hydrochloride | As HCl salt: $^1$H NMR (400 MHz, DMSO-*d6*) δ ppm 1.61 (br d, *J* = 13 Hz, 1 H), 1.68 (br d, *J* = 13 Hz, 1 H), 1.85 - 1.95 (m, 2 H), 2.19 (s, 3 H), 2.55(d, *J* = 3 Hz, 3 H), 2.97 (d, *J* = 16 Hz, 1 H), 3.16 (d, *J* = 16 Hz, 1 H), 3.38 - 3.48 (m, 2 H), 3.77 (s, 3 H), 4.31 - 4.38 (m, 1 H), 4.45 (br d, *J* = 11 Hz, 1 H), 4.53 (br d, *J* = 10 Hz, 1 H), 6.93 (dd, *J* = 8, 2 Hz, 1 H), 7.10 (s, 1 H), 7.22 - 7.27 (m, 2 H), 7.50 (t, *J* = 5 Hz, 1 H), 7.93 (d, *J* = 2 Hz, 1 H), 8.48 (br d, *J* = 5 Hz, 1 H), 8.51 (br s, 3 H). LCMS m/z $[M+H]^+$ = 473.2 | 4 |
| **119** | (1S)-1'-[7-(3-fluoro-2-methyl-4-pyridyl)-6-methyl-pyrazolo[1,5-a]pyrazin-4-yl]-6-methoxy-spiro [indane-2,4'-piperidine]-1-amine hydrochloride | As HCl salt: $^1$H NMR (400 MHz, DMSO-*d6*) δ ppm 1.61 (br d, *J* = 13 Hz, 1 H), 1.69 (br d, *J* = 13 Hz, 1 H), 1.84 - 1.95 (m, 2 H), 2.11 (s, 3 H), 2.98 (d, *J* = 16 Hz, 1 H), 3.16 (d, *J* = 16 Hz, 1 H), 3.36 - 3.46 (m, 2 H), 3.77 (s, 3 H), 4.35 (br d, *J* = 5 Hz, 1 H), 4.44 (br d, *J* = 14 Hz, 1 H), 4.52 (br d, *J* = 14 Hz, 1 H), 6.94 (dd, *J* = 8, 2 Hz, 1 H), 7.08 (d, *J* = 2 Hz, 1 H), 7.25 (dd, *J* = 5, 3 Hz, 2 H), 7.60 (d, *J* = 5 Hz, 1 H), 7.90 (d, *J* = 3 Hz, 1 H), 8.52 (br s, 3 H), 8.70 (d, *J* = 5 Hz, 1 H), 8.86 (s, 1 H). LCMS m/z $[M+H]^+$ = 475.1 | 4 |

(continued)

| Cpd. No. | Name | Characterization | SHP2 $IC_{50}$ (nM) |
|---|---|---|---|
| **120** | (1S)-1'-[7-(1,5-dimethylpyrazol-4-yl)-6-methyl-pyrazolo[1,5-a]pyrazin-4-yl]-6-methoxy-spiro[indane-2,4'-piperidine]-1-amine hydrochloride | As HCl salt: $^1$H NMR (400 MHz, DMSO-*d6*) δ ppm 1.62 (br d, *J* = 13 Hz, 1 H), 1.71 (br d, *J* = 13 Hz, 1 H), 1.88 - 1.98 (m, 2 H), 2.07 (s, 3 H), 2.23 (s, 3 H), 2.96 (d, *J* = 16 Hz, 1 H), 3.16 (d, *J* = 15 Hz, 1 H), 3.48 (m, *J* = 11 Hz, 2 H), 3.77 (s, 3 H), 3.83 (s, 3 H), 4.29 - 4.46 (m, 3 H), 6.93 (dd, *J* = 8, 2 Hz, 1 H), 7.13 (br s, 1 H), 7.21 - 7.27 (m, 2 H), 7.47 (s, 1 H), 7.95 (s, 1 H), 8.54 (br s, 3 H). LCMS m/z $[M+H]^+$ = 458.2 | 11 |
| **121** | (7S)-1'-[7-(3-chloro-2-methyl-4-pyridyl)-6-methyl-pyrazolo [1,5-a]pyrazin-4-yl]-3 -methoxy -spiro [5,7-dihydrocyclopenta [c]pyridine -6,4'-piperidine]-7-amine | $^1$H NMR (400 MHz, DMSO-*d6*) δ ppm 1.26 (d, *J* = 14 Hz, 1 H), 1.57 (d, *J* = 13 Hz, 1 H), 1.76 - 1.89 (m, 2 H), 2.07 (s, 3 H), 2.66 (s, 3 H), 2.69 (m, 1 H), 3.08 (d, *J* = 17 Hz, 1 H), 3.34 - 3.46 (m, 2 H), 3.83 (s, 3 H), 3.93 (s, 1 H), 4.31 - 4.43 (m, 2 H), 6.66 (s, 1 H), 7.01(m, 1 H), 7.41 (d, *J*= 5 Hz, 1 H), 7.85 (d, *J* = 2 Hz, 1 H), 8.04 (s, 1 H), 8.55 (d, *J* = 5 Hz, 1 H). LCMS m/z $[M+H]^+$ = 490.2 | 8 |
| **122** | (7S)-1''-[7-(2-fluorophenyl)-6-methyl-pyrazolo[1,5-a]pyrazin-4-yl] -3 -methoxy-spiro [5,7-dihydrocyclopenta[c]pyridine -6,4'-piperidine]-7-amine | $^1$H NMR (400 MHz, DMSO-*d6*) δ ppm 1.24 (br d, *J* = 13 Hz, 1 H), 1.55 (br d, *J* = 13 Hz, 1 H), 1.77 - 1.87 (m, 2 H), 2.13 (s, 3 H), 2.66 (d, *J* = 16 Hz, 1 H), 3.07 (d, *J* = 16 Hz, 1 H), 3.34 - 3.42 (m, 2 H), 3.82 (s, 3 H), 3.91 (s, 1 H), 4.33 (dd, *J* = 12, 5 Hz, 2 H), 6.65 (s, 1 H), 6.97 (d, *J* = 2 Hz, 1 H), 7.32 - 7.38 (m, 2 H), 7.50 (t, *J* = 7 Hz, 1 H), 7.54 (m, 1 H), 7.83 (d, *J* = 2 Hz, 1 H), 8.03 (s, 1 H). LCMS m/z $[M+H]^+$ = 459.2 | 12 |
| **123** | (1S)-1'-[7-(2,5-dimethylpyrazol-3-yl)-6-methyl-pyrazolo[1,5-a]pyrazin-4-yl]-6-methoxy-spiro[indane-2,4'-piperidine]-1-amine hydrochloride | As HCl salt: $^1$H NMR (400 MHz, DMSO-*d6*) δ ppm 1.60 (br d, *J* = 13 Hz, 1 H), 1.67 (br d, *J* = 12 Hz, 1 H), 1.88 (t, *J* = 12 Hz, 2 H), 2.17 (s, 3 H), 2.22 (s, 3 H), 2.97 (d, *J* = 15 Hz, 1 H), 3.16 (d, *J* = 15 Hz, 1 H), 3.35 - 3.45 (m, 2 H), 3.46 (s, 3 H), 3.76 (s, 3 H), 4.34 (m, 1 H), 4.43 (d, *J* = 13 Hz, 1 H), 4.52 (m, 1 H), 6.21 (s, 1 H), 6.93 (dd, *J* = 8, 3 Hz, 1 H), 7.07 (s, 1 H), 7.22 - 7.26 (m, 2 H), 7.93 (d, *J* = 2 Hz, 1 H), 8.49 (br s, 3 H). LCMS m/z $[M+H]^+$ = 458.2 | 9 |
| **124** | (5S)-1'-[6-methyl-7-(6-methy lpyridazin-4-yl)pyrazolo[1,5-a] pyrazin-4-yl]spiro[5,7-dihydrocyclopenta[b]pyridine -6,4'-piperidine]-5-amine hydrochloride | As HCl salt: $^1$H NMR (400 MHz, DMSO-*d6*) δ ppm 1.67 (br d, *J* = 13 Hz, 1H), 1.80 (br d, *J* = 13 Hz, 1 H), 1.87 - 2.02 (m, 2 H), 2.33 (s, 3 H), 2.79 (s, 3 H), 3.28 (d, *J* = 17 Hz, 1 H), 3.43 - 3.56 (m, 3 H), 4.48 - 4.64 (m, 3 H), 7.16 (d, *J* = 3 Hz, 1 H), 7.60 (dd, *J* = 7, 6 Hz, 1 H), 7.98 (d, *J* = 3 Hz, 1 H), 8.14 (d, *J* = 2 Hz, 1 H), 8.34 (br d, *J* = 8 Hz, 1 H), 8.67 (d, *J* = 5 Hz, 1 H), 8.90 (br s, 3 H), 9.40 (d, *J* = 2 Hz, 1 H). LCMS m/z $[M+H]^+$ = 427.2 | 391 |
| **125** | (1S)-1'-[7-(1-ethyl-5-methyl-pyrazol-4-yl)-6-methyl-pyrazolo[1,5-a]pyrazin-4-yl]-6-methoxy-spiro [indane-2,4'-piperidine]-1-amine hydrochloride | As HCl salt: $^1$H NMR (400 MHz, DMSO-*d6*) δ ppm 1.38 (t, *J* = 7 Hz, 3 H), 1.55 - 1.68 (m, 2 H), 1.84 - 1.92 (m, 2 H), 2.07 (s, 3 H), 2.19 (s, 3 H), 2.97 (d, *J* = 16 Hz, 1 H), 3.13 (d, *J* = 16 Hz, 1 H), 3.40 - 3.51 (m hidden, 2 H), 3.77 (s, 3 H), 4.15 (q, *J* = 7 Hz, 2 H), 4.29 - 4.43 (m, 3 H), 6.92 - 6.98 (m, 2 H), 7.17 (m, 1 H), 7.25 (d, *J* = 8 Hz, 1 H), 7.48 (s, 1 H), 7.89 (m, 1 H), 8.33 (br s, 3 H). LCMS m/z $[M+H]^+$ = 472.2 | 9 |
| **126** | (1S)-1'-[7-(5-chloro-1-methyl-pyrazol-4-yl)-6-methy l-pyrazolo[1,5-a]pyrazin-4-yl] -6-methoxy-spiro[indane-2,4'-piperidine]-1-amine hydrochloride | As HCl salt: $^1$H NMR (400 MHz, DMSO-*d6*) δ ppm 1.51 - 1.71 (m, 2 H), 1.78 - 1.90 (m, 2 H), 2.19 (s, 3 H), 2.97 (d, *J* = 16 Hz, 1 H), 3.13 (d, *J* = 16 Hz, 1 H), 3.46 - 3.54 (m hidden, 2 H), 3.76 (s, 3 H), 3.91 (s, 3 H), 4.28 - 4.53 (m, 3 H), 6.94 (dd, *J* = 8, 2 Hz, 1 H), 7.00 (d, *J* = 3 Hz, 1 H), 7.17 (d, *J* = 3 Hz, 1 H), 7.25 (d, *J* = 8 Hz, 1 H), 7.73 (s, 1 H), 7.89 (d, *J* = 3 Hz, 1 H), 8.34 (br s, 3 H). LCMS m/z $[M+H]^+$ = 478.2 | 4 |

(continued)

| Cpd. No. | Name | Characterization | SHP2 $IC_{50}$ (nM) |
|---|---|---|---|
| **127** | (1S)-6-methoxy-1'-[6-methyl-7-[2-methyl-5-(trifluoromethyl) pyrazol-3-yl]pyrazolo[1,5-a] pyrazin-4-yl]spiro[indane-2,4'-pipendine]-1-amine hydrochloride | As HCl salt: $^1$H NMR (400 MHz, DMSO-*d6*) δ ppm 1.04 (d, *J* = 6 Hz, 1 H), 1.57 - 1.71 (m, 2 H), 1.84 - 1.93 (m, 2 H), 2.18 (s, 3 H), 2.97 (m, 1 H), 3.15 (m, 1 H), 3.37 - 3.50 (m hidden, 2 H), 3.61 - 3.64 (m, 2 H), 3.74 - 3.79 (m, 3 H), 4.34 (m, 1 H), 4.43 - 4.60 (m, 2 H), 6.93 (dd, *J* = 8, 2 Hz, 1 H), 7.00 (s, 1 H), 7.10 (t, *J* = 2 Hz, 1 H), 7.21 - 7.26 (m, 2 H), 7.95 (d, *J* = 2 Hz, 1 H), 8.48 (br s, 3 H). LCMS m/z $[M+H]^+$ = 512.2 | 22 |
| **128** | (3R)-1'-[7-(3-chloro-2-methyl-4-pyridyl)-6-methyl-pyrazolo [1,5-a]pyrazin-4-yl]spiro[3H-benzofuran-2,4'-piperidine]-3-amine | $^1$H NMR (500 MHz, DMSO-*d6*) δ ppm 1.85 - 2.00 (m, 3 H), 2.01 - 2.14 (m, 3 H), 2.14 (s, 3 H), 2.72 (s, 3 H), 3.58 - 3.71 (m, 2 H), 4.18 (s, 1 H), 4.40 - 4.56 (m, 2 H), 6.85 (d, *J* = 8 Hz, 1 H), 6.94 (t, *J* = 7 Hz, 1H), 7.12 (d, *J* = 2 Hz, 1 H), 7.21 (t, *J* = 8 Hz, 1 H), 7.39 (d, *J* = 7 Hz, 1 H), 7.47 (d, *J* = 5 Hz, 1 H), 7.92 (d, *J* = 2 Hz, 1 H), 8.61 (d, *J* = 5 Hz, 1 H). LCMS m/z $[M+H]^+$ = 461.2 | 17 |
| **129** | (3R)-1'-[7-[3-fluoro-2-(trifluoromethyl)phenyl]-6-methyl-pyrazolo[1,5-a]pyrazin-4-yl]spiro[3H-furo[2,3-b] pyridine-2,4'-piperidine]-3-amine | $^1$H NMR (400 MHz, DMSO-*d6*) δ ppm 1.81 - 1.97 (m, 3 H), 2.02 - 2.13 (m, 6 H), 3.51 - 3.65 (m, 2 H), 4.17 (s, 1 H), 4.28 - 4.53 (m, 2 H), 6.93 (dd, *J* = 7, 5 Hz, 1 H), 7.05 (d, *J* = 2 Hz, 1 H), 7.38 (d, *J* = 8 Hz, 1 H), 7.65 - 7.75 (m, 2 H), 7.84 - 7.92 (m, 2 H), 8.02 (dd, *J* = 5, 1 Hz, 1 H). LCMS m/z $[M+H]^+$ = 499.2 | > 1000 |
| **130** | (7S)-1'-[7-(1,5-dimethylpyrazol-4-yl)-6-methyl-pyrazolo[1,5-a]pyrazin-4-yl]spiro[5,7-dihydrocyclopenta[b]pyridine -6,4'-piperidine]-7-amine | $^1$H NMR (400 MHz, DMSO-*d6*) δ ppm 1.22 (br d, *J* = 14 Hz, 1 H), 1.63 (br d, *J* = 13 Hz, 1 H), 1.82 (br s, 2 H), 1.85 - 2.01 (m, 2 H), 2.06 (s, 3 H), 2.18 (s, 3 H), 2.70 *(d, J*= 16 Hz, 1 H), 3.10 (d, *J* = 16 Hz, 1 H), 3.40 (tdd, *J* = 13, 13, 11, 3 Hz, 2 H), 3.83 (s, 3 H), 3.91 (s, 1 H), 4.23 (tt, *J* = 12, 4 Hz, 2 H), 6.93 (d, *J* = 3 Hz, 1 H), 7.17 (dd, *J* = 7, 5 Hz, 1 H), 7.45 (s, 1 H), 7.62 (dd, *J* = 8, 1 Hz, 1 H), 7.84 (d, *J* = 2 Hz, 1 H), 8.35 (dd, *J* = 5, 2 Hz, 1 H). LCMS m/z $[M+H]^+$ = 429.2 | 60 |
| **131** | (5S)-1'-[7-(2-fluorophenyl)-6-methyl-pyrazolo[1,5-a]pyrazin-4-yl] -3 -methoxy-spiro [5,7-dihydrocyclopenta[c]pyridine -6,4'-piperidine]-5-amine | $^1$H NMR (400 MHz, DMSO-*d6*) δ ppm 1.10 (br dd, *J* = 14, 2 Hz, 1 H), 1.62 (br dd, *J* = 13, 2 Hz, 1 H), 1.76 (tt, *J* = 13, 4 Hz, 1 H), 1.87 (br s, 2 H), 1.96 (tt, *J* = 13, 4 Hz, 1 H), 2.14 (s, 3 H), 2.58 (d, *J* = 15 Hz, 1 H), 3.12 (d, *J* = 15 Hz, 1 H), 3.22 - 3.40 (m, 2 H), 3.82 (s, 3 H), 3.84 (s, 1 H), 4.33 - 4.48 (m, 2 H), 6.73 (s, 1 H), 6.98 (d, *J* = 2 Hz, 1 H), 7.31 - 7.39 (m, 2 H), 7.50 (td, *J* = 8, 2 Hz, 1 H), 7.53 - 7.60 (m, 1 H), 7.84 (d, *J* = 2 Hz, 1 H), 7.96 (s, 1 H). LCMS m/z $[M+H]^+$ = 459.2 | 13 |
| **132** | (5S)-1-[7-(1,5-dimethylpyrazol-4-yl)-6-methyl-pyrazolo[1,5-a] pyrazin-4-yl]-3-methoxy-spiro [5,7-dihydrocyclopenta[c] pyridine -6,4'-piperidine]-5-amine | $^1$H NMR (400 MHz, DMSO-*d6*) δ ppm 1.09 (br d, *J* = 13 Hz, 1 H), 1.61 (br d, *J* = 13 Hz, 1 H), 1.75 (td, *J* = 13, 4 Hz, 1 H), 1.95 (td, *J* = 13, 4 Hz, 1 H), 2.06 (s, 3 H), 2.10 - 2.24 (m, 2 H), 2.18 (s, 3 H), 2.57 (d, *J* = 15 Hz, 1 H), 3.10 (d, *J*= 16 Hz, 1 H), 3.18 - 3.36 (m, 2 H), 3.29 - 3.31 (m, 3 H), 3.82 (s, 3 H), 3.84 (s, 1 H), 4.24 - 4.45 (m, 2 H), 6.73 (s, 1 H), 6.92 (d, *J* = 3 Hz, 1 H), 7.45 (s, 1 H), 7.84 (d, *J* = 3 Hz, 1 H), 7.95 (s, 1 H). LCMS m/z $[M+H]^+$ = 459.2 | 34 |

(continued)

| Cpd. No. | Name | Characterization | SHP2 $IC_{50}$ (nM) |
|---|---|---|---|
| **133** | (7S)-1-[7-(1,5-dimethylpyrazol-4-yl)-6-methyl-pyrazolo[1,5-a] pyrazin-4-yl]spiro[5,7-dihydrocyclopenta[c]pyridine -6,4'-piperidine]-7-amine | $^1$H NMR (400 MHz, DMSO-*d6*) δ ppm 1.22 (br d, *J* = 14 Hz, 1 H), 1.57 (br d, *J* = 13 Hz, 1 H), 1.78 - 1.92 (m, 4 H), 2.06 (s, 3 H), 2.18 (s, 3 H), 2.71 (d, *J* = 17 Hz, 1 H), 3.12 (d, *J* = 16 Hz, 1 H), 3.26 - 3.39 (m, 2 H), 3.83 (s, 3 H), 3.98 (s, 1 H), 4.28 (m, 2 H), 6.92 (d, *J* = 2 Hz, 1 H), 7.26 (d, *J* = 5 Hz, 1 H), 7.45 (s, 1 H), 7.84 (d, *J* = 2 Hz, 1 H), 8.37 (d, *J* = 5 Hz, 1 H), 8.50 (s, 1 H). LCMS m/z $[M+H]^+$ = 429.2 | 21 |
| **134** | (7S)-1'-[7-(2-fluorophenyl)-6-methyl-pyrazolo[1,5-a]pyrazin-4-yl]spiro[5,7-dihydrocyclopenta[c]pyridine -6,4'-piperidine]-7-amine | $^1$H NMR (400 MHz, DMSO-*d6*) δ ppm 1.17 (br d, *J* = 13 Hz, 1 H), 1.52 (br d, *J* = 13 Hz, 1 H), 1.66 - 1.88 (m, 2 H), 1.90 - 2.05 (m, 2 H), 2.07 (s, 3 H), 2.66 (d, *J* = 17 Hz, 1 H), 3.07 (d, *J* = 16 Hz, 1 H), 3.25 - 3.39 (m, 2 H), 3.93 (s, 1 H), 4.21 - 4.35 (m, 2 H), 6.91 (dd, *J* = 3, 1 Hz, 1 H), 7.19 (d, *J* = 5 Hz, 1 H), 7.24 - 7.33 (m, 2 H), 7.43 (td, *J* = 8, 2 Hz, 1 H), 7.46 - 7.54 (m, 1 H), 7.77 (d, *J* = 2 Hz, 1 H), 8.30 (d, *J* = 5 Hz, 1 H), 8.43 (s, 1 H). LCMS m/z $[M+H]^+$ = 429.2 | 4 |
| **135** | (5S)-3-methoxy-1'-(6-methyl-7-phenyl-pyrazolo[1,5-a] pyrazin-4-yl)spiro[5,7-dihydrocyclopenta[c]pyridine -6,4'-piperidine]-5-amine | $^1$H NMR (400 MHz, DMSO-*d6*) δ ppm 1.09 (br d, *J* = 13 Hz, 1 H), 1.62 (br d, *J* = 13 Hz, 1 H), 1.76 (td, *J* = 13, 4 Hz, 1 H), 1.87 (br s, 2 H), 1.96 (td, *J*= 13, 4 Hz, 1 H), 2.17 (s, 3 H), 2.57 (d, *J* = 15 Hz, 1 H), 3.11 (d, *J* = 15 Hz, 1 H), 3.24 (m, 1 H), 3.33 (m, 1 H), 3.83 (s, 3 H), 3.84 (s, 1 H), 4.30 - 4.43 (m, 2 H), 6.73 (s, 1 H), 6.95 (d, *J* = 2 Hz, 1 H), 7.43 - 7.53 (m, 5 H), 7.84 (d, *J* = 2 Hz, 1 H), 7.96 (s, 1 H). LCMS m/z $[M+H]^+$ = 441.2 | 12 |
| **136** | (5R)-1'-[7-(2-fluorophenyl)-6-methyl-pyrazolo[1,5-a]pyrazin-4-yl]spiro[5,7-dihydrocyclopenta[b]pyridine -6,4'-piperidine]-5-amine | $^1$H NMR (400 MHz, DMSO-*d6*) δ ppm 1.23 (br d, *J* = 13 Hz, 1 H), 1.62 (br d, *J* = 13 Hz, 1 H), 1.80 - 2.02 (m, 4 H), 2.14 (s, 3 H), 2.80 (d, *J* = 16 Hz, 1 H), 3.16 (d, *J* = 16 Hz, 1 H), 3.33 - 3.44 (m, 2 H), 3.94 (s, 1 H), 4.35 - 4.44 (m, 2 H), 6.99 (d, *J* = 2 Hz, 1 H), 7.18 (dd, *J* = 8, 5 Hz, 1 H), 7.33 - 7.39 (m, 2 H), 7.51 (m, 1 H), 7.56 (m, 1 H), 7.67 (d, *J* = 7 Hz, 1 H), 7.84 (d, *J* = 2 Hz, 1 H), 8.33 (d, *J* = 5 Hz, 1 H). LCMS m/z $[M+H]^+$ = 429.2 | 383 |
| **137** | (5S)-1'-[7-(3-chloro-2-methyl-4-pyridyl)-6-methyl-pyrazolo [1,5-a]pyrazin-4-yl]-3-methoxy-spiro[5,7-dihydrocyclopenta[c] pyridine -6,4'-piperidine]-5-amine | $^1$H NMR (400 MHz, DMSO-*d6*) δ ppm 1.11 (br d, *J* = 14 Hz, 1 H), 1.64 (br d, *J* = 13 Hz, 1 H), 1.75 (td, *J* = 13, 4 Hz, 1 H), 1.89 (br s, 2 H), 1.95 (td, *J* = 13, 4 Hz, 1 H), 2.07 (s, 3 H), 2.58 (d, *J* = 15 Hz, 1 H), 2.66 (s, 3 H), 3.12 (d, *J* = 15 Hz, 1 H), 3.23 - 3.42 (m, 2 H), 3.82 (s, 3 H), 3.85 (s, 1 H), 4.34 - 4.53 (m, 2 H), 6.73 (s, 1 H), 7.01 (dd, *J* = 3, 2 Hz, 1 H), 7.41 (d, *J* = 5 Hz, 1 H), 7.85 (d, *J* = 2 Hz, 1 H), 7.96 (s, 1 H), 8.55 (d, *J* = 5 Hz, 1 H). LCMS m/z $[M+H]^+$ = 490.2 | 6 |
| **138** | (7S)-1'-(6-methyl-7-phenyl-pyrazolo[1,5-a]pyrazin-4-yl) spiro[5,7-dihydrocyclopenta[c] pyridine -6,4'-piperidine]-7-amine | $^1$H NMR (400 MHz, DMSO-*d6*) δ ppm 1.24 (br s, 1 H), 1.58 (br d, *J* = 13 Hz, 1 H), 1.75 - 1.95 (m, 4 H), 2.17 (s, 3 H), 2.72 (d, *J* = 16 Hz, 1 H), 3.13 (d, *J* = 17 Hz, 1 H), 3.32 - 3.44 (m, 2 H), 3.99 (s, 1 H), 4.23 - 4.38 (m, 2 H), 6.96 (d, *J* = 3 Hz, 1 H), 7.26 (dd, *J* = 5, 1 Hz, 1 H), 7.38 - 7.56 (m, 5 H), 7.84 (d, *J* = 2 Hz, 1 H), 8.37 (d, *J* = 5 Hz, 1 H), 8.50 (s, 1 H). LCMS m/z $[M+H]^+$ = 411.2 | 4 |

(continued)

| Cpd. No. | Name | Characterization | SHP2 $IC_{50}$ (nM) |
|---|---|---|---|
| **139** | (7S)-1'-[7-(3-chloro-2-methyl-4-pyridyl)-6-methyl-pyrazolo [1,5-a]pyrazin-4-yl]spiro[5,7-dihydrocyclopenta[c]pyridine -6,4'-piperidine]-7-amine | $^1$H NMR (400 MHz, DMSO-*d6*) δ ppm 1.24 (br d, *J* = 13 Hz, 1 H), 1.60 (br d, *J* = 13 Hz, 1 H), 1.77 - 1.95 (m, 4 H), 2.08 (s, 3 H), 2.66 (s, 3 H), 2.73 (d, *J* = 17 Hz, 1 H), 3.14 (d, *J* = 17 Hz, 1 H), 3.32 - 3.52 (m, 2 H), 4.00 (s, 1 H), 4.32 - 4.45 (m, 2 H), 7.01 (dd, *J* = 2, 2 Hz, 1 H), 7.26 (d, *J* = 5 Hz, 1 H), 7.42 (d, *J* = 5 Hz, 1 H), 7.85 (d, *J* = 2 Hz, 1 H), 8.37 (d, *J* = 5 Hz, 1 H), 8.50 (s, 1 H), 8.55 (d, *J* = 5 Hz, 1 H). LCMS m/z $[M+H]^+$ = 460.2 | 4 |
| **140** | (7S)-1'-[7-(3-fluoro-2-methyl-4-pyridyl)-6-methyl-pyrazolo[1,5-a]pyrazin-4-yl]spiro[5,7-dihydrocyclopenta[c]pyridine -6,4'-piperidine]-7-amine | $^1$H NMR (400 MHz, DMSO-*d6*) δ ppm 1.19 - 1.32 (m, 1 H), 1.59 (br d, *J* = 13 Hz, 1 H), 1.76 - 1.95 (m, 3 H), 2.16 (s, 3 H), 2.53 - 2.54 (m, 3 H), 2.73 (d, *J* = 17 Hz, 1 H), 3.14 (d, *J* = 17 Hz, 1 H), 3.38 - 3.50 (m hidden, 2 H), 4.40 (br t, *J* = 12 Hz, 2 H), 7.03 (dd, *J* = 3, 1 Hz, 1 H), 7.26 (dd, *J* = 5, 1 Hz, 1 H), 7.44 (t, *J* = 5 Hz, 1 H), 7.88 (d, *J* = 3 Hz, 1 H), 8.37 (d, *J* = 5 Hz, 1 H), 8.43 (d, *J* = 5 Hz, 1 H), 8.50 (s, 1 H). LCMS m/z $[M+H]^+$ = 444.0 | 7 |
| **141** | (5R)-1'-(6-methyl-7-phenyl-pyrazolo[1,5-a]pyrazin-4-yl) spiro[5,7-dihydrocyclopenta[b] pyridine -6,4'-piperidine]-5-amine | $^1$H NMR (500 MHz, DMSO-*d6*) δ ppm 1.22 (br d, *J* = 13 Hz, 1 H), 1.61 (br d, *J* = 13 Hz, 1 H), 1.80 - 1.96 (m, 2 H), 1.98 - 2.08 (m, 2 H), 2.17 (s, 3 H), 2.79 (d, *J* = 16 Hz, 1 H), 3.14 (d, *J*= 16 Hz, 1 H), 3.33 - 3.51 (m, 2 H), 3.93 (s, 1 H), 4.30 - 4.40 (m, 2 H), 6.95 (d, *J* = 2 Hz, 1 H), 7.18 (dd, *J* = 7, 5 Hz, 1 H), 7.43 - 7.47 (m, 3 H), 7.47 - 7.52 (m, 2 H), 7.67 (d, *J* = 7 Hz, 1 H), 7.83 (d, *J* = 2 Hz, 1 H), 8.32 (d, *J*= 4 Hz, 1 H). LCMS m/z $[M+H]^+$ = 411.2 | 853 |
| **142** | (5S)-1'-[3-fluoro-7-(2-fluoropheny l)-6-methyl-pyrazolo [1,5-a]pyrazin-4-yl] spiro[5,7-dihydrocyclopenta[b] pyridine -6,4'-piperidine]-5-amine hydrochloride | $^1$H NMR (400 MHz, DMSO-*d6*) δ ppm 1.69 (br d, *J* = 14 Hz, 1 H), 1.82 (br d, *J* = 13 Hz, 1 H), 1.88 - 2.11 (m, 2 H), 2.15 (s, 3 H), 3.21 - 3.43 (m, 3 H), 3.52 (br d, *J* = 17 Hz, 1 H), 3.99 - 4.14 (m, 2 H), 4.63 (m, 1 H), 7.32 - 7.41 (m, 2 H), 7.51 (td, *J* = 7, 2 Hz, 1 H), 7.59 (m, 1 H), 7.69 (dd, *J* = 8, 5 Hz, 1 H), 8.00 (d, *J* = 4 Hz, 1 H), 8.46 (d, *J* = 7 Hz, 1 H), 8.72 (dd, *J* = 6, 1 Hz, 1 H), 8.94 (br d, *J* = 4 Hz, 3 H). LCMS m/z $[M+H]^+$ = 447.3 | 26 |
| **143** | (5S)-1'-(3-fluoro-6-methyl-7-phenyl-pyrazolo[1,5-a]pyrazin-4-yl)spiro[5,7-dihydrocyclopenta[b]pyridine -6,4'-piperidine]-5-amine hydrochloride | $^1$H NMR (400 MHz, DMSO-*d6*) δ ppm 1.68 (br d, *J* = 13 Hz, 1 H), 1.80 (br d, *J* = 13 Hz, 1 H), 1.89 - 2.09 (m, 2 H), 2.17 (s, 3 H), 3.20 - 3.36 (m, 3 H), 3.46 (br d, *J* = 17 Hz, 1 H), 3.94 (br d, *J* = 14 Hz, 1 H), 4.04 (br d, *J* = 14 Hz, 1 H), 4.61(m, 1 H), 7.39 -7.57 (m, 5 H), 7.62 (dd, *J* = 8, 5 Hz, 1 H), 7.99 (d, *J* = 4 Hz, 1 H), 8.36 (br d, *J* = 8 Hz, 1 H), 8.68 (dd, *J* = 5, 2 Hz, 1 H), 8.86 (br s, 3 H). LCMS m/z $[M+H]^+$ = 429.3 | 39 |
| **144** | (5S)-1'-[7-(3-chloro-2-methyl-4-pyridyl)-3-fluoro-6-methyl-pyrazolo[1,5-a]pyrazin-4-yl] spiro[5,7-dihydrocyclopenta[b] pyridine -6,4'-piperidine]-5-amine hydrochloride | $^1$H NMR (400 MHz, DMSO-*d6*) δ ppm 1.70 (br d, *J* = 13 Hz, 1 H), 1.83 (br d, *J* = 14 Hz, 1 H), 1.88 - 2.07 (m, 2 H), 2.09 (s, 3 H), 2.68 (s, 3 H), 3.23 - 3.42 (m, 4 H), 3.96 - 4.22 (m, 2 H), 4.64 (d, *J* = 5 Hz, 1 H), 7.49 (d, *J* = 5 Hz, 1 H), 7.71 (dd, *J* = 8, 6 Hz, 1 H), 8.02 (d, *J* = 4 Hz, 1 H), 8.47 (d, *J* = 8 Hz, 1 H), 8.60 (d, *J* = 5 Hz, 1 H), 8.73 (dd, *J* = 5, 1 Hz, 1 H), 8.93 (br s, 3 H). LCMS m/z $[M+H]^+$ = 478.3 | 17 |
| **145** | [4-[(5S)-5-aminospiro[5,7-dihydrocyclopenta[b]pyridine -6,4'-piperidine]-1'-yl]-6-methyl-7-phenyl-pyrazolo[1,5-a]pyrazin-3-yl]methanol | $^1$H NMR (500 MHz, DMSO-*d6*) δ ppm 1.26 (br d, *J* = 14 Hz, 1 H), 1.64 (br d, *J* = 13 Hz, 1 H), 1.84 - 2.07 (m, 4 H), 2.20 (s, 3 H), 2.76 (d, *J* = 16 Hz, 1 H), 3.02 - 3.15 (m, 3 H), 3.72 (br dd, *J* = 18, 13 Hz, 2 H), 3.97 (s, 1 H), 4.79 (d, *J* = 5 Hz, 2 H), 5.12 (t, *J* = 5 Hz, 1 H), 7.18 (dd, *J* = 7, 5 Hz, 1 H), 7.41 - 7.58 (m, 5 H), 7.69 (dt, *J* = 8, 1 Hz, 1 H), 7.86 (s, 1 H), 8.32 (dd, *J* = 5, 1 Hz, 1 H). LCMS m/z $[M+H]^+$ = 441.3 | > 1000 |

(continued)

| Cpd. No. | Name | Characterization | SHP2 $IC_{50}$ (nM) |
|---|---|---|---|
| **146** | (5S)-1'-[3-(difluoromethyl)-6-methyl-7-phenyl-pyrazolo [1,5-a]pyrazin-4-yl]spiro[5,7-dihydrocyclopenta[b]pyridine -6,4'-piperidine]-5-amine | $^1$H NMR (400 MHz, DMSO-*d6*) δ ppm 1.28 (br d, *J* = 13 Hz, 1 H), 1.65 (br d, *J* = 13 Hz, 1 H), 1.91 - 2.11 (m, 2 H), 2.10 - 2.22 (m, 2 H), 2.26 (s, 3 H), 2.77 (d, *J*= 16 Hz, 1 H), 3.05 - 3.26 (m, 3 H), 3.61 (br t, *J* = 13 Hz, 2 H), 3.99 (s, 1 H), 7.19 (dd, *J* = 8, 5 Hz, 1 H), 7.39 (t, *J* = 54 Hz, 1 H), 7.48 - 7.58 (m, 5 H), 7.69 (dt, *J* = 7, 1 Hz, 1 H), 8.25 (s, 1 H), 8.33 (d, *J* = 5 Hz, 1 H). LCMS m/z $[M+H]^+$ = 461.3 | > 1000 |
| **147** | (5S)-1'-[3-(difluoromethyl)-7-(2-fluorophenyl)-6-methyl-pyrazolo[1,5-a]pyrazin-4-yl] spiro[5,7-dihydrocyclopenta[b] pyridine -6,4'-piperidine]-5-amine | $^1$H NMR (400 MHz, DMSO-*d6*) δ ppm 1.27 (br d, *J* = 14 Hz, 1 H), 1.65 (br d, *J* = 13 Hz, 1 H), 1.85 - 2.12 (m, 4 H), 2.24 (s, 3 H), 2.77 (d, *J* = 16 Hz, 1 H), 3.10 (d, *J* = 16 Hz, 1 H), 3.12 - 3.27 (m, 2 H), 3.56 - 3.72 (m, 2 H), 3.98 (s, 1 H), 7.02 - 7.81 (m, 7 H), 8.26 (s, 1 H), 8.33 (m, 1 H). LCMS m/z $[M+H]^+$ = 479.3 | > 1000 |

**Example S9. Compounds 148-152.**

The following compounds listed in Table 8 were made using the procedure or modifications to the procedure, as exemplified for **Example S5** (Compound **62**) but using SPhos/SPhos Pd G4 instead of XPhos/XPhos Pd G4 for Suzuki cross coupling reactions using appropriate starting materials and intermediates.

**Table 8.**

| Cpd. No. | Name | Characterization | SHP2 $IC_{50}$ (nM) |
|---|---|---|---|
| **148** | (1S)-1'-[7-(1-ethylpyrazol-4-yl)-6-methyl-pyrazolo[1,5-a] pyrazin-4-yl]-5-methoxy-spiro[indane-2,4'-piperidine]-1-amine | $^1$H NMR (400 MHz, DMSO-*d6*) δ ppm 1.22 (br d, *J* = 14 Hz, 1 H), 1.44 (t, *J* = 7 Hz, 3 H), 1.54 (br d, *J* = 13 Hz, 1 H), 1.62 - 1.93 (m, 4 H), 2.37 (s, 3 H), 2.63 (d, *J* = 16 Hz, 1 H), 3.04 (d, *J* = 16 Hz, 1 H), 3.30 - 3.33 (m hidden, 2 H), 3.72 (s, 3 H), 3.80 (s, 1 H), 4.22 (q, *J* = 7 Hz, 2 H), 4.24 - 4.31 (m, 2 H), 6.73 (dd, *J* = 8, 2 Hz, 1 H), 6.78 (d, *J* = 3 Hz, 1 H), 6.93 (d, *J* = 2 Hz, 1 H), 7.20 (d, *J* = 8 Hz, 1 H), 7.80 (s, 1 H), 7.92 (d, *J* = 2 Hz, 1 H), 8.18 (s, 1 H). LCMS m/z $[M+H]^+$ = 458.2 | 9 |
| **149** | (1S)-1'-[7-(1,5-dimethylpyrazol-4-yl)-6-methyl-pyrazolo[1,5-a] pyrazin-4-yl]spiro[indane-2,4'-piperidine]-1-amine | $^1$H NMR (400 MHz, DMSO-*d6*) δ ppm 1.18 (br d, *J* = 13 Hz, 1 H), 1.58 (br d, *J* = 13 Hz, 1 H), 1.72 - 1.94 (m, 4 H), 2.05 (s, 3 H), 2.17 (s, 3 H), 2.65 (br d, *J* = 16 Hz, 1 H), 3.10 (d, *J* = 16 Hz, 1 H), 3.24 - 3.37 (m, 2 H), 3.82 (s, 3 H), 3.86 (s, 1 H), 4.30 (br t, *J* = 14 Hz, 2 H), 6.91 (d, *J* = 2 Hz, 1 H), 7.12 - 7.22 (m, 3 H), 7.31 (br d, *J* = 6 Hz, 1 H), 7.44 (s, 1 H), 7.83 (d, *J* = 2 Hz, 1 H). LCMS m/z $[M+H]^+$ = 428.2 | 17 |
| **150** | (1S)-1'-[6-methyl-7-(5-methylisoxazol-4-yl) pyrazolo[1,5-a]pyrazin-4-yl] spiro[indane-2,4'-piperidine]-1-amine | $^1$H NMR (400 MHz, DMSO-*d6*) δ ppm 1.12 - 1.29 (m, 1 H), 1.59 (br d, *J* = 12 Hz, 1 H), 1.78 (m, 1 H), 1.89 (t, *J* = 10 Hz, 1 H), 1.98 - 2.17 (m, 2 H), 2.18 (s, 3 H), 2.32 (s, 3 H), 2.67 (br d, *J* = 15 Hz, 1 H), 3.11 (br d, *J* = 16 Hz, 1 H), 3.29 - 3.43 (m, 2 H), 3.88 (s, 1 H), 4.37 (t, *J* = 14 Hz, 2 H), 6.97 - 7.02 (m, 1 H), 7.13 - 7.23 (m, 3 H), 7.32 (d, *J* = 6 Hz, 1 H), 7.90 (m, 1 H), 8.70 (s, 1 H). LCMS m/z $[M+H]^+$ = 415.1 | 140 |

(continued)

| Cpd. No. | Name | Characterization | SHP2 $IC_{50}$ (nM) |
|---|---|---|---|
| **151** | (1S)-1'-[7-(3-fluoro-2-methyl-4-pyridyl)-6-methyl-pyrazolo [1 ,5-a]pyrazin-4-yl] spiro[indane-2,4'-piperidine]-1-amine | $^1$H NMR (400 MHz, DMSO-*d6*) δ ppm 1.21 (br d, *J* = 13 Hz, 1 H), 1.61 (br d, *J* = 13 Hz, 1 H), 1.76 - 2.06 (m, 4 H), 2.16 (s, 3 H), 2.53 (d, *J* = 4 Hz, 3 H), 2.68 (d, *J* = 16 Hz, 1 H), 3.13 (d, *J* = 16 Hz, 1 H), 3.32 - 3.45 (m, 2 H), 3.88 (s, 1 H), 4.43 (br t, *J* = 15 Hz, 2 H), 7.03 (d, *J* = 2 Hz, 1 H), 7.09 - 7.26 (m, 3 H), 7.32 (d, *J* = 6 Hz, 1 H), 7.44 (t, *J* = 5 Hz, 1 H), 7.88 (d, *J* = 2 Hz, 1 H), 8.43 (d, *J* = 5 Hz, 1 H). LCMS m/z $[M+H]^+$ = 443.2 | 9 |
| **152** | (5S)-1'-[6-methyl-7-(2-methylthiazol-5-yl)pyrazolo [1,5-a]pyrazin-4-yl]spiro[5,7-dihydrocyclopenta[b] pyridine-6,4'-piperidine] -5-amine | $^1$H NMR (400 MHz, DMSO-*d6*) δ ppm 1.19 (br d, *J* = 14 Hz, 1 H), 1.60 (br d, *J* = 13 Hz, 1 H), 1.69 - 2.08 (m, 4 H), 2.33 (s, 3 H), 2.73 (s, 3 H), 2.78 (d, *J* = 16 Hz, 1 H), 3.15 (d, *J* = 16 Hz, 1 H), 3.35 - 3.45 (m, 2 H), 3.90 (s, 1 H), 4.36 - 4.49 (m, 2 H), 7.04 (d, *J* = 3 Hz, 1 H), 7.18 (dd, *J* = 8, 5 Hz, 1 H), 7.65 (dt, *J* = 7, 1 Hz, 1 H), 7.85 (s, 1 H), 7.93 (d, *J* = 3 Hz, 1 H), 8.32 (dd, *J* = 5, 1 Hz, 1 H). LCMS m/z $[M+H]^+$ = 432.2 | 9 |

**Example S10**. **Compounds 153-163.**

The following compounds identified in Table 9 were made using the procedure or modifications to the procedure, as exemplified for **Example S7** (Compound 64**)** but using SPhos/SPhos Pd G4 instead of XPhos/XPhos Pd G4 for Suzuki cross coupling reactions using appropriate starting materials and intermediates. Compounds were isolated as their free base or hydrochloride salt.

**Table 9.**

| Cpd. No. | Name | Characterization | SHP2 $IC_{50}$ (nM) |
|---|---|---|---|
| **153** | (3R)-1'-[7-(2-fluorophenyl)-6-methyl-pyrazolo[1,5-a] pyrazin-4-yl]spiro[indoline-2,4'-piperidine]-3-amine hydrochloride | As HCl salt: $^1$H NMR (400 MHz, DMSO-*d6*) δ ppm 1.68 (br d, J=13 Hz, 1 H), 1.79 - 1.97 (m, 2 H), 2.12 (m, 1 H), 2.19 (s, 3 H), 3.59 - 3.77 (m, 2 H), 4.32 - 4.46 (m, 2 H), 4.53 (m, 1 H), 6.61 - 6.73 (m, 2 H), 7.11 - 7.21 (m, 2 H), 7.33 - 7.42 (m, 2 H), 7.45 (d, J=7 Hz, 1 H), 7.51 (m, 1 H), 7.59 (m, 1 H), 7.93 (d, J=3 Hz, 1 H), 8.47 (br s, 3 H).<br>LCMS m/z $[M+H]^+$ = 429.2 | 15 |
| **154** | (3R)-1'-[7-(3-chloro-2-methyl-4-pyridyl)-6-methyl-pyrazolo [1,5-a]pyrazin-4-yl]spiro [indoline-2,4'-piperidine]-3-amine hydrochloride | As HCl salt: $^1$H NMR (400 MHz, DMSO-*d6*) δ ppm 1.67 (br d, J=13 Hz, 1 H), 1.79 - 1.92 (m, 2 H), 2.08 - 2.15 (m, 4 H), 2.68 (s, 3 H), 3.51 - 3.73 (m, 2 H), 4.32 - 4.47 (m, 2 H), 4.54 (m, 1 H), 6.62 - 6.71 (m, 2 H), 7.12 (d, J=2 Hz, 1 H), 7.16 (td, J=8, 1 Hz, 1 H), 7.45 (d, J=7 Hz, 1 H), 7.48 (d, J=5 Hz, 1 H), 7.91 (d, J=2 Hz, 1 H), 8.44 (br s, 3 H), 8.59 (d, J=5 Hz, 1 H).<br>LCMS m/z $[M-H+HCO_2H]$ = 503.9 | 4 |
| **155** | (3R)-1'-[7-(2-fluorophenyl)-6-methyl-pyrazolo[1,5-a] pyrazin-4-yl]-1-methyl-spiro [indoline-2,4'-piperidine]-3-amine | $^1$H NMR (400 MHz, DMSO-*d6*) δ ppm 1.31 (d, J=13 Hz, 1 H), 1.70 - 1.99 (m, 4 H), 2.06 - 2.19 (m, 4 H), 2.61 (s, 3 H), 3.32 - 3.44 (m, 2 H), 4.21 (s, 1 H), 4.43 - 4.58 (m, 2 H), 6.38 (d, J=8 Hz, 1 H), 6.59 (td, J=7, 1 Hz, 1 H), 7.01 (d, J=3 Hz, 1 H), 7.04 (td, J=8, 1 Hz, 1 H), 7.21 (d, J=7 Hz, 1 H), 7.30 - 7.39 (m, 2 H), 7.45 - 7.60 (m, 2 H), 7.84 (d, J=3 Hz, 1 H).<br>LCMS m/z $[M+H]^+$ = 443.2 | 202 |

(continued)

| Cpd. No. | Name | Characterization | SHP2 $IC_{50}$ (nM) |
|---|---|---|---|
| **156** | (3R)-1'-[7-(3-chloro-2-methyl-4-pyridyl)-6-methyl-pyrazolo [1,5-a]pyrazin-4-yl]-1-methyl-spiro[indoline-2,4'-piperidine]-3-amine | $^1$H NMR (400 MHz, DMSO-*d6*) δ ppm 1.33 (d, J=13 Hz, 1 H), 1.86 (td, J=13, 5 Hz, 1 H), 1.96 (td, J=13, 5 Hz, 1 H), 2.08 (s, 3 H), 2.13(m, 1 H), 2.63 (s, 3 H), 2.66 (s, 3 H), 2.83 - 3.14 (m, 2 H), 3.34 - 3.50 (m, 2 H), 4.29 (s, 1 H), 4.41 - 4.66 (m, 2 H), 6.41 (d, J=8 Hz, 1 H), 6.62 (td, J=8, 1 Hz, 1 H), 7.04 (dd, J=3, 2 Hz, 1 H), 7.07 (td, J=8, 2 Hz, 1 H), 7.23 (dd, J=7, 1 Hz, 1 H), 7.41 (d, J=5 Hz, 1 H), 7.86 (d, J=3 Hz, 1 H), 8.55 (d, J=5 Hz, 1 H).<br>LCMS m/z $[M+H]^+$ = 474.1 (as a~1/1 mixture of atropoisomers) | 35 |
| **157** | (5S)-1'-(6-methyl-7-thiazol-5-yl-pyrazolo[1,5-a]pyrazin-4-yl) spiro[5,7-dihydrocyclopenta [b]pyridine-6,4'-piperidine] -5-amine hydrochloride | As HCl salt: $^1$H NMR (400 MHz, DMSO-*d6*) δ ppm 1.69 (d, J=13 Hz, 1 H), 1.82 (d, J=13 Hz, 1 H), 1.89 - 2.07 (m, 2 H), 2.37 (s, 3 H), 3.33 (d, J=17 Hz, 1 H), 3.43 - 3.55 (m, 2 H), 3.58 (d, J=17 Hz, 1 H), 4.41 - 4.63 (m, 3 H), 7.15 (d, J=2 Hz, 1 H), 7.73 (dd, J=8, 6 Hz, 1 H), 8.01 (d, J=2 Hz, 1 H), 8.18 (s, 1 H), 8.51 (d, J=8 Hz, 1 H), 8.74 (d, J=5 Hz, 1 H), 8.99 (br s, 3 H), 9.36 (s, 1 H).<br>LCMS m/z $[M+H]^+$ = 418.1 | 72 |
| **158** | (5S)-1'-[6-methyl-7-(2-methylthiazol-4-yl)pyrazolo [1,5-a]pyrazin-4-yl]spiro[5,7-dihydrocyclopenta[b]pyridine-6,4'-piperidine] -5-amine hydrochloride | As HCl salt: $^1$H NMR (400 MHz, DMSO-d6) δ ppm 1.70 (d, J=13 Hz, 1 H), 1.82 (d, J=13 Hz, 1 H), 1.91 - 2.08 (m, 2 H), 2.31 (s, 3 H), 2.74 (s, 3 H), 3.31 (d, J=17 Hz, 1 H), 3.43 - 3.59 (m, 3 H), 4.43 (d, J=14 Hz, 1 H), 4.52 (d, J=14 Hz, 1 H), 4.61 (m, 1 H), 7.16 (s, 1 H), 7.68 (t, J=7 Hz, 1 H), 7.89 (s, 1 H), 7.98 (d, J=2 Hz, 1 H), 8.44 (d, J=8 Hz, 1 H), 8.72 (d, J=5 Hz, 1 H), 8.95 (br s, 3 H).<br>LCMS m/z $[M+H]^+$ = 432.2 | 47 |
| **159** | (5S)-1'-(6-methyl-7-pyridazin-4-yl-pyrazolo[1,5-a]pyrazin-4-yl)spiro[5,7-dihydrocyclopenta[b]pyridine-6,4'-piperidine]-5-amine hydrochloride | As HCl salt: $^1$H NMR (500 MHz, DMSO-*d6*) δ ppm 1.64 (br d, J=13 Hz, 1 H), 1.74 (br d, J=14 Hz, 1 H), 1.83 - 1.97 (m, 2 H), 2.28 (s, 3 H), 3.22 (d, J=17 Hz, 1 H), 3.40 (d, J=17 Hz, 1 H), 3.44 - 3.53 (m, 2 H), 4.44 - 4.62 (m, 3 H), 7.11 (d, J=2 Hz, 1 H), 7.48 (dd, J=8, 5 Hz, 1 H), 7.92 - 7.97 (m, 2 H), 8.17 (br d, J=8 Hz, 1 H), 8.61 (dd, J=5, 1 Hz, 1 H), 8.70 (br s, 3 H), 9.41 (dd, J=5, 1 Hz, 1 H), 9.43 (dd, J=2, 1 Hz, 1 H).<br>LCMS m/z $[M+H]^+$ = 413.2 | 649 |
| **160** | (5S)-1'-(6-methyl-7-thiazol-4-yl-pyrazolo[1,5-a]pyrazin-4-yl) spiro[5,7-dihydrocyclopenta [b]pyridine-6,4'-piperidine]-5-amine hydrochloride | $^1$H NMR (400 MHz, DMSO-*d6*) δ ppm 1.68 (br d, J=13 Hz, 1 H), 1.80 (br d, J=13 Hz, 1 H), 1.88 - 2.06 (m, 2 H), 2.31 (s, 3 H), 3.29 (d, J=17 Hz, 1 H), 3.41 - 3.59 (m, 3 H), 4.44 (br d, J=14 Hz, 1 H), 4.52 (br d, J=14 Hz, 1 H), 4.60 (m, 1 H), 7.14 (s, 1 H), 7.64 (dd, J=8, 6 Hz, 1 H), 7.97 (d, J=3 Hz, 1 H), 8.16 (d, J=2 Hz, 1 H), 8.38 (d, J=8 Hz, 1 H), 8.70 (dd, J=5, 1 Hz, 1 H), 8.89 (br s, 3 H), 9.28 (d, J=2 Hz, 1 H).<br>LCMS m/z $[M+H]^+$ = 418.2 | 63 |
| **161** | (5S)-1'-[7-(2,4-dimethy lthiazol-5-yl)-6-methyl-pyrazolo[1,5-a]pyrazin-4-yl] spiro [5,7-dihydrocyclopenta [b]pyridine-6,4'-piperidine]-5-amine hydrochloride | $^1$H NMR (400 MHz, DMSO-*d6*) δ ppm 1.70 (br d, J=14 Hz, 1 H), 1.82 (br d, J=14 Hz, 1 H), 1.89 - 2.06 (m, 2 H), 2.13 (s, 3 H), 2.21 (s, 3 H), 2.72 (s, 3 H), 3.31 (d, J=18 Hz, 1 H), 3.42 - 3.69 (m, 3 H), 4.30 - 4.71 (m, 3 H), 7.15 (d, J=2 Hz, 1 H), 7.69 (dd, J=8, 6 Hz, 1 H), 7.96 (d, J=3 Hz, 1 H), 8.46 (d, J=8 Hz, 1 H), 8.72 (dd, J=5, 1 Hz, 1 H), 8.83 - 9.13 (m, 3 H).<br>LCMS m/z $[M+H]^+$ = 446.1 | 29 |

(continued)

| Cpd. No. | Name | Characterization | SHP2 $IC_{50}$ (nM) |
|---|---|---|---|
| 162 | (5S)-1'-[7-(2,3-dihydrobenzofuran-6-yl)-6-methyl-pyrazolo[1,5-a] pyrazin-4-yl]spiro[5,7-dihydrocyclopenta[b]pyridine-6,4'-piperidine]-5-amine | $^1$H NMR (400 MHz, DMSO-*d6*) δ ppm 1.22 (br d, J=13 Hz, 1 H), 1.61 (br d, J=12 Hz, 1 H), 1.72 - 2.12 (m, 4 H), 2.17 (s, 3 H), 2.78 (d, J=16 Hz, 1 H), 3.14 (d, J=16 Hz, 1 H), 3.21 - 3.45 (m, 4 H), 3.93 (s, 1 H), 4.33 (td, J=9, 4 Hz, 2 H), 4.60 (t, J=9 Hz, 2 H), 6.82 (d, J=1 Hz, 1 H), 6.88 (dd, J=8, 2 Hz, 1 H), 6.94 (d, J=3 Hz, 1 H), 7.18 (dd, J=8, 5 Hz, 1 H), 7.34 (d, J=8 Hz, 1 H), 7.67 (d, J=8 Hz, 1 H), 7.82 (d, J=3 Hz, 1 H), 8.33 (dd, J=5, 1 Hz, 1 H). LCMS m/z $[M+H]^+$ = 453.2 | 4 |
| 163 | (5S)-1'-[6-methyl-7-[2-methyl-5-(trifluoromethyl)pyrazol-3-yl]pyrazolo[1,5-a]pyrazin-4-yl] spiro[5,7-dihydrocyclopenta [b]pyridine-6,4'-piperidine]-5-amine | $^1$H NMR (400 MHz, DMSO-*d6*) δ ppm 1.24 (br d, J=13 Hz, 1 H), 1.64 (br d, J=13 Hz, 1 H), 1.76 - 1.96 (m, 2 H), 2.03 (br d, J=14 Hz, 2 H), 2.17 (s, 3 H), 2.81 (d, J=16 Hz, 1 H), 3.17 (t, J=14 Hz, 1 H), 3.36 - 3.53 (m, 2 H), 3.65 (s, 3 H), 3.94 (s, 1 H), 4.42 - 4.54 (m, 2 H), 7.01 (s, 1 H), 7.07 (t, J=2 Hz, 1 H), 7.19 (dd, J=7, 5 Hz, 1 H), 7.67 (d, J=7 Hz, 1 H), 7.92 (d, J=2 Hz, 1 H), 8.33 (d, J=5 Hz, 1 H). LCMS m/z $[M+H]^+$ = 483.1 | 30 |

**Example S11. (5S)-1'-[7-(2,3-dimethylphenyl)-6-methyl-pyrazolo[1,5-a]pyrazin-4-yl]spiro[5,7-dihydrocyclopenta[b]pyridine-6,4'-piperidine]-5-amine (Compound 164)**

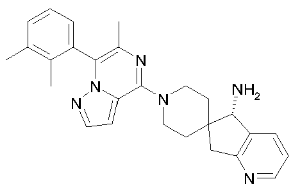

A mixture of **intermediate D-2** (20.6 mg, 50 μmol), 2,3-dimethylphenylboronic acid (15 mg, 100 μmol), $K_3PO_4$ (24 mg, 110 μmol) and XPhos (2.4 mg, 10 μmol) was degassed with Ar for 5 min, XPhos Pd G4 (4.3 mg, 10 μmol) was then added and the mixture was stirred at 75 °C for 2 h. The reaction mixture was diluted with ethyl acetate (10 mL) and a 5% aqueous solution of NaCl (5 mL). The organic layer was dried by passage through a Chromabond® XTR cartridge and rinsed with ethyl acetate. 100 mg of Si-Thiol scavenger was added to the organic phase. The mixture was shaken over 48 h at RT, filtered over a cartridge with frit and rinsed with ethyl acetate. The filtrate was concentrated under reduced pressure. The residue was dissolved in 1.0 ml DMF, filtered over Captiva® cartridge, rinsed with 0.50 ml DMF and purified by SFC on a Viridis BEH-2-ethylpyridine column ($CO_2$/MeOH= 85/15 to 70/30) to give (5S)-1'-[7-(2,3-dimethylphenyl)-6-methyl-pyrazolo[1,5-a]pyrazin-4-yl]spiro[5,7-dihydrocyclopenta[b]pyridine-6,4'-piperidine]-5-amine (4 mg, 0.009 mmol). $^1$H NMR (600 MHz, DMSO-*d6*) δ ppm 1.23 (br d, *J* = 13 Hz, 1 H), 1.62 (br d, *J* = 13 Hz, 1 H), 1.81-1.96 (m, 5 H), 2.04 (s, 3 H), 2.32 (s, 3 H), 2.80 (d, *J* = 16 Hz, 1 H), 3.15 (d, *J* = 16 Hz, 1 H), 3.30 - 3.40 (m, 2 H), 3.95 (s, 1 H), 4.30-4.40 (m, 2 H), 6.93 - 6.95 (m, 1 H), 7.07 (d, *J* = 7 Hz, 1 H), 7.16 - 7.22 (m, 2 H), 7.27 (d, *J* = 7 Hz, 1 H), 7.68 (d, *J* = 7.5 Hz, 1 H), 7.81 (d, *J* = 2 Hz, 1 H), 8.33 (d, *J* = 5 Hz, 1 H). LCMS m/z $[M+H]^+$ = 439.1. SHP2 $IC_{50}$ is 7 nM.

**Example S11a. Compounds 165-206**

The following compounds listed in Table 10 were made using the procedure or modifications to the procedure as exemplified for **Example S11** (Compound **164**) using appropriate starting materials.

**Table 10.**

| Cpd. No. | Structure and Name | Characterization | SHP2 $IC_{50}$ (nM) |
|---|---|---|---|
| **165** | (5S)-1'-(6-methyl-7-pyrimidin-5-yl-pyrazolo[1,5-a]pyrazin-4-yl)spiro[5,7-dihydrocyclopenta [b]pyridine-6,4'-piperidine]-5-amine | $^1$H NMR (600 MHz, DMSO-*d6*) δ ppm 1.23 (br d, *J* = 14 Hz, 1 H), 1.62 (br d, *J* = 14 Hz, 1 H), 1.77 - 1.93 (m, 2 H), 2.39 (s, 3 H), 2.80 (d, *J* = 16 Hz, 1 H), 3.16 *(d, J*= 16 Hz, 1 H), 3.32 - 3.47 (m, 2 H), 3.94 (s, 1 H), 4.38-4.47 (m, 2 H), 7.05 (d, *J* = 2 Hz, 1 H), 7.18 (dd, *J* = 7, 5, Hz, 1 H), 7.67 (d, *J* = 7 Hz, 1 H), 7.89 (d, *J* = 2 Hz, 1 H), 8.33 (d, *J* = 5 Hz, 1 H), 8.99 (s, 2 H), 9.25 (s, 1 H). LCMS m/z $[M+H]^+$ = 413.1 | 370 |
| **166** | (5S)-1'-[7-(2-fluoro-3-methyl-phenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.61 (br d, *J* = 13 Hz, 1 H), 1.76 - 1.95 (m, 2 H), 2.13 (s, 3 H), 2.30 (s, 3 H), 2.82 (d, *J* = 16 Hz, 1 H), 3.15 (d, *J*= 16 Hz, 1 H), 3.30 - 3.42 (m, 2 H), 3.97 (s, 1 H), 4.35 - 4.45 (m, 2 H), 6.96 - 6.99 (m, 1 H), 7.15 - 7.25 (m, 2H), 7.28 (t, *J* = 7 Hz, 1 H), 7.42 (t, *J* = 7 Hz, 1 H), 7-65 - 7.70 (m, 1H), 7.84 (d, *J* = 2 Hz, 1 H), 8.34 (d, *J* = 5 Hz, 1 H). LCMS m/z $[M+H]^+$ = 443.2 | 7 |
| **167** | (5S)-1'-(7-chroman-6-yl-6-methyl-pyrazolo[1,5-a]pyrazin-4-yl)spiro[5,7-dihydrocyclopenta[b]pyr idine-6,4'-piperidine]-5-amine | $^1$H NMR (600 MHz, DMSO-*d6*) δ ppm 1.23 (br d, *J* = 13 Hz, 1 H), 1.60 (br d, *J* = 13 Hz, 1 H), 1.83 (td, *J* = 13, 4 Hz, 1H), 1.90 (td, *J* = 13, 4 Hz, 1H), 1.93 - 2.00 (m, 2H), 2.17 (s, 3 H), 2.78 (br t, *J* = 6 Hz, 2 H), 3.14 (d, *J* = 16 Hz, 1 H), 3.25 - 3.38 (m, 3H, partially masked by water), 3.95 (s, 1 H), 4.20 (t, *J* = 5 Hz, 2H), 4.28 - 4.36 (m, 2 H), 6.83 (d, *J* = 8 Hz, 1 H), 6.92 (d, *J* = 2 Hz, 1 H), 7.09 - 7.15 (m, 2H), 7.18 (dd, *J* = 7, 5 Hz, 1 H), 7.67 (d, *J* = 8 Hz, 1 H), 7.83 (d, *J* = 2 Hz, 1 H), 8.33 (d, *J* = 5 Hz, 1 H). LCMS m/z $[M+H]^+$ = 467.1 | 9 |
| **168** | (5S)-1'-[6-methyl-7-[2-(methylamino)-4-pyridyl] pyrazolo[1,5-a]pyrazin-4-yl] spiro[5,7-dihydrocyclopenta[b] pyr idine-6,4'-piperidine]-5-amine | $^1$H NMR (600 MHz, DMSO-*d6*) δ ppm 1.24 (br d, *J* = 13 Hz, 1 H), 1.62 (br d*, J* = 13 Hz, 1 H), 1.82 (td, *J* = 13, 4 Hz, 1 H), 1.89 (td, *J* = 13, 4 Hz, 1 H), 2.18 (s, 3 H), 2.81 (d, *J* = 5 Hz, 3 H), 3.15 (d, *J* = 16 Hz, 1 H), 3.30 - 3.40 (m, 2 H), 3.96 (s, 1 H), 4.32 - 4.40 (m, 2 H), 6.49 - 6.57 (m, 3 H), 6.96 (d, *J* = 2 Hz, 1 H), 7.19 (dd, *J* = 7, 5 Hz, 1 H), 7.68 (d, *J* = 7 Hz, 1 H), 7.86 (d, *J* = 2 Hz, 1 H), 8.08 (d, *J* = 5 Hz, 1 H), 8.33 (d, *J* = 5 Hz, 1 H). LCMS m/z $[M+H]^+$ = 441.1 | 30 |
| **169** | (5S)-1'-[7-(6-isoquinolyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.63 (br d*, J* = 13 Hz, 1 H), 1.85 (td, *J* = 13, 4 Hz, 1 H), 1.92 (td, *J* = 13, 4 Hz, 1 H), 2.22 (s, 3 H), 2.83 (d, *J* = 16 Hz, 1 H), 3.17 (d, *J* = 16 Hz, 1 H), 3.34 - 3.45 (m, 2 H), 3.97 (s, 1 H), 4.37 - 4.46 (m, 2 H), 7.02 (d, *J* = 2 Hz, 1 H), 7.20 (dd, *J* = 7, 5 Hz, 1 H), 7.68 (d, *J* = 7 Hz, 1 H), 7.76 (dd, *J* = 8, 2 Hz, 1 H), 7.86 (d, *J* = 2 Hz, 1 H), 7.88 (d, *J* = 6 Hz, 1 H), 8.10 (s, 1 H), 8.22 (d, *J* = 8 Hz, 1 H), 8.34 (d, *J* = 5 Hz, 1 H), 8.56 (d, *J* = 6 Hz, 1 H), 9.39 (s, 1 H). LCMS m/z $[M+H]^+$ = 462.1 | 16 |
| **170** | (5S)-1'-[7-(1,3-benzothiazol-5-yl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.62 (br d, *J* = 13 Hz, 1 H), 1.85 (td, *J* = 13, 4 Hz, 1 H), 1.92 (td, *J* = 13, 4 Hz, 1 H), 2.21 (s, 3 H), 2.82 (d, *J* = 16 Hz, 1 H), 3.16 (d, *J* = 16 Hz, 1 H), 3.30 - 3.42 (m, 2 H, partially masked by water), 3.97 (s, 1 H), 4.35 - 4.43 (m, 2 H), 6.99 (d, *J* = 2 Hz, 1 H), 7.19 (dd, *J* = 7, 5 Hz, 1 H), 7.57 (dd, *J* = 8, 2 Hz, 1 H), 7.67 - 7.70 (m, 1 H), 7.84 (d, *J* = 2 Hz, 1 H), 8.18 (d, *J* = 2 Hz, 1 H), 8.29 (d, *J* = 8 Hz, 1 H), 8.34 (d, *J* = 5 Hz, 1 H), 9.47 (s, 1 H). LCMS m/z $[M+H]^+$ = 468.2 | 11 |

(continued)

| Cpd. No. | Structure and Name | Characterization | SHP2 $IC_{50}$ (nM) |
|---|---|---|---|
| **171** | (5S)-1'-[6-methyl-7-(1-methylindolin-5-yl)pyrazolo[1,5-a]pyrazin-4-yl]spiro[5,7-dihydrocyclopenta[b]pyr idine-6,4'-piperidine]-5-amine | $^1H$ NMR (600 MHz, DMSO-*d6*) δ ppm 1.25 (br d, *J* = 13 Hz, 1 H), 1.60 (br d, *J* = 13 Hz, 1 H), 1.84 (td, *J* = 13, 4 Hz, 1 H), 1.91 (td, *J* = 13, 4 Hz, 1 H), 2.19 (s, 3 H), 2.77 (s, 3 H), 2.81 *(d, J*= 16 Hz, 1 H), 2.93 (t, *J* = 8 Hz, 2 H), 3.14 (d, *J* = 16 Hz, 1 H), 3.27 - 3.37 (m, 4 H, partially masked by water), 3.97 (s, 1 H), 4.26 - 4.33 (m, 2 H), 6.58 (d, *J* = 8 Hz, 1 H), 6.90 (d, *J* = 2 Hz, 1 H), 7.05 (dd, *J* = 8, 2 Hz, 1 H), 7.09 (s, 1H), 7.19 (dd, *J* = 7, 5 Hz, 1 H), 7.69 (d, *J* = 7 Hz, 1 H), 7.81 (d, *J* = 2 Hz, 1 H), 8.34 (d, *J* = 5 Hz, 1 H). LCMS m/z $[M+H]^+$ = 466.3 | 20 |
| **172** | 4-fluoro-N,N-dimethyl-3-[6-methyl-4-[(5S)-5-aminospiro [5,7-dihydrocyclopenta[b]pyr idine-6,4'-piperidine]-1'-yl] pyrazolo[1,5-a]pyrazin-7-yl] benzamide | $^1H$ NMR (600 MHz, DMSO-*d6*) δ ppm 1.27 (br d, *J* = 13 Hz, 1 H), 1.62 (br d, *J* = 13 Hz, 1 H), 1.79 - 1.95 (m, 2 H), 2.16 (s, 3 H), 2.83 (d, *J* = 16 Hz, 1 H), 2.98 (br s, 6 H), 3.16 (d, *J* = 16 Hz, 1 H), 3.30 - 3.44 (m, 2 H), 3.99 (s, 1 H), 4.37 - 4.46 (m, 2 H), 6.99-7.01 (m, 1 H), 7.20 (dd, *J* = 7, 5 Hz, 1 H), 7.42 (t, *J* = 9 Hz, 1 H), 7.55 - 7.58 (m, 1 H), 7.60 (ddd, *J* = 7, 5, 2 Hz, 1 H), 7.69 (d, *J* = 7 Hz, 1 H), 7.86 (d, *J* = 2 Hz, 1 H), 8.35 (d, *J* = 4 Hz, 1 H). LCMS m/z $[M+H]^+$ = 500.3 | > 1000 |
| **173** | (5S)-1'-[7-(2-chloro-5-methoxy-phenyl)-6-methyl-pyrazolo[1,5-a]pyrazin-4-yl]spiro[5,7-dihydrocyclopenta[b]pyr idine-6,4'-piperidine]-5-amine | $^1H$ NMR (600 MHz, DMSO-*d6*) δ ppm 1.26 (br d, *J* = 13 Hz, 1 H), 1.62 (br d, *J* = 13 Hz, 1 H), 1.79 - 1.94 (m, 2 H), 2.08 (s, 3 H), 2.82 (d, *J* = 16 Hz, 1 H), 3.16 (d, *J* = 16 Hz, 1 H), 3.31- 3.43 (m, 2 H), 3.78 (s, 3 H), 3.97 (s, 1 H), 4.35 - 4.43 (m, 2 H), 6.97 (t, *J* = 2 Hz, 1 H), 7.05 (dd, *J* = 3, 1 Hz, 1 H), 7.10 (dd, *J* = 9, 3 Hz, 1 H), 7.19 (dd, *J* = 7, 5 Hz, 1 H), 7.52 (d, *J* = 9 Hz, 1 H), 7.69 (d, *J* = 7 Hz, 1 H), 7.83 (d, *J* = 2 Hz, 1 H), 8.34 (d, *J* = 5 Hz, 1 H).<br>LCMS m/z $[M+H]^+$ = 475.2 | 15 |
| **174** | (5S)-1'-[7-[2-fluoro-3-(trifluoromethyl)phenyl] -6-methyl-pyrazolo[1,5-a]pyrazin-4-yl]spiro[5,7-dihydrocyclopenta[b]pyr idine-6,4'-piperidine]-5-amine | $^1H$ NMR (600 MHz, DMSO-*d6*) δ ppm 1.25 (br d, *J* = 13 Hz, 1 H), 1.62 (br d, *J* = 13 Hz, 1 H), 1.79 - 1.94 (m, 2 H), 2.15 (s, 3 H), 2.82 (d, *J* = 16 Hz, 1 H), 3.16 (d, *J* = 16 Hz, 1 H), 3.34 - 3.46 (m, 2 H), 3.96 (s, 1 H), 4.40 - 4.48 (m, 2 H), 7.01-7.05 (m, 1 H), 7.19 *(dd, J*= 7, 5 Hz, 1 H), 7.57 (t, *J* = 8 Hz, 1 H), 7.68 (d, *J* = 7 Hz, 1 H), 7.85-7.91 (m, 2 H), 7.93 (t, *J* = 7 Hz, 1 H), 8.34 (d, *J* = 5 Hz, 1 H).<br>LCMS m/z $[M+H]^+$ = 497.2 | 5 |
| **175** | (5 S)-1'-[7-(2,3-difluoro-4-methoxy-phenyl)-6-methyl-pyrazolo[1,5-a]pyrazin-4-yl] spiro[5,7-dihydrocyclopenta[b] pyr idine-6,4'-piperidine]-5-amine | $^1H$ NMR (600 MHz, DMSO-*d6*) δ ppm 1.25 (br d, *J* = 13 Hz, 1 H), 1.61 (br d, *J* = 13 Hz, 1 H), 1.79 - 1.94 (m, 2 H), 2.16 (s, 3 H), 2.81 (d, *J* = 16 Hz, 1 H), 3.16 (d, *J*= 16 Hz, 1 H), 3.32 - 3.43 (m, 2 H), 3.96 (s, 4 H), 4.36 - 4.44 (m, 2 H), 6.98-7.01 (m, 1 H), 7.13 - 7.20 (m, 2 H), 7.27 (br t, *J* = 8 Hz, 1 H), 7.68 (d, *J* = 7 Hz, 1 H), 7.85 (d, *J* = 2 Hz, 1 H), 8.34 (d, *J* = 5 Hz, 1 H).<br>LCMS m/z $[M+H]^+$ = 477.2 | 4 |

(continued)

| Cpd. No. | Structure and Name | Characterization | SHP2 $IC_{50}$ (nM) |
|---|---|---|---|
| **176** | (5S)-1'-[7-[6-(dimethylamino)-3-pyridyl]-6-methyl-pyrazolo [1 , 5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.24 (br d, *J* = 13 Hz, 1 H), 1.60 (br d, *J* = 13 Hz, 1 H), 1.83 (td, *J* = 12, 4 Hz, 1 H), 1.90 (td, *J* = 12, 4 Hz, 1 H), 2.22 (s, 3 H), 2.80 (d, *J* = 16 Hz, 1 H), 3.09 (s, 6 H), 3.15 (d, *J* = 16 Hz, 1 H), 3.30 - 3.38 (m, 2 H, partially masked by water), 3.96 (s, 1 H), 4.29 - 4.37 (m, 2 H), 6.74 (d, *J* = 9 Hz, 1 H), 6.94 (d, *J* = 2 Hz, 1 H), 7.19 (dd, *J* = 7, 5 Hz, 1 H), 7.60 (d, *J* = 9 Hz, 1 H), 7.68 (d, *J* = 8 Hz, 1 H), 7.84 (d, *J* = 2 Hz, 1 H), 8.14 (d, *J* = 2 Hz, 1 H), 8.34 (d, *J* = 5 Hz, 1 H). LCMS m/z $[M+H]^+$ = 455.3 | 6 |
| **177** | (5S)-1'-[7-(6-amino-3-pyridyl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.28 (br d, *J* = 13 Hz, 1 H), 1.59 (br d, *J* = 13 Hz, 1 H), 1.83 (td, *J* = 12, 4 Hz, 1 H), 1.89 (td, *J* = 12, 4 Hz, 1 H), 2.21 (s, 3 H), 2.84 (d, *J* = 16 Hz, 1 H), 3.15 (d, *J* = 16 Hz, 1 H), 3.30 - 3.38 (m, 2 H, partially masked by water), 4.01 (br s, 1 H), 4.29 - 4.36 (m, 2 H), 6.15 (s, 2 H), 6.55 (d, *J* = 8.62 Hz, 1 H), 6.93 (d, *J* = 2 Hz, 1 H), 7.20 (dd, *J* = 7, 5 Hz, 1 H), 7.45 (d, *J* = 8 Hz, 1 H), 7.70 (d, *J* = 8 Hz, 1 H), 7.84 (d, *J* = 2 Hz, 1 H), 7.95 (d, *J* = 2 Hz, 1 H), 8.36 (d, *J* = 5 Hz, 1 H). LCMS m/z $[M+H]^+$ = 427.2 | 27 |
| **178** | (5S)-1'-[7-(2,3-dihydrobenzofuran-7-yl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.27 (br d, *J* = 13 Hz, 1 H), 1.60 (br d, *J* = 13 Hz, 1 H), 1.83 (td, *J* = 12, 4 Hz, 1 H), 1.90 (td, *J* = 12, 4 Hz, 1 H), 2.12 (s, 3 H), 2.83 (d, *J* = 16 Hz, 1 H), 3.16 (d, *J* = 16 Hz, 1 H), 3.26 (t, *J* = 8 Hz, 2 H), 3.30 - 3.40 (m, 2 H, partially masked by water), 4.00 (s, 1 H), 4.31 - 4.39 (m, 2 H), 4.42 - 4.51 (m, 2 H), 6.91 - 6.96 (m, 2 H), 7.12 (d, *J* = 7 Hz, 1 H), 7.20 (dd, *J* = 7, 5 Hz, 1 H), 7.33 (dd, *J* = 7, 1 Hz, 1 H), 7.69 (d, *J* = 7 Hz, 1 H), 7.81 (d, *J* = 2 Hz, 1 H), 8.35 (d, *J* = 5 Hz, 1 H). LCMS m/z $[M+H]^+$ = 453.2 | 14 |
| **179** | (5S)-1'-[7-(5-fluoro-3-pyridyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.25 (br d, *J* = 13 Hz, 1 H), 1.62 (br *d, J* = 13 Hz, 1 H), 1.82 (td*, J* = 12, 4 Hz, 1 H), 1.89 (td*, J* = 12, 4 Hz, 1 H), 2.21 (s, 3 H), 2.82 (d, *J* = 16 Hz, 1 H), 3.16 (d, *J* = 16 Hz, 1 H), 3.33 - 3.45 (m, 2 H), 3.97 (s, 1 H), 4.38 - 4.48 (m, 2 H), 7.03 (d, *J* = 2 Hz, 1 H), 7.19 (dd, *J* = 7, 5 Hz, 1 H), 7.68 (d, *J* = 7 Hz, 1 H), 7.88 (d, *J* = 2 Hz, 1 H), 7.97 (ddd, *J* = 9, 2, 1 Hz, 1 H), 8.34 (d, *J* = 5 Hz, 1 H), 8.57 (t*, J* = 1.65, 1.65 Hz, 1 H), 8.66 (d*, J* = 3 Hz, 1 H).<br>LCMS m/z $[M+H]^+$ = 430.2 | 18 |
| **180** | 2-fluoro-6-[6-methyl-4-[(5S)-5-aminospiro[5,7-dihydrocyclopenta[b]pyr idine-6,4'-piperidine]-1'-yl]pyrazolo [1,5-a]pyrazin-7-yl]benzonitrile | $^1$H NMR (600 MHz, DMSO-*d6*) δ ppm 1.28 (br d, *J* = 13 Hz, 1 H), 1.62 (br d, *J*= 13 Hz, 1 H), 1.80 - 1.95 (m, 2 H), 2.15 (s, 3 H), 2.83 (d, *J* = 16 Hz, 1 H), 3.17 (d, *J* = 16 Hz, 1 H), 3.36 - 3.49 (m, 2 H), 3.98 (s, 1 H), 4.41 - 4.51 (m, 2 H), 7.06 (d, *J* = 2 Hz, 1 H), 7.19 (dd*, J* = 7, 5 Hz, 1 H), 7.55 (d, *J*= 8 Hz, 1 H), 7.64 - 7.70 (m, 2H), 7.89 (d, *J* = 2 Hz, 1 H), 7.93 (dt*, J* = 8, 6 Hz, 1 H), 8.34 (d, *J* = 5 Hz, 1 H).<br>LCMS m/z $[M+H]^+$ = 454.2 | 39 |

(continued)

| Cpd. No. | Structure and Name | Characterization | SHP2 $IC_{50}$ (nM) |
|---|---|---|---|
| **181** | 2-fluoro-3-[6-methyl-4-[(5S)-5-aminospiro[5,7-dihydrocyclopenta[b]pyr idine-6,4'-piperidine]-1'-yl]pyrazolo [1,5-a]pyrazin-7-yl]benzonitrile | $^1$H NMR (600 MHz, DMSO-*d6*) δ ppm 1.24 (br d, *J* = 13 Hz, 1 H), 1.62 (br d, *J* = 13 Hz, 1 H), 1.78 - 1.95 (m, 2 H), 2.15 (s, 3 H), 2.81 (d, *J* = 16 Hz, 1 H), 3.16 (d, *J*= 16 Hz, 1 H), 3.35 - 3.47 (m, 2 H), 3.95 (s, 1 H), 4.40 - 4.49 (m, 2 H), 7.02 - 7.05 (m, 1 H), 7.19 (dd, *J* = 7, 5 Hz, 1 H), 7.57 (t, *J* = 8 Hz, 1 H), 7.68 (d, *J* = 7 Hz, 1 H), 7.88 (d, *J* = 2 Hz, 1 H), 7.93 (t, *J*= 8 Hz, 1 H), 8.06 - 8.10 (m, 1 H), 8.33 (d, *J* = 5 Hz, 1 H).<br>LCMS m/z $[M+H]^+$ = 454.2 | 5 |
| **182** | (5S)-1'-[6-methyl-7-(1-methylindol-4-yl)pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.27 (br d, *J* = 13 Hz, 1 H), 1.63 (br d, *J* = 13 Hz, 1 H), 1.83 - 1.98 (m, 2 H), 2.08 (s, 3 H), 2.82 (d, *J* = 16 Hz, 1 H), 3.16 (d, *J* = 16 Hz, 1 H), 3.30 - 3.41 (m, 2 H, partially masked by water), 3.85 (s, 3 H), 3.98 (s, 1 H), 4.32 - 4.40 (m, 2 H), 5.86 (d, *J* = 3 Hz, 1 H), 6.92 - 6.95 (m, 1 H), 7.09 (d, *J* = 7 Hz, 1 H), 7.19 (dd, *J* = 7, 5 Hz, 1 H), 7.24 - 7.32 (m, 2H), 7.54 (d, *J* = 8 Hz, 1 H), 7.69 (d, *J* = 7 Hz, 1 H), 7.75 (d, *J* = 2 Hz, 1 H), 8.34 (d, *J* = 5 Hz, 1 H).<br>LCMS m/z $[M+H]^+$ = 464.3 | 23 |
| **183** | (5S)-1'-[7-(2,3-dihydro-1,4-benzodioxin-6-yl)-6-methyl-pyrazolo[1,5-a]pyrazin-4-yl] spiro[5,7-dihydrocyclopenta[b] pyr idine-6,4'-piperidine]-5-amine | $^1$H NMR (600 MHz, DMSO-*d6*) δ ppm 1.25 (br d, *J* = 13 Hz, 1 H), 1.60 (br d, *J* = 13 Hz, 1 H), 1.83 (td, *J* = 12, 4 Hz, 1 H), 1.89 (td, *J* = 12, 4 Hz, 1 H), 2.18 (s, 3 H), 2.81 (d, *J* = 16 Hz, 1 H), 3.15 (d, *J* = 16 Hz, 1 H), 3.30 - 3.37 (m, 2 H, partially masked by water), 3.97 (s, 1 H), 4.27 - 4.37 (m, 6 H), 6.88 (dd, *J* = 8, 2, 1 H), 6.92 - 6.97 (m, 3 H), 7.19 (dd, *J* = 7, 5 Hz, 1 H), 7.69 (d, *J* = 7 Hz, 1 H), 7.83 (d, *J* = 2 Hz, 1 H), 8.35 (d, *J* = 5 Hz, 1 H).<br>LCMS m/z $[M+H]^+$ = 469.2 | 3 |
| 184 | (5S)-1'-[6-methyl-7-(1-methylindol-6-yl)pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.27 (br d, *J* = 13 Hz, 1 H), 1.61 (br d, *J* = 13 Hz, 1 H), 1.85 (td, *J* = 12, 4 Hz, 1 H), 1.92 (td, *J* = 12, 4 Hz, 1 H), 2.19 (s, 3 H), 2.83 (d, *J* = 16 Hz, 1 H), 3.16 (d, *J* = 16 Hz, 1 H), 3.30 - 3.40 (m, 2 H, partially masked by water), 3.80 (s, 3 H), 3.99 (s, 1 H), 4.30 - 4.38 (m, 2 H), 6.50 (d, *J* = 3 Hz, 1 H), 6.94 (d, *J* = 2 Hz, 1 H), 7.05 (dd, *J* = 8, 1 Hz, 1 H), 7.20 (dd, *J* = 7, 5 Hz, 1 H), 7.41 (d, *J* = 3 Hz, 1 H), 7.52 (s, 1 H), 7.64 (d, *J* = 8 Hz, 1 H), 7.70 (d, *J* = 8 Hz, 1 H), 7.82 (d, *J* = 2 Hz, 1 H), 8.35 (d, *J* = 5 Hz, 1 H).<br>LCMS m/z $[M+H]^+$ = 464.3 | 11 |
| **185** | (5S)-1'-[7-(2-fluoro-4-methoxy-phenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.61 (br d, *J* = 13 Hz, 1 H), 1.79 - 1.94 (m, 2 H), 2.14 (s, 3 H), 2.83 (d, *J* = 16 Hz, 1 H), 3.16 (d, *J* = 16 Hz, 1 H), 3.30 - 3.41 (m, 2 H), 3.85 (s, 3 H), 3.98 (s, 1 H), 4.34 - 4.42 (m, 2 H), 6.92 (dd, *J* = 8, 2 Hz, 1 H), 6.95-7.00 (m, 2 H), 7.19 (dd, *J* = 7, 5 Hz, 1 H), 7.38 (t, *J* = 8 Hz, 1 H), 7.69 (d, *J* = 7 Hz, 1 H), 7.83 (d, *J* = 2 Hz, 1 H), 8.35 (d, *J* = 5 Hz, 1 H).<br>LCMS m/z $[M+H]^+$ = 459.2 | 7 |

(continued)

| Cpd. No. | Structure and Name | Characterization | SHP2 $IC_{50}$ (nM) |
|---|---|---|---|
| **186** | (5S)-1'-[7-(2-fluoro-4-methyl-phenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.25 (br d, *J* = 13 Hz, 1 H), 1.61 (br d, *J*= 13 Hz, 1 H), 1.79 - 1.95 (m, 2 H), 2.13 (s, 3 H), 2.41 (s, 3 H), 2.81 (d, *J* = 16 Hz, 1 H), 3.15 (d, *J*= 16 Hz, 1 H), 3.30 - 3.41 (m, 2 H, partially masked by water), 3.96 (s, 1 H), 4.34 - 4.43 (m, 2 H), 6.95-6.98 (m, 1 H), 7.13 - 7.21 (m, 3 H), 7.35 (t, *J*= 8 Hz, 1 H), 7.68 (d, *J* = 8 Hz, 1 H), 7.83 (d, *J* = 2 Hz, 1 H), 8.34 (d, *J* = 5 Hz, 1 H).<br>LCMS m/z $[M+H]^+$ = 443.2 | 2 |
| **187** | (5S)-1'-[7-[2-fluoro-5-(trifluoromethyl)phenyl] -6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.25 (br d, *J* = 13 Hz, 1 H), 1.62 (br d, *J* = 13 Hz, 1 H), 1.79 - 1.94 (m, 2 H), 2.15 (s, 3 H), 2.82 (d, *J* = 16 Hz, 1 H), 3.16 (d, *J* = 16 Hz, 1 H), 3.34 - 3.45 (m, 2 H), 3.96 (s, 1 H), 4.39 - 4.48 (m, 2 H), 7.01 -7.04 (m, 1 H), 7.19 (dd, *J* = 7, 5 Hz, 1 H), 7.63 (t, *J* = 9 Hz, 1 H), 7.68 (d, *J* = 8 Hz, 1 H), 7.87 (d, *J* = 2 Hz, 1 H), 7.94 - 8.00 (m, 2 H), 8.34 (d, *J* = 5 Hz, 1 H).<br>LCMS m/z $[M+H]^+$ = 497.2 | 250 |
| **188** | (5S)-1'-[7-(2,3-dihydrobenzofuran-5-yl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.24 (br d, *J* = 13 Hz, 1 H), 1.60 (br d, *J* = 13 Hz, 1 H), 1.83 (td, *J* = 12, 4 Hz, 1 H), 1.90 (td, *J* = 12, 4 Hz, 1 H), 2.17 (s, 3 H), 2.80 (d, *J* = 16 Hz, 1 H), 3.14 (d, *J* = 16 Hz, 1 H), 3.24 (t, *J* = 9 Hz, 2 H), 3.27 - 3.38 (m, 2 H, partially masked by water), 3.96 (s, 1 H), 4.28 - 4.36 (m, 2 H), 4.60 (t, *J* = 9 Hz, 2 H), 6.86 (d, *J* = 8 Hz, 1 H), 6.93 (d, *J* = 2 Hz, 1 H), 7.14 (d, *J* = 8 Hz, 1 H), 7.19 (dd, *J* = 7, 5 Hz, 1 H), 7.29 (s, 1 H), 7.68 (d, *J* = 7 Hz, 1 H), 7.83 (d, *J* = 2 Hz, 1 H), 8.34 (d, *J* = 5 Hz, 1 H).<br>LCMS m/z $[M+H]^+$ = 453.2 | 7 |
| **189** | 2-[4-[6-methyl-4-[(5S)-5-aminospiro[5,7-dihydrocyclopenta[b]pyr idine-6,4'-piperidine]-1'-yl]pyrazolo [1,5-a]pyrazin-7-yl]phenyl] acetonitrile | $^1$H NMR (600 MHz, DMSO-*d6*) δ ppm 1.22 (br d, *J* = 13 Hz, 1 H), 1.61 (br d, *J* = 13 Hz, 1 H), 1.83 (td, *J* = 12, 4 Hz, 1 H), 1.91 (td, *J* = 12, 4 Hz, 1 H), 2.17 (s, 3 H), 2.79 (d, *J* = 16 Hz, 1 H), 3.15 (d, *J* = 16 Hz, 1 H), 3.30 - 3.40 (m, 2 H, partially masked by water), 3.94 (s, 1 H), 4.14 (s, 2 H), 4.32 - 4.40 (m, 2 H), 6.96 (d, *J* = 2 Hz, 1 H), 7.18 (dd, *J* = 7, 5 Hz, 1 H), 7.45 - 7.51 (m, 4 H), 7.67 (d, *J* = 7 Hz, 1 H), 7.83 (d, *J* = 2 Hz, 1 H), 8.33 (d, *J* = 5 Hz, 1 H).<br>LCMS m/z $[M+H]^+$ = 450.2 | 4 |
| **190** | 5-[6-methyl-4-[(5S)-5-aminospiro[5,7-dihydrocyclopenta[b]pyr idine-6,4'-piperidine]-1'-yl]pyrazolo [1,5-a]pyrazin-7-yl]thiophene-2-carbonitrile | $^1$H NMR (600 MHz, DMSO-*d6*) δ ppm 1.23 (br d, *J* = 13 Hz, 1 H), 1.62 (br d, *J* = 13 Hz, 1 H), 1.80 (td, *J* = 12, 4 Hz, 1 H), 1.88 (td, *J* = 12, 4 Hz, 1 H), 2.41 (s, 3 H), 2.81 (d, *J* = 16 Hz, 1 H), 3.16 (d, *J* = 16 Hz, 1 H), 3.38 - 3.50 (m, 2 H), 3.94 (s, 1 H), 4.44 - 4.53 (m, 2 H), 7.11 (d, *J* = 2 Hz, 1 H), 7.18 (dd, *J* = 7, 5 Hz, 1 H), 7.56 (d, *J* = 4 Hz, 1 H), 7.67 (d, *J* = 7 Hz, 1 H), 8.02 (dd, *J* = 6, 3 Hz, 2 H), 8.33 (d, *J* = 5 Hz, 1 H).<br>LCMS m/z $[M+H]^+$ = 442.2 | 31 |

(continued)

| Cpd. No. | Structure and Name | Characterization | SHP2 $IC_{50}$ (nM) |
|---|---|---|---|
| **191** | [3-[6-methyl-4-[(5S)-5-amino spiro [5,7-dihydrocyclopenta[b] pyr idine-6,4'-piperidine]-1'-yl] pyrazolo[1,5-a]pyrazin-7-yl] phenyl]methanol | $^1$H NMR (600 MHz, DMSO-*d6*) δ ppm 1.24 (br d, *J* = 13 Hz, 1 H), 1.61 (br d, *J* = 13 Hz, 1 H), 1.84 (td, *J* = 12, 4 Hz, 1 H), 1.91 (td, *J* = 12, 4 Hz, 1 H), 2.16 (s, 3 H), 2.80 (d, *J* = 16 Hz, 1 H), 3.15 (d, *J* = 16 Hz, 1 H), 3.30 - 3.40 (m, 2 H, partially masked by water), 3.96 (s, 1 H), 4.31 - 4.39 (m, 2 H), 4.56 (d, *J* = 6 Hz, 2 H), 5.24 (t, *J* = 6 Hz, 1 H), 6.95 (d, *J* = 2 Hz, 1 H), 7.19 (dd, *J* = 7, 5 Hz, 1 H), 7.30 (d, *J* = 7 Hz, 1 H), 7.36 - 7.48 (m, 3 H), 7.68 (d, *J* = 7 Hz, 1 H), 7.83 (d, *J* = 2 Hz, 1 H), 8.33 (d, *J* = 5 Hz, 1 H).<br>LCMS m/z $[M+H]^+$ = 441.2 | 16 |
| **192** | (5S)-1'-[7-(2,3-dichlorophenyl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.23 (br d, *J* = 13 Hz, 1 H), 1.63 (br d, *J* = 13 Hz, 1 H), 1.83 (td, *J* = 12, 4 Hz, 1 H), 1.91 (td, *J* = 12, 4 Hz, 1 H), 2.07 (s, 3 H), 2.80 (d, *J* = 16 Hz, 1 H), 3.15 (d, *J* = 16 Hz, 1 H), 3.32 - 3.44 (m, 2 H), 3.94 (s, 1 H), 4.36 - 4.46 (m, 2 H), 6.99 (t, *J* = 2 Hz, 1 H), 7.18 (dd, *J* = 7, 5 Hz, 1 H), 7.47 - 7.54 (m, 2 H), 7.67 (d, *J* = 7 Hz, 1 H), 7.79 (dd, *J* = 8, 2 Hz, 1 H), 7.84 (d, *J* = 2 Hz, 1 H), 8.33 (d, *J* = 5 Hz, 1 H).<br>LCMS m/z $[M+H]^+$ = 479.1 | 2 |
| **193** | (5S)-1'-[7-(2,4-difluorophenyl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.24 (br d, *J* = 13 Hz, 1 H), 1.62 (br d, *J* = 13 Hz, 1 H), 1.78 - 1.94 (m, 2 H), 2.15 (s, 3 H), 2.81 (d, *J* = 16 Hz, 1 H), 3.16 (d, *J*= 16 Hz, 1 H), 3.33 - 3.43 (m, 2 H), 3.95 (s, 1 H), 4.37 - 4.45 (m, 2 H), 6.99 - 7.02 (m, 1 H), 7.19 (dd, *J* = 7, 5 Hz, 1 H), 7.39 - 7.47 (m, 3 H), 7.67 (d, *J* = 7 Hz, 1 H), 7.86 (d, *J* = 2 Hz, 1 H), 8.33 (d, *J* = 5 Hz, 1 H).<br>LCMS m/z $[M+H]^+$ = 447.2 | 3 |
| **194** | (5S)-1'-[6-methyl-7-(3-thienyl) pyrazolo[1,5-a]pyrazin-4-yl] spiro[5,7-dihydrocyclopenta[b] pyr idine-6,4'-piperidine]-5-amine | $^1$H NMR (600 MHz, DMSO-*d6*) δ ppm 1.21 (br d, *J* = 13 Hz, 1 H), 1.60 (br d, *J* = 13 Hz, 1 H), 1.82 (td, *J* = 12, 4 Hz, 1 H), 1.90 (td, *J* = 12, 4 Hz, 1 H), 2.26 (s, 3 H), 2.78 (d, *J* = 16 Hz, 1 H), 3.14 (d, *J* = 16 Hz, 1 H), 3.30 - 3.40 (m, 2 H, partially masked by water), 3.92 (s, 1 H), 4.30 - 4.39 (m, 2 H), 6.96 (d, *J* = 2 Hz, 1 H), 7.18 (dd, *J* = 7, 5 Hz, 1 H), 7.32 (dd, *J* = 5, 1 Hz, 1 H), 7.64 - 7.68 (m, 2 H), 7.77 (dd, *J* = 3, 1 Hz, 1 H), 7.83 (d, *J* = 2 Hz, 1 H), 8.32 (d, *J* = 5 Hz, 1 H).<br>LCMS m/z $[M+H]^+$ = 417.1 | 5 |
| **195** | (5S)-1'-[7-(2,5-difluorophenyl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.24 (br d, *J* = 13 Hz, 1 H), 1.62 (br d, *J* = 13 Hz, 1 H), 1.78 - 1.94 (m, 2 H), 2.15 (s, 3 H), 2.81 (d, *J* = 16 Hz, 1 H), 3.16 (d, *J*= 16 Hz, 1 H), 3.33 - 3.43 (m, 2 H), 3.95 (s, 1 H), 4.37 - 4.45 (m, 2 H), 6.99 - 7.02 (m, 1 H), 7.19 (dd, *J* = 7, 5 Hz, 1 H), 7.39 - 7.47 (m, 3 H), 7.67 (d, *J* = 7 Hz, 1 H), 7.86 (d, *J* = 2 Hz, 1 H), 8.33 (d, *J* = 5 Hz, 1 H).<br>LCMS m/z $[M+H]^+$ = 447.2 | 4 |

(continued)

| Cpd. No. | Structure and Name | Characterization | SHP2 $IC_{50}$ (nM) |
|---|---|---|---|
| **196** | (5S)-1'-[7-(3,6-dihydro-2H-pyran-4-yl)-6-methyl-pyrazolo [1,5-a]pyrazin-4-yl]spiro[5,7-dihydrocyclopenta[b]pyridine-6,4'-piperidine]-5-amine | $^1$H NMR (600 MHz, DMSO-*d6*) δ ppm 1.22 (br d, *J* = 13 Hz, 1 H), 1.58 (br d, *J* = 13 Hz, 1 H), 1.80 (td, *J* = 12, 4 Hz, 1 H), 1.87 (td, *J* = 12, 4 Hz, 1 H), 2.30 (s, 3 H), 2.37 - 2.44 (m, 2 H), 2.79 (d, *J* = 16 Hz, 1 H), 3.13 (d, *J* = 16 Hz, 1 H), 3.25 - 3.35 (m, 2 H, partially masked by water), 3.86 (t, *J* = 5 Hz, 2 H), 3.95 (s, 1 H), 4.24 - 4.34 (m, 4 H), 5.91 - 5.95 (m, 1H), 6.91 (d, *J* = 2 Hz, 1 H), 7.18 (dd, *J* = 7, 5 Hz, 1 H), 7.67 (d, *J* = 7 Hz, 1 H), 7.91 (d, *J* = 2 Hz, 1 H), 8.33 (d, *J* = 5 Hz, 1 H). LCMS m/z $[M+H]^+$ = 417.2 | 83 |
| **197** | (5S)-1'-[7-(3-fluoro-2-methyl-phenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.62 (br d, *J*= 13 Hz, 1 H), 1.81 - 1.95 (m, 5H), 2.07 (s, 3 H), 2.81 (d, *J* = 16 Hz, 1 H), 3.15 (d, *J*= 16 Hz, 1 H), 3.30 - 3.42 (m, 2 H), 3.97 (s, 1 H), 4.33 - 4.43 (m, 2 H), 6.97 - 7.00 (m, 1 H), 7.13 (d, *J* = 7 Hz, 1 H), 7.19 (dd, *J* = 7, 5 Hz, 1 H), 7.28 (t, *J* = 9 Hz, 1H), 7.33 -7.38 (m, 1 H), 7.68 (d, *J* = 7 Hz, 1 H), 7.84 (d, *J* = 2 Hz, 1 H), 8.34 (d, *J* = 5 Hz, 1 H). LCMS m/z $[M+H]^+$ = 443.2 | 7 |
| **198** | (5S)-1'-[7-(3-fluoro-2-morpholino-4-pyridyl)-6-methyl-pyrazolo[1,5-a]pyrazin-4-yl] spiro[5,7-dihydrocyclopenta[b] pyr idine-6,4'-piperidine]-5-amine | $^1$H NMR (600 MHz, DMSO-*d6*) δ ppm 1.24 (br d, *J* = 13 Hz, 1 H), 1.61 (br d, *J*= 13 Hz, 1 H), 1.78 - 1.94 (m, 2 H), 2.16 (s, 3 H), 2.81 (d, *J* = 16 Hz, 1 H), 3.15 (d, *J*= 16 Hz, 1 H), 3.34 - 3.45 (m, 6 H), 3.71 - 3.76 (m, 4 H), 3.96 (s, 1 H), 4.38 - 4.46 (m, 2 H), 6.99 - 7.03 (m, 2 H), 7.18 (dd, *J* = 7, 5 Hz, 1 H), 7.67 (d, *J* = 7 Hz, 1 H), 7.88 (d, *J* = 2 Hz, 1 H), 8.14 (d, *J* = 5 Hz, 1 H), 8.33 (d, *J* = 5 Hz, 1 H). LCMS m/z $[M+H]^+$ = 515.3 | 437 |
| **199** | (5S)-1'-[7-(5-chloro-3-pyridyl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.62 (br d, *J* = 13 Hz, 1 H), 1.82 (td, *J* = 12, 4 Hz, 1 H), 1.89 (td, *J* = 12, 4 Hz, 1 H), 2.21 (s, 3 H), 2.82 (d, *J* = 16 Hz, 1 H), 3.16 (d, *J* = 16 Hz, 1 H), 3.33 - 3.45 (m, 2 H), 3.97 (s, 1 H), 4.38 - 4.47 (m, 2 H), 7.03 (d, *J* = 2 Hz, 1 H), 7.19 (dd, *J* = 7, 5 Hz, 1 H), 7.68 (d, *J* = 7 Hz, 1 H), 7.89 (d, *J* = 2 Hz, 1 H), 8.15 (t, *J* = 2 Hz, 1 H), 8.34 (d, *J* = 5 Hz, 1 H), 8.65 (d, *J* = 2 Hz, 1 H), 8.71 (d, *J* = 2 Hz, 1 H). LCMS m/z $[M+H]^+$ = 446.2 | 14 |
| **200** | (5S)-1'-[7-(2-fluoro-3-methoxy-phenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.26 (br d, *J* = 13 Hz, 1 H), 1.62 (br d, *J* = 13 Hz, 1 H), 1.84 (td, *J* = 12, 4 Hz, 1 H), 1.91 (td, *J* = 12, 4 Hz, 1 H), 2.10 (s, 3 H), 2.82 (d, *J* = 16 Hz, 1 H), 3.16 (d, *J* = 16 Hz, 1 H), 3.30 - 3.40 (m, 2 H, partially masked by water), 3.61 (d, *J* = 1 Hz, 3 H), 3.97 (s, 1 H), 4.33 - 4.42 (m, 2 H), 6.94 - 6.98 (m, 1 H), 7.13 (d, *J* = 8 Hz, 1 H), 7.17 - 7.24 (m, 2 H), 7.40 (ddd, *J* = 12, 8, 1 Hz, 1 H), 7.69 (d, *J* = 7 Hz, 1 H), 7.83 (d, *J* = 2 Hz, 1 H), 8.34 (d, *J* = 5 Hz, 1 H). LCMS m/z $[M+H]^+$ = 459.2 | 44 |

(continued)

| Cpd. No. | Structure and Name | Characterization | SHP2 $IC_{50}$ (nM) |
|---|---|---|---|
| **201** | (5S)-1'-[7-(3,4-difluorophenyl)-6-methyl-pyrazolo[1,5-a] pyrazin-4-yl]spiro[5,7-dihydrocyclopenta[b]pyr idine-6,4'-piperidine]-5-amine | $^1$H NMR (600 MHz, DMSO-*d6*) δ ppm 1.21 (br d, *J* = 13 Hz, 1 H), 1.61 (br d, *J* = 13 Hz, 1 H), 1.82 (td, *J* = 12, 4 Hz, 1 H), 1.90 (td, *J* = 12, 4 Hz, 1 H), 2.18 (s, 3 H), 2.79 (d, *J* = 16 Hz, 1 H), 3.15 (d, *J* = 16 Hz, 1 H), 3.30 - 3.41 (m, 2 H, partially masked by water), 3.92 (s, 1 H), 4.33 - 4.41 (m, 2 H), 6.99 (d, *J* = 2 Hz, 1 H), 7.18 (dd, *J* = 7, 5 Hz, 1 H), 7.31 - 7.36 (m, 1 H), 7.51 - 7.64 (m, 2 H), 7.66 (d, *J* = 7 Hz, 1 H), 7.86 (d, J = 2 Hz, 1 H), 8.32 (d, *J* = 5 Hz, 1 H).<br>LCMS m/z $[M+H]^+$ = 447.2 | 3 |
| **202** | (5S)-1'-[6-methyl-7-(6-quinolyl) pyrazolo[1,5-a]pyrazin-4-yl] spiro[5,7-dihydrocyclopenta[b] pyr idine-6,4'-piperidine]-5-amine | $^1$H NMR (600 MHz, DMSO-*d6*) δ ppm 1.26 (br d, *J* = 13 Hz, 1 H), 1.63 (br d, *J* = 13 Hz, 1 H), 1.86 (td, *J* = 12, 4 Hz, 1 H), 1.93 (td, *J* = 12, 4 Hz, 1 H), 2.24 (s, 3 H), 2.83 (d, *J* = 16 Hz, 1 H), 3.17 (d, *J* = 16 Hz, 1 H), 3.30 - 3.44 (m, 2 H, partially masked by water), 3.98 (br s, 1 H), 4.37 - 4.45 (m, 2 H), 7.01 (d, *J* = 2 Hz, 1 H), 7.20 (dd, *J* = 7, 5 Hz, 1 H), 7.59 (dd, *J* = 8, 4 Hz, 1 H), 7.69 (d, *J* = 7 Hz, 1 H), 7.81 - 7.89 (m, 2 H), 8.10 - 8.14 (m, 2 H), 8.35 (d, *J* = 5 Hz, 1 H), 8.42 (d, *J* = 7 Hz, 1 H), 8.97 (dd, *J* = 4, 2 Hz, 1 H).<br>LCMS m/z $[M+H]^+$ = 462.3 | 26 |
| **203** | (5S)-1'-[7-(3-fluorophenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.21 (br d, *J* = 13 Hz, 1 H), 1.61 (br d, *J* = 13 Hz, 1 H), 1.83 (td, *J* = 12, 4 Hz, 1 H), 1.90 (td, *J* = 12, 4 Hz, 1 H), 2.18 (s, 3 H), 2.78 (d, *J* = 16 Hz, 1 H), 3.15 (d, *J* = 16 Hz, 1 H), 3.30 - 3.41 (m, 2 H, partially masked by water), 3.92 (br s, 1 H), 4.33 - 4.41 (m, 2 H), 6.98 (d, *J* = 2 Hz, 1 H), 7.18 (dd, *J* = 7, 5 Hz, 1 H), 7.27 - 7.32 (m, 2 H), 7.32 - 7.37 (m, 1 H), 7.51 - 7.57 (m, 1 H), 7.66 (d, *J* = 7 Hz, 1 H), 7.85 (d, *J* = 2 Hz, 1 H), 8.32 (d, *J* = 5 Hz, 1 H).<br>LCMS m/z $[M+H]^+$ = 429.2 | 6 |
| **204** | 1-[4-fluoro-3-[6-methyl-4-[(5S)-5-amino spiro [5,7-dihydrocyclopenta[b]pyr idine-6,4'-piperidine]-1'-yl]pyrazolo [1,5-a]pyrazin-7-yl]phenyl] ethanone | $^1$H NMR (600 MHz, DMSO-*d6*) δ ppm 1.25 (br d, *J* = 13 Hz, 1 H), 1.62 (br d, *J* = 13 Hz, 1 H), 1.76 - 1.94 (m, 2 H), 2.16 (s, 3 H), 2.60 (s, 3 H), 2.82 (d, *J* = 16 Hz, 1 H), 3.16 (d, *J* = 16 Hz, 1 H), 3.30 - 3.45 (m, 2 H, partially masked by water), 3.96 (br s, 1 H), 4.39 - 4.47 (m, 2 H), 7.00 - 7.04 (m, 1 H), 7.19 (dd, *J* = 7, 5 Hz, 1 H), 7.52 (t, *J* = 9 Hz, 1 H), 7.68 (d, *J* = 7 Hz, 1 H), 7.85 (d, *J* = 2 Hz, 1 H), 8.09 - 8.12 (m, 1 H), 8.13 - 8.17 (m, 1 H), 8.34 (d, *J* = 5 Hz, 1 H).<br>LCMS m/z $[M+H]^+$ = 471.3 | 55 |
| **205** | (5S)-1'-[7-(2-fluoro-5-methyl-phenyl)-6-methyl-pyrazolo[1,5-a]pyrazin-4-yl]spiro[5,7-dihydrocyclopenta[b]pyr idine-6,4'-piperidine]-5-amine | $^1$H NMR (600 MHz, DMSO-*d6*) δ ppm 1.25 (br d, *J* = 13 Hz, 1 H), 1.61 (br d, *J* = 13 Hz, 1 H), 1.76 - 1.94 (m, 2 H), 2.14 (s, 3 H), 2.34 (s, 3 H), 2.81 (d, *J* = 16 Hz, 1 H), 3.15 (d, *J* = 16 Hz, 1 H), 3.30 - 3.42 (m, 2 H, partially masked by water), 3.95 (s, 1 H), 4.34 - 4.43 (m, 2 H), 6.96 - 6.99 (m, 1 H), 7.16 - 7.25 (m, 2 H), 7.28 (br d, *J* = 6 Hz, 1 H), 7.31 - 7.35 (m, 1 H), 7.68 (d, *J* = 7 Hz, 1 H), 7.84 (d, *J* = 2 Hz, 1 H), 8.34 (d, *J* = 5 Hz, 1 H).<br>LCMS m/z $[M+H]^+$ = 471.3 | 11 |

(continued)

| Cpd. No. | Structure and Name | Characterization | SHP2 $IC_{50}$ (nM) |
|---|---|---|---|
| **206** | 3-fluoro-4-[6-methyl-4-[(5S)-5-aminospiro[5,7-dihydrocyclopenta[b]pyr idine-6,4'-piperidine]-1'-yl]pyrazolo [1,5-a]pyrazin-7-yl]benzonitrile | $^1$H NMR (600 MHz, DMSO-*d6*) δ ppm 1.25 (br d, *J* = 13 Hz, 1 H), 1.61 (br d, *J* = 13 Hz, 1 H), 1.76 - 1.94 (m, 2 H), 2.15 (s, 3 H), 2.82 (d, *J* = 16 Hz, 1 H), 3.16 (d, *J* = 16 Hz, 1 H), 3.30 - 3.46 (m, 2 H, partially masked by water), 3.96 (s, 1 H), 4.40 - 4.48 (m, 2 H), 7.01 - 7.04 (m, 1 H), 7.16 - 7.21 (m, 1 H), 7.68 (d, *J* = 7 Hz, 1 H), 7.78 (d, *J* = 7 Hz, 1 H), 7.83 - 7.88 (m, 2H), 8.00 - 8.05 (m, 1H), 8.33 (d, *J* = 5 Hz, 1 H). LCMS m/z $[M+H]^+$ = 454.2 | 12 |

**Example S12. [4-[4-[(5S)-5-aminospiro[5,7-dihydrocyclopenta[b]pyridine-6,4'-pipetidine]-1'-yl]-6-methyl-pyrazolo[1,5-a]pyrazin-7-yl]-3-chloro-2-pyridyl]methanol (Compound 207)**

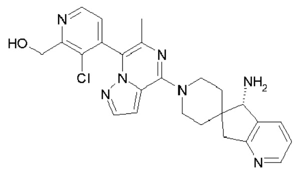

**Step a:** As described for **Example S4** (Compound 31), a similar procedure was performed using **intermediate A-1** (220 mg, 0.425 mmol) and **intermediate C-4** (173 mg, 0.64 mmol) to give (R)-N-[(5S)-1'-[7-[3-chloro-2-(tetrahydropyran-2-yloxymethyl)-4-pyridyl]-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (225 mg, 0.34 mmol) as a brown wax. LCMS m/z $[M+H]^+$ = 664.2.

**Step b:** As described for **Example S4** (Compound **31**) - step b, a similar procedure was performed using (R)-N-[(5S)-1'-[7-[3-chloro-2-(tetrahydropyran-2-yloxymethyl)-4-pyridyl]-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (94 mg, 0.14mmol) and a solution of HCl (4 N in dioxane, 0.25 mL, 1 mmol) to give [4-[4-[(5S)-5-aminospiro[5,7-dihydrocyclopenta[b]pyridine-6,4'-piperidine]-1'-yl]-6-methyl-pyrazolo[1,5-a]pyrazin-7-yl]-3-chloro-2-pyridyl]methanol (52 mg, 0.11 mmol) as a beige solid. LCMS m/z $[M+H]^+$ = 476.2. $^1$H NMR (400 MHz, DMSO-*d6*) δ 1.25 (br d, *J* = 13 Hz, 1 H), 1.64 (br d, *J* = 13 Hz, 1 H), 1.76 - 1.98 (m, 2 H), 2.08 (s, 3 H), 2.08 - 2.21 (m, 2 H), 2.80 (d, *J*= 16 Hz, 1 H), 3.16 (d, *J* = 16 Hz, 1 H), 3.35 - 3.50 (m, 2 H), 3.95 (s, 1 H), 4.44 (ddd, *J* = 13, 8, 4 Hz, 2 H), 4.74 (d, *J*= 6 Hz, 2 H), 5.28 (t, *J* = 6 Hz, 1 H), 7.02 (dd, *J* = 3, 2 Hz, 1 H), 7.19 (dd, *J* = 8, 5 Hz, 1 H), 7.52 (d, *J* = 5 Hz, 1 H), 7.68 (dt, *J* = 7, 1 Hz, 1 H), 7.85 (d, *J* = 3 Hz, 1 H), 8.33 (dd, *J* = 4, 1 Hz, 1 H), 8.65 (d, *J* = 5 Hz, 1 H). SHP2 $IC_{50}$ is 16 nM.

**Example S13. (5S)-1'-[7-(4-fluoro-1H-indol-7-yl)-6-methyl-pyrazolo[1,5-a]pyrazin-4-yl]spiro[5,7-dihydrocyclopenta[b]pyridine-6,4'-piperidine]-5-amine (Compound 208)**

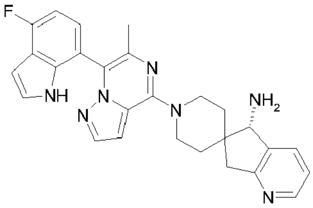

**Step a:** A mixture of **intermediate D-1** (150 mg, 0.29 mmol), 4-fluoro-7-(4,4,5,5-etramethyl-1,3,2-dioxaborolan-2-yl)-1H-indole (100 mg, 0.38 mmol), potassium carbonate (140 mg, 1.01 mmol) and $Pd(dppf)Cl_2.CH_2Cl_2$ (25 mg, 0.03 mmol) in a mixture of dioxane (1.8 mL) and water (0.3 mL) was degassed with Ar for 5 min and then heated under

microwave irradiation at 110 °C for 45 min. EtOAc (10 mL) and water (10 mL) were added, the aqueous layer was separated and then extracted with EtOAc (10 mL). The combined organic layers were washed with brine (10 mL), dried over $Na_2SO_4$ and filtered and concentrated under reduced pressure. The residue was purified by column chromatography ($SiO_2$, DCM/methanol = 1/0 to 95/5) to give N-[(5S)-1'-[7-(4-fluoro-1H-indol-7-yl)-6-methyl-pyrazolo[1,5-a]pyridin-4-yl]spiro[5,7-dihydrocyclopenta[b]pyridine-6,4'-piperidine]-5-yl]-2-methyl-propane-2-sulfinamide (110 mg, 0.19 mmol) as a light brown solid. LCMS m/z $[M+H]^+$ = 572.3.

**Step b:** As described for **Example S1** - step b, a similar procedure was performed using N-[(5S)-1'-[7-(4-fluoro-1H-indol-7-yl)-6-methyl-pyrazolo[1,5-a]pyridin-4-yl]spiro[5,7-dihydrocyclopenta[b]pyridine-6,4'-piperidine]-5-yl]-2-methyl-propane-2-sulfinamide (105 mg, 0.18 mmol) and a solution of HCl (4 N in dioxane, 0.5 mL, 2 mmol) to give (5S)-1'-[7-(4-fluoro-1H-indol-7-yl)-6-methyl-pyrazolo[1,5-a]pyrazin-4-yl]spiro[5,7-dihydrocyclopenta[b]pyridine-6,4'-piperidine]-5-amine (37 mg, 0.08 mmol) as a white solid. LCMS m/z $[M+H]^+$ = 468.3. $^1H$ NMR (400 MHz, DMSO-*d6*) δ ppm 1.26 (br d, *J* = 13 Hz, 1 H), 1.65 (br d, *J* = 13 Hz, 1 H), 1.82 - 2.03 (m, 4 H), 2.09 (s, 3 H), 2.81 (d, *J* = 16 Hz, 1 H), 3.17 (d, *J* = 16 Hz, 1 H), 3.34 - 3.44 (m, 2 H), 3.95 (s, 1 H), 4.35 - 4.46 (m, 2 H), 6.56 (dd, *J* = 3, 2 Hz, 1 H), 6.90 (dd, *J* = 10, 8 Hz, 1 H), 6.95 (d, *J* = 2 Hz, 1 H), 7.09 (dd, *J* = 8, 5 Hz, 1 H), 7.19 (dd, *J* = 7, 5 Hz, 1 H), 7.27 (t, *J* = 3 Hz, 1 H), 7.68 (d, *J* = 7 Hz, 1 H), 7.76 (d, *J* = 2 Hz, 1 H), 8.33 (d, *J* = 5 Hz, 1 H), 11.05 (br s, 1 H). SHP2 $IC_{50}$ is 3 nM.

**Example S14. (5S)-5-amino-1'-[7-(2-fluorophenyl)-6-methyl-pyrazolo[1,5-a]pyrazin-4-yl]spiro[5,7-dihydrocyclopenta[b]pyridine-6,4'-piperidine]-3-carbonitrile (Compound 209)**

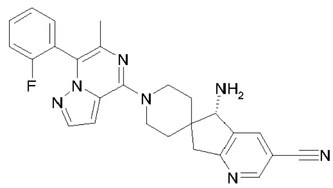

**Step a:** As described for **Example S4** (Compound **31**), a similar procedure was performed using **intermediate D-4** (165 mg, 0.3 mmol) and 2-fluorophenylboronic acid (65 mg, 0.46 mmol) to give N-[(5S)-3-chloro-1'-[7-(2-fluorophenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (44 mg, crude).

**Step b:** A solution of N-[(5S)-3-chloro-1'-[7-(2-fluorophenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (44 mg, crude) in DMF (1.5 mL) was degassed for 5 min with Ar, then zinc cyanide (20 mg, 0.17 mmol) and XPhos Pd G4 (7 mg, 8.1 μmol) were added. The mixture was stirred at 120 °C for 45min, the mixture was then let to cool down to RT. Ethyl acetate (20 mL) and water (20 mL) were added, the pH value of the aqueous phase was adjusted to 11-12 with 1 N aqueous NaOH solution. The organic layer was separated and the aqueous phase was extracted with ethyl acetate (20 mL). The combined organic phase was washed with brine, dried with anhydrous $Na_2SO_4$, filtered and concentrated under vacuum to give N-[(5S)-3-cyano-1'-[7-(2-fluorophenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (53 mg, crude) that was used without further purification.

**Step c:** As described for **Example S4** (Compound **31**) - step b, a similar procedure was performed using N-[(5S)-3-cyano-1'-[7-(2-fluorophenyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (53 mg, crude) and a solution of HCl (4 N in dioxane, 0.25 mL, 1 mmol) to give (5S)-5-amino-1'-[7-(2-fluorophenyl)-6-methyl-pyrazolo[1,5-a]pyrazin-4-yl]spiro[5,7-dihydrocyclopenta[b]pyridine-6,4'-piperidine]-3-carbonitrile (6 mg, 13 μmol) as a white solid. LCMS m/z $[M+H]^+$ = 454.2. $^1H$ NMK (400 MHz, DMSO-*d6*) δ ppm 1.27 (m, 1 H), 1.65 (br d, *J* = 13 Hz, 1 H), 1.78 - 2.02 (m, 2 H), 2.14 (s, 3 H), 2.95 (d, *J* = 16 Hz, 1 H), 3.26 (m, 1 H), 3.32 - 3.45 (m, 2 H), 4.04 (s, 1 H), 4.40 (br t, *J* = 11 Hz, 2 H), 6.99 (d, *J* = 2 Hz, 1 H), 7.32 - 7.40 (m, 2 H), 7.48 - 7.60 (m, 2 H), 7.85 (d, *J* = 2 Hz, 1 H), 8.11 (s, 1 H), 8.82 (d, *J* = 2 Hz, 1 H). SHP2 $IC_{50}$ is 21 nM.

**Example S15. (5S)-1'-(7-cyclopropyl-6-methyl-pyrazolo[1,5-a]pyrazin-4-yl)spiro[5,7-dihydrocyctopenta[b]pyridine-6,4'-piperidine]-5-amine (Compound 210)**

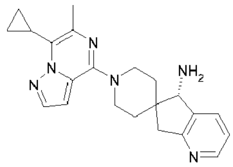

**Step a:** A mixture of **intermediate D-1** (60 mg, 0.11 mmol), potassium cyclopropyltrifluoroborate (52 mg, 0.35 mmol), potassium carbonate (56 mg, 0.40 mmol) and tetrakis(triphenylphosphine)palladium (6.8 mg, 5.8 μmol) in a mixture of toluene (0.6 mL) and water (0.06 mL) was degassed with Ar for 5 min and then heated at 100 °C for 18 h. The mixture was allowed to cool down to RT. EtOAc (10 mL) and water (5 mL) were added. The aqueous layer was separated and extracted with EtOAc (5 mL * 2). The combined organic layers were washed with a 1 N solution of NaOH (10 mL), brine (10 mL), dried over $Na_2SO_4$ and filtered and concentrated under reduced pressure. The residue was purified by column chromatography ($SiO_2$, dichloromethane/methanol = 1/0 to 95/5) to give (R)-N-((S)-1'-(7-cyclopropyl-6-methylpyrazolo[1,5-a]pyrazin-4-yl)-5,7-dihydrospiro[cyclopenta[b]pyridine-6,4'-piperidin]-5-yl)-2-methylpropane-2-sulfinamide (47 mg, 0.10 mmol) as an orange wax. LCMS m/z $[M+H]^+$ = 479.2.

**Step b:** As described for **Example S1** - step b, a similar procedure was performed using (R)-N-((S)-1'-(7-cyclopropyl-6-methylpyrazolo[1,5-a]pyrazin-4-yl)-5,7-dihydrospiro[cyclopenta[b]pyridine-6,4'-piperidin]-5-yl)-2-methylpropane-2-sulfinamide (47 mg, 0.10 mmol) and a solution of HCl (4 N in dioxane, 0.125 mL, 0.50 mmol) to give (5S)-1'-(7-cyclopropyl-6-methyl-pyrazolo[1,5-a]pyrazin-4-yl)spiro[5,7-dihydrocyclopenta[b]pyridine-6,4'-piperidine]-5-amine (31 mg, 0.08 mmol) as an off-white solid. LCMS m/z $[M+H]^+$ = 375.3. $^1$H NMR (400 MHz, DMSO-*d6*) δ 0.79 (m, *J* = 4 Hz, 2 H), 1.06 - 1.27 (m, 3 H), 1.57 (br d, *J* = 13 Hz, 1 H), 1.75 - 2.01 (m, 5 H), 2.41 (s, 3 H), 2.75 (d, *J* = 16 Hz, 1 H), 3.11 (d, *J* = 16 Hz, 1 H), 3.16 - 3.28 (m, 2 H), 3.90 (s, 1 H), 4.18 - 4.28 (m, 2 H), 6.87 (d, *J* = 2 Hz, 1 H), 7.17 (dd, *J* = 7, 5 Hz, 1 H), 7.66 (d, *J* = 7 Hz, 1 H), 7.94 (d, *J* = 2 Hz, 1 H), 8.31 (d, *J* = 5 Hz, 1 H). SHP2 $IC_{50}$ is > 1000 nM.

**Example S16. (3S)-3-amino-1'-[7-(1,5-dimethylpyrazol-4-yl)-6-methyl-pyrazolo[1,5-a]pyrazin-4-yl]spiro[indane-2,4'-piperidine]-5-carbonitrile (Compound 211) and (3R)-3-amino-1'-[7-(1,5-dimethylpyrazol-4-yl)-6-methyl-pyrazolo[1,5-a]pyrazin-4-yl]spiro[indane-2,4'-piperidine]-5-carbonitrile (Compound 212)**

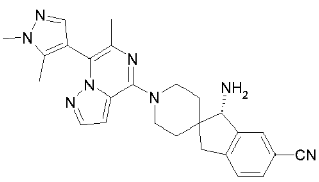

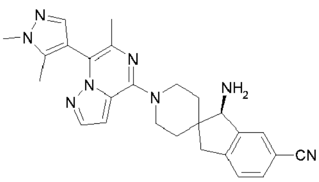

As described for **Example S4** (Compound **31),** a similar procedure was performed using **intermediate D-9** (160 mg, 0.37 mmol) and 1,5-dimethyl-4-(4,4,5,5-tetramethyl-1,3,2-dioxaborolan-2-yl)-1H-pyrazole (125 mg, 0.56 mmol) to give a mixture of enantiomers that was subjected to SFC purification using supercritical $CO_2$ and MeOH (+0.1% TEA) as a mobile phase on an IH-5μm, 3 x 25 cm column at 40 °C, with isocratic elution at 20% MeOH (+0.1% TEA) in $CO_2$, to give, as first eluting product, (3R)-3-amino-1'-[7-(1,5-dimethylpyrazol-4-yl)-6-methyl-pyrazolo[1,5-a]pyrazin-4-yl]spiro[indane-2,4'-piperidine]-5-carbonitrile (20 mg, 0.04 mmol) as a white solid followed by (3 S)-3-amino-1'-[7-(1,5-dimethylpyrazol-4-yl)-6-methyl-pyrazolo[1,5-a]pyrazin-4-yl]spiro [indane-2,4'-piperidine]-5-carbonitrile (56 mg, 0.12 mmol) as a white solid. LCMS m/z $[M+H]^+$ = 453.2. $^1$H NMR (400 MHz, DMSO-*d6*) 1.13 (br d, *J* = 13 Hz, 1 H), 1.61 *(d, J* = 13 Hz, 1 H), 1.79 (td, *J* = 12, 4 Hz, 1 H), 1.85 - 1.98 (m, 3 H), 2.06 (s, 3 H), 2.18 (s, 3 H), 2.75 (d, *J* = 17 Hz, 1 H), 3.21 (br d, *J* = 17 Hz, 1 H), 3.26 - 3.38 (m, 2 H), 3.83 (s, 3 H), 3.92 (s, 1 H), 4.18 - 4.45 (m, 2 H), 6.92 *(d, J=* 3 Hz, 1 H), 7.42 (d, *J* = 8 Hz, 1 H), 7.45 (s, 1 H), 7.64 *(d, J=* 8 Hz, 1 H), 7.71 (s, 1 H), 7.84 (d, *J* = 2 Hz, 1 H). Compound **211,** SHP2 $IC_{50}$ is 8 nM. Compound **212,** SHP2 $IC_{50}$ is 288 nM.

**Example S17. (7S)-1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-7-amine (Compound 213) and (7R)-1'-[7-(2,3-difluorophenyl)-6-methyl-pyrazolo [1,5-a]pyrazin-4-yl]-2-methoxy-spiro[5,7-dihydrocyclopenta[b]pyridine-6,4'-piperidine]-7-amine (Compound 214)**

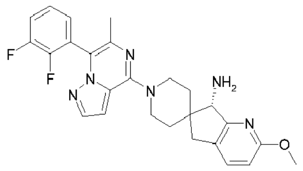 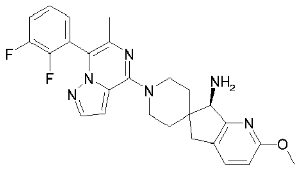

As described for **Example S1** - step a, a similar procedure was performed using **intermediate D-8** (250 mg, 0.46 mmol) and (2,3-difluorophenyl)boronic acid (109 mg, 0.69 mmol) to give tert-butyl N-[1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-7-yl]carbamate (198 mg, 0.34 mmol) as a mixture of diastereoisomers. This mixture was treated with TFA (0.38 mL, 5.1 mmol) in dichloromethane (0.5 mL) and after 2 h, the mixture was concentrated under vacuum. The residue was taken up in ethyl acetate (5 mL) and 1 N aqueous NaOH solution (5 mL), the aqueous layer was separated and extracted with ethyl acetate (5 mL). The combined organic phase was washed with brine (5 mL), dried with anhydrous $Na_2SO_4$, filtered and concentrated under vacuum. The residue was subjected to SFC purification using supercritical $CO_2$ and MeOH (+0.1% TEA) as a mobile phase on an IH-5μm, 3 x 25 cm column at 40 °C, with isocratic elution at 25% MeOH (+0.1% TEA) in $CO_2$, to give (7S)-1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-7-amine (38 mg, 80 μmol) and (7R)-1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-7-amine (21 mg, 44 μmol) as white foams. LCMS m/z $[M+H]^+$ = 477.2. $^1$H NMR (400 MHz, DMSO-*d6*) δ 1.35 (br d, *J* = 13 Hz, 1 H), 1.64 (br d, *J* = 13 Hz, 1 H), 1.77 - 2.04 (m, 4 H), 2.16 (s, 3 H), 2.65 (br d, *J* = 13 Hz, 1 H), 2.98 *(d, J* = 16 Hz, 1 H), 3.44 - 3.55 (m, 2 H), 3.87 (s, 3 H), 3.89 (s, 1 H), 4.24 - 4.35 (m, 2 H), 6.62 (d, *J* = 8 Hz, 1 H), 7.01 (dd, *J* = 3, 1 Hz, 1 H), 7.33 - 7.39 (m, 2 H), 7.51 - 7.62 (m, 2 H), 7.86 (d, *J* = 2 Hz, 1 H). Compound **213,** SHP2 $IC_{50}$ is 22 nM. Compound 214, SHP2 $IC_{50}$ is 594 nM.

**Example S18. (7S)-1'-[7-(2-chloro-3-fluoro-phenyl)-6-methyl-pyrazolo[1,5-a]pyrazin-4-yl]-2-methoxy-spiro[5,7-dihydrocyclopenta[b]pyridine-6,4'-piperidine]-7-amine (Compound 215) and (7R)-1'-[7-(2-chloro-3-fluoro-phenyl)-6-methyl-pyrazolo[1,5-a]pyrazin-4-yl]-2-methoxy-spiro[5,7-dihydrocyclopenta[b]pyridine-6,4'-piperidine]-7-amine (Compound 216)**

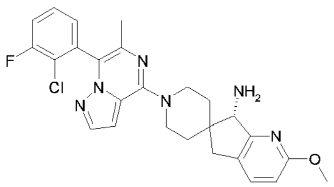 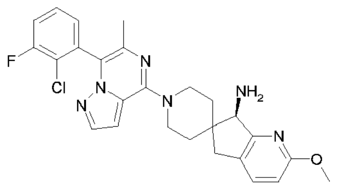

As described for **Example S17** (Compounds **213** and **214),** a similar procedure was performed using **intermediate D-8** (250 mg, 0.46 mmol) and 2-chloro-3-fluorophenylboronic acid (96 mg, 0.55 mmol) to give after deprotection a mixture of enantiomers that was subjected to SFC purification to give (7S)-1'-[7-(2-chloro-3-fluorophenyl)-6-methyl-pyrazolo[1,5-a]pyrazin-4-yl]-2-methoxy-spiro[5,7-dihydrocyclopenta[b]pyridine-6,4'-piperidine]-7-amine (41 mg, 83 μmol) and (7R)-1'-[7-(2-chloro-3-fluoro-phenyl)-6-methyl-pyrazolo[1,5-a]pyrazin-4-yl]-2-methoxy-spiro[5,7-dihydrocyclopenta[b]pyridine-6,4'-piperidine]-7-amine (22 mg, 44 μmol). LCMS m/z $[M+H]^+$ = 493.2. $^1$H NMR (500 MHz, DMSO-*d6*) δ 1.35 (br d, *J*= 14 Hz, 1 H), 1.64 (br d, *J*= 13 Hz, 1 H), 1.79 - 2.00 (m, 2 H), 2.08 (s, 3 H), 2.65 *(d, J* = 15 Hz, 1 H), 2.98 *(d, J* = 15 Hz, 1 H), 3.43 - 3.58 (m, 2 H), 3.87 (s, 3 H), 3.88 (s, 1 H), 4.29 (m, 2 H), 6.62 *(d, J* = 8 Hz, 1 H), 6.99 (dd, *J* = 3, 1 Hz, 1 H), 7.37 (m, 1 H), 7.49 - 7.60 (m, 3 H), 7.84 *(d, J* = 2 Hz, 1 H). Compound **215,** SHP2 $IC_{50}$ is 23 nM. Compound **216,** SHP2 $IC_{50}$ is 685 nM.

**Example S19. (1S)-1'-[7-(3-Chloro-2-methyl-4-pyridyl)-6-methyl-pyrazolo[1,5-a]pyrazin-4-yl]-5-fluoro-spiro[indane-2,4'-piperidine]-1-amine formate (Compound 217)**

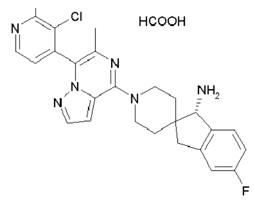

**Step a:** A mixture of 1'-(7-bromo-6-methyl-pyrazolo[1,5-a]pyrazin-4-yl)-5-fluoro-spiro[indane-2,4'-piperidine]-1-one (150 mg, 0.35 mmol), 3-chloro-2-methylpyridine-4-boronic acid (183 mg, 1.05 mmol), and sodium hydrogen carbonate (88 mg, 1.05 mmol) in dioxane (4 mL) and water (1 mL), was degassed with Ar for 10 min. After addition of palladium triphenylphosphine (48.5 mg, 0.042 mmol), the mixture was degassed again with Ar for 5 min, and then heated under microwave irradiation at 150 °C for 20 min. The reaction medium was allowed to cool down to RT and filtered through a 0.45 μm pore Whatman filter. Water was added to the filtrate and the resulting mixture was extracted with ethyl acetate. The combined organic layers were washed with brine, dried over $MgSO_4$, filtered and concentrated under reduced pressure. The residue was purified by column chromatography ($SiO_2$, Heptane/Ethyl acetate= 100/0 to 0/100) to give 1'-[7-(3-chloro-2-methyl-4-pyridyl)-6-methyl-pyrazolo[1,5-a]pyrazin-4-yl]-5-fluoro-spiro[indane-2,4'-piperidine]-1-one (35 mg, 0.074 mmol) as a pink solid. LCMS m/z $[M+H]^+$ = 476.

**Step b:** A mixture of 1'-[7-(3-chloro-2-methyl-4-pyridyl)-6-methyl-pyrazolo[1,5-a]pyrazin-4-yl]-5-fluoro-spiro[indane-2,4'-piperidine]-1-one (34 mg, 0.071 mmol), (R)-2-methylpropane-2-sulfinamide (17 mg, 0.143 mmol) and tetraethoxytitanium (65 mg, 0.286 mmol) was stirred at 100 °C for 15 h. The mixture was diluted with dichloromethane and water. Salts were removed by filtration and washed by dichloromethane. The organic layer was decanted from the filtrate and concentrated under reduced pressure. The residue was purified by column chromatography ($SiO_2$, Heptane/Ethyl acetate= 100/0 to 0/100) to give (NZ,R)-N-[1'-[7-(3-chloro-2-methyl-4-pyridyl)-6-methyl-pyrazolo[1,5-a]pyrazin-4-yl]-5-fluoro-spiro[indane-2,4'-piperidine]-1-ylidene]-2-methyl-propane-2-sulfinamide (32 mg, 0.055 mmol). LCMS m/z $[M+H]^+$ = 579.

**Step c:** To a solution of (NZ,R)-N-[1'-[7-(3-chloro-2-methyl-4-pyridyl)-6-methyl-pyrazolo[1,5-a]pyrazin-4-yl]-5-fluoro-spiro[indane-2,4'-piperidine]-1-ylidene]-2-methyl-propane-2-sulfinamide (32 mg, 0.055 mmol) in dry THF (1 ml) was added dropwise DIBAL-H (1 M in toluene, 0,265 mL, 0.265 mmol) at -65 °C under Ar. The mixture was stirred at -65 °C for 45 min and then a 1 M aqueous solution of Rochelle salts (10 mL) was added. The mixture was allowed to warm to RT, stirred for 20 min, diluted with water and extracted with ethyl acetate. The combined organic phase was washed with brine, dried with anhydrous $MgSO_4$, filtered and concentrated under reduced pressure to give (R)-N-[(1S)-1'-[7-(3-chloro-2-methyl-4-pyridyl)-6-methyl-pyrazolo[1,5-a]pyrazin-4-yl]-5-fluoro-spiro[indane-2,4'-piperidine]-1-yl]-2-methyl-propane-2-sulfinamide as a white solid (52 mg) used without further purification.

**Step d:** As described for **Example S1** - step b, a similar procedure was performed using (R)-N-[(1S)-1'-[7-(3-chloro-2-methyl-4-pyridyl)-6-methyl-pyrazolo[1,5-a]pyrazin-4-yl]-5-fluoro-spiro[indane-2,4'-piperidine]-1-yl]-2-methyl-propane-2-sulfinamide (32 mg, 0.055 mmol) and a solution of HCl (4 N in dioxane, 1 mL, 4 mmol). After completion of the reaction, the mixture was concentrated under reduced pressure and the residue was purified by preparative HPLC chromatography (column C18 Sun Fire 30x100 5 μm, Acetonitrile with 0.1 % formic acid/Water with 0.01% formic acid= 10/90 to 60/40) to give (1S)-1'-[7-(3-chloro-2-methyl-4-pyridyl)-6-methyl-pyrazolo[1,5-a]pyrazin-4-yl]-5-fluoro-spiro[indane-2,4'-piperidine]-1-amine formate (12 mg, 0.023 mmol) as a white solid. LCMS m/z $[M+H]^+$ = 477.2. $^1H$ NMR (500 MHz, DMSO-*d6*) δ ppm 1.36 (br d, *J* = 12 Hz, 1 H), 1.60 (br d, *J* = 13 Hz, 1 H), 1.78 - 1.92 (m, 2 H), 2.07 (s, 3 H), 2.65 (s, 3 H), 2.81 (d, *J* = 16 Hz, 1 H), 3.14 (d, *J* = 16 Hz, 1 H), 3.41 - 3.44 (m hidden, 2 H), 4.01 (s, 1 H), 4.41 (d, *J* = 13 Hz, 2 H), 6.99 - 7.06 (m, 2 H), 7.08 (dd, *J* = 9, 2 Hz, 1 H), 7.38 (d, *J* = 5 Hz, 1 H), 7.40 (d, *J* = 5 Hz, 1 H), 7.85 (d, J = 2 Hz, 1 H), 8.18 (s, 1 H), 8.54 (d, *J* = 5 Hz, 1 H). SHP2 $IC_{50}$ is 8 nM.

**Example S20. (6S)-6-Amino-1'-[7-(3-chloro-2-methyl-4-pyridyl)-6-methyl-pyrazolo[1,5-a]pyrazin-4-yl]spiro[4,6-dihydrocyclopenta[d]thiazole-5,4'-piperidine]-2-ol hydrochloride (Compound 218)**

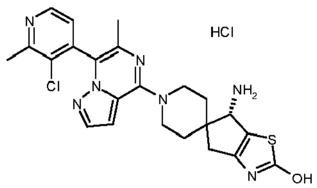

**Step a:** A mixture of **intermediate D-43** (97 mg, 0.1765 mmol), (3-chloro-2-methyl-4-pyridyl)boronic acid (45 mg, 0.265 mmol), potassium phosphate tribasic (79 mg, 0.371 mmol), and tetrahydrofuran (4 mL) and water (1 mL) was degassed under Ar for 10 min. After addition of XPhos (8.4 mg, 0.0176 mmol) and Xphos Pd G4 (15.1 mg, 0.0176 mmol), the reaction medium was degassed with Ar for 5 min and heated under microwave irradiation at 85 °C for 2.5 h. After addition of more (3-chloro-2-methyl-4-pyridyl)boronic acid (45 mg, 0.265 mmol), XPhos (8.4 mg, 0.0176 mmol) and Xphos Pd G4 (15.1 mg, 0.0176 mmol), the mixture was degassed under Ar for 5 min, heated under microwave irradiation at 85 °C for 3.5 h and left to cool to RT. Ethyl acetate and water were added to the reaction mixture, the organic layer was separated, and the aqueous phase was extracted with ethyl acetate (2 * 10 mL). The combined organic layers were washed with brine, dried over $MgSO_4$, filtered and concentrated under reduced pressure. The residue was purified by column chromatography ($SiO_2$, Heptane/Ethyl acetate= 100/0 to 0/100) to give tert-butyl N-[(6S)-1'-[7-(3-chloro-2-methyl-4-pyridyl)-6-methyl-pyrazolo[1,5-a]pyrazin-4-yl]-2-methoay-spiro[4,6-dihydrocyclopenta[d]thiazole-5,4'-piperidine]-6-yl]carbamate (52 mg, 0.087 mmol) as a yellow solid. LCMS m/z $[M+H]^+$ = 596.

**Step b:** A mixture of tert-butyl N-[(6S)-1'-[7-(3-chloro-2-methyl-4-pyridyl)-6-methyl-pyrazolo[1,5-a]pyrazin-4-yl]-2-methoxy-spiro[4,6-dihydrocyclopenta[d]thiazole-5,4'-pipendine]-6-yl]carbamate (52 mg, 0.087 mmol) and a solution of hydrochloride acid (4 M in dioxane, 5 mL, 20 mmol) was stirred 3 h at RT, and was concentrated under reduced pressure to dryness. The residue was triturated in diisopropyl ether and isolated by filtration to give (6S)-6-amino-1'-[7-(3-chloro-2-methyl-4-pyridyl)-6-methyl-pyrazolo[1,5-a]pyrazin-4-yl]spiro[4,6-dihydrocyclopenta[d]thiazole-5,4'-piperidine]-2-ol hydrochloride (30 mg, 0.062 mmol) as a light orange solid. LCMS m/z $[M+H]^+$ = 482.1. $^1H$ NMR (400 MHz, DMSO-*d6*) $\delta$ ppm 1.68 - 2.04 (m, 4 H), 2.09 (s, 3 H), 2.67 (s, 3 H), 2.72 - 2.87 (m, 2 H), 3.27 (t, *J* = 12 Hz, 1 H), 3.42 (t, *J* = 12 Hz, 1 H), 4.13 (s, 1 H), 4.46 (d, *J* = 14 Hz, 1 H), 4.54 (d, *J* = 14 Hz, 1 H), 7.06 (d, *J* = 2 Hz, 1 H), 7.43 (d, *J* = 5 Hz, 1 H), 7.89 (d, *J* = 3 Hz, 1 H), 8.32 (br s, 3 H), 8.57 (d, *J* = 5 Hz, 1 H), 11.70 (s, 1 H). SHP2 $IC_{50}$ is 179 nM.

**Example S21. (6S)-1'-[7-(3-Chloro-2-methyl-4-pyiidyl)-6-methyl-pyrazolo[1,5-a]pyrazin-4-yl]-2-methoxy-spiro[4,6-dihydrocyclopenta[d]thiazole-5,4'-piperidine]-6-amine (Compound 219)**

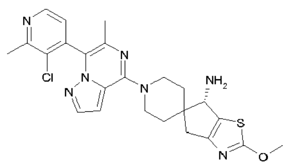

To solution in dichloromethane (3 mL) tert-butyl N-[(6S)-1'-[7-(3-chloro-2-methyl-4-pyridyl)-6-methyl-pyrazolo[1,5-a]pyrazin-4-yl]-2-methoxy-spiro[4,6-dihydrocyclopenta[d]thiazole-5,4'-piperidine]-6-yl]carbamate (70 mg, 0.117 mmol) prepared as depicted in **Example S20** (Compound **218)** was added trifluoroacetic acid (0.6 mL, 8.0 mmol). The mixture was stirred 2 h at RT and was then concentrated under reduced pressure to dryness. To the residue cooled to 4 °C was added a saturated solution of sodium hydrogen carbonate (20 mL). The resulting mixture was extracted by dichloromethane. The combined organic layers were washed with brine, dried over $MgSO_4$, filtered and concentrated under reduced pressure to give (6S)-1'-[7-(3-chloro-2-methyl-4-pyridyl)-6-methyl-pyrazolo[1,5-a]pyrazin-4-yl]-2-methoxy-spiro[4,6-dihydrocyclopenta[d]thiazole-5,4'-piperidine]-6-amine (28 mg, 0.056 mmol) as an off-white solid. LCMS m/z $[M+H]^+$ = 495.9. $^1H$ NMR (400 MHz, DMSO-*d6*) $\delta$ ppm 1.58 - 1.84 (m, 3 H), 1.92 (m, 1 H), 2.07 (s, 3 H), 2.65 (s, 3 H), 2.68 - 2.81 (m, 2 H), 3.39 - 3.60 (m, 2 H), 3.96 (br s, 1 H), 4.01 (s, 3 H), 4.20 - 4.46 (m, 2 H), 7.02 *(d, J* = 3 Hz, 1 H), 7.40 *(d, J* = 5 Hz, 1 H), 7.85 *(d, J* = 3 Hz, 1 H), 8.54 (d, *J* = 5 Hz, 1 H). SHP2 $IC_{50}$ is 5 nM.

**Example S22. (6S)-1'-[7-(1,5-Dimethylpyrazol-4-yl)-6-methyl-pyrazolo[1,5-a]pyrazin-4-yl]-2-methoxy-spiro[4,6-dihydrocyclopenta[d]thiazole-5,4'-piperidine]-6-amine (Compound 220)**

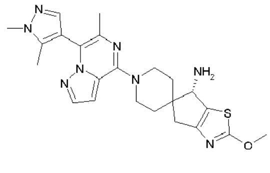

**Step a:** A mixture of **intermediate D-43** (85 mg, 0.155 mmol), 1,5-dimethyl-4-(4,4,5,5-tetramethyl-1,3,2-dioxaborolan-2-yl)-1H-pyrazole (53 mg, 0.232 mmol), potassium phosphate tribasic (69 mg, 0.325 mmol), and tetrahydrofuran (4 mL) and water (1 mL) was degassed under Ar for 10 min. After addition of XPhos (7.4 mg, 0.016 mmol) and Xphos Pd G4 (13.3 mg, 0.016 mmol), the reaction medium was degassed with Ar for 5 min and heated under microwave irradiation at 85 °C for 3.5 h and left to cool to RT. Ethyl acetate and water were added to the reaction mixture, the organic layer was separated, and the aqueous phase was extracted with ethyl acetate (2 * 10 mL). The combined organic layers were washed with brine, dried over $MgSO_4$, filtered and concentrated under reduced pressure. The residue was purified by column chromatography ($SiO_2$, Heptane/Ethyl acetate= 100/0 to 20/80) to give tert-butyl N-[(6S)-1'-[7-(1,5-dimethylpyrazol-4-yl)-6-methyl-pyrazolo[1,5-a]pyrazin-4-yl]-2-methoxy-spiro[4,6-dihydrocyclopenta[d]thiazole-5,4'-piperidine]-6-yl]carbamate (81 mg, 0.143 mmol) as a yellow solid. LCMS m/z $[M+H]^+$ = 565.

**Step b:** To a solution of tert-butyl N-[(6S)-1'-[7-(1,5-dimethylpyrazol-4-yl)-6-methyl-pyrazolo[1,5-a]pyrazin-4-yl]-2-methoxy-spiro[4,6-dihydrocyclopenta[d]thiazole-5,4'-pipendine]-6-yl]carbamate (81 mg, 0.143 mmol) in dichloromethane (5 mL) was added trifluoroacetic acid (0.6 mL, 8.0 mmol). The mixture was stirred 1 h at RT and was then concentrated under reduced pressure to dryness. To the residue cooled to 4 °C was added a saturated solution of sodium hydrogen carbonate (20 mL). The resulting mixture was extracted by dichloromethane. The combined organic layers were washed with brine, dried over $MgSO_4$, filtered and concentrated under reduced pressure to give (6S)-1'-[7-(1,5-dimethylpyrazol-4-yl)-6-methyl-pyrazolo[1,5-a]pyrazin-4-yl]-2-methoxy-spiro[4,6-dihydrocyclopenta[d]thiazole-5,4'-piperidine]-6-amine (47 mg, 0.10 mmol) as pale pink solid. LCMS m/z $[M+H]^+$ = 465.2. $^1H$ NMR (500 MHz, DMSO-*d6*) $\delta$ ppm 1.60 - 1.67 (m, 2 H), 1.72 - 1.80 (m, 1 H), 1.87 - 1.97 (m, 1 H), 2.06 (s, 3 H), 2.18 (s, 3 H), 2.60 - 2.74 (m, 2 H), 3.30 - 3.48 (m, 2 H, partially masked by water), 3.82 (s, 3 H), 3.90 (br s, 1 H), 4.00 (s, 3H), 4.14 - 4.27 (m, 2 H), 6.93 (d, *J* = 2 Hz, 1 H), 7.45 (s, 1 H), 7.84 (d, *J* = 2 Hz, 1 H). SHP2 $IC_{50}$ is 26 nM.

**Example S23. (5S)-1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-5-amine) (Compound 221) and ((5S)-5-amino-1'-[7-(2,3-dif)uorophenyt)-6-methyt-pyrazoto[1,5-a]pyrazin-4-yl]spiro[5,7-dihydrocyctopenta[b]pyridine-6,4'-piperidine]-2-ol hydrochloride (Compound 222)**

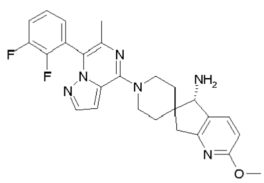

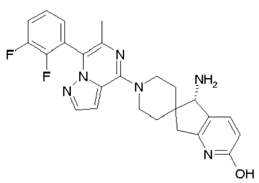

**Step a:** As described for **Example S1** - step a, a similar procedure was performed using **intermediate D-13** (109 mg, 0.20 mmol) and (2,3-difluorophenyl)boronic acid (38 mg, 0.24 mmol) to give (R)-N-[(5S)-1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-5-yl]-2-methyl-propane-2-sulfinamide (89 mg, 0.15 mmol).

**Step b:** To a solution of (R)-N-[(5S)-1'-[7-(2,3-difluorophenyl)-6-methyl-pyrazolo[1,5-a]pyrazin-4-yl]-2-methoxy-spiro[5,7-dihydrocyclopenta[b]pyndine-6,4'-pipendine]-5-yl]-2-methyl-propane-2-sulfinamide (84 mg, 0.14 mmol) in dichloromethane (0.7 mL) and methanol (0.7 mL) was added a solution of HCl (4 N in dioxane, 0.18 mL, 0.72 mmol) at 0 °C. The mixture was stirred at RT for 2 h and then concentrated under reduced pressure. Water (5 mL) was added and the aqueous layer was washed with diethyl ether (5 mL * 2). The pH value of the aqueous phase was adjusted to 11-12 with a 1 N aqueous solution of NaOH. The aqueous phase was extracted with ethyl acetate (10 mL * 2). The combined organic phase was washed with brine (10 mL), dried with anhydrous $Na_2SO_4$, filtered and concentrated under vacuum. The residue was purified by column chromatography ($SiO_2$, dichloromethane/methanol = 95/5). The white solid obtained was taken up in dichloromethane (1 mL) and a solution of HCl (2 N in diethyl ether, 0.22 mL, 0.44 mmol) was added and the mixture was stirred for 10 min at RT and then filtered. The solid was purified by preparative HPLC chromatography (0.1% HCl in $H_2O$ / $CH_3CN$ with gradient elution 84:16 to 0:100 XSELECT CSH Prep C18 5$\mu$m OBD 50 * 250mm) to give (5S)-5-amino-1'-[7-(2,3-difluorophenyl)-6-methyl-pyrazolo[1,5-a]pyrazin-4-yl]spiro[5,7-dihydrocyclopenta[b]pyridine-6,4'-piperidine]-2-ol hydrochloride (15 mg, 0.03 mmol) as a white solid and (5S)-1'-[7-(2,3-difluorophenyl)-6-methyl-pyrazolo[1,5-a]pyrazin-4-yl]-2-methoxy-spiro[5,7-dihydrocyclopenta[b]pyridine-6,4'-piperidine]-5-amine (15 mg, 0.03 mmol) as a white solid, obtained after being desalified by treatment with saturated solution of sodium bicarbonate.

**Compound 221:** LCMS m/z $[M+H]^+$ = 477.2. $^1H$ NMR (500 MHz, DMSO-*d6*) $\delta$ ppm 1.38 (br d, *J* = 13 Hz, 1 H), 1.60 (br d, *J* = 13 Hz, 1 H), 1.78 - 1.90 (m, 2 H), 2.16 (s, 3 H), 2.77 (d, *J* = 16 Hz, 1 H), 3.05 (d, J = 17 Hz, 1 H), 3.38 - 3.49 (m, 2 H), 3.84 (s, 3 H), 3.88 (s, 1 H), 4.34 - 4.45 (m, 2 H), 6.62 (d, *J* = 8 Hz, 1 H), 7.01 (dd, *J* = 2, 1 Hz, 1 H), 7.35

(td, $J$ = 6, 3 Hz, 2 H), 7.54 - 7.60 (m, 1 H), 7.62 (d, $J$ = 8 Hz, 1 H), 7.87 (d, $J$ = 2 Hz, 1 H). SHP2 $IC_{50}$ is 21 nM.

**Compound 222:** LCMS m/z $[M+H]^+$ = 463.2. $^1H$ NMR (400 MHz, DMSO-*d6*) $\delta$ ppm 1.65 (br d, $J$ = 13 Hz, 1 H), 1.73 - 1.90 (m, 2 H), 1.99 (m, 1 H), 2.19 (s, 3 H), 2.89 (d, $J$ = 18 Hz, 1 H), 3.04 (d, $J$ = 18 Hz, 1 H), 3.23 - 3.42 (m, 2 H), 4.16 (q, $J$ = 5 Hz, 1 H), 4.30 - 4.59 (m, 2 H), 6.26 (d, $J$ = 9 Hz, 1 H), 7.10 (t, $J$ = 2 Hz, 1 H), 7.26 - 7.45 (m, 2 H), 7.59 (m, 1 H), 7.65 (d, $J$ = 13 Hz, 1 H), 7.93 (d, $J$ = 2 Hz, 1 H), 8.38 (br d, $J$ = 1 Hz, 3 H). SHP2 $IC_{50}$ is 1 nM.

**Example S24. [(5S)-5-amino-1'-(6-methyl-7-phenyl-pyrazolo[1,5-a]pyrazin-4-yl)spiro[5,7-dihydrocyclopenta[b]pyridine-6,4'-piperidine]-2-yl]methanol (Compound 223)**

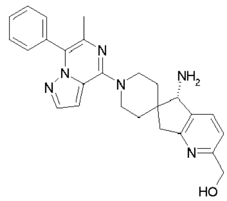

**Step a:** As described for **intermediate D-13,** a similar procedure was performed using **intermediate B-33** (159 mg, 0.41 mmol) and **intermediate A-2** (135 mg, 0.44 mmol) to give 1'-(7-bromo-6-methyl-pyrazolo[1,5-a]pyrazin-4-yl)-2-(triisopropylsilyloxymethyl)spiro[7H-cyclopenta[b]pyridine-6,4'-piperidine]-5-one (118 mg, 0.20 mmol) as a white foam. LCMS m/z $[M+H]^+$ = 598.4.

**Step b:** As described for **intermediate D-1 -** step b, a similar procedure was performed using 1'-(7-bromo-6-methyl-pyrazolo[1,5-a]pyrazin-4-yl)-2-(trisopropylsilyloxymethyl)spiro[7H-cyclopenta[b]pyridine-6,4'-pipendine]-5-one (115 mg, 0.19 mmol), (R)-2-methylpropane-2-sulfinamide (47 mg, 0.38 mmol) and $Ti(OEt)_4$ (0.26 mL) to give rac-(NZ,R)-N-[1'-(7-bromo-6-methyl-pyrazolo[1,5-a]pyrazin-4-yl)-2-(triisopropylsilyloxymethyl)spiro[7H-cyclopenta[b]pyridine-6,4'-piperidine]-5-ylidene]-2-methyl-propane-2-sulfinamide (91 mg, 0.13 mmol) as a yellow wax. LCMS m/z $[M+H]^+$ = 701.5.

**Step c:** As described for **intermediate D-1** - step c, a similar procedure was performed rac-(NZ,R)-N-[1'-(7-bromo-6-methyl-pyrazolo[1,5-a]pyrazin-4-yl)-2-(triisopropylsilyloxymethyl)spiro[7H-cyclopenta[b]pyridine-6,4'-piperidine]-5-ylidene]-2-methyl-propane-2-sulfinamide (87 mg, 0.12 mmol) and DIBAL-H (1 M in toluene, 0.3 mL, 0.3 mmol) to give (R)-N-[(5S)-1'-(7-bromo-6-methyl-pyrazolo[1,5-a]pyrazin-4-yl)-2-(triisopropylsilyloxymethyl)spiro[5,7-dihydrocyclopenta[b]pyridine-6,4'-piperidine]-5-yl]-2-methyl-propane-2-sulfinamide (87 mg, crude) as a yellow wax that was used without further purification.

**Step d:** As described for **Example S4** (Compound **31),** a similar procedure was performed using (R)-N-[(5S)-1'-(7-bromo-6-methyl-pyrazolo[1,5-a]pyrazin-4-yl)-2-(triisopropylsilyloxymethyl)spiro[5,7-dihydrocyclopenta[b]pyridine-6,4'-piperidine]-5-yl]-2-methyl-propane-2-sulfinamide (87 mg) and phenylboronic acid (23 mg, 0.19 mmol) to give (R)-2-methyl-N-[(5S)-1'-(6-methyl-7-phenyl-pyrazolo[1,5-a]pyrazin-4-yl)-2-(triisopropylsilyloxymethyl)spiro[5,7-dihydrocyclopenta[b]pyridine-6,4'-piperidine]-5-yl]propane-2-sulfinamide (61 mg, 0.09 mmol) as a yellow wax. LCMS m/z $[M+H]^+$ = 701.6.

**Step e:** To a mixture of (R)-2-methyl-N-[(5S)-1'-(6-methyl-7-phenyl-pyrazolo[1,5-a]pyrazin-4-yl)-2-(triisopropylsilyloxymethyl)spiro[5,7-dihydrocyclopenta[b]pyridine-6,4'-piperidine]-5-yl]propane-2-sulfinamide (59 mg, 0.08 mmol) in dichloromethane (1 mL) and methanol (0.5 mL) was added a solution of HCl (4 M in dioxane, 0.15 mL, 0.6 mmol) at 0 °C. The mixture was stirred for 18 h, a solution of HCl (4 M in dioxane, 0.1 mL, 0.4 mmol) was added and the mixture was stirred for 24 h and then concentrated under vacuo. The residue was taken up in water and diethyl ether, a few drops of 1 N aqueous solution of HCl was added. The aqueous layer was separated and the pH value of the aqueous phase was adjusted to 11-12 with 1 N aqueous NaOH solution. The solid was collected by filtration and washed with water to give [(5S)-5-amino-1'-(6-methyl-7-phenyl-pyrazolo[1,5-a]pyrazin-4-yl)spiro[5,7-dihydrocyclopenta[b]pyridine-6,4'-piperidine]-2-yl]methanol (10 mg, 0.03 mmol) as an off-white solid. LCMS m/z $[M+H]^+$ = 441.4. $^1H$ NMR (400 MHz, DMSO-*d6*) $\delta$ ppm 1.24 (br d, $J$ = 13 Hz, 1 H), 1.61 (br d, $J$ = 12 Hz, 1 H), 1.77 - 1.97 (m, 4 H), 2.17 (s, 3 H), 2.75 (br d, $J$ = 16 Hz, 1 H), 3.11 (d, $J$ = 16 Hz, 1 H), 3.32 - 3.44 (m, 2 H), 3.91 (s, 1 H), 4.27 - 4.40 (m, 2 H), 4.53 (br d, $J$ = 5 Hz, 2 H), 5.29 (t, $J$ = 6 Hz, 1 H), 6.96 (d, $J$ = 3 Hz, 1 H), 7.26 (d, $J$ = 8 Hz, 1 H), 7.40 - 7.55 (m, 5 H), 7.66 (d, $J$ = 8 Hz, 1 H), 7.84 (d, $J$ = 3 Hz, 1 H). SHP2 $IC_{50}$ is 13 nM.

**Example S25. (6S)-1'-[7-(2-fluorophenyl)-6-methyl-pyrazolo[1,5-a]pyrazin-4-yl]-2-methyl-spiro[4,6-dihydrocyclopenta[d]oxazole-5,4'-piperidine]-6-amine (Compound 224)**

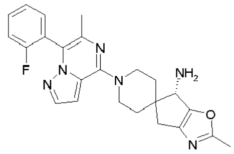

Preparation of 5-bromo-4-(bromomethyl)-2-methyl-oxazole: To a mixture of ethyl 5-bromo-2-methyloxazole-4-carboxylate (2.49 g, 10.6 mmol) in anhydrous dichloromethane (22 mL), cooled at -78°C, was added dropwise DIBAL-H (1 M in toluene, 20 mL, 20 mmol). The reaction mixture was stirred at -78°C for 1 h, and then poured into a saturated aqueous solution of Rochelle salt (75 mL) and ethyl acetate (50 mL). The mixture was vigorously stirred at RT for 2 h The organic layer was separated and the aqueous phase extracted with ethyl acetate (50 mL). The combined organic phase was washed with brine, dried with anhydrous $Na_2SO_4$, filtered and concentrated under vacuum. The residue was re-engaged following the same protocol to finally give (5-bromo-2-methyl-oxazol-4-yl)methanol (1.25 g, crude) as a yellow semi-solid that was used without any further purification.

To a solution of (5-bromo-2-methyl-oxazol-4-yl)methanol previously obtained (1.25 g, crude) and $CBr_4$ (2.6 g, 7.8 mmol) in DCM (22 mL) was added portionwise $PPh_3$ (2.05 g, 7.8 mmol) at 0 °C under Ar. The reaction was stirred at RT for 1 h and then concentrated under vacuum. The residue was purified by column chromatography ($SiO_2$, cyclohexane/ethyl acetate = 1/0 to 4/1) to give 5-bromo-4-(bromomethyl)-2-methyl-oxazole (1.06 g, 4.17 mmol) as a yellow oil. $^1H$ NMR (400 MHz, DMSO-$d_6$) $\delta$ ppm 4.44 (s, 2H), 2.42 (s, 3H).

Synthesis of Compound **224.** The title compound was prepared as outlined in the following scheme:

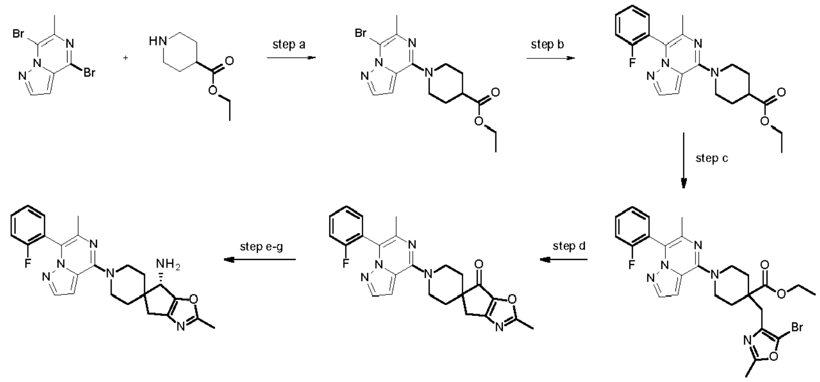


**Step a:** A mixture of ethyl piperidine-4-carboxylate (0.54 mL, 3.45 mmol), **intermediate A-2** (1.0 g, 3.26 mmol), and potassium carbonate (1.36 g, 9.8 mmol) in dimethylformamide (16 mL) was stirred at 85 °C for 18 h. The reaction mixture was then cooled down to RT and poured into water (100 mL) and ethyl acetate (50 mL). The aqueous layer was separated and then extracted with ethyl acetate (50 mL). The combined organic layer was washed with brine (50 mL), dried over anhydrous $Na_2SO_4$ and concentrated under reduced pressure to give ethyl 1-(7-bromo-6-methyl-pyrazolo[1,5-a]pyrazin-4-yl)piperidine-4-carboxylate (1.4 g, crude) as a pink oil.

**Step b:** As described for Example S4 (Compound **31)** - step a, a similar procedure was performed using ethyl 1-(7-bromo-6-methyl-pyrazolo[1,5-a]pyrazin-4-yl)piperidine-4-carboxylate (1.2 g, crude) and 2-fluorophenylboronic acid (0.7 g, 4.9 mmol) to give ethyl 1-[7-(2-fluorophenyl)-6-methyl-pyrazolo[1,5-a]pyrazin-4-yl]piperidine-4-carboxylate (1.24 g, 3.24 mmol) as a yellow solid. LCMS m/z $[M+H]^+$ = 383.3.

**Step c:** To a solution of ethyl 1-[7-(2-fluorophenyl)-6-methyl-pyrazolo[1,5-a]pyrazin-4-yl]piperidine-4-carboxylate (1.12 g, 2.93 mmol) in THF (3 mL) was added LDA (2 M in THF, 1.5 mL, 3.0 mmol) at -78 °C and the mixture was stirred at this temperature for 1 h. 5-Bromo-4-(bromomethyl)-2-methyl-oxazole (0.65 g, 2.5 mmol) in THF (2 mL) was then added at -78 °C and the mixture stirred at this temperature for 3.5 h The reaction mixture was poured into water (15 mL) and extracted with ethyl acetate (15 mL * 2), the organic layer was dried over $Na_2SO_4$, filtered and concentrated under reduced pressure. The residue was purified by column chromatography ($SiO_2$, cyclohexane/ethyl acetate = 1/0 to 7/3) to give ethyl 4-[(5-bromo-2-methyl-oxazol-4-yl)methyl]-1-[7-(2-fluorophenyl)-6-methyl-pyrazolo[1,5-a]pyrazin-4-yl]piperidine-4-carboxylate (1.13 g, 2.03 mmol) as an off-white foam. LCMS m/z [M+H]+ = 556.3.

**Step d:** To a solution of ethyl 4-[(5-bromo-2-methyl-oxazol-4-yl)methyl]-1-[7-(2-fluorophenyl)-6-methyl-pyrazolo[1,5-a]pyrazin-4-yl]piperidine-4-carboxylate (1.13 g, 2.03 mmol) inTHF (8.4 mL) was added dropwise Turbo Grignard reagent iPrMgCl.LiCl complex (1.3 M in THF, 2.4 mL, 3.1 mmol) at -78 °C, and was then allowed to warm slowy to 0 °C. The mixture was stirred at 0 °C for 2 h and then was poured into an aqueous solution of $NH_4Cl$ (40 mL), and extracted with EtOAc (50 mL * 3). The combined organic layers were washed with brine (50 mL), dried over $Na_2SO_4$, filtered and concentrated under reduced pressure. The residue was first purified by column chromatography ($SiO_2$, cyclohexane/ethyl acetate = 1/0 to 1/1) and then subjected to SFC purification to give 1'-[7-(2-fluorophenyl)-6-methyl-pyrazolo[1,5-a]pyrazin-4-yl]-2-methyl-spiro[4H-cyclopenta[d]oxazole-5,4'-piperidine]-6-one (127 mg, 0.29 mmol) as an off-white solid. LCMS m/z $[M+H]^+$ = 432.3.

**Step e:** To a mixture of 1'-[7-(2-fluorophenyl)-6-methyl-pyrazolo[1,5-a]pyrazin-4-yl]-2-methyl-spiro[4H-cyclopenta[d]oxazole-5,4'-piperidine]-6-one (126 mg, 0.29 mmol) in 2-MeTHF (0.3 mL) was added (R)-2-methylpropane-2-sulfinamide (145 mg, 1.17 mmol) and $Ti(OEt)_4$ (0.65 mL, 2.95 mmol) and the mixture was then stirred at 110 °C for 22 h. Water and dichloromethane were added and the mixture was filtered on a hydrophobic cartridge (liquid / liquid extraction column, Radleys®) and then concentrated in vacuum. The residue was subjected to SFC purification followed by column chromatography ($SiO_2$, dichloromethane / methanol = 1/0 to 9/1) to give (NZ,R)-N-[1'-[7-(2-fluorophenyl)-6-methyl-pyrazolo[1,5-a]pyrazin-4-yl]-2-methyl-spiro[4H-cyclopenta[d]oxazole-5,4'-piperidine]-6-ylidene]-2-methyl-propane-2-sulfinamide (25 mg, 47 μmol) as a yellow wax. LCMS m/z $[M+H]^+$ = 535.3.

**Step f:** To a solution of (NZ,R)-N-[1'-[7-(2-fluorophenyl)-6-methyl-pyrazolo[1,5-a]pyrazin-4-yl]-2-methyl-spiro[4H-cyclopenta[d]oxazole-5,4'-piperidine]-6-ylidene]-2-methyl-propane-2-sulfinamide (25 mg, 47 μmol) in THF (0.5 ml) was added dropwise DIBAL-H (1 M in toluene, 0.11 mL, 0.11 mmol) at -78 °C under Ar. The mixture was stirred at -78 °C for 2 h and then a saturated solution of Rochelle salts (6 mL) followed by ethyl acetate (5 mL) were added. The mixture was allowed to warm to RT, stirred for 1.5 h. The aqueous layer was separated and extracted with ethyl acetate (5 mL * 2). The combined organic phase was washed with brine, dried with anhydrous $Na_2SO_4$, filtered and concentrated under reduced pressure. The residue was purified by column chromatography ($SiO_2$, dichloromethane / methanol = 1/0 to 93/7) to give (R)-N-[(6S)-1'-[7-(2-fluorophenyl)-6-methyl-pyrazolo[1,5-a]pyrazin-4-yl]-2-methyl-spiro[4,6-dihydrocyclopenta[d]oxazole-5,4'-piperidine]-6-yl]-2-methyl-propane-2-sulfinamide (5 mg, 9.3 μmol) as a yellow wax. LCMS m/z $[M+H]^+$ = 537.3.

**Step g:** As described for Example S4 (Compound **31)** - step b, a similar procedure was performed using (R)-N-[(6S)-1'-[7-(2-fluorophenyl)-6-methyl-pyrazolo[1,5-a]pyrazin-4-yl]-2-methyl-spiro[4,6-dihydrocyclopenta[d]oxazole-5,4'-piperidine]-6-yl]-2-methyl-propane-2-sulfinamide (5 mg, 9.3 μmol) and a HCl solution (4 M in dioxane, 15 μL, 60 μmol) to give (6S)-1'-[7-(2-fluorophenyl)-6-methyl-pyrazolo[1,5-a]pyrazin-4-yl]-2-methyl-spiro[4,6-dihydrocyclopenta[d]oxazole-5,4'-piperidine]-6-amine (2 mg, 4.6 μmol) as an off-white solid. LCMS m/z $[M+H]^+$ = 433.2. $^1H$ NMR (400 MHz, DMSO-*d6*) δ ppm 1.20 - 1.29 (m, 1 H), 1.64 - 1.82 (m, 3 H), 1.90 - 2.00 (m, 2 H), 2.15 (s, 3 H), 2.42 (s, 3 H), 3.40 - 3.50 (m, 1 H), 3.51 - 3.60 (m, 1H), 3.79 (s, 1 H), 4.18 - 4.34 (m, 2 H), 7.00 (t, d=2 Hz, 1 H), 7.32 - 7.40 (m, 2 H), 7.47 - 7.60 (m, 2 H), 7.85 (d, J=2 Hz, 1 H). SHP2 $IC_{50}$: 27 nM.

**Example S26. (5S)-1'-[7-(2-fluorophenyl)-3-(methoxymethyl)-6-methyl-pyrazolo[1,5-a]pyrazin-4-yl]spiro[5,7-dihydrocyclopenta[b]pyridine-6,4'-piperidine]-5-amine (Compound 225) and [4-[(5S)-5-aminospiro[5,7-dihydrocyclopenta[b]pyridine-6,4'-piperidine]-1'-yl]-7-(2-fluorophenyl)-6-methyl-pyrazolo[1,5-a]pyrazin-3-yl]methanol (Compound 226)**

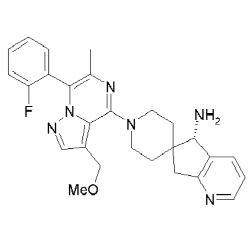

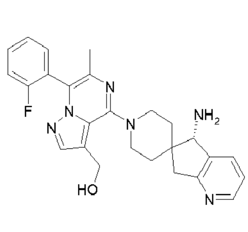

**Step a:** As described for Example S4 (Compound **31)** - step a, a similar procedure was performed using **intermediate D-48** (130 mg, 0.24 mmol) and 2-fluorophenylboronic acid (50 mg, 0.36 mmol) to give (R)-N-[(5S)-1'-[7-(2-fluorophenyl)-3-(hydroxymethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (115 mg, 0.20 mmol) as a yellow solid. LCMS m/z $[M+H]^+$ = 563.4.

**Step b:** To a solution of mixture of (R)-N-[(5S)-1'-[7-(2-fluorophenyl)-3-(hydroxymethyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (110 mg, 0.19 mmol) in methanol (2 mL) was added a solution of HCl (4 N in dioxane, 1 mL, 4 mmol), the mixture was then stirred at RT for 1.5 h. The mixture was concentrated under reduced pressure and the residue was taken up in water and

dichloromethane. The pH value of the aqueous phase was adjusted to 11-12 with a 1 N aqueous solution of NaOH. The mixture was filtered on a hydrophobic cartridge (liquid / liquid extraction column, Radleys®) and the organic layer concentrated in vacuum. The residue was purified by column chromatography ($SiO_2$, dichloromethane/methanol containing 10% of conc. $NH_3$ (aq) = 95/5) to give (5S)-1'-[7-(2-fluorophenyl)-3-(methoxymethyl)-6-methyl-pyrazolo[1,5-a]pyrazin-4-yl]spiro[5,7-dihydrocyclopenta[b]pyridine-6,4'-piperidine]-5-amine (36 mg, 0.07 mmol), as a white solid, and [4-[(5S)-5-aminospiro[5,7-dihydrocyclopenta[b]pyridine-6,4'-piperidine]-1'-yl]-7-(2-fluorophenyl)-6-methyl-pyrazolo[1,5-a]pyrazin-3-yl]methanol (38 mg, 0.08 mmol) as a white solid.

**((5S)-1'-[7-(2-fluorophenyl)-3-(methoxymethyl)-6-methyl-pyrazolo[1,5-a]pyrazin-4-yl]spiro[5,7-dihydrocyclopenta[b]pyridine-6,4'-piperidine]-5-amine) (Compound 225):** LCMS m/z $[M+H]^+$ = 473.3. $^1H$ NMR (400 MHz, DMSO-*d6*) δ ppm 1.27 (br d, *J* = 13 Hz, 1 H), 1.65 (br d, *J* = 13 Hz, 1 H), 1.87 - 2.14 (m, 2 H), 2.19 (s, 3 H), 2.77 (d, *J* = 16 Hz, 1 H), 3.04 - 3.35 (m, 5 H), 3.37 (s, 3 H), 3.68 - 3.85 (m, 2 H), 3.98 (s, 1 H), 4.59 - 4.71 (m, 2 H), 7.18 (dd, *J* = 8, 5 Hz, 1 H), 7.31 - 7.43 (m, 2 H), 7.48 - 7.64 (m, 2 H), 7.69 (d, *J* = 8 Hz, 1 H), 7.92 (s, 1 H), 8.33 (dd, *J* = 5, 1 Hz, 1 H). SHP2 $IC_{50}$ is > 1000 nM.

**([4-[(5S)-5-aminospiro[5,7-dihydrocyclopenta[b]pyridine-6,4'-piperidine]-1'-yl]-7-(2-fluorophenyl)-6-methyl-pyrazolo[1,5-a]pyrazin-3-yl]methanol) (Compound 226):** LCMS m/z $[M+H]^+$ = 459.3. $^1H$ NMR (400 MHz, DMSO-*d6*) δ ppm 1.27 (br d, *J* = 13 Hz, 1 H), 1.64 (br d, *J* = 13 Hz, 1 H), 1.89 - 2.09 (m, 4 H), 2.17 (s, 3 H), 2.76 (d, *J* = 16 Hz, 1 H), 3.04 - 3.22 (m, 4 H), 3.76 (m, 1 H), 3.97 (s, 1 H), 4.78 (d, *J* = 5 Hz, 2 H), 5.14 (t, *J* = 5 Hz, 1 H), 7.18 (dd, *J* = 8, 5 Hz, 1 H), 7.33 - 7.42 (m, 2 H), 7.49 - 7.62 (m, 2 H), 7.69 (dt, *J* = 8, 1 Hz, 1 H), 7.87 (s, 1 H), 8.32 (d, *J* = 5 Hz, 1 H). SHP2 $IC_{50}$ is > 1000 nM.

**Example S27. 3-[4-[(5S)-5-aminospiro[5,7-dihydrocyclopenta[b]pyridine-6,4'-piperidine]-1'-yl]-6-methyl-pyrazolo[1,5-a]pyrazin-7-yl]-1-methyl-pyridin-2-one (Compound 227)**

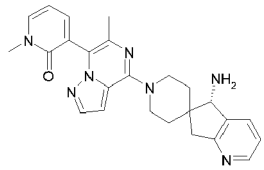

**Step a:** A mixture of 3-bromo-1-methyl-pyridin-2-one (500 mg, 2.52 mmol), hexabutylditin (1.8 mL, 3.54 mmol), tetrakis(triphenylphosphine)palladium (146 mg, 0.13 mmol) in dioxane (12 mL) was degassed with Ar for 5 min and then heated under microwave irradiation at 150 °C for 30 min. The reaction mixture was concentrated under vacuum. The residue was purified by column chromatography ($SiO_2$, cyclohexane/ethyl acetate = 95/5 to 1/1) to give 1-methyl-3-tributylstannyl-pyridin-2-one (541 mg, 1.36 mmol) as a colourless oil.

**Step b:** A mixture of 1-methyl-3-tributylstannyl-pyridin-2-one (541 mg, 1.36 mmol), (R)-N-[(5S)-1'-(7-bromo-6-methyl-pyrazolo[1,5-a]pyrazin-4-yl)spiro[5,7-dihydrocyclopenta[b]pyridine-6,4'-piperidine]-5-yl]-2-methyl-propane-2-sulfinamide (740 mg, 1.36 mmol), tetrakis(triphenylphosphine)palladium (78 mg, 0.08 mmol) in dioxane (7 mL) was degassed with Ar for 5 min and then heated at 110 °C for 16 h. The reaction mixture was allowed to cool down to RT, ethyl acetate was added and the mixture filtered through a GF/F filter paper. The filtrate was washed with water and then brine. The aqueous layer was extracted with a mixture DCM/iPrOH 9/1. The combined organic layers were dried over $Na_2SO_4$, filtered and concentrated under reduced pressure. The residue was purified by column chromatography ($SiO_2$, dichloromethane/MeOH containing 1% conc. $NH_3$ (aq) = 1/0 to 92/8). The solid obtained was triturated in a dichloromethane/pentane mixture and then filtered to give (R)-2-methyl-N-[(5S)-1'-[6-methyl-7-(1-methyl-2-oxo-3-pyridyl)pyrazolo[1,5-a]pyrazin-4-yl]spiro[5,7-dihydrocyclopenta[b]pyridine-6,4'-piperidine]-5-yl]propane-2-sulfinamide (186 mg, 0.34 mmol) as a yellow solid. LCMS m/z $[M+H]^+$ = 546.3.

**Step c:** As described for **Example S1** - step b, a similar procedure was performed using (R)-2-methyl-N-[(5S)-1'-[6-methyl-7-(1-methyl-2-oxo-3-pyridyl)pyrazolo[1,5-a]pyrazin-4-yl]spiro[5,7-dihydrocyclopenta[b]pyridine-6,4'-piperidine]-5-yl]propane-2-sulfinamide (186 mg, 0.34 mmol) and a solution of HCl (4 N in dioxane, 0.5 mL, 2 mmol) to give 3-[4-[(5S)-5-aminospiro[5,7-dihydrocyclopenta[b]pyridine-6,4'-piperidine]-1'-yl]-6-methyl-pyrazolo[1,5-a]pyrazin-7-yl]-1-methyl-pyridin-2-one (95 mg, 0.21 mmol) as a yellow wax. LCMS m/z $[M+H]^+$ = 442.2. $^1H$ NMR (400 MHz, DMSO-*d6*) δppm 1.21 *(br d, J*= 13 Hz, 1 H), 1.60 *(br d, J*= 13 Hz, 1 H), 1.73 - 1.99 (m, 4H), 2.13 (s, 3 H), 2.78 (br d, *J* = 16 Hz, 1 H), 3.14 (br d, *J* = 16 Hz, 1 H), 3.36 - 3.43 (m hidden, 2 H), 3.50 (s, 3 H), 3.95 (s, 1 H), 4.28 - 4.41 (m, 2 H), 6.34 (t, *J* = 7 Hz, 1 H), 6.91 (d, *J* = 2 Hz, 1 H), 7.18 (t, *J* = 6 Hz, 1 H), 7.55 (dd, *J* = 7, 2 Hz, 1 H), 7.67 (d, *J*= 7 Hz, 1 H), 7.79 (d, *J*= 2 Hz, 1 H), 7.84 (dd, *J*= 7, 2 Hz, 1 H), 8.33 (d, *J*= 5 Hz, 1 H). SHP2 $IC_{50}$ is > 1000 nM.

**Example S28. 4-[4-[(5S)-5-aminospiro[5,7-dihydrocyclopenta[b]pyridine-6,4'-piperidine]-1'-yl]-6-methyl-pyrazolo[1,5-a]pyrazin-7-yl]-3-fluoro-1-methyl-pyridin-2-one (Compound 228)**

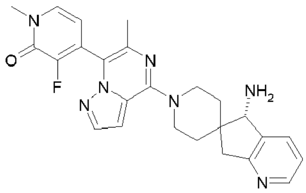

**Step a:** A mixture of 4-bromo-3-fluoro-1H-pyridin-2-one (352 mg, 1.83 mmol), cesium carbonate (740 mg, 2.75 mmol) and iodomethane (0.17 mL, 2.75 mmol) in dioxane (9.2 mL) was stirred at 80 °C for 16 h. The mixture was allowed to cool down to RT and was then filtered. Water (20 mL) was added and the aqueous layer was extracted with ethyl acetate (20 mL * 2). The combined organic layers were dried over $Na_2SO_4$, filtered and concentrated under reduced pressure to give 4-bromo-3-fluoro-1-methyl-pyridin-2-one (393 mg, crude) that was used without further purification.

**Step b:** A mixture of 4-bromo-3-fluoro-1-methyl-pyridin-2-one (393 mg, crude), hexabutylditin (1.3 mL, 2.6 mmol), tetrakis(triphenylphosphine)palladium (109 mg, 0.09 mmol) in dioxane (9.5 mL) was degassed with Ar for 5 min and then heated under microwave irradiation at 150 °C for 30 min. Tetrakis(triphenylphosphine)palladium (109 mg, 0.09 mmol) was added and the mixture heated under microwave irradiation at 150 °C for 30 min; this operation was then repeated one more time. The reaction mixture was concentrated under reduced pressure and the residue purified by column chromatography ($SiO_2$, cyclohexane/ethyl acetate 1/0 to 1/1) to give 3-fluoro-1-methyl-4-tributylstannyl-pyridin-2-one (326 mg, 0.78 mmol) as a colourless oil.

**Step c:** A mixture of 3-fluoro-1-methyl-4-tributylstannyl-pyridin-2-one (140 mg, 0.33 mmol), (R)-N-[(5S)-1'-(7-bromo-6-methyl-pyrazolo[1,5-a]pyrazin-4-yl)spiro[5,7-dihydrocyclopenta[b]pyridine-6,4'-piperidine]-5-yl]-2-methyl-propane-2-sulfinamide (183 mg, 0.33 mmol), tetrakis(triphenylphosphine)palladium (19 mg, 17 $\mu$mol) and CuI (26 mg, 0.14 mmol) in dioxane (1.7 mL) was degassed with Ar for 5 min and then stirred at 110 °C for 16 h The reaction mixture was concentrated under vacuum and the residue was purified by column chromatography ($SiO_2$, dichloromethane/MeOH containing 1% concentrated $NH_3$ (aq) = 1/0 to 9/1) to give (R)-N-[(5S)-1'-[7-(3-fluoro-1-methyl-2-oxo-4-pyridyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (53 mg, 0.09 mmol) as an off-white solid. LCMS m/z $[M+H]^+$ = 564.3.

**Step d:** As described for **Example S1** - step b, a similar procedure was performed using (R)-N-[(5S)-1'-[7-(3-fluoro-1-methyl-2-oxo-4-pyridyl)-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (52 mg, 0.09 mmol) and a solution of HCl (4 N indioxane, 0.14 mL, 0.55 mmol) to give 4-[4-[(5S)-5-aminospiro[5,7-dihydrocyclopenta[b]pyridine-6,4'-piperidine]-1'-yl]-6-methyl-pyrazolo[1,5-a]pyrazin-7-yl]-3-fluoro-1-methyl-pyridin-2-one (33 mg, 0.07 mmol) as a white solid. LCMS m/z $[M+H]^+$ = 460.0. $^1$HNMR (500 MHz, DMSO-*d6*) $\delta$ ppm 1.22 (br d, *J* = 14 Hz, 1 H), 1.61 (br d, *J* = 13 Hz, 1 H), 1.76 - 1.96 (m, 4 H), 2.19 (s, 3 H), 2.79 (d, *J* = 16 Hz, 1 H), 3.15 (m, 1 H), 3.38 - 3.44 (m, 2 H), 3.57 (s, 3 H), 3.92 (s, 1 H), 4.37 - 4.47 (m, 2 H), 6.30 (t, *J* = 6 Hz, 1 H), 7.02 (d, *J* = 2 Hz, 1 H), 7.18 (dd, *J* = 7, 5 Hz, 1 H), 7.63 - 7.67 (m, 2 H), 7.89 *(d, J* = 2 Hz, 1 H), 8.32 (d, *J*= 4 Hz, 1 H). SHP2 $IC_{50}$ is 86 nM.

**Example S29. (5S)-1'-[7-[(3-fluoro-2-methyl-4-pyridyl)sulfanyl]-6-methyl-pyrazolo[1,5-a]pyrazin-4-yl]spiro[5,7-dihydrocyclopenta[b]pyridine-6,4'-piperidine]-5-amine) (Compound 229)**

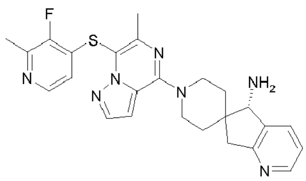

**Step a:** A mixture of 4-bromo-3-fluoro-2-methylpyridine (500 mg, 2.63 mmol), DIEA (0.92 mL, 5.28 mmol), XantPhos (155 mg, 0.27 mmol), 2-ethylhexyl 3-mercaptopropionate (0.66 mL, 2.9 mmol) and $Pd_2(dba)_3$ (120 mg, 0.13 mmol) in dioxane (12 mL) was degassed with Ar for 5 min and then heated under microwave irradiation at 110 °C for 1

h. The reaction mixture was poured into water (50 mL) and then extracted with ethyl acetate (50 mL * 3). The combined organic phase was washed with brine (3.00 L), dried with anhydrous $Na_2SO_4$, filtered and concentrated under vacuum. The residue was purified by column chromatography ($SiO_2$, cyclohexane/ethyl acetate = 95/5 to 8/2) to give 2-ethylhexyl 3-[(3-fluoro-2-methyl-4-pyridyl)sulfanyl]propanoate (730 mg, 2.23 mmol) as an orange oil. LCMS m/z $[M+H]^+$ = 328.6.

**Step b:** To a solution of 2-ethylhexyl 3-[(3-fluoro-2-methyl-4-pyridyl)sulfanyl]propanoate (725 mg, 2.21 mmol) in ethanol (20 mL) was added slowly a solution of sodium ethylate (21% in ethanol, 1.7 mL, 4.6 mmol). The mixture was stirred for 45 min and then a saturated aqueous solution of $NH_4Cl$ (6 mL) was added. The mixture was stirred for 10 mL and then filtered. The filtrate was concentrated under vacuum. The residue was taken up in diethyl ether and filtered to give 3-fluoro-2-methyl-pyridine-4-thiol (890 mg, crude) along with ammonium salt. The product was used without further purification.

**Step c:** A mixture of **intermediate D-2** (100 mg, 0.24 mmol), DIEA (0.17 mL, 0.98 mmol), XantPhos (15 mg, 0.026 mmol), 3-fluoro-2-methyl-pyridine-4-thiol (70 mg, crude previously obtained) and $Pd_2(dba)_3$ (12 mg, 0.013 mmol) in dioxane (2.5 mL) was degassed with Ar for 5 min and then heated under microwave irradiation at 110 °C for 45 min. XantPhos (15 mg, 0.026 mmol), 3-fluoro-2-methyl-pyridine-4-thiol (70 mg, crude previously obtained) and $Pd_2(dba)_3$ (12 mg, 0.013 mmol) were added, and the reaction mixture was heated under microwave irradiation at 110 °C for 1 h The reaction mixture was poured into water (20 mL) and then extracted with ethyl acetate (25 mL * 2). The combined organic phase was washed with brine (25 mL), dried with anhydrous $Na_2SO_4$, filtered and concentrated under vacuum. The residue was purified by column chromatography ($SiO_2$, dichloromethane/methanol = 1/0 to 95/5) to give (5S)-1'-[7-[(3-fluoro-2-methyl-4-pyridyl)sulfanyl]-6-methyl-pyrazolo[1,5-a]pyrazin-4-yl]spiro[5,7-dihydrocyclopenta[b]pyridine-6,4'-piperidine]-5-amine (21 mg, 44 μmol) as an orange solid. LCMS m/z $[M+H]^+$ = 476.2. $^1$H NMK (400 MHz, DMSO-*d6*) δ ppm 1.26 (br d, *J* = 11 Hz, 1 H), 1.67 (m, 1 H), 1.79 - 1.97 (m, 2 H), 1.98 - 2.12 (m, 2 H), 2.45 (d, *J* = 3 Hz, 3 H), 2.49 (s, 3 H), 2.81 (d, *J* = 16 Hz, 1 H), 3.17 (d, *J* = 16 Hz, 1 H), 3.32 - 3.57 (m, 2 H), 3.95 (s, 1 H), 4.48 - 4.58 (m, 2 H), 6.34 (t, *J* = 5 Hz, 1 H), 7.13 *(d, J*= 3 Hz, 1 H), 7.19 (dd, *J* = 7, 5 Hz, 1 H), 7.67 (d, *J* = 7 Hz, 1 H), 7.91 (d, *J* = 3 Hz, 1 H), 7.95 (d, *J* = 5 Hz, 1 H), 8.33 (d, *J* = 4 Hz, 1 H). SHP2 $IC_{50}$ is 14 nM.

**Example S30. (5S)-1'-(6-methyl-7-((2-(trifluoromethyl)pyridin-3-yl)thio)pyrazolo[1,5-a]pyrazin-4-yl)-5,7-dihydrospiro[cyclopenta[b]pyridine-6,4'-piperidin]-5-amine (Compound 230)**

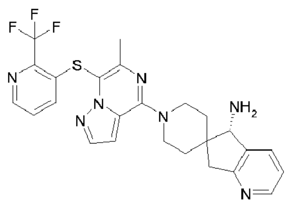

**Step** a: A mixture of **intermediate D-1** (115 mg, 0.22 mmol), DIEA (0.08 mL, 0.46 mmol), XantPhos (30 mg, 0.05 mmol), 2-(trifluoromethyl)pyridine-3-thiol (50 mg, 0.26 mmol) and $Pd_2(dba)_3$ (20 mg, 0.02 mmol) in dioxane (2.5 mL) was degassed with Ar for 5 min and then heated twice under microwave irradiation at 120 °C for 45 min. XantPhos (30 mg, 0.05 mmol), 2-(trifluoromethyl)pyridine-3-thiol (50 mg, 0.26 mmol) and $Pd_2(dba)_3$ (20 mg, 0.02 mmol) were added, and the reaction mixture was heated twice under microwave irradiation at 120 °C for 45 min. XantPhos (30 mg, 0.05 mmol), 2-(trifluoromethyl)pyridine-3-thiol (50 mg, 0.26 mmol) and $Pd_2(dba)_3$ (20 mg, 0.02 mmol) were added, and the reaction mixture was heated under microwave irradiation at 120 °C for 45 min. The reaction mixture was poured into water (25 mL) and then extracted with ethyl acetate (25 mL * 2). The combined organic phase was washed with brine (25 mL), dried with anhydrous $Na_2SO_4$, filtered and concentrated under vacuum. The residue was purified by column chromatography ($SiO_2$, dichloromethane/methanol = 1/0 to 95/5) to give (R)-2-methyl-N-((S)-1'-(6-methyl-7-((2-(trifluoromethyl)pyridin-3-yl)thio)pyrazolo[1,5-a]pyrazin-4-yl)-5,7-dihydrospiro[cyclopenta[b]pyridine-6,4'-piperidin]-5-yl)propane-2-sulfinamide (55 mg, 0.09 mmol) as an orange solid.

**Step b:** As described for **Example S1** - step b, a similar procedure was performed using (R)-2-methyl-N-((S)-1'-(6-methyl-7-((2-(trifluoromethyl)pyridin-3-yl)thio)pyrazolo[1,5-a]pyrazin-4-yl)-5,7-dihydrospiro[cyclopenta[b]pyridine-6,4'-piperidin]-5-yl)propane-2-sulfinamide (55 mg, 0.09 mmol) and a solution of HCl (4 N in dioxane, 0.25 mL, 1 mmol) to give (5S)-1'-(6-methyl-7-((2-(trifluoromethyl)pyridin-3-yl)thio)pyrazolo[1,5-a]pyrazin-4-yl)-5,7-dihydrospiro[cyclopenta[b]pyridine-6,4'-piperidin]-5-amine (25 mg, 0.05 mmol) as an orange solid. LCMS m/z $[M+H]^+$ = 512.3. $^1$H NMR (400 MHz, DMSO-*d6*) δ 1.26 (d, *J*= 13 Hz, 1 H), 1.66 (d, *J*= 13 Hz, 1 H), 1.78 - 1.94 (m, 2 H), 1.93 - 2.12 (m, 2 H), 2.52 (s hidden, 3 H), 2.81 (d, *J* = 16 Hz, 1 H), 3.17 (d, *J* = 16 Hz, 1 H), 3.37 - 3.54 (m, 2 H), 3.94 (s, 1 H), 4.48 - 4.60 (m, 2 H), 7.08 - 7.15 (m, 2 H), 7.19 (dd, *J* = 7, 5 Hz, 1 H), 7.43 (dd, *J*= 8, 5 Hz, 1 H), 7.67 (d, *J* = 7 Hz, 1 H), 7.90 (d, *J* = 2 Hz, 1

H), 8.33 (d, *J* = 4 Hz, 1 H), 8.47 (d, *J* = 4 Hz, 1 H). SHP2 $IC_{50}$ is 15 nM.

**Example S31. (S)-1'-(7-((3-chloro-2-methylpyridin-4-yl)thio)-6-methylpyrazolo[1,5-a]pyrazin-4-yl)-5,7-dihydrospiro[eyelopenta[b]pytidine-6,4'-piperidin]-5-amine (Compound 231)**

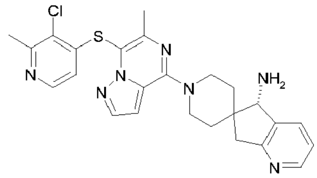

As described for **Example S30** (Compound **230),** a similar procedure was performed using **intermediate D-1** (150 mg, 0.29 mmol) and 3-chloro-2-methylpyridine-4-thiol (70 mg, 0.42 mmol) to give (S)-1'-(7-((3-chloro-2-methylpyridin-4-yl)thio)-6-methylpyrazolo[1,5-a]pyrazin-4-yl)-5,7-dihydrospiro[cyclopenta[b]pyridine-6,4'-piperidin]-5-amine (5 mg, 0.01 mmol) as a white solid. LCMS m/z $[M+H]^+$ = 492.2. $^1$H NMR (500 MHz, DMSO-*d6*) $\delta$ 1.26 (br d, *J*= 14 Hz, 1 H), 1.66 (br d, *J* = 13 Hz, 1 H), 1.79 - 1.95 (m, 2 H), 2.04 - 2.32 (m, 2 H), 2.47 (s, 3 H), 2.57 (s, 3 H), 2.82 (d, *J*= 16 Hz, 1 H), 3.17 (d, *J* = 16 Hz, 1 H), 3.43 - 3.55 (m, 2 H), 3.95 (s, 1 H), 4.54 (t, J= 13 Hz, 2 H), 6.24 (d, *J* = 5 Hz, 1 H), 7.14 (d, *J* = 2 Hz, 1 H), 7.19 (dd, *J* = 7, 5 Hz, 1 H), 7.68 (d, *J* = 7 Hz, 1 H), 7.90 *(d, J* = 2 Hz, 1 H), 8.02 (d, *J* = 5 Hz, 1 H), 8.34 (d, *J*= 4 Hz, 1 H). SHP2 $IC_{50}$ is 8 nM.

**Example S32. (5S)-1'-[7-[(2-amino-3-chloro-4-pyridyl)sulfanyl]-6-methyl-pyrazolo[1,5-a]pyrazin-4-yl]spiro[5,7-dihydrocyclopenta[b]pyridine-6,4'-piperidine]-5-amine (Compound 232)**

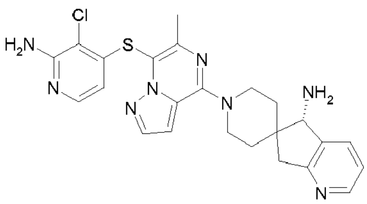

**Step a:** A mixture of **intermediate D-1** (150 mg, 0.29 mmol), DIEA (0.15 mL, 0.87 mmol), XantPhos (33.5 mg, 0.058 mmol), 2-amino-3-chloro-pyridine-4-thiol (51 mg, 0.32 mmol) in a mixture of dioxane (2.4 mL) and NMP (0.8 mL) was degassed with Ar for 5 min and then $Pd_2(dba)_3$ (28 mg, 0.03 mmol) was added and the mixture was heated under microwave irradiation at 120 °C for 1.5 h. The reaction mixture was poured into water (15 mL) and then extracted with ethyl acetate (15 mL * 3). The combined organic phase was washed with brine (15 mL), dried with anhydrous $Na_2SO_4$, filtered and concentrated under vacuum. The residue was purified by column chromatography ($SiO_2$, cyclohexane/EtOAc = 1/0 to 0/1 followed by EtOAc/methanol = 1/0 to 85/15) to give (R)-N-[(5S)-1'-[7-[(2-amino-3-chloro-4-pyridyl)sulfanyl]-6-methyl-pyrazolo[1,5-a]pyrazin-4-yl]spiro[5,7-dihydrocyclopenta[b]pyndine-6,4'-piperidine]-5-yl]-2-methyl-propane-2-sulfinamide (124 mg, 0.21 mmol) as a brown solid. LCMS m/z $[M+H]^+$ = 597.3.

**Step b:** As described for **Example S1** step b, a similar procedure was performed using (R)-N-[(5S)-1'-[7-[(2-amino-3-chloro-4-pyridyl)sulfanyl]-6-methyl-pyrazolo[1,5-a]pyrazin-4-yl]spiro[5,7-dihydrocyclopenta[b]pyridine-6,4'-piperidine]-5-yl]-2-methyl-propane-2-sulfinamide (123 mg, 0.21 mmol) and a solution of HCl (4 N in dioxane, 0.3 mL, 1.2 mmol) to give (5S)-1'-[7-[(2-amino-3-chloro-4-pyridyl)sulfanyl]-6-methyl-pyrazolo[1,5-a]pyrazin-4-yl]spiro[5,7-dihydrocyclopenta[b]pyridine-6,4'-piperidine]-5-amine (85 mg, 0.17 mmol) as a beige solid. LCMS m/z $[M+H]^+$ = 493.0. $^1$HNMR (400 MHz, DMSO-*d6*) $\delta$ ppm 1.24 (br d, *J* = 13 Hz, 1 H), 1.64 (br d, *J* = 13 Hz, 1 H), 1.75 - 2.02 (m, 4 H), 2.45 (s, 3 H), 2.80 (d, *J* = 16 Hz, 1 H), 3.16 (d, *J* = 16 Hz, 1 H), 3.39 - 3.55 (m, 2 H), 3.93 (s, 1 H), 4.45 - 4.59 (m, 2 H), 5.56 *(d, J*= 5 Hz, 1 H), 6.32 (s, 2 H), 7.11 (d, *J* = 3 Hz, 1 H), 7.18 (dd, *J* = 8, 5 Hz, 1 H), 7.53 *(d, J*= 5 Hz, 1 H), 7.66 *(d, J* = 8 Hz, 1 H), 7.90 (d, *J* = 3 Hz, 1 H), 8.32 (d, *J* = 4 Hz, 1 H). SHP2 $IC_{50}$ is 4 nM.

*Biological Examples*

**Example B1. SHP2 Phosphatase Assay**

Inhibition of SHP2 phosphatase activity was evaluated using human recombinant 6His-Nterm SHP2 full-length (Thr2-Arg593, R&D system), NsCs bi-phosphorylated activating peptide ($NH_2$-LN(pY)AQLWHA(PEG8)LTI(pY)ATIRRF-Amide.acetate, Thermofischer Scientific) and DiFMUP as surrogate phosphatase substrate (Molecular Probes). All reactions were performed at room temperature in 384-well black polystyrene non-binding plate (Corning) with the assay buffer (50 mM HEPES-NaOH pH 7.2, 100 mM NaCl, 0.5 mM EDTA, 0.001% Brij35 including 0.02% BSA and 1 mM DTT added extemporaneously). SHP2 was pre-incubated at least 10 min with the activating peptide (0.2 nM and 100 nM, respectively). 10 μL of this mixture was added to the assay plate, that already contained 15 nL of the compound (1000-0.05 nM). After 30 min pre-incubation, the enzymatic reaction was initiated by the addition of 5 μL DiFMUP in assay buffer without BSA (20 μM). The reaction was stopped by the addition of 15 μL sodium pervanadate solution (100 μM) after 45 min incubation. The fluorescence signal was measured at excitation and emission wavelengths of 350 nm and 450 nm, respectively, using a Pherastar instrument (BMG-LabTech). Percentage of inhibition (1%) were calculated from the fluorescence signal at 450 nm of the positive and negative control using the following formula:

$$I\% = \left(\frac{signal\ Max\ - signal\ Sample}{signal\ Max\ - signal\ Min\ control}\right) \times\ 100$$

Positive control (Max): corresponds to the maximum fluorescence signal obtained in the presence of 15 nL DMSO replacing the inhibitor in the pre-incubation step.

Negative control (Min): corresponds to the minimum signal obtained in the presence of 1 μM of RMC-4550, the structure of which is shown below:

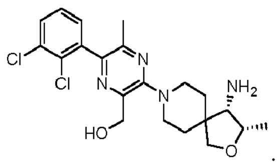.

The $IC_{50}$ were then calculated from a dose-response curve of 10 concentrations of tested compound using the 4-parameter logistic model:

$$I\% = A + \frac{C}{1 + exp(-B \times log(X) - M)}$$

The parameters A and (A+C) are the lower and upper limits, B is the slope of the curve at the inflexion point, M parameter is the logarithm of the abscissa at the inflexion point, and X is the concentration of compound.

The $IC_{50}$ values for tested compounds are provided above in Examples S1-S32 and Tables 2-10, and are summarized in Table 11 below.

**Table 11.**

| Compound No. | SHP2 $IC_{50}$ (nM) | Compound No. | SHP2 $IC_{50}$ (nM) |
|---|---|---|---|
| **1** | 6 | **2** | 6 |
| **3** | 3 | **4** | 7 |
| **5** | 3 | **6** | 8 |
| **7** | 13 | **8** | 6 |
| **9** | 43 | **10** | 11 |
| **11** | 23 | **12** | 14 |
| **13** | 7 | **14** | 40 |

(continued)

| Compound No. | SHP2 $IC_{50}$ (nM) | Compound No. | SHP2 $IC_{50}$ (nM) |
|---|---|---|---|
| **15** | 20 | **16** | 42 |
| **17** | 39 | **18** | 132 |
| **19** | 102 | **20** | 115 |
| **21** | 58 | **22** | 11 |
| **23** | 10 | **24** | 11 |
| **25** | 6 | **26** | 23 |
| **27** | 16 | **28** | 15 |
| **29** | 38 | **30** | 17 |
| **31** | 13 | **32** | 6 |
| **33** | > 1000 | **34** | 3 |
| **35** | 8 | **36** | 228 |
| **37** | 9 | **38** | 12 |
| **39** | 5 | **40** | 10 |
| **41** | 15 | **42** | 3 |
| **43** | 4 | **44** | 15 |
| **45** | 8 | **46** | 73 |
| **47** | 154 | **48** | 17 |
| **49** | 130 | **50** | 10 |
| **51** | 5 | **52** | 24 |
| **53** | 3 | **54** | 4 |
| **55** | 30 | **56** | 17 |
| **57** | 10 | **58** | 35 |
| **59** | 11 | **60** | 7 |
| **61** | 211 | **62** | 17 |
| **63** | 112 | **64** | 46 |
| **65** | 22 | **66** | 22 |
| **67** | 115 | **68** | 18 |
| **69** | 10 | **70** | 73 |
| **71** | 30 | **72** | 27 |
| **73** | 5 | **74** | 5 |
| **75** | 7 | **76** | 72 |
| **77** | 4 | **78** | 12 |
| **79** | 10 | **80** | 17 |
| **81** | 4 | **82** | 27 |
| **83** | 10 | **84** | 5 |
| **85** | 27 | **86** | 60 |
| **87** | 22 | **88** | 346 |
| **89** | 9 | **90** | 13 |

(continued)

| Compound No. | SHP2 $IC_{50}$ (nM) | Compound No. | SHP2 $IC_{50}$ (nM) |
|---|---|---|---|
| **91** | 19 | **92** | 8 |
| **93** | 46 | **94** | 146 |
| **95** | 19 | **96** | 11 |
| **97** | 42 | **98** | 145 |
| **99** | 9 | **100** | 13 |
| **101** | 15 | **102** | 17 |
| **103** | 12 | **104** | 9 |
| **105** | 6 | **106** | 14 |
| **107** | 12 | **108** | 6 |
| **109** | 10 | **110** | 11 |
| **111** | 16 | **112** | 5 |
| **113** | 28 | **114** | 10 |
| **115** | 11 | **116** | 8 |
| **117** | 10 | **118** | 4 |
| **119** | 4 | **120** | 11 |
| **121** | 8 | **122** | 12 |
| **123** | 9 | **124** | 391 |
| **125** | 9 | **126** | 4 |
| **127** | 22 | **128** | 17 |
| **129** | > 1000 | **130** | 60 |
| **131** | 13 | **132** | 34 |
| **133** | 21 | **134** | 4 |
| **135** | 12 | **136** | 383 |
| **137** | 6 | **138** | 4 |
| **139** | 4 | **140** | 7 |
| **141** | 853 | **142** | 26 |
| **143** | 39 | **144** | 17 |
| **145** | > 1000 | **146** | > 1000 |
| **147** | > 1000 | **148** | 9 |
| **149** | 17 | **150** | 140 |
| **151** | 9 | **152** | 9 |
| **153** | 15 | **154** | 4 |
| **155** | 202 | **156** | 35 |
| **157** | 72 | **158** | 47 |
| **159** | 649 | **160** | 63 |
| **161** | 29 | **162** | 4 |
| **163** | 30 | **164** | 7 |
| **165** | 370 | **166** | 7 |

(continued)

| Compound No. | SHP2 $IC_{50}$ (nM) | Compound No. | SHP2 $IC_{50}$ (nM) |
|---|---|---|---|
| **167** | 9 | **168** | 30 |
| **169** | 16 | **170** | 11 |
| **171** | 20 | **172** | > 1000 |
| **173** | 15 | **174** | 5 |
| **175** | 4 | **176** | 6 |
| **177** | 27 | **178** | 14 |
| **179** | 18 | **180** | 39 |
| **181** | 5 | **182** | 23 |
| **183** | 3 | **184** | 11 |
| **185** | 7 | **186** | 2 |
| **187** | 250 | **188** | 7 |
| **189** | 4 | **190** | 31 |
| **191** | 16 | **192** | 2 |
| **193** | 3 | **194** | 5 |
| **195** | 4 | **196** | 83 |
| **197** | 7 | **198** | 437 |
| **199** | 14 | **200** | 44 |
| **201** | 3 | **202** | 26 |
| **203** | 6 | **204** | 55 |
| **205** | 11 | **206** | 12 |
| **207** | 16 | **208** | 3 |
| **209** | 21 | **210** | > 1000 |
| **211** | 8 | **212** | 288 |
| **213** | 22 | **214** | 594 |
| **215** | 23 | **216** | 685 |
| **217** | 8 | **218** | 179 |
| **219** | 5 | **220** | 26 |
| **221** | 21 | **222** | 1 |
| **223** | 13 | **224** | 27 |
| **225** | > 1000 | **226** | > 1000 |
| **227** | > 1000 | **228** | 86 |
| **229** | 14 | **230** | 15 |
| **231** | 8 | **232** | 4 |

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby. The disclosures of all patent and scientific

literature cited herein are expressly incorporated herein in their entirety by reference. To the extent that any incorporated material is inconsistent with the express content of this disclosure, the express content controls.

## Claims

1. A compound of Formula (I):

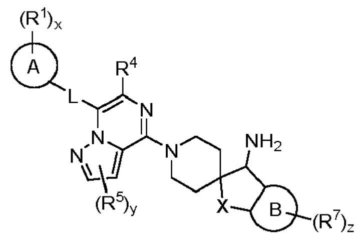

(I)

or a pharmaceutically acceptable salt thereof, wherein:

Ring A is $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S;
each $R^1$ is independently halo, cyano, $-NR^{2a}R^{2b}$, $C_1$-$C_6$ alkyl, oxo, hydroxy, $C_1$-$C_6$ haloalkyl, $C_1$-$C_6$ alkoxy, $C_1$-$C_6$ alkyl-OH, $C_1$-$C_6$ alkyl-CN, $-C(O)NR^{2a}R^{2b}$, $-C(O)(C_1$-$C_6$ alkyl), $-CO_2H$, $-CO_2(C_1$-$C_6$ alkyl), $-Si(R^a)(R^b)(R^c)$, $-P(O)(R^a)(R^b)$, $-OP(O)(R^a)(R^b)$, $C_3$-$C_6$ cycloalkyl, phenyl, 5- to 6-membered heterocycloalkyl, or 5- to 6-membered heteroaryl, wherein the heterocycloalkyl and heteroaryl contain 1-3 heteroatoms selected from N, O, and S;
or two $R^1$ groups are taken together with the carbon atoms or heteroatoms to which they are attached to form a fused phenyl, 5- to 6-membered heterocycloalkyl, or 5- to 6-membered heteroaryl, each of which is optionally substituted with 1-4 $R^6$ groups, wherein the fused heterocycloalkyl and heteroaryl contain 1-3 heteroatoms selected from N, O, and S;
each $R^a$, $R^b$, and $R^c$ is independently hydroxy, $C_1$-$C_6$ alkyl, or $C_1$-$C_6$ alkoxy;
each $R^{2a}$ and $R^{2b}$ is independently H, $C_1$-$C_6$ alkyl, or $C_3$-$C_6$ cycloalkyl;
L is a bond, S, O, C(O), or $N(R^d)$;
$R^d$ is H or $C_1$-$C_6$ alkyl;
X is $CR^{3a}R^{3b}$, $NR^{3a}$, or O;
$R^{3a}$ and $R^{3b}$ are independently H or $C_1$-$C_6$ alkyl;
$R^4$ is H, $C_1$-$C_6$ alkyl, $C_1$-$C_6$ alkyl-OH, $C_1$-$C_6$ haloalkyl, or $-NH_2$;
each $R^5$ is independently halo, $C_1$-$C_6$ alkyl, $C_1$-$C_6$ haloalkyl, $-(C_1$-$C_6$ alkylene)($C_1$-$C_6$ alkoxy), or $C_1$-$C_6$ alkyl-OH;
Ring B is fused phenyl or 5- to 6-membered heteroaryl containing 1-3 heteroatoms selected from N, O, and S;
each $R^6$ is independently $C_1$-$C_6$ alkyl, halo, or $C_1$-$C_6$ haloalkyl;
each $R^7$ is independently $C_1$-$C_6$ alkyl, halo, $C_1$-$C_6$ alkoxy, $C_1$-$C_6$ alkyl-OH, hydroxy, cyano, $-Si(R^a)(R^b)(R^c)$, $-P(O)(R^a)(R^b)$, $-OP(O)(R^a)(R^b)$, $-NR^{2a}R^{2b}$, or $C_1$-$C_6$ haloalkyl;
x is 0-5;
y is 0-2; and
z is 0-4;

wherein one or more hydrogen atoms in the compound are optionally replaced by deuterium.

2. The compound of claim 1, or a pharmaceutically acceptable salt thereof, wherein:

Ring A is $C_3$-$C_5$ cycloalkyl, phenyl, 6-membered heterocycloalkyl, or 5- to 6-membered heteroaryl, wherein the heterocycloalkyl and heteroaryl contain 1-2 heteroatoms selected from N, O, and S; or wherein:
Ring A is cyclopropyl, phenyl, dihydropyridinyl, dihydropyranyl, pyridinyl, pyridazinyl, pyrimidinyl, pyrazolyl, imidazolyl, pyrrolyl, thiazolyl, isoxazolyl, or thiophenyl; or wherein:

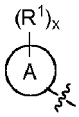

is:

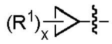, 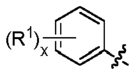, 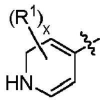, 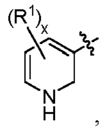, 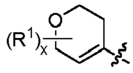, 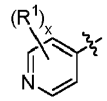,

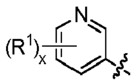, 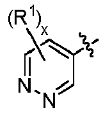, 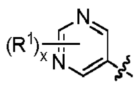, 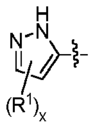, 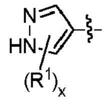, 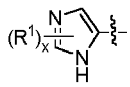,

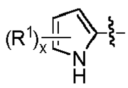, 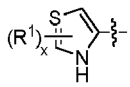, 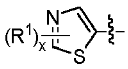, 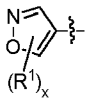, 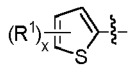, or 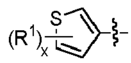.

**3.** The compound of any one of claims 1-2, or a pharmaceutically acceptable salt thereof, wherein:

each $R^1$ is independently halo, cyano, $-NR^{2a}R^{2b}$, $C_1$-$C_3$ alkyl, oxo, hydroxy, $C_1$-$C_3$ haloalkyl, $C_1$-$C_3$ alkoxy, $C_1$-$C_3$ alkyl-OH, $C_1$-$C_3$ alkyl-CN, $-C(O)NR^{2a}R^{2b}$, $-C(O)(C_1$-$C_3$ alkyl), $-CO_2H$, $-CO_2(C_1$-$C_3$ alkyl), $-Si(R^a)(R^b)(R^c)$, $-P(O)(R^a)(R^b)$, $-OP(O)(R^a)(R^b)$, $C_3$-$C_5$ cycloalkyl, phenyl, or 6-membered heterocycloalkyl, wherein the heterocycloalkyl contains 1-2 heteroatoms selected from N and O;
or two $R^1$ groups are taken together with the carbon atoms or heteroatoms to which they are attached to form a fused phenyl, 5- to 6-membered heterocycloalkyl, or 5- to 6-membered heteroaryl, each of which is optionally substituted with 1-2 $R^6$ groups, wherein the fused heterocycloalkyl and heteroaryl contain 1-2 heteroatoms selected from N, O, and S;
each $R^a$, $R^b$, and $R^c$ is independently $C_1$-$C_3$ alkyl or $C_1$-$C_3$ alkoxy;
each $R^{2a}$ and $R^{2b}$ is independently H, $C_1$-$C_3$ alkyl, or $C_3$-$C_5$ cycloalkyl; and
each $R^6$ is independently $C_1$-$C_3$ alkyl, halo, or $C_1$-$C_3$ haloalkyl.

**4.** The compound of any one of claims 1-3, or a pharmaceutically acceptable salt thereof, wherein:

each $R^1$ is independently F, Cl, -CN, $-CH_2CN$, $-NH_2$, $-N(H)CH_3$, $-N(CH_3)_2$, $-CH_3$, $-CH_2CH_3$, $-CH(CH_3)_2$, oxo, $-CF_3$, $-OCH_3$, $-CH_2OH$, $-C(O)N(CH_3)_2$, $-C(O)CH_3$, cyclopropyl, or

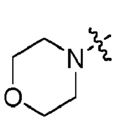;

or two $R^1$ groups are taken together with the carbon atoms or heteroatoms to which they are attached to form a fused group selected from:

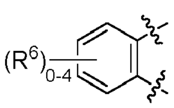, 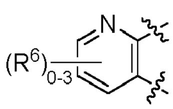, 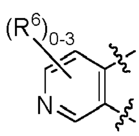, 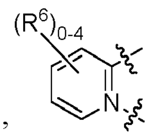, 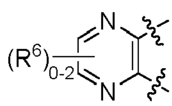, 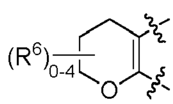,

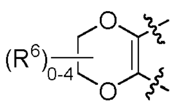, 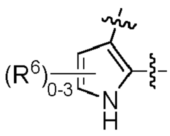, 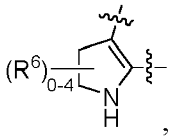, 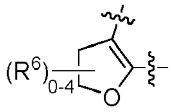, and 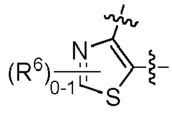

**5.** The compound of any one of claims 1-4, or a pharmaceutically acceptable salt thereof, wherein:

each $R^7$ is independently $C_1$-$C_3$ alkyl, halo, $C_1$-$C_3$ alkoxy, $C_1$-$C_3$ alkyl-OH, hydroxy, cyano, -Si($R^a$)($R^b$)($R^c$), -P(O)($R^a$)($R^b$), -OP(O)($R^a$)($R^b$), -$NR^{2a}R^{2b}$, or $C_1$-$C_3$ haloalkyl;
each $R^a$, $R^b$, and $R^c$ is independently hydroxy, $C_1$-$C_3$ alkyl, or $C_1$-$C_3$ alkoxy; and
each $R^{2a}$ and $R^{2b}$ is independently H, $C_1$-$C_3$ alkyl, or $C_3$-$C_5$ cycloalkyl; or whereinL
each $R^7$ is independently $CH_3$, F, -$OCH_3$, -$CH_2OH$, hydroxy, -CN, -$N(CH_3)_2$, or -$CHF_2$.

**6.** The compound of any one of claims 1-5, or a pharmaceutically acceptable salt thereof, wherein the compound is of Formula (IIa), (IIb), (IIc), or (IId):

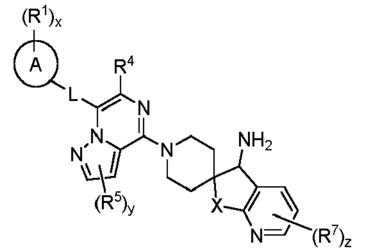

(IIa)

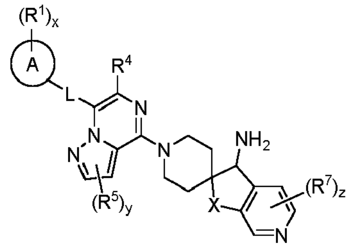

(IIb)

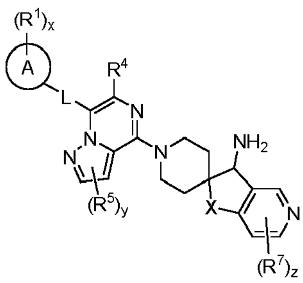

(IIc)

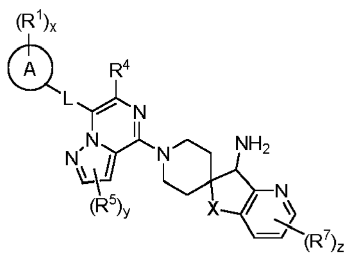

(IId)

.

**7.** The compound of claim 6, or a pharmaceutically acceptable salt thereof, wherein the compound is of Formula (IIa-1):

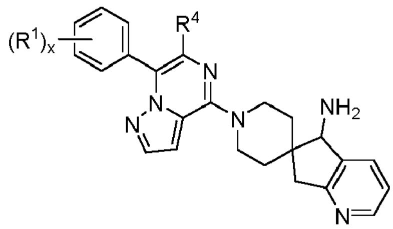

(IIa-1)

.

**8.** The compound of any one of claims 1-7, or a pharmaceutically acceptable salt thereof, wherein the compound is of Formula (IIIa), (IIIb), (IIIc), (IIId), (IIIe), or (IIIf):

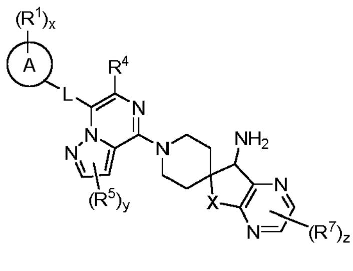
(IIIa)

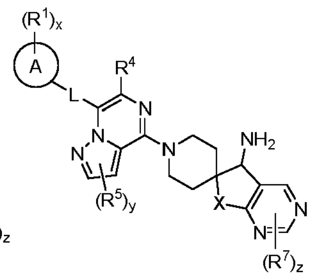
(IIIb)

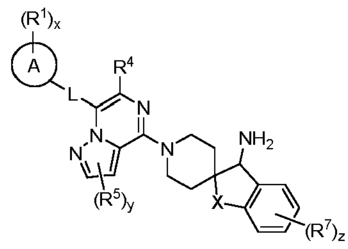
(IIIc)

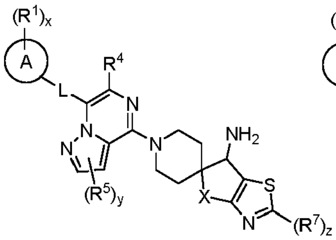
(IIId)

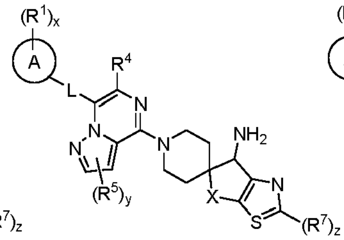
(IIIe)

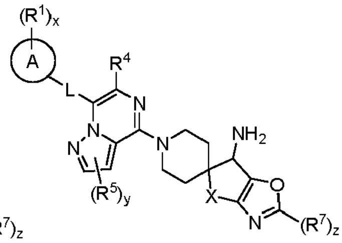
(IIIf)

.

**9.** The compound of any one of claims 1-8, or a pharmaceutically acceptable salt thereof, wherein the compound is of Formula (IVa):

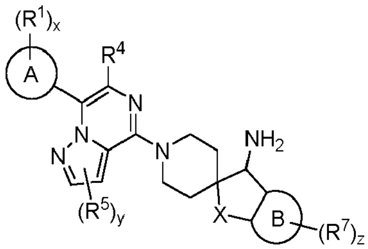
(IVa)

**10.** The compound of any one of claims 1-9, or a pharmaceutically acceptable salt thereof, wherein the compound is of Formula (IVb), (IVc), (IVd), or (IVe):

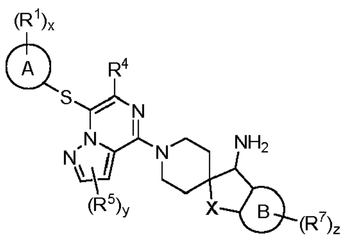
(IVb)

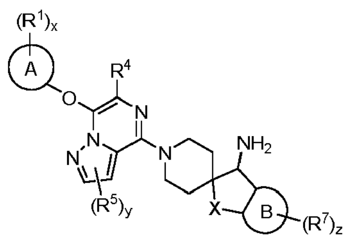
(IVc)

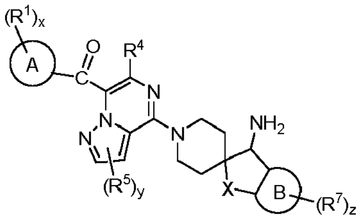

(IVd)

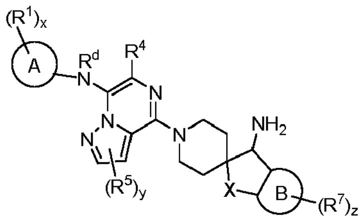

(IVe)

.

**11.** A compound selected from the compounds of Table 1 or a pharmaceutically acceptable salt thereof.

**12.** A pharmaceutical composition comprising the compound of any one of claims 1-11, or a pharmaceutically acceptable salt thereof, and a pharmaceutically acceptable excipient.

**13.** A method of inhibiting SHP2 comprising contacting SHP2 with an effective amount of the compound of any one of claims 1-10, or a pharmaceutically acceptable salt thereof, or the pharmaceutical composition of claim 12.

**14.** A method of treating a disease associated with SHP2 modulation in a subject in need thereof, comprising administering to the subject an effective amount of the compound of any one of claims 1-10, or a pharmaceutically acceptable salt thereof, or the pharmaceutical composition of claim 12.

**15.** The method of claim 38, wherein the disease is Noonan Syndrome, Leopard Syndrome, juvenile myelomonocytic leukemias, neuroblastoma, melanoma, acute myeloid leukemia, breast cancer, lung cancer, colon cancer, or brain cancer; or wherein the brain cancer is glioblastoma.

European Patent Office

# EUROPEAN SEARCH REPORT

Application Number
EP 22 15 6277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/063760 A1 (JACOBIO PHARMACEUTICALS CO LTD [CN]) 2 April 2020 (2020-04-02) * abstract * * page 117 - page 128; compounds 89, 90, 183, 184, * * claim 1 * ----- | 1-15 | INV. C07D487/04 C07D519/00 A61K31/4985 A61P35/00 |
| X | CN 111 704 611 A (SHANGHAI RINGENE BIOPHARMA CO LTD) 25 September 2020 (2020-09-25) * abstract * * page 7, paragraph 32 - page 8, paragraph 33 * * claim 1 * ----- | 1-15 | |
| Y | WO 2020/072656 A1 (GILEAD SCIENCES INC [US]) 9 April 2020 (2020-04-09) * abstract * * page 249 - page 308; compounds 85, 86, 121, 132, 135, 137-149 * * claim 1 * ----- | 1-15 | TECHNICAL FIELDS SEARCHED (IPC) |
| Y | EP 3 878 853 A1 (SHANGHAI RINGENE BIOPHARMA CO LTD [CN]) 15 September 2021 (2021-09-15) * abstract * * page 52 - page 62; examples 1-20 * * claim 1 * ----- | 1-15 | C07D A61P A61K |
| Y | EP 3 889 153 A1 (TUOJIE BIOTECH SHANGHAI CO LTD [CN]) 6 October 2021 (2021-10-06) * abstract * * page 31 - page 64; examples 8, 27-44 * * claim 1 * ----- | 1-15 | |

The present search report has been drawn up for all claims

1

| Place of search | Date of completion of the search | Examiner |
|---|---|---|
| Munich | 9 June 2022 | Bissmire, Stewart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 15 6277

This annex lists the patent family members relating to the patent documents cited in the above-mentioned European search report. The members are as contained in the European Patent Office EDP file on
The European Patent Office is in no way liable for these particulars which are merely given for the purpose of information.

09-06-2022

| Patent document cited in search report | Publication date | Patent family member(s) | Publication date |
|---|---|---|---|
| WO 2020063760 A1 | 02-04-2020 | CN 112839935 A | 25-05-2021 |
| | | US 2021393623 A1 | 23-12-2021 |
| | | WO 2020063760 A1 | 02-04-2020 |
| CN 111704611 A | 25-09-2020 | NONE | |
| WO 2020072656 A1 | 09-04-2020 | EP 3860717 A1 | 11-08-2021 |
| | | US 2020108071 A1 | 09-04-2020 |
| | | WO 2020072656 A1 | 09-04-2020 |
| EP 3878853 A1 | 15-09-2021 | CN 111153901 A | 15-05-2020 |
| | | EP 3878853 A1 | 15-09-2021 |
| | | JP 2022506887 A | 17-01-2022 |
| | | KR 20210089716 A | 16-07-2021 |
| | | US 2022127271 A1 | 28-04-2022 |
| | | WO 2020094104 A1 | 14-05-2020 |
| EP 3889153 A1 | 06-10-2021 | AU 2019386036 A1 | 27-05-2021 |
| | | BR 112021009880 A2 | 17-08-2021 |
| | | CA 3120791 A1 | 04-06-2020 |
| | | CN 113272303 A | 17-08-2021 |
| | | EP 3889153 A1 | 06-10-2021 |
| | | JP 2022509149 A | 20-01-2022 |
| | | KR 20210097144 A | 06-08-2021 |
| | | TW 202039498 A | 01-11-2020 |
| | | WO 2020108590 A1 | 04-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6352694 B
- US 6534055 B
- US 6905680 B
- US 6692964 B
- US 5858358 A
- US 6887466 B
- US 6905681 B
- US 7144575 B
- US 7067318 B
- US 7172869 B
- US 7232566 B
- US 7175843 B
- US 7572631 B
- US 5883223 A
- US 6905874 B
- US 6797514 B
- US 6867041 B
- US 6258812 B
- US 6630500 B
- US 6515004 B
- US 6713485 B
- US 5521184 A
- US 5770599 A
- US 5747498 A
- US 5990141 A
- US 6235764 B
- US 8623885 B
- WO 0137820 A
- WO 0132651 A
- WO 0268406 A
- WO 0266470 A
- WO 0255501 A
- WO 0405279 A
- WO 0407481 A
- WO 0407458 A
- WO 0409784 A
- WO 0259110 A
- WO 9945009 A
- WO 0059509 A
- WO 9961422 A
- WO 0012089 A
- WO 0002871 A
- WO 05016894 A
- WO 2021173524 A
- WO 2021130731 A
- WO 2021127429 A
- WO 2021092115 A
- WO 2021105960 A
- WO 2021074227 A
- WO 2020180768 A
- WO 2020180770 A
- WO 2020173935 A
- WO 2020146470 A
- WO 2019201848 A
- WO 2019122129 A
- WO 2018172250 A
- WO 2018115380 A
- WO 2021091982 A
- WO 2021091967 A
- WO 2021091956 A
- WO 2020132597 A
- WO 2021239058 A
- WO 2021236920 A
- WO 2021231526 A
- WO 2021228161 A
- WO 2021222333 A
- WO 2021219091 A
- WO 2021219090 A
- WO 2021219072 A
- WO 2021218939 A
- WO 2021217019 A
- WO 2021216770 A
- WO 2021215545 A
- WO 2021215544 A
- WO 2021211864 A
- WO 2021197499 A
- WO 2021190467 A
- WO 2021185233 A
- WO 2021180181 A
- WO 2021175199 A
- WO 2021173923 A
- WO 2021169990 A
- WO 2021169963 A
- WO 2021168193 A
- WO 2021158071 A
- WO 2021155716 A
- WO 2021152149 A
- WO 2021150613 A
- WO 2021147967 A
- WO 2021147965 A
- WO 2021143693 A
- WO 2021142252 A
- WO 2021141628 A
- WO 2021139748 A
- WO 2021139678 A
- WO 2021129824 A
- WO 2021129820 A
- WO 2021127404 A

- WO 2021126816 A
- WO 2021126799 A
- WO 2021124222 A
- WO 2021121371 A
- WO 2021121367 A
- WO 2021121330 A
- WO 2020050890 A
- WO 2020047192 A
- WO 2020035031 A
- WO 2020028706 A
- WO 2019241157 A
- WO 2019232419 A
- WO 2019217691 A
- WO 2019217307 A
- WO 2019215203 A
- WO 2019213526 A
- WO 2019213516 A
- WO 2019155399 A
- WO 2019150305 A
- WO 2019110751 A
- WO 2019099524 A
- WO 2019051291 A
- WO 2018218070 A
- WO 2018217651 A
- WO 2018218071 A
- WO 2018218069 A
- WO 2018206539 A
- WO 2018143315 A
- WO 2018140600 A
- WO 2018140599 A
- WO 2018140598 A
- WO 2018140514 A
- WO 2018140513 A
- WO 2018140512 A
- WO 2018119183 A
- WO 2018112420 A
- WO 2018068017 A
- WO 2018064510 A
- WO 2017201161 A
- WO 2017172979 A
- WO 2017100546 A
- WO 2017087528 A
- WO 2017058807 A
- WO 2017058805 A
- WO 2017058728 A
- WO 2017058902 A
- WO 2017058792 A
- WO 2017058768 A
- WO 2017058915 A
- WO 2017015562 A
- WO 2016168540 A
- WO 2016164675 A
- WO 2016049568 A
- WO 2016049524 A
- WO 2015054572 A
- WO 2014152588 A
- WO 2014143659 A
- WO 2013155223 A
- EP 0520722 A
- EP 0566226 A
- WO 9633980 A
- WO 9630347 A
- EP 0787772 A
- WO 9730034 A
- WO 9730044 A
- WO 9738994 A
- WO 9749688 A
- EP 837063 A
- WO 9802434 A
- WO 9738983 A
- WO 9519774 A
- WO 9519970 A
- WO 9713771 A
- WO 9802437 A
- WO 9802438 A
- WO 9732881 A
- DE 19629652
- WO 9833798 A
- WO 9732880 A
- EP 682027 A
- WO 9702266 A
- WO 9727199 A
- WO 9807726 A
- WO 9734895 A
- WO 9631510 A
- WO 9814449 A
- WO 9814450 A
- WO 9814451 A
- WO 9509847 A
- WO 9719065 A
- WO 9817662 A
- US 5789427 A
- US 5650415 A
- US 5656643 A
- WO 9935146 A
- WO 9935132 A
- WO 9907701 A
- WO 9220642 A
- WO 06044453 A
- WO 09036082 A
- WO 09055730 A
- WO 06122806 A
- WO 08070740 A
- WO 05011700 A
- US 6656963 B
- WO 9409010 A
- WO 9802441 A
- WO 0114387 A
- WO 05005434 A
- US 5258389 A
- US 5118677 A
- US 5118678 A
- US 5100883 A
- US 5151413 A
- US 5120842 A
- US 5256790 A

- WO 94090101 A
- WO 9205179 A
- WO 93111130 A
- WO 9402136 A
- WO 9402485 A
- WO 9514023 A
- WO 9516691 A
- WO 9641807 A
- WO 2018204416 A
- WO 05016252 A
- WO 2019212990 A
- WO 2019212991 A
- WO 06121168 A1
- US 6111090 A
- US 8586023 B
- WO 2010003118 A
- WO 2011090754 A
- US 7025962 B
- EP 1947183 A
- US 7812135 B
- US 8388967 B
- US 8591886 B
- US 7618632 B
- EP 1866339 A
- WO 2011028683 A
- WO 2013039954 A
- WO 05007190 A
- WO 07133822 A
- WO 05055808 A
- WO 9940196 A
- WO 0103720 A
- WO 9920758 A
- WO 06083289 A
- WO 05115451 A
- WO 2011051726 A
- WO 9633172 A
- WO 9627583 A
- WO 9807697 A
- WO 9803516 A
- WO 9834918 A
- WO 9834915 A
- WO 9833768 A
- WO 9830566 A
- WO 9005719 A
- WO 9952910 A
- WO 9952889 A
- WO 9929667 A
- WO 99007675 A
- EP 0606046 A
- EP 0780386 A
- EP 1786785 A
- EP 1181017 A
- EP 0818442 A
- EP 1004578 A
- US 20090012085 A
- US 5863949 A
- US 5861510 A
- US 20030162712 A
- US 6413932 B
- US 6727225 B
- US 20020042368 A
- US 5981245 A
- US 5728813 A
- US 5969110 A
- US 6596852 B
- US 6232447 B
- US 6057124 A
- US 5712291 A
- US 5892112 A
- US 5792783 A
- EP 0970070 A
- JP 02233610 A
- EP 407122 A

**Non-patent literature cited in the description**


- **CHAN, G. et al.** *Cancer Metastasis Rev,* 2008, vol. 27, 179-192
- **ZHANG, J. et al.** *J. Cell. Mol. Med.,* 2015, vol. 19, 2075-2083
- **ROCCOGRANDI L. et al.** *J. Neuro-Oncol.,* 2017, vol. 135, 487-496
- **MITRA R. et al.** *ChemMedChem,* 2021, vol. 16, 777-787
- **FOSTER.** Deuterium Isotope Effects in Studies of Drug Metabolism. *Trends Pharmacol. Sci.,* 1984, vol. 5 (12), 524-527
- Remington: The Science and Practice of Pharmacy. Lippincott Williams & Wilkins, 2005
- **BLACK et al.** *Neurology,* 2005, vol. 65, S3-S6
- **PREUSSER, M. et al.** *Nat. Rev. Neurol.,* 2015
- **SALTZ et al.** *Proc. Am. Soc. Clin. Oncol.,* 1999, vol. 18, 233
- **DOUILLARD et al.** *Lancet,* 2000, vol. 355 (9209), 1041-1047
- *Agnew, Chem. Intl. Ed Engl.,* 1994, vol. 33, 183-186
- *Cancers (Basel),* September 2015, vol. 7 (3), 1758-1784
- *PLoS One,* 25 November 2014, vol. 9 (11
- *Clin Cancer Res.,* 01 March 2011, vol. 17 (5), 989-1000
- **MODJTAHEDI et al.** *Br. J. Cancer,* 1993, vol. 67, 247-253
- **TERAMOTO et al.** *Cancer,* 1996, vol. 77, 639-645
- **GOLDSTEIN et al.** *Clin. Cancer Res.,* 1995, vol. 1, 1311-1318
- **HUANG et al.** *Cancer Res.,* 1999, vol. 59 (8), 1935-40

- **YANG et al.** *Cancer Res.,* 1999, vol. 59, 1236-1243
- **YAN et al.** Pharmacogenetics and Pharmacogenomics In Oncology Therapeutic Antibody Development. *BioTechniques,* 2005, vol. 39 (4), 565-8
- **PAEZ et al.** EGFR Mutations In Lung Cancer Correlation With Clinical Response To Gefitinib Therapy. *Science,* 2004, vol. 304 (5676), 1497-500
- **TRAXLER et al.** *Exp. Opin. Ther. Patents,* 1998, vol. 8 (12), 1599-1625
- **BARNETT et al.** *Biochem. J.,* 2005, vol. 385, 399-408
- **JIN et al.** *Br. J. Cancer,* 2004, vol. 91, 1808-12
- **SARKAR ; LI.** *J Nutr.,* 2004, vol. 134 (12), 3493S-3498S
- **DASMAHAPATRA et al.** *Clin. Cancer Res.,* 2004, vol. 10 (15), 5242-52
- **GILLS ; DENNIS.** *Expert. Opin. Investig. Drugs,* 2004, vol. 13, 787-97
- **YANG et al.** *Cancer Res.,* 2004, vol. 64, 4394-9
- **GOLDBERG et al.** *Blood,* 2007, vol. 110 (1), 186-192
- **THOMPSON et al.** *Clin. Cancer Res.,* 2007, vol. 13 (6), 1757-1761